# EUROPEAN PATENT APPLICATION

(11) **EP 2 090 576 A1**
(43) Date of publication of application: **19.08.2009**
(21) Application number: 08250387.1
(22) Date of filing: 01.02.2008
(51) Int. Cl.: C07D 471/04, C07D 487/04, C07D 493/04, C07D 495/04, C07D 498/04, A61K 31/437, A61P 25/00

(54) **6-halo-pyrazolo[1,5-a]pyridines, a process for their preparation and their use as metabotropic glutamate receptor (mGluR) modulators**

(71) Applicant: Merz Pharma GmbH & Co. KGaA, 60318 Frankfurt (DE)
(72) Inventor: The designation of the inventor has not yet been filed
(74) Representative: Goddard, Christopher Robert

(57) **Abstract**

The invention relates to 6-halo-pyrazolo[1,5-a]pyridines of formula (I) as well as their pharmaceutically acceptable salts. The invention further relates to a process for the preparation of such compounds. The compounds of the invention are mGluR5 modulators and are therefore useful for the control and prevention of acute and/or chronic neurological disorders. 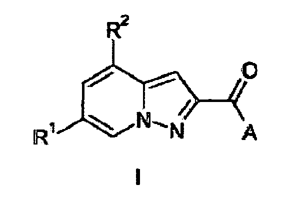 wherein A represents -NR³R⁴
with R³ and R⁴ as described herein.

## Description

### FIELD OF THE INVENTION

The present invention relates to pyrazolopyridine derivatives, which can act as novel metabotropic glutamate receptor (mGluR) modulators, methods for their synthesis and their use as a medicament for the treatment of various diseases and/or prevention of disorders, e. g. neurological disorders, by administration of such substances.

### BACKGROUND OF THE INVENTION

Neuronal stimuli are transmitted by the central nervous system (CNS) through the interaction of a neurotransmitter released by a neuron, which neurotransmitter has a specific effect on a neuroreceptor of another neuron. L-glutamic acid is considered to be a major excitatory neurotransmitter in the mammalian CNS, consequently playing a critical role in a large number of physiological processes. Glutamate-dependent stimulus receptors are divided into two main groups. The first group comprises ligand-controlled ion channels whereas the other comprise metabotropic glutamate receptors (mGluR). Metabotropic glutamate receptors are a subfamily of G-protein-coupled receptors (GPCR). There is increasing evidence for a peripheral role of both ionotropic and metabotropic glutamate receptors outside the CNS e.g, in chronic pain states.

At present, eight different members of these mGluRs are known. On the basis of structural parameters such as sequence homology, the second messenger system utilized by these receptors and their different affinity to low-molecular weight compounds, these eight receptors can be divided into three groups. MGluR1 and mGluR5 belong to Group I which are positively coupled to phospholipase C and their activation leads to a mobilization of intracellular calcium ions. MGluR2 and mGluR3 belong to Group II and mGluR4, mGluR6, mGluR7 and mGluR8 belong to Group III, both of which are negatively coupled to adenylyl cyclase, i.e., their activation causes a reduction in second messenger cAMP and thus a dampening of neuronal activity.

The mGluR5 modulators have been shown to modulate the effects of the presynaptically released neurotransmitter glutamate via postsynaptic mechanisms. Moreover, as these modulators can be both positive and/or negative mGluR5 modulators, such modulators may increase or inhibit the effects mediated through these metabotropic glutamate receptors.

Of particular interest are those modulators which are negative mGluR5 modulators. Such modulators decrease the effects mediated through metabotropic glutamate receptors. Since a variety of patho-physiological processes and disease states affecting the CNS are thought to be related to abnormal glutamate neurotransmission, and mGluR5 receptors are shown to be expressed in many areas of the CNS and in PNS (peripheral nervous system), modulators of these receptors could be therapeutically beneficial in the treatment of diseases involving CNS and PNS.

Therefore, mGluR5 positive or negative modulators may be administered to provide neuroprotection and/or disease modification in the following acute or chronic pathological conditions or to provide a symptomatological effect on the following conditions:
Alzheimer's disease, Creutzfeld-Jakob's syndrome/disease, bovine spongiform encephalopathy (BSE), prion related infections, diseases involving mitochondrial dysfunction, diseases involving β-amyloid and/or tauopathy, Down's syndrome, hepatic encephalopathy, Huntington's disease, motor neuron diseases, amyotrophic lateral sclerosis (ALS), olivoponto-cerebellar atrophy, post-operative cognitive deficit (POCD), systemic lupus erythematosus, systemic sclerosis, Sjogren's syndrome, Neuronal Ceroid Lipofuscinosis, neurodegenerative cerebellar ataxias, Parkinson's disease, Parkinson's dementia, mild cognitive impairment, cognitive deficits in various forms of mild cognitive impairment, cognitive deficits in various forms of dementia, dementia pugilistica, vascular and frontal lobe dementia, cognitive impairment, learning impairment, eye injuries, eye diseases, eye disorders, glaucoma, retinopathy, macular degeneration, head or brain or spinal cord injuries, head or brain or spinal cord trauma, trauma, hypoglycaemia, hypoxia, perinatal hypoxia, ischaemia, ischaemia resulting from cardiac arrest or stroke or bypass operations or transplants, convulsions, epileptic convulsions, epilepsy, temporal lobe epilepsy, myoclonic epilepsy, inner ear insult, inner ear insult in tinnitus, tinnitus, sound-or drug-induced inner ear insult, sound- or drug-induced tinnitus, hyperacusis, L-dopa-induced dykinesias, L-dopa-induced dykinesias in Parkinson's disease therapy, dyskinesias, dyskinesia in Huntington's disease, drug induced dyskinesias, neuroleptic-induced dyskinesias, haloperidol-induced dyskinesias, dopaminomimetic-induced dyskinesias, chorea, Huntington's chorea, athetosis, dystonia, stereotypy, ballism, tardive dyskinesias, neuroleptics-induced dyskinesia, tic disorder, torticollis spasmodicus, blepharospasm, focal and generalized dystonia, nystagmus, hereditary cerebellar ataxias, corticobasal degeneration, tremor, essential tremor, abuse, addiction, nicotine addiction, nicotine abuse, alcohol addiction, alcohol abuse, opiate addiction, opiate abuse, cocaine addiction, cocaine abuse, amphetamine addiction, amphetamine abuse, anxiety disorders, panic disorders, anxiety and panic disorders, social anxiety disorder (SAD), attention deficit hyperactivity disorder (ADHD), attention deficit syndrome (ADS), restless leg syndrome (RLS), hyperactivity in children, autism, dementia, dementia in Alzheimer's disease, dementia in Korsakoff syndrome, Korsakoff syndrome, vascular dementia, dementia related to HIV infections, HIV-1 encephalopathy, AIDS encephalopathy, AIDS dementia complex, AIDS-related dementia, major depressive disorder, major depression, depression, depression resulting from Borna virus infection, major depression resulting from Borna virus infection, bipolar manic-depressive disorder, drug tolerance, drug tolerance to opioids, movement disorders, fragile-X syndrome, irritable bowel syndrome (IBS), migraine, multiple sclerosis (MS), muscle spasms, pain, chronic pain, acute pain, inflammatory pain, neuropathic pain, diabetic neuropathic pain (DNP), pain related to rheumatic arthritis, allodynia, hyperalgesia, nociceptive pain, cancer pain, posttraumatic stress disorder (PTSD), schizophrenia, positive or cognitive or negative symptoms of schizophrenia, spasticity, Tourette's syndrome, urinary incontinence, vomiting, pruritic conditions, pruritis, sleep disorders, micturition disorders, neuromuscular disorder in the lower urinary tract, gastroesophageal reflux disease (GERD), gastrointestinal dysfunction, lower esophageal sphincter (LES) disease, functional gastrointestinal disorders, dyspepsia, regurgitation, respiratory tract infection, bulimia nervosa, chronic laryngitis, asthma, reflux-related asthma, lung disease, eating disorders, obesity, obesity-related disorders, obesity abuse, food addiction, binge eating disorders, agoraphobia, generalized anxiety disorder, obsessive-compulsive disorder, panic disorder, posttraumatic stress disorder, social phobia, phobic disorders, substance-induced anxiety disorder, delusional disorder, schizoaffective disorder, schizophreniform disorder, substance-induced psychotic disorder, or delirium, diabetes, hyperammonemia and liver failure and sleep disturbances..

The mGluR5 negative or positive modulators may also be administered to provide inhibition of tumour cell growth, migration, invasion, adhesion and toxicity in the peripheral tissues, peripheral nervous system and CNS. MGluR5 modulators may be administered to provide therapeutic intervention in neoplasia, hyperplasia, dysplasia, cancer, carcinoma, sarcoma, oral cancer, squamous cell carcinoma (SCC), oral squamous cell carcinoma (SCC), lung cancer, lung adenocarcinoma, breast cancer, prostate cancer, gastric cancer, liver cancer, colon cancer, colorectal carcinoma, rhabdomyosarcoma, brain tumour, tumour of a nerve tissue, glioma, malignant glioma, astroglioma, neuroglioma, neuroblastoma, glioblastoma, medulloblastoma, cancer of skin cells, melanoma, malignant melanoma, epithelial neoplasm, lymphoma, myeloma, Hodgkin's disease, Burkitt's lymphoma, leukemia, thymoma, and other tumours.

The mGluR5 positive or negative modulators may also be administered to provide disease modification an/or to provide a symptomatological effect on the following conditions: diabetes, hyperammonemia and liver failure.

Further indications for mGluR5 negative or positive modulators include those indications wherein a particular condition does not necessarily exist but wherein a particular physiological parameter may be improved through administration of the instant compounds, for example cognitive enhancement, learning impairment and/or neuroprotection.

Positive modulators may be particularly useful in the treatment of positive and negative symptoms in schizophrenia and cognitive deficits in various forms of dementia and mild cognitive impairment.

In the literature, several types of modulators of mGluR5 have already been described.

Furthermore, several types of pyrazolopyridine compounds have been disclosed in the prior art.

In WO 2004/087153, compounds of the following structure are disclosed as small molecule immuno-potentiators: 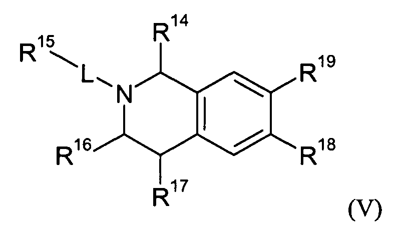 wherein L is a covalent bond or selected from the group consisting of CH₂-, -CO-, -O-, -S-, CHF, -NH-, -NR²⁰-, and R¹⁵ is selected from a group consisting of substituted or unsubstituted carbocyclyl, aryl, arylalkyl, alkoxyaryl, heteroaryl.

In WO 2003/078435 some pyrazolopyridine compounds and their use in the treatment of CNS disorders are disclosed. WO 2003/078435 discloses compounds of the following general formula (I) 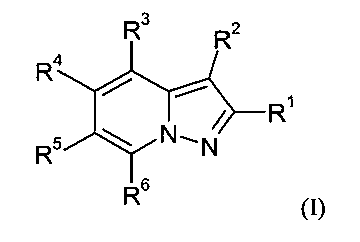 wherein:
R² is selected from a diverse set of substituents e.g., -NR⁷R⁸, -OR⁷, alkyl, substituted alkyl, cycloalkyl, which substituents do not include hydrogen.

In WO 2006/01537 compounds of formula (I) and their use in the treatment of CNS disorders are disclosed:

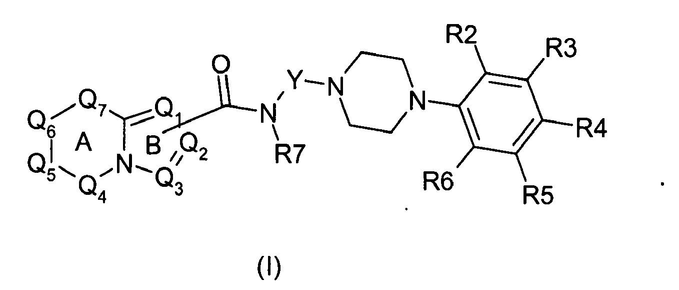

In the compounds of formula (I), the AB ring may represent a optionally substituted pyrazolopyridine ring.

In WO 2005/009947 compounds of formula IA and formula IB and their use as anticancer agents are disclosed: 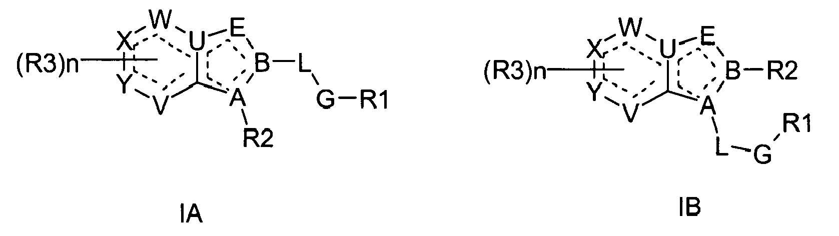 L-G-R1 represents

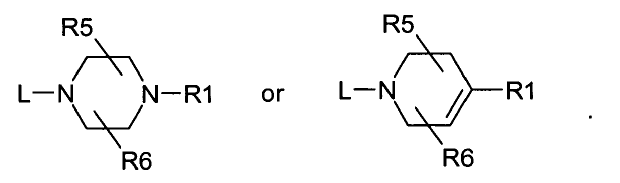

In the compounds of formula IA and formula IB, the bicyclic ring A-W may represent a pyrazolopyridine ring.

### THE PRESENT INVENTION

It now has been found that certain pyrazolopyridine derivatives which differ in structure from the known pyrazolopyridines, are potent mGluR5 modulators. Therefore, these substances may be therapeutically beneficial in the treatment of conditions which involve abnormal glutamate neurotransmission or in which modulation of mGluR5 receptors results in therapeutic benefit. These substances are preferably administered in the form of a pharmaceutical composition, wherein they are present together with one or more pharmaceutically acceptable diluents, carriers, or excipients.

### OBJECTS OF THE INVENTION

It is an object of the present invention to provide novel pharmaceutical compounds which are mGluR5 modulators and pharmaceutical compositions thereof. It is a further object of the invention to provide a novel method of treating, eliminating, alleviating, palliating, or ameliorating undesirable CNS disorders which involve abnormal glutamate neurotransmission by employing a compound of the invention or a pharmaceutical composition containing the same.

An additional object of the invention is the provision of processes for producing the pyrazolopyridine derivatives.

### SUMMARY OF THE INVENTION

What we therefore believe to be comprised by our invention may be summarized inter alia in the following words:
A compound selected from those of Formula I 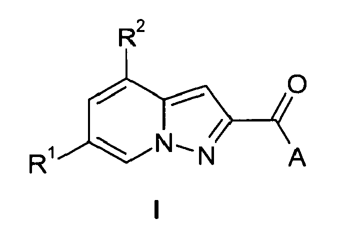 wherein
R¹ represents chloro or bromo;
R² represents hydrogen, C₁₋₆alkyl, amino, hydroxy, halogen, trifluoromethyl, C₁₋₆alkoxy, nitro, cyano, trifluoromethoxy, aminocarbonyl, C₁₋₆alkylaminocarbonyl, di-C₁₋₆alkylaminocarbonyl, hydroxyC₁₋₆alkyl, C₂₋₆alkenyl, C₁₋₆alkoxycarbonyl, C₁₋₆alkylcarbonyloxy, C₁₋₆alkylamino, aryl, heteroaryl, heterocyclyl, or cycloC₃₋₁₂alkyl; and
A represents -NR³R⁴, wherein
R³ represents hydrogen, C₁₋₆alkyl, cycloC₃₋₁₂alkyl, aryl, heteroaryl, arylC₁₋₆alkyl, and heteroarylC₁₋₆alkyl and
R⁴ represents C₁₋₆alkyl, cycloC₃₋₁₂alkyl, aryl, heteroaryl, arylC₁₋₆alkyl, and heteroarylC₁₋₆alkyl; or
R³ and R⁴, together with the nitrogen to which they are attached, combine to form a partially unsaturated bicyclic or tricyclic ring system having 9 to 14 members comprising 1 to 8 heteroatoms selected from oxygen, sulfur, and nitrogen, wherein the ring system is optionally substituted with a group selected from halogen, amino, hydroxy, nitro, cyano, trifluoromethyl, trifluoromethoxy, aryl, C₁₋₆alkyl, cycloC₃₋₁₂alkyl, C₂₋₆alkenyl, C₂₋₆alkynyl, C₁₋₆alkoxy, cycloC₃₋₁₂alkyloxy, C₂₋₆alkenyloxy, C₂₋₆alkynyloxy, heteroaryl, heterocyclyl, aryloxy, heteroaryloxy, heterocyclyloxy, C₁₋₆alkylamino, di-C₁₋₆alkylamino, cycloC₃₋₁₂alkylamino, di-cycloC₃₋₁₂alkylamino, N-C₁₋₆alkyl-N- cycloC₃₋₁₂alkylamino, C₂₋₆alkenylamino, C₂₋₆alkynylamino, di-C₂₋₆alkenylamino, di-C₂₋₆alkynylamino, N-C₁₋₆alkyl-N-C₂₋₆alkenylamino, N-C₁₋₆alkyl-N-C₂₋₆alkynylamino, N-C₂₋₆alkenyl-N-cycloC₃₋₁₂alkylamino, N-C₂₋₆alkynyl-N-cycloC₃₋₁₂alkylamino, N-C₂₋₆alkenyl-N-C₂₋₆alkynylamino arylamino, diarylamino, aryl-C₁₋₆alkylamino, aryl-C₂₋₆alkenylamino, aryl-C₂₋₆alkynylamino, N-aryl-N-cycloC₃₋₁₂alkylamino, heteroarylamino, diheteroarylamino, heteroaryl-C₁₋₆alkylamino, heteroaryl-C₂₋₆alkenylamino, heteroaryl-C₂₋₆alkynylamino, N-heteroaryl-N-cycloC₃₋₁₂alkylamino, N-heteroaryl-N-arylamino, heterocyclylamino, diheterocyclylamino, heterocyclyl-C₁₋₆alkylamino, heterocyclyl-C₂₋₆alkenylamino, heterocyclyl-C₂₋₆alkynylamino, N-heterocyclyl-N-cycloC₃₋₁₂alkylamino, N-heterocyclyl-N-arylamino, N-heterocyclyl-N-heteroarylamino, acyl, acyloxy, acylamino, C₁₋₆alkoxycarbonyl, cycloC₃₋₁₂alkoxycarbonyl, C₂₋₆alkenyloxycarbonyl, C₂₋₆alkynyloxycarbonyl, aryloxycarbonyl, heteroaryloxycarbonyl, heterocyclyloxycarbonyl, aminocarbonyl, C₁₋₆alkylaminocarbonyl, di-C₁₋₆alkylaminocarbonyl, cycloC₃₋₁₂alkylaminocarbonyl, di-cycloC₃₋₁₂alkylaminocarbonyl, N-C₁₋₆alkyl-N-cycloC₃₋₁₂alkylaminocarbonyl, C₂₋₆alkenylaminocarbonyl, C₂₋₆alkynylaminocarbonyl, di-C₂₋₆alkenylaminocarbonyl, di-C₂₋₆alkynylaminocarbonyl, N-C₁₋₆alkyl-N-C₂₋₆alkenylaminocarbonyl, N-C₁₋₆alkyl-N-C₂₋₆alkynylaminocarbonyl, N-C₂₋₆alkenyl-N-cycloC₃₋₁₂alkylaminocarbonyl, N-C₂₋₆alkynyl-N-cycloC₃₋₁₂alkylaminocarbonyl, N-C₂₋₆alkenyl-N-C₂₋₆alkynylaminocarbonyl, arylaminocarbonyl, diarylaminocarbonyl, aryl-C₁₋₆alkylaminocarbonyl, aryl-C₂₋₆alkenylaminocarbonyl, aryl-C₂₋₆alkynylamino-carbonyl, N-aryl-N-cyclo C₃₋₁₂alkylaminocarbonyl, heteroarylaminocarbonyl, diheteroarylaminocarbonyl, heteroarylC₁₋₆alkylaminocarbonyl, heteroaryl-C₂₋₆alkenylaminocarbonyl, heteroaryl-C₂₋₆alkynylaminocarbonyl, N-heteroaryl-N-cycloC₃₋₁₂alkylaminocarbonyl, N-heteroaryl-N-arylaminocarbonyl, heterocyclylaminocarbonyl, diheterocyclylaminocarbonyl, heterocyclyl-C₁₋₆alkylaminocarbonyl, heterocyclyl-C₂₋₆alkenylaminocarbonyl, heterocyclyl-C₂₋₆alkynylaminocarbonyl, N-heterocyclyl-N-cycloC₃₋₁₂alkylaminocarbonyl, N-heterocyclyl-N-arylaminocarbonyl, N-heterocyclyl-Nheteroarylaminocarbonyl, C₁₋₆alkylsulfinyl, cycloC₃₋₁₂alkylsulfinyl, C₂₋₆alkenylsulfinyl, C₂₋₆alkynylsulfinyl, arylsulfinyl, heteroarylsulfinyl, heterocyclylsulfinyl, C₁₋₆alkylsulfonyl, cycloC₃₋₁₂alkylsulfonyl, C₂₋₆alkenylsulfonyl, C₂₋₆alkynylsulfonyl, arylsulfonyl, heteroarylsulfonyl, heterocyclylsulfonyl, C₁₋₆alkylsulfonylamino, arylsulfonylamino, and oxo;
and optical isomers, pharmaceutically acceptable salts, hydrates, solvates and polymorphs thereof.

Such a compound of Formula I, wherein A represents 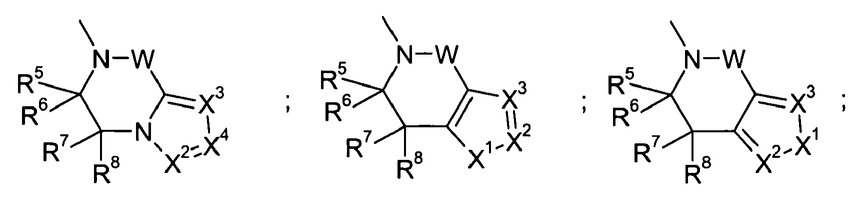 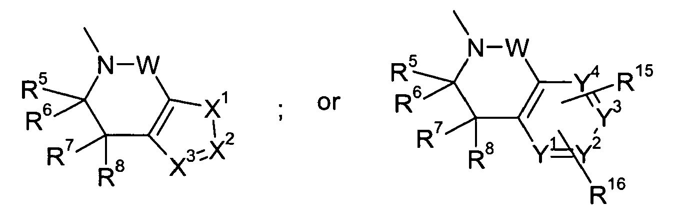 wherein
R⁵, R⁶, R⁷, and R⁸, which may be the same or different, each independently represent hydrogen, C₁₋₆alkyl, cycloC₃₋₁₂alkyl, amino, hydroxy, halogen, or trifluoromethyl;
W represents NR⁹ or CR¹⁰R¹¹, wherein
R⁹ represents hydrogen, C₁₋₆alkyl_{;} trifluoromethyl or cycloC₃₋₁₂alkyl and
R¹⁰ and R¹¹, which may be the same or different each independently represent hydrogen, C₁₋₆alkyl, cycloC₃₋₁₂alkyl, or trifluoromethyl;
X¹ represents CR¹²R¹³, NR¹⁴, S, or O; and X², X³, and X⁴, which may be the same or different each independently represent CR¹² or N, wherein
R¹² and R¹³, which may be the same or different, each independently represent hydrogen, halogen, amino, hydroxy, nitro, cyano, trifluoromethyl, trifluoromethoxy, aryl, C₁₋₆alkyl, cycloC₃₋₁₂alkyl, C₂₋₆alkenyl, C₂₋₆alkynyl, C₁₋₆alkoxy, cycloC₃₋₁₂alkyloxy, C₂₋₆alkenyloxy, C₂₋₆alkynyloxy, heteroaryl, heterocyclyl, aryloxy, heteroaryloxy, heterocyclyloxy, C₁₋₆alkylamino, di-C₁₋₆alkylamino, cycloC₃₋₁₂alkylamino, di-cycloC₃₋₁₂alkylamino, N-C₁₋₆alkyl-N- cycloC₃₋₁₂alkylamino, C₂₋₆alkenylamino, C₂₋₆alkynylamino, di-C₂₋₆alkenylamino, di-C₂₋₆alkynylamino, N-C₁₋₆alkyl-N-C₂₋₆alkenylamino, N-C₁₋₆alkyl-N-C₂₋₆alkynylamino, N-C₂₋₆alkenyl-N-cycloC₃₋₁₂alkylamino, N-C₂₋₆alkynyl-N-cycloC₃₋₁₂alkylamino, N-C₂₋₆alkenyl-N-C₂₋₆alkynylamino arylamino, diarylamino, aryl-C₁₋₆alkylamino, aryl-C₂₋₆alkenylamino, aryl-C₂₋₆alkynylamino, N-aryl-N-cycloC₃₋₁₂alkylamino, heteroarylamino, diheteroarylamino, heteroaryl-C₁₋₆alkylamino, heteroaryl-C₂₋₆alkenylamino, heteroaryl-C₂₋₆alkynylamino, N-heteroaryl-N-cycloC₃₋₁₂alkylamino, N-heteroaryl-N-arylamino, heterocyclylamino, diheterocyclylamino, heterocyclyl-C₁₋₆alkylamino, heterocyclyl-C₂₋₆alkenylamino, heterocyclyl-C₂₋₆alkynylamino, N-heterocyclyl-N-cycloC₃₋₁₂alkylamino, N-heterocyclyl-N-arylamino, N-heterocyclyl-N-heteroarylamino, acyl, acyloxy, acylamino, C₁₋₆alkoxycarbonyl, cycloC₃₋₁₂alkoxycarbonyl, C₂₋₆alkenyloxycarbonyl, C₂₋₆alkynyloxycarbonyl, aryloxycarbonyl, heteroaryloxycarbonyl, heterocyclyloxycarbonyl, aminocarbonyl, C₁₋₆alkylaminocarbonyl, di-C₁₋₆alkylaminocarbonyl, cycloC₃₋₁₂alkylaminocarbonyl, di-cycloC₃₋₁₂alkylaminocarbonyl, N-C₁₋₆alkyl-N-cycloC₃₋₁₂alkylaminocarbonyl, C₂₋₆alkenylaminocarbonyl, C₂₋₆alkynylaminocarbonyl, di-C₂₋₆alkenylaminocarbonyl, di-C₂₋₆alkynylaminocarbonyl, N-C₁₋₆alkyl-N-C₂₋₆alkenylaminocarbonyl, N-C₁₋₆alkyl-N-C₂₋₆alkynylaminocarbonyl, N-C₂₋₆alkenyl-N-cycloC₃₋₁₂alkylaminocarbonyl, N-C₂₋₆alkynyl-N-cycloC₃₋₁₂alkylaminocarbonyl, N-C₂₋₆alkenyl-N-C₂₋₆alkynylaminocarbonyl, arylaminocarbonyl, diarylaminocarbonyl, aryl-C₁₋₆alkylaminocarbonyl, aryl-C₂₋₆alkenylaminocarbonyl, aryl-C₂₋₆alkynylaminocarbonyl, N-aryl-N-cyclo C₃₋₇alkylaminocarbonyl, heteroarylaminocarbonyl, diheteroarylaminocarbonyl, heteroaryl-C₁₋₆alkylaminocarbonyl, heteroaryl-C₂₋₆alkenylaminocarbonyl, heteroaryl-C₂₋₆alkynylaminocarbonyl, N-heteroaryl-N-cycloC₃₋₁₂alkylaminocarbonyl, N-heteroaryl-N-arylaminocarbonyl, heterocyclylaminocarbonyl, diheterocyclylaminocarbonyl, heterocyclyl-C₁₋₆alkylaminocarbonyl, heterocyclyl-C₂₋₆alkenylaminocarbonyl, heterocyclyl-C₂₋₆alkynylaminocarbonyl, N-heterocyclyl-N-cycloC₃₋₁₂alkylaminocarbonyl, N-heterocyclyl-N-arylaminocarbonyl, N-heterocyclyl-Nheteroarylaminocarbonyl, C₁₋₆alkylsulfinyl, cycloC₃₋₁₂alkylsulfinyl, C₂₋₆alkenylsulfinyl, C₂₋₆alkynylsulfinyl, arylsulfinyl, heteroarylsulfinyl, heterocyclylsulfinyl, C₁₋₆alkylsulfonyl, cycloC₃₋₁₂alkylsulfonyl, C_{2- 6}alkenylsulfonyl, C₂₋₆alkynylsulfonyl, arylsulfonyl, heteroarylsulfonyl, heterocyclylsulfonyl, C₁₋₆alkylsulfonylamino, or arylsulfonylamino and
R¹⁴ represents hydrogen, C₁₋₆alkyl, cycloC₃₋₁₂alkyl, C₂₋₆alkenyl, acyl, aryl, heteroaryl, heterocyclyl, C₁₋₆alkylaminocarbonyl, di-C₁₋₆alkylaminocarbonyl, C₁₋₆alkylsulfonyl, arylsulfonyl or heteroarylsulfonyl;
Y¹, Y², Y³, and Y⁴ represent C or N, wherein at least two of Y¹, Y², Y³, and Y⁴ represent C;
R¹⁵ and R¹⁶, which may be the same or different, each independently represent hydrogen, halogen, amino, hydroxy, nitro, cyano, trifluoromethyl, trifluoromethoxy, aryl, C₁₋₆alkyl, cycloC₃₋₁₂alkyl, C₂₋₆alkenyl, C₂₋₆alkynyl, C₁₋₆alkoxy, cycloC₃₋₁₂alkyloxy, C₂₋₆alkenyloxy, C₂₋₆alkynyloxy, heteroaryl, heterocyclyl, aryloxy, heteroaryloxy, heterocyclyloxy, C₁₋₆alkylamino, di-C₁₋₆alkylamino, cycloC₃₋₁₂alkylamino, di-cycloC₃₋₁₂alkylamino, N-C₁₋₆alkyl-N-cycloC₃₋₁₂alkylamino, C₂₋₆alkenylamino, C₂₋₆alkynylamino, di-C₂₋₆alkenylamino, di-C₂₋₆alkynylamino, N-C₁₋₆alkyl-N-C₂₋₆alkenylamino, N-C₁₋₆alkyl-N-C₂₋₆alkynylamino, N-C₂₋₆alkenyl-N-cycloC₃₋₁₂alkylamino, N-C₂₋₆alkynyl-N-cycloC₃₋₁₂alkylamino, N-C₂₋₆alkenyl-N-C₂₋₆alkynylamino arylamino, diarylamino, aryl-C₁₋₆alkylamino, aryl-C₂₋₆alkenylamino, aryl-C₂₋₆alkynylamino, N-aryl-N-cycloC₃₋₁₂alkylamino, heteroarylamino, diheteroarylamino, heteroaryl-C₁₋₆alkylamino, heteroaryl-C₂₋₆alkenylamino, heteroaryl-C₂₋₆alkynylamino, N-heteroaryl-N-cycloC₃₋₁₂alkylamino, N-heteroaryl-N-arylamino, heterocyclylamino, diheterocyclylamino, heterocyclyl-C₁₋₆alkylamino, heterocyclyl-C₂₋₆alkenylamino, heterocyclyl-C₂₋₆alkynylamino, N-heterocyclyl-N-cycloC₃₋₁₂alkylamino, N-heterocyclyl-N-arylamino, N-heterocyclyl-N-heteroarylamino, acyl, acyloxy, acylamino, C₁₋₆alkoxycarbonyl, cycloC₃₋₁₂alkoxycarbonyl, C₂₋₆alkenyloxycarbonyl, C₂₋₆alkynyloxycarbonyl, aryloxycarbonyl, heteroaryloxycarbonyl, heterocyclyloxycarbonyl, aminocarbonyl, C₁₋₆alkylaminocarbonyl, di-C₁₋₆alkylaminocarbonyl, cycloC₃₋₁₂alkylaminocarbonyl, di-cycloC₃₋₁₂alkylaminocarbonyl, N-C₁₋₆alkyl-N-cycloC₃₋₁₂alkylaminocarbonyl, C₂₋₆alkenylaminocarbonyl, C₂₋₆alkynylaminocarbonyl, di-C₂₋₆alkenylaminocarbonyl, di-C_{2- 6}alkynylaminocarbonyl, N-C₁₋₆alkyl-N-C₂₋₆alkenylaminocarbonyl, N-C₁₋₆alkyl-N-C₂₋₆alkynylaminocarbonyl, N-C₂₋₆alkenyl-N-cycloC₃₋₁₂alkylaminocarbonyl, N-C₂₋₆alkynyl-N-cycloC₃₋₁₂alkylaminocarbonyl, N-C₂₋₆alkenyl-N-C₂₋₆alkynylaminocarbonyl, arylaminocarbonyl, diarylaminocarbonyl, aryl-C₁₋₆alkylaminocarbonyl, aryl-C₂₋₆alkenylaminocarbonyl, aryl-C₂₋₆alkynylamino-carbonyl, N-aryl-N-cyclo C₃₋₇alkylaminocarbonyl, heteroarylaminocarbonyl, diheteroarylaminocarbonyl, heteroaryl-C₁₋₆alkylaminocarbonyl, heteroaryl-C₂₋₆alkenylaminocarbonyl, heteroaryl-C₂₋₆alkynylaminocarbonyl, N-heteroaryl-N-cycloC₃₋₁₂alkylaminocarbonyl, N-heteroaryl-N-arylaminocarbonyl, heterocyclylaminocarbonyl, diheterocyclylaminocarbonyl, heterocyclyl-C₁₋₆alkylaminocarbonyl, heterocyclyl-C₂₋₆alkenylaminocarbonyl, heterocyclyl-C₂₋₆alkynylaminocarbonyl, N-heterocyclyl-N-cycloC₃₋₁₂alkylaminocarbonyl, N-heterocyclyl-N-arylaminocarbonyl, N-heterocyclyl-Nheteroarylaminocarbonyl, C₁₋₆alkylsulfinyl, cycloC₃₋₁₂alkylsulfinyl, C₂₋₆alkenylsulfinyl, C₂₋₆alkynylsulfinyl, arylsulfinyl, heteroarylsulfinyl, heterocyclylsulfinyl, C₁₋₆alkylsulfonyl, cycloC₃-₁₂alkylsulfonyl, C₂₋₆alkenylsulfonyl, C₂₋₆alkynylsulfonyl, arylsulfonyl, heteroarylsulfonyl, heterocyclylsulfonyl, C₁₋₆alkylsulfonylamino, or arylsulfonylamino;
or R¹⁵ and R¹⁶ together with the two carbon atoms carying them represent an aryl group which may be optionally substituted by a group selected from halogen, hydroxy, nitro, cyano, trifluoromethyl, trifluoromethoxy, C₁₋₆alkyl, and C₁₋₆alkoxy; a heteroaryl group having 5 or 6 ring members which may be optionally substituted by a group selected from halogen, hydroxy, nitro, cyano, trifluoromethyl, trifluoromethoxy, C₁₋₆alkyl, and C₁₋₆alkoxy; or a heterocyclyl group having 5 or 6 ring members, which may be optionally substituted by a group selected from oxo, halogen, hydroxy, nitro, cyano, trifluoromethyl, trifluoromethoxy, C₁₋₆alkyl, and C₁₋₆alkoxy;
and optical isomers, pharmaceutically acceptable salts, hydrates, solvates, and polymorphs thereof.

Such a compound of Formula I, wherein A represents 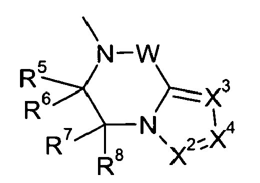 wherein R⁵, R⁶, R⁷, and R⁸, which may be the same or different, each independently represent hydrogen, C₁₋₆alkyl, and X², X³, and X⁴, which may be the same or different, each independently represent CR¹² or N.

Such a compound of Formula (I), wherein W represents CR¹⁰R¹¹, and R¹⁰ and R¹¹, which may be the same or different, each independently represent hydrogen or C₁₋₆alkyl, and R¹² represents hydrogen, halogen, cyano, C₁₋₆alkyl, C₁₋₆alkoxycarbonyl, C₁₋₆alkylaminocarbonyl, cycloC₃₋₁₂alkylaminocarbonyl, aryl or heteroaryl.

Such a compound of Formula (I), wherein R¹⁰ and R¹¹, which may be the same or different, each independently represent hydrogen or methyl, and R¹² represents hydrogen, halogen, cyano, C₁₋₆alkyl, C₁₋₆alkylcarbonyl, C₁₋₆alkoxycarbonyl, C₁₋₆alkylaminocarbonyl, cycloC₃₋₁₂alkylaminocarbonyl or a heteroaryl group selected from tetrazolyl, furyl, thienyl, pyridinyl, pyrimidinyl, wherein the heteroaryl group may be optionally substituted by one or more groups selected from halogen and C₁₋₆alkyl.

Such a compound of Formula I, wherein A represents 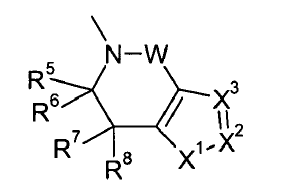 wherein R⁵, R⁶, R⁷, and R⁸ which may be the same or different, each independently represent hydrogen or C₁₋₆alkyl.

Such a compound of Formula I, wherein W represents CR¹⁰R¹¹; X¹ represents CR¹²R¹³, NR¹⁴, S, or O; and X² and X³, which may be the same or different, each independently represent CR¹² or N.

Such a compound of Formula I, wherein R¹⁰ and R¹¹, which may be the same or different, each independently represent hydrogen, C₁₋₆alkyl, or trifluoromethyl; R¹² and R¹³, which may be the same or different, each independently represent hydrogen, halogen, C₁₋₆alkyl, trifluoromethyl, C₁₋₆alkoxy, or aryl; and R¹⁴ represents hydrogen, C₁₋₆alkylcarbonyl, or heteroaryl (e.g., tetrazolyl).

Such a compound of Formula I, wherein W represents NR⁹; X¹ represents NR¹⁴, S, or O, and X² and X³, which may be the same or different, each independently represent CR¹² or N.

Such a compound of Formula I, wherein R⁹ represents hydrogen or C₁₋₆alkyl and R¹² represents hydrogen, halogen, or C₁₋₆alkyl.

Such a compound of Formula I, wherein A represents 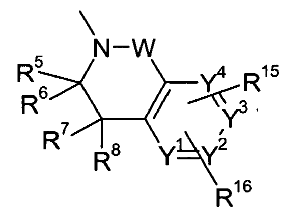 wherein R⁵, R⁶, R⁷, and R⁸ which may be the same or different, each independently represent hydrogen or C₁₋₆alkyl.

Such a compound of Formula **I**, wherein R⁵, R⁶, R⁷, and R⁸, which may be the same or different, each independently represent hydrogen, methyl, or ethyl.

Such a compound of Formula **I**, wherein W represents CR¹⁰R¹¹ and R¹⁰ and R¹¹, which may be the same or different, each independently represent hydrogen, C₁₋₆alkyl, trifluromethyl, or cycloC₃₋₁₂alkyl.

Such a compound of Formula **I**, wherein one of R¹⁰ and R¹¹ represents hydrogen or methyl and the other represents methyl, ethyl, propyl, isopropyl, trifluoromethyl, or cyclohexyl.

Such a compound of Formula **I**, wherein R¹⁵ and R¹⁶, which may be the same or different, each independently represent hydrogen, hydroxy, halogen, trifluoromethyl, cyano, nitro, C₁₋₆alkyl, C₁₋₆alkoxy, C₁₋₆alkylcarbonylamino, di-C₁₋₆alkylaminocarbonyl, C₁₋₆alkylsulfonyl, C₁₋₆alkoxycarbonyl, C₁₋₆alkylcarbonyl, di-C₁₋₆alkylamino, heterocyclylcarbonyl, heterocycyl, or heteroaryl.

Such a compound of Formula **I**, wherein R¹⁵ and R¹⁶, which may be the same or different, each independently represent hydrogen, hydroxy, halogen, trifluoromethyl, cyano, nitro, C₁₋₆alkyl, C₁₋₆alkoxy, C₁₋₆alkylcarbonylamino, di-C₁₋₆alkylaminocarbonyl, C₁₋₆alkylsulfonyl, C₁₋₆alkoxycarbonyl, C₁₋₆alkylcarbonyl, di-C₁₋₆alkylamino, heterocyclylcarbonyl, morpholinyl, piperidinyl, pyrrolidinyl, or a heteroaryl group selected from pyridinyl, pyrimidinyl, furyl, isoxazolyl, pyrazolyl, and tetrazolyl, wherein the heteroaryl group may be optionally substituted by one or more groups selected from halogen, C₁₋₆alkyl, and C₁₋₆alkoxy.

Such a compound of Formula **I**, wherein R¹⁵ and R¹⁶, together with the carbon atoms carrying them, represent a heterocyclyl group having 5 or 6 ring members, which may be optionally substituted by a group selected from oxo, halogen, or C₁₋₆alkyl.

Such a compound of Formula **I**, wherein W represents NR⁹ and R⁹ represents hydrogen or C₁₋₆alkyl.

Such a compound of Formula **I**, wherein R⁹ represents hydrogen or methyl.

Such a compound of Formula **I**, wherein R¹⁵ and R¹⁶, which may be the same or different, each independently represent hydrogen, halogen, trifluoromethyl, C₁₋₆alkyl, C₁₋₆alkoxy, C₁₋₆alkylcarbonylamino, C₁₋₆alkylsulfonyl, or heteroaryl.

Specific compounds of Formula **I** within the present invention include, but are not limited to, the following compounds (or salts therof):
(6-Bromo-pyrazolo[1,5-a]pyridin-2-yl)-(4-methyl-6,7-dihydro-4H-thieno[3,2-c]pyridin-5-yl)-methanone
(6-Chloro-pyrazolo[1,5-a]pyridin-2-yl)-(4-methyl-6,7-dihydro-4H-thieno[3,2-c]pyridin-5-yl)-methanone
(6-Chloro-pyrazolo[1,5-a]pyridin-2-yl)-(6,7-dihydro-4H-thieno[3,2-c]pyridin-5-yl)-methanone
(6-Bromo-pyrazolo[1,5-a]pyridin-2-yl)-(6,7-dihydro-4H-thieno[3,2-c]pyridin-5-yl)-methanone
(6-Chloro-pyrazolo[1,5-a]pyridin-2-yl)-(4,4-dimethyl-6,7-dihydro-4H-thieno[3,2-c]pyridin-5-yl)-methanone
(6-Bromo-pyrazolo[1,5-a]pyridin-2-yl)-(4,4-dimethyl-6,7-dihydro-4H-thieno[3,2-c]pyridin-5-yl)-methanone
(6-Chloro-pyrazolo[1,5-a]pyridin-2-yl)-(6-methyl-6,7-dihydro-4H-thieno[3,2-c]pyridin-5-yl)-methanone
(6-Bromo-pyrazolo[1,5-a]pyridin-2-yl)-(6-methyl-6,7-dihydro-4H-thieno[3,2-c]pyridin-5-yl)-methanone
(6-Bromo-pyrazolo[1,5-a]pyridin-2-yl)-(4-ethyl-6,7-dihydro-4H-thieno[3,2-c]pyridin-5-yl)-methanone
(6-Chloro-pyrazolo[1,5-a]pyridin-2-yl)-(4-ethyl-6,7-dihydro-4H-thieno[3,2-c]pyridin-5-yl)-methanone
(6-Bromo-pyrazolo[1,5-a]pyridin-2-yl)-(4-trifluoromethyl-6,7-dihydro-4H-thieno[3,2-c]pyridin-5-yl)-methanone
(6-Chloro-pyrazolo[1,5-a]pyridin-2-yl)-(4-trifluoromethyl-6,7-dihydro-4H-thieno[3,2-c]pyridin-5-yl)-methanone
6-Bromo-pyrazolo[1,5-a]pyridin-2-yl)-(6,6-dimethyl-6,7-dihydro-4H-thieno[3,2-c]pyridin-5-yl)-methanone
6-Chloro-pyrazolo[1,5-a]pyridin-2-yl)-(6,6-dimethyl-6,7-dihydro-4H-thieno[3,2-c]pyridin-5-yl)-methanone
(6-Bromo-pyrazolo[1,5-a]pyridin-2-yl)-(2,4-dimethyl-6,7-dihydro-4H-thieno[3,2-c]pyridin-5-yl)-methanone
(6-Chloro-pyrazolo[1,5-a]pyridin-2-yl)-(2,4-dimethyl-6,7-dihydro-4H-thieno[3,2-c]pyridin-5-yl)-methanone
(6-Bromo-pyrazolo[1,5-a]pyridin-2-yl)-(3,4-dimethyl-6,7-dihydro-4H-thieno[3,2-c]pyridin-5-yl)-methanone
6-Bromo-2-[(2,3,4-trimethyl-6,7-dihydrothieno[3,2-c]pyridin-5(4H)-yl)carbonyl]-pyrazolo[1,5-a]pyridine
6-Chloro-2-[(2,3,4-trimethyl-6,7-dihydrothieno[3,2-c]pyridin-5(4H)-yl)carbonyl]-pyrazolo[1,5-a]pyridine
(6-Bromo-pyrazolo[1,5-a]pyridin-2-yl)-(2-fluoro-4-methyl-6,7-dihydro-4H-thieno[3,2-c]pyridin-5-yl)-methanone
(6-Chloro-pyrazolo[1,5-a]pyridin-2-yl)-(2-fluoro-4-methyl-6,7-dihydro-4H-thieno[3,2-c]pyridin-5-yl)-methanone
(6-Bromo-pyrazolo[1,5-a]pyridin-2-yl)-(3-fluoro-4-methyl-6,7-dihydro-4H-thieno[3,2-c]pyridin-5-yl)-methanone
(6-Chloro-pyrazolo[1,5-a]pyridin-2-yl)-(3-fluoro-4-methyl-6,7-dihydro-4H-thieno[3,2-c]pyridin-5-yl)-methanone
(6-Bromo-pyrazolo[1,5-a]pyridin-2-yl)-(2,3-difluoro-4-methyl-6,7-dihydro-4H-thieno[3,2-c]pyridin-5-yl)-methanone
(6-Chloro-pyrazolo[1,5-a]pyridin-2-yl)-(2,3-difluoro-4-methyl-6,7-dihydro-4H-thieno[3,2-c]pyridin-5-yl)-methanone
(6-Bromo-pyrazolo[1,5-a]pyridin-2-yl)-(4-methyl-2-trifluoromethyl-6,7-dihydro-4H-thieno[3,2-c]pyridin-5-yl)-methanone
(6-Chloro-pyrazolo[1,5-a]pyridin-2-yl)-(4-methyl-2-trifluoromethyl-6,7-dihydro-4H-thieno[3,2-c]pyridin-5-yl)-methanone
6-Bromo-2-[(4-methyl-6,7-dihydrofuro[3,2-c]pyridin-5(4H)-yl)carbonyl]pyrazolo[1,5-a]pyridine
6-Chloro-2-[(4-methyl-6,7-dihydrofuro[3,2-c]pyridin-5(4H)-yl)carbonyl]pyrazolo[1,5-a]pyridine
(6-Chloro-pyrazolo[1,5-a]pyridin-2-yl)-(6,7-dihydro-4H-furo[3,2-c]pyridin-5-yl)-methanone
(6-Bromo-pyrazolo[1,5-a]pyridin-2-yl)-(6,7-dihydro-4H-furo[3,2-c]pyridin-5-yl)-methanone
(6-Bromo-pyrazolo[1,5-a]pyridin-2-yl)-(6-methyl-6,7-dihydro-4H-furo[3,2-c]pyridin-5-yl)-methanone
(6-Chloro-pyrazolo[1,5-a]pyridin-2-yl)-(6-methyl-6,7-dihydro-4H-furo[3,2-c]pyridin-5-yl)-methanone
(6-Bromo-pyrazolo[1,5-a]pyridin-2-yl)-(1,4,6,7-tetrahydro-pyrrolo[3,2-c]pyridin-5-yl)-methanone
(6-Chloro-pyrazolo[1,5-a]pyridin-2-yl)-(1,4,6,7-tetrahydro-pyrrolo[3,2-c]pyridin-5-yl)-methanone
(6-Chloro-pyrazolo[1,5-a]pyridin-2-yl)-(4-methyl-1,4,6,7-tetrahydro-pyrrolo[3,2-c]pyridin-5-yl)-methanone
(6-Bromo-pyrazolo[1,5-a]pyridin-2-yl)-(4-methyl-1,4,6,7-tetrahydro-pyrrolo[3,2-c]pyridin-5-yl)-methanone
(6-Bromo-pyrazolo[1,5-a]pyridin-2-yl)-(1-methyl-1,3,4,5-tetrahydro-2-pyrindin-2-yl)-methanone
(6-Chloro-pyrazolo[1,5-a]pyridin-2-yl)-(1-methyl-1,3,4,5-tetrahydro-2-pyrindin-2-yl)-methanone
5-[(6-Bromopyrazolo[1,5-a]pyridin-2-yl)carbonyl]-3-phenyl-4,5,6,7-tetrahydro-isoxazolo[4,5-c]pyridine
(6-Bromo-pyrazolo[1,5-a]pyridin-2-yl)-(4-methyl-6,7-dihydro-4H-thieno[3,2-c]pyridin-5-yl)-methanone
(6-Chloro-pyrazolo[1,5-a]pyridin-2-yl)-(2,4-dimethyl-6,7-dihydro-4H-furo[3,2-c]pyridin-5-yl)-methanone
(6-Bromo-pyrazolo[1,5-a]pyridin-2-yl)-(2,4-dimethyl-6,7-dihydro-4H-furo[3,2-c]pyridin-5-yl)-methanone
(6-Chloro-pyrazolo[1,5-a]pyridin-2-yl)-(3,4-dimethyl-6,7-dihydro-4H-thieno[3,2-c]pyridin-5-yl)-methanone
(6-Bromo-pyrazolo[1,5-a]pyridin-2-yl)-(1,7,7-trimethyl-1,3,4,7-tetrahydro-2-pyrindin-2-yl)-methanone
(6-Chloro-pyrazolo[1,5-a]pyridin-2-yl)-(1,7,7-trimethyl-1,3,4,7-tetrahydro-2-pyrindin-2-yl)-methanone
(6-Bromo-pyrazolo[1,5-a]pyridin-2-yl)-(1,6,6-trimethyl-1,3,4,6-tetrahydro-2-pyrindin-2-yl)-methanone
(6-Chloro-pyrazolo[1,5-a]pyridin-2-yl)-(1,6,6-trimethyl-1,3,4,6-tetrahydro-2-pyrindin-2-yl)-methanone
(6-Bromo-pyrazolo[1,5-a]pyridin-2-yl)-(1,5,5-trimethyl-1,3,4,5-tetrahydro-2-pyrindin-2-yl)-methanone
(6-Chloro-pyrazolo[1,5-a]pyridin-2-yl)-(1,5,5-trimethyl-1,3,4,5-tetrahydro-2-pyrindin-2-yl)-methanone
(6-Bromo-pyrazolo[1,5-a]pyridin-2-yl)-[4-methyl-1-(1H-tetrazol-5-yl)-1,4,6,7-tetrahydro-pyrrolo[3,2-c]pyridin-5-yl]-methanone
(6-Chloro-pyrazolo[1,5-a]pyridin-2-yl)-[4-methyl-1-(1H-tetrazol-5-yl)-1,4,6,7-tetrahydro-pyrrolo[3,2-c]pyridin-5-yl]-methanone
(6-Bromo-pyrazolo[1,5-a]pyridin-2-yl)-[7-methyl-1-(1H-tetrazol-5-yl)-1,4,5,7-tetrahydro-pyrrolo[2,3-c]pyridin-6-yl]-methanone
(6-Chloro-pyrazolo[1,5-a]pyridin-2-yl)-[7-methyl-1-(1H-tetrazol-5-yl)-1,4,5,7-tetrahydro-pyrrolo[2,3-c]pyridin-6-yl]-methanone
(6-Bromo-pyrazolo[1,5-a]pyridin-2-yl)-[4-methyl-1-(1H-tetrazol-5-yl)-2,4,6,7-tetrahydro-pyrrolo[3,4-c]pyridin-5-yl]-methanone
(6-Chloro-pyrazolo[1,5-a]pyridin-2-yl)-[4-methyl-1-(1H-tetrazol-5-yl)-2,4,6,7-tetrahydro-pyrrolo[3,4-c]pyridin-5-yl]-methanone
(2-Bromo-7-methyl-4,7-dihydro-5H-furo[2,3-c]pyridin-6-yl)-(6-bromo-pyrazolo[1,5-a]pyridin-2-yl)-methanone
(2-Chloro-7-methyl-4,7-dihydro-5H-furo[2,3-c]pyridin-6-yl)-(6-bromo-pyrazolo[1,5-a]pyridin-2-yl)-methanone
2-Bromo-7-methyl-4,7-dihydro-5H-furo[2,3-c]pyridin-6-yl)-(6-chloro-pyrazolo[1,5-a]pyridin-2-yl)-methanone
2-Chloro-7-methyl-4,7-dihydro-5H-furo[2,3-c]pyridin-6-yl)-(6-chloro-pyrazolo[1,5-a]pyridin-2-yl)-methanone
(2-Bromo-7-methyl-4,7-dihydro-5H-thieno[2,3-c]pyridin-6-yl)-(6-bromo-pyrazolo[1,5-a]pyridin-2-yl)-methanone
(2-Chloro-7-methyl-4,7-dihydro-5H-thieno[2,3-c]pyridin-6-yl)-(6-bromo-pyrazolo[1,5-a]pyridin-2-yl)-methanone
(2-Bromo-7-methyl-4,7-dihydro-5H-thieno[2,3-c]pyridin-6-yl)-(6-chloro-pyrazolo[1,5-a]pyridin-2-yl)-methanone
(2-Chloro-7-methyl-4,7-dihydro-5H-thieno[2,3-c]pyridin-6-yl)-(6-chloro-pyrazolo[1,5-a]pyridin-2-yl)-methanone
(3-Bromo-7-methyl-4,7-dihydro-5H-furo[2,3-c]pyridin-6-yl)-(6-bromo-pyrazolo[1,5-a]pyridin-2-yl)-methanone
(3-Chloro-7-methyl-4,7-dihydro-5H-furo[2,3-c]pyridin-6-yl)-(6-bromo-pyrazolo[1,5-a]pyridin-2-yl)-methanone
(3-Bromo-7-methyl-4,7-dihydro-5H-furo[2,3-c]pyridin-6-yl)-(6-chloro-pyrazolo[1,5-a]pyridin-2-yl)-methanone
(3-Chloro-7-methyl-4,7-dihydro-5H-furo[2,3-c]pyridin-6-yl)-(6-chloro-pyrazolo[1,5-a]pyridin-2-yl)-methanone
(3-Bromo-7-methyl-4,7-dihydro-5H-thieno[2,3-c]pyridin-6-yl)-(6-bromo-pyrazolo[1,5-a]pyridin-2-yl)-methanone
(3-Chloro-7-methyl-4,7-dihydro-5H-thieno[2,3-c]pyridin-6-yl)-(6-bromo-pyrazolo[1,5-a]pyridin-2-yl)-methanone
(3-Bromo-7-methyl-4,7-dihydro-5H-thieno[2,3-c]pyridin-6-yl)-(6-chloro-pyrazolo[1,5-a]pyridin-2-yl)-methanone
(3-Chloro-7-methyl-4,7-dihydro-5H-thieno[2,3-c]pyridin-6-yl)-(6-chloro-pyrazolo[1,5-a]pyridin-2-yl)-methanone
1-[5-(6-Bromo-pyrazolo[1,5-a]pyridine-2-carbonyl)-4-methyl-4,5,6,7-tetrahydro-pyrrolo[3,2-c]pyridin-1-yl]-ethanone
1-[5-(6-Chloro-pyrazolo[1,5-a]pyridine-2-carbonyl)-4-methyl-4,5,6,7-tetrahydro-pyrrolo[3,2-c]pyridin-1-yl]-ethanone
1-[6-(6-Bromo-pyrazolo[1,5-a]pyridine-2-carbonyl)-7-methyl-4,5,6,7-tetrahydro-pyrrolo[2,3-c]pyridin-1-yl]-ethanone
1-[6-(6-Chloro-pyrazolo[1,5-a]pyridine-2-carbonyl)-7-methyl-4,5,6,7-tetrahydro-pyrrolo[2,3-c]pyridin-1-yl]-ethanone
(6-Bromo-pyrazolo[1,5-a]pyridin-2-yl)-(4-methyl-6,7-dihydro-4H-oxazolo[4,5-c]pyridin-5-yl)-methanone
6-Bromo-pyrazolo[1,5-a]pyridine-2-carboxylic acid (1-phenyl-ethyl)-amide
6-Chloro-pyrazolo[1,5-a]pyridine-2-carboxylic acid (1-phenyl-ethyl)-amide
(6-Bromo-pyrazolo[1,5-a]pyridin-2-yl)-(2,4-dimethyl-6,7-dihydro-4H-oxazolo[4,5-c]pyridin-5-yl)-methanone
(6-Bromo-pyrazolo[1,5-a]pyridin-2-yl)-(4-methyl-2-trifluoromethyl-6,7-dihydro-4H-oxazolo[4,5-c]pyridin-5-yl)-methanone
(6-Bromo-pyrazolo[1,5-a]pyridin-2-yl)-(2-methoxy-4-methyl-6,7-dihydro-4H-oxazolo[4,5-c]pyridin-5-yl)-methanone
(6-Bromo-pyrazolo[1,5-a]pyridin-2-yl)-(6-hydroxy-7-methoxy-1-methyl-3,4-dihydro-1H-isoquinolin-2-yl)-methanone
(6-Chloro-pyrazolo[1,5-a]pyridin-2-yl)-(1-methyl-3,4-dihydro-1H-isoquinolin-2-yl)-methanone
(6-Chloro-pyrazolo[1,5-a]pyridin-2-yl)-(3-methyl,-3,4-dihydro-1H-isoquinolin-2-yl)-methanone
(6-Bromo-pyrazolo[1,5-a]pyridin-2-yl)-(3-methyl-3,4-dihydro-1H-isoquinolin-2-yl)-methanone
(6-Bromo-pyrazolo[1,5-a]pyridin-2-yl)-(3,3-dimethyl-3,4-dihydro-1H-isoquinolin-2-yl)-methanone
(6-Chloro-pyrazolo[1,5-a]pyridin-2-yl)-(3,3-dimethyl-3,4-dihydro-1H-isoquinolin-2-yl)-methanone
(6-Chloro-pyrazolo[1,5-a]pyridin-2-yl)-(3,4-dihydro-1H-isoquinolin-2-yl)-methanone (6-Chloro-pyrazolo[1,5-a]pyridin-2-yl)-(6,7-dimethoxy-1-methyl-3,4-dihydro-1H-isoquinolin-2-yl)-methanone
(6-Chloro-pyrazolo[1,5-a]pyridin-2-yl)-(6,7-dimethoxy-3,4-dihydro-1H-isoquinolin-2-yl)-methanone
(6-Bromo-pyrazolo[1,5-a]pyridin-2-yl)-(6,7-dimethoxy-3,4-dihydro-1H-isoquinolin-2-yl)-methanone
(6-Bromo-pyrazolo[1,5-a]pyridin-2-yl)-(6,7-dimethoxy-1-methyl-3,4-dihydro-1H-isoquinolin-2-yl)-methanone
(6-Bromo-pyrazolo[1,5-a]pyridin-2-yl)-(6-hydroxy-7-methoxy-3,4-dihydro-1H-isoquinolin-2-yl)-methanone
(6-Chloro-pyrazolo[1,5-a]pyridin-2-yl)-(6-hydroxy-7-methoxy-3,4-dihydro-1H-isoquinolin-2-yl)-methanone
(6-Chloro-pyrazolo[1,5-a]pyridin-2-yl)-(6-hydroxy-7-methoxy-1-methyl-3,4-dihydro-1H-isoquinolin-2-yl)-methanone
(6-Bromo-pyrazolo[1,5-a]pyridin-2-yl)-(6,7-dimethoxy-3-methyl-3,4-dihydro-1H-isoquinolin-2-yl)-methanone
(6-Chloro-pyrazolo[1,5-a]pyridin-2-yl)-(6,7-dimethoxy-3-methyl-3,4-dihydro-1H-isoquinolin-2-yl)-methanone
(6-Bromo-pyrazolo[1,5-a]pyridin-2-yl)-(6,7-dimethoxy-3,3-dimethyl-3,4-dihydro-1H-isoquinolin-2-yl)-methanone
(6-Chloro-pyrazolo[1,5-a]pyridin-2-yl)-(6,7-dimethoxy-3,3-dimethyl-3,4-dihydro-1H-isoquinolin-2-yl)-methanone
(6-Bromo-pyrazolo[1,5-a]pyridin-2-yl)-(8-methyl-5,8-dihydro-6H-[1,7]naphthyridin-7-yl)-methanone
(6-Chloro-pyrazolo[1,5-a]pyridin-2-yl)-(8-methyl-5,8-dihydro-6H-[1,7]naphthyridin-7-yl)-methanone
(6-Bromo-pyrazolo[1,5-a]pyridin-2-yl)-(1-methyl-3,4-dihydro-1H-[2,7]naphthyridin-2-yl)-methanone
(6-Chloro-pyrazolo[1,5-a]pyridin-2-yl)-(1-methyl-3,4-dihydro-1H-[2,7]naphthyridin-2-yl)-methanone
(6-Bromo-pyrazolo[1,5-a]pyridin-2-yl)-(1-methyl-3,4-dihydro-1H-[2,6]naphthyridin-2-yl)-methanone
(6-Chloro-pyrazolo[1,5-a]pyridin-2-yl)-(1-methyl-3,4-dihydro-1H-[2,6]naphthyridin-2-yl)-methanone
(6-Bromo-pyrazolo[1,5-a]pyridin-2-yl)-(5-methyl-7,8-dihydro-5H-[1,6]naphthyridin-6-yl)-methanone
(6-Chloro-pyrazolo[1,5-a]pyridin-2-yl)-(5-methyl-7,8-dihydro-5H-[1,6]naphthyridin-6-yl)-methanone
(6-Bromo-pyrazolo[1,5-a]pyridin-2-yl)-(1-methyl-3,4-dihydro-1H-isoquinolin-2-yl)-methanone
(6-Bromo-pyrazolo[1,5-a]pyridin-2-yl)-(3,4-dihydro-1H-isoquinolin-2-yl)-methanone
(6-Bromo-pyrazolo[1,5-a]pyridin-2-yl)-(7-fluoro-1-methyl-3,4-dihydro-1H-isoquinolin-2-yl)-methanone
(6-Bromo-pyrazolo[1,5-a]pyridin-2-yl)-(8-fluoro-1-methyl-3,4-dihydro-1H-isoquinolin-2-yl)-methanone
(6-Bromo-pyrazolo[1,5-a]pyridin-2-yl)-(6-fluoro-1-methyl-3,4-dihydro-1H-isoquinolin-2-yl)-methanone
(6-Bromo-pyrazolo[1,5-a]pyridin-2-yl)-(5-fluoro-1-methyl-3,4-dihydro-1H-isoquinolin-2-yl)-methanone
(6-Chloro-pyrazolo[1,5-a]pyridin-2-yl)-(7-fluoro-1-methyl-3,4-dihydro-1H-isoquinolin-2-yl)-methanone

(6-Chloro-pyrazolo[1,5-a]pyridin-2-yl)-(8-fluoro-1-methyl-3,4-dihydro-1H-isoquinolin-2-yl)-methanone
(6-Chloro-pyrazolo[1,5-a]pyridin-2-yl)-(6-fluoro-1-methyl-3,4-dihydro-1H-isoquinolin-2-yl)-methanone
(6-Chloro-pyrazolo[1,5-a]pyridin-2-yl)-(5-fluoro-1-methyl-3,4-dihydro-1H-isoquinolin-2-yl)-methanone
(6-Bromo-pyrazolo[1,5-a]pyridin-2-yl)-(8-fluoro-1-ethyl-3,4-dihydro-1H-isoquinolin-2-yl)-methanone
(6-Bromo-pyrazolo[1,5-a]pyridin-2-yl)-(7-fluoro-1-ethyl-3,4-dihydro-1H-isoquinolin-2-yl)-methanone
(6-Bromo-pyrazolo[1,5-a]pyridin-2-yl)-(6-fluoro-1-ethyl-3,4-dihydro-1H-isoquinolin-2-yl)-methanone
(6-Bromo-pyrazolo[1,5-a]pyridin-2-yl)-(5-fluoro-1-ethyl-3,4-dihydro-1H-isoquinolin-2-yl)-methanone
(6-Chloro-pyrazolo[1,5-a]pyridin-2-yl)-(8-fluoro-1-ethyl-3,4-dihydro-1H-isoquinolin-2-yl)-methanone
(6-Chloro-pyrazolo[1,5-a]pyridin-2-yl)-(7-fluoro-1-ethyl-3,4-dihydro-1H-isoquinolin-2-yl)-methanone
(6-Chloro-pyrazolo[1,5-a]pyridin-2-yl)-(6-fluoro-1-ethyl-3,4-dihydro-1H-isoquinolin-2-yl)-methanone
(6-Chloro-pyrazolo[1,5-a]pyridin-2-yl)-(5-fluoro-1-ethyl-3,4-dihydro-1H-isoquinolin-2-yl)-methanone
(6-Bromo-pyrazolo[1,5-a]pyridin-2-yl)-(8-fluoro-1-trifluoromethyl-3,4-dihydro-1H-isoquinolin-2-yl)-methanone
(6-Bromo-pyrazolo[1,5-a]pyridin-2-yl)-(7-fluoro-1-trifluoromethyl-3,4-dihydro-1H-isoquinolin-2-yl)-methanone
(6-Bromo-pyrazolo[1,5-a]pyridin-2-yl)-(6-fluoro-1-trifluoromethyl-3,4-dihydro-1H-isoquinolin-2-yl)-methanone
(6-Bromo-pyrazolo[1,5-a]pyridin-2-yl)-(5-fluoro-1-trifluoromethyl-3,4-dihydro-1H-isoquinolin-2-yl)-methanone
(6-Chloro-pyrazolo[[1,5-a]pyridin-2-yl)-(8-fluoro-1-trifluoromethyl-3,4-dihydro-1H-isoquinolin-2-yl)-methanone
(6-Chloro-pyrazolo[1,5-a]pyridin-2-yl)-(7-fluoro-1-trifluoromethyl-3,4-dihydro-1H-isoquinolin-2-yl)-methanone
(6-Chloro-pyrazolo[1,5-a]pyridin-2-yl)-(6-fluoro-1-trifluoromethyl-3,4-dihydro-1H-isoquinolin-2-yl)-methanone
(6-Chloro-pyrazolo[1,5-a]pyridin-2-yl)-(5-fluoro-1-trifluoromethyl-3,4-dihydro-1H-isoquinolin-2-yl)-methanone
(6-Bromo-pyrazolo[1,5-a]pyridin-2yl)-(3-ethyl-1-methyl-3,4-dihydro-1H-isoquinolin-2-yl)methanone
(6-Bromo-pyrazolo[1,5-a]pyridin-2yl)-((R)-3-methyl-3,4-dihydro-1H-isoquinolin-2-yl)-methanone
(6-Bromo-pyrazolo[1,5-a]pyridin-2y1)-((S)-3-methyl-3,4-dihydro-1H-isoquinolin-2-yl)-methanone
(6-Chloro-pyrazolo[1,5-a]pyridin-2-yl)-(1-(S)-methyl-3,4-dihydro-1H-isoquinolin-2-yl)-methanone
(7-Bromo-3,4-dihydro-11H-isoquinolin-2-yl)-(6-chloro-pyrazolo[1,5-a]pyridin-2-yl)-methanone
(7-Bromo-3,4-dihydro-1H-isoquinolin-2-yl)-(6-bromo-pyrazolo[1,5-a]pyridin-2-yl)-methanone
(6-Chloro-pyrazolo[1,5-a]pyridin-2-yl)-(7-fluoro-1-methyl-3,4-dihydro-1H-isoquinolin-2-yl)-methanone
(6-Chloro-pyrazolo[1,5-a]pyridin-2-yl)-(1,4-dimethyl-3,4-dihydro-1H-isoquinolin-2-yl)-methanone
(6-Bromo-pyrazolo[1,5-a]pyridin-2-yl)-(1,4-dimethyl-3,4-dihydro-1H-isoquinolin-2-yl)-methanone
(6-Bromo-pyrazolo[1,5-a]pyridin-2-yl)-((1R,4S)-1,4-dimethyl-3,4-dihydro-1H-isoquinolin-2-yl)-methanone
(6-Bromo-pyrazolo[1,5-a]pyridin-2-yl)-((1S,4S)-1,4-dimethyl-3,4-dihydro-1H-isoquinolin-2-yl)-methanone
(6-Bromo-pyrazolo[1,5-a]pyridin-2-yl)-((1S,4R)-1,4-dimethyl-3,4-dihydro-1H-isoquinolin-2-yl)-methanone
(6-Bromo-pyrazolo [1,5-a]pyridin-2-yl)-((1R,4R)-1,4-dimethyl-3,4-dihydro-1H-isoquinolin-2-yl)-methanone
(6-Bromo-pyrazolo[1,5-a]pyridin-2-yl)-(6-methoxy-1-methyl-3,4-dihydro-1H-isoquinolin-2-yl)-methanone
(6-Chloro-pyrazolo[1,5-a]pyridin-2-yl)-(6-methoxy-1-methyl-3,4-dihydro-1H-isoquinolin-2-yl)-methanone
(6-Bromopyrazolo[1,5-a]pyridin-2yl)-(1-ethyl-3,4-dihydro-1H-isoquinolin-2-yl)methanone
(6-Bromopyrazolo[1,5-a]pyridin-2yl)-(1-isopropyl-3,4-dihydro-1H-isoquinolin-2-yl)methanone
(6-Chloro-pyrazolo[1,5-a]pyridin-2-yl)-(1-isopropyl-3,4-dihydro-1H-isoquinolin-2-yl)-methanone
(6-Chloro-pyrazolo[1,5-a]pyridin-2-yl)-(1-ethyl-3,4-dihydro-1H-isoquinolin-2-yl)-methanone
(6-Bromo-pyrazolo[1,5-a]pyridin-2-yl)-(1-trifluoromethyl-3,4-dihydro-1H-isoquinolin-2-yl)-methanone
(6-Choro-pyrazolo[1,5-a]pyridin-2-yl)-(1-trifluoromethyl-3,4-dihydro-1H-isoquinolin-2-yl)-methanone
(7-Bromo-1-methyl-3,4-dihydro-1H-isoquinolin-2-yl)-(6-bromo-pyrazolo[1,5-a]pyridin-2-yl)-methanone
(7-Bromo-1-methyl-3,4-dihydro-1H-isoquinolin-2-yl)-(6-chloro-pyrazolo[1,5-a]pyridin-2-yl)-methanone
(6-Bromo-pyrazolo[1,5-a]pyridin-2-yl)-(1-cyclohexyl-3,4-dihydro-1H-isoquinolin-2-yl)-methanone
(6-Chloro-pyrazolo[1,5-a]pyridin-2-yl)-(1-cyclohexyl-3,4-dihydro-1H-isoquinolin-2-yl)-methanone
(6-Bromo-pyrazolo[1,5-a]pyridin-2-yl)-(1-propyl-3,4-dihydro-1H-isoquinolin-2yl)-methanone
(6-Bromo-pyrazolo[1,5-a]pyridin-2-yl)-((S)1-methyl-3,4-dihydro-1H-isoquinolin-2yl)-methanone
(6-Bromo-pyrazolo[1,5-a]pyridin-2-yl)-((R)1-methyl-3,4-dihydro-1H-isoquinolin-2yl)-methanone
(5-Bromo-1-methyl-3,4-dihydro-1H-isoquinolin-2-yl)-(6-chloro-pyrazolo[1,5-a]pyridin-2yl)-methanone
(6-Bromo-pyrazolo[1,5-a]pyridin-2-yl)-5-methoxy-1-methyl-3,4-dihydro-1H-isoquinolin-2-yl)-methanone
(5-Bromo-1-methyl-3,4-dihydro-1H-isoquinolin-2-yl)-(6-bromo-pyrazolo[1,5-a]pyridin-2-yl)-methanone
(6-Bromo-pyrazolo[1,5-a]pyridin-2-yl)-(7-pyridin-4-yl-3,4-dihydro-1H-isoquinolin-2-yl)-methanone
(6-Bromo-pyrazolo[1,5-a]pyridin-2-yl)-(7-methoxy-1-methyl-3,4-dihydro-1H-isoquinolin-2-yl)-methanone
(6-Chloro-pyrazolo[1,5-a]pyridin-2-yl)-(7-methoxy-1-methyl-3,4-dihydro-1H-isoquinolin-2-yl)-methanone
(6-Bromo-1-methyl-3,4-dihydro-1H-isoquinolin-2yl)-(6-bromo-pyrazolo[1,5-a]pyridin-2-yl)-methanone
(6-Bromo-pyrazolo[1,5-a]pyridin-2-yl)-(5,6-dimethoxy-1-methyl-3,4-dihydro-1H-isoquinolin-2-yl)-methanone
(6-Bromo-pyrazolo[1,5-a]pyridin-2-yl)-(5,8-difluoro-1-methyl-3,4-dihydro-1H-isoquinolin-2-yl)-methanone
(6-Chloro-pyrazolo[1,5-a]pyridin-2-yl)-(5,8-difluoro-1-methyl-3,4-dihydro-1H-isoquinolin-2-yl)-methanone
(6-Bromopyrazolo[1,5-a]pyridin-2-yl)-(1-methyl-5-trifluoromethyl-3,4-dihydro-1H-isoquinolin-2-yl)-methanone
(6-Chloro-pyrazolo[1,5-a]pyridin-2-yl)-(1-methyl-5-trifluoromethyl-3,4-dihydro-1H-isoquinolin-2-yl)-methanone
(6-Chloro-pyrazolo[1,5-a]pyridin-2-yl)-(5-methyl-7,8-dihydro-5H-[1,6]naphthyridin-6-yl)-methanone
(3-Bromo-7,8-dihydro-5H-[1,6]naphthyridin-6-yl)-(6-chloro-pyrazolo[1,5-a]pyridin-2-yl)-methanone
(6-Bromo-pyrazolo[1,5-a]pyridin-2-yl)-(1-methyl-6-pyridin-3-yl-3,4-dihydro-1H-isoquinolin-2-yl)-methanone
(3-Bromo-7,8-dihydro-5H-[1,6]naphthyridin-6-yl)-(6-bromo-pyrazolo[1,5-a]pyridin-2-yl)-methanone
(6-Bromo-pyrazolo[1,5-a]pyridin-2-yl)-(1-methyl-7-pyridin-3-yl-3,4-dihydro-1H-isoquinolin-2-yl)-methanone
2-(6-Bromo-pyrazolo[1,5-a]pyridine-2-carbonyl-)1,2,3,4-tetrahydro-isoquinoline-7-carbonitrile

(6-Bromo-pyrazolo[1,5-a]pyridin-2-yl)-(l-methyl-7-pyridin-4-yl-3,4-dihydro-1H-isoquinolin-2-yl)-methanone
(6-Bromo-pyrazolo[1,5-a]pyridin-2-yl)-(5-nitro-3,4-dihydro-1H-isoquinolin-2-yl)-methanone
(6-Chloro-pyrazolo[1,5-a]pyridin-2-yl)-(5-nitro-3,4-dihydro-1H-isoquinolin-2-yl)-methanone
(6-Bromo-pyrazolo[1,5-a]pyridin-2-yl)-(1-methyl-7-pyrimidin-5-yl-3,4-dihydro-1H-isoquinolin-2-yl)-methanone
(6-Bromo-pyrazolo[1,5-a]pyridin-2-yl)-(7-morpholin-4-yl-3,4-dihydro-1H-isoquinolin-2-yl)-methanone
(6-Chloro-pyrazolo[1,5-a]pyridin-2-yl)-(7-morpholin-4-yl-3,4-dihydro-1H-isoquinolin-2-yl)-methanone
(6-Bromo-pyrazolo[1,5-a]pyridin-2-yl)-(8-morpholin-4-yl-3,4-dihydro-1H-isoquinolin-2-yl)-methanone
(6-Chloro-pyrazolo[1,5-a]pyridin-2-yl)-(8-morpholin-4-yl-3,4-dihydro-1H-isoquinolin-2-yl)-methanone
(6-Bromo-pyrazolo[1,5-a]pyridin-2-yl)-(6-morpholin-4-yl-3,4-dihydro-1H-isoquinolin-2-yl)-methanone
(6-Chloro-pyrazolo[1,5-a]pyridin-2-yl)₋(6-morpholin-4-yl-3,4-dihydro-1H-isoquinolin-2-yl)-methanone
(6-Bromo-pyrazolo[1,5-a]pyridin-2-yl)-(5-morpholin-4-yl-3,4-dihydro-1H-isoquinolin-2-yl)-methanone
(6-Chloro-pyrazolo[1,5-a]pyridin-2-yl)-(5-morpholin-4-yl-3,4-dihydro-1H-isoquinolin-2-yl)-methanone
(6-Bromo-pyrazolo[1,5-a]pyridin-2-yl)-(1-methyl-6-pyrimidin-5-yl-3,4-dihydro-1H-isoquinolin-2-yl)-methanone
(6-Bromo-pyrazolo[1,5-a]pyridin-2-yl)-(1-methyl-5-pyrimidin-5-yl-3,4-dihydro-1H-isoquinolin-2-yl)-methanone
(6-Bromo-pyrazolo[1,5-a]pyridin-2-yl)-(1-methyl-5-pyridin-3-yl-3,4-dihydro-1H-isoquinolin-2-yl)-methanone
(3-Bromo-7,8-dihydro-5H-[1,6]naphthyridin-6-yl)-(6-bromo-pyrazolo[1,5-a]pyridin-2-yl)-methanone
(6-Chloro-pyrazolo[1,5-a]pyridin-2-yl)-(1-methyl-7-trifluoromethyl-3,4-dihydro-1H-isoquinolin-2-yl)-methanone
(6-Bromo-pyrazolo[1,5-a]pyridin-2-yl)-(1-methyl-7-trifluoromethyl-3,4-dihydro-1H-isoquinolin-2-yl)-methanone
(6-Chloro-pyrazolo[1,5-a]pyridin-2-yl)-(1,5-dimethyl-3,4-dihydro-1H-isoquinolin-2-yl)-methanone
(6-Chloro-pyrazolo[1,5-a]pyridin-2-yl)-(1,7-dimethyl-3,4-dihydro-1H-isoquinolin-2-yl)-methanone
(6-Bromo-pyrazolo[1,5-a]pyridin-2-yl)-(1,7-dimethyl-3,4-dihydro-1H-isoquinolin-2-yl)-methanone
(6-Bromo-pyrazolo[1,5-a]pyridin-2-yl)-(1,5-dimethyl-3,4-dihydro-1H-isoquinolin-2-yl)-methanone
(7-Chloro-1-methyl-3,4-dihydro-1H-isoquinolin-2-yl)-(6-chloro-pyrazolo[1,5-a]pyridin-2-yl)-methanone
(6-Bromo-pyrazolo[1,5-a]pyridin-2-yl)-(7-chloro-1-methyl-3,4-dihydro-1H-isoquinolin-2-yl)-methanone
(5-Chloro-1-methyl-3,4-dihydro-1H-isoquinolin-2-yl)-(6-chloro-pyrazolo[1,5-a]pyridin-2-yl)-methanone
N-[2-(6-Chloro-pyrazolo[ 1,5-a]pyridine-2-carbonyl)-1,2,3,4-tetrahydro-isoquinolin-5-yl]-acetamide
N-[2-(6-Bromo-pyrazolo[1,5-a]pyridine-2-carbonyl)-1,2,3,4-tetrahydro-isoquinolin-5-yl]-acetamide
N-[2-(6-Chloro-pyrazolo[1,5-a]pyridine-2-carbonyl)-1-methyl-1,2,3,4-tetrahydro-isoquinolin-5-yl]-acetamide
N-[2-(6-Bromo-pyrazolo[1,5-a]pyridine-2-carbonyl)-1-methyl-1,2,3,4-tetrahydro-isoquinolin-5-yl]-acetamide
N-[2-(6-Chloro-pyrazolo[1,5-a]pyridine-2-carbonyl)-1-methyl-1,2,3,4-tetrahydro-isoquinolin-5-yl]-acetamide
2-(6-Bromo-pyrazolo[1,5-a]pyridine-2-carbonyl)-1-methyl-1,2,3,4-tetrahydro-isoquinoline-5-carboxylic acid dimethylamide
2-(6-Chloro-pyrazolo[1,5-a]pyridine-2-carbonyl)-1-methyl-1,2,3,4-tetrahydro-isoquinoline-5-carboxylic acid dimethylamide

(6-Bromo-pyrazolo[1,5-a]pyridin-2-yl)-(8-methyl-2,3,5,8-tetrahydro-6H-1,4-dioxa-7-azaphenanthren-7-yl)-methanone
2-(6-Bromo-pyrazolo[1,5-a]pyridine-2-carbonyl)-1,2,3,4-tetrahydro-isoquinoline-5-carbonitrile
2-(6-Bromo-pyrazolo[1,5-a]pyridine-2-carbonyl)-1,2,3,4-tetrahydro-isoquinoline-7-carbonitrile
(6-Bromo-pyrazolo[1,5-a]pyridin-2-yl)-(6-pyridin-4-yl-3,4-dihydro-1H-isoquinolin-2-yl)-methanone
(6-Chloro-pyrazolo[1,5-a]pyridin-2-yl)-(6-pyridin-4-yl-3,4-dihydro-1H-isoquinolin-2-yl)-methanone
(6-Chloro-pyrazolo[1,5-a]pyridin-2-yl)-(1-methyl-6-pyridin-4-yl-3,4-dihydro-1H-isoquinolin-2-yl)-methanone
(6-Bromo-pyrazolo[1,5-a]pyridin-2-yl)-(1-methyl-6-pyridin-4-yl-3,4-dihydro-1H-isoquinolin-2-yl)-methanone
(6-Bromo-pyrazolo[1,5-a]pyridin-2-yl)-(6-methyl-8,9-dihydro-6H-[1,3]dioxolo[4,5-f]isoquinolin-7-yl)-methanone
(6-Bromo-pyrazolo[1,5-a]pyridin-2-yl)-(1-methyl-5-pyridin-4-yl-3,4-dihydro-1H-isoquinolin-2-yl)-methanone
(6-Chloro-pyrazolo[1,5-a]pyridin-2-yl)-(1-methyl-5-pyridin-4-yl-3,4-dihydro-1H-isoquinolin-2-yl)-methanone
(6-Bromo-3,4-dihydro-1H-isoquinolin-2-yl)-(6-bromo-pyrazolo[1,5-a]pyridin-2-yl)-methanone
(6-Bromo-pyrazolo[1,5-a]pyridin-2-yl)-(6-pyridin-2-yl-3,4-dihydro-1H-isoquinolin-2-yl)-methanone
(6-Bromo-pyrazolo[1,5-a]pyridin-2-yl)-(1-methyl-6-pyridin-2-yl-3,4-dihydro-1H-isoquinolin-2-yl)-methanone
(6-Chloro-pyrazolo[1,5-a]pyridin-2-yl)-(1-methyl-6-pyridin-2-yl-3,4-dihydro-1H-isoquinolin-2-yl)-methanone
(6-Chloro-pyrazolo[1,5-a]pyridin-2-yl)-(1-methyl-7-pyridin-2-yl-3,4-dihydro-1H-isoquinolin-2-yl)-methanone
(5-Bromo-3,4-dihydro-1H-isoquinolin-2-yl)-(6-bromo-pyrazolo[1,5-a]pyridin-2-yl)-methanone

(5-Bromo-3,4-dihydro-1H-isoquinolin-2-yl)-(6-chloro-pyrazolo[1,5-a]pyridin-2-yl)-methanone
(6-Bromo-pyrazolo[1,5-a]pyridin-2-yl)-(5-pyridin-2-yl-3,4-dihydro-1H-isoquinolin-2-yl)-methanone
(6-Bromo-pyrazolo[1,5-a]pyridin-2-yl)-[5-(2,4-dimethoxy-pyrimidin-5-yl)-1-methyl-3,4-dihydro-1H-isoquinolin-2-yl]-methanone
(6-Chloro-pyrazolo[1,5-a]pyridin-2-yl)-[5-(2,4-dimethoxy-pyrimidin-5-yl)-1-methyl-3,4-dihydro-1H-isoquinolin-2-yl]-methanone
(6-Bromo-pyrazolo[1,5-a]pyridin-2-yl)-[5-(2,6-dimethoxy-pyridin-3-yl)-1-methyl-3,4-dihydro-1H-isoquinolin-2-yl]-methanone
(6-Chloro-pyrazolo[1,5-a]pyridin-2-yl)-[5-(2,6-dimethoxy-pyridin-3-yl)-1-methyl-3,4-dihydro-1H-isoquinolin-2-yl]-methanone
(6-Bromo-pyrazolo[1,5-a]pyridin-2-yl)-[5-(6-fluoro-pyridin-3-yl)-1-methyl-3,4-dihydro-1H-isoquinolin-2-yl]-methanone
(6-Chloro-pyrazolo[1,5-a]pyridin-2-yl)-[5-(6-fluoro-pyridin-3-yl)-1-methyl-3,4-dihydro-1H-isoquinolin-2-yl]-methanone
(6-Bromo-pyrazolo[1,5-a]pyridin-2-yl)-[5-(2-methoxy-pyridin-3-yl)-1-methyl-3,4-dihydro-1H-isoquinolin-2-yl]-methanone
(6-Chloro-pyrazolo[1,5-a]pyridin-2-yl)-[5-(2-methoxy-pyridin-3-yl)-1-methyl-3,4-dihydro-1H-isoquinolin-2-yl]-methanone
(6-Chloro-pyrazolo[1,5-a]pyridin-2-yl)-[5-(6-methoxy-pyridin-3-yl)-1-methyl-3,4-dihydro-1H-isoquinolin-2-yl]-methanone
(6-Bromo-pyrazolo[1,5-a]pyridin-2-yl)-[5-(6-methoxy-pyridin-3-yl)-1-methyl-3,4-dihydro-1H-isoquinolin-2-yl]-methanone
(6-Bromo-pyrazolo[1,5-a]pyridin-2-yl)-[6-(2-methoxy-pyridin-3-yl)-1-methyl-3,4-dihydro-1H-isoquinolin-2-yl]-methanone
(6-Chloro-pyrazolo[1,5-a]pyridin-2-yl)-[6-(2-methoxy-pyridin-3-yl)-1-methyl-3,4-dihydro-1H-isoquinolin-2-yl]-methanone
(6-Bromo-pyrazolo[1,5-a]pyridin-2-yl)-[6-(2-fluoro-pyridin-3-yl)-1-methyl-3,4-dihydro-1H-isoquinolin-2-yl]-methanone
(6-Chloro-pyrazolo[1,5-a]pyridin-2-yl)-[6-(2-fluoro-pyridin-3-yl)-1-methyl-3,4-dihydro-1H-isoquinolin-2-yl]-methanone

(6-Bromo-pyrazolo[1,5-a]pyridin-2-yl)-[6-(6-fluoro-pyridin-3-yl)-1-methyl-3,4-dihydro-1H-isoquinolin-2-yl]-methanone
(6-Chloro-pyrazolo[1,5-a]pyridin-2-yl)-[6-(6-fluoro-pyridin-3-yl)-1-methyl-3,4-dihydro-1H-isoquinolin-2-yl]-methanone
(6-Bromo-pyrazolo[1,5-a]pyridin-2-yl)-[6-(2,6-dimethoxy-pyridin-3-yl)-1-methyl-3,4-dihydro-1H-isoquinolin-2-yl]-methanone
(6-Chloro-pyrazolo[1,5-a]pyridin-2-yl)-[6-(2,6-dimethoxy-pyridin-3-yl)-1-methyl-3,4-dihydro-1H-isoquinolin-2-yl]-methanone
(6-Bromo-pyrazolo[1,5-a]pyridin-2-yl)-[6-(2,4-dimethoxy-pyrimidin-5-yl)-1-methyl-3,4-dihydro-1H-isoquinolin-2-yl]-methanone
(6-Chloro-pyrazolo[1,5-a]pyridin-2-yl)-[6-(2,4-dimethoxy-pyrimidin-5-yl)-1-methyl-3,4-dihydro-1H-isoquinolin-2-yl]-methanone
(6-Chloro-pyrazolo[1,5-a]pyridin-2-yl)-[7-(6-methoxy-pyridin-3-yl)-1-methyl-3,4-dihydro-1H-isoquinolin-2-yl]-methanone
(6-Chloro-pyrazolo[1,5-a]pyridin-2-yl)-[7-(2-methoxy-pyridin-3-yl)-1-methyl-3,4-dihydro-1H-isoquinolin-2-yl]-methanone
(6-Bromo-pyrazolo[1,5-a]pyridin-2-yl)-[7-(2,4-dimethoxy-pyrimidin-5-yl)-1-methyl-3,4-dihydro-1H-isoquinolin-2-yl]-methanone
(6-Chloro-pyrazolo[1,5-a]pyridin-2-yl)-[7-(2,4-dimethoxy-pyrimidin-5-yl)-1-methyl-3,4-dihydro-1H-isoquinolin-2-yl]-methanone
(6-Bromo-pyrazolo[1,5-a]pyridin-2-yl)-[7-(2-fluoro-pyridin-3-yl)-1-methyl-3,4-dihydro-1H-isoquinolin-2-yl]-methanone
(6-Chloro-pyrazolo[1,5-a]pyridin-2-yl)-[7-(2-fluoro-pyridin-3-yl)-1-methyl-3,4-dihydro-1H-isoquinolin-2-yl]-methanone
(6-Bromo-pyrazolo[1,5-a]pyridin-2-yl)-[5-(2-fluoro-pyridin-3-yl)-1-methyl-3,4-dihydro-1H-isoquinolin-2-yl]-methanone
(6-Chloro-pyrazolo[1,5-a]pyridin-2-yl)-[5-(2-fluoro-pyridin-3-yl)-1-methyl-3,4-dihydro-1H-isoquinolin-2-yl]-methanone
(6-Bromo-pyrazolo[1,5-a]pyridin-2-yl)-(8-furan-2-yl-1-methyl-3,4-dihydro-1H-isoquinolin-2-yl)-methanone
(6-Chloro-pyrazolo[1,5-a]pyridin-2-yl)-(8-furan-2-yl-1-methyl-3,4-dihydro-1H-isoquinolin-2-yl)-methanone

(6-Bromo-pyrazolo[1,5-a]pyridin-2-yl)-(7-furan-2-yl-1-methyl-3,4-dihydro-1H-isoquinolin-2-yl)-methanone
(6-Chloro-pyrazolo[1,5-a]pyridin-2-yl)-(7-furan-2-yl-1-methyl-3,4-dihydro-1H-isoquinolin-2-yl)-methanone
(6-Bromo-pyrazolo[1,5-a]pyridin-2-yl)-(6-furan-2-yl-1-methyl-3,4-dihydro-1H-isoquinolin-2-yl)-methanone
(6-Chloro-pyrazolo[1,5-a]pyridin-2-yl)-(6-furan-2-yl-1-methyl-3,4-dihydro-1H-isoquinolin-2-yl)-methanone
(6-Bromo-pyrazolo[1,5-a]pyridin-2-yl)-(5-furan-2-yl-1-methyl-3,4-dihydro-1H-isoquinolin-2-yl)-methanone
(6-Chloro-pyrazolo[1,5-a]pyridin-2-yl)-(5-furan-2-yl-1-methyl-3,4-dihydro-1H-isoquinolin-2-yl)-methanone
(6-Bromo-pyrazolo[1,5-a]pyridin-2-yl)-[1-methyl-8-(5-methyl-furan-2-yl)-3,4-dihydro-1H-isoquinolin-2-yl]-methanone
(6-Chloro-pyrazolo[1,5-a]pyridin-2-yl)-[1-methyl-8-(5-methyl-furan-2-yl)-3,4-dihydro-1H-isoquinolin-2-yl]-methanone
(6-Bromo-pyrazolo[1,5-a]pyridin-2-yl)-[1-methyl-7-(5-methyl-furan-2-yl)-3,4-dihydro-1H-isoquinolin-2-yl]-methanone
(6-Chloro-pyrazolo[1,5-a]pyridin-2-yl)-[1-methyl-7-(5-methyl-furan-2-yl)-3,4-dihydro-1H-isoquinolin-2-yl]-methanone
(6-Bromo-pyrazolo[1,5-a]pyridin-2-yl)-[1-methyl-6-(5-methyl-furan-2-yl)-3,4-dihydro-1H-isoquinolin-2-yl]-methanone
(6-Chloro-pyrazolo[1,5-a]pyridin-2-yl)-[I-methyl-6-(5-methyl-fiaran-2-yl)-3,4-dihydro-1H-isoquinolin-2-yl]-methanone
(6-Bromo-pyrazolo[1,5-a]pyridin-2-yl)-[1-methyl-5-(5-methyl-furan-2-yl)-3,4-dihydro-1H-isoquinolin-2-yl]-methanone
(6-Chloro-pyrazolo[1,5-a]pyridin-2-yl)-[1-methyl-5-(5-methyl-furan-2-yl)-3,4-dihydro-1H-isoquinolin-2-yl]-methanone
(6-Bromo-pyrazolo[1,5-a]pyridin-2-yl)-(8-furan-3-yl-1-methyl-3,4-dihydro-1H-isoquinolin-2-yl)-methanone
(6-Chloro-pyrazolo[1,5-a]pyridin-2-yl)-(8-furan-3-yl-1-methyl-3,4-dihydro-1H-isoquinolin-2-yl)-methanone

(6-Bromo-pyrazolo[1,5-a]pyridin-2-yl)-(7-furan-3-yl-1-methyl-3,4-dihydro-1H-isoquinolin-2-yl)-methanone
(6-Chloro-pyrazolo[1,5-a]pyridin-2-yl)-(7-furan-3-yl-1-methyl-3,4-dihydro-1H-isoquinolin-2-yl)-methanone
(6-Bromo-pyrazolo[1,5-a]pyridin-2-yl)-(6-furan-3-yl-1-methyl-3,4-dihydro-1H-isoquinolin-2-yl)-methanone
(6-Chloro-pyrazolo[1,5-a]pyridin-2-yl)-(6-furan-3-yl-1-methyl-3,4-dihydro-1H-isoquinolin-2-yl)-methanone
(6-Bromo-pyrazolo[1,5-a]pyridin-2-yl)-(5-furan-3-yl-1-methyl-3,4-dihydro-1H-isoquinolin-2-yl)-methanone
(6-Chloro-pyrazolo[1,5-a]pyridin-2-yl)-(5-furan-3-yl-1-methyl-3,4-dihydro-1H-isoquinolin-2-yl)-methanone
(6-Bromo-pyrazolo[1,5-a]pyridin-2-yl)-[1-methyl-5-(1H-pyrazol-4-yl)-3,4-dihydro-1H-isoquinolin-2-yl]-methanone
(6-Chloro-pyrazolo[1,5-a]pyridin-2-yl)-[1-methyl-5-(1H-pyrazol-4-yl)-3,4-dihydro-1H-isoquinolin-2-yl]-methanone
(6-Bromo-pyrazolo[1,5-a]pyridin-2-yl)-[7-(3,5-dimethyl-isoxazol-4-yl)-1-methyl-3,4-dihydro-1H-isoquinolin-2-yl]-methanone
(6-Chloro-pyrazolo[1,5-a]pyridin-2-yl)-[7-(3,5-dimethyl-isoxazol-4-yl)-1-methyl-3,4-dihydro-1H-isoquinolin-2-yl]-methanone
(6-Bromo-pyrazolo[1,5-a]pyridin-2-yl)-[6-(3,5-dimethyl-isoxazol-4-yl)-1-methyl-3,4-dihydro-1H-isoquinolin-2-yl]-methanone
(6-Chloro-pyrazolo[1,5-a]pyridin-2-yl)-[6-(3,5-dimethyl-isoxazol-4-yl)-1-methyl-3,4-dihydro-1H-isoquinolin-2-yl]-methanone
(6-Bromo-pyrazolo[1,5-a]pyridin-2-yl)-[5-(3,5-dimethyl-isoxazol-4-yl)-1-methyl-3,4-dihydro-1H-isoquinolin-2-yl]-methanone
(6-Chloro-pyrazolo[1,5-a]pyridin-2-yl)-[5-(3,5-dimethyl-isoxazol-4-yl)-1-methyl-3,4-dihydro-1H-isoquinolin-2-yl]-methanone
(6-Bromo-pyrazolo[1,5-a]pyridm-2-yl)-[1-methyl-8-(1H-pyrazol-4-yl)-3,4-dihydro-1H-isoquinolin-2-yl]-methanone
(6-Chloro-pyrazolo[1,5-a]pyridin-2-yl)-[1-methyl-8-(1H-pyrazol-4-yl)-3,4-dihydro-1H-isoquinolin-2-yl]-methanone
(6-Bromo-pyrazolo[1,5-a]pyridin-2-yl)-[1-methyl-7-(1H-pyrazol-4-yl)-3,4-dihydro-1H-isoquinolin-2-yl]-methanone
(6-Chloro-pyrazolo[1,5-a]pyridin-2-yl)-[1-methyl-7-(1H-pyrazol-4-yl)-3,4-dihydro-1H-isoquinolin-2-yl]-methanone
(6-Bromo-pyrazolo[1,5-a]pyridin-2-yl)-[1-methyl-6-(1H-pyrazol-4-yl)-3,4-dihydro-1H-isoquinolin-2-yl]-methanone
(6-Chloro-pyrazolo[1,5-a]pyridin-2-yl)-[1-methyl-6-(1H-pyrazol-4-yl)-3,4-dihydro-1H-isoquinolin-2-yl]-methanone
6-Bromo-pyrazolo[1,5-a]pyridine-2-carboxylic acid phenethyl-amide
6-Chloro-pyrazolo[1,5-a]pyridine-2-carboxylic acid phenethyl-amide
(6-Bromo-pyrazolo[1,5-a]pyridin-2-yl)-[1-methyl-8-(1-methyl-1H-pyrazol-4-yl)-3,4-dihydro-1H-isoquinolin-2-yl]-methanone
(6-Chloro-pyrazolo[1,5-a]pyridin-2-yl)-[1-methyl-8-(1-methyl-1H-pyrazol-4-yl)-3,4-dihydro-1H-isoquinolin-2-yl]-methanone
(6-Bromo-pyrazolo[1,5-a]pyridin-2-yl)-[1-methyl-7-(1-methyl-1H-pyrazol-4-yl)-3,4-dihydro-1H-isoquinolin-2-yl]-methanone
(6-Chloro-pyrazolo[1,5-a]pyridin-2-yl)-[1-methyl-7-(1-methyl-1H-pyrazol-4-yl)-3,4-dihydro-1H-isoquinolin-2-yl]-methanone
(6-Bromo-pyrazolo[1,5-a]pyridin-2-yl)-[1-methyl-6-(1-methyl-1H-pyrazol-4-yl)-3,4-dihydro-1H-isoquinolin-2-yl]-methanone
(6-Chloro-pyrazolo[1,5-a]pyridin-2-yl)-[1-methyl-6-(1-methyl-1H-pyrazol-4-yl)-3,4-dihydro-1H-isoquinolin-2-yl]-methanone
(6-Bromo-pyrazolo[1,5-a]pyridin-2-yl)-[1-methyl-5-(1-methyl-1H-pyrazol-4-yl)-3,4-dihydro-1H-isoquinolin-2-yl]-methanone
(6-Chloro-pyrazolo[1,5-a]pyridin-2-yl)-[1-methyl-5-(1-methyl-1H-pyrazol-4-yl)-3,4-dihydro-1H-isoquinolin-2-yl]-methanone
(6-Bromo-pyrazolo[1,5-a]pyridin-2-yl)-[1-methyl-8-(3-methyl-1H-pyrazol-4-yl)-3,4-dihydro-1H-isoquinolin-2-yl]-methanone
(6-Chloro-pyrazolo[1,5-a]pyridin-2-yl)-[1-methyl-8-(3-methyl-1H-pyrazol-4-yl)-3,4-dihydro-1H-isoquinolin-2-yl]-methanone
(6-Bromo-pyrazolo[1,5-a]pyridin-2-yl)-[1-methyl-7-(3-methyl-1H-pyrazol-4-yl)-3,4-dihydro-1H-isoquinolin-2-yl]-methanone
(6-Chloro-pyrazolo[1,5-a]pyridin-2-yl)-[1-methyl-7-(3-methyl-1H-pyrazol-4-yl)-3,4-dihydro-1H-isoquinolin-2-yl]-methanone
(6-Bromo-pyrazolo[1,5-a]pyridin-2-yl)-[1-methyl-6-(3-methyl-1H-pyrazol-4-yl)-3,4-dihydro-1H-isoquinolin-2-yl]-methanone
(6-Chloro-pyrazolo[1,5-a]pyridin-2-yl)-[1-methyl-6-(3-methyl-1H-pyrazol-4-yl)-3,4-dihydro-1H-isoquinolin-2-yl]-methanone
(6-Bromo-pyrazolo[1,5-a]pyridin-2-yl)-[1-methyl-5-(3-methyl-1H-pyrazol-4-yl)-3,4-dihydro-1H-isoquinolin-2-yl]-methanone
(6-Chloro-pyrazolo[1,5-a]pyridin-2-yl)-[1-methyl-5-(3-methyl-1H-pyrazol-4-yl)-3,4-dihydro-1H-isoquinolin-2-yl]-methanone
(6-Bromo-pyrazolo[1,5-a]pyridin-2-yl)-[1-methyl-8-(2H-tetrazol-5-yl)-3,4-dihydro-1H-isoquinolin-2-yl]-methanone
(6-Chloro-pyrazolo[1,5-a]pyridin-2-yl)-[1-methyl-8-(2H-tetrazol-5-yl)-3,4-dihydro-1H-isoquinolin-2-yl]-methanone
(6-Bromo-pyrazolo[1,5-a]pyridin-2-yl)-[1-methyl-7-(2H-tetrazol-5-yl)-3,4-dihydro-1H-isoquinolin-2-yl]-methanone
(6-Chloro-pyrazolo[1,5-a]pyridin-2-yl)-[1-methyl-7-(2H-tetrazol-5-yl)-3,4-dihydro-1H-isoquinolin-2-yl]-methanone
(6-Bromo-pyrazolo[1,5-a]pyridin-2-yl)-[1-methyl-6-(2H-tetrazol-5-yl)-3,4-dihydro-1H-isoquinolin-2-yl]-methanone
(6-Chloro-pyrazolo[1,5-a]pyridin-2-yl)-[1-methyl-6-(2H-tetrazol-5-yl)-3,4-dihydro-1H-isoquinolin-2-yl]-methanone
(6-Bromo-pyrazolo[1,5-a]pyridin-2-yl)-(1-methyl-5-nitro-3,4-dihydro-1H-2,7-naphthyridin-2-yl)-methanone
(6-Chloro-pyrazolo[1,5-a]pyridin-2-yl)-(1-methyl-5-nitro-3,4-dihydro-1H-2,7-naphthyridin-2-yl)-methanone
6-Bromo-pyrazolo[1,5-a]pyridin-2-yl)-(1-methyl-3,4-dihydro-1H-[2,7]naphthyridin-2-yl)-methanone
6-Chloro-pyrazolo[1,5-a]pyridin-2-yl)-(1-methyl-3,4-dihydro-1H-[2,7]naphthyridin-2-yl)-methanone
(6-Bromo-pyrazolo[1,5-a]pyridin-2-yl)-(1-methyl-3,4-dihydro-1H-[2,6]naphthyridin-2-yl)-methanone

(6-Chloro-pyrazolo[1,5-a]pyridin-2-yl)-(1-methyl-3,4-dihydro-1H-[2,6]naphthyridin-2-yl)-methanone
(6-Bromo-pyrazolo[1,5-a]pyridin-2-yl)-(5-methyl-7,8-dihydro-5H-pyrido[4,3-d]pyrimidin-6-yl)-methanone
(6-Chloro-pyrazolo[1,5-a]pyridin-2-yl)-(5-methyl-7,8-dihydro-5H-pyrido[4,3-d]pyrimidin-6-yl)-methanone
(6-Bromo-pyrazolo[1,5-a]pyridin-2-yl)-(8-methyl-5,8-dihydro-6H-pyrido[3,4-d]pyrimidin-7-yl)-methanone
(6-Chloro-pyrazolo[1,5-a]pyridin-2-yl)-(8-methyl-5,8-dihydro-6H-pyrido[3,4-d]pyrimidin-7-yl)-methanone
(6-Bromo-pyrazolo[1,5-a]pyridin-2-yl)-(1-methyl-5-nitro-3,4-dihydro-1H-isoquinolin-2-yl)-methanone
(6-Chloro-pyrazolo[1,5-a]pyridin-2-yl)-(1-methyl-5-nitro-3,4-dihydro-1H-isoquinolin-2-yl)-methanone
(6-Bromo-pyrazolo[1,5-a]pyridin-2-yl)-(1-methyl-6-nitro-3,4-dihydro-1H-isoquinolin-2-yl)-methanone
(6-Chloro-pyrazolo[1,5-a]pyridin-2-yl)-(1-methyl-6-nitro-3,4-dihydro-1H-isoquinolin-2-yl)-methanone
(6-Bromo-pyrazolo[1,5-a]pyridin-2-yl)-(1-methyl-7-nitro-3,4-dihydro-1H-isoquinolin-2-yl)-methanone
(6-Chloro-pyrazolo[1,5-a]pyridin-2-yl)-(1-methyl-7-nitro-3,4-dihydro-1H-isoquinolin-2-yl)-methanone
6-Bromo-pyrazolo[1,5-a]pyridine-2-carboxylic acid cycloheptylamide
6-Chloro-pyrazolo[1,5-a]pyridine-2-carboxylic acid cycloheptylamide
6-Bromo-pyrazolo[1,5-a]pyridine-2-carboxylic acid cyclohexylamide
6-Chloro-pyrazolo[1,5-a]pyridine-2-carboxylic acid cyclohexylamide
2-(6-Bromo-pyrazolo[1,5-a]pyridine-2-carbonyl)-1-methyl-1,2,3,4-tetrahydro-isoquinoline-5-carbonitrile
2-(6-Chloro-pyrazolo[1,5-a]pyridine-2-carbonyl)-1-methyl-1,2,3,4-tetrahydro-isoquinoline-5-carbonitrile
2-(6-Bromo-pyrazolo[1,5-a]pyridine-2-carbonyl)-1-methyl-1,2,3,4-tetrahydro-isoquinoline-5-carboxylic acid methyl ester

2-(6-Chloro-pyrazolo[1,5-a]pyridine-2-carbonyl)-1-methyl-1,2,3,4-tetrahydro-isoquinoline-5-carboxylic acid methyl ester
(6-Bromo-pyrazolo[1,5-a]pyridin-2-yl)-(2,6-dimethyl-8,9-dihydro-6H-3-oxa-1,7-diazacyclopenta[a]naphthalen-7-yl)-methanone
(6-Chloro-pyrazolo[1,5-a]pyridin-2-yl)-(2,6-dimethyl-8,9-dihydro-6H-3-oxa-1,7-diazacyclopenta[a]naphthalen-7-yl)-methanone
(6-Bromo-pyrazolo[1,5-a]pyridin-2-yl)-(4-methyl-1,4-dihydro-2H-[3,7]phenanthrolin-3-yl)-methanone
(6-Chloro-pyrazolo[1,5-a]pyridin-2-yl)-(4-methyl-1,4-dihydro-2H-[3,7]phenanthrolin-3-yl)-methanone
(6-Bromo-pyrazolo[1,5-a]pyridin-2-yl)-(2,6-dimethyl-8,9-dihydro-6H-thiazolo[4,5-f]isoquinolin-7-yl)-methanone
(6-Chloro-pyrazolo[1,5-a]pyridin-2-yl)-(2,6-dimethyl-8,9-dihydro-6H-thiazolo[4,5-f]isoquinolin-7-yl)-methanone
(6-Bromo-pyrazolo[1,5-a]pyridin-2-yl)-(5-methanesulfonyl-1-methyl-3,4-dihydro-1H-isoquinolin-2-yl)-methanone
(6-Chloro-pyrazolo[1,5-alpyridin-2-yl)-(5-methanesulfonyl-1-methyl-3,4-dihydro-1H-isoquinolin-2-yl)-methanone
(6-Bromo-pyrazolo[1,5-a]pyridin-2-yl)-(6-methanesulfonyl-1-methyl-3,4-dihydro-1H-isoquinolin-2-yl)-methanone
(6-Chloro-pyrazolo[1,5-a]pyridin-2-yl)-(6-methanesulfonyl-1-methyl-3,4-dihydro-1H-isoquinolin-2-yl)-methanone
(6-Bromo-pyrazolo[1,5-a]pyridin-2-yl)-(7-methanesulfonyl-1-methyl-3,4-dihydro-1H-isoquinolin-2-yl)-methanone
(6-Chloro-pyrazolo[1,5-a]pyridin-2-yl)-(7-methanesulfonyl-1-methyl-3,4-dihydro-1H-isoquinolin-2-yl)-methanone
2-(6-Bromo-pyrazolo[1,5-a]pyridine-2-carbonyl)-1-methyl-1,2,3,4-tetrahydro-isoquinoline-6-carboxylic acid methyl ester
2-(6-Chloro-pyrazolo[1,5-a]pyridine-2-carbonyl)-1-methyl-1,2,3,4-tetrahydro-isoquinoline-6-carboxylic acid methyl ester
(6-Bromo-pyrazolo[1,5-a]pyridin-2-yl)-(4,7-dimethyl-1,4,7,8,9,10-hexahydro-2H-[3,7]phenanthrolin-3-yl)-methanone

(6-Chloro-pyrazolo[1,5-a]pyridin-2-yl)-(4,7-dimethyl-1,4,7,8,9,10-hexahydro-2H-[3,7]phenanthrolin-3-yl)-methanone
1-[2-(6-Bromo-pyrazolo[1,5-a]pyridine-2-carbonyl)-1-methyl-1,2,3,4-tetrahydro-isoquinolin-5-yl]-ethanone
1-[2-(6-Chloro-pyrazolo[1,5-a]pyridine-2-carbonyl)-1-methyl-1,2,3,4-tetrahydro-isoquinolin-5-yl]-ethanone
6-Bromo-pyrazolo[1,5-a]pyridine-2-carboxylic acid adamantyl-1 amide
6-Chloro-pyrazolo[1,5-a]pyridine-2-carboxylic acid adamantyl-1 amide
2-(6-Bromo-pyrazolo[1,5-a]pyridine-2-carbonyl)-1-methyl-1,2,3,4-tetrahydro-isoquinoline-7-carboxylic acid methyl ester
2-(6-Chloro-pyrazolo[1,5-a]pyridine-2-carbonyl)-1-methyl-1,2,3,4-tetrahydro-isoquinoline-7-carboxylic acid methyl ester
(6-Bromo-pyrazolo[1,5-a]pyridin-2-yl)-[1-methyl-5-(morpholine-4-carbonyl)-3,4-dihydro-1H-isoquinolin-2-yl]-methanone
((6-Chloro-pyrazolo[1,5-a]pyridin-2-yl)-[1-methyl-5-(morpholine-4-carbonyl)-3,4-dihydro-1H-isoquinolin-2-yl]-methanone
(6-Bromo-pyrazolo[1,5-a]pyridin-2-yl)-[1-methyl-7-(morpholine-4-carbonyl)-3,4-dihydro-1H-isoquinolin-2-yl]-methanone
(6-Chloro-pyrazolo[1,5-a]pyridin-2-yl)-[1-methyl-7-(morpholine-4-carbonyl)-3,4-dihydro-1H-isoquinolin-2-yl]-methanone
(6-Bromo-pyrazolo[1,5-a]pyridin-2-yl)-[1-methyl-6-(morpholine-4-carbonyl)-3,4-dihydro-1H-isoquinolin-2-yl]-methanone
(6-Chloro-pyrazolo[1,5-a]pyridin-2-yl)-[1-methyl-6-(morpholine-4-carbonyl)-3,4-dihydro-1H-isoquinolin-2-yl]-methanone
(6-Bromo-pyrazolo[1,5-a]pyridin-2-yl)-(6-methyl-8,9-dihydro-6H-furo[3,2-f]isoquinolin-7-yl)-methanone
(6-Chloro-pyrazolo[1,5-a]pyridin-2-yl)-(6-methyl-8,9-dihydro-6H-furo[3 ,2-f]isoquinolin-7-yl)-methanone
(6-Bromo-pyrazolo[1,5-a]pyridin-2-yl)-(3,6-dimethyl-3,6,8,9-tetrahydro-pyrrolo[3,2-f]isoquinolin-7-yl)-methanone
(6-Chloro-pyrazolo[1,5-a]pyridin-2-yl)-(3,6-dimethyl-3,6,8,9-tetrahydro-pyrrolo[3,2-f]isoquinolin-7-yl)-methanone

(6-Bromo-pyrazolo[1,5-a]pyridin-2-yl)-(8-methyl-5,8-dihydro-6H-furo[3,2-g]isoquinolin-7-yl)-methanone
(6-Chloro-pyrazolo[1,5-a]pyridin-2-yl)-(8-methyl-5,8-dihydro-6H-furo[3,2-g]isoquinolin-7-yl)-methanone
7-(6-Bromo-pyrazolo[1,5-a]pyridine-2-carbonyl)-2,6-dimethyl-1,2,6,7,8,9-hexahydro-pyrrolo[3,4-f]isoquinolin-3-one
7-(6-Chloro-pyrazolo[1,5-a]pyridine-2-carbonyl)-2,6-dimethyl-1,2,6,7,8,9-hexahydro-pyrrolo[3,4-f]isoquinolin-3-one
7-(6-Bromo-pyrazolo[1,5-a]pyridine-2-carbonyl)-2,6-dimethyl-2,3,6,7,8,9-hexahydro-pyrrolo[3,4-f]isoquinolin-1-one
7-(6-Chloro-pyrazolo[1,5-a]pyridine-2-carbonyl)-2,6-dimethyl-2,3,6,7,8,9-hexahydro-pyrrolo[3,4-f]isoquinolin-1-one
(6-Bromo-pyrazolo[1,5-a]pyridin-2-yl)-(6-diethylamino-1-methyl-3,4-dihydro-1H-isoquinolin-2-yl)-methanone
(6-Chloro-pyrazolo[1,5-a]pyridin-2-yl)-(6-diethylamino-1-methyl-3,4-dihydro-1H-isoquinolin-2-yl)-methanone
(6-Bromo-pyrazolo[1,5-a]pyridin-2-yl)-(5-diethylamino-1-methyl-3,4-dihydro-1H-isoquinolin-2-yl)-methanone
(6-Chloro-pyrazolo[1,5-a]pyridin-2-yl)-(5-diethylamino-1-methyl-3,4-dihydro-1H-isoquinolin-2-yl)-methanone
(6-Bromo-pyrazolo[1,5-a]pyridin-2-yl)-(8-diethylamino-1-methyl-3,4-dihydro-1H-isoquinolin-2-yl)-methanone
(6-Chloro-pyrazolo[1,5-a]pyridin-2-yl)-(8-diethylamino-1-methyl-3,4-dihydro-1H-isoquinolin-2-yl)-methanone
(6-Bromo-pyrazolo[1,5-a]pyridin-2-yl)-(7-diethylamino-1-methyl-3,4-dihydro-1H-isoquinolin-2-yl)-methanone
(6-Chloro-pyrazolo[1,5-a]pyridin-2-yl)-(7-diethylamino-1-methyl-3,4-dihydro-1H-isoquinolin-2-yl)-methanone
(6-Bromo-pyrazolo[1,5-a]pyridin-2-yl)-(6,7-difluoro-1-methyl-3,4-dihydro-1H-isoquinolin-2-yl)-methanone
(6-Chloro-pyrazolo[1,5-a]pyridin-2-yl)-(6,7-difluoro-1-methyl-3,4-dihydro-1H-isoquinolin-2-yl)-methanone

(6-Bromo-pyrazolo[1,5-a]pyridin-2-yl)-(6,7-dichloro-1-methyl-3,4-dihydro-1H-isoquinolin-2-yl)-methanone
(6-Chloro-pyrazolo[1,5-a]pyridin-2-yl)-(6,7-dichloro-1-methyl-3,4-dihydro-1H-isoquinolin-2-yl)-methanone
(6-Bromo-pyrazolo[1,5-a]pyridin-2-yl)-(6,7-dibromo-1-methyl-3,4-dihydro-1H-isoquinolin-2-yl)-methanone
(6-Chloro-pyrazolo[1,5-a]pyridin-2-yl)-(6,7-dibromo-1-methyl-3,4-dihydro-1H-isoquinolin-2-yl)-methanone
(6-Bromo-pyrazolo[1,5-a]pyridin-2-yl)-(1-methyl-7-morpholin-4-yl-3,4-dihydro-1H-isoquinolin-2-yl)-methanone
(6-Chloro-pyrazolo[1,5-a]pyridin-2-yl)-(1-methyl-7-morpholin-4-yl-3,4-dihydro-1H-isoquinolin-2-yl)-methanone
(6-Bromo-pyrazolo[1,5-a]pyridin-2-yl)-(1-methyl-8-morpholin-4-yl-3,4-dihydro-1H-isoquinolin-2-yl)-methanone
(6-Chloro-pyrazolo[1,5-a]pyridin-2-yl)-(1-methyl-8-morpholin-4-yl-3,4-dihydro-1H-isoquinolin-2-yl)-methanone
(6-Bromo-pyrazolo[1,5-a]pyridin-2-yl)-(1-methyl-6-morpholin-4-yl-3,4-dihydro-1H-isoquinolin-2-yl)-methanone
(6-Chloro-pyrazolo[1,5-a]pyridm-2-yl)-(1-methyl-6-morpholin-4-yl-3,4-dihydro-1H-isoquinolin-2-yl)-methanone
(6-Bromo-pyrazolo[1,5-a]pyridin-2-yl)-(1-methyl-5-morpholin-4-yl-3,4-dihydro-1H-isoquinolin-2-yl)-methanone
(6-Chloro-pyrazolo[1,5-a]pyridin-2-yl)-(1-methyl-5-morpholin-4-yl-3,4-dihydro-1H-isoquinolin-2-yl)-methanone
(6-Bromo-pyrazolo[1,5-a]pyridin-2-yl)-(1-methyl-7-piperidin-1-yl-3,4-dihydro-1H-isoquinolin-2-yl)-methanone
(6-Chloro-pyrazolo[1,5-a]pyridin-2-yl)-(1-methyl-7-piperidin-1-yl-3,4-dihydro-1H-isoquinolin-2-yl)-methanone
(6-Bromo-pyrazolo[1,5-a]pyridin-2-yl)-(1-methyl-7-pyrrolidin-1-yl-3,4-dihydro-1H-isoquinolin-2-yl)-methanone
(6-Chloro-pyrazolo[1,5-a]pyridin-2-yl)-(1-methyl-7-pyrrolidin-1-yl-3,4-dihydro-1H-isoquinolin-2-yl)-methanone

(6-Chloro-pyrazolo[1,5-a]pyridin-2-yl)-(1-methyl-3,4-dihydro-1H-pyrrolo[1,2-a]pyrazin-2-yl)-methanone
(6-Chloro-pyrazolo[1,5-a]pyridin-2-yl)-(3,4-dihydro-1H-pyrrolo[1,2-a]pyrazin-2-yl)-methanone
(6-Bromo-pyrazolo[1,5-a]pyridin-2-yl)-(1-methyl-3,4-dihydro-1H-pyrrolo[1,2-a]pyrazin-2-yl)-methanone
(6-Bromo-pyrazolo[1,5-a]pyridin-2-yl)-(3,4-dihydro-1H-pyrrolo[1,2-a]pyrazin-2-yl)-methanone
(6-Chloro-pyrazolo[1,5-a]pyridin-2-yl)-(1,6-dimethyl-3,4-dihydro-1H-pyrrolo[1,2-a]pyrazin-2-yl)-methanone
(6-Chloro-pyrazolo[1,5-a]pyridin-2-yl)-(6-methyl-3,4-dihydro-1H-pyrrolo[1,2-a]pyrazin-2-yl)-methanone
(6-Bromo-pyrazolo[1,5-a]pyridin-2-yl)-(1,6-dimethyl-3,4-dihydro-1H-pyrrolo[1,2-a]pyrazin-2-yl)-methanone
(6-Bromo-pyrazolo[1,5-a]pyridin-2-yl)-(6-methyl-3,4-dihydro-1H-pyrrolo[1,2-a]pyrazin-2-yl)-methanone
(6-Chloro-pyrazolo[1,5-a]pyridin-2-yl)-(4-methyl-6,7-dihydro-4H-[1,2,3]triazolo[1,5-a]pyrazin-5-yl)-methanone
(6-Chloro-pyrazolo[1,5-a]pyridin-2-yl)-(6,7-dihydro-4H-[1,2,3]triazolo[1,5-a]pyrazin-5-yl)-methanone
(6-Bromo-pyrazolo[1,5-a]pyridin-2-yl)-(4-methyl-6,7-dihydro-4H-[1,2,3]triazolo[1,5-a]pyrazin-5-yl)-methanone
(6-Bromo-pyrazolo[1,5-a]pyridin-2-yl)-(6,7-dihydro-4H-[1,2,3]triazolo[1,5-a]pyrazin-5-yl)-methanone
7-(6-Chloro-pyrazolo[1,5-a]pyridine-2-carbonyl)-8-methyl-5,6,7,8-tetrahydro-imidazo[1,2-a]pyrazine-2-carboxylic acid ethyl ester
7-(6-Chloro-pyrazolo[1,5-a]pyridine-2-carbonyl)-5,6,7,8-tetrahydro-imidazo[1,2-a]pyrazine-2-carboxylic acid ethyl ester
7-(6-Bromo-pyrazolo[1,5-a]pyridine-2-carbonyl)-8-methyl-5,6,7,8-tetrahydro-imidazo[1,2-a]pyrazine-2-carboxylic acid ethyl ester
7-(6-Bromo-pyrazolo[1,5-a]pyridine-2-carbonyl)-5,6,7,8-tetrahydro-imidazo[1,2-a]pyrazine-2-carboxylic acid ethyl ester
7-(6-Chloro-pyrazolo[1,5-a]pyridine-2-carbonyl)-8-methyl-5,6,7,8-tetrahydro-[1,2,4]triazolo[4,3-a]pyrazine-3-carboxylic acid ethyl ester
7-(6-Chloro-pyrazolo[1,5-a]pyridine-2-carbonyl)-5,6,7,8-tetrahydro-[1,2,4]triazolo[4,3-a]pyrazine-3-carboxylic acid ethyl ester
7-(6-Bromo-pyrazolo[1,5-a]pyridine-2-carbonyl)-8-methyl-5,6,7,8-tetrahydro-[1,2,4]triazolo[4,3-a]pyrazine-3-carboxylic acid ethyl ester
7-(6-Bromo-pyrazolo[1,5-a]pyridine-2-carbonyl)-5,6,7,8-tetrahydro-[1,2,4]triazolo[4,3-a]pyrazine-3-carboxylic acid ethyl ester
(3-Bromo-8-methyl-5,6-dihydro-8H-[1,2,4]triazolo[4,3-a]pyrazin-7-yl)-(6-chloro-pyrazolo[1,5-a]pyridin-2-yl)-methanone
(3-Bromo-5,6-dihydro-8H-[1,2,4]triazolo[4,3-a]pyrazin-7-yl)-(6-chloro-pyrazolo[1,5-a]pyridin-2-yl)-methanone
(3-Bromo-8-methyl-5,6-dihydro-8H-[1,2,4]triazolo[4,3-a]pyrazin-7-yl)-(6-bromo-pyrazolo[1,5-a]pyridin-2-yl)-methanone
(3-Bromo-5,6-dihydro-8H-[1,2,4]triazolo[4,3-a]pyrazin-7-yl)-(6-bromo-pyrazolo[1,5-a]pyridin-2-yl)-methanone
(6-Chloro-pyrazolo[1,5-a]pyridin-2-yl)-(8-methyl-5,6-dihydro-8H-[1,2,4]triazolo[4,3-a]pyrazin-7-yl)-methanone
(6-Chloro-pyrazolo[1,5-a]pyridin-2-yl)-(5,6-dihydro-8H-[1,2,4]triazolo[4,3-a]pyrazin-7-yl)-methanone
(6-Bromo-pyrazolo[1,5-a]pyridin-2-yl)-(8-methyl-5,6-dihydro-8H-[1,2,4]triazolo[4,3-a]pyrazin-7-yl)-methanone
(6-Bromo-pyrazolo[1,5-a]pyridin-2-yl)-(5,6-dihydro-8H-[1,2,4]triazolo[4,3-a]pyrazin-7-yl)-methanone
(6-Chloro-1-methyl-3,4-dihydro-1H-pyrrolo[1,2-a]pyrazin-2-yl)-(6-chloro-pyrazolo[1,5-a]pyridin-2-yl)-methanone
(6-Chloro-3,4-dihydro-1H-pyrrolo[1,2-a]pyrazin-2-yl)-(6-chloro-pyrazolo[1,5-a]pyridin-2-yl)-methanone
(6-Chloro-1-methyl-3,4-dihydro-1H-pyrrolo[1,2-a]pyrazin-2-yl)-(6-bromo-pyrazolo[1,5-a]pyridin-2-yl)-methanone
(6-Chloro-3,4-dihydro-1H-pyrrolo[1,2-a]pyrazin-2-yl)-(6-bromo-pyrazolo[1,5-a]pyridin-2-yl)-methanone
(6-Chloro-pyrazolo[1,5-a]pyridin-2-yl)-(6,7-dibromo-1-methyl-3,4-dihydro-1H-pyrrolo[1,2-a]pyrazin-2-yl)-methanone
(6-Chloro-pyrazolo[1,5-a]pyridin-2-yl)-(6,7-dibromo-3,4-dihydro-1H-pyrrolo[1,2-a]pyrazin-2-yl)-methanone
(6-Bromo-pyrazolo[1,5-a]pyridin-2-yl)-(6,7-dibromo-1-methyl-3,4-dihydro-1H-pyrrolo[1,2-a]pyrazin-2-yl)-methanone
(6-Bromo-pyrazolo[1,5-a]pyridin-2-yl)-(6,7-dibromo-3,4-dihydro-1H-pyrrolo[1,2-a]pyrazin-2-yl)-methanone
(6-Bromo-1-methyl-3,4-dihydro-1H-pyrrolo[1,2-a]pyrazin-2-yl)-(6-chloro-pyrazolo[1,5-a]pyridin-2-yl)-methanone
(6-Bromo-3,4-dihydro-1H-pyrrolo[1,2-a]pyrazin-2-yl)-(6-chloro-pyrazolo[1,5-a]pyridin-2-yl)-methanone
(6-Bromo-1-methyl-3,4-dihydro-1H-pyrrolo[1,2-a]pyrazin-2-yl)-(6-bromo-pyrazolo[1,5-a]pyridin-2-yl)-methanone
(6-Bromo-3,4-dihydro-1H-pyrrolo[1,2-a]pyrazin-2-yl)-(6-bromo-pyrazolo[1,5-a]pyridin-2-yl)-methanone
(6-Chloro-pyrazolo[1,5-a]pyridin-2-yl)-(4-methyl-6,7-dihydro-4H-pyrazolo[1,5-a]pyrazin-5-yl)-methanone
(6-Chloro-pyrazolo[1,5-a]pyridin-2-yl)-(6,7-dihydro-4H-pyrazolo[1,5-a]pyrazin-5-yl)-methanone
(6-Bromo-pyrazolo[1,5-a]pyridin-2-yl)-(4-methyl-6,7-dihydro-4H-pyrazolo[1,5-a]pyrazin-5-yl)-methanone
(6-Bromo-pyrazolo[1,5-a]pyridin-2-yl)-(6,7-dihydro-4H-pyrazolo[1,5-a]pyrazin-5-yl)-methanone
(6-Chloro-pyrazolo[1,5-a]pyridin-2-yl)-(8-methyl-5,6-dihydro-8H-imidazo[1,2-a]pyrazin-7-yl)-methanone
(6-Chloro-pyrazolo[1,5-a]pyridin-2-yl)-(5,6-dihydro-8H-imidazo[1,2-a]pyrazin-7-yl)-methanone
(6-Bromo-pyrazolo[1,5-a]pyridin-2-yl)-(8-methyl-5,6-dihydro-8H-imidazo[1,2-a]pyrazin-7-yl)-methanone
(6-Bromo-pyrazolo[1,5-a]pyridin-2-yl)-(5,6-dihydro-8H-imidazo[1,2-a]pyrazin-7-yl)-methanone
(6-Chloro-pyrazolo[1,5-a]pyridin-2-yl)-(2,8-dimethyl-5,6-dihydro-8H-imidazo[1,2-a]pyrazin-7-yl)-methanone
(6-Chloro-pyrazolo[1,5-a]pyridin-2-yl)-(2-methyl-5,6-dihydro-8H-imidazo[1,2-a]pyrazin-7-yl)-methanone
(6-Bromo-pyrazolo[1,5-a]pyridin-2-yl)-(2,8-dimethyl-5,6-dihydro-8H-imidazo[1,2-a]pyrazin-7-yl)-methanone
(6-Bromo-pyrazolo[1,5-a]pyridin-2-yl)-(2-methyl-5,6-dihydro-8H-imidazo[1,2-a]pyrazin-7-yl)-methanone
(6-Chloro-pyrazolo[1,5-a]pyridin-2-yl)-(3,8-dimethyl-5,6-dihydro-8H-imidazo[1,2-a]pyrazin-7-yl)-methanone
(6-Chloro-pyrazolo[1,5-a]pyridm-2-yl)-(3-methyl-5,6-dihydro-8H-imidazo[1,2-a]pyrazin-7-yl)-methanone
(6-Bromo-pyrazolo[1,5-a]pyridin-2-yl)-(3,8-dimethyl-5,6-dihydro-8H-imidazo[1,2-a]pyrazin-7-yl)-methanone
(6-Bromo-pyrazolo[1,5-a]pyridin-2-yl)-(3-methyl-5,6-dihydro-8H-imidazo[1,2-a]pyrazin-7-yl)-methanone
(6-Chloro-pyrazolo[1,5-a]pyridin-2-yl)-(3,8-dimethyl-5,6-dihydro-8H-[1,2,4]triazolo[4,3-a]pyrazin-7-yl)-methanone
(6-Chloro-pyrazolo[1,5-a]pyridin-2-yl)-(3-methyl-5,6-dihydro-8H-[1,2,4]triazolo[4,3-a]pyrazin-7-yl)-methanone
(6-Bromo-pyrazolo[1,5-a]pyridin-2-yl)-(3,8-dimethyl-5,6-dihydro-8H-[1,2,4]triazolo[4,3-a]pyrazin-7-yl)-methanone
(6-Bromo-pyrazolo[1,5-a]pyridin-2-yl)-(3-methyl-5,6-dihydro-8H-[1,2,4]triazolo[4,3-a]pyrazin-7-yl)-methanone
2-(6-Chloro-pyrazolo[1,5-a]pyridine-2-carbonyl)-1-methyl-1,2,3,4-tetrahydro-pyrrolo[1,2-a]pyrazine-6-carbonitrile
2-(6-Chloro-pyrazolo[1,5-a]pyridine-2-carbonyl)-1,2,3,4-tetrahydro-pyrrolo[1,2-a]pyrazine-6-carbonitrile
2-(6-Bromo-pyrazolo[1,5-a]pyridine-2-carbonyl)-1-methyl-1,2,3,4-tetrahydro-pyrrolo[1,2-a]pyrazine-6-carbonitrile
2-(6-Bromo-pyrazolo[1,5-a]pyridine-2-carbonyl)-1,2,3,4-tetrahydro-pyrrolo[1,2-a]pyrazine-6-carbonitrile
2-(6-Chloro-pyrazolo[1,5-a]pyridine-2-carbonyl)-1-methyl-1,2,3,4-tetrahydro-pyrrolo[1,2-a]pyrazine-6-carboxylic acid methylamide
2-(6-Chloro-pyrazolo[1,5-a]pyridine-2-carbonyl)-1,2,3,4-tetrahydro-pyrrolo[1,2-a]pyrazine-6-carboxylic acid methylamide
2-(6-Bromo-pyrazolo[1,5-a]pyridine-2-carbonyl)-1-methyl-1,2,3,4-tetrahydro-pyrrolo[1,2-a]pyrazine-6-carboxylic acid methylamide
2-(6-Bromo-pyrazolo[1,5-a]pyridine-2-carbonyl)-1,2,3,4-tetrahydro-pyrrolo[1,2-a]pyrazine-6-carboxylic acid methylamide
2-(6-Chloro-pyrazolo[1,5-a]pyridine-2-carbonyl)-1-methyl-1,2,3,4-tetrahydro-pyrrolo[1,2-a]pyrazine-6-carboxylic acid cyclopropylamide
2-(6-Chloro-pyrazolo[1,5-a]pyridine-2-carbonyl)-1,2,3,4-tetrahydro-pyrrolo[1,2-a]pyrazine-6-carboxylic acid cyclopropylamide
2-(6-Bromo-pyrazolo[1,5-a]pyridine-2-carbonyl)-1-methyl-1,2,3,4-tetrahydro-pyrrolo[1,2-a]pyrazine-6-carboxylic acid cyclopropylamide
2-(6-Bromo-pyrazolo[1,5-a]pyridine-2-carbonyl)-1,2,3,4-tetrahydro-pyrrolo[ 1,2-a]pyrazine-6-carboxylic acid cyclopropylamide
2-(6-Chloro-pyrazolo[1,5-a]pyridine-2-carbonyl)-1-methyl-1,2,3,4-tetrahydro-pyrrolo[1,2-a]pyrazine-6-carboxylic acid ethylamide
2-(6-Chloro-pyrazolo[1,5-a]pyridine-2-carbonyl)-1,2,3,4-tetrahydro-pyrrolo[1,2-a]pyrazine-6-carboxylic acid ethylamide
2-(6-Bromo-pyrazolo[1,5-a]pyridine-2-carbonyl)-1-methyl-1,2,3,4-tetrahydro-pyrrolo[1,2-a]pyrazine-6-carboxylic acid ethylamide
2-(6-Bromo-pyrazolo[1,5-a]pyridine-2-carbonyl)-1,2,3,4-tetrahydro-pyrrolo[1,2-a]pyrazine-6-carboxylic acid ethylamide
2-(6-Chloro-pyrazolo[1,5-a]pyridine-2-carbonyl)-1-methyl-1,2,3,4-tetrahydro-pyrrolo[1,2-a]pyrazine-6-carboxylic acid isopropylamide
2-(6-Chloro-pyrazolo[1,5-a]pyridine-2-carbonyl)-1,2,3,4-tetrahydro-pyrrolo[1,2-a]pyrazine-6-carboxylic acid isopropylamide
2-(6-Bromo-pyrazolo[1,5-a]pyridine-2-carbonyl)-1-methyl-1,2,3,4-tetrahydro-pyrrolo[1,2-a]pyrazine-6-carboxylic acid isopropylamide
2-(6-Bromo-pyrazolo[1,5-a]pyridine-2-carbonyl)-1,2,3,4-tetrahydro-pyrrolo[1,2-a]pyrazine-6-carboxylic acid isopropylamide
(6-Chloro-pyrazolo[1,5-a]pyridin-2-yl)-[1-methyl-6-(1H-tetrazol-5-yl)-3,4-dihydro-1H-pyrrolo[1,2-a]pyrazin-2-yl]-methanone
(6-Chloro-pyrazolo[1,5-a]pyridin-2-yl)-[6-(1H-tetrazol-5-yl)-3,4-dihydro-1H-pyrrolo[1,2-a]pyrazin-2-yl]-methanone
(6-Bromo-pyrazolo[1,5-a]pyridin-2-yl)-[1-methyl-6-(1H-tetrazol-5-yl)-3,4-dihydro-1H-pyrrolo[1,2-a]pyrazin-2-yl]-methanone
(6-Bromo-pyrazolo[1,5-a]pyridin-2-yl)-[6-(1H-tetrazol-5-yl)-3,4-dihydro-1H-pyrrolo[1,2-a]pyrazin-2-yl]-methanone
(6-Chloro-pyrazolo[1,5-a]pyridin-2-yl)-(1-methyl-6-pyridin-2-yl-3,4-dihydro-1H-pyrrolo[1,2-a]pyrazin-2-yl)-methanone
(6-Chloro-pyrazolo[1,5-a]pyridin-2-yl)-(6-pyridin-2-yl-3,4-dihydro-1H-pyrrolo[1,2-a]pyrazin-2-yl)-methanone
(6-Bromo-pyrazolo[1,5-a]pyridin-2-yl)-(1-methyl-6-pyridin-2-yl-3,4-dihydro-1H-pyrrolo[1,2-a]pyrazin-2-yl)-methanone
(6-Bromo-pyrazolo[1,5-a]pyridin-2-yl)-(6-pyridin-2-yl-3,4-dihydro-1H-pyrrolo[1,2-a]pyrazin-2-yl)-methanone
(6-Chloro-pyrazolo[1,5-a]pyridin-2-yl)-(1-methyl-6-pyridin-3-yl-3,4-dihydro-1H-pyrrolo[1,2-a]pyrazin-2-yl)-methanone
(6-Chloro-pyrazolo[1,5-a]pyridin-2-yl)-(6-pyridin-3-yl-3,4-dihydro-1H-pyrrolo[1,2-a]pyrazin-2-yl)-methanone
(6-Bromo-pyrazolo[1,5-a]pyridin-2-yl)-(1-methyl-6-pyridin-3-yl-3,4-dihydro-1H-pyrrolo[1,2-a]pyrazin-2-yl)-methanone
(6-Bromo-pyrazolo[1,5-a]pyridin-2-yl)-(6-pyridin-3-yl-3,4-dihydro-1H-pyrrolo[1,2-a]pyrazin-2-yl)-methanone
(6-Chloro-pyrazolo[1,5-a]pyridin-2-yl)-(1-methyl-6-pyridin-4-yl-3,4-dihydro-1H-pyrrolo[1,2-a]pyrazin-2-yl)-methanone
(6-Chloro-pyrazolo[1,5-a]pyridin-2-yl)-(6-pyridin-4-yl-3,4-dihydro-1H-pyrrolo[1,2-a]pyrazin-2-yl)-methanone
(6-Bromo-pyrazolo[1,5-a]pyridin-2-yl)-(1-methyl-6-pyridin-4-yl-3,4-dihydro-1H-pyrrolo[1,2-a]pyrazin-2-yl)-methanone
(6-Bromo-pyrazolo[1,5-a]pyridin-2-yl)-(6-pyridin-4-yl-3,4-dihydro-1H-pyrrolo[1,2-a]pyrazin-2-yl)-methanone
(6-Chloro-pyrazolo[1,5-a]pyridin-2-yl)-(1-methyl-6-thiophen-2-yl-3,4-dihydro-1H-pyrrolo[1,2-a]pyrazin-2-yl)-methanone
(6-Chloro-pyrazolo[1,5-a]pyridin-2-yl)-(6-thiophen-2-yl-3,4-dihydro-1H-pyrrolo[1,2-a]pyrazin-2-yl)-methanone
(6-Bromo-pyrazolo[1,5-a]pyridin-2-yl)-(1-methyl-6-thiophen-2-yl-3,4-dihydro-1H-pyrrolo[1,2-a]pyrazin-2-yl)-methanone
(6-Bromo-pyrazolo[1,5-a]pyridin-2-yl)-(6-thiophen-2-yl-3,4-dihydro-1H-pyrrolo[1,2-a]pyrazin-2-yl)-methanone
(6-Chloro-pyrazolo[1,5-a]pyridin-2-yl)-(6-furan-2-yl-1-methyl-3,4-dihydro-1H-pyrrolo[1,2-a]pyrazin-2-yl)-methanone
(6-Chloro-pyrazolo[1,5-a]pyridin-2-yl)-(6-furan-2-yl-3,4-dihydro-1H-pyrrolo[1,2-a]pyrazin-2-yl)-methanone
(6-Bromo-pyrazolo[1,5-a]pyridin-2-yl)-(6-furan-2-yl-1-methyl-3,4-dihydro-1H-pyrrolo[1,2-a]pyrazin-2-yl)-methanone
(6-Bromo-pyrazolo[1,5-a]pyridin-2-yl)-(6-furan-2-yl-3,4-dihydro-1H-pyrrolo[1,2-a]pyrazin-2-yl)-methanone
(6-Chloro-pyrazolo[1,5-a]pyridin-2-yl)-(1-methyl-6-thiophen-3-yl-3,4-dihydro-1H-pyrrolo[1,2-a]pyrazin-2-yl)-methanone
(6-Chloro-pyrazolo[1,5-a]pyridin-2-yl)-(6-thiophen-3-yl-3,4-dihydro-1H-pyrrolo[1,2-a]pyrazin-2-yl)-methanone
(6-Bromo-pyrazolo[1,5-a]pyridin-2-yl)-(1-methyl-6-thiophen-3-yl-3,4-dihydro-1H-pyrrolo[1,2-a]pyrazin-2-yl)-methanone
(6-Bromo-pyrazolo[1,5-a]pyridin-2-yl)-(6-thiophen-3-yl-3,4-dihydro-1H-pyrrolo[1,2-a]pyrazin-2-yl)-methanone
(6-Chloro-pyrazolo[1,5-a]pyridin-2-yl)-(6-furan-3-yl-1-methyl-3,4-dihydro-1H-pyrrolo[1,2-a]pyrazin-2-yl)-methanone
(6-Chloro-pyrazolo[1,5-a]pyridin-2-yl)-(6-furan-3-yl-3,4-dihydro-1H-pyrrolo[1,2-a]pyrazin-2-yl)-methanone
(6-Bromo-pyrazolo[1,5-a]pyridin-2-yl)-(6-furan-3-yl-1-methyl-3,4-dihydro-1H-pyrrolo[1,2-a]pyrazin-2-yl)-methanone
(6-Bromo-pyrazolo[1,5-a]pyridin-2-yl)-(6-furan-3-yl-3,4-dihydro-1H-pyrrolo[1,2-a]pyrazin-2-yl)-methanone

(6-Chloro-pyrazolo[1,5-a]pyridin-2-yl)-(1-methyl-6-pyrimidin-4-yl-3,4-dihydro-1H-pyrrolo[1,2-a]pyrazin-2-yl)-methanone
(6-Chloro-pyrazolo[1,5-a]pyridin-2-yl)-(6-pyrimidin-4-yl-3,4-dihydro-1H-pyrrolo[1,2-a]pyrazin-2-yl)-methanone
(6-Bromo-pyrazolo[1,5-a]pyridin-2-yl)-(1-methyl-6-pyrimidin-4-yl-3,4-dihydro-1H-pyrrolo[1,2-a]pyrazin-2-yl)-methanone
(6-Bromo-pyrazolo[1,5-a]pyridin-2-yl)-(6-pyrimidin-4-yl-3,4-dihydro-1H-pyrrolo[1,2-a]pyrazin-2-yl)-methanone
(6-Chloro-pyrazolo[1,5-a]pyridin-2-yl)-(1-methyl-6-pyrimidin-5-yl-3,4-dihydro-1H-pyrrolo[1,2-a]pyrazin-2-yl)-methanone
(6-Chloro-pyrazolo[1,5-a]pyridin-2-yl)-(6-pyrimidin-5-yl-3,4-dihydro-1H-pyrrolo[1,2-a]pyrazin-2-yl)-methanone
(6-Bromo-pyrazolo[1,5-a]pyridin-2-yl)-(1-methyl-6-pyrimidin-5-yl-3,4-dihydro-1H-pyrrolo[1,2-a]pyrazin-2-yl)-methanone
(6-Bromo-pyrazolo[1,5-a]pyridin-2-yl)-(6-pyrimidin-5-yl-3,4-dihydro-1H-pyrrolo[1,2-a]pyrazin-2-yl)-methanone
(6-Chloro-pyrazolo[1,5-a]pyridin-2-yl)-(8-methyl-3-pyridin-2-yl-5,6-dihydro-8H-[1,2,4]triazolo[4,3-a]pyrazin-7-yl)-methanone
(6-Chloro-pyrazolo[1,5-a]pyridin-2-yl)-(3-pyridin-2-yl-5,6-dihydro-8H-[1,2,4]triazolo[4,3-a]pyrazin-7-yl)-methanone
(6-Bromo-pyrazolo[1,5-a]pyridin-2-yl)-(8-methyl-3-pyridin-2-yl-5,6-dihydro-8H-[1,2,4]triazolo[4,3-a]pyrazin-7-yl)-methanone
(6-Bromo-pyrazolo[1,5-a]pyridin-2-yl)-(3-pyridin-2-yl-5,6-dihydro-8H-[1,2,4]triazolo[4,3-a]pyrazin-7-yl)-methanone
(6-Chloro-pyrazolo[1,5-a]pyridin-2-yl)-(8-methyl-3-pyridin-3-yl-5,6-dihydro-8H-[1,2,4]triazolo[4,3-a]pyrazin-7-yl)-methanone
(6-Chloro-pyrazolo[1,5-a]pyridin-2-yl)-(3-pyridin-3-yl-5,6-dihydro-8H-[1,2,4]triazolo[4,3-a]pyrazin-7-yl)-methanone
(6-Bromo-pyrazolo[1,5-a]pyridin-2-yl)-(8-methyl-3-pyridin-3-yl-5,6-dihydro-8H-[1,2,4]triazolo[4,3-a]pyrazin-7-yl)-methanone
(6-Bromo-pyrazolo[1,5-a]pyridin-2-yl)-(3-pyridin-3-yl-5,6-dihydro-8H-[1,2,4]triazolo[4,3-a]pyrazin-7-yl)-methanone
(6-Chloro-pyrazolo[1,5-a]pyridin-2-yl)-(8-methyl-3-pyridin-4-yl-5,6-dihydro-8H-[1,2,4]triazolo[4,3-a]pyrazin-7-yl)-methanone
(6-Chloro-pyrazolo[1,5-a]pyridin-2-yl)-(3-pyridin-4-yl-5,6-dihydro-8H-[1,2,4]triazolo[4,3-a]pyrazin-7-yl)-methanone
(6-Bromo-pyrazolo[1,5-a]pyridin-2-yl)-(8-methyl-3-pyridin-4-yl-5,6-dihydro-8H-[1,2,4]triazolo[4,3-a]pyrazin-7-yl)-methanone
(6-Bromo-pyrazolo[1,5-a]pyridin-2-yl)-(3-pyridin-4-yl-5,6-dihydro-8H-[1,2,4]triazolo[4,3-a]pyrazin-7-yl)-methanone
(6-Chloro-pyrazolo[1,5-a]pyridin-2-yl)-(8-methyl-3-thiophen-2-yl-5,6-dihydro-8H-[1,2,4]triazolo[4,3-a]pyrazin-7-yl)-methanone
(6-Chloro-pyrazolo[1,5-a]pyridin-2-yl)-(3-thiophen-2-yl-5,6-dihydro-8H-[1,2,4]triazolo[4,3-a]pyrazin-7-yl)-methanone
(6-Bromo-pyrazolo[1,5-a]pyridin-2-yl)-(8-methyl-3-thiophen-2-yl-5,6-dihydro-8H-[1,2,4]triazolo[4,3-a]pyrazin-7-yl)-methanone
(6-Bromo-pyrazolo[1,5-a]pyridin-2-yl)-(3-thiophen-2-yl-5,6-dihydro-8H-[1,2,4]triazolo[4,3-a]pyrazin-7-yl)-methanone
(6-Chloro-pyrazolo[1,5-a]pyridin-2-yl)-(3-furan-2-yl-8-methyl-5,6-dihydro-8H-[1,2,4]triazolo[4,3-a]pyrazin-7-yl)-methanone
(6-Chloro-pyrazolo[1,5-a]pyridin-2-yl)-(3-furan-2-yl-5,6-dihydro-8H-[1,2,4]triazolo[4,3-a]pyrazin-7-yl)-methanone
(6-Bromo-pyrazolo[1,5-a]pyridin-2-yl)-(3-furan-2-yl-8-methyl-5,6-dihydro-8H-[1,2,4]triazolo[4,3-a]pyrazin-7-yl)-methanone
(6-Bromo-pyrazolo[1,5-a]pyridin-2-yl)-(3-furan-2-yl-5,6-dihydro-8H-[1,2,4]triazolo[4,3-a]pyrazin-7-yl)-methanone
(6-Chloro-pyrazolo[1,5-a]pyridin-2-yl)-(8-methyl-3-thiophen-3-yl-5,6-dihydro-8H-[1,2,4]triazolo[4,3-a]pyrazin-7-yl)-methanone
(6-Chloro-pyrazolo[1,5-a]pyridin-2-yl)-(3-thiophen-3-yl-5,6-dihydro-8H-[1,2,4]triazolo[4,3-a]pyrazin-7-yl)-methanone
(6-Bromo-pyrazolo[1,5-a]pyridin-2-yl)-(8-methyl-3-thiophen-3-yl-5,6-dihydro-8H-[1,2,4]triazolo[4,3-a]pyrazin-7-yl)-methanone
(6-Bromo-pyrazolo[1,5-a]pyridin-2-yl)-(3-thiophen-3-yl-5,6-dihydro-8H-[1,2,4]triazolo[4,3-a]pyrazin-7-yl)-methanone

(6-Chloro-pyrazolo[1,5-a]pyridin-2-yl)-(3-furan-3-yl-8-methyl-5,6-dihydro-8H-[1,2,4]triazolo[4,3-a]pyrazin-7-yl)-methanone
(6-Chloro-pyrazolo[1,5-a]pyridin-2-yl)-(3-furan-3-yl-5,6-dihydro-8H-[1,2,4]triazolo[4,3-a]pyrazin-7-yl)-methanone
(6-Bromo-pyrazolo[1,5-a]pyridin-2-yl)-(3-furan-3-yl-8-methyl-5,6-dihydro-8H-[1,2,4]triazolo[4,3-a]pyrazin-7-yl)-methanone
(6-Bromo-pyrazolo[1,5-a]pyridin-2-yl)-(3-furan-3-yl-5,6-dihydro-8H-[1,2,4]triazolo[4,3-a]pyrazin-7-yl)-methanone
(6-Chloro-pyrazolo[1,5-a]pyridin-2-yl)-(8-methyl-3-pyrimidin-4-yl-5,6-dihydro-8H-[1,2,4]triazolo[4,3-a]pyrazin-7-yl)-methanone
(6-Chloro-pyrazolo[1,5-a]pyridin-2-yl)-(3-pyrimidin-4-yl-5,6-dihydro-8H-[1,2,4]triazolo[4,3-a]pyrazin-7-yl)-methanone
(6-Bromo-pyrazolo[1,5-a]pyridin-2-yl)-(8-methyl-3-pyrimidin-4-yl-5,6-dihydro-8H-[1,2,4]triazolo[4,3-a]pyrazin-7-yl)-methanone
(6-Bromo-pyrazolo[1,5-a]pyridin-2-yl)-(3-pyrimidin-4-yl-5,6-dihydro-8H-[1,2,4]triazolo[4,3-a]pyrazin-7-yl)-methanone
(6-Chloro-pyrazolo[1,5-a]pyridin-2-yl)-(8-methyl-3-pyrimidin-5-yl-5,6-dihydro-8H-[1,2,4]triazolo[4,3-a]pyrazin-7-yl)-methanone
(6-Chloro-pyrazolo[1,5-a]pyridin-2-yl)-(3-pyrimidin-5-yl-5,6-dihydro-8H-[1,2,4]triazolo[4,3-a]pyrazin-7-yl)-methanone
(6-Bromo-pyrazolo[1,5-a]pyridin-2-yl)-(8-methyl-3-pyrimidin-5-yl-5,6-dihydro-8H-[1,2,4]triazolo[4,3-a]pyrazin-7-yl)-methanone
(6-Bromo-pyrazolo[1,5-a]pyridin-2-yl)-(3-pyrimidin-5-yl-5,6-dihydro-8H-[1,2,4]triazolo[4,3-a]pyrazin-7-yl)-methanone
(6-Chloro-pyrazolo[1,5-a]pyridin-2-yl)-[3-(6-fluoro-pyridin-3-yl)-8-methyl-5,6-dihydro-8H-imidazo[1,2-a]pyrazin-7-yl]-methanone
(6-Chloro-pyrazolo[1,5-a]pyridin-2-yl)-[3-(6-fluoro-pyridin-3-yl)-5,6-dihydro-8H-imidazo[1,2-a]pyrazin-7-yl]-methanone
(6-Bromo-pyrazolo[1,5-a]pyridin-2-yl)-[3-(6-fluoro-pyridin-3-yl)-8-methyl-5,6-dihydro-8H-imidazo[1,2-a]pyrazin-7-yl]-methanone
(6-Bromo-pyrazolo[1,5-a]pyridin-2-yl)-[3-(6-fluoro-pyridin-3-yl)-5,6-dihydro-8H-imidazo[1,2-a]pyrazin-7-yl]-methanone

(6-Chloro-pyrazolo[1,5-a]pyridin-2-yl)-[3-(2-fluoro-pyridin-3-yl)-8-methyl-5,6-dihydro-8H-imidazo[1,2-a]pyrazin-7-yl]-methanone
(6-Chloro-pyrazolo[1,5-a]pyridin-2-yl)-[3-(2-fluoro-pyridin-3-yl)-5,6-dihydro-8H-imidazo[1,2-a]pyrazin-7-yl]-methanone
(6-Bromo-pyrazolo[1,5-a]pyridin-2-yl)-[3-(2-fluoro-pyridin-3-yl)-8-methyl-5,6-dihydro-8H-imidazo[1,2-a]pyrazin-7-yl]-methanone
(6-Bromo-pyrazolo[1,5-a]pyridin-2-yl)-[3-(2-fluoro-pyridin-3-yl)-5,6-dihydro-8H-imidazo[1,2-a]pyrazin-7-yl]-methanone
(6-Chloro-pyrazolo[1,5-a]pyridin-2-yl)-[2-(6-fluoro-pyridin-3-yl)-8-methyl-5,6-dihydro-8H-imidazo[1,2-a]pyrazin-7-yl]-methanone
(6-Chloro-pyrazolo[1,5-a]pyridin-2-yl)-[2-(6-fluoro-pyridin-3-yl)-5,6-dihydro-8H-imidazo[1,2-a]pyrazin-7-yl]-methanone
(6-Bromo-pyrazolo[1,5-a]pyridin-2-yl)-[2-(6-fluoro-pyridin-3-yl)-8-methyl-5,6-dihydro-8H-imidazo[1,2-a]pyrazin-7-yl]-methanone
(6-Bromo-pyrazolo[1,5-a]pyridin-2-yl)-[2-(6-fluoro-pyridin-3-yl)-5,6-dihydro-8H-imidazo[1,2-a]pyrazin-7-yl]-methanone
(6-Chloro-pyrazolo[1,5-a]pyridin-2-yl)-[2-(2-fluoro-pyridin-3-yl)-8-methyl-5,6-dihydro-8H-imidazo[1,2-a]pyrazin-7-yl]-methanone
(6-Chloro-pyrazolo[1,5-a]pyridin-2-yl)-[2-(2-fluoro-pyridin-3-yl)-5,6-dihydro-8H-imidazo[1,2-a]pyrazin-7-yl]-methanone
(6-Bromo-pyrazolo[1,5-a]pyridin-2-yl)-[2-(2-fluoro-pyridin-3-yl)-8-methyl-5,6-dihydro-8H-imidazo[1,2-a]pyrazin-7-yl]-methanone
(6-Bromo-pyrazolo[1,5-a]pyridin-2-yl)-[2-(2-fluoro-pyridin-3-yl)-5,6-dihydro-8H-imidazo[1,2-a]pyrazin-7-yl]-methanone
(6-Chloro-pyrazolo[1,5-a]pyridin-2-yl)-[3-(6-fluoro-pyridin-3-yl)-8-methyl-5,6-dihydro-8H-[1,2,4]triazolo[4,3-a]pyrazin-7-yl]-methanone
(6-Chloro-pyrazolo[1,5-a]pyridin-2-yl)-[3-(6-fluoro-pyridin-3-yl)-5,6-dihydro-8H-[1,2,4]triazolo[4,3-a]pyrazin-7-yl]-methanone
(6-Bromo-pyrazolo[1,5-a]pyridin-2-yl)-[3-(6-fluoro-pyridin-3-yl)-8-methyl-5,6-dihydro-8H-[1,2,4]triazolo[4,3-a]pyrazin-7-yl]-methanone
(6-Bromo-pyrazolo[1,5-a]pyridin-2-yl)-[3-(6-fluoro-pyridin-3-yl)-5,6-dihydro-8H-[1,2,4]triazolo[4,3-a]pyrazin-7-yl]-methanone

(6-Chloro-pyrazolo[1,5-a]pyridin-2-yl)-[3-(2-fluoro-pyridin-3-yl)-8-methyl-5,6-dihydro-8H-[1,2,4]triazolo[4,3-a]pyrazin-7-yl]-methanone
(6-Chloro-pyrazolo[1,5-a]pyridin-2-yl)-[3-(2-fluoro-pyridin-3-yl)-5,6-dihydro-8H-[1,2,4]triazolo[4,3-a]pyrazin-7-yl]-methanone
(6-Bromo-pyrazolo[1,5-a]pyridin-2-yl)-[3-(2-fluoro-pyridin-3-yl)-8-methyl-5,6-dihydro-8H-[1,2,4]triazolo[4,3-a]pyrazin-7-yl]-methanone
(6-Bromo-pyrazolo[1,5-a]pyridin-2-yl)-[3-(2-fluoro-pyridin-3-yl)-5,6-dihydro-8H-[1,2,4]triazolo[4,3-a]pyrazin-7-yl]-methanone
(6-Chloro-pyrazolo[1,5-a]pyridin-2-yl)-(1-methyl-3,4-dihydro-1H-cinnolin-2-yl)-methanone
(6-Bromo-pyrazolo[1,5-a]pyridin-2-yl)-(1-methyl-3,4-dihydro-1H-cinnolin-2-yl)-methanone
(6-Chloro-pyrazolo[1,5-a]pyridin-2-yl)-(1-methyl-3,4-dihydro-1H-thieno[3,2-c]pyridazin-2-yl)-methanone
(6-Bromo-pyrazolo[1,5-a]pyridin-2-yl)-(1-methyl-3,4-dihydro-1H-thieno[3,2-c]pyridazin-2-yl)-methanone
(6-Chloro-pyrazolo[1,5-a]pyridin-2-yl)-(1,6-dimethyl-3,4-dihydro-1H-thieno[3,2-c]pyridazin-2-yl)-methanone
(6-Bromo-pyrazolo[1,5-a]pyridin-2-yl)-(1,6-dimethyl-3,4-dihydro-1H-thieno[3,2-c]pyridazin-2-yl)-methanone
(6-Chloro-pyrazolo[1,5-a]pyridin-2-yl)-(1,7-dimethyl-3,4-dihydro-1H-thieno[3,2-c]pyridazin-2-yl)-methanone
(6-Bromo-pyrazolo[1,5-a]pyridin-2-yl)-(1,7-dimethyl-3,4-dihydro-1H-thieno[3,2-c]pyridazin-2-yl)-methanone
(6-Chloro-pyrazolo[1,5-a]pyridin-2-yl)-(1,6,7-trimethyl-3,4-dihydro-1H-thieno[3,2-c]pyridazin-2-yl)-methanone
(6-Bromo-pyrazolo[1,5-a]pyridin-2-yl)-(1,6,7-trimethyl-3,4-dihydro-1H-thieno[3,2-c]pyridazin-2-yl)-methanone
(6-Bromo-1-methyl-3,4-dihydro-1H-thieno[3,2-c]pyridazin-2-yl)-(6-chloro-pyrazolo[1,5-a]pyridin-2-yl)-methanone
(6-Bromo-1-methyl-3,4-dihydro-1H-thieno[3,2-c]pyridazin-2-yl)-(6-bromo-pyrazolo[1,5-a]pyridin-2-yl)-methanone

(6-Chloro-1-methyl-3,4-dihydro-1H-thieno[3,2-c]pyridazin-2-yl)-(6-chloro-pyrazolo[1,5-a]pyridin-2-yl)-methanone
(6-Bromo-1-methyl-3,4-dihydro-1H-thieno[3,2-c]pyridazin-2-yl)-(6-chloro-pyrazolo[1,5-a]pyridin-2-yl)-methanone
(6-Chloro-pyrazolo[1,5-a]pyridin-2-yl)-(1-methyl-3,4-dihydro-1H-furo[3,2-c]pyridazin-2-yl)-methanone
(6-Bromo-pyrazolo[1,5-a]pyridin-2-yl)-(1-methyl-3,4-dihydro-1H-furo[3,2-c]pyridazin-2-yl)-methanone
(6-Chloro-pyrazolo[1,5-a]pyridin-2-yl)-(1,6-dimethyl-3,4-dihydro-1H-furo[3,2-c]pyridazin-2-yl)-methanone
(6-Bromo-pyrazolo[1,5-a]pyridin-2-yl)-(1,6-dimethyl-3,4-dihydro-1H-furo[3,2-c]pyridazin-2-yl)-methanone
(6-Chloro-pyrazolo[1,5-a]pyridin-2-yl)-(1,7-dimethyl-3,4-dihydro-1H-furo[3,2-c]pyridazin-2-yl)-methanone
(6-Bromo-pyrazolo[1,5-a]pyridin-2-yl)-(1,7-dimethyl-3,4-dihydro-1H-furo[3,2-c]pyridazin-2-yl)-methanone
(6-Chloro-pyrazolo[1,5-a]pyridin-2-yl)-(1,6,7-trimethyl-3,4-dihydro-1H-furo[3,2-c]pyridazin-2-yl)-methanone
(6-Bromo-pyrazolo[1,5-a]pyridin-2-yl)-(1,6,7-trimethyl-3,4-dihydro-1H-furo[3,2-c]pyridazin-2-yl)-methanone
(6-Bromo-1-methyl-3,4-dihydro-1H-furo[3,2-c]pyridazin-2-yl)-(6-chloro-pyrazolo[1,5-a]pyridin-2-yl)-methanone
(6-Bromo-1-methyl-3,4-dihydro-1H-furo[3,2-c]pyridazin-2-yl)-(6-bromo-pyrazolo[1,5-a]pyridin-2-yl)-methanone
(6-Chloro-1-methyl-3,4-dihydro-1H-furo[3,2-c]pyridazin-2-yl)-(6-chloro-pyrazolo[1,5-a]pyridin-2-yl)-methanone
(6-Bromo-1-methyl-3,4-dihydro-1H-furo[3,2-c]pyridazin-2-yl)-(6-chloro-pyrazolo[1,5-a]pyridin-2-yl)-methanone
(6-Chloro-pyrazolo[1,5-a]pyridin-2-yl)-(1-methyl-1,3,4,5-tetrahydro-pyrrolo[3,2-c]pyridazin-2-yl)-methanone
(6-Bromo-pyrazolo[1,5-a]pyridin-2-yl)-(1-methyl-1,3,4,5-tetrahydro-pyrrolo[3,2-c]pyridazin-2-yl)-methanone

(6-Chloro-pyrazolo[1,5-a]pyridin-2-yl)-(1,6-dimethyl-1,3,4,5-tetrahydro-pyrrolo[3,2-c]pyridazin-2-yl)-methanone
(6-Bromo-pyrazolo[1,5-a]pyridin-2-yl)-(1,6-dimethyl-1,3,4,5-tetrahydro-pyrrolo[3,2-c]pyridazin-2-yl)-methanone
(6-Chloro-pyrazolo[1,5-a]pyridin-2-yl)-(1,7-dimethyl-1,3,4,5-tetrahydro-pyrrolo[3,2-c]pyridazin-2-yl)-methanone
(6-Bromo-pyrazolo[1,5-a]pyridin-2-yl)-(1,7-dimethyl-1,3,4,5-tetrahydro-pyrrolo[3,2-c]pyridazin-2-yl)-methanone
(6-Chloro-pyrazolo[1,5-a]pyridin-2-yl)-(1,6,7-trimethyl-1,3,4,5-tetrahydro-pyrrolo[3,2-c]pyridazin-2-yl)-methanone
(6-Bromo-pyrazolo[1,5-a]pyridin-2-yl)-(1,6,7-trimethyl-1,3,4,5-tetrahydro-pyrrolo[3,2-c]pyridazin-2-yl)-methanone
(6-Bromo-1-methyl-1,3,4,5-tetrahydro-pyrrolo[3,2-c]pyridazin-2-yl)-(6-chloro-pyrazolo[1,5-a]pyridin-2-yl)-methanone
(6-Bromo-1-methyl-1,3,4,5-tetrahydro-pyrrolo[3,2-c]pyridazin-2-yl)-(6-bromo-pyrazolo[1,5-a]pyridin-2-yl)-methanone
(6-Chloro-1-methyl-1,3,4,5-tetrahydro-pyrrolo[3,2-c]pyridazin-2-yl)-(6-chloro-pyrazolo[1,5-a]pyridin-2-yl)-methanone
(6-Bromo-1-methyl-1,3,4,5-tetrahydro-pyrrolo[3,2-c]pyridazin-2-yl)-(6-chloro-pyrazolo[1,5-a]pyridin-2-yl)-methanone
(8-Chloro-1-methyl-3,4-dihydro-1H-cinnolin-2-yl)-(6-chloro-pyrazolo[1,5-a]pyridin-2-yl)-methanone
(8-Bromo-1-methyl-3,4-dihydro-1H-cinnolin-2-yl)-(6-chloro-pyrazolo[1,5-a]pyridin-2-yl)-methanone
(7-Chloro-1-methyl-3,4-dihydro-1H-cinnolin-2-yl)-(6-chloro-pyrazolo[1,5-a]pyridin-2-yl)-methanone
(7-Bromo-1-methyl-3,4-dihydro-1H-cinnolin-2-yl)-(6-chloro-pyrazolo[1,5-a]pyridin-2-yl)-methanone
(6-Chloro-1-methyl-3,4-dihydro-1H-cinnolin-2-yl)-(6-chloro-pyrazolo[1,5-a]pyridin-2-yl)-methanone
(6-Bromo-1-methyl-3,4-dihydro-1H-cinnolin-2-yl)-(6-chloro-pyrazolo[1,5-a]pyridin-2-yl)-methanone

(5-Chloro-1-methyl-3,4-dihydro-1H-cinnolin-2-yl)-(6-chloro-pyrazolo[1,5-a]pyridin-2-yl)-methanone
(5-Bromo-1-methyl-3,4-dihydro-1H-cinnolin-2-yl)-(6-chloro-pyrazolo[1,5-a]pyridin-2-yl)-methanone
(8-Bromo-1-methyl-3,4-dihydro-1H-cinnolin-2-yl)-(6-chloro-pyrazolo[1,5-a]pyridin-2-yl)-methanone
(8-Bromo-1-methyl-3,4-dihydro-1H-cinnolin-2-yl)-(6-bromo-pyrazolo[1,5-a]pyridin-2-yl)-methanone
(7-Bromo-1-methyl-3,4-dihydro-1H-cinnolin-2-yl)-(6-chloro-pyrazolo[1,5-a]pyridin-2-yl)-methanone
(7-Bromo-1-methyl-3,4-dihydro-1H-cinnolin-2-yl)-(6-bromo-pyrazolo[1,5-a]pyridin-2-yl)-methanone
(6-Bromo-1-methyl-3,4-dihydro-1H-cinnolin-2-yl)-(6-chloro-pyrazolo[1,5-a]pyridin-2-yl)-methanone
(6-Bromo-1-methyl-3,4-dihydro-1H-cinnolin-2-yl)-(6-bromo-pyrazolo[1,5-a]pyridin-2-yl)-methanone
(5-Bromo-1-methyl-3,4-dihydro-1H-cinnolin-2-yl)-(6-chloro-pyrazolo[1,5-a]pyridin-2-yl)-methanone
(5-Bromo-1-methyl-3,4-dihydro-1H-cinnolin-2-yl)-(6-bromo-pyrazolo[1,5-a]pyridin-2-yl)-methanone
(8-Fluoro-1-methyl-3,4-dihydro-1H-cinnolin-2-yl)-(6-chloro-pyrazolo[1,5-a]pyridin-2-yl)-methanone
(8-Fluoro-1-methyl-3,4-dihydro-1H-cinnolin-2-yl)-(6-bromo-pyrazolo[1,5-a]pyridin-2-yl)-methanone
(7-Fluoro-1-methyl-3,4-dihydro-1H-cinnolin-2-yl)-(6-chloro-pyrazolo[1,5-a]pyridin-2-yl)-methanone
(7-Fluoro-1-methyl-3,4-dihydro-1H-cinnolin-2-yl)-(6-bromo-pyrazolo[1,5-a]pyridin-2-yl)-methanone
(6-Fluoro-1-methyl-3,4-dihydro-1H-cinnolin-2-yl)-(6-chloro-pyrazolo[1,5-a]pyridin-2-yl)-methanone
(6-Fluoro-1-methyl-3,4-dihydro-1H-cinnolin-2-yl)-(6-bromo-pyrazolo[1,5-a]pyridin-2-yl)-methanone

(5-Fluoro-1-methyl-3,4-dihydro-1H-cinnolin-2-yl)-(6-chloro-pyrazolo[1,5-a]pyridin-2-yl)-methanone
(5-Fluoro-1-methyl-3,4-dihydro-1H-cinnolin-2-yl)-(6-bromo-pyrazolo[1,5-a]pyridin-2-yl)-methanone
(6-Chloro-pyrazolo[1,5-a]pyridin-2-yl)-(1,8-dimethyl-3,4-dihydro-1H-cinnolin-2-yl)-methanone
(6-Bromo-pyrazolo[1,5-a]pyridin-2-yl)-(1,8-dimethyl-3,4-dihydro-1H-cinnolin-2-yl)-methanone
(6-Chloro-pyrazolo[1,5-a]pyridin-2-yl)-(1,7-dimethyl-3,4-dihydro-1H-cinnolin-2-yl)-methanone
(6-Bromo-pyrazolo[1,5-a]pyridin-2-yl)-(1,7-dimethyl-3,4-dihydro-1H-cinnolin-2-yl)-methanone
(6-Chloro-pyrazolo[1,5-a]pyridin-2-yl)-(1,6-dimethyl-3,4-dihydro-1H-cinnolin-2-yl)-methanone
(6-Bromo-pyrazolo[1,5-a]pyridin-2-yl)-(1,6-dimethyl-3,4-dihydro-1H-cinnolin-2-yl)-methanone
(6-Chloro-pyrazolo[1,5-a]pyridin-2-yl)-(1,5-dimethyl-3,4-dihydro-1H-cinnolin-2-yl)-methanone
(6-Bromo-pyrazolo[1,5-a]pyridin-2-yl)-(1,5-dimethyl-3,4-dihydro-1H-cinnolin-2-yl)-methanone
(6-Chloro-pyrazolo[1,5-a]pyridin-2-yl)-(8-methoxy-1-methyl-3,4-dihydro-1H-cinnolin-2-yl)-methanone
(6-Bromo-pyrazolo[1,5-a]pyridin-2-yl)-(8-methoxy-1-methyl-3,4-dihydro-1H-cinnolin-2-yl)-methanone
(6-Chloro-pyrazolo[1,5-a]pyridin-2-yl)-(7-methoxy-1-methyl-3,4-dihydro-1H-cinnolin-2-yl)-methanone
(6-Bromo-pyrazolo[1,5-a]pyridin-2-yl)-(7-methoxy-1-methyl-3,4-dihydro-1H-cinnolin-2-yl)-methanone
(6-Chloro-pyrazolo[1,5-a]pyridin-2-yl)-(6-methoxy-1-methyl-3,4-dihydro-1H-cinnolin-2-yl)-methanone
(6-Bromo-pyrazolo[1,5-a]pyridin-2-yl)-(6-methoxy-1-methyl-3,4-dihydro-1H-cinnolin-2-yl)-methanone

(6-Chloro-pyrazolo[1,5-a]pyridin-2-yl)-(5-methoxy-1-methyl-3,4-dihydro-1H-cinnolin-2-yl)-methanone
(6-Bromo-pyrazolo[1,5-a]pyridin-2-yl)-(5-methoxy-1-methyl-3,4-dihydro-1H-cinnolin-2-yl)-methanone
(6-Chloro-pyrazolo[1,5-a]pyridin-2-yl)-(1-methyl-8-trifluoromethyl-3,4-dihydro-1H-cinnolin-2-yl)-methanone
(6-Bromo-pyrazolo[1,5-a]pyridin-2-yl)-(1 -methyl-8-trifluoromethyl-3,4-dihydro-1H-cinnolin-2-yl)-methanone
(6-Chloro-pyrazolo[1,5-a]pyridin-2-yl)-(1-methyl-7-trifluoromethyl-3,4-dihydro-1H-cinnolin-2-yl)-methanone
(6-Bromo-pyrazolo[1,5-a]pyridin-2-yl)-(1-methyl-7-trifluoromethyl-3,4-dihydro-1H-cinnolin-2-yl)-methanone
(6-Chloro-pyrazolo[1,5-a]pyridin-2-yl)-(1-methyl-6-trifluoromethyl-3,4-dihydro-1H-cinnolin-2-yl)-methanone
(6-Bromo-pyrazolo[1,5-a]pyridin-2-yl)-(1-methyl-6-trifluoromethyl-3,4-dihydro-1H-cinnolin-2-yl)-methanone
(6-Chloro-pyrazolo[1,5-a]pyridin-2-yl)-(1-methyl-5-trifluoromethyl-3,4-dihydro-1H-cinnolin-2-yl)-methanone
(6-Bromo-pyrazolo[1,5-a]pyridin-2-yl)-(1-methyl-5-trifluoromethyl-3,4-dihydro-1H-cinnolin-2-yl)-methanone
N-[2-(6-Chloro-pyrazolo[1,5-a]pyridine-2-carbonyl)-1-methyl-1,2,3,4-tetrahydro-cinnolin-8-yl]-acetamide
N-[2-(6-Bromo-pyrazolo[1,5-a]pyridine-2-carbonyl)-1-methyl-1,2,3,4-tetrahydro-cinnolin-8-yl]-acetamide
N-[2-(6-Chloro-pyrazolo[1,5-a]pyridine-2-carbonyl)-1-methyl-1,2,3,4-tetrahydro-cinnolin-7-yl]-acetamide
N-[2-(6-Bromo-pyrazolo[1,5-a]pyridine-2-carbonyl)-1-methyl-1,2,3,4-tetrahydro-cinnolin-7-yl]-acetamide
N-[2-(6-Chloro-pyrazolo[1,5-a]pyridine-2-carbonyl)-1-methyl-1,2,3,4-tetrahydro-cinnolin-6-yl]-acetamide
N-[2-(6-Bromo-pyrazolo[1,5-a]pyridine-2-carbonyl)-1-methyl-1,2,3,4-tetrahydro-cinnolin-6-yl]-acetamide

N-[2-(6-Chloro-pyrazolo[1,5-a]pyridine-2-carbonyl)-1-methyl-1,2,3,4-tetrahydro-cinnolin-5-yl]-acetamide
N-[2-(6-Bromo-pyrazolo[1,5-a]pyridine-2-carbonyl)-1-methyl-1,2,3,4-tetrahydro-cinnolin-5-yl]-acetamide
(6-Chloro-pyrazolo[1,5-a]pyridin-2-yl)-(8-methanesulfonyl-1-methyl-3,4-dihydro-1H-cinnolin-2-yl)-methanone
(6-Bromo-pyrazolo[1,5-a]pyridin-2-yl)-(8-methanesulfonyl-1-methyl-3,4-dihydro-1H-cinnolin-2-yl)-methanone
(6-Chloro-pyrazolo[1,5-a]pyridin-2-yl)-(7-methanesulfonyl-1-methyl-3,4-dihydro-1H-cinnolin-2-yl)-methanone
(6-Bromo-pyrazolo[1,5-a]pyridin-2-yl)-(7-methanesulfonyl-1-methyl-3,4-dihydro-1H-cinnolin-2-yl)-methanone
(6-Chloro-pyrazolo[1,5-a]pyridin-2-yl)-(6-methanesulfonyl-1-methyl-3,4-dihydro-1H-cinnolin-2-yl)-methanone
(6-Bromo-pyrazolo[1,5-a]pyridin-2-yl)-(6-methanesulfonyl-1-methyl-3,4-dihydro-1H-cinnolin-2-yl)-methanone
(6-Chloro-pyrazolo[1,5-a]pyridin-2-yl)-(5-methanesulfonyl-1-methyl-3,4-dihydro-1H-cinnolin-2-yl)-methanone
(6-Bromo-pyrazolo[1,5-a]pyridin-2-yl)-(5-methanesulfonyl-1-methyl-3,4-dihydro-1H-cinnolin-2-yl)-methanone
(6-Chloro-pyrazolo[1,5-a]pyridin-2-yl)-(1-methyl-3,4-dihydro-1H-pyrido[2,3-c]pyridazin-2-yl)-methanone
(6-Bromo-pyrazolo[1,5-a]pyridin-2-yl)-(1-methyl-3,4-dihydro-1H-pyrido[2,3-c]pyridazin-2-yl)-methanone
(6-Chloro-pyrazolo[1,5-a]pyridin-2-yl)-(1-methyl-3,4-dihydro-1H-pyrido[3,4-c]pyridazin-2-yl)-methanone
(6-Bromo-pyrazolo[1,5-a]pyridin-2-yl)-(1-methyl-3,4-dihydro-1H-pyrido[3,4-c]pyridazin-2-yl)-methanone
(6-Chloro-pyrazolo[1,5-a]pyridin-2-yl)-(1-methyl-3,4-dihydro-1H-pyrido[4,3-c]pyridazin-2-yl)-methanone
(6-Bromo-pyrazolo[1,5-a]pyridin-2-yl)-(1-methyl-3,4-dihydro-1H-pyrido[4,3-c]pyridazin-2-yl)-methanone

(6-Chloro-pyrazolo[1,5-a]pyridin-2-yl)-(1-methyl-3,4-dihydro-1H-pyrido[3,2-c]pyridazin-2-yl)-methanone
(6-Bromo-pyrazolo[1,5-a]pyridin-2-yl)-(1-methyl-3,4-dihydro-1H-pyrido[3,2-c]pyridazin-2-yl)-methanone
(7-Bromo-1-methyl-3,4-dihydro-1H-pyrido[3,2-c]pyridazin-2-yl)-(6-chloro-pyrazolo[1,5-a]pyridin-2-yl)-methanone
(7-Bromo-1-methyl-3,4-dihydro-1H-pyrido[3,2-c]pyridazin-2-yl)-(6-bromo-pyrazolo[1,5-a]pyridin-2-yl)-methanone
(6-Chloro-pyrazolo[1,5-a]pyridin-2-yl)-(1-methyl-8-pyridin-4-yl-3,4-dihydro-1H-cinnolin-2-yl)-methanone
(6-Bromo-pyrazolo[1,5-a]pyridin-2-yl)-(1-methyl-8-pyridin-4-yl-3,4-dihydro-1H-cinnolin-2-yl)-methanone
(6-Chloro-pyrazolo[1,5-a]pyridin-2-yl)-(1-methyl-7-pyridin-4-yl-3,4-dihydro-1H-cinnolin-2-yl)-methanone
(6-Bromo-pyrazolo[1,5-a]pyridin-2-yl)-(1-methyl-7-pyridin-4-yl-3,4-dihydro-1H-cinnolin-2-yl)-methanone
(6-Chloro-pyrazolo[1,5-a]pyridin-2-yl)-(1-methyl-6-pyridin-4-yl-3,4-dihydro-1H-cinnolin-2-yl)-methanone
(6-Bromo-pyrazolo[1,5-a]pyridin-2-yl)-(1-methyl-6-pyridin-4-yl-3,4-dihydro-1H-cinnolin-2-yl)-methanone
(6-Chloro-pyrazolo[1,5-a]pyridin-2-yl)-(1-methyl-5-pyridin-4-yl-3,4-dihydro-1H-cinnolin-2-yl)-methanone
(6-Bromo-pyrazolo[1,5-a]pyridin-2-yl)-(1-methyl-5-pyridin-4-yl-3,4-dihydro-1H-cinnolin-2-yl)-methanone
(6-Chloro-pyrazolo[1,5-a]pyridin-2-yl)-[8-(2-fluoro-pyridin-4-yl)-1-methyl-3,4-dihydro-1H-cinnolin-2-yl]-methanone
(6-Bromo-pyrazolo[1,5-a]pyridin-2-yl)-[8-(2-fluoro-pyridin-4-yl)-1-methyl-3,4-dihydro-1H-cinnolin-2-yl]-methanone
(6-Chloro-pyrazolo[1,5-a]pyridin-2-yl)-[7-(2-fluoro-pyridin-4-yl)-1-methyl-3,4-dihydro-1H-cinnolin-2-yl]-methanone
(6-Bromo-pyrazolo[1,5-a]pyridin-2-yl)-[7-(2-fluoro-pyridin-4-yl)-1-methyl-3,4-dihydro-1H-cinnolin-2-yl]-methanone

(6-Chloro-pyrazolo[1,5-a]pyridin-2-yl)-[6-(2-fluoro-pyridin-4-yl)-1-methyl-3,4-dihydro-1H-cinnolin-2-yl]-methanone
(6-Bromo-pyrazolo[1,5-a]pyridin-2-yl)-[6-(2-fluoro-pyridin-4-yl)-1-methyl-3,4-dihydro-1H-cinnolin-2-yl]-methanone
(6-Chloro-pyrazolo[1,5-a]pyridin-2-yl)-[5-(2-fluoro-pyridin-4-yl)-1-methyl-3,4-dihydro-1H-cinnolin-2-yl]-methanone
(6-Bromo-pyrazolo[1,5-a]pyridin-2-yl)-[5-(2-fluoro-pyridin-4-yl)-1-methyl-3,4-dihydro-1H-cinnolin-2-yl]-methanone
6-Bromo-pyrazolo[1,5-a]pyridine-2-carboxylic acid benzylamide
6-Chloro-pyrazolo[1,5-a]pyridine-2-carboxylic acid benzylamide
6-Chloro-pyrazolo[1,5-a]pyridine-2-carboxylic acid (1-methyl-1-phenyl-ethyl)-amide
6-Bromo-pyrazolo[1,5-a]pyridine-2-carboxylic acid (1-methyl-1-phenyl-ethyl)-amide
6-Bromo-pyrazolo[1,5-a]pyridine-2-carboxylic acid (2-methyl-2-phenyl-propyl)-amide
6-Chloro-pyrazolo[1,5-a]pyridine-2-carboxylic acid (2-methyl-2-phenyl-propyl)-amide
6-Chloro-pyrazolo[1,5-a]pyridine-2-carboxylic acid benzyl-methyl-amide
6-Bromo-pyrazolo[1,5-a]pyridine-2-carboxylic acid benzyl-methyl-amide
6-Bromo-pyrazolo[1,5-a]pyridine-2-carboxylic acid methyl-(1-phenyl-ethyl)-amide
6-Chloro-pyrazolo[1,5-a]pyridine-2-carboxylic acid methyl-(1-phenyl-ethyl)-amide
(4,6-Dichloro-pyrazolo[1,5-a]pyridin-2-yl)-(3,4-dihydro-1H-isoquinolin-2-yl)-methanone
(4,6-Dichloro-pyrazolo[1,5-a]pyridin-2-yl)-(1-methyl-3,4-dihydro-1H-isoquinolin-2-yl)-methanone
(4,6-Dichloro-pyrazolo[1,5-a]pyridin-2-yl)-(7-fluoro-1-methyl-3,4-dihydro-1H-isoquinolin-2-yl)-methanone
(3-Bromo-7,8-dihydro-5H-1,6-naphthyridin-6-yl)-(4,6-dichloro-pyrazolo[1,5-a]pyridin-2-yl)-methanone
(4,6-Dichloro-pyrazolo[1,5-a]pyridin-2-yl)-(7-pyridin-2-yl-3,4-dihydro-1H-isoquinolin-2-yl)-methanone
(4,6-Dichloro-pyrazolo[1,5-a]pyridin-2-yl)-(4-methyl-6,7-dihydro-4H-thieno[3,2-c]pyridin-5-yl)-methanone
(4,6-Dichloro-pyrazolo[1,5-a]pyridin-2-yl)-(4-methyl-6,7-dihydro-4H-furo[3,2-c]pyridin-5-yl)-methanone
(4,6-Dichloro-pyrazolo[1,5-a]pyridin-2-yl)-(1-methyl-3,4-dihydro-1H-cinnolin-2-yl)-methanone

(4,6-Dichloro-pyrazolo[1,5-a]pyridin-2-yl)-(5,6-dihydro-8H-imidazo[1,2-a]pyrazin-7-yl)-methanone
(4,6-Dichloro-pyrazolo[1,5-a]pyridin-2-yl)-(5,6-dihydro-8H-[1,2,4]triazolo[4,3-a]pyrazin-7-yl)-methanone
(4,6-Dichloro-pyrazolo[1,5-a]pyridin-2-yl)-(1-methyl-3,4-dihydro-1H-pyrrolo[1,2-a]pyrazin-2-yl)-methanone
4,6-Dichloro-pyrazolo[1,5-a]pyridine-2-carboxylic acid methyl-(1-phenyl-ethyl)-amide
4,6-Dichloro-pyrazolo[1,5-a]pyridine-2-carboxylic acid benzyl-methyl-amide
4,6-Dichloro-pyrazolo[1,5-a]pyridine-2-carboxylic acid (2-methyl-2-phenyl-propyl)-amide
4,6-Dichloro-pyrazolo[1,5-a]pyridine-2-carboxylic acid adamantyl-1 amide
(4,6-Dichloro-pyrazolo[1,5-a]pyridin-2-yl)-((R)-1-methyl-3,4-dihydro-1H-isoquinolin-2-yl)-methanone
(4,6-Dibromo-pyrazolo[1,5-a]pyridin-2-yl)-(3,4-dihydro-1H-isoquinolin-2-yl)-methanone
(4,6-Dibromo-pyrazolo[1,5-a]pyridin-2-yl)-(1-methyl-3,4-dihydro-1H-isoquinolin-2-yl)-methanone
(4,6-Dibromo-pyrazolo[1,5-a]pyridin-2-yl)-(7-fluoro-1-methyl-3,4-dihydro-1H-isoquinolin-2-yl)-methanone
(3-Bromo-7,8-dihydro-5H-1,6-naphthyridin-6-yl)-(4,6-dibromo-pyrazolo[1,5-a]pyridin-2-yl)-methanone
(4,6-Dibromo-pyrazolo[1,5-a]pyridin-2-yl)-(7-pyridin-2-yl-3,4-dihydro-1H-isoquinolin-2-yl)-methanone
(4,6-Dibromo-pyrazolo[1,5-a]pyridin-2-yl)-(4-methyl-6,7-dihydro-4H-thieno[3,2-c]pyridin-5-yl)-methanone
(4,6-Dibromo-pyrazolo[1,5-a]pyridin-2-yl)-(4-methyl-6,7-dihydro-4H-furo[3,2-c]pyridin-5-yl)-methanone
(4,6-Dibromo-pyrazolo[1,5-a]pyridin-2-yl)-(1-methyl-3,4-dihydro-1H-cinnolin-2-yl)-methanone
(4,6-Dibromo-pyrazolo[1,5-a]pyridin-2-yl)-(5,6-dihydro-8H-imidazo[1,2-a]pyrazin-7-yl)-methanone
(4,6-Dibromo-pyrazolo[1,5-a]pyridin-2-yl)-(5,6-dihydro-8H-[1,2,4]triazolo[4,3-a]pyrazin-7-yl)-methanone
(4,6-Dibromo-pyrazolo[1,5-a]pyridin-2-yl)-(1-methyl-3,4-dihydro-1H-pyrrolo[1,2-a]pyrazin-2-yl)-methanone
4,6-Dibromo-pyrazolo[1,5-a]pyridine-2-carboxylic acid methyl-(1-phenyl-ethyl)-amide
4,6-Dibromo-pyrazolo[1,5-a]pyridine-2-carboxylic acid benzyl-methyl-amide
4,6-Dibromo-pyrazolo[1,5-a]pyridine-2-carboxylic acid (2-methyl-2-phenyl-propyl)-amide
4,6-Dibromo-pyrazolo[1,5-a]pyridine-2-carboxylic acid adamantyl-1 amide
(4,6-Dibromo-pyrazolo[1,5-a]pyridin-2-yl)-((R)-1-methyl-3,4-dihydro-1H-isoquinolin-2-yl)-methanone
(6-Bromo-4-methoxy-pyrazolo[1,5-a]pyridin-2-yl)-(3,4-dihydro-1H-isoquinolin-2-yl)-methanone
(6-Bromo-4-methoxy-pyrazolo[1,5-a]pyridin-2-yl)-(1-methyl-3,4-dihydro-1H-isoquinolin-2-yl)-methanone
(6-Bromo-4-methoxy-pyrazolo[1,5-a]pyridin-2-yl)-(7-fluoro-1-methyl-3,4-dihydro-1H-isoquinolin-2-yl)-methanone
(3-Bromo-7,8-dihydro-5H-1,6-naphthyridin-6-yl)-(6-bromo-4-methoxy-pyrazolo[1,5-a]pyridin-2-yl)-methanone
(6-Bromo-4-methoxy-pyrazolo[1,5-a]pyridin-2-yl)-(7-pyridin-2-yl-3,4-dihydro-1H-isoquinolin-2-yl)-methanone
(6-Bromo-4-methoxy-pyrazolo[1,5-a]pyridin-2-yl)-(4-methyl-6,7-dihydro-4H-thieno[3,2-c]pyridin-5-yl)-methanone
(6-Bromo-4-methoxy-pyrazolo[1,5-a]pyridin-2-yl)-(4-methyl-6,7-dihydro-4H-furo[3,2-c]pyridin-5-yl)-methanone
(6-Bromo-4-methoxy-pyrazolo[1,5-a]pyridin-2-yl)-(1-methyl-3,4-dihydro-1H-cinnolin-2-yl)-methanone
(6-Bromo-4-methoxy-pyrazolo[1,5-a]pyridin-2-yl)-(5,6-dihydro-8H-imidazo[1,2-a]pyrazin-7-yl)-methanone
(6-Bromo-4-methoxy-pyrazolo[1,5-a]pyridin-2-yl)-(5,6-dihydro-8H-[1,2,4]triazolo[4,3-a]pyrazin-7-yl)-methanone
(6-Bromo-4-methoxy-pyrazolo[1,5-a]pyridin-2-yl)-(1-methyl-3,4-dihydro-1H-pyrrolo[1,2-a]pyrazin-2-yl)-methanone
6-Bromo-4-methoxy-pyrazolo[1,5-a]pyridine-2-carboxylic acid methyl-(1-phenyl-ethyl)-amide
6-Bromo-4-methoxy-pyrazolo[1,5-a]pyridine-2-carboxylic acid benzyl-methyl-amide
6-Bromo-4-methoxy-pyrazolo[1,5-a]pyridine-2-carboxylic acid (2-methyl-2-phenylpropyl)-amide
6-Bromo-4-methoxy-pyrazolo[1,5-a]pyridine-2-carboxylic acid adamantyl-1 amide
(6-Bromo-4-methoxy-pyrazolo[1,5-a]pyridin-2-yl)-((R)-1-methyl-3,4-dihydro-1H-isoquinolin-2-yl)-methanone
(6-Chloro-4-methoxy-pyrazolo[1,5-a]pyridin-2-yl)-(3,4-dihydro-1H-isoquinolin-2-yl)-methanone
(6-Chloro-4-methoxy-pyrazolo[1,5-a]pyridin-2-yl)-(1-methyl-3,4-dihydro-1H-isoquinolin-2-yl)-methanone
(6-Chloro-4-methoxy-pyrazolo[1,5-a]pyridin-2-yl)-(7-fluoro-1-methyl-3,4-dihydro-1H-isoquinolin-2-yl)-methanone
(3-Bromo-7,8-dihydro-5H-1,6-naphthyridin-6-yl)-(6-chloro-4-methoxy-pyrazolo[1,5-a]pyridin-2-yl)-methanone
(6-Chloro-4-methoxy-pyrazolo[1,5-a]pyridin-2-yl)-(7-pyridin-2-yl-3,4-dihydro-1H-isoquinolin-2-yl)-methanone
(6-Chloro-4-methoxy-pyrazolo[1,5-a]pyridin-2-yl)-(4-methyl-6,7-dihydro-4H-thieno[3,2-c]pyridin-5-yl)-methanone
(6-Chloro-4-methoxy-pyrazolo[1,5-a]pyridin-2-yl)-(4-methyl-6,7-dihydro-4H-furo[3,2-c]pyridin-5-yl)-methanone
(6-Chloro-4-methoxy-pyrazolo[1,5-a]pyridin-2-yl)-(1-methyl-3,4-dihydro-1H-cinnolin-2-yl)-methanone
(6-Chloro-4-methoxy-pyrazolo[1,5-a]pyridin-2-yl)-(5,6-dihydro-8H-imidazo[1,2-a]pyrazin-7-yl)-methanone
(6-Chloro-4-methoxy-pyrazolo[1,5-a]pyridin-2-yl)-(5,6-dihydro-8H-[1,2,4]triazolo[4,3-a]pyrazin-7-yl)-methanone
(6-Chloro-4-methoxy-pyrazolo[1,5-a]pyridin-2-yl)-(1-methyl-3,4-dihydro-1H-pyrrolo[1,2-a]pyrazin-2-yl)-methanone
6-Chloro-4-methoxy-pyrazolo[1,5-a]pyridine-2-carboxylic acid methyl-(1-phenyl-ethyl)-amide
6-Chloro-4-methoxy-pyrazolo[1,5-a]pyridine-2-carboxylic acid benzyl-methyl-amide
6-Chloro-4-methoxy-pyrazolo[1,5-a]pyridine-2-carboxylic acid (2-methyl-2-phenylpropyl)-amide
6-Chloro-4-methoxy-pyrazolo[1,5-a]pyridine-2-carboxylic acid adamantyl-1 amide
(6-Chloro-4-methoxy-pyrazolo[1,5-a]pyridin-2-yl)-((R)-1-methyl-3,4-dihydro-1H-isoquinolin-2-yl)-methanone

(6-Chloro-4-nitro-pyrazolo[1,5-a]pyridin-2-yl)-(3,4-dihydro-1H-isoquinolin-2-yl)-methanone
(6-Chloro-4-nitro-pyrazolo[1,5-a]pyridin-2-yl)-(1-methyl-3,4-dihydro-1H-isoquinolin-2-yl)-methanone
(6-Chloro-4-nitro-pyrazolo[1,5-a]pyridin-2-yl)-(7-fluoro-1-methyl-3,4-dihydro-1H-isoquinolin-2-yl)-methanone
(3-Bromo-7,8-dihydro-5H-1,6-naphthyridin-6-yl)-(6-chloro-4-nitro-pyrazolo[1,5-a]pyridin-2-yl)-methanone
(6-Chloro-4-nitro-pyrazolo[1,5-a]pyridin-2-yl)-(7-pyridin-2-yl-3,4-dihydro-1H-isoquinolin-2-yl)-methanone
(6-Chloro-4-nitro-pyrazolo[1,5-a]pyridin-2-yl)-(4-methyl-6,7-dihydro-4H-thieno[3,2-c]pyridin-5-yl)-methanone
(6-Chloro-4-nitro-pyrazolo[1,5-a]pyridin-2-yl)-(4-methyl-6,7-dihydro-4H-furo[3,2-c]pyridin-5-yl)-methanone
(6-Chloro-4-nitro-pyrazolo[1,5-a]pyridin-2-yl)-(1-methyl-3,4-dihydro-1H-cinnolin-2-yl)-methanone
(6-Chloro-4-nitro-pyrazolo[1,5-a]pyridin-2-yl)-(5,6-dihydro-8H-imidazo[1,2-a]pyrazin-7-yl)-methanone
(6-Chloro-4-nitro-pyrazolo[1,5-a]pyridin-2-yl)-(5,6-dihydro-8H-[1,2,4]triazolo[4,3-a]pyrazin-7-yl)-methanone
(6-Chloro-4-nitro-pyrazolo[1,5-a]pyridin-2-yl)-(1-methyl-3,4-dihydro-1H-pyrrolo[1,2-a]pyrazin-2-yl)-methanone
6-Chloro-4-nitro-pyrazolo[1,5-a]pyridine-2-carboxylic acid methyl-(1-phenyl-ethyl)-amide
6-Chloro-4-nitro-pyrazolo[1,5-a]pyridine-2-carboxylic acid benzyl-methyl-amide
6-Chloro-4-nitro-pyrazolo[1,5-a]pyridine-2-carboxylic acid (2-methyl-2-phenyl-propyl)-amide
6-Chloro-4-nitro-pyrazolo[1,5-a]pyridine-2-carboxylic acid adamantyl-1 amide
(6-Chloro-4-nitro-pyrazolo[1,5-a]pyridin-2-yl)-((R)-1-methyl-3,4-dihydro-1H-isoquinolin-2-yl)-methanone
(6-Bromo-4-nitro-pyrazolo[1,5-a]pyridin-2-yl)-(3,4-dihydro-1H-isoquinolin-2-yl)-methanone
(6-Bromo-4-nitro-pyrazolo[1,5-a]pyridin-2-yl)-(1-methyl-3,4-dihydro-1H-isoquinolin-2-yl)-methanone
(6-bromo-4-nitro-pyrazolo[1,5-a]pyridin-2-yl)-(7-fluoro-1-methyl-3,4-dihydro-1H-isoquinolin-2-yl)-methanone
(3-Bromo-7,8-dihydro-5H-1,6-naphthyridin-6-yl)-(6-bromo-4-nitro-pyrazolo[1,5-a]pyridin-2-yl)-methanone
(6-Bromo-4-nitro-pyrazolo[1,5-a]pyridin-2-yl)-(7-pyridin-2-yl-3,4-dihydro-1H-isoquinolin-2-yl)-methanone
(6-bromo-4-nitro-pyrazolo[1,5-a]pyridin-2-yl)-(4-methyl-6,7-dihydro-4H-thieno[3,2-c]pyridin-5-yl)-methanone
(6-Bromo-4-nitro-pyrazolo[1,5-a]pyridin-2-yl)-(4-methyl-6,7-dihydro-4H-furo[3,2-c]pyridin-5-yl)-methanone
(6-Bromo-4-nitro-pyrazolo[1,5-a]pyridin-2-yl)-(1-methyl-3,4-dihydro-1H-cinnolin-2-yl)-methanone
(6-Bromo-4-nitro-pyrazolo[1,5-a]pyridin-2-yl)-(5,6-dihydro-8H-imidazo[1,2-a]pyrazin-7-yl)-methanone
(6-Bromo-4-nitro-pyrazolo[1,5-a]pyridin-2-yl)-(5,6-dihydro-8H-[1,2,4]triazolo[4,3-a]pyrazin-7-yl)-methanone
(6-Bromo-4-nitro-pyrazolo[1,5-a]pyridin-2-yl)-(1-methyl-3,4-dihydro-1H-pyrrolo[1,2-a]pyrazin-2-yl)-methanone
6-Bromo-4-nitro-pyrazolo[1,5-a]pyridine-2-carboxylic acid methyl-(1-phenyl-ethyl)-amide
6-Bromo-4-nitro-pyrazolo[1,5-a]pyridine-2-carboxylic acid benzyl-methyl-amide
6-Bromo-4-nitro-pyrazolo[1,5-a]pyridine-2-carboxylic acid (2-methyl-2-phenyl-propyl)-amide
6-Bromo-4-nitro-pyrazolo[1,5-a]pyridine-2-carboxylic acid adamantyl-1 amide
(6-Bromo-4-nitro-pyrazolo[1,5-a]pyridin-2-yl)-((R)-1-methyl-3,4-dihydro-1H-isoquinolin-2-yl)-methanone

The invention also relates to compounds of the Formula I which are marked by radioactive atoms. Typical compounds include those where one or more hydrogens are substituted by tritium, where one or more C¹² are substituted by C¹⁴, where one or more fluor atoms are substituted by F¹⁸ or other isotopes. These can be used for the treatment of diseases (e.g. cancer) but also for diagnostic purposes. The radioactive atoms exchanged in the molecule are often isotopes of carbon, hydrogen, halogen, sulphur or phosphorus.

The invention in general relates to the use of a metabotropic glutamate receptor modulator (and in particular of a mGluR5 modulator) for the preparation of a medicament and for the treatment of various diseases as mentioned hereunder in a mammal, including humans.

Moreover, the invention relates to the use of a compound of Formula **I** as defined above or an optical isomer, pharmaceutically acceptable salt, hydrate, solvate or polymorph thereof for the preparation of a medicament and for the treatment of a mammal, including humans.

Further, the invention relates to the use of a compound for the preparation of a medicament for treating or preventing a condition or disease associated with abnormal glutamate neurotransmission. Such a use includes the use of a compound for the preparation of a medicament for the prevention and/or treatment of a condition or disease in an animal including a human being which condition or disease is affected or facilitated by the negative modulatory effect of mGluR5 modulators.

The invention is dealing with the use of a mGluR5 modulator and in particular a compound according to Formula **I**, for the preparation of a medicament, including for the conditions or diseases selected from those mentioned earlier in the description.

The invention also relates to the use of a mGluR5 modulator, in particular a compound according to Formula **I** wherein the condition associated with abnormal glutamate neurotransmission is selected from those mentioned earlier in the description.

Further, the invention relates to the use of a compound wherein the condition associated with abnormal glutamate neurotransmission is selected from: neuropathic pain, diabetic neuropathic pain (DNP), cancer pain, pain related to rheumathic arthritis, inflammatory pain, L-dopa-induced and tardive dyskinesias, Parkinson's disease, anxiety disorders, Huntington's chorea, epilepsy, Alzheimer's disease, positive and negative symptoms of schizophrenia, cognitive impairment, reflux, migräne or for cognitive enhancement and/or neuroprotection.

Further, the invention relates to a pharmaceutical composition comprising as active ingredient at least one compound of Formula **I** as defined above or an optical isomer, pharmaceuctically acceptable salt, hydrate, solvate or polymorph thereof, together with one or more pharmaceutically acceptable excipients.

The invention also relates to a process for the synthesis or preparation of a compound of Formula **I** 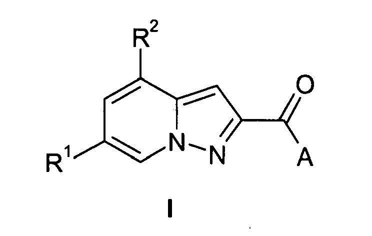 and optical isomers, pharmaceutically acceptable salts, hydrates, solvates, and polymorphs thereof, wherein a compound of Formula **II** 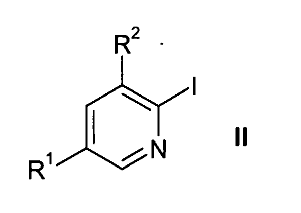 is dissolved in THF and treated with *iso*-propylmagnesium chloride solution (in THF), followed by reaction with dimethyl foramide and hydrochloric acid to yield a compound of Formula **III** 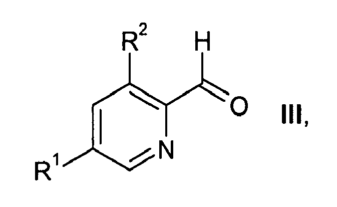 which then is reacted with a compound of Formula **IV** 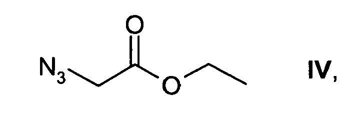 which compound **IV** may be obtained by treatment of a compound of formula **VI** with sodium azide 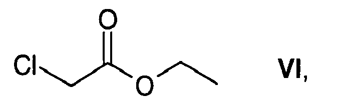 to yield a compound of Formula **V** 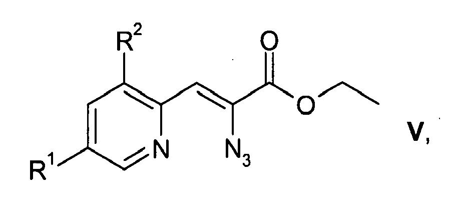 which compound **V** is heated to yield a pyrazolopyridine compound of Formula **VII** 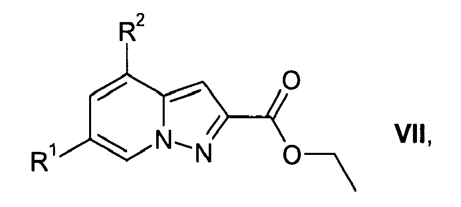 which is hydrolyzed under basic conditions to yield a compound of Formula **VIII** 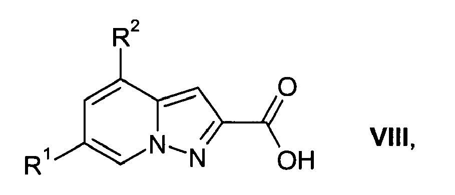 which then is treated with an amine of Formula **IX**

A-H IX

(e.g., in the presence of a condensing agent), to yield a compound of Formula **I**, which is converted, if desired, to a pharmaceutically acceptable salt, hydrate, solvate, or polymorph.

The invention also relates to a process for the synthesis or preparation of a compound of Formula **I** 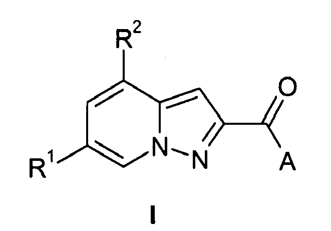 and optical isomers, pharmaceutically acceptable salts, hydrates, solvates, and polymorphs thereof, wherein a compound of Formula **X** 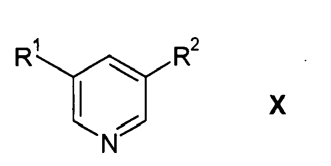 is treated with hydroxylamine-O-sulfonic acid in the presence of potassium carbonate to form an intermediate N-iminopyridinium species **XI** 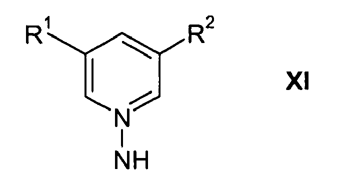 which is treated with hydriodic acid to yield a 1-aminopyridinium iodide **XII** 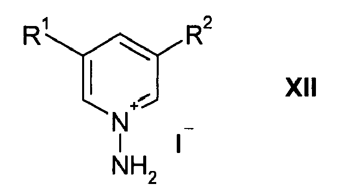 which is treated with dimethyl acetylenedicarboxylate **XIII** 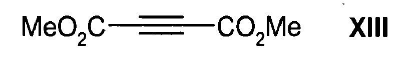 to yield a mixture of compounds **XIV** and **XV** 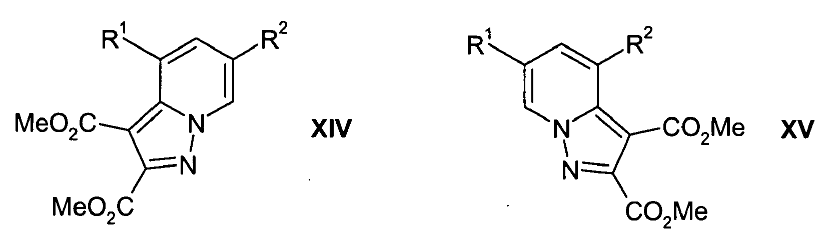 which compounds are separated via column chromatography followed by heating with concentrated sulfuric acid to yield a compound of Formula **VIII** 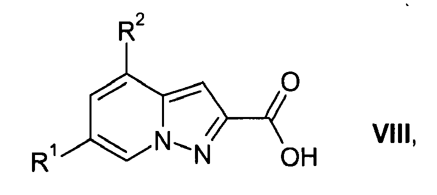 which then is treated with an amine of Formula **IX**

A-H IX

(e.g., in the presence of a condensing agent), to yield a compound of Formula **I**, which is converted, if desired, to a pharmaceutically acceptable salt, hydrate, solvate, or polymorph.

Moreover, the mGluR modulators as described above are expected to have a high activity when administered in combination with other substances exhibiting neurological effects via different mechanisms.

The invention also relates to a pharmaceutical composition comprising at least two different active ingredients, containing at least one compound of Formula **I** as defined above, and furthermore containing at least one NMDA-antagonist, together with one or more pharmaceutically acceptable excipients. These compositions can be used for the treatment of CNS-related diseases, cognitive enhancement and for neuro-protection.

Simultaneous administration of Group I mGluR modulators and NMDA receptor antagonists has also been shown to provide neuroprotection in animal models (see e.g. Zieminska et al. Neurochemistry International, 2006, 66, 301-309; Zieminska et al. Neurochemistry International, 2003, 43, 481-492; Zieminska et al. Neurochemistry International, 2006, 48, 491-497).

With respect to the specific compounds as described above, the combined therapy exhibits a greater neuroprotective effect than monotherapy with either an mGluR modulator or an NMDA receptor antagonist. As particularly active NMDA receptor antagonist, the compound Memantine can be named, which is also known as 1-amino-3,5-dimethyladamantane (see US 4,122,193; US 4,273,774; and US 5,061,703).

Furthermore, the compound Neramexane, which also is known as 1-amino-1,3,3,5,5-pentamethylcyclohexane, is a further active NMDA receptor antagonist and is disclosed in detail in US 6,034,134 and US 6,071,966. Memantine and Neramexane are systemically-active noncompetitive NMDA receptor antagonists having moderate affinity for the receptor. They exhibit strong voltage dependent characteristics and fast blocking/unblocking kinetics (see e.g. Görtelmeyer et al., Arzneim-Forsch/Drug Res., 1992, 42:904-913; Winblad et al., Int. J. Geriat. Psychiatry, 1999, 14:135-146; Rogawski, Amino Acids, 2000, 19: 133-49; Danysz et al., Curr. Pharm. Des., 2002, 8:835-43; Jirgensons et. al. Eur. J. Med. Chem., 2000, 35: 555-565).

The combination of NMDA antagonists with mGluR5 modulators can be realized in a single pharmaceutical composition (as principally described in the prior art) comprising a mGluR5 modulator of the present invention and an NMDA receptor antagonist, in one pharmaceutical formulation, or in two separate pharmaceutical compositions or formulations, one comprising a mGluR5 modulator of the present invention and one comprising an NMDA receptor antagonist in a pharmaceutical formulation, to be administered conjointly (simultaneously or sequentially). For the sequential administration to be considered "conjoint", however, the mGluR5 modulator of the present invention and the NMDA receptor antagonist must be administered separated by a time interval that still permits the resultant beneficial effect in a mammal. For example, the mGluR5 modulator of the present invention and the NMDA receptor antagonist must be administered on the same day (e.g., each - once or twice daily), preferably within an hour of each other, and most preferably simultaneously.

This invention also relates to a pharmaceutical composition comprising a combination of a compound of Formula **I** as described above and an NMDA receptor antagonist. Of particular interest is a composition, wherein the NMDA receptor antagonist is selected from Memantine and Neramexane (or a combination thereof) and pharmaceutically acceptable salts, polymorphs, hydrates and solvates thereof.

The invention also relates to a pharmaceutical composition comprising at least two different active ingredients, containing at least one compound of Formula **I** as defined above, and furthermore containing at least one of L-DOPA, another dopaminomimetics (in particular an antiparkinsonian dopaminomimetics e.g. bromocriptine, cabergolin, ropinirole, pramiperole, pergolide, rotigotine), and a neuroleptic (in particular a classical neuroleptic, e.g. haloperidol, perphenazin, chlorpromazine, metoclopramide).

These combination products can e.g. be used for the treatment of CNS-related disorders and diseases. Because of the antidyskinetic effect of the compounds of Formula **I**, drug induced dyskinesias, neuroleptic-induced dyskinesias, haloperidol-induced dyskinesias, dopaminomi-metic-induced dyskinesias can be treated in addition to the conditions which are typically treated with L-Dopa, dopaminomimetics or neuroleptics.

The invention also relates to a method of providing neuroprotection to a living animal, including a human, comprising the step of administering to a living animal, including a human, a therapeutically effective amount of a composition as described.

This invention is also dealing with the compounds of Formula **I** for the use as a medicament. Furthermore, the invention relates to the use of a compound of Formula **I** for the manufacture of a medicament for the treatment of the diseases and conditions mentioned above.

Furthermore, the invention relates to the use of a composition as described for the manufacture of a medicament to provide neuroprotection in an animal, including a human.

Furthermore, the invention relates to the use of a compound of Formula **I** in the manufacture of a medicament for treatment of a condition associated with abnormal glutamate neurotransmission or in which modulation of mGluR5 receptors results in therapeutic benefit. The disorders which can be treated have already been described above. Such conditions and indications include:
a) For mGluR5 modulators: chronic pain, neuropathic pain, diabetic neuropathic pain (DNP), cancer pain, pain related to rheumathic arthritis, inflammatory pain, L-dopa-induced dyskinesias, dopaminomimetic-induced dyskinesias, L-dopa-induced dyskinesias in Parkinson's disease therapy, dopaminomimetic-induced dyskinesias in Parkinson's disease therapy, tardive dyskinesias, Parkinson's disease, anxiety disorders, panic disorders, anxiety and panic disorders, social anxiety disorder (SAD), generalized anxiety disorder, substance-induced anxiety disorder, eating disorders, obesity, binge eating disorders, Huntington's chorea, epilepsy, Alzheimer's disease, positive and negative symptoms of schizophrenia, cognitive impairment, functional gastrointestinal disorders, gastroesophageal reflux disease (GERD), migraine, irritable bowel syndrome (IBS), or for cognitive enhancement and/or neuroprotection.
b) For negative modulation of mGluR5: chronic pain, neuropathic pain, diabetic neuropathic pain (DNP), cancer pain, pain related to rheumathic arthritis, inflammatory pain, L-dopa-induced dyskinesias, dopaminomimetic-induced dyskinesias, L-dopa-induced dyskinesias in Parkinson's disease therapy, dopaminomimetic-induced dyskinesias in Parkinson's disease therapy, tardive dyskinesias, Parkinson's disease, anxiety disorders, panic disorders, anxiety and panic disorders, social anxiety disorder (SAD), generalized anxiety disorder, substance-induced anxiety disorder, eating disorders, obesity, binge eating disorders, migraine, irritable bowel syndrome (IBS), functional gastrointestinal disorders, gastroesophageal reflux disease (GERD), Huntington's chorea and/or epilepsy.
c) For positive modulation of mGluR5: Alzheimer's disease, positive and/or negative symptoms of schizophrenia, cognitive impairment, or for cognitive enhancement and/or neuroprotection.

The mGluR5 negative modulators in general and in particular the compounds of Formula **I** according to the invention can be used for the treatment of binge eating disorders.

### DETAILED DESCRIPTION OF THE INVENTION

For the purpose of the present invention, in the compounds of Formula **I** the carbon atom content of various hydrocarbon-containing moieties is indicated by a prefix designating the minimum and maximum number of carbon atoms in the moiety, i.e., the prefix Cᵢ₋ⱼ indicates a moiety of the integer "i" to the integer "j" carbon atoms, inclusive. Thus, for example, (C₁₋₃)alkyl refers to alkyl of one to three carbon atoms (i.e. 1, 2 or 3 carbon atoms), inclusive, (methyl, ethyl, propyl, and isopropyl), straight and branched forms thereof, (C₁₋₆) for instance refers to a radical of one to six carbon atoms (i.e. 1, 2, 3, 4, 5 or 6 carbon atoms).

As used herein, the following definitions are applicable unless otherwise described, the term "C₁₋₆alkyl" represents straight or branched chain alkyl groups which may be optionally substituted by one or more substituents selected from halogen, trifluoromethyl, C₁₋₆alkoxy, amino, hydroxy, C₁₋₆alkylamino, and di-(C₁₋₆alkyl)amino: Examples of such alkyl groups include methyl, ethyl, n-propyl, 2-propyl, n-butyl, tert-butyl, -CF₃, -C₂F₅, - CBr₃ and -CCl₃.

The term "C₂₋₆alkenyl" represents straight or branched chain alkenyl groups. The term "C₁₋₆alkoxy" represents straight or branched chain -O-C₁₋₆alkyl groups which may be optionally substituted by one or more substituents selected from halogen, trifluoromethyl, amino, hydroxy, C₁₋₆alkylamino and di-(C₁₋₆alkyl)amino. Examples of such alkoxy groups include methoxy, ethoxy, n-propoxy, i-propoxy, -OCF₃ and -OC₂F₅.

The term "cycloC₃₋₁₂alkyl" represents monocyclic or bicyclic, or tricyclic alkyl groups, including cyclopropyl, cyclobutyl, cyclopentyl, cyclohexyl, bicyclo[2.2.1]heptyl and adamantanyl, which may be optionally substituted by one or more substituents, which may be the same or different, selected independently from halogen, trifluoromethyl, trifluoromethoxy, C₁₋₆alkyl, C₂₋₆alkenyl, C₁₋₆alkoxy, amino, hydroxy, nitro, cyano, cyanomethyl, C₁₋₆alkoxycarbonyl, C₁₋₆alkylamino, and di-(C₁₋₆alkyl)amino, C₁₋₆alkylcarbonylamino, and C₁₋₆alkylenedioxy.

The term "aryl" represents phenyl or naphthyl, wherein the phenyl or naphthyl group is optionally substituted by one or more substituents, which may be the same or different, selected independently from halogen, trifluoromethyl, trifluoromethoxy, C₁₋₆alkyl, hydroxyC₁₋₆alkyl C₂₋₆alkenyl, C₁₋₆alkoxy, amino, hydroxy, nitro, cyano, cyanomethyl, C₁₋₆alkoxycarbonyl, C₁₋₆alkylcarbonyloxy, C₁₋₆alkylamino, di-(C₁₋₆alkyl)amino, C₁₋₆alkylcarbonylamino, aminocarbonyl, N-C₁₋₆alkylaminocarbonyl, di-N,N-C₁₋₆alkylaminocarbonyl, pyrrolidinyl, piperidinyl, morpholinyl, and piperazinyl, cycloC₃₋₁₂alkyl or optionally C₁₋₆alkylenedioxy.

The term "heteroaryl" represents an aromatic 5-6 membered ring containing from one to four heteroatoms selected from oxygen, sulfur and nitrogen, or a bicyclic group comprising a 5-6 membered ring containing from one to four heteroatoms selected from oxygen, sulfur and nitrogen fused with a benzene ring or a 5-6 membered ring containing from one to four heteroatoms selected from oxygen, sulfur and nitrogen, wherein the heteroaryl group may be optionally substituted by one or more substituents, which may be the same or different, selected independently from halogen, trifluoromethyl, trifluoromethoxy, C₁₋₆alkyl, hydroxyC₁₋₆alkyl, C₂₋₆alkenyl, C₁₋₆alkoxy, amino, hydroxy, nitro, cyano, C₁₋₆alkoxycarbonyl, C₁₋₆alkoxycarbonyloxy, C₁₋₆alkylamino, and di-(C₁₋₆alkyl)amino, C₁₋₆alkylcarbonylamino, aminocarbonyl, N-C₁₋₆alkylaminocarbonyl, di-N,N-C₁₋₆alkylaminocarbonyl, pyrrolidinyl, piperidinyl, morpholinyl, cycloC₃₋₁₂alkyl, C₁₋₆alkylenedioxy and aryl. Representative heteroaryl groups include furyl, thienyl, pyrrolyl, oxazolyl, isoxazolyl, isothiazolyl, oxadiazolyl, pyrazolyl, triazolyl, thiadiazolyl, thiazolyl, imidazolyl, oxadiazolyl, tetrazolyl, pyridinyl, pyrimidinyl, pyridazinyl, pyrazinyl, triazinyl, purinyl, pyrazolyl, benzofuryl, benzothienyl, indolyl, indolizinyl, isoindolyl, indolinyl, indazolyl, benzimidazolyl, benzoxazolyl, benzothiazolyl, quinolinyl, quinazolinyl, quinoxalinyl, cinnolinyl, naphthyridinyl, isoquinolinyl, quinolizinyl, phthalazinyl, theridinyl.

The term "heterocyclyl" represents a saturated or unsaturated non-aromatic 3 to 12 membered ring comprising one to four heteroatoms selected from oxygen, sulfur and nitrogen, and a saturated or unsaturated non-aromatic bicyclic ring system having 3 to 12 members comprising one to six heteroatoms selected from oxygen, sulfur and nitrogen, wherein the heterocyclic ring or ring system is optionally substituted by one or more substituents selected independently from a halogen, trifluoromethyl, C₁₋₆alkyl, C₂₋₆alkenyl, C₁₋₆alkoxy, amino, hydroxy, nitro, cyano,C₁₋₆alkoxycarbonyl, C₁₋₆alkylamino, di-C₁₋₆alkylamino, pyrrolidinyl, piperidinyl, morpholinyl, pyridinyl, and aryl; examples of such heterocyclyl groups include piperidinyl, morpholinyl, thiomorpholinyl, imidazolinyl, imidazolidinyl, pyrazolinyl, pyrazolidinyl, pyrrolinyl, pyrrolidinyl, or piperazinyl, wherein the heterocyclic ring or ring system is linked to the group to which it is attached optionally via nitrogen or a carbon atom.

The term "acyl" includes C₁₋₆alkylcarbonyl, cycloC₃₋₁₂alkylcarbonyl, C₂₋₆alkenylcarbonyl, C₂₋₆alkynylcarbonyl, arylcarbonyl, arylC₁₋₆alkylcarbonyl, heteroarylcarbonyl and heterocyclylcarbonyl, wherein the terms alkyl, aryl, heteroaryl, and heterocyclyl are defined as above. Examples are acetyl, propionyl, benzoyl or pivaloyl.

The term "halogen" represents fluorine, chlorine, bromine and iodine.

The compounds of the present invention are usually named according to the IUPAC or CAS nomenclature system. Abbreviations which are well known to one of ordinary skill in the art may be used (e.g. "Ph" for phenyl, "Me" for methyl, "Et" for ethyl, "h" for hour or hours, and "rt" for room temperature).

The term "analog" or "derivative" is used herein in the conventional pharmaceutical sense, to refer to a molecule that structurally resembles a reference molecule, but has been modified in a targeted and controlled manner to replace one or more specific substituents of the referent molecule with an alternate substituent, thereby generating a molecule which is structurally similar to the reference molecule. Synthesis and screening of analogs (e.g., using structural and/or biochemical analysis), to identify slightly modified versions of a known compound which may have improved or biased traits (such as higher potency and/or selectivity at a specific targeted receptor type, greater ability to penetrate blood-brain barriers, fewer side effects, etc.) is a drug design approach that is well known in pharmaceutical chemistry.

In addition, using methods known to those skilled in the art, analogs and derivatives of the compounds of the invention can be created which have improved therapeutic efficacy, i.e., higher potency and/or selectivity at a specific targeted receptor type, either greater or lower ability to penetrate mammalian blood-brain barriers (e.g., either higher or lower blood-brain barrier permeation rate), fewer side effects, etc.

The phrase "pharmaceutically acceptable", as used in connection with compositions of the invention, refers to molecular entities and other ingredients of such compositions that are physiologically tolerable and do not typically produce untoward reactions when administered to a mammal (e.g., human). Preferably, as used herein, the term "pharmaceutically acceptable" means approved by a regulatory agency of the Federal or a state government or listed in the U.S. Pharmacopeia or other generally recognized pharmacopeia for use in mammals, and more particularly in humans.

Compounds of the present invention may be in the form of pharmaceutically acceptable salts. "Pharmaceutically acceptable salts" refers to those salts which possess the biological effectiveness and properties of the parent compound and which are not biologically or otherwise undesirable. The nature of the salt is not critical, provided that it is non-toxic and does not substantially interfere with the desired pharmacological activity.

It will be appreciated by those skilled in the art that compounds of the invention having a chiral center may exist in and be isolated in optically active and racemic forms. Some compounds may exhibit polymorphism. It is to be understood that the present invention encompasses any racemic, optically active, polymorphic, tautomeric, or stereoisomeric form, or mixture thereof, of a compound of the invention, which possesses the useful properties described herein.

All patents, applications, publications, test methods, literature, and other materials cited in this application are hereby incorporated by reference.

The following Schemes 1-3 describe the preparation of compounds of Formula **I** of the present invention. All of the starting materials may be prepared by procedures described in these schemes, by procedures well known to one of ordinary skill in organic chemistry, or may be obtained commercially. All of the final compounds of the present invention may be prepared by procedures described in these charts or by procedures analogous thereto, which would be well known to one of ordinary skill in organic chemistry. All of the variables used in the schemes are as defined below or as in the claims. The compounds containing one or more chiral centers can be prepared as racemates or mixtures of various stereoisomers and then separated. However, they also can be prepared by a special enantioselective synthesis. For several of the chiral compounds, the enantiomers differ in pharmacological activity.

Pyrazolo[1,5-a]pyridine-2-carboxylic acids **5** may be synthesized according to Scheme 1. 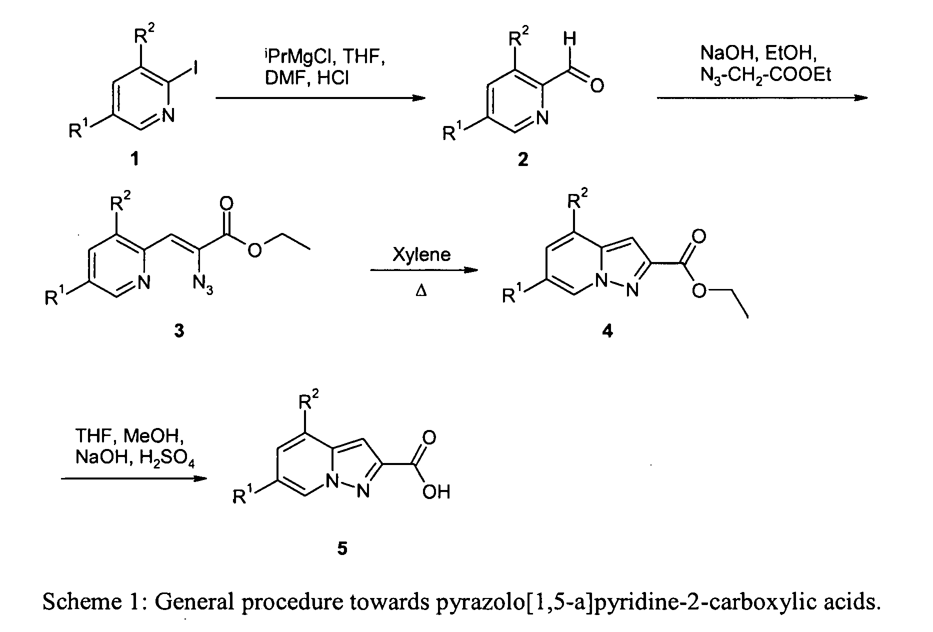 5-Bromo-2-iodopyridine or 5-chloro-2-iodopyridine compound (**1**) is treated with *iso-*propylmagnesium chloride solution (in THF) and reacted with dimethyl formamide to yield 5-Bromopyridine-2-carboxaldehyde or 5**-**Chloropyridine-2-carboxaldehyde compound (**2**). Compound **2** is reacted with ethyl azidoacetate, which is formed by reaction of ethyl chloroacetate with sodium azide, to yield ethyl 2-azido-3-(5-bromopyridin-2-yl)-acrylate or ethyl 2-azido-3-(5-chloropyridin-2-yl)-acrylate compound (**3**). By heating, compound 3 is converted to ethyl 6-bromopyrazolo[1,5a]pyridine carboxylate or ethyl 6-chloropyrazolo[1,5a]pyridine carboxylate compound (**4**). Treatment of **4** with base leads to 6-bromopyrazolo[1,5a]pyridine carboxylic acid or 6-chloropyrazolo[1,5a]pyridine carboxylic acid compound (**5**). Similarly, 6-bromo-4-nitro-pyrazolo[1,5a]pyridine carboxylic acid and 6-chloro-4-nitro-pyrazolo[1,5a]pyridine carboxylic acid are prepared from 2-iodo-3-nitro-5-bromo (or chloro) pyridine accordingly.

Substituted pyrazolo[1,5-a]pyridine-2-carboxylic acids **5** may be prepared according to Scheme 2.

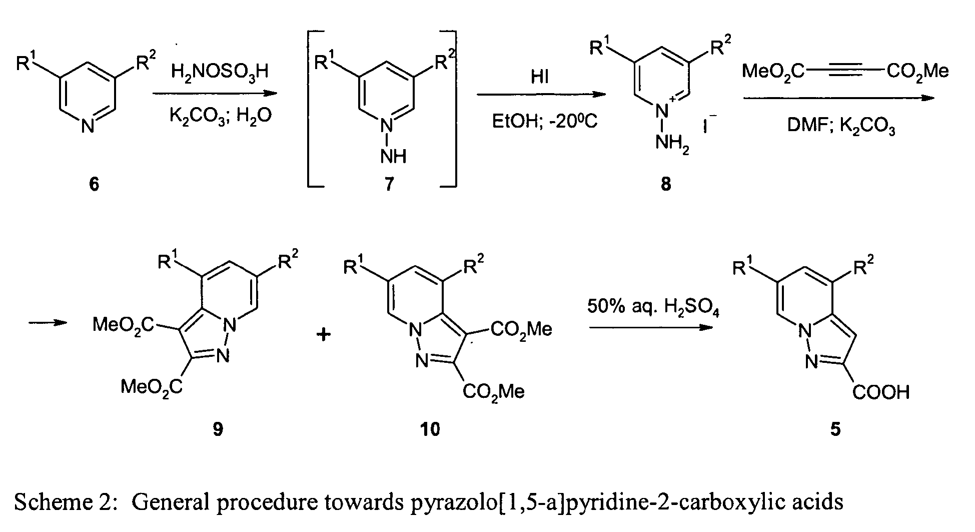

Treatment of 3- substituted or 3,5-disubstituted pyridines (**6**) with hydroxylamine -O-sulfonic acid in the presence of potassium carbonate results in formation of the intermediate N-iminopyridinium species (**7**), which upon addition of hydroiodic acid cleanly affords 1-amino-pyridinium iodides (**8**). 1,3-dipolar cycloaddition of these aminopyridinium salts with dimethyl acetylenedicarboxylate proceeds without regioselectivity to provide *ca.* 1:1 mixture of pyrazolo[1,5-a]pyridine-2,3-dicarboxylic acid dimethyl esters (**9** and **10**). These isomeric compounds are easily separable by flash column chromatography. Heating of the separated desired isomers in aqueous sulfuric acid results in ester hydrolysis with concomitant decarboxylation to give pyrazolo[1,5-a]pyridine-2-carboxylic acids (**5**).

Compound **5** may be converted to a compound of Formula **I** as shown in Scheme 3.

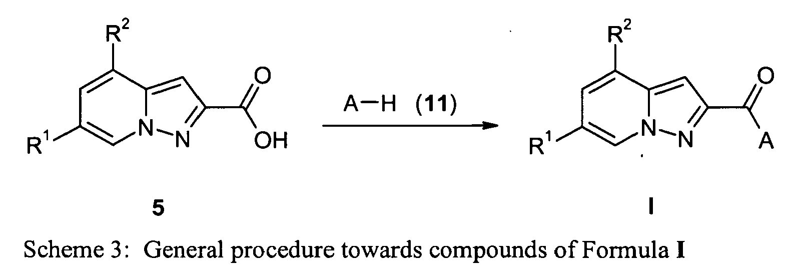

A compound of Formula **I** is prepared from **5** via reaction with an appropriate secondary amine **11** in the presence of a condensation agent, including, for example, O-(benzotriazol-1-yl)-N,N,N',N'-tetramethyluronium tetrafluoroborate ("TBTU") or N-(3-dimethylaminopropyl)-N'-ethylcarbodiimide (EDC).

The amines (**11**) are commercially available or may be prepared according to literature procedures (see, for example, Bull. Soc. Chim. Belg., v.71, 1962; p. 592; US 2002/049223 A1 (2002/04/25); Chem. Ber., 84, 1951, p. 795-798; Bull. Soc. Chim. Fr.5, 4, 1937, p. 1265-1269; Zh. Obshch. Khim., 7, 1937, p. 1999-2004; Chem. Pharm. Bull., EN, 31, 8, 1983, p. 2583-2592; Tetrahedron, 28, 1972, p. 5999-6004; J. Org. Chem., 34, 8, 1969, p. 2478; Pharm. Chem. J. (Engl.Tran.); 5; 5; 1971, p. 260; Khfzan; Khim.Farm.Zh., 5, 5, 1971, p. 13; J. Chem. Soc., 1965, p. 5391-5401; Zh. Organi. Khimii, 22, 1986, p. 2610-2614).

It will be appreciated that in the above transformations it may be necessary or desirable to protect any sensitive groups in the molecule of the compound in question in order to avoid undesirable side reactions.

### EXPERIMENTAL PART

The compounds and their preparation of the present invention will be better understood in connection with the following examples, which are intended as an illustration of and not a limitation upon the scope of the invention.

Hereinafter, "DMF" is defined as N,N-dimethylformamide, "THF" as tetrahydrofurane, "HCl" as hydrochloric acid, "NaOH" as sodium hydroxide, "MeOH" as methanol, "DMSO" as dimethylsulfoxide and "TBTU" as O-(benzotriazol-1-yl)-N,N,N',N'-tetra-methyluronium tetrafluoroborate.

### Preparation 1

### 5-Bromopyridine-2-carboxaldehyde

A 6 L three-necked round bottom flask was equipped with a mechanical stirrer, nitrogen inlet, thermometer and addition funnel. It was charged with 5-bromo-2-iodopyridine (401.11 g; 1.41 mol) and dry THF (2 L). The solution was cooled to - 12 °C and iso-propylmagnesium chloride solution (2M in THF; 760 mL; 1.52 mol) was added dropwise over a period of 30 min keeping the internal temperature below - 5 °C. The resulting suspension was stirred for one hour at this temperature. Then dry DMF (160 mL; 2.07 mol) was added over a 30 min period at an internal temperature of - 5 °C. Stirring was continued at this temperature for 15 min and for one hour at rt. It was cooled again and hydrochloric acid (2N; 1.1 L; 2.2 mol) was added at an internal temperature below 15 °C over a period of 15 min. The mixture was stirred at rt over night. After separation the organic phase was concentrated by evaporation yielding a wet paste. The aqueous phase was extracted with 1.5 L of dichloromethane. The organic extract was used to take up the paste from the THF-phase. It was washed with water, dried over sodium sulfate and filtered. After evaporation of the solvent the crude product was crystallized from ethyl acetate yielding (**1**) as a beige solid (204.36 g; 1.11 mol; 77.9 %) with a purity > 98 % by HPLC.

### Preparation 2

### Ethyl azido acetate

A 10 L three-necked round bottom flask was equipped with a mechanical stirrer, nitrogen inlet and addition funnel. It was charged with ethyl chloroacetate ( 1.69 kg; 13.81 mol) and 2.5 L of ethanol. A solution of sodium azide (997.0 g; 15.34 mol) in 2.5 L of water was added at rt within 30 min. The resulting suspension was heated at reflux for three hours. After cooling the phases were separated and the organic phase was washed with 300 mL of brine, dried over sodium sulfate and filtered.

The filtrate was distilled at 12 Torr to yield 1.21 kg (**2**) (9.39 mol; 68.0 %; bp₁₂ = 58 °C).

### Preparation 3

### Ethyl 2-azido-3-(5-bromopyridin-2-yl)-acrylate

A 4 L four-necked round bottom flask was equipped with a nitrogen inlet, thermometer addition funnel and a stirrer. It was charged with dry ethanol (1.6 L). Finely powdered sodium (20.34 g; 884.7 mmol) was added to give a colourless solution in a strongly exothermic reaction. After cooling to - 20 °C a solution of 5-bromopyridine carboxaldehyde (**1**) (40.06 g; 215.4 mmol) and ethyl azidoacetate (**2**) (115.02 g; 890.8 mmol) in 250 mL of dry THF was added over a period of 90 min keeping the internal temperature below - 18 °C. The resulting orange solution was stirred for 1 h at this temperature and was then allowed to warm to - 4 °C. Then HCl (2N in diethylether; 450 mL; 900 mmol) was added at an internal temperature below 15 °C. After stirring for 15 min at rt the solvents were evaporated. The residue was suspended in 450 mL of dichloromethane and was dropped into 1.7 L of hexane with stirring to give a yellow suspension, which was filtered. After evaporation of the solvent from the filtrate the remaining crude product was purified by flash chromatography on silica gel using a hexane/ ethyl acetate (17+3) eluent yielding crude **3** as a yellow oil. This was crystallized from ethyl acetate to yield 6.45 g (**3**) (21.71 mmol; 10.1 %) as a pale yellow solid.

### Preparation 4

### Ethyl 6-bromo-pyrazolo[1,5-a]pyridine carboxylate

A 1 L round bottom flask was charged with the azide **3** (6.67 g; 22.45 mmol) and 350 mL of toluene. It was heated at reflux for 35 min. After cooling to rt the solvent was evaporated and the residue was dried at 30 °C / 10⁻³ mbar to yield 5.84 g of a red paste. This was purified by flash chromatography using hexane / ethyl acetate (17+3) as eluent to yield 650 mg (**4**) (2.41 mmol; 10.7 %).

### Preparation 5

### 6-Bromo-pyrazolo[1,5-a]pyridine carboxylic acid

A 250 mL round-bottom flask was charged with Ethyl 6-bromo-pyrazolo[1,5-a]pyridine carboxylate (**4**) (2.43 g; 8.70 mmol) and 36 mL each of THF and MeOH. To the resulting orange solution NaOH (2N; 4.45 mL; 8.9 mmol) was added with stirring. After 30 min at rt a suspension formed, which could not be stirred until the addition of 20 mL of water. After stirring over night the mixture was concentrated to about half of its volume. Then sulfuric acid (5.4 N; 1.68 mL; 9.1 mmol) was added. The suspension was stirred over night and filtered with suction. The residue was washed with 200 mL of water and dried in a desiccator over silica gel to yield 2.05 g (**5**) (8.50 mmol; 97.7 %).

The syntheses of 6-chloro-pyrazolo[1,5-a]pyridine carboxylic acid, 6-chloro-4-nitro-pyrazolo[1,5-a]pyridine carboxylic acid and 6-bromo-4-nitro-pyrazolo[1,5-a]pyridine carboxylic acid are performed under similar conditions as described above for 6-bromo-pyrazolo[1,5-a]pyridine carboxylic acid using accordingly substituted 2-iodopyridines as starting materials.

### Preparation 6

### 1-Amino-3-chloropyridinium iodide 4 and 1-amino-3-bromopyridinium iodide.

To a freshly prepared solution of 11.3 g (0.1 mol) of hydroxylamine-O-sulfonic acid in 60 mL of cold water is added 0.1 mol of 3-chloropyridine or 3-bromopyridine. The mixture is heated at 90°C for 20 min, then cooled to room temperature and potassium carbonate (13.8g, 0.1 mol) is added, followed by the removal of water by evaporation *in vacuo.* The residue is treated with 120 mL of ethanol and the insoluble precipitate of potassium sulfate is removed by filtration. The filtrate is treated with 14 mL of 57% hydroiodic acid and stored at -20°C. The solid that separates is collected by filtration to give the title compounds that are used without further purification.

### Preparation 7

### 6-Chloro-pyrazolo[1,5-a]pyridine-2,3-dicarboxylic acid dimethyl ester and 6-bromo-pyrazolo[1,5-a]pyridine-2,3-dicarboxylic acid dimethyl ester.

Dimethyl acetylenedicarboxylate (3.50 g, 24.6 mmol) is added dropwise to a stirred suspension of 1-amino-3-chloropyridinium iodide or 1-amino-3-bromopyridinium iodide (23.5 mmol) and potassium carbonate (4.70g, 47.5 mmol) in 40 mL of DMF at room temperature. The mixture is stirred for 2h while a stream of air is introduced under the liquid level. After filtration and subsequent evaporation of the solvent *in vacuo,* the residue is treated with water (100 mL) and extracted with diethyl ether (3x100 mL). The organic layer was dried over anhydrous sodium sulfate, filtered and evaporated *in vacuo.* Purification of the residue by flash column chromatography (hexane-EtOAc 1:1) provides the title compounds as crystalline solids.

6-Chloro-pyrazolo[1,5-a]pyridine-2,3-dicarboxylic acid dimethyl ester: ¹H NMR spectrum: (CDCl₃, TMS) δ: 3.93 (3H, s); 4.03 (3H, s); 7.43 (1H, dd); 8.14 (1H, dd); 8.55 (1H, dd).

6-Bromo-pyrazolo[1,5-a]pyridine-2,3-dicarboxylic acid dimethyl ester: ¹H NMR spectrum: (CDCl₃, TMS) δ: 3.93 (3H, s); 4.03 (3H, s); 7.52 (1H, dd); 8.08 (1H, dd); 8.66 (1H, dd).

### Preparation 8

### 6-Chloro-pyrazolo[1,5-a]pyridine-2-carboxylic acid and 6-bromo-pyrazolo[1,5-a]pyridine-2-carboxylic acid.

A solution of 6-chloro-pyrazolo[1,5-a]pyridine-2,3-dicarboxylic acid dimethyl ester or 6-bromo-pyrazolo[1,5-a]pyridine-2,3-dicarboxylic acid dimethyl ester (2.4 mmol) in 50% aqueous sulfuric acid (20 mL) is heated at 80°C for 3h. The cooled mixture is treated with 5N NaOH solution followed by acidification with 2N aqueous HCl solution until pH 2-3 is reached. The formed precipitate is collected by filtration to provide sufficiently pure title compounds as white solids.

6-Chloro-pyrazolo[1,5-a]pyridine-2-carboxylic acid: ¹H NMR spectrum: (CDCl₃, TMS) δ: 7.19 (1H, s); 7.30 (1H, dd); 7.55 (1H,d); 8.84 (1H, s).

6-Bromo-pyrazolo[1,5-a]pyridine-2-carboxylic acid: ¹H NMR spectrum: (DMSO-d₆, TMS) δ: 7.11 (1H, s); 7.35 (1H, dd); 7.82 (1H,d); 9.09 (1H, s).

4,6-dibromo-pyrazolo[1,5-a]pyridine-2-carboxylic acid, 4,6-dichloro-pyrazolo[1,5-a]pyridine-2-carboxylic acid, 6-bromo-4-methoxy-pyrazolo[1,5-a]pyridine-2-carboxylic acid and 6-chloro-4-methoxy-pyrazolo[1,5-a]pyridine-2-carboxylic acid are prepared in close analogy to the methods given above.

It will be appreciated that in the above transformations it may be necessary or desirable to protect any sensitive groups in the molecule of the compound in question in order to avoid undesirable side reactions.

In order to prepare the amine component of the pyrazolopyridine compounds of formula (I) the following General Schemes can be used:

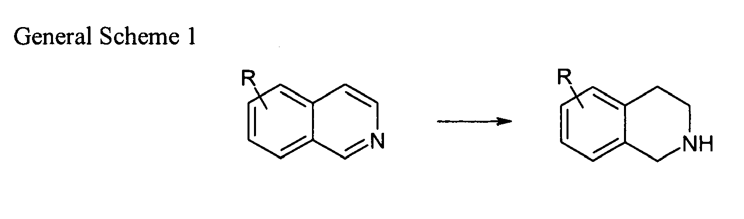

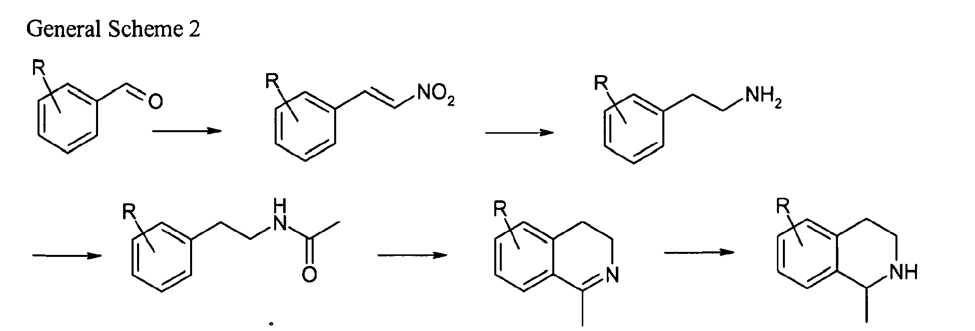

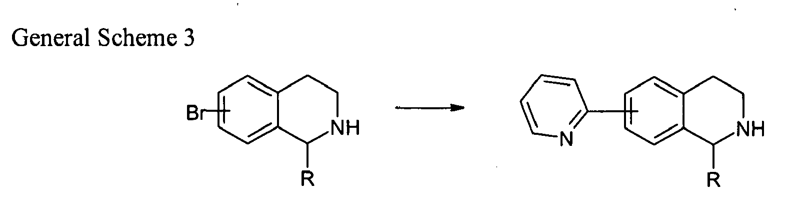

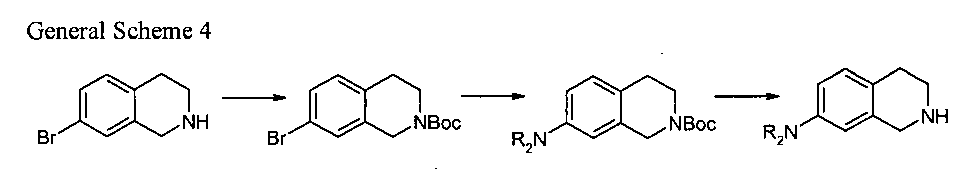

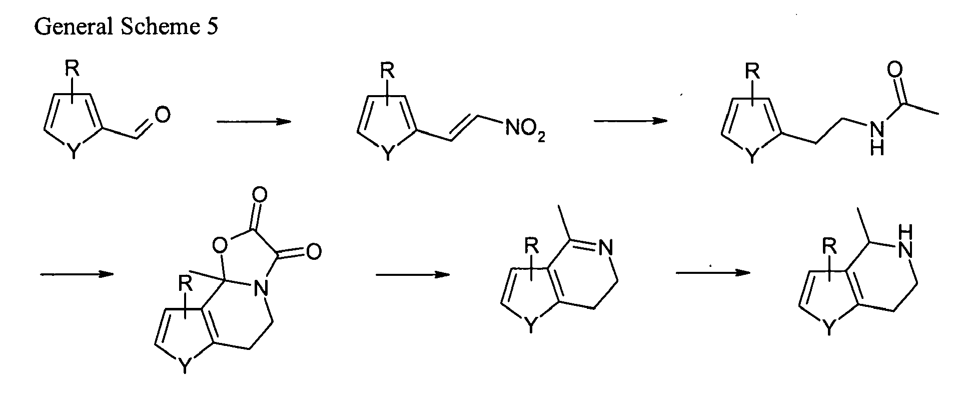

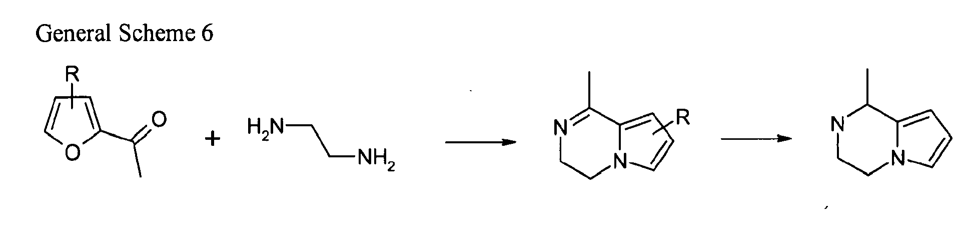

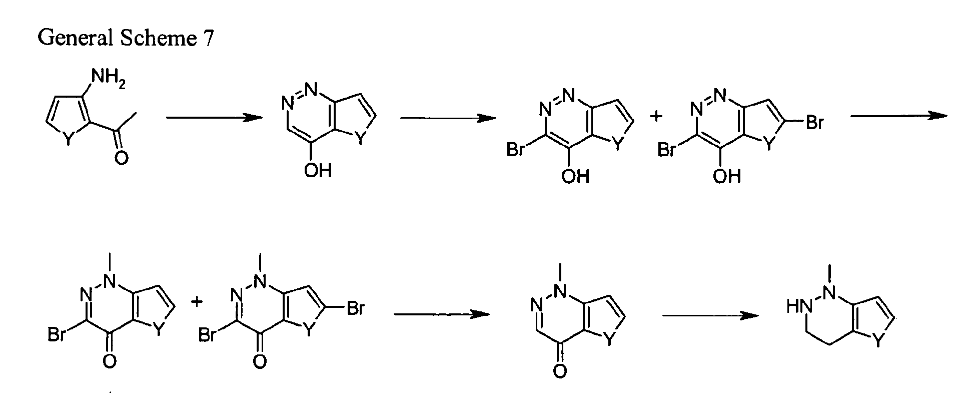

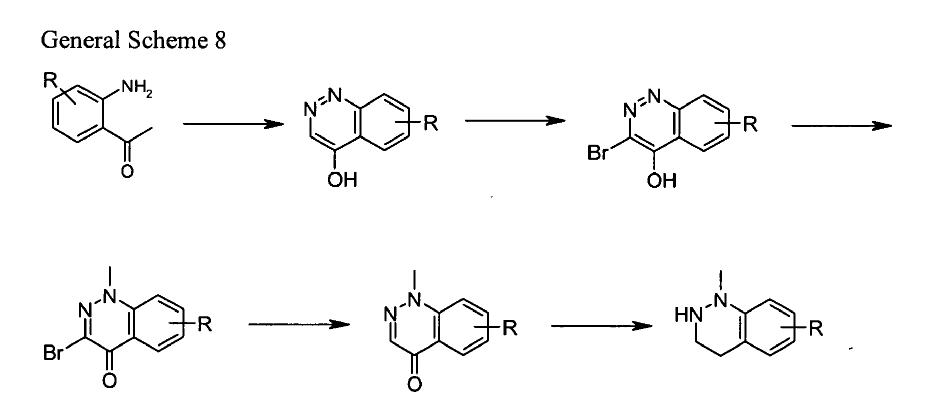

There are several routes for the synthesis of chiral pyrazolopyridines which are outlined in the following:

### A) Enantiomer synthesis by reduction of amine building block with a chiral reducing agent:

Chiral 1-methyl-1,2,3,4-tetrahydroisoquinolines are prepared by reduction of 1-methyl-3,4-dihydroisoquinolines with amino acid-derived chiral reducing agents. Examples of such reducing agents are triacyloxy sodium borohydrides 12 and 13, prepared by mixing chiral N-isobutoxycarbonyl proline with sodium borohydride. Low temperature reduction of 1-methyl-3,4-dihydroisoquinoline 14 with these reducing agents affords 1-methyl-1,2,3,4-tetrahydroisoquinolines with 74% enantiomeric excess. The L-proline-derived reducing agent 13 provides S-isomer of 1-methyl-1,2,3,4-tetrahydroisoquinoline 16, whereas the agent derived from D-proline 12 gives R-isomer of the product 15. The enantiomeric purity is further increased by recrystallization at the stage of amine or after coupling of amine with the corresponding acid.

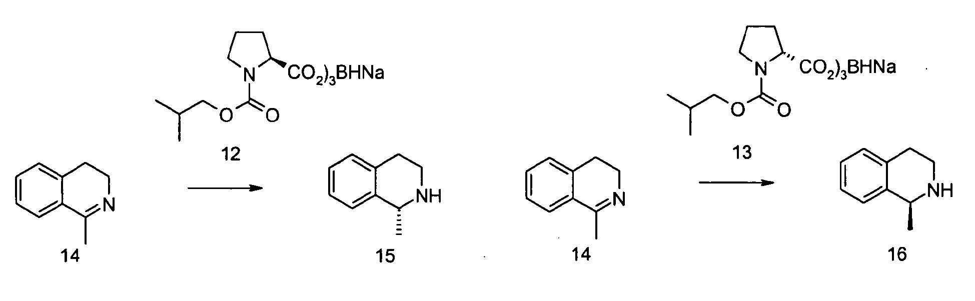

### Preparation of reducing agents 12 and 13

A solution of isobutyl chloroformate (4.4 mL, 34.04 mmol) in acetone is added dropwise to a solution of L-or D-proline (4.0 g, 34.74 mmol) and sodium bicarbonate (10 g, 125 mmol) in water (50 mL). The mixture is stirred overnight. After removal of acetone, the aqueous solution is acidified to pH 2 with hydrochloric acid while maintaining the temperature below 20 °C, and then extracted with ethyl acetate (3 x 25 mL). The combined organic phase is dried over anhydrous magnesium sulfate and evaporated in vacuo to give (S)-N-isobutoxycarbonylproline (from L-proline) or (R)-N-isobutoxycarbonylproline (from D-proline) correspondingly, as colorless oil.

A solution of (R)-N-isobutoxycarbonylproline (502 mg, 2.33 mmol) in THF (1.5 mL) is added dropwise over 10 min to a stirred solution of sodium borohydride (29 mg, 0.77 mmol) in THF (1.5 mL) at 0°C. The mixture is stirred overnight and then concentrated in vacuo to give the reducing agent 12. Analogously, reducing agent 13 is prepared from (S)-N-isobutoxycarbonylproline.

Reduction of 1-methyl-3, 4-dihydroisoquinoline with reducing agents 12 and 13

To a cooled (-35°C) solution of reducing agent 12 (526 mg, (0.78 mmol)in dichloromethane (3 mL) is added solution of 1-methyl-3,4-dihydroisoquinoline (32mg, 0.22 mmol) in dichloromethane (3 mL) dropwise over 15 min. The mixture is allowed to warm to -10°C over 1h and then kept at 0°C for 4h. The solvent is removed in vacuo and the residue is dissolved in methanol (10 mL). After cooling to 0°C, Amberlyst 15 (1g) is added and the mixture is shaken for 1h at room temperature. The resin is filtered off and washed with dichloromethane and methanol, then solution of ammonia in methanol (2M, 3 mL) is added and the mixture is shaken overnight, then filtered. The filtrate is evaporated in vacuo to give (R)-1-Methyl-1,2,3,4-tetrahydroisoquinoline (26 mg, 82%, e.e. = 74%, determined by chiral HPLC).

Analogously, (S)-1-Methyl-1,2,3,4-tetrahydroisoquinoline is prepared using the reducing agent 13, derived from L-proline.

Chiral amine building blocks may also be prepared by asymmetric transfer hydrogenation of bicyclic imines using a chiral ruthenium catalyst:

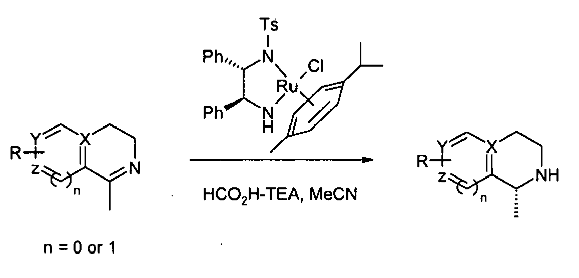

Asymmetric transfer hydrogenation of 1-methyl-3,4-dihydro-isoquinoline

### Preparation of catalyst.

A mixture of [RuCl₂(η⁶-p-cymene)]₂ (1.53 g, 2.5 mmol), (1S, 2S)-*N*-p-toluenesulfonyl-1,2-diphenylethylenediamine (1.83 g, 5.0 mmol) and triethylamine (1.4 mL, 10 mmol) in 2-propanol (50 mL) was heated at 80°C for 1h. The orange solution was concentrated to 1/3 of volume and the precipitate was collected by filtration, washed with small amount of water to give RuCl[(1S, 2S)-p-TsNCHPhCHPhNH2](η⁶-p-cymene) (2.99 g, 94%) as an air-stable orange solid.

### Transfer hydrogenation.

Catalyst (32 mg, 2.5 mol%) was added to a solution of 1-methyl-3,4-dihydro-isoquinoline (300 mg, 2.07 mmol) in acetonitrile (2mL) and triethylamine-formic acid aceotrope (2 mL). The mixture was stirred at room temperature for 24h, then diluted with sodium bicarbonate solution (30 mL) and extracted 3 times with DCM (3x10 mL). The combined organic extracts were dried over anhydrous sodium sulfate, filtered and evaporated *in vacuo.* The oily residue was dissolved in 1:1 mixture of diethyl ether and THF and treated with excess ethereal HCl solution. The precipitate was filtered, washed with ether and dried to give 270 mg (71%) of (R)-1-methyl-1,2,3,4-tetrahydro-isoquinoline hydrochloride as white solid.

The enantiomeric purity of the product was determined 92% ee, based on its conversion to an acetylated material and analysis of later by chiral-phase HPLC (Chiracel OD-H column), ratio of R and S isomers 95.6:3.4.

### B) Enantiomer Synthesis via Chiral Synthesis

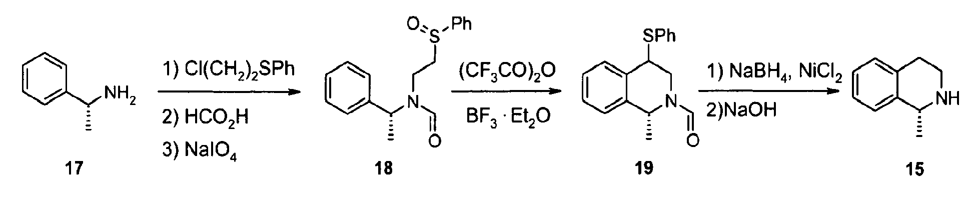

Alternatively, chiral 1-Methyl-1,2,3,4-tetrahydroisoquinolines 15 and 16 are prepared using a 6-step synthesis starting from chiral α-phenethylamine 17, (developed by Shinohara et al. Chemical & Pharmaceutical Bulletin (1998), 46(3), 430-433). This sequence utilizes a Pummerer rearrangement of sulfoxide 15 as a key step. No racemization occurs during the sequence, and the commercially available R and S α-phenetylamines with 99.5 % enantimeric purity yield chiral 1-Methyl-1,2,3,4-tetrahydroisoquinolines with the same enantiomeric purity.

### C) Separation via Chiral Column Chromatography

The racemate of the amino building block 20 is reacted with tert. butyl dicarbonate (Boc₂O) in dichloromethane leading to 4-Methyl-6,7-dihydro-4H-thieno[3,2-c]pyridine-5-carboxylic acid tert-butyl ester 21 in good yields. The separation of the boc-protected derivative 21 by preparative HPLC on chiral column Chiralpak AD-H 20×250 leads to the S and R enantiomers 22a and 22b.

The hydrochloride salt is formed by reaction with hydrochloric acid in dioxane at room temperature. The chiral purity of the enantiomers is confirmed by analytical HPLC (Chiralcel OD-H 4.6x250 mm). 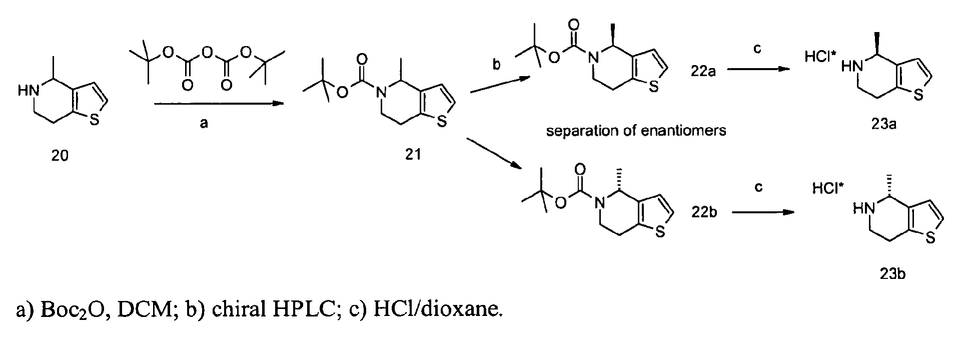 (S)-4-Methyl-4,5,6,7-tetrahydro-thieno[3,2-c]pyridine (23a) and (R)-4-Methyl-4,5,6,7-tetrahydro-thieno[3,2-c]pyridine (23b) hydrochlorides.

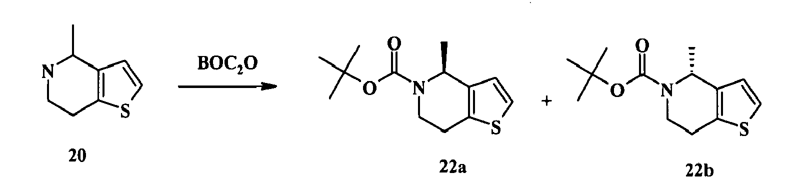

An amine 20 (624 mg, 4.007 mmol) is dissolved in dry dichloromethane, and Boc₂O (1068 mg, 4.9 mmol) is added. The reaction is stirred for 4 hrs at r.t. The reaction is monitored by TLC. When the reaction is over the reaction mixture is washed with 10% solution of K₂CO₃, with water and a saturated solution of NaCl in separation funnel, dried over Na₂SO₄, and concentrated under reduced pressure. As a result, 0.469 g of the mixture 22a and 22b is obtained as transparent oil. The isomers are separated by preparative column Chiralpak AD-H 20×250 (particle size 5 µm, eluent hexane/isopropyl alcohol, 95/5, flow speed 10 mL/min, detection by UV 230 nm).

The isomer 22a which has the S-configration, has been isolated in the amount of 120 mg (∼24%; RT 5.143 min; 3 - 61 mg; ∼12%; RT 5.975 min).

### Cleavage of Boc-protective group:

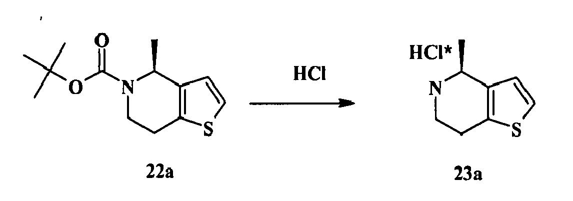

The compound 22a (120 mg) is stirred in dioxane saturated by HCl (content of HCl is 12%) for 3 hrs at r.t., and concentrated under reduced pressure. As a result, 23a having the S-configuration is obtained in the amount of 81 mg (∼89% counting on hydrochloride) as white powder.

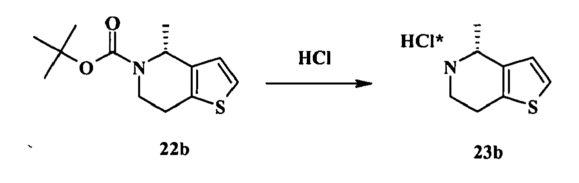

The compound 23b having the R-configuration is synthesized according to the same method as 23a in the amount 43 mg (∼93%) (counting on hydrochloride).

In the following, specific compounds according to the invention are described in more detail:

### Building Block Syntheses:

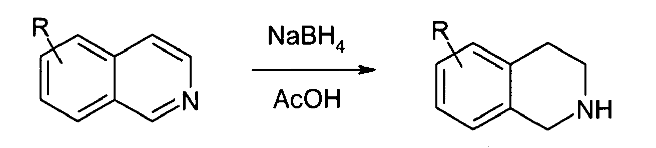

1 mmol of substituted isoquinoline is dissolved in acetic acid (5 mL) and 3.5 eq of NaBH₄ is added portionwise. The reaction mixture is stirred for 1 h, then poured into ice and NH₄OH is added to pH 9. The mixture is extracted with CH₂Cl₂ and the organic layer is dried over Na₂SO₄, filtered and evaporated. The residue is purified by flash chromatography to give the title compound.

### 5-Bromo-1,2,3,4-tetrahydro-isoquinoline

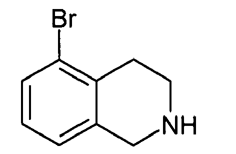

In close analogy to the procedure described above, 5-Bromo-isoquinoline is reacted with sodium borohydride to provide the title compound.
LC/MS: m/z = 212 (MH⁺)
¹H NMR (CDCl₃) δ: 2.73, 3.16, 3.98, 7.00, 7.37-7.42.

### 6-Bromo-1,2,3,4-tetrahydro-isoquinoline

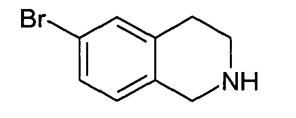

In close analogy to the procedure described above, 6-Bromo-isoquinoline is reacted with sodium borohydride to provide the title compound.
LC/MS: m/z = 212 (MH⁺)
¹H NMR (CDCl₃) δ: 2.76, 3.11, 3.94, 6.86, 7.22, 7.55-7.99.

### 5-Nitro-1,2,3,4-tetrahydro-isoquinoline

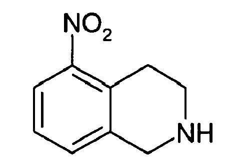

In close analogy to the procedure described above, 5-Nitro-isoquinoline is reacted with sodium borohydride to provide the title compound.
LC/MS: m/z = 178 (MH⁺)

### 2-Chloro-N-methoxy-N-methyl-nicotinamide

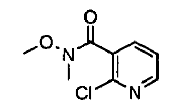

2-Chloro-nicotinic acid (10.0 g, 63.5 mmol, 1.0 equiv) is suspended in dichloromethane (400 mL) and THF (100 mL). N,O-Dimethyl-hydroxylamine hydrochloride (6.81 g, 69.8 mmol, 1.1 equiv) and triethylamine (9.70 mL, 69.8 mmol, 1.1 equiv) are added, resulting in a clear solution. The mixture is cooled in an ice bath and EDCI (13.4 g, 69.8 mmol, 1.1 equiv) and HOAt (0.86 g, 6.35 mmol, 0.1 equiv) are added. The resulting mixture is stirred overnight at room temperature and concentrated in vacuo. The residue is redissolved in ethyl acetate (200 mL) and washed with 1N NaOH solution, twice with sat. NaHCO₃ solution and brine. The organic phase is dried over Na₂SO₄, filtered and concentrated in vacuo. This gives 7.53 g of the title compound as a white solid. Yield: 59%
LC/MS: m/z = 142 (MH⁺)
¹H NMR (CDCl₃) δ: 3.08, 3.31, 3.46, 3.78, 7.53, 8.01, 8.49.

### 1-(2-Chloro-pyridin-3-yl)-ethanone

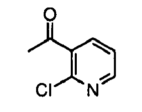

2-Chloro-N-methoxy-N-methyl-nicotinamide (3.90 g, 19.4 mmol, 1.0 equiv) is dissolved in THF (200 mL) under nitrogen and the mixture is cooled in an ice bath. Methylmagnesium chloride (17.0 ml, 48.6 mmol, 2.5 equiv) is added within 2 minutes. After 15 min a grey precipitate is formed. Stirring is continued for 2 h, the mixture is cooled in an ice bath and sat. NH₄Cl (150 mL) is added. The solution is acidified with 1N HCl solution (60 mL) followed by addition of sat. NaHCO₃ solution (30 mL). The organic layer is separated and concentrated in vacuo. The residue is combined with the remaining water layer and extracted with diethyl ether (200 mL). The combined etheral layers are washed twice with brine (50 mL), dried over Na₂SO₄, filtered and concentrated in vacuo. This provides 2.57 g of the title compound as a yellow oil.
Yield: 85%
¹H NMR (CDCl₃) δ: 2.71, 7.35, 7.92, 8.50.

### 2-Chloro-3-(2-methyl-[1,3]dioxolan-2-yl)-pyridine

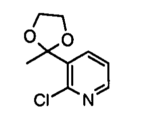

1-(2-Chloro-pyridin-3-yl)-ethanone (2.21 g, 14.2 mmol, 1.0 equiv), ethylene glycol (4.41 g, 71.0 mmol, 5.0 equiv) and p-toluenesulfonic acid monohydrate (1.35 g, 7.1 mmol, 0.5 equiv) are dissolved in toluene (100 mL). The resulting mixture is heated overnight under Dean Stark conditions. After cooling to room temperature the mixture is washed twice with saturated NaHCO₃ solution, brine, dried over Na₂SO₄ and evaporated in vacuo. The residue is purified by flash column chromatography (10% to 50% ethyl acetate in heptane) to yield 2.19 g of the title compound.
Yield: 77%
¹H NMR (CDCl₃) δ: 1.82, 3.79, 4.09, 7.24, 7.98, 8.35.

### [3-(2-Methyl-1,3-dioxolan-2-yl)-2-pyridyl]methyl cyanide

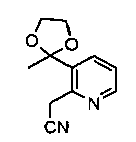

A solution of 2-chloro-3-(2-methyl-[1,3]dioxolan-2-yl)-pyridine (2.19 g, 11.0 mmol, 1.0 equiv) and acetonitrile (577 µL, 11.0 mmol, 1.0 equiv) in THF (75 mL) is cooled to 5°C under nitrogen atmosphere. 2M Sodium bis(trimethylsilyl)amide solution in THF (11.0 mL, 21.9 mmol, 2.0 equiv) is added within 1 minute. This resultes in a bright yellow solution which turnes orange over time. Stirring at 0°C is continued for exact 1 h. According to TLC there is complete conversion. Saturated NH₄Cl solution (50 mL) is added. The organic layer is separated and concentrated in vacuo. The residue is combined with the water layer and extracted with ethyl acetate (75 mL). The organic layer is washed three times with sat. NH₄Cl solution, dried over Na₂SO₄ and evaporated in vacuo. The residue is purified by flash column chromatography (10% to 50% ethyl acetate in heptane) and gave 540 mg of the title compound as an oil.
Yield: 24%
¹H NMR (CDCl₃) δ: 1.82, 3.79, 4.10, 7.24, 7.98, 8.35.

### 2-[3-(2-Methyl-[1,3]dioxolan-2-yl)-pyridin-2-yl]-ethylamine

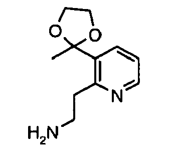

[3-(2-Methyl-1,3-dioxolan-2-yl)-2-pyridyl]methyl cyanide (540 mg, 2.64 mmol, 1.0 equiv) is dissolved in ethanol. A catalytic amount of Raney Nickel is added and the resulting mixture is placed under a hydrogen atmosphere. The mixture is stirred for 7 h using a hot water bath to warm the reaction mixture. After flushing with nitrogen, the mixture is filtered over kieselguhr and rinsed with ethyl acetate. The filtrate is concentrated in vacuo with replenishment of dichloromethane. This gave 490 mg of the title compound as a yellow oil.
Yield: 99%
¹H NMR (CDCl₃) δ: 1.71, 3.16, 3.75, 4.06, 7.11, 7.86, 8.48.

### 5-Methyl-7,8-dihydro-[1,6]naphthyridine

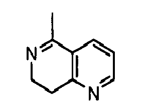

2-[3-(2-Methyl-[1,3]dioxolan-2-yl)-pyridin-2-yl]-ethylamine (490 mg, 2.35 mmol, 1.0 equiv) is dissolved in 1N HCl solution (47 mL, 47.0 mmol, 20.0 equiv) and heated at 100°C for 2 hours. According to TLC analysis the reaction is completed. The mixture is cooled and 1N NaOH solution is added. The mixture is extracted twice with dichloromethane, washed with brine, dried over Na₂SO₄, filtered and concentrated in vacuo. This gave the title compound.
¹H NMR (CDCl₃) δ: 2.39, 2.93, 3.84, 7.26, 7.73, 8.53.

### 5-Methyl-5,6,7,8-tetrahydro-[1,6]naphthyridine

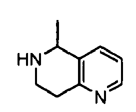

5-Methyl-7,8-dihydro-[1,6]naphthyridine (1.0 equiv) dissolved in ethanol (50 mL) and PtO₂ (100 mg) is added. The mixture is placed under hydrogen atmosphere and stirred for 2 h. The catalyst is filtered off over kieselguhr and the filter is rinsed with ethyl acetate. The filtrate is evaporated in vacuo and purified by flash column chromatography (5 to 40% 10% 7N NH₃ in methanol in CH₂Cl₂/CH₂Cl₂). This provided 178 mg of the title compound as a yellow oil.
Yield: 51 % for two steps
¹H NMR (CDCl₃) δ: 1.46, 1.61, 2.93, 3.09-3.13, 3.36, 4.12, 7.10, 7.44, 8.40.

### General Scheme:

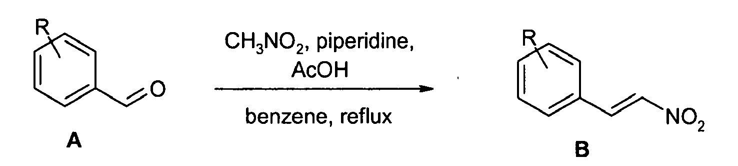

To a solution of aldehyde A (1 mol) in benzene (approx. 20 ml per 1 g of the starting compound) nitromethane (1.2 mol), piperidine (0.1 mol) and acetic acid (0.1 mol) are added. The reaction mixture is refluxed for 2.5 h with azeotrope removing of water. Then, the solvent is evaporated in vacuo. The residue is triturated with cold methanol, filtered, washed with cold methanol that resulted in product crystals in good to moderate yields.

### 1-(2-Nitro-vinyl)-4-trifluoromethyl-benzene

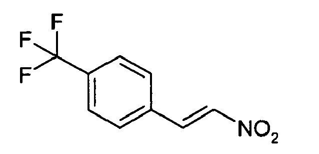

In close analogy to the procedure described above, 4-Trifluoromethyl-benzaldehyde is reacted with nitromethane to provide the title compound.
Yield: 98%
LC/MS: m/z = 218 (MH⁺)

### 1-(2-Nitro-vinyl)-2-trifluoromethyl-benzene

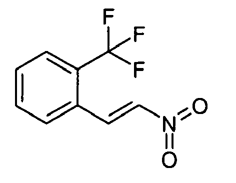

In close analogy to the procedure described above, 2-Trifluoromethyl-benzaldehyde is reacted with nitromethane to provide the title compound.
LC/MS: m/z = 217 (MH⁺)

### 1,2-Dimethoxy-3-(2-nitro-vinyl)-benzene

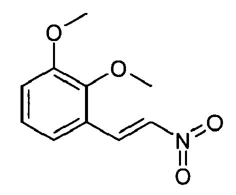

In close analogy to the procedure described above, 2,3-Dimethoxy-benzaldehyde is reacted with nitromethane to provide the title compound.
LC/MS: m/z = 209 (MH⁺)

### 4-(2-Nitro-vinyl)-benzo[1,3]dioxole

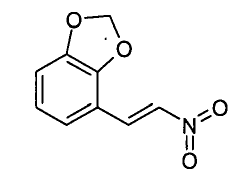

In close analogy to the procedure described above, Benzo[1,3]dioxole-4-carbaldehyde is reacted with nitromethane to provide the title compound.
¹H NMR (CDCl₃) δ: 6.12, 6.91, 7.87.

### 5-(2-Nitro-vinyl)-2,3-dihydro-benzo[1,4]dioxine

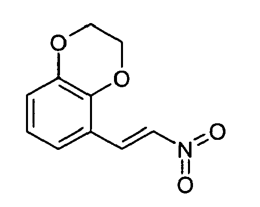

In close analogy to the procedure described above, 2,3-Dihydro-benzo[1,4]dioxine-5-carbaldehyde is reacted with nitromethane to provide the title compound.
LC/MS: m/z = 207 (MH⁺)

### General Scheme:

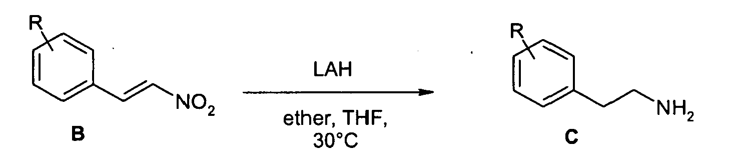

To a suspension of lithium aluminium hydride (2 mol) in diethyl ether (approx. 20 ml per 1 g of LAH) solution of the corresponding nitro derivative B (1 mol) in dry THF is added dropwise at stirring within 30 min. The reaction mixture is stirred at 35°C for 2h, and then, cooled with ice water. Then, 20% aqueous NaOH and water (n ml, n ml, 3n ml per n g LAH) are added step by step carefully. The resulting mixture is stirred for 30 min, filtered and concentrated on rotary evaporator. The residue is purified by flash column chromatography on silica gel (eluent: chloroform/methanol - 0→20%) that gave products in good to moderate yields.

### 2-(4-Trifluoromethyl-phenyl)-ethylamine

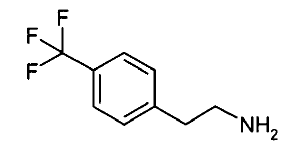

In close analogy to the procedure described above, 1-(2-Nitro-vinyl)-4-trifluoromethylbenzene is reacted with lithium aluminium hydride to provide the title compound.
LC/MS:m/z=190(MH⁺)

### 2-(2-Trifluoromethylphenyl)-ethylamine

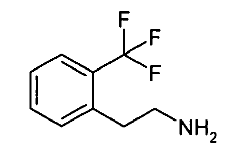

In close analogy to the procedure described above, 1-(2-Nitro-vinyl)-2-trifluoromethylbenzene is reacted with lithium aluminium hydride to provide the title compound.
LC/MS: m/z = 189 (MH⁺)

### 2-(2,3-Dimethoxyphenyl)-ethylamine

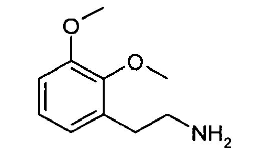

In close analogy to the procedure described above, 1,2-Dimethoxy-3-(2-nitro-vinyl)-benzene is reacted with lithium aluminium hydride to provide the title compound.
LC/MS: m/z = 181 (MH⁺)

### 2-Benzo[1,3]dioxol-4-yl-ethylamine

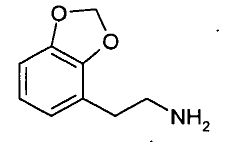

In close analogy to the procedure described above, 4-(2-Nitro-vinyl)-benzo[1,3]dioxole is reacted with lithium aluminium hydride to provide the title compound.
¹H NMR (CDCl₃) δ:1.36, 2.71, 5.91, 6.68.

### 2-(2,3-Dihydro-benzo[1,4]dioxin-5-yl)-ethylamine

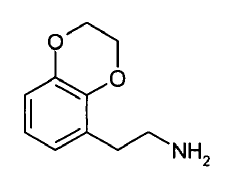

In close analogy to the procedure described above, 5-(2-Nitro-vinyl)-2,3-dihydro-benzo[1,4]dioxine is reacted with lithium aluminium hydride to provide the title compound.
LC/MS: m/z = 180 (MH⁺)

### General Scheme:

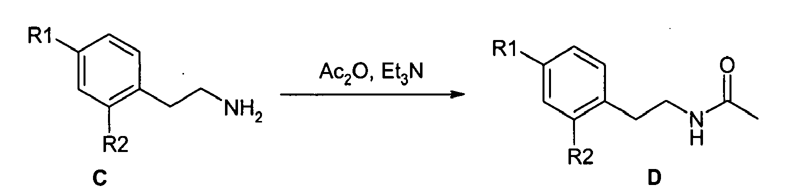

To a solution of the amine C (1 mol) in CH₂Cl₂ (approx. 10 ml per 1 g of the starting compound) triethylamine (1.1 mol) and acetic anhydride (1.2 mol) are added. The reaction mixture is stirred at room temperature for 1h and concentrated on rotary evaporator. The residue is triturated with hexane, filtered, washed with hexane to give a crystalline compound in good yields.

### N-[2-(4-Trifluoromethyl-phenyl)-ethyl]-acetamide

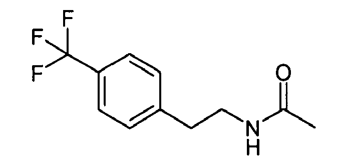

In close analogy to the procedure described above, 2-(4-trifluoromethyl-phenyl)-ethylamine is reacted with acetic anhydride to provide the title compound.
Yield: 29% for two steps
LC/MS: m/z = 232 (MH⁺)

### N-(2-o-Tolyl-ethyl)-acetamide

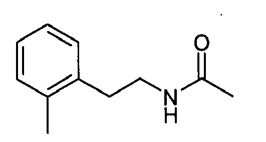

In close analogy to the procedure described above, 2-o-tolyl-ethylamine is reacted with acetic anhydride to provide the title compound.
Yield: 88%
LC/MS: m/z = 178 (MH⁺)

### N-(2-p-Tolyl-ethyl)-acetamide

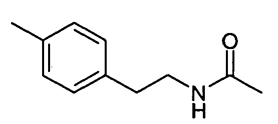

In close analogy to the procedure described above, 2-p-tolyl-ethylamine is reacted with acetic anhydride to provide the title compound.
LC/MS: m/z = 178 (MH⁺)

### N-[2-(4-Chloro-phenyl)-ethyl]-acetamide

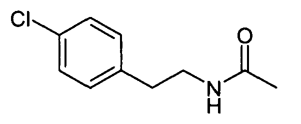

In close analogy to the procedure described above, 2-(4-chloro-phenyl)-ethylamine is reacted with acetic anhydride to provide the title compound.
LC/MS: m/z = 198 (MH⁺)

### N-[2-(2-Chloro-phenyl)-ethyl]-acetamide

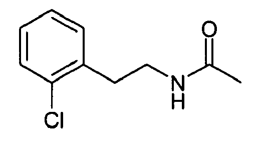

In close analogy to the procedure described above, 2-(2-chloro-phenyl)-ethylamine is reacted with acetic anhydride to provide the title compound.
LC/MS: m/z = 198 (MH⁺)

### N-Phenethyl-acetamide

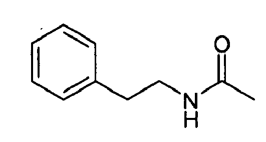

In close analogy to the procedure described above, phenethylamine is reacted with acetic anhydride to provide the title compound.
LC/MS: m/z = 164 (MH⁺)

### N-[2-(2-Fluoro-phenyl)-ethyl]-acetamide

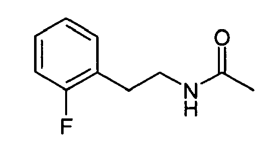

In close analogy to the procedure described above, 2-(2-fluoro-phenyl)-ethylamine is reacted with acetic anhydride to provide the title compound.
LC/MS: m/z = 181 (MH⁺)

### N-[2-(3-Fluoro-phenyl)-ethyl]-acetamide

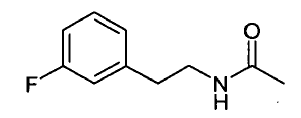

In close analogy to the procedure described above, 2-(3-fluoro-phenyl)-ethylamine is reacted with acetic anhydride to provide the title compound.
LC/MS: m/z = 181 (MH⁺)

### N-[2-(4-Fluoro-phenyl)-ethyl]-acetamide

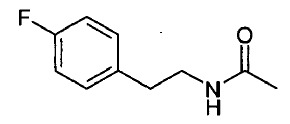

In close analogy to the procedure described above, 2-(4-fluoro-phenyl)-ethylamine is reacted with acetic anhydride to provide the title compound.
LC/MS: m/z = 181 (MH⁺)

### N-[2-(2-Bromo-phenyl)-ethyl]-acetamide

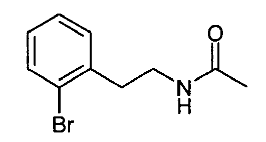

In close analogy to the procedure described above, 2-(2-Bromo-phenyl)-ethylamine is reacted with acetic anhydride to provide the title compound.
LC/MS: m/z = 184 (MH⁺)
¹H NMR (CDCl₃) δ:1.94, 2.95, 3.49, 7.07, 7.22-7.53.

### N-[2-(3-Bromo-phenyl)-ethyl]-acetamide

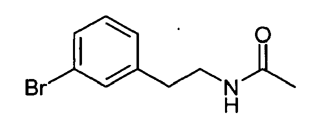

In close analogy to the procedure described above, 2-(3-bromo-phenyl)-ethylamine is reacted with acetic anhydride to provide the title compound.
LC/MS: m/z = 243 (MH⁺)

### N-[2-(4-Bromo-phenyl)-ethyl]-acetamide

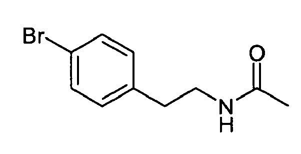

In close analogy to the procedure described above, 2-(4-bromo-phenyl)-ethylamine is reacted with acetic anhydride to provide the title compound.
LC/MS: m/z = 243 (MH⁺)
¹H NMR (CDCl₃) δ:1.38, 2.76, 3.47, 5.41, 7.06, 7.42.

### N-[2-(2-Methoxy-phenyl)-ethyl]-acetamide

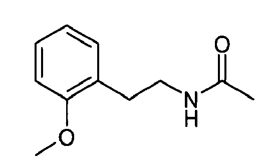

In close analogy to the procedure described above, 2-(2-methoxy-phenyl)-ethylamine is reacted with acetic anhydride to provide the title compound.
LC/MS: m/z = 193 (MH⁺)

### N-[2-(3-Methoxy-phenyl)-ethyl]-acetamide

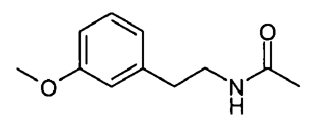

In close analogy to the procedure described above, 2-(3-methoxy-phenyl)-ethylamine is reacted with acetic anhydride to provide the title compound.
LC/MS: m/z = 193 (MH⁺)

### N-[2-(4-Methoxy-phenyl)-ethyl]-acetamide

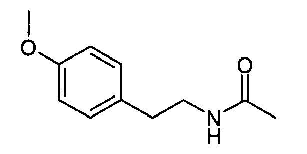

In close analogy to the procedure described above, 2-(4-methoxy-phenyl)-ethylamine is reacted with acetic anhydride to provide the title compound.
LC/MS: m/z = 193 (MH⁺)

### N-[2-(2-Trifluoromethyl-phenyl)-ethyl]-acetamide

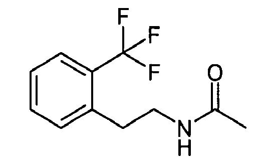

In close analogy to the procedure described above, 2-(2-trifluoromethyl-phenyl)-ethylamine is reacted with acetic anhydride to provide the title compound.
LC/MS: m/z = 223 (MH⁺)

### N-[2-(2,3-Dimethoxy-phenyl)-ethyl]-acetamide

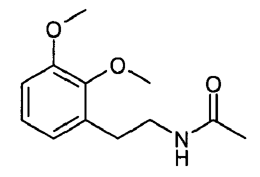

In close analogy to the procedure described above, 2-(2,3-dimethoxy-phenyl)-ethylamine is reacted with acetic anhydride to provide the title compound.
LC/MS: m/z = 231 (MH⁺)

### N-(2-Benzo[1,3]dioxol-4-yl-ethyl)-acetamide

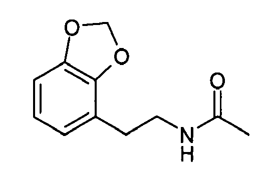

In close analogy to the procedure described above, 2-benzo[1,3]dioxol-4-yl-ethylamine is reacted with acetic anhydride to provide the title compound.
¹H NMR (DMSO-d₆) δ: 1.77, 2.65, 3.24 , 5.98, 7.74, 7.93.

### N-[2-(2,3-Dihydro-benzo[1,4]dioxin-5-yl)-ethyl]-acetamide

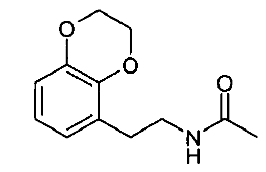

In close analogy to the procedure described above, 2-(2,3-dihydro-benzo[1,4]dioxin-5-yl)-ethylamine is reacted with acetic anhydride to provide the title compound.
LC/MS: m/z = 221 (MH⁺)

### General Scheme:

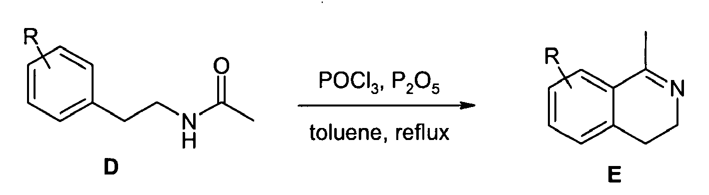

To a solution of the amide D (1 mol) in toluene (approx. 10 ml per 1 g of the starting compound) phosphorus pentoxide (approx. 1.5 g per 1 g of the starting compound) and phosphorus oxychloride (2-3 mol) are added. The reaction mixture is refluxed for 3h and cooled to 0°C. Some ice is carefully added to the reaction mixture, and it is stirred for 1 h. Then, the mixture is filtered. The organic layer is separated and washed with water. The combined aqueous layers are washed with benzene, then alkalified with 25% sodium hydroxide, extracted with benzene, dried over Na₂SO₄ and concentrated on rotary evaporator. Resulting oil is used in the next step without purification.

### 1-Methyl-7-trifluoromethyl-3,4-dihydro-isoquinoline

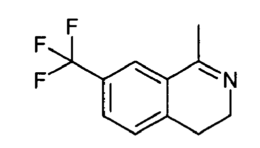

In close analogy to the procedure described above, N-[2-(4-trifluoromethyl-phenyl)-ethyl]-acetamide is reacted with phosphorus pentoxide to provide the title compound.
LC/MS: m/z = 214 (MH⁺)

### 1,5-Dimethyl-3,4-dihydro-isoquinoline

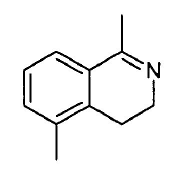

In close analogy to the procedure described above, N-(2-o-tolyl-ethyl)-acetamide is reacted with phosphorus pentoxide to provide the title compound.
Yield: 59%
LC/MS: m/z = 160 (MH⁺)

### 1,7-Dimethyl-3,4-dihydro-isoquinoline

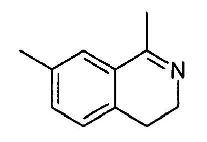

In close analogy to the procedure described above, N-(2-p-tolyl-ethyl)-acetamide is reacted with phosphorus pentoxide to provide the title compound.
LC/MS: m/z = 160 (MH⁺)

### 7-Chloro-1-methyl-3,4-dihydro-isoquinoline

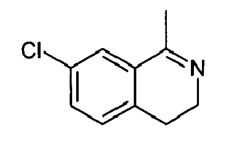

In close analogy to the procedure described above, N-[2-(4-chloro-phenyl)-ethyl]-acetamide is reacted with phosphorus pentoxide to provide the title compound.
LC/MS: m/z = 180 (MH⁺)

### S-Chloro-1-methyl-3,4-dihydro-isoquinoline

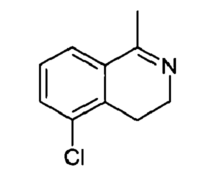

In close analogy to the procedure described above, N-[2-(2-chloro-phenyl)-ethyl]-acetamide is reacted with phosphorus pentoxide to provide the title compound.
LC/MS: m/z = 180 (MH⁺)

### 1-Methyl-3,4-dihydro-isoquinoline

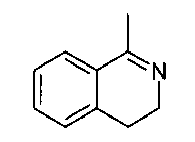

In close analogy to the procedure described above, N-phenethyl-acetamide is reacted with phosphorus pentoxide to provide the title compound.
¹H NMR (CDCl₃) δ: 2.38, 2.69, 3.65, 7.15-7.49.

### 5-Fluoro-1-methyl-3,4-dihydro-isoquinoline

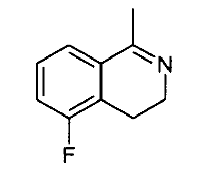

In close analogy to the procedure described above, N-[2-(2-fluoro-phenyl)-ethyl]-acetamide is reacted with phosphorus pentoxide to provide the title compound.
LC/MS: m/z = 163 (MH⁺)

### 7-Fluoro-1-methyl-3,4-dihydro-isoquinoline

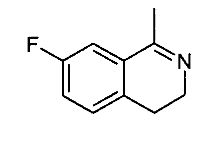

In close analogy to the procedure described above, N-[2-(4-fluoro-phenyl)-ethyl]-acetamide is reacted with phosphorus pentoxide to provide the title compound.
LC/MS: m/z = 163 (MH⁺)

### 6-Fluoro-1-methyl-3,4-dihydro-isoquinoline

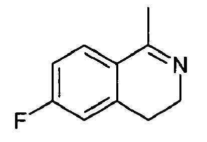

In close analogy to the procedure described above, N-[2-(3-fluoro-phenyl)-ethyl]-acetamide is reacted with phosphorus pentoxide to provide the title compound.
LC/MS: m/z = 163 (MH⁺)

### 8-Fluoro-1-methyl-3,4-dihydro-isoquinoline

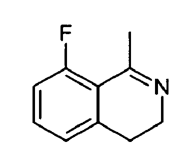

In close analogy to the procedure described above, N-[2-(3-fluoro-phenyl)-ethyl]-acetamide is reacted with phosphorus pentoxide to provide the title compound.
LC/MS: m/z = 163 (MH⁺)

### 5-Bromo-1-methyl-3,4-dihydro-isoquinoline

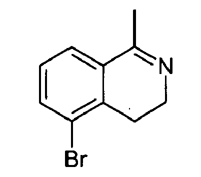

In close analogy to the procedure described above, N-[2-(2-bromo-phenyl)-ethyl]-acetamide is reacted with phosphorus pentoxide to provide the title compound. LC/MS: m/z = 224 (MH⁺)
¹H NMR (CDCl₃) δ: 2.37, 2.79, 3.68, 7.17, 7.44, 7.52.

### 7-Bromo-1-methyl-3,4-dihydro-isoquinoline

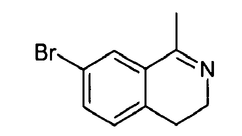

In close analogy to the procedure described above, N-[2-(4-bromo-phenyl)-ethyl]-acetamide is reacted with phosphorus pentoxide to provide the title compound.
LC/MS: m/z = 224 (MH⁺)
¹H NMR (CDCl₃) δ: 2.25, 2.64, 3.66, 7.06, 7.47, 7.58.

### 6-Bromo-1-methyl-3,4-dihydro-isoquinoline

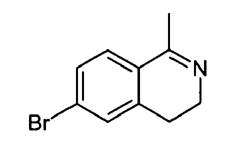

In close analogy to the procedure described above, N-[2-(3-bromo-phenyl)-ethyl]-acetamide is reacted with phosphorus pentoxide to provide the title compound.
LC/MS: m/z = 224 (MH⁺)
¹H NMR (CDCl₃) δ: 2.35, 2.67, 3.64, 7.32-7.44.

### 5-Methoxy-1-methyl-3,4-dihydro-isoquinoline

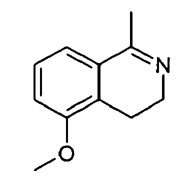

In close analogy to the procedure described above, N-[2-(2-methoxy-phenyl)-ethyl]-acetamide is reacted with phosphorus pentoxide to provide the title compound.
LC/MS: m/z = 175 (MH⁺)

### 7-Methoxy-1-methyl-3,4-dihydro-isoquinoline

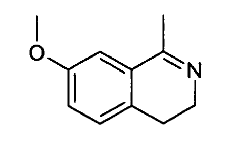

In close analogy to the procedure described above, N-[2-(4-methoxy-phenyl)-ethyl]-acetamide is reacted with phosphorus pentoxide to provide the title compound.

LC/MS: m/z = 175 (MH⁺)

### 5,6-Dimethoxy-1-methyl-3,4-dihydro-isoquinoline

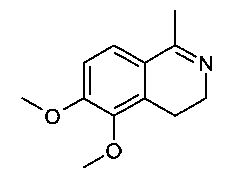

In close analogy to the procedure described above, N-[2-(2,3-dimethoxy-phenyl)-ethyl]-acetamide is reacted with phosphorus pentoxide to provide the title compound.
LC/MS: m/z = 205 (MH⁺)

### 1-Methyl-5-trifluoromethyl-3,4-dihydro-isoquinoline

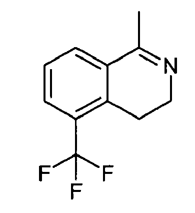

In close analogy to the procedure described above, N-[2-(2-trifluormethyl-phenyl)-ethyl]-acetamide is reacted with phosphorus pentoxide to provide the title compound.
LC/MS: m/z = 213 (MH⁺)

### 6-Methoxy-1-methyl-3,4-dihydro-isoquinoline

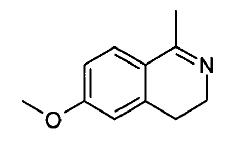

In close analogy to the procedure described above, N-[2-(3-methoxy-phenyl)-ethyl]-acetamide is reacted with phosphorus pentoxide to provide the title compound.
LC/MS: m/z = 175 (MH⁺)

### 6-Methyl-8,9-dihydro-1,3-dioxa-7-aza-cyclopenta[a]naphthalene

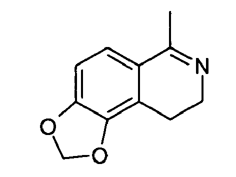

In close analogy to the procedure described above, N-(2-Benzo[1,3]dioxol-4-yl-ethyl)-acetamide is reacted with phosphorus pentoxide to provide the title compound.
LC/MS: m/z = 189 (MH⁺)
¹H NMR (CDCl₃) δ: 2.34, 2.65, 3.65, 6.01, 6.72, 7.06.

### 8-Methyl-2,3,5,6-tetrahydro-1,4-dioxa-7-aza-phenanthrene

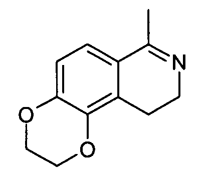

In close analogy to the procedure described above, N-[2-(2,3-dihydro-benzo[1,4]dioxin-5-yl)-ethyl]-acetamide is reacted with phosphorus pentoxide to provide the title compound.
LC/MS: m/z = 203 (MH⁺)

### General Scheme:

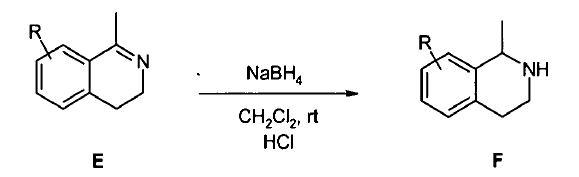

To a solution of compound E (1 mol) in CH₂Cl₂ (approx. 20 ml per 1 g of the starting compound) sodium borohydride (1.5 mol) is added. The reaction mixture is stirred for 12h at room temperature. The reaction mixture is poured into saturated sodium bicarbonate solution and stirred for 30 min. The organic layer is separated, and aqueous layer is extracted with chloroform. The combined organic phases are dried over Na₂SO₄ and concentrated on rotary evaporator. The residue is purified by flash column chromatography on silica gel (eluent: chloroform/methanol - 0→20%) to give yellowish oil. Then, 6 N isopropanolic HCl is added to the oil, stirred for 5 min, concentrated on rotary evaporator, triturated with diethyl ether, filtered off, washed with ether and dried in vacuo to give crystalline compound in acceptable yields on 2 steps.

### 1-Methyl-7-trifluoromethyl-1,2,3,4-tetrahydro-isoquinoline

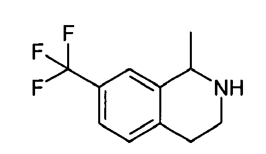

In close analogy to the procedure described above, 1-methyl-7-trifluoromethyl-3,4-dihydro-isoquinoline is reacted with sodium triacetoxyborohydride to provide the title compound.
Yield: 44% for two steps
LC/MS: m/z = 216 (MH⁺)

### 1,5-Dimethyl-1,2,3,4-tetrahydro-isoquinoline

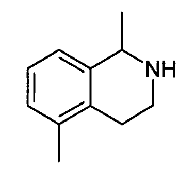

In close analogy to the procedure described above, 1,5-dimethyl-3,4-dihydro-isoquinoline is reacted with sodium triacetoxyborohydride to provide the title compound.
Yield: 12%
LC/MS: m/z = 162 (MH⁺)

### 1,7-Dimethyl-1,2,3,4-tetrahydro-isoquinoline

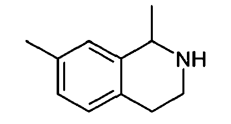

In close analogy to the procedure described above, 1,7-dimethyl-3,4-dihydro-isoquinoline is reacted with sodium triacetoxyborohydride to provide the title compound.
LC/MS: m/z = 162 (MH⁺)

### 7-Chloro-1-methyl-1,2,3,4-tetrahydro-isoquinoline

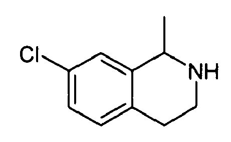

In close analogy to the procedure described above, 7-chloro-1-methyl-3,4-dihydroisoquinoline is reacted with sodium triacetoxyborohydride to provide the title compound.
LC/MS: m/z = 182 (MH⁺)

### 5-Chloro-1-methyl-1,2,3,4-tetrahydro-isoquinoline

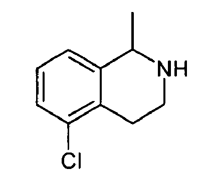

In close analogy to the procedure described above, 5-chloro-1-methyl-3,4-dihydroisoquinoline is reacted with sodium triacetoxyborohydride to provide the title compound.
LC/MS: m/z = 182 (MH⁺)

### 1-Methyl-1,2,3,4-tetrahydro-isoquinoline

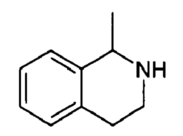

In close analogy to the procedure described above, 1-methyl-3,4-dihydro-isoquinoline is reacted with sodium triacetoxyborohydride to provide the title compound.
LC/MS: m/z = 147 (MH⁺)
¹H NMR (CDCl₃) δ: 1.45, 2.67-3.05, 3.21-3.29, 4.12, 7.09-7.14.

### 5-Fluoro-1-methyl-1,2,3,4-tetrahydro-isoquinoline

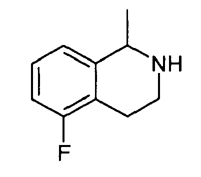

In close analogy to the procedure described above, 5-fluoro-1-methyl-3,4-dihydroisoquinoline is reacted with sodium triacetoxyborohydride to provide the title compound.
LC/MS: m/z = 165 (MH⁺)
¹H NMR (CDCl₃) δ: 1.50, 2.79-3.35, 4.15, 6.82-7.14.

### 7-Fluoro-1-methyl-1,2,3,4-tetrahydro-isoquinoline

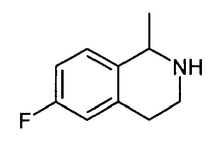

In close analogy to the procedure described above, 7-fluoro-1-methyl-3,4-dihydroisoquinoline is reacted with sodium triacetoxyborohydride to provide the title compound.
LC/MS: m/z = 165 (MH⁺)
¹H NMR (CDCl₃) δ: 1.44, 2.58-2.83, 2.98, 3.25, 4.04, 6.79-7.06.

### 6-Fluoro-1-methyl-1,2,3,4-tetrahydro-isoquinoline

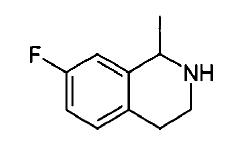

In close analogy to the procedure described above, 6-fluoro-1-methyl-3,4-dihydroisoquinoline is reacted with sodium triacetoxyborohydride to provide the title compound.
LC/MS: m/z = 165 (MH⁺)
¹H NMR (CDCl₃) δ: 1.44 , 2.72-2.92, 3.04, 3.25, 4.05, 6.74, 6.83-6.88, 7.04-7.11.

### 8-Fluoro-1-methyl-1,2,3,4-tetrahydro-isoquinoline

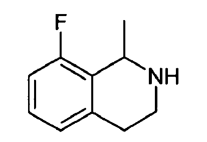

In close analogy to the procedure described above, 8-fluoro-1-methyl-3,4-dihydro-isoquinolines is reacted with sodium triacetoxyborohydride to provide the title compound.
LC/MS: m/z = 165 (MH⁺)
¹H NMR (CDCl₃) δ: 1.48, 2.75-3.35, 4.34, 6.79-6.89, 7.04-7.15.

### 5-Bromo-1-methyl-1,2,3,4-tetrahydro-isoquinoline

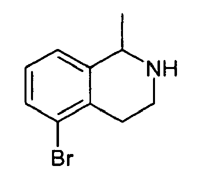

In close analogy to the procedure described above, 5-bromo-1-methyl-3,4-dihydroisoquinoline is reacted with sodium triacetoxyborohydride to provide the title compound.
LC/MS: m/z = 226 (MH⁺)
¹H NMR (CDCl₃) δ: 1.44, 1.56, 2.72-2.78, 2.96-3.05, 3.23-3.33, 4.08, 7.01-7.11, 7.39.

### 6-Bromo-1-methyl-1,2,3,4-tetrahydro-isoquinoline

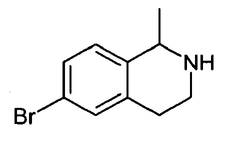

In close analogy to the procedure described above, 6-bromo-1-methyl-3,4-dihydroisoquinoline is reacted with sodium triacetoxyborohydride to provide the title compound.
LC/MS: m/z = 226 (MH⁺)
¹H NMR (CDCl₃) δ: 1.42, 1.55, 2.62-2.83, 2.92-3.04, 3.18-3.29, 4.02 , 6.99, 7.25, 7.22.

### 7-Bromo-1-methyl-1,2,3,4-tetrahydro-isoquinoline

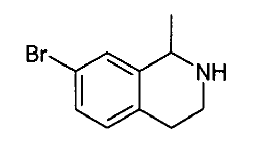

In close analogy to the procedure described above, 7-bromo-1-methyl-3,4-dihydroisoquinoline is reacted with sodium triacetoxyborohydride to provide the title compound.
LC/MS: m/z = 226 (MH⁺)
¹H NMR (CDCl₃) δ: 1.43, 2.66-2.75, 2.91-3.01, 3.19-3.27, 4.05, 6.92-7.25.

### 1-Methyl-5-methoxy-1,2,3,4-tetrahydro-isoquinoline

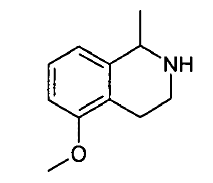

In close analogy to the procedure described above, 1-Methyl-5-methoxy-3,4-dihydroisoquinoline is reacted with sodium triacetoxyborohydride to provide the title compound.
LC/MS: m/z = 177 (MH⁺)
¹H NMR (CDCl₃) δ: 1.45, 1.98, 2.67-2.69, 2.95-3.05, 3.21-3.32, 3.81, 4.03-4.13, 6.68, 6.76, 7.13.

### 1-Methyl-6-methoxy-1,2,3,4-tetrahydro-isoquinoline

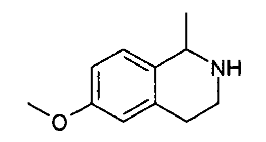

In close analogy to the procedure described above, 1-Methyl-6-methoxy-3,4-dihydroisoquinoline is reacted with sodium triacetoxyborohydride to provide the title compound.
LC/MS: m/z = 177 (MH⁺)
¹H NMR (CDCl₃) δ: 1.43, 2.62-3.28, 3.77, 4.04, 6.60, 6.69-6.74, 7.05.

### 1-Methyl-7-methoxy-1,2,3,4-tetrahydro-isoquinoline

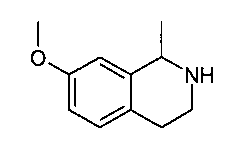

In close analogy to the procedure described above, 1-Methyl-7-methoxy-3,4-dihydroisoquinoline is reacted with sodium triacetoxyborohydride to provide the title compound.
LC/MS: m/z = 177 (MH⁺)
¹H NMR (CDCl₃) δ: 1.45, 1.6, 2.58-3.00, 3.19-3.30, 3.77, 4.01-4.11, 6.68, 6.70, 6.99.

### 1-Methyl-5,6-dimethoxy-1,2,3,4-tetrahydro-isoquinoline

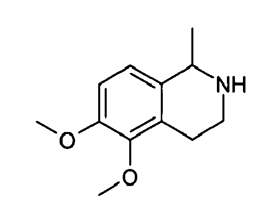

In close analogy to the procedure described above, 1-Methyl-5,6-dimethoxy-3,4-dihydroisoquinoline is reacted with sodium triacetoxyborohydride to provide the title compound.
LC/MS: m/z = 207 (MH⁺)
¹HNMR (CDCl₃) δ: 1.42, 1.64, 2.73-3.02, 3.20-3.31, 3.79, 3.83, 4.02, 6.73-6.88.

### 1-Methyl-5-trifluoromethyl-1,2,3,4-tetrahydro-isoquinoline

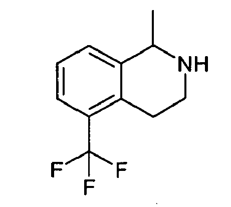

In close analogy to the procedure described above, I-Methyl-5-trifluoromethyl-3,4-dihydro-isoquinoline is reacted with sodium triacetoxyborohydride to provide the title compound.
LC/MS: m/z = 215 (MH⁺)
¹H NMR (CDCl₃) δ: 1.46, 1.64, 2.96, 3.02-3.07, 3.22-3.32, 4.16, 7.18-7-34, 7.47.

### 6-Methyl-6,7, 8,9-tetrahydro-1,3-dioxa-7-aza-cyclopenta[a]naphthalene

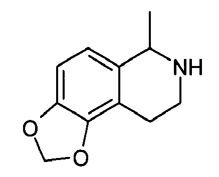

In close analogy to the procedure described above, 6-Methyl-8,9-dihydro-1,3-dioxa-7-azacyclopenta[a]naphthalene is reacted with sodium triacetoxyborohydride to provide the title compound.
LC/MS: m/z = 191 (MH⁺)
¹H NMR (CDCl₃) δ: 1.42, 1.62, 2.68, 2.98, 3.27, 4.02, 5.92, 6.64.

### 8-Methyl-2,3, 5,6,7,8-hexahydro-1,4-dioxa-7-aza-phenanthrene

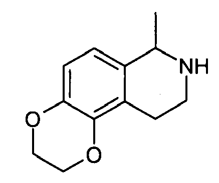

In close analogy to the procedure described above, 8-methyl-2,3,5,6-tetrahydro-1,4-dioxa-7-aza-phenanthrene is reacted with sodium triacetoxyborohydride to provide the title compound.
LC/MS: m/z = 205 (MH⁺)
¹H NMR (CDCl₃) δ: 1.43, 2.49, 2.66, 2.99, 3.25, 4.05, 4.24, 6.63, 6.71.

### General Scheme:

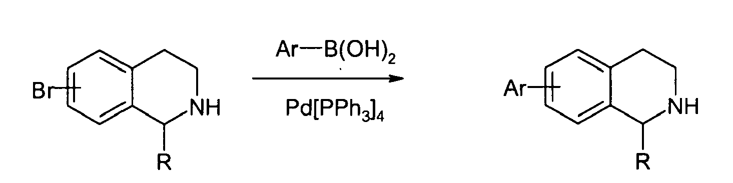

1 mmol of either isomer of bromo-1,2,3,4-tetrahydro-isoquinoline, 2 eq. of corresponding boronic acid, and 12,5 mol % of Pd(PPh₃)₄ are placed in a microwave reactor vial (Personal Chemistry). 3 ml of THF and 2 eq of K₂CO₃ in 1 ml H₂O are added via syringe and the vial is purged with argon. The vial is heated by microwaves at 120 °C for 40 minutes. After cooling the mixture is partitioned between CH₂Cl₂ and water (30 mL each). The organic layer is washed with water, brine, dried over anhydrous Na₂SO₄, filtered and evaporated at reduced pressure. The residue is dissolved in diethyl ether (2 mL) and treated with excess of ethereal solution of hydrogen chloride. The precipitate is collected by filtration, washed and dried to give sufficiently pure title compound as a hydrochloride salt.

### 5-Pyridin-4-yl-1,2,3,4-tetrahydro-isoquinoline

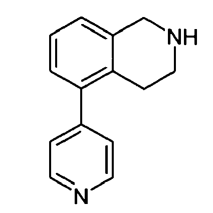

In close analogy to the procedure described above, 5-Bromo-1,2,3,4-tetrahydro-isoquinoline is reacted with the corresponding boronic acid to provide the title compound.
LC/MS: m/z = 211 (MH⁺)

### 6-Pyridin-4-yl-1,2,3,4-tetrahydro-isoquinoline

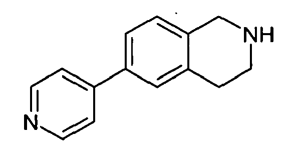

In close analogy to the procedure described above, 6-Bromo-1,2,3,4-tetrahydro-isoquinoline is reacted with the corresponding boronic acid to provide the title compound.
LC/MS: m/z = 211 (MH⁺)

### 7-Pyridin-4-yl-1,2,3,4-tetrahydro-isoquinoline

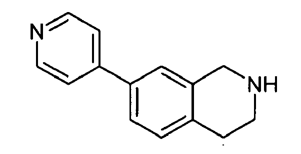

In close analogy to the procedure described above, 7-Bromo-1,2,3,4-tetrahydro-isoquinoline is reacted with the corresponding boronic acid to provide the title compound.
LC/MS: m/z = 211 (MH⁺)

### 5-Pyridin-3 -yl- 1,2,3,4-tetrahydro-isoquinoline

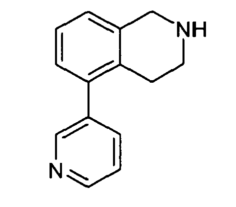

In close analogy to the procedure described above, 5-Bromo-1,2,3,4-tetrahydro-isoquinoline is reacted with the corresponding boronic acid to provide the title compound.
LC/MS: m/z = 211 (MH⁺)

### 6-Pyridin-3-yl-1,2,3,4-tetrahydro-isoquinoline

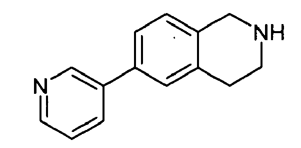

In close analogy to the procedure described above, 6-Bromo-1,2,3,4-tetrahydro-isoquinoline is reacted with the corresponding boronic acid to provide the title compound.
LC/MS: m/z = 211 (MH⁺)

### 7-Pyridin-3-yl-1,2,3,4-tetrahydro-isoquinoline

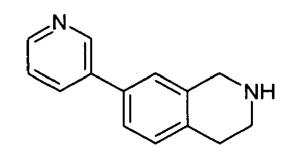

In close analogy to the procedure described above, 7-Bromo-1,2,3,4-tetrahydro-isoquinoline is reacted with the corresponding boronic acid to provide the title compound.
LC/MS: m/z = 211 (MH⁺)

### 1-Methyl-5-pyridin-4-yl-1,2,3,4-tetrahydro-isoquinoline

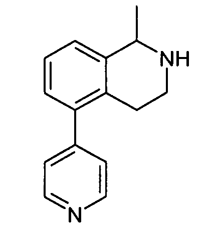

In close analogy to the procedure described above, 5-Bromo-1-methyl-1,2,3,4-tetrahydro-isoquinoline is reacted with the corresponding boronic acid to provide the title compound.
LC/MS: m/z = 225 (MH⁺)

### 1-Methyl-6-pyridin-4-yl-1,2,3,4-tetrahydro-isoquinoline

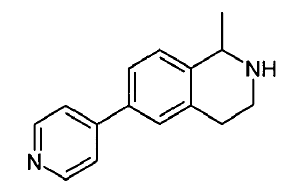

In close analogy to the procedure described above, 6-Bromo-1-methyl-1,2,3,4-tetrahydro-isoquinoline is reacted with the corresponding boronic acid to provide the title compound.
LC/MS: m/z = 225 (MH⁺)

### 1-Methyl-7-pyridin-4-yl-1,2,3,4-tetrahydro-isoquinoline

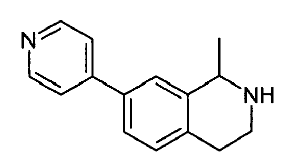

In close analogy to the procedure described above, 7-Bromo-1-methyl-1,2,3,4-tetrahydro-isoquinoline is reacted with the corresponding boronic acid to provide the title compound.
LC/MS: m/z = 225 (MH⁺)

### 1-Methyl-5-pyridin-3-yl-1,2,3,4-tetrahydro-isoquinoline

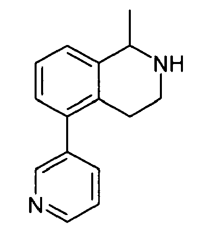

In close analogy to the procedure described above, 5-Bromo-1-methyl-1,2,3,4-tetrahydro-isoquinoline is reacted with the corresponding boronic acid to provide the title compound.
LC/MS: m/z = 225 (MH⁺)

### 1-Methyl-6-pyridin-3-yl-1,2,3,4-tetrahydro-isoquinoline

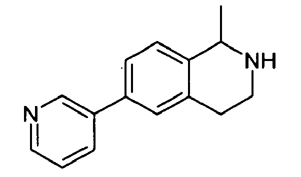

In close analogy to the procedure described above, 6-Bromo-1-methyl-1,2,3,4-tetrahydro-isoquinoline is reacted with the corresponding boronic acid to provide the title compound.
LC/MS: m/z = 225 (MH⁺)

### 1-Methyl-7-pyridin-3-yl-1,2,3,4-tetrahydro-isoquinoline

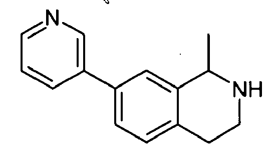

In close analogy to the procedure described above, 7-Bromo-1-methyl-1,2,3,4-tetrahydro-isoquinoline is reacted with the corresponding boronic acid to provide the title compound.
LC/MS: m/z = 225 (MH⁺)

### 1-Methyl-5-pyrimidin-5-yl-1,2,3,4-tetrahydro-isoquinoline

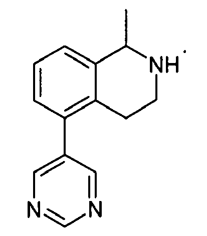

In close analogy to the procedure described above, 5-Bromo-1-methyl-1,2,3,4-tetrahydro-isoquinoline is reacted with the corresponding boronic acid to provide the title compound.
LC/MS: m/z = 226 (MH⁺)

### 1-Methyl-6-pyrimidin-5-yl-1,2,3,4-tetrahydro-isoquinoline

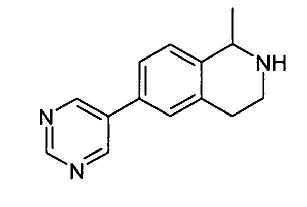

In close analogy to the procedure described above, 6-Bromo-1-methyl-1,2,3,4-tetrahydro-isoquinoline is reacted with the corresponding boronic acid to provide the title compound.
LC/MS: m/z = 226 (MH⁺)

### 1-Methyl-7-pyrimidin-5-yl-1,2,3,4-tetrahydro-isoquinoline

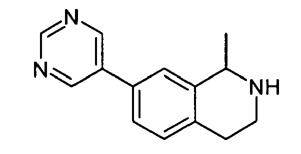

In close analogy to the procedure described above, 7-Bromo-1-methyl-1,2,3,4-tetrahydro-isoquinoline is reacted with the corresponding boronic acid to provide the title compound.
LC/MS: m/z = 226 (MH⁺)

### 5-(6-Methoxy-pyridin-3-yl)-1-methyl-1,2,3,4-tetrahydro-isoquinoline

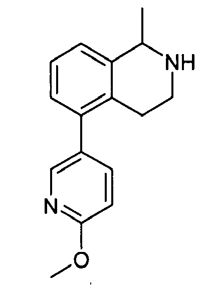

In close analogy to the procedure described above, 5-Bromo-1-methyl-1,2,3,4-tetrahydro-isoquinoline is reacted with the corresponding boronic acid to provide the title compound.
LC/MS: m/z = 255 (MH⁺)

### 6-(6-Methoxy-pyridin-3-yl)-1-methyl-1,2,3,4-tetrahydro-isoquinoline

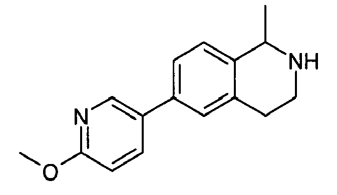

In close analogy to the procedure described above, 6-Bromo-1-methyl-1,2,3,4-tetrahydro-isoquinoline is reacted with the corresponding boronic acid to provide the title compound.
LC/MS: m/z = 255 (MH⁺)

### 7-(6-Methoxy-pyridin-3-yl)-1-methyl-1,2,3,4-tetrahydro-isoquinoline

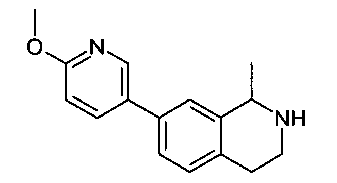

In close analogy to the procedure described above, 7-Bromo-1-methyl-1,2,3,4-tetrahydro-isoquinoline is reacted with the corresponding boronic acid to provide the title compound.
LC/MS: m/z = 255 (MH⁺)

### 5-(2-Methoxy-pyridin-3-yl)-1-methyl-1,2,3,4-tetrahydro-isoquinoline

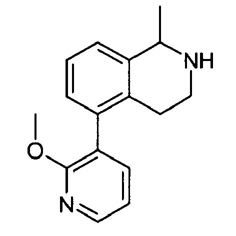

In close analogy to the procedure described above, 5-Bromo-1-methyl-1,2,3,4-tetrahydro-isoquinoline is reacted with the corresponding boronic acid to provide the title compound.
LC/MS: m/z = 255 (MH⁺)

### 6-(2-Methoxy-pyridin-3-yl)-1-methyl-1,2,3,4-tetrahydro-isoquinoline

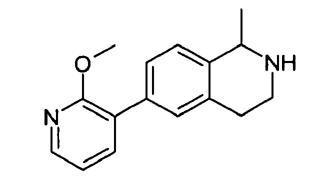

In close analogy to the procedure described above, 6-Bromo-1-methyl-1,2,3,4-tetrahydro-isoquinoline is reacted with the corresponding boronic acid to provide the title compound.
LC/MS: m/z = 255 (MH⁺)

### 7-(2-Methoxy-pyridin-3-yl)-1-methyl-1,2,3,4-tetrahydro-isoquinoline

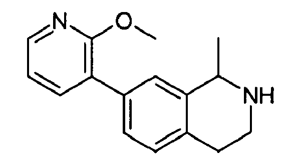

In close analogy to the procedure described above, 7-Bromo-1-methyl-1,2,3,4-tetrahydro-isoquinoline is reacted with the corresponding boronic acid to provide the title compound.
LC/MS: m/z = 255 (MH⁺)

### 5-(2-Fluoro-pyridin-3-yl)-1-methyl-1,2,3,4-tetrahydro-isoquinoline

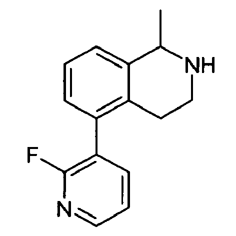

In close analogy to the procedure described above, 5-Bromo-1-methyl-1,2,3,4-tetrahydro-isoquinoline is reacted with the corresponding boronic acid to provide the title compound.
LC/MS: m/z = 243 (MH⁺)

### 6-(2-Fluoro-pyridin-3-yl)-1-methyl-1,2,3,4-tetrahydro-isoquinoline

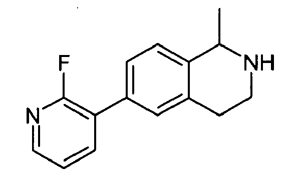

In close analogy to the procedure described above, 6-Bromo-1-methyl-1,2,3,4-tetrahydro-isoquinoline is reacted with the corresponding boronic acid to provide the title compound.
LC/MS: m/z = 243 (MH⁺)

### 7-(2-Fluoro-pyridin-3-yl)-1-methyl-1,2,3,4-tetrahydro-isoquinoline

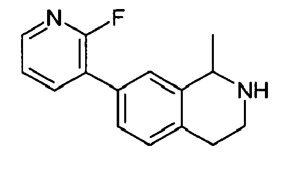

In close analogy to the procedure described above, 7-Bromo-1-methyl-1,2,3,4-tetrahydro-isoquinoline is reacted with the corresponding boronic acid to provide the title compound.
LC/MS: m/z = 243 (MH⁺)

### 5-(6-Fluoro-pyridin-3-yl)-1-methyl-1,2,3,4-tetrahydro-isoquinoline

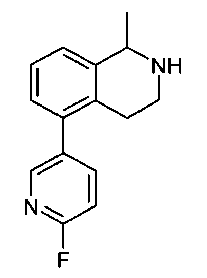

In close analogy to the procedure described above, 5-Bromo-1-methyl-1,2,3,4-tetrahydro-isoquinoline is reacted with the corresponding boronic acid to provide the title compound.
LC/MS: m/z = 243 (MH⁺)

### 6-(6-Fluoro-pyridin-3-yl)-1-methyl-1,2,3,4-tetrahydro-isoquinoline

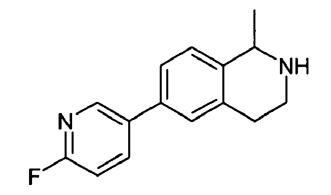

In close analogy to the procedure described above, 6-Bromo-1-methyl-1,2,3,4-tetrahydro-isoquinoline is reacted with the corresponding boronic acid to provide the title compound.
LC/MS: m/z = 243 (MH⁺)

### 7-(6-Fluoro-pyridin-3-yl)-1-methyl-1,2,3,4-tetrahydro-isoquinoline

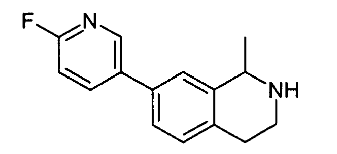

In close analogy to the procedure described above, 7-Bromo-1-methyl-1,2,3,4-tetrahydro-isoquinoline is reacted with the corresponding boronic acid to provide the title compound.
LC/MS: m/z = 243 (MH⁺)

### 5-(2,6-Dimethoxy-pyridin-3-yl)-1-methyl-1,2,3,4-tetrahydro-isoquinoline

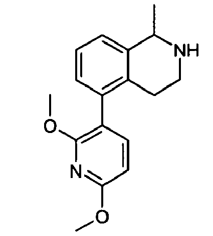

In close analogy to the procedure described above, 5-Bromo-1-methyl-1,2,3,4-tetrahydro-isoquinoline is reacted with the corresponding boronic acid to provide the title compound.
LC/MS: m/z = 285 (MH⁺)

### 6-(2,6-Dimethoxy-pyridin-3-yl)-1-methyl-1,2,3,4-tetrahydro-isoquinoline

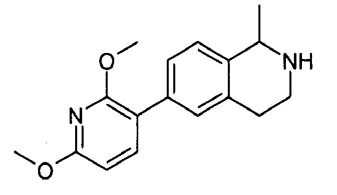

In close analogy to the procedure described above, 6-Bromo-1-methyl-1,2,3,4-tetrahydro-isoquinoline is reacted with the corresponding boronic acid to provide the title compound.
LC/MS: m/z = 285 (MH⁺)

### 7-(2,6-Dimethoxy-pyridin-3-yl)-1-methyl-1,2,3,4-tetrahydro-isoquinoline

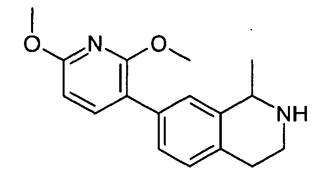

In close analogy to the procedure described above, 7-Bromo-1-methyl-1,2,3,4-tetrahydro-isoquinoline is reacted with the corresponding boronic acid to provide the title compound.
LC/MS: m/z = 285 (MH⁺)

### 5-(2,4-Dimethoxy-pyrimidin-5-yl)-1-methyl-1,2,3,4-tetrahydro-isoquinoline

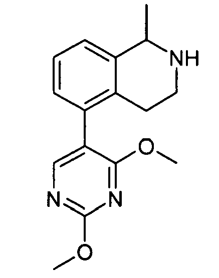

In close analogy to the procedure described above, 5-Bromo-1-methyl-1,2,3,4-tetrahydro-isoquinoline is reacted with the corresponding boronic acid to provide the title compound.
LC/MS: m/z = 286 (MH⁺)

### 6-(2,4-Dimethoxy-pyrimidin-5-yl)-1-methyl-1,2,3,4-tetrahydro-isoquinoline

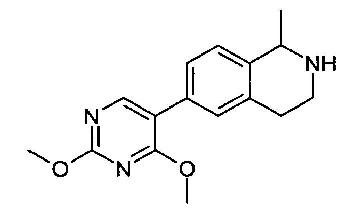

In close analogy to the procedure described above, 6-Bromo-1-methyl-1,2,3,4-tetrahydro-isoquinoline is reacted with the corresponding boronic acid to provide the title compound.
LC/MS: m/z = 286 (MH⁺)

### 7-(2,4-Dimethoxy-pyrimidin-5-yl)-1-methyl-1,2,3,4-tetrahydro-isoquinoline

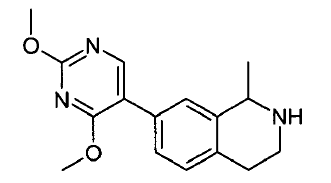

In close analogy to the procedure described above, 7-Bromo-1-methyl-1,2,3,4-tetrahydro-isoquinoline is reacted with the corresponding boronic acid to provide the title compound.
LC/MS: m/z = 286 (MH⁺)

### General Scheme:

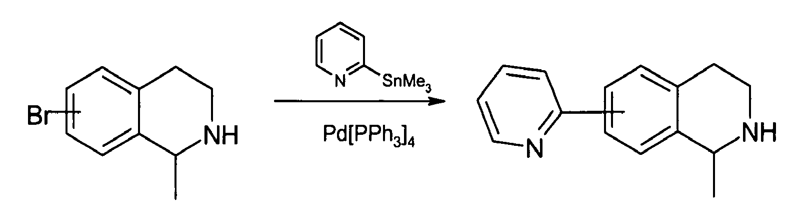

1 mmol of either isomer of bromo-tetrahydroisoquinoline, 1.5 mmol of 2-trimethylstannyl-pyridine, 5 mol.% of Pd(PPh₃)₄ and 5 mol.% of AsPh₃ in 7 ml of toluene are heated at 120 °C for 48 hrs. The reaction mixture is cooled and aqueous 2M K₂CO₃ solution (5 mL) is added. The mixture is partitioned between EtOAc and water. The organic layer is dried over Na₂SO₄, filtered through Celite and evaporated. The residue is dissolved in ether (2 mL) and treated with excess ethereal hydrogen chloride solution. The precipitate is filtered, washed with ether and dried to give sufficiently pure title compound as a hydrochloride salt.

### 1-Methyl-7-pyridin-2-yl-1,2,3,4-tetrahydro-isoquinoline

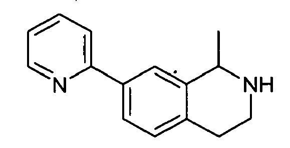

In close analogy to the procedure described above, 7-Bromo-1-methyl-1,2,3,4-tetrahydro-isoquinoline is reacted with 2-trimethylstannyl-pyridine to provide the title compound.
LC/MS: m/z = 225 (MH⁺)

### 1-Methyl-6-pyridin-2-yl-1,2,3,4-tetrahydro-isoquinoline

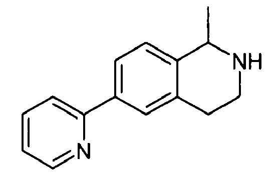

In close analogy to the procedure described above, 6-Bromo-1-methyl-1,2,3,4-tetrahydro-isoquinoline is reacted with 2-trimethylstannyl-pyridine to provide the title compound.
LC/MS: m/z = 225 (MH⁺)

### 1-Methyl-5-pyridin-2-yl-1,2,3,4-tetrahydro-isoquinoline

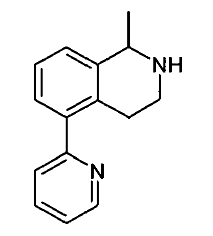

In close analogy to the procedure described above, 5-Bromo-1-methyl-1,2,3,4-tetrahydro-isoquinoline is reacted with 2-trimethylstannyl-pyridine to provide the title compound.
LC/MS: m/z = 225 (MH⁺)

### General Scheme:

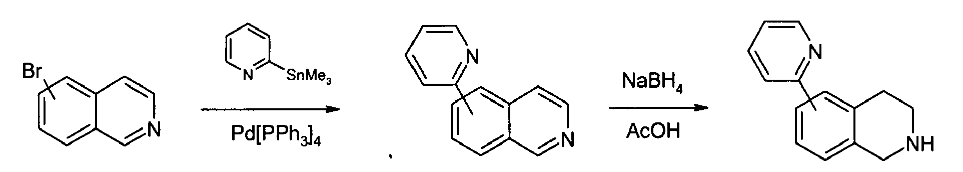

1 mmol of either isomer of bromo-isoquinoline, 1.5 mmol of 2-trimethylstannyl-pyridine, 5 mol.% of Pd(PPh₃)₄ and 5 mol.% of AsPh₃ in 7 ml of toluene are heated at 120 °C for 48 hrs. The reaction mixture is cooled and aqueous 2M K₂CO₃ solution (5 mL) is added. The mixture is partitioned between EtOAc and water. The organic layer is dried over Na₂SO₄, filtered through Celite and evaporated. The residue is purified by flash chromatography (eluent hexane/EtOAc = 1:1 to 1:3).

The purified product is dissolved in acetic acid and 3.5 eq of NaBH₄ is added portionwise. The reaction mixture is stirred for 1 h, then poured into ice and NH₄OH is added to pH 9. The mixture is extracted with CH₂Cl₂ and the organic layer is dried over Na₂SO₄, filtered and evaporated to give sufficiently pure title compound.

### 7-Pyridin-2-yl-1,2,3,4-tetrahydro-isoquinoline

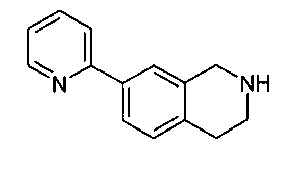

In close analogy to the procedure described above, 7-Bromo-isoquinoline is reacted with 2-trimethylstannyl-pyridine and sodium borohydride to provide the title compound.
LC/MS: m/z = 211 (MH⁺)

### 6-Pyridin-2-yl-1,2,3,4-tetrahydro-isoquinoline

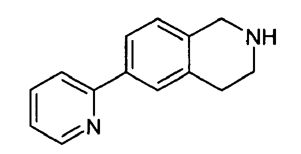

In close analogy to the procedure described above, 6-Bromo-isoquinoline is reacted with 2-trimethylstannyl-pyridine and sodium borohydride to provide the title compound.
LC/MS: m/z = 211 (MH⁺)

### 5-Pyridin-2-yl-1,2,3,4-tetrahydro-isoquinoline

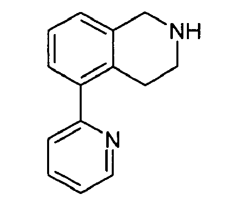

In close analogy to the procedure described above, 5-Bromo-isoquinoline is reacted with 2-trimethylstannyl-pyridine and sodium borohydride to provide the title compound.
LC/MS: m/z = 211 (MH⁺)

### 7-Bromo-3,4-dihydro-1H-isoquinoline-2-carboxylic acid tert-butyl ester

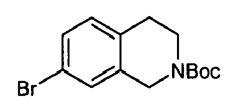

7-Bromotetrahydroisoquinoline (1 mmol) is dissolved in CH₂Cl₂ (5 mL) and treated with 1.2 eq. of Boc₂O in the presence of 0.1 eq. of DMAP. The reaction mixture is left overnight stirring overnight. The solution is washed with 1M HCl, aq. NaHCO₃, and brine. The organic layer is dryed over Na₂SO₄, filtered and evaporated. The product is purified by flash chromatography (eluent hexane:EtOAc = 5:1).
LC/MS: m/z = 313 (MH⁺)
¹H NMR (CDCl₃) δ:1.48, 2.77, 3.62, 4.53, 7.02, 7.25, 7.29.

### 7-Morpholin-4-yl-3,4-dihydro-1H-isoquinoline-2-carboxylic acid tert-butyl ester

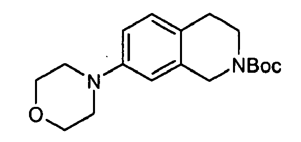

The purified N-Boc protected 7-bromotetrahydroisoquinoline, 0.1 eq. Pd₂(dba)₃ 0.15 eq. BINAP, 2.0 eq. Cs₂CO₃, 1,5 eq. of morpholine are dissolved in toluene (3 mL) and the mixture is heated at 120 °C for 48 hrs, cooled and evaporated at reduced pressure. The residue is dissolved in EtOAc and washed with water and brine. Organic layer is washed with water and brine, dried over Na₂SO₄, filtered and evaporated. The crude product is purified by flash chromatography (eluent hexane:EtOAc = 3:1).
LC/MS: m/z = 319 (MH⁺)
¹H NMR (CDCl₃) δ:1.48, 2.75, 3.11, 3.62, 3.85, 4.53, 6.64, 6.76, 7.04.

### 7-Morpholin-4-yl-1,2,3,4-tetrahydro-isoquinoline

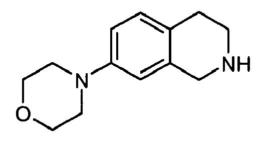

The obtained compound is dissolved in CH₂Cl₂ containing 20 % TFA, and left stirring for 1 h. The reaction mixture is washed with aq. NaHCO₃, water and brine, the organic layer is dryed over Na₂SO₄, filtered and evaporated to give sufficiently pure title compound as colorless oil.
LC/MS: m/z = 220 (MH⁺)
¹H NMR (CDCl₃) δ: 2.71, 3.10, 3.85, 3.97, 6.55, 6.74, 7.00.

### General Scheme:

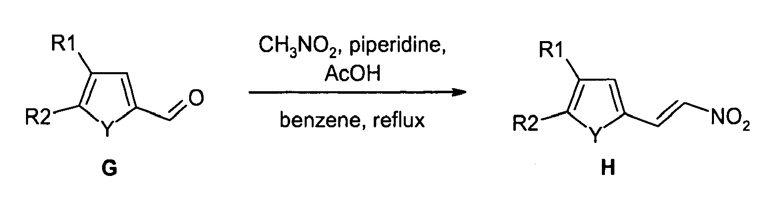

To a solution of aldehyde G (1 mol) in benzene (approx. 20 ml per 1 g of the starting compound) nitromethane (1.2 mol), piperidine (0.1 mol) and acetic acid (0.1 mol) were added. The reaction mixture is refluxed for 2.5 h with azeotrope removing of water. Then, the solvent is evaporated in vacuo. The residue is triturated with cold methanol, filtered, washed with cold methanol that resulted in product crystals in good to moderate yields.

### 2-Methyl-5-(2-nitro-vinyl)-thiophene

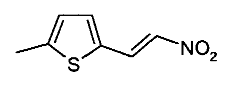

In close analogy to the procedure described above, 5-Methyl-thiophene-2-carbaldehyde is reacted with nitromethane to provide the title compound.
Yield: 76%
LC/MS: m/z = 170 (MH⁺)

### 4-Methyl-2-(2-nitro-vinyl)-thiophene

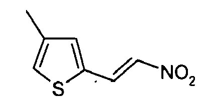

In close analogy to the procedure described above, 4-Methyl-thiophene-2-carbaldehyde is reacted with nitromethane to provide the title compound.
Yield: 78%
LC/MS: m/z = 170 (MH⁺)

### 2,3-Dimethyl-5-(2-nitro-vinyl)-thiophene

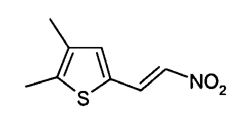

In close analogy to the procedure described above, 2,3-dimethyl-thiophene-2-carbaldehyde is reacted with nitromethane to provide the title compound.
Yield: 58%
LC/MS: m/z = 184 (MH⁺)

### 2-Methyl-5-(2-nitro-vinyl)-furan

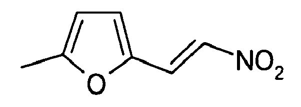

In close analogy to the procedure described above, 5-Methyl-furan-2-carbaldehyde is reacted with nitromethane to provide the title compound.
Yield: 88%
LC/MS: m/z = 154 (MH⁺)
¹H NMR (CDCl₃) δ: 2.25, 2.39, 4.37-4.40, 4.79, 6.21, 6.81, 7.48, 7.71.

### General Scheme:

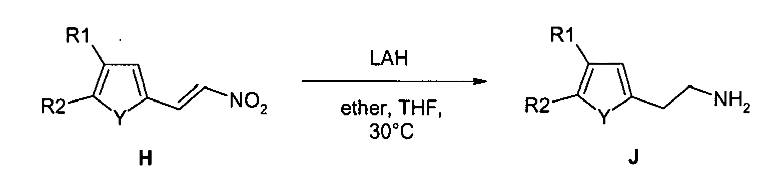

To a suspension of lithium aluminium hydride (2 mol) in diethyl ether (approx. 20 ml per 1 g of LAH) solution of the corresponding nitro derivative H (1 mol) in dry THF is added dropwise at stirring within 30 min. The reaction mixture is stirred at 35°C for 2h, and then, cooled with ice water. Then, 20% aqueous NaOH and water (n ml, n ml, 3n ml per n g LAH) were added step by step carefully. The resulting mixture is stirred for 30 min, filtered and concentrated on rotary evaporator. The residue is purified by flash column chromatography on silica gel (eluent: chloroform/methanol - 0-20%) that gave products in good to moderate yields.

### 2-(5-Methyl-thiophen-2-yl)-ethylamine

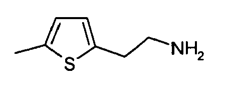

In close analogy to the procedure described above, 2-Methyl-5-(2-nitro-vinyl)-thiophene is reacted with lithium aluminium hydride to provide the title compound.
Yield: 85%
LC/MS: m/z = 142 (MH⁺)

### 2-(4-Methyl-thiophen-2-yl)-ethylamine

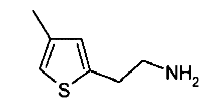

In close analogy to the procedure described above, 4-Methyl-2-(2-nitro-vinyl)-thiophene is reacted with lithium aluminium hydride to provide the title compound.
Yield: 85%
LC/MS: m/z = 142 (MH⁺)

### 2-(4,5-Dimethyl-thiophen-2-yl)-ethylamine

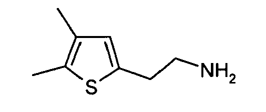

In close analogy to the procedure described above, 2,3-Dimethyl-5-(2-nitro-vinyl)-thiophene is reacted with lithium aluminium hydride to provide the title compound.
Yield: 55%
LC/MS: m/z = 156 (MH⁺)

### 2-(5-Methyl-furan-2-yl)-ethylamine

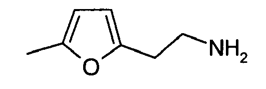

In close analogy to the procedure described above, 2-Methyl-5-(2-nitro-vinyl)-furan is reacted with lithium aluminium hydride to provide the title compound.
LC/MS: m/z = 126 (MH⁺)

### General Scheme:

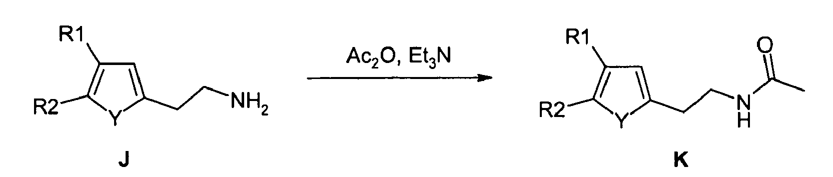

To a solution of the amine J (1 mol) in CH₂Cl₂ (approx. 10 ml per 1 g of the starting compound) triethylamine (1.1 mol) and acetic anhydride (1.2 mol) were added. The reaction mixture is stirred at room temperature for 1h and concentrated on rotary evaporator. The residue is triturated with hexane, filtered, washed with hexane to give a crystalline compound in good yields.

### N-(2-Thiophen-2-yl-ethyl)-acetamide

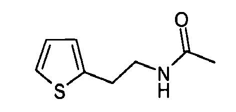

In close analogy to the procedure described above, 2-Thiophen-2-yl-ethylamine is reacted with acetic anhydride to provide the title compound.
Yield: 90%
LC/MS: m/z = 170 (MH⁺)

### N-[2-(5-Methyl-thiophen-2-yl)-ethyl]-acetamide

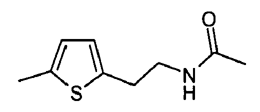

In close analogy to the procedure described above, 2-(5-Methyl-thiophen-2-yl)-ethylamine is reacted with acetic anhydride to provide the title compound.
Yield: 78%
LC/MS: m/z = 184 (MH⁺)

### N-[2-(4-Methyl-thiophen-2-yl)-ethyl]-acetamide

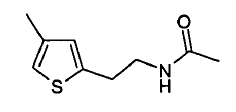

In close analogy to the procedure described above, 2-(4-Methyl-thiophen-2-yl)-ethylamine is reacted with acetic anhydride to provide the title compound.
Yield: 95%
LC/MS: m/z = 184 (MH⁺)

N-[2-(4,5-Dimethyl-thiophen-2-yl)-ethyl]-acetamide

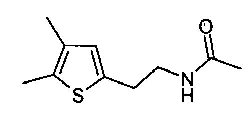

In close analogy to the procedure described above, 2-(4,5-Dimethyl-thiophen-2-yl)-ethylamine is reacted with acetic anhydride to provide the title compound.
Yield: 79%
LC/MS: m/z = 198 (MH⁺)

### N-(2-Furan-2-yl-ethyl)-acetamide

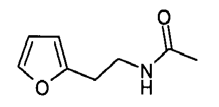

In close analogy to the procedure described above, 2-Furan-2-yl-ethylamine is reacted with acetic anhydride to provide the title compound.
Yield: 41 %
LC/MS: m/z = 154 (MH⁺)
¹H NMR (CDCl₃) δ: 2.05, 2.85, 3.53, 5.68, 6.07, 6.31, 7.34

### 2,2,2-Trifluoro-N-(2-thiophen-2-yl-ethyl)-acetamide

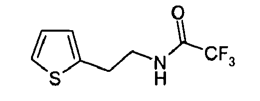

In close analogy to the procedure d, escribed above, 2-Thiophen-2-yl-ethylamine is reacted with ethyl trifluoracetate to provide the. title compound.
Yield: 86%
LC/MS: m/z = 224 (MH⁺)

### N-[2-(5-Methyl-furan-2-yl)-ethyl]-acetamide

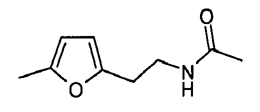

In close analogy to the procedure described above, 2-(5-Methyl-furan-2-yl)-ethylamine is reacted with acetic anhydride to provide the title compound.
Yield: 55%
LC/MS: m/z = 167 (MH⁺)
¹H NMR (d₆-DMSO) δ: 1.97, 2.26, 2.78, 3.51, 5.62, 5.86, 5.94

### General Scheme:

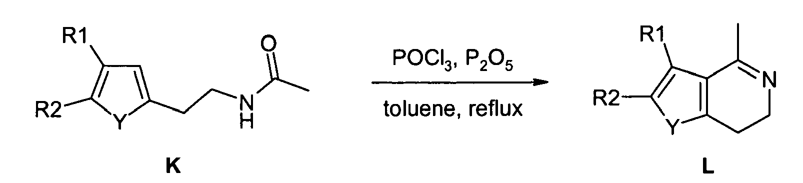

To a solution of the amide K (1 mol) in toluene (approx. 10 ml per 1 g of the starting compound) phosphorus pentoxide (approx. 1.5 g per 1 g of the starting compound) and phosphorus oxychloride (2-3 mol) were added. The reaction mixture is refluxed for 3h and cooled to 0°C. Some ice is carefully added to the reaction mixture, and it is stirred for 1 h. Then, the mixture is filtered. The organic layer is separated and washed with water. The combined aqueous layers were washed with benzene, then alkalified with 25% sodium hydroxide, extracted with benzene, dried over Na₂SO₄ and concentrated on rotary evaporator. Resulting oil is used in the next step without purification.

### 4-Methyl-6,7-dihydro-thieno[3,2-c]pyridine

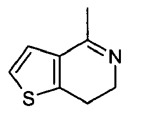

In close analogy to the procedure described above, N-(2-Thiophen-2-yl-ethyl)-acetamide is reacted with phosphorus pentoxide to provide the title compound.
LC/MS: m/z = 152 (MH⁺)

### 2,4-Dimethyl-6,7-dihydro-thieno[3,2-c]pyridine

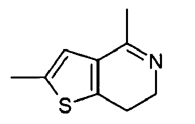

In close analogy to the procedure described above, N-[2-(5-Methyl-thiophen-2-yl)-ethyl]-acetamide is reacted with phosphorus pentoxide to provide the title compound.
LC/MS: m/z = 166 (MH⁺)

### 3,4-Dimethyl-6,7-dihydro-thieno[3,2-c]pyridine

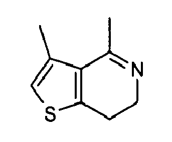

In close analogy to the procedure described above, N-[2-(4-Methyl-thiophen-2-yl)-ethyl]-acetamide is reacted with phosphorus pentoxide to provide the title compound.
LC/MS: m/z = 167 (MH⁺)

### 2,3,4-Trimethyl-6,7-dihydro-thieno[3,2-c]pyridine

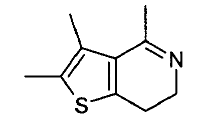

In close analogy to the procedure described above, N-[2-(4,5-Dimethyl-thiophen-2-yl)-ethyl]-acetamide is reacted with phosphorus pentoxide to provide the title compound.
LC/MS: m/z = 180 (MH⁺)

### 4-Methyl-6,7-dihydro-furo[3,2-c]pyridine

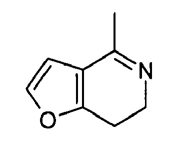

In close analogy to the procedure described above, N-(2-Furan-2-yl-ethyl)-acetamide is reacted with phosphorus pentoxide to provide the title compound.
Yield: 94%
LC/MS: m/z = 136 (MH⁺)

### 4-Methyl-6,7-dihydro-thieno[3,2-c]pyridine

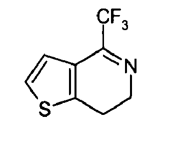

In close analogy to the procedure described above, 2,2,2-Trifluoro-N-(2-thiophen-2-yl-ethyl)-acetamide is reacted with phosphorus pentoxide to provide the title compound.
Yield: 25%
LC/MS: m/z = 206 (MH⁺)

### 2,4-Dimethyl-6,7-dihydro-furo[3,2-c]pyridine

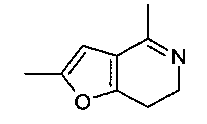

In close analogy to the procedure described above, N-[2-(5-Methyl-furan-2-yl)-ethyl]-acetamide is reacted with phosphorus pentoxide to provide the title compound.
LC/MS: m/z = 150 (MH⁺)
¹H NMR (CDCl₃) δ: 2.18, 2.30, 2.70, 3.84, 3.88, 6.01.

### General Scheme:

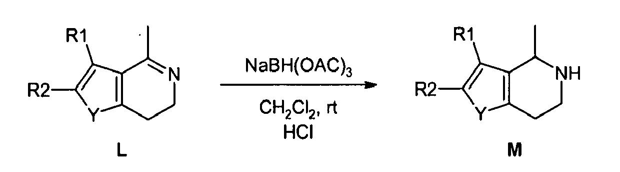

To a solution of compound L (1 mol) in CH₂Cl₂ (approx. 20 ml per 1 g of the starting compound) sodium triacetoxyborohydride (1.5 mol) is added. The reaction mixture is stirred for 12h at room temperature. The reaction mixture is poured into saturated sodium bicarbonate solution and stirred for 30 min. The organic layer is separated, and aqueous layer is extracted with chloroform. The combined organic phases were dried over Na₂SO₄ and concentrated on rotary evaporator. The residue is purified by flash column chromatography on silica gel (eluent: chloroform/methanol - 0→20%) to give yellowish oil. Then, 6 N isopropanolic HCl is added to the oil, stirred for 5 min, concentrated on rotary evaporator, triturated with diethyl ether, filtered off, washed with ether and dried in vacuo to give crystalline compound in acceptable yields on 2 steps.

### 4-Methyl-4,5,6,7-tetrahydro-thieno[3,2-c]pyridine

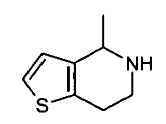

In close analogy to the procedure described above, 4-Methyl-6,7-dihydro-thieno[3,2-c]pyridine is reacted with sodium triacetoxyborohydride to provide the title compound.
Yield: 22% for two steps
LC/MS: m/z = 155 (MH⁺)
¹H NMR (d₆-DMSO) δ: 1.59, 2.95-3.57, 4.49-4.52, 6.99, 7.42, 9.70.

### 2,4-Dimethyl-4,5,6,7-tetrahydro-thieno[3,2-c]pyridine

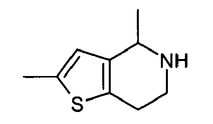

In close analogy to the procedure described above, 2,4-Dimethyl-6,7-dihydro-thieno[3,2-c]pyridine is reacted with sodium triacetoxyborohydride to provide the title compound.
Yield: 14% for two steps
LC/MS: m/z = 169 (MH⁺)
¹H NMR (d₆-DMSO) δ: 1.51, 2.48, 2.83-3.55, 6.65, 9.62.

### 3,4-Dimethyl-4,5,6,7-tetrahydro-thieno[3,2-c]pyridine

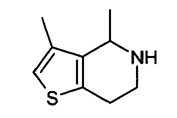

In close analogy to the procedure described above, 3,4-Dimethyl-6,7-dihydro-thieno[3,2-c]pyridine is reacted with sodium triacetoxyborohydride to provide the title compound.
Yield: 8% for two steps
LC/MS: m/z = 169 (MH⁺)

### 2,3,4-Trimethyl-4,5,6,7-tetrahydro-thieno[3,2-c]pyridine

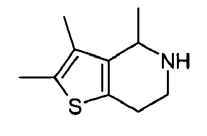

In close analogy to the procedure described above, 2,3,4-Trimethyl-6,7-dihydro-thieno[3,2-c]pyridine is reacted with sodium triacetoxyborohydride to provide the title compound.
Yield: 14% for two steps
LC/MS: m/z = 183 (MH⁺)
¹H NMR (d₆-DMSO) δ: 1.68, 2.00, 2.28, 2.89-3.60, 4.50-4.61, 10.09

### 4-Methyl-4,5,6,7-tetrahydro-furo[3,2-c]pyridine

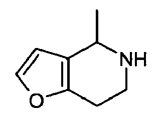

In close analogy to the procedure described above, 4-Methyl-6,7-dihydro-furo[3,2-c]pyridine is reacted with sodium triacetoxyborohydride to provide the title compound.
LC/MS: m/z = 138 (MH⁺)

### 4-Methyl-4,5,6,7-tetrahydro-thieno[3,2-c]pyridine

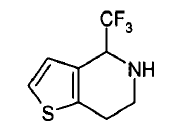

In close analogy to the procedure described above, 4-Methyl-6,7-dihydro-thieno[3,2-c]pyridine is reacted with sodium triacetoxyborohydride to provide the title compound.
Yield: 25%
LC/MS: m/z = 206 (MH⁺)

### 2,4-Dimethyl-4,5,6,7-tetrahydro-furo[3,2-c]pyridine

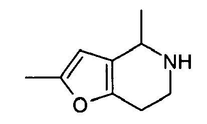

In close analogy to the procedure described above, 2,4-Dimethyl-6,7-dihydro-furo[3,2-c]pyridine is reacted with sodium triacetoxyborohydride to provide the title compound.
Yield: 45% for two steps
LC/MS: m/z = 151 (MH⁺)

### 2-(2-Isocyanato-propyl)-thiophene

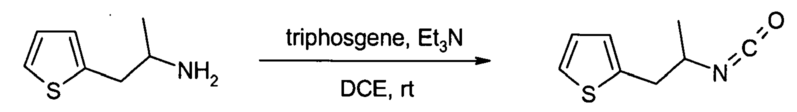

To a solution of 1-methyl-2-thiophen-2-ylethylamine (2 g, 14.2 mmol) in dichloroethane (30 ml) triethylamine (4.3 g, 42.6 mmol) and triphosgene (2.1 g, 7.1 mmol) were added. The reaction mixture stirred for 1h and concentrated on rotary evaporator. Diethyl ether is added, and the resulting mixture is filtered. Ether solution is concentrated, and the resulting oil is used in the next step without purification.

### 6-Methyl-6,7-dihydro-5H-thieno[3,2-c]pyridin-4-one

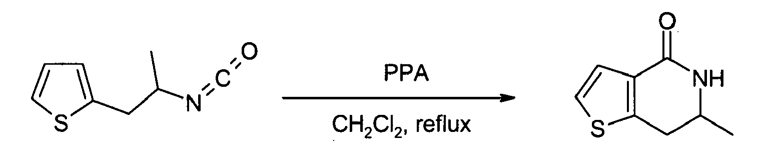

To PPA (15 ml) a solution of 2-(2-isocyanatopropyl)-thiophene in CH₂Cl₂ (15 ml) was added. The reaction mixture was stirred under reflux for 3 h, cooled, poured into ice and alkalified with concentrated ammonia. Then, the mixture was extracted with chloroform, dried over Na₂SO₄ and concentrated on rotary evaporator. The residue was purified by flash column chromatography on silica gel (eluent: chloroform/acetone - 0→30%) to give 0.5 g of the target compound as a white solid.
Yield: 22% for two steps
LC/MS: m/z = 168 (MH⁺)

### 6-Methyl-4,5,6,7-tetrahydro-thieno[3,2-c]pyridine

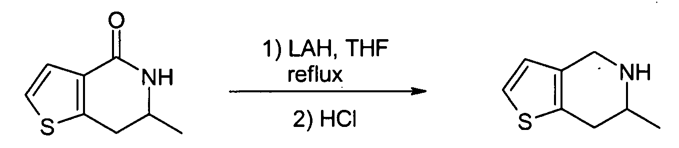

To a suspension of LAH (0.38 g, 10 mmol) in THF (10 ml) a solution of 6-methyl-6,7-dihydro-5H-thieno[3,2-c]pyridin-4-one (0.5 g, 3 mmol) in THF (10 ml) was added dropwise. The mixture was stirred for 3h under reflux and cooled to 0°C with ice. The solution of 0.4 ml of water in 2 ml THF, 25% NaOH (0.4 ml) and water (1.2 ml) were added carefully step by step. The mixture was stirred for 30 min, filtered and concentrated on rotary evaporator. The residue was purified by flash column chromatography on silica gel (eluent: chloroform/methanol - 0→20%) to give yellowish oil. Then, 6 N isopropanolic HCl was added to the oil, stirred for 5 min, concentrated on rotary evaporator, triturated with diethyl ether, filtered, washed with ether and dried in vacuo to give 0.37g of crystalline compound.
Yield: 67%
LC/MS: m/z = 155 (MH⁺)
¹H NMR (d₆-DMSO) δ: 1.40, 2.76-3.19, 3.49-3.65, 4.08-4.37, 6.93, 7.44, 9.69.

### 1-Methyl-3,4-dihydro-pyrrolo[1,2-a]pyrazine

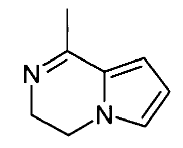

Conc. HCl (8.8 ml) and water (10 ml) are carefully added to ethylene diamine (20 g, 0.33 mol) at stirring and ice bath cooling followed by addition of 2-acetylfuran (16 g, 0.145 mol). The mixture is refluxed for 15 min, then stirred without heating for 1 h, saturated with K₂CO₃ and extracted with dichloromethane (3x50 ml). The combined extracts are washed with 30% K₂CO₃ solution (30 ml), dried over K₂CO₃ and evaporated. The residue is distilled (b.p. 90-92°C/1 mm) to give the title compound. The yield is 12 g (61 %).
m/z (APCI+) 135 (M+H⁺)

### 1-Methyl-1,2,3,4-tetrahydro-pyrrolo[1,2-a]pyrazine

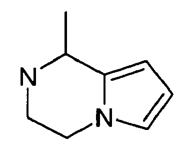

NaBH₄ (1g, 26.3 mmol) is added portionwise to the solution of 1-methyl-3,4-dihydropyrrolo[1,2-a]pyrazine (2.68 g, 20 mmol) in methanol (15 ml) and the mixture is stirred for 1 h at r.t. and evaporated. The residue is dissolved in water (10 ml) and extracted with dichloromethane (3x20 ml). The combined extracts are dried over K₂CO₃ and evaporated. The residue is purified by column chromatography on silica gel (eluent: chloroform-ethanol 0→5%) to give the title compound. The yield is 0.95 g (35%).

### 1,6-Dimethyl-3,4-dihydro-pyrrolo[1,2-a]pyrazine

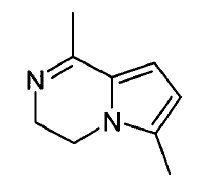

Conc. HCl (8.8 ml) and water (10 ml) are carefully added to ethylene diamine (43 g, 0.725 mol) at stirring and ice-bath cooling followed by addition of 5-methyl-2-acetylfuran (30 g, 0.242 mol). The mixture is refluxed for 15 min, then stirred without heating for 1 h and extracted with dichloromethane (2x50 ml). The combined extracts are washed with water (40 ml), dried over K₂CO₃ and evaporated. The residue is distilled (b.p. 97C/1 mm) to give the title compound. The yield is 30 g (70%).
m/z (APCI+) 149 (M+H⁺)

### 1,6-Dimethyl-1,2,3,4-tetrahydro-pyrrolo[1,2-a]pyrazine

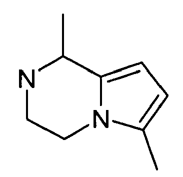

NaBH₄ (1g, 26.3 mmol) is added portionwise to the solution of 1,6-dimethyl-3,4-dihydropyrrolo[1,2-a]pyrazine (3 g, 20.2 mmol) in methanol (15 ml) and the mixture is stirred for 1 h at r.t. and evaporated. The residue is dissolved in water (10 ml) and extracted with dichloromethane (2x20 ml). The combined extracts are dried over K₂CO₃ and evaporated. The residue is purified by column chromatography on silica gel (eluent: chloroform-ethanol 0→5%) to give the title compound. The yield is 2.3 g (78%).

### N,N-Dibenzyl-N-[2-(1H-1,2,3-triazol-1-yl)ethyl]amine

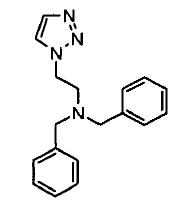

N-(2-Chloroethyl) dibenzylamine hydrochloride (1.0 g, 3.38 mmol, 1.0 equiv) is suspended in t-butanol (25 mL). Added as followed 1,2,3-1H-triazole (391 µl, 6.75 mmol, 2.0 equiv), KI (561 mg, 3.38 mmol, 1.0 equiv), LiCl (286 mg, 6.75 mmol, 2.0 equiv) and NaOtBu (975 mg, 10.1 mmol, 3.0 equiv). The resulting mixture is heated to 90°C for 4 h. Water (40 mL) is added and the mixture is extracted twice with toluene (40 mL). The organic layer is washed sequentially with water (40 mL) and brine (40 mL), dried over Na₂SO₄, filtered off and concentrated in vacuo. The residue is purified by flash column chromatography, (10 to 50% ethyl acetate in heptane) to provide 300 mg (30%) of the title compound.
LC-MS m/z 293 (MH+); ¹H-NMR (400 MHz, CDCl₃): δ (ppm) 1.26, 1.71, 2.95, 6.62, 4.38, 7.21-7.29, 7.38, 7.65.

### N-Methoxy-N-methyl-acetamide

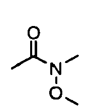

Acetic acid (2.47 mL, 42.8 mmol, 1.0 equiv) and N,O-dimethyl-hydroxylamine hydrochloride (4.60 g, 47.1 mmol, 1.1 equiv) are dissolved in dichloromethane (400 mL) and the mixture is cooled in an ice bath. Triethylamine (6.62 mL, 47.1 mmol, 1.1 equiv), EDCI (9.03 g, 47.1 mmol, 1.1 equiv) and HOAt (0.583 g, 4.28 mmol, 0.1 equiv) are added. The resulting mixture is stirred overnight at room temperature under nitrogen atmosphere. The mixture is successively washed with 1N HCl solution (150 mL), sat. NaHCO₃ solution (150 mL) and brine (100 mL), dried over Na₂SO₄, filtered off and concentrated in vacuo, co-evaporation with diethyl ether to yield 2.38 g (54%) of the title compound as a colorless oil.
GC-MS m/z 103; ¹H-NMR (400 MHz, CDCl₃): δ (ppm) 2.13, 3.18, 3.70.

### 1-[3-(2-Dibenzylamino-ethyl)-3H-[1,2,3]triazol-4-yl]-ethanone

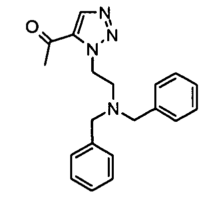

*N,N-*Dibenzyl-*N-*[2-(1*H-*1,2,3-triazol-1-yl)ethyl]amine (300 mg, 1.03 mmol, 1.0 equiv) is dissolved in THF (23 mL) and cooled to -78°C. 1.6M n-BuLi solution in hexane (0.79 mL, 1.26 mmol, 1.2 equiv) is slowly added and stirred for 1.5 hour. This gave a clear grey solution. At -78°C a solution of N-methoxy-N-methyl-acetamide (127 mg, 1.26 mmol, 1.2 equiv) in THF (2 mL) is added. The resulting mixture is stirred at room temperature for 2 h. Water (10 mL) is added and the mixture is concentrated in vacuo. An additional portion of water is added and the aqueous mixture is extracted twice with diethyl ether. The combined organic layers are washed with brine, dried over Na₂SO₄ and evaporated. The residue is purified by flash column chromatography, (20 to 50% ethyl acetate in heptane) to yield 147 mg (43%) of the title compound as a white solid.
¹H-NMR (400 MHz, CDCl₃): δ (ppm) 2.29, 2.85, 3.56, 4.80, 7.14-7.26, 8.04.

### 4-Methyl-4,5,6,7-tetrahydro-[1,2,3]triazolo[1,5-a]pyrazine

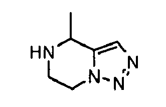

1-[3-(2-Dibenzylamino-ethyl)-3H-[1,2,3]triazol-4-yl]-ethanone (50 mg, 0.150 mmol, 1.0 equiv) is dissolved in ethanol (20 mL). Pd/C (10 mg, cat.) is added and placed under hydrogen atmosphere using a balloon. The resulting mixture is stirred at room temperature for 2 h. The reaction mixture is flushed with nitrogen. The mixture is filtered off over kieselguhr, rinsed with ethyl acetate and concentrated in vacuo to give 20 mg (95%) of the title compound as an colorless oil.
GC-MS m/z 138/123; ¹H-NMR (400 MHz, CDCl₃): δ (ppm) 1.49, 1.66, 3.18-3.25, 3.46-3.51, 4.13-4.28, 4.43-4.48, 7.47.

### 6-Chloro-1-methyl-3,4-dihydro-pyrrolo[1,2-a]pyrazine

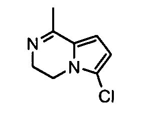

N-Chlorosuccinimide (8.42 gr, 63.1 mmol, 2.1 equiv.). is added to a cooled (<-5°C) solution of 1-methyl-1,2,3,4-tetrahydropyrrolo[1,2-a]pyrazine (4.09 gr, 30.0 mmol, 1.0 equiv.) in DCM (80 ml). After addition the mixture is stirred for 30 minutes. A 30% NaOH solution (40 ml) is added and the mixture is stirred vigorously for 1 hour at room temperature. The layers are separated and the aqueous layer is extracted with DCM (2x). The combined organic extracts are dried over Na₂SO₄ and concentrated. The residue is purified by flash column chromatography (Aluminium oxide, 20% EtOAc in heptane to 100% EtOAc) to afford 0.4 gr of product as a brown oil.
Flushing the column with 5% MeOH in DCM afforded more material; total yield: 4.16 gr (yield: 65% corrected for purity).
Purity:∼80%, contains product and some baseline material (Alumina, 50% Heptane in EtOAc).
LC-MS m/z 169/171 (MH+); ¹H-NMR (400 MHz, CDCl₃): δ (ppm) 2.39, 3.89-3.98, 6.19-6.20, 662-6.63.

### Cinnolin-4-ol

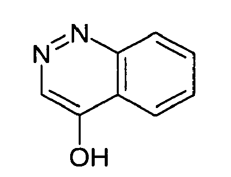

A mixture of 2'-aminoacetophenone (21 g, 0.155 mol) in concentrated HCl (105 mL) is cooled to -5°C. A solution of sodium nitrite (11.8 g, 0.171 mol) in water (32 mL) is added slowly maintaining the temperature below 0°C. After that the mixture is stirred at 65°C for 3 h. The mixture is cooled to room temperature. The precipitate is filtered off, washed with ether, dried and dissolved in water (minimal amount). Sodium hydrocarbonate (8 g) is carefully added to the solution, the mixture is stirred for 1 h and filtered. The precipitate is washed with water and dried on air to give 7.47 g (33%) of the title compound.
m/z (APCI+) 147 (M+H⁺)

### 3-Bromo-cinnolin-4-ol

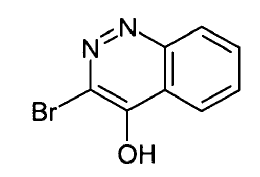

A solution of bromine (4.01 mL, 78 mmol) in glacial acetic acid (24 mL) is carefully added within 30 min to refluxing mixture of cinnolin-4-ol (11.4 g, 78 mmol), potassium acetate (7.73 g, 79 mmol) and glacial acetic acid (105 mL). After that the reaction mixture is refluxed for 45 min, cooled to room temperature and poured into ice water (500 mL). Formed yellow precipitate is filtered off, washed with water, dried on air to give 13.1 g (75%) of the title compound.
m/z (APCI+) 225 (M+H⁺)

### 3-Bromo-1-methyl-1H-cinnolin-4-one

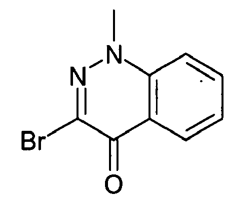

Potassium tert-butoxide (6.0 g, 53.2 mmol) is added to a suspension of 3-bromo-cinnolin-4-ol (8.0 g, 35.6 mmol) in dry tetrahydrofuran (180 mL), then methyl iodide (3.54 mL, 42.8 mmol) is added hereto. Then the reaction mixture is refluxed for 16 h, cooled to room temperature, diluted with water (500 mL) and extracted with dichloromethane (2×200 mL). The combined organic extracts are dried over sodium sulfate and evaporated. The residue is purified by column chromatography on silica gel (eluent: ethyl acetate-hexane 50 to 70%) to obtain 5.96 g (70%) of the title compound.
m/z (APCI+) 239 (M+H⁺)

### 1-Methyl-1H-cinnolin-4-one

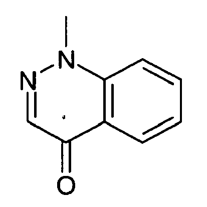

A suspension of 3-bromo-1-methyl-1H-cinnolin-4-one (5.9 g, 24.7 mmol), 10% Pd/C (0.5 g) and triethylamine (25 mL) in ethanol (150 mL) is stirred under hydrogen atmosphere for 4 h. The mixture is filtered, the filtrate is evaporated. The residue is partitioned between dichloromethane and water. The organic layer is washed with water, dried over sodium sulfate and evaporated to give 3.55 g (90%) of the title compound.
m/z (APCI+) 161 (M+H⁺)

### 1-Methyl-1,2,3,4-tetrahydro-cinnoline

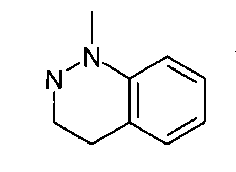

A solution of 1-methyl-1H-cinnolin-4-one (2.0 gr, 12.5 mmol, equiv) in toluene (100 mL) is treated with 4N LiA1H₄ in Et₂O (7.8 mL, 31.2 mmol, 2.5 equiv) and refluxed for 4 hrs. After cooling to room temp., another batch of LiA1H₄ (7.8 mL, 31.2 mmol, 2.5 equiv) is added and refluxing continued for 16 hrs. The mixture is allowed to stir at room temp. for 24 hrs before adding 5N NaOH (aq) sol. (8 mL). The mixture is refluxed for another hour, filtered and concentrated to yield 2.14 gr of crude material containing the title compound. This material is used in the next steps without further purification due to poor stability of. the product.
GC-MS m/z 148 (purity: 44%).

### 1-Methyl-3,4-dihydro-1H-pyrrolo[1,2-a]pyrazine-2-carboxylic acid tert-butyl ester

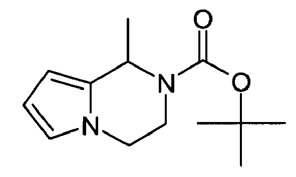

To a solution of 1-Methyl-1,2,3,4-tetrahydro-pyrrolo[1,2-a]pyrazine (69 g, 0.52 mol) in THF (600 ml) triethylamine (85 ml, 0.6 mol) and Boc₂O (130 ml) were added. The reaction mixture was stirred till the gas evolution completed (during 6 hours). The solvent was removed in vacuo, the residue was distilled in vacuo (bp=145-155 °C/12mm Hg) yielding the title compound (65 g, 52%).
LC/MS: m/z = 237 (MH⁺)

### 6-Cyano-1-methyl-3,4-dihydro-1H-pyrrolo[1,2-a]pyrazine-2-carboxylic acid tert-butyl ester

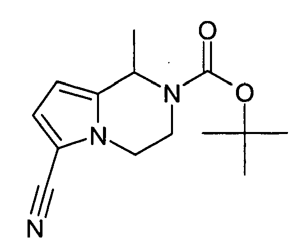

To a solution of 1-Methyl-3,4-dihydro-1H-pyrrolo[1,2-a]pyrazine-2-carboxylic acid tert-butyl ester (10 g, 0.042 mol) and triethylamine (35 ml, 0.25 mol) in toluene (100 ml) chlorosulfonyl isocyanate (4 ml, 0.045 mol) was added dropwise at 15°C. Then reaction mixture was refluxed for 6 hours. The toluene layer was washed with water (1 L), dried and the solvent was removed in vacuo. The residue was purified by chromatography on silica gel using hexane:ethylacetate 80:20 mixture as eluent yielding the title compound (4.5 g, 41 %).
LC/MS: m/z = 262 (MH⁺)

### 1-Methyl-1,2,3,4-tetrahydro-pyrrolo[1,2-a]pyrazine-6-carbonitrile

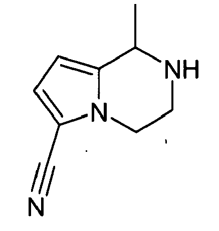

To a solution of 6-Cyano-1-methyl-3,4-dihydro-1H-pyrrolo[1,2-a]pyrazine-2-carboxylic acid tert-butyl ester (4.5 g, 0.017 mol) in MeOH (100 ml) dioxane saturated with HCl (20 ml) was added. The reaction mixture was stirred for 5 min, then solvent was evaporated in vacuo and the residue treated with acetone resulting in 1.3 g (39%) of hydrochloride of the title compound.
LC/MS: m/z = 162 (MH⁺)

### 1-Methyl-6-(2,2,2-trichloro-acetyl)-3,4-dihydro-1H-pyrrolo[1,2-a]pyrazine-2-carboxylic acid tert-butyl ester

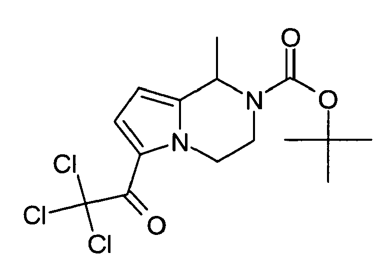

To a solution of 1-Methyl-3,4-dihydro-1H-pyrrolo[1,2-a]pyrazine-2-carboxylic acid tert-butyl ester (23.5 g, 0.1 mol) and triethylamine (20 ml, 0.14 mol) in CHCl₃ (250 ml) trichloroacetylchloride (12.7 ml) was added dropwise keeping the temperature of reaction mixture below 15°C. After 3-4 hours of stirring (reaction was monitored by ¹³C NMR spectra) the reaction mixture was poured into saturated K₂CO₃ solution (0.5 L), organic layer was separated and dried over Na₂SO₄. The solvent was removed in vacuo, the residue was treated with hexane yielding the title compound (34 g, 89%).
LC/MS: m/z = 382 (MH⁺)

### 1-Methyl-6-methylcarbamoyl-3,4-dihydro-1H-pyrrolo[1,2-a]pyrazine-2-carboxylic acid tert-butyl ester

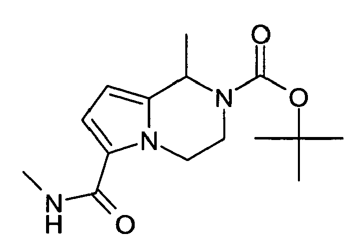

The solution of 1-Methyl-6-(2,2,2-trichloro-acetyl)-3,4-dihydro-1H-pyrrolo[1,2-a]pyrazine-2-carboxylic acid tert-butyl ester (15 g, 0.039 mol) in MeOH saturated with MeNH₂ (120 ml) was heated for 24 hours at 140°C in autoclave. Then the solvent was removed in vacuo and the residue was recrystallized from hexane yielding 6 g (53%) of the title compound.
LC/MS: m/z = 294 (MH⁺)

### 1-Methyl-1,2,3,4-tetrahydro-pyrrolo[1,2-a]pyrazine-6-carboxylic acid methylamide

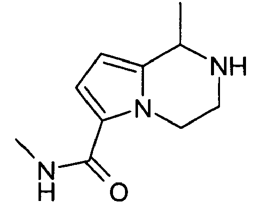

To a solution of 1-Methyl-6-methylcarbamoyl-3,4-dihydro-1H-pyrrolo[1,2-a]pyrazine-2-carboxylic acid tert-butyl ester (5 g, 0.017 mol) in MeOH (100 ml) dioxane saturated with HCl (20 ml) was added. The reaction mixture was stirred for 5 min, then solvent was evaporated in vacuo and the residue treated with acetone. The resulted hydrochloride was dissolved in CHCl₃ (50 ml) and treated with saturated K₂CO₃ solution (50 ml). Organic layer was separated, dried over Na₂SO₄, the solvent was removed in vacuo and the residue was re-crystallized from hexane resulting in 1.3 g (39%) of the title compound.
LC/MS: m/z = 194 (MH⁺)

### 6-Cyclopropylcarbamoyl-1-methyl-3,4-dihydro-1H-pyrrolo[1,2-a]pyrazine-2-carboxylic acid tert-butyl ester

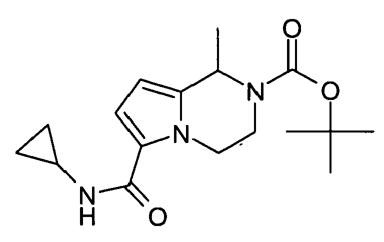

The solution of 1-Methyl-6-(2,2,2-trichloro-acetyl)-3,4-dihydro-1H-pyrrolo[1,2-a]pyrazine-2-carboxylic acid tert-butyl ester (15 g, 0.039 mol) and cyclopropylamine (11 g, 0.2 mol) in CHCl₂ was refluxed till compound 5 disappeared from reaction mixture (reaction was monitored by ¹³C NMR spectra). Then the solvent was removed in vacuo and the residue was recrystallized from hexane yielding 5.5 g (44%) of the title compound.
LC/MS: m/z = 320 (MH⁺)

### 1-Methyl-1,2,3,4-tetrahydro-pyrrolo[1,2-a]pyrazine-6-carboxylic acid cyclopropylamide

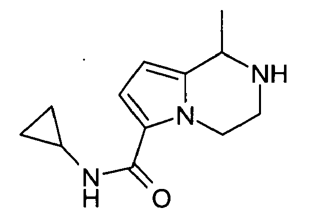

To a solution of 6-Cyclopropylcarbamoyl-l-methyl-3,4-dihydro-1H-pyrrolo[1,2-a]pyrazine-2-carboxylic acid tert-butyl ester (5 g, 0.015 mol) in MeOH (100 ml) dioxane saturated with HCl (20 ml) was added. The reaction mixture was stirred for 5 min, then solvent was evaporated in vacuo and the residue treated with acetone. The resulted hydrochloride was dissolved in CHCl₃ (50 ml) and treated with saturated K₂CO₃ solution (50 ml). Organic layer was separated, dried over Na₂SO₄, the solvent was removed in vacuo and the residue was re-crystallized from hexane resulting in 2.1 g (56%) of the title compound.
LC/MS: m/z = 220 (MH⁺)

### 6-Ethylcarbamoyl-l-methyl-3,4-dihydro-1H-pyrrolo[1,2-a]pyrazine-2-carboxylic acid tert-butyl ester

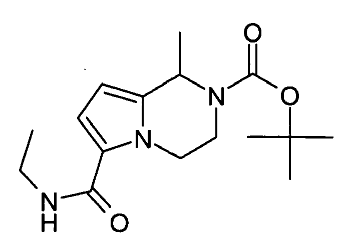

The solution of 1-Methyl-6-(2,2,2-trichloro-acetyl)-3,4-dihydro-1H-pyrrolo[1,2-a]pyrazine-2-carboxylic acid tert-butyl ester (15 g, 0.039 mol) and ethylamine (9 g, 0.2 mol) in CHCl₂ was refluxed till compound 5 disappeared from reaction mixture (reaction was monitored by ¹³C NMR spectra). Then the solvent was removed in vacuo and the residue was recrystallized from hexane yielding 5.8 g (48%) of the title compound.
LC/MS: m/z = 308 (MH⁺)

### 1-Methyl-1,2,3,4-tetrahydro-pyrrolo[1,2-a]pyrazine-6-carboxylic acid ethylamide

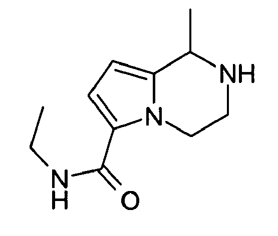

To a solution of 6-Ethylcarbamoyl-1-methyl-3,4-dihydro-1H-pyrrolo[1,2-a]pyrazine-2-carboxylic acid tert-butyl ester (5 g, 0.017 mol) in MeOH (100 ml) dioxane saturated with HCl (20 ml) was added. The reaction mixture was stirred for 5 min, then solvent was evaporated in vacuo and the residue treated with acetone. The resulted hydrochloride was dissolved in CHCl₃ (50 ml) and treated with saturated K₂CO₃ solution (50 ml). Organic layer was separated, dried over Na₂SO₄, the solvent was removed in vacuo and the residue was re-crystallized from hexane resulting in 2.3 g (65%) of the title compound.
LC/MS: m/z = 208 (MH⁺)

### 6-Isopropylcarbamoyl-1-methyl-3,4-dihydro-1H-pyrrolo[1,2-a]pyrazine-2-carboxylic acid tert-butyl ester

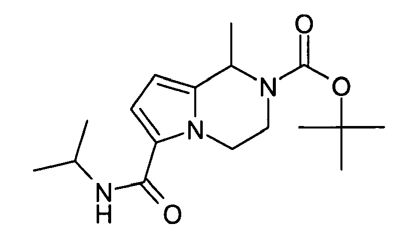

The solution of 1-Methyl-6-(2,2,2-trichloro-acetyl)-3,4-dihydro-1H-pyrrolo[1,2-a]pyrazine-2-carboxylic acid tert-butyl ester (15 g, 0.039 mol) and i-propylamine (11 g, 0.2 mol) in CHCl₂ was refluxed till compound 5 disappeared from reaction mixture (reaction was monitored by ¹³C NMR spectra). Then the solvent was removed in vacuo and the residue was recrystallized from hexane yielding 5.5 g (43%) of the title compound.
LC/MS: m/z = 322 (MH⁺)

### 1-Methyl-1,2,3,4-tetrahydro-pyrrolo[1,2-a]pyrazine-6-carboxylic acid isopropylamide

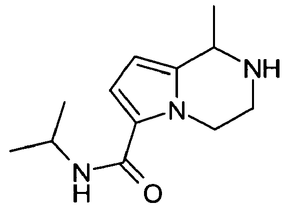

To a solution of 6-Isopropylcarbamoyl-1-methyl-3,4-dihydro-1H-pyrrolo[1,2-a]pyrazine-2-carboxylic acid tert-butyl ester (5 g, 0.015 mol) in MeOH (100 ml) dioxane saturated with HCl (20 ml) was added. The reaction mixture was stirred for 5 min, then solvent was evaporated in vacuo and the residue treated with acetone. The resulted hydrochloride was dissolved in CHCl₃ (50 ml) and treated with saturated K₂CO₃ solution (50 ml). Organic layer was separated, dried over Na₂SO₄, the solvent was removed in vacuo and the residue was re-crystallized from hexane resulting in 1.5 g (45%) of the title compound.
LC/MS: m/z = 222 (MH⁺)

### (2,4-Difluorophenyl)(oxo)acetaldehyde

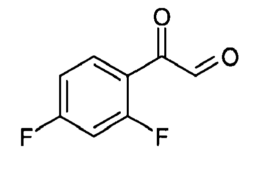

SeO₂ (35.6 g, 0.32 mol, 1 equiv) was added to a solution of 2,4-difluoroacetophenone (50 g, 0.32 mol, 1 equiv) in a mixture dioxane-H₂O (175 ml : 7 ml, respectively) and the mixture was refluxed for 5 hrs. The reaction mixture was evaporated. Distillation yielded (b.p 130-135 C/1 mm Hg) 30 g (55%) of the title compound.
LC/MS: m/z = 171 (MH⁺)

### (2,4-Difluorophenyl)(oxo)acetaldehyde methylhydrazone

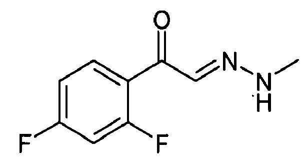

Methyl hydrazine (18.5 g, 0.4 mol, 2.3 equiv) was dropwise added to a solution of (2,4-difluorophenyl)(oxo)acetaldehyde (30 g, 0.176 mol, 1.0 equiv) in MeOH:H₂O:HOAc (300 ml: 300 ml: 50 ml) and the mixture was stirred at room temperature for 24 hrs. The precipitate was filtered off and washed with water to yield 18 g (51.5%) of the title compound.
LC/MS: m/z = 199 (MH⁺)

### 7-Fluorocinnolin-4(1H)-one

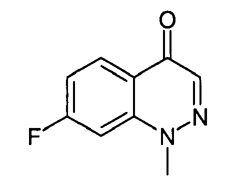

K₂CO₃ (21.2 g, 0.1534 mol, 2 equiv) was added to a solution of (2,4-ifluorophenyl)(oxo)acetaldehyde methylhydrazone (15.2 g, 0.0767 mol, 1.0 equiv) in DMF (30 ml) and the mixture was stirred at 100-105 C for 8 hrs. The reaction mixture was diluted with water. The precipitate was filtered off and washed with water to yield 6.2 g (45.5%) of the title compound.
LC/MS: m/z = 179 (MH⁺)

### 7-Fuoro-1-methyl-1,2,3,4-tetrahydro-cinnoline dihidrochloride

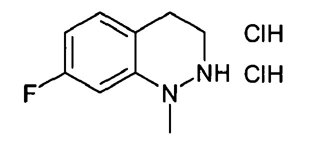

A solution of 7-fluorocinnolin-4(1H)-one (7.3 g, 0.0225 mol, 1 equiv) in benzene (100 ml) was treated with LiA1H₄ (6.08 g, 0.16 mol, 4 equiv) in Et₂O (100 ml) and refluxed for 5 hrs. The mixture was allowed to stir at room temp. for 24 hrs before adding NaOH (5 g) in H2O (15 ml). The mixture was refluxed for 1 hour, filtered and concentrated. Distillation yielded 7-fluoro-1-methyl-1,2,3,4-tetrahydro-cinnoline (b.p 150-155 C/1 mm). Amine was dissolved in MeOH (30 ml) and HCl was bubbled (10 min), methanol was evaporated and residue was treated with Et₂O to yielded 2.55 g (32%) of title compound.
LC/MS: m/z = 137 (MH⁺)

### 2-Azido-ethanol

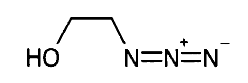

To a solution of NaN₃ (157 g, 3 mol) in H₂O (500 ml) 2-chloro-ethanol (161 g, 0.2 mol) was added and reaction mixture was stirred at 70°C for 60 hours. Then it was saturated with Na₂SO₄, extracted with CH₂Cl₂, organic layer was separated and dried over Na₂SO₄. The solvent was removed in vacuo affording the title compound as a colorless liquid (156.6 g, 90%).
LC/MS: m/z = 88 (MH⁺)

### Toluene-4-sulfonic acid 2-azido-ethyl ester

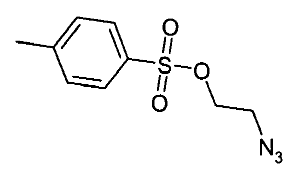

To a solution of 2-Azido-ethanol (100 g, 1.15 mol) in CH₂Cl₂ (600 ml) a solution of TosCl (220 g, 1.15 mol) in CH₂Cl₂ (400ml) was added dropwise keeping temperature at 0°C. Then the reaction mixture was stirred for 4 hours, washed with water (4x300 ml) and the organic layer was dried over Na₂SO₄. The solvent was removed in vacuo affording the title compound as a pale-yellow oil (235.6 g, 85%).
LC/MS: m/z = 242 (MH⁺)

### (2-Azido-ethyl)-benzyl-amine

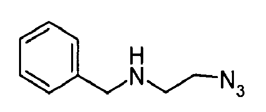

A solution of benzylamine (95 ml, 1 mol) and Toluene-4-sulfonic acid 2-azido-ethyl ester (100 g, 0.41 mol) in CH₃CN (400ml) was refluxed with stirring for 7 hours, then the precipitate was filtered off and the solvent was removed in vacuo. The crude residue was subjected to column chromatography over SiO₂ using hexane as eluent affording the title compound as a pale-yellow liquid (55 g, 75%).
LC/MS: m/z = 177 (MH⁺)

### [(2-Azido-ethyl)-benzyl-amino]-acetic acid ethyl ester

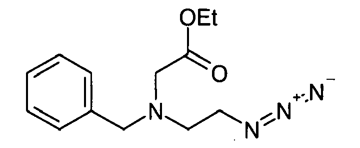

To a solution of (2-Azido-ethyl)-benzyl-amine (20 g, 0.11 mol), bromo-acetic acid ethyl ester (23 g, 0.13 mol) in EtOH (150 ml) K₂CO₃ (25 g, 0.18 mol) and Trident ((N(CH. ₂CH₂OCH₂CH₂OMe)₃, 3.7 g, 0.011 mol) were added. The reaction mixture was refluxed for 3 hours, cooled, poured into water and extracted with CCl₄. The organic layer was washed with water, dried over Na₂SO₄, the solvent was removed in vacuo yielding the title compound as a colorless oil (23 g, 77%).
LC/MS: m/z = 263 (MH⁺)

### 1-Benzyl-5-ethoxy-1,2,3,6-tetrahydro-pyrazine

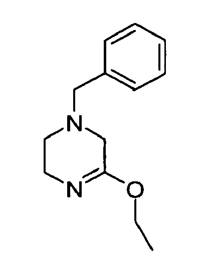

Triphenylphosphine (21 g, 0.8 mol) was added to a solution of [(2-Azido-ethyl)-benzylamino]-acetic acid ethyl ester (21 g, 0.8 mol) in toluene under Ar atmosphere, then the reaction mixture was stirred at reflux for 8 hours and the solvent was removed in vacuo. The product was extracted from the residue with hot hexane. The solvent was evaporated and the crude product was subjected to flash column chromatography over SiO₂ using hexane as eluent affording mixture of the title compound with by-product - 4-benzyl-1-ethyl-piperazin-2-one in 3:1 ratio (10 g). This mixture was used in the next step without additional purification.
LC/MS: m/z = 219 (MH⁺)

### 7-Benzyl-3-methyl-5,6,7,8-tetrahydro-imidazo[1,2-a]pyrazine dihydrochloride

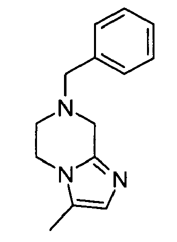

To the mixture of 1-Benzyl-5-ethoxy-1,2,3,6-tetrahydro-pyrazine and 4-benzyl-1-ethyl-piperazin-2-one (4 g) dissolved in toluene (20 ml) prop-2-ynylamine (0.8 g, 0.014 mol) was added and the reaction mixture was refluxed for 8-9 hours. Then the solvent was removed in vacuo and the product was extracted from the residue with hot hexane. The solvent was evaporated and the crude product obtained was subjected to flash column chromatography over SiO₂ using hexane as eluent. The residue was dissolved in minimal amount of Et₂O (∼ 30 mL) and dry HCl was bubbled at -5 °C through the solution obtained. The precipitate formed was filtered off and re-crystallized from MeCN affording the title compound as its dihydrochloride salt (2.35 g, 60%).
LC/MS: m/z = 228 (MH⁺)

### 3-Methyl-5,6,7,8-tetrahydro-imidazo[1,2-a]pyrazine dihydrochloride

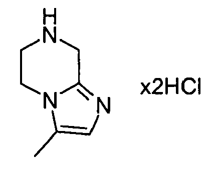

To a solution of 7-Benzyl-3-methyl-5,6,7,8-tetrahydro-imidazo[1,2-a]pyrazine dihydrochloride (2.9 g, 0.012 mol) in MeOH (100 ml) Pd/C (200 mg) was added and the reaction mixture was kept at 60°C in autoclave for 5 hours (at 50 atm) and then stirred for 14 hours at RT. The precipitate was filtered off, and the solvent was removed in vacuo yielding the title compound (1.51 g, 74%).
LC/MS: m/z = 138 (MH⁺)

The following compounds according to the invention are prepared as examples, which are intended as an illustration of and not a limitation upon the scope of the invention:

### Example 1

### (6-Bromo-pyrazolo[1,5-a]pyridin-2-yl)-(4-methyl-6,7-dihydro-4H-thieno[3,2-c]pyridin-5-yl)-methanone

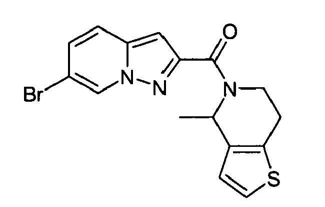

To a solution of 6-bromo-pyrazolo[1,5-a]pyridine-2-carboxylic acid (0.7 g, 2.9 mmol, 1.0 equiv) and TBTU (1.0 g, 3.1 mmol, 1.1 equiv) in dry acetonitrile (20 mL), triethylamine (1 mL, 7.25 mmol, 2.5 equiv) and 4-methyl-4,5,6,7-tetrahydro-thieno[3,2-c]pyridine (0.45 g, 2.9 mmol, 1 equiv) are added. The reaction mixture is stirred at 50°C for 2 h and then at room temperature for 8 h. The reaction mixture is diluted with water (10ml). The precipitate is filtered, washed sequentially with 50% aqueous ethanol, water, 5% aqueous ammonia, and diethyl ether, and then dried to provide 0.524 g of the title compound.
LC/MS: m/z = 376 (MH⁺)
The stereo-isomers of this compound are separated. The S-configurated compound has a different activity than the R-configurated compound.

### Example 2

### (6-Chloro-pyrazolo[1,5-a]pyridin-2-yl)-(4-methyl-6,7-dihydro-4H-thieno[3,2-c]pyridin-5-yl)-methanone

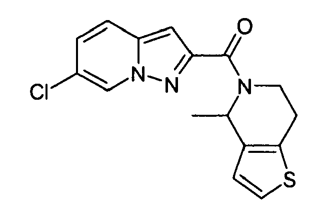

In close analogy to the procedure described in Example 1, 6-chloro-pyrazolo[1,5-a]pyridine-2-carboxylic acid is reacted with 4-methyl-4,5,6,7-tetrahydro-thieno[3,2-c]pyridine to provide the title compound.
LC/MS: m/z = 332 (MH⁺)
The stereo-isomers of this compound are separated. The S-configurated compound has a different activity than the R-configurated compound.

### Example 3 .

### (6-Chloro-pyrazolo[1,5-a]pyridin-2-yl)-(6,7-dihydro-4H-thieno[3,2-c]pyridin-5-yl)-methanone

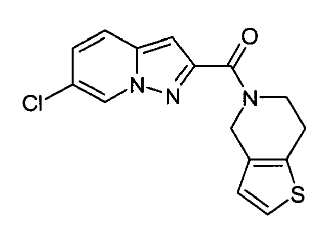

In close analogy to the procedure described in Example 1, 6-chloro-pyrazolo[1,5-a]pyridine-2-carboxylic acid is reacted with 4,5,6,7-tetrahydro-thieno[3,2-c]pyridine to provide the title compound in moderate yield.
LC/MS: m/z = 319 (MH⁺)

### Example 4

### (6-Bromo-pyrazolo[1,5-a]pyridin-2-yl)-(6,7-dihydro-4H-thieno[3,2-c]pyridin-5-yl)-methanone

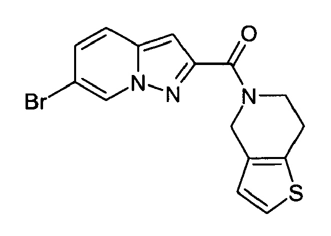

In close analogy to the procedure described in Example 1, 6-bromo-pyrazolo[1,5-a]pyridine-2-carboxylic acid is reacted with 4,5,6,7-tetrahydro-thieno[3,2-c]pyridine to provide the title compound in moderate yield.
LC/MS: m/z = 363 (MH⁺)

### Example 5

### (6-Chloro-pyrazolo[1,5-a]pyridin-2-yl)-(4,4-dimethyl-6,7-dihydro-4H-thieno[3,2-c]pyridin-5-yl)-methanone

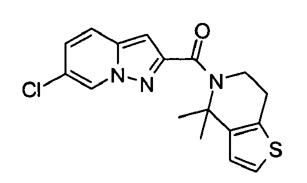

In close analogy to the procedure described in Example 1, 6-chloro-pyrazolo[1,5-a]pyridine-2-carboxylic acid is reacted with 4,4-dimethyl-4,5,6,7-tetrahydro-thieno[3,2-c]pyridine to provide the title compound in moderate yield.
LC/MS: m/z = 347 (MH⁺)

### Example 6

### (6-Bromo-pyrazolo[1,5-a]pyridin-2-yl)-(4,4-dimethyl-6,7-dihydro-4H-thieno[3,2-c]pyridin-5-yl)-methanone

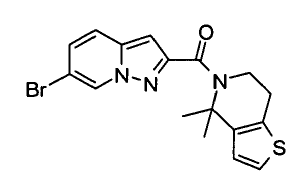

In close analogy to the procedure described in Example 1, 6-chloro-pyrazolo[1,5-a]pyridine-2-carboxylic acid is reacted with 4,4-dimethyl-4,5,6,7-tetrahydro-thieno[3,2-c]pyridine to provide the title compound in moderate yield.
LC/MS: m/z = 391 (MH⁺)

### Example 7

### (6-Chloro-pyrazolo[1,5-a]pyridin-2-yl)-(6-methyl-6,7-dihydro-4H-thieno[3,2-c]pyridin-5-yl)-methanone

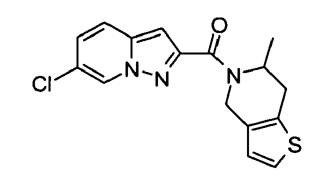

In close analogy to the procedure described in Example 1, 6-chloro-pyrazolo[1,5-a]pyridine-2-carboxylic acid is reacted with 6-methyl-4,5,6,7-tetrahydro-thieno[3,2-c]pyridine to provide the title compound.
LC/MS: m/z = 332 (MH+)
The stereo-isomers of this compound are separated. The S-configurated compound has a different activity than the R-configurated compound.

### Example 8

### (6-Bromo-pyrazolo[1,5-a]pyridin-2-yl)-(6-methyl-6,7-dihydro-4H-thieno[3,2-c]pyridin-5-yl)-methanone

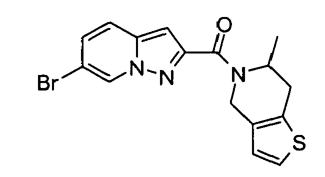

In close analogy to the procedure described in Example 1, 6-bromo-pyrazolo[1,5-a]pyridine-2-carboxylic acid is reacted with 6-methyl-4,5,6,7-tetrahydro-thieno[3,2-c]pyridine to provide the title compound.
LC/MS: m/z = 377 (MH+)
The stereo-isomers of this compound are separated. The S-configurated compound has a different activity than the R-configurated compound.

### Example 9

### (6-Bromo-pyrazolo[1,5-a]pyridin-2-yl)-(4-ethyl-6,7-dihydro-4H-thieno[3,2-c]pyridin-5-yl)-methanone

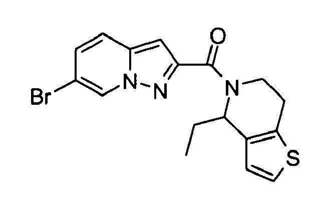

In close analogy to the procedure described in Example 1, 6-bromo-pyrazolo[1,5-a]pyridine-2-carboxylic acid is reacted with 4-ethyl-4,5,6,7-tetrahydro-thieno[3,2-c]pyridine to provide the title compound.
LC/MS: m/z = 391 (MH⁺)
The stereo-isomers of this compound are separated. The S-configurated compound has a different activity than the R-configurated compound.

### Example 10

### (6-Chloro-pyrazolo[1,5-a]pyridin-2-yl)-(4-ethyl-6,7-dihydro-4H-thieno[3,2-c]pyridin-5-yl)-methanone

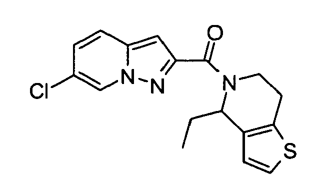

In close analogy to the procedure described in Example 1, 6-chloro-pyrazolo[1,5-a]pyridine-2-carboxylic acid is reacted with 4-ethyl-4,5,6,7-tetrahydro-thieno[3,2-c]pyridine to provide the title compound.
LC/MS: m/z = 346 (MH⁺)
The stereo-isomers of this compound are separated. The S-configurated compound has a different activity than the R-configurated compound.

### Example 11

### (6-Bromo-pyrazolo[1,5-a]pyridin-2-yl)-(4-trifluoromethyl-6,7-dihydro-4H-thieno[3,2-c]pyridin-5-yl)-methanone

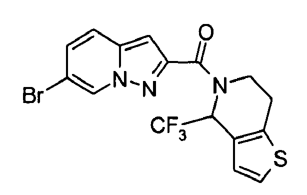

In close analogy to the procedure described in Example 1, 6-bromo-pyrazolo[1,5-a]pyridine-2-carboxylic acid is reacted with 4-trifluoromethyl-4,5,6,7-tetrahydro-thieno[3,2-c]pyridine to provide the title compound in moderate yield.
LC/MS: m/z = 377 (MH⁺)

The stereo-isomers of this compound are separated. The S-configurated compound has a different activity than the R-configurated compound.

### Example 12

### (6-Chloro-pyrazolo[1,5-a]pyridin-2-yl)-(4-trifluoromethyl-6,7-dihydro-4H-thieno[3,2-c]pyridin-5-yl)-methanone

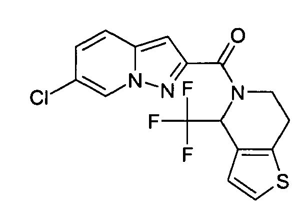

In close analogy to the procedure described in Example 1, 6-chloro-pyrazolo[1,5 a]pyridine-2-carboxylic acid is reacted with 4-trifluoromethyl-4,5,6,7-tetrahydro-thieno[3,2-c]pyridine to provide the title compound.
LC/MS: m/z = 386 (MH+)
The stereo-isomers of this compound are separated. The S-configurated compound has a different activity than the R-configurated compound.

### Example 13

### 6-Bromo-pyrazolo[1,5-a]pyridin-2-yl)-(6,6-dimethyl-6,7-dihydro-4H-thieno[3,2-c]pyridin-5-yl)-methanone

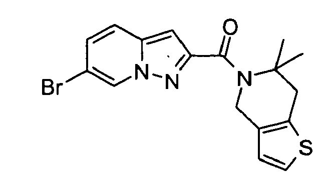

In close analogy to the procedure described in Example 1, 6-bromo-pyrazolo[1,5-a]pyridine-2-carboxylic acid is reacted with 6,6-dimethyl-4,5,6,7-tetrahydro-thieno[3,2-c]pyridine to provide the title compound in moderate yield.
LC/MS: m/z = 391 (MH⁺)

### Example 14

### 6-Chloro-pyrazolo[1,5-a]pyridin-2-yl)-(6,6-dimethyl-6,7-dihydro-4H-thieno[3,2-c]pyridin-5-yl)-methanone

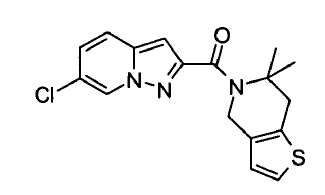

In close analogy to the procedure described in Example 1, 6-chloro-pyrazolo[1,5-a]pyridine-2-carboxylic acid is reacted with 6,6-dimethyl-4,5,6,7-tetrahydro-thieno[3,2-c]pyridine to provide the title compound in moderate yield.
LC/MS: m/z = 347 (MH⁺)

### Example 15

### (6-Bromo-pyrazolo[1,5-a]pyridin-2-yl)-(2,4-dimethyl-6,7-dihydro-4H-thieno[3,2-c]pyridin-5-yl)-methanone

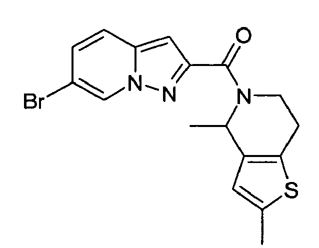

In close analogy to the procedure described in Example 1, 6-bromo-pyrazolo[1,5-a]pyridine-2-carboxylic acid is reacted with 2,4-dimethyl-4,5,6,7-tetrahydro-thieno[3,2-c]pyridine to provide the title compound.
LC/MS: m/z = 391 (MH⁺)
The stereo-isomers of this compound are separated. The S-configurated compound has a different activity than the R-configurated compound.

### Example 16

### (6-Chloro-pyrazolo[1,5-a]pyridin-2-yl)-(2,4-dimethyl-6,7-dihydro-4H-thieno[3,2-c]pyridin-5-yl)-methanone

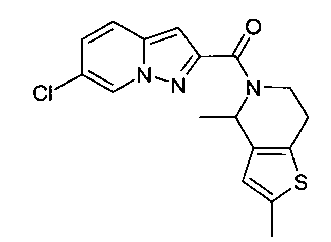

In close analogy to the procedure described in Example 1, 6-chloro-pyrazolo[1,5-a]pyridine-2-carboxylic acid is reacted with 2,4-dimethyl-4,5,6,7-tetrahydro-thieno[3,2-c]pyridine to provide the title compound.
LC/MS: m/z = 346 (MH⁺).
The stereo-isomers of this compound are separated. The S-configurated compound has a different activity than the R-configurated compound.

### Example 17

### (6-Bromo-pyrazolo[1,5-a]pyridin-2-yl)-(3,4-dimethyl-6,7-dihydro-4H-thieno[3,2-c]pyridin-5-yl)-methanone

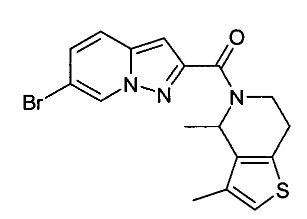

In close analogy to the procedure described in Example 1, 6-bromo-pyrazolo[1,5-a]pyridine-2-carboxylic acid is reacted with 2,5-dimethyl-4,5,6,7-tetrahydro-thieno[3,2-c]pyridine to provide the title compound.
LC/MS: m/z = 391 (MH⁺)
The stereo-isomers of this compound are separated. The S-configurated compound has a different activity than the R-configurated compound.

### Example 18

### 6-Bromo-2-[(2,3,4-trimethyl-6,7-dihydrothieno[3,2-c]pyridin-5(4H)-yl)carbonyl]-pyrazolo[1,5-a]pyridine

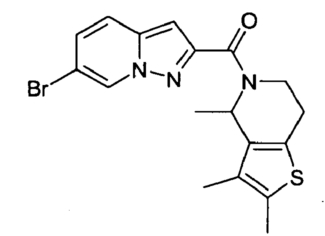

In close analogy to the procedure described in Example 1, 6-bromo-pyrazolo[1,5-a]pyridine-2-carboxylic acid is reacted with 2,3,4-trimethyl-4,5,6,7-tetrahydro-thieno[3,2-c]pyridine to provide the title compound.
LC/MS: m/z = 405 (MH+)
The stereo-isomers of this compound are separated. The S-configurated compound has a different activity than the R-configurated compound.

### Example 19

### 6-Chloro-2-[(2,3,4-trimethyl-6,7-dihydrothieno[3,2-c]pyridin-5(4H)-yl)carbonyl]-pyrazolo[1,5-a]pyridine

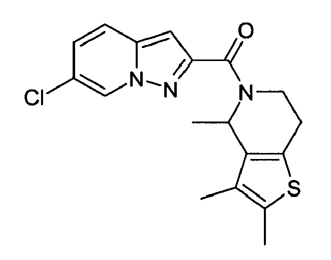

In close analogy to the procedure described in Example 1, 6-chloro-pyrazolo[1,5-a]pyridine-2-carboxylic acid is reacted with 2,3,4-trimethyl-4,5,6,7-tetrahydro-thieno[3,2-c]pyridine to provide the title compound.
LC/MS: m/z = 360 (MH⁺)
The stereo-isomers of this compound are separated. The S-configurated compound has a different activity than the R-configurated compound.

### Example 20

### (6-Bromo-pyrazolo[1,5-a]pyridin-2-yl)-(2-fluoro-4-methyl-6,7-dihydro-4H-thieno[3,2-c]pyridin-5-yl)-methanone

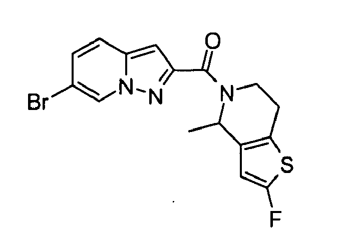

In close analogy to the procedure described in Example 1, 6-bromo-pyrazolo[1,5-a]pyridine-2-carboxylic acid is reacted with 2-fluoro-4-methyl-4,5,6,7-tetrahydro-thieno[3,2-c]pyridine to provide the title compound in moderate yield.
LC/MS: m/z = 395 (MH⁺)
The stereo-isomers of this compound are separated. The S-configurated compound has a different activity than the R-configurated compound.

### Example 21

### (6-Chloro-pyrazolo[1,5-a]pyridin-2-yl)-(2-fluoro-4-methyl-6,7-dihydro-4H-thieno[3,2-c]pyridin-5-yl)-methanone

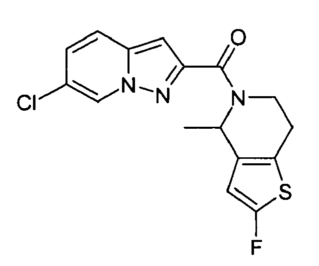

In close analogy to the procedure described in Example 1, 6-chloro-pyrazolo[1,5-a]pyridine-2-carboxylic acid is reacted with 2-fluoro-4-methyl-4,5,6,7-tetrahydro-thieno[3,2-c]pyridine to provide the title compound in moderate yield.
LC/MS: m/z = 351 (MH⁺)
The stereo-isomers of this compound are separated. The S-configurated compound has a different activity than the R-configurated compound.

### Example 22

### (6-Bromo-pyrazolo[1,5-a]pyridin-2-yl)-(3-fluoro-4-methyl-6,7-dihydro-4H-thieno[3,2-c]pyridin-5-yl)-methanone

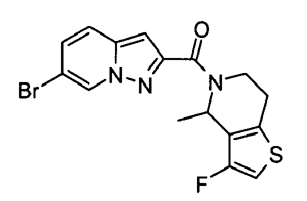

In close analogy to the procedure described in Example 1, 6-bromo-pyrazolo[1,5-a]pyridine-2-carboxylic acid is reacted with 3-fluoro-4-methyl-4,5,6,7-tetrahydro-thieno[3,2-c]pyridine to provide the title compound in moderate yield.
LC/MS: m/z = 395 (MH⁺)
The stereo-isomers of this compound are separated. The S-configurated compound has a different activity than the R-configurated compound.

### Example 23

### (6-Chloro-pyrazolo[1,5-a]pyridin-2-yl)-(3-fluoro-4-methyl-6,7-dihydro-4H-thieno[3,2-c]pyridin-5-yl)-methanone

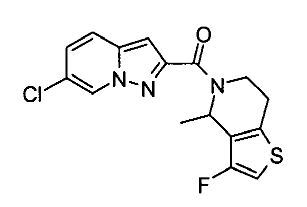

In close analogy to the procedure described in Example 1, 6-chloro-pyrazolo[1,5-a]pyridine-2-carboxylic acid is reacted with 3-fluoro-4-methyl-4,5,6,7-tetrahydro-thieno[3,2-c]pyridine to provide the title compound in moderate yield.
LC/MS: m/z = 351 (MH⁺)
The stereo-isomers of this compound are separated. The S-configurated compound has a different activity than the R-configurated compound.

### Example 24

### (6-Bromo-pyrazolo[1,5-a]pyridin-2-yl)-(2,3-difluoro-4-methyl-6,7-dihydro-4H-thieno[3,2-c]pyridin-5-yl)-methanone

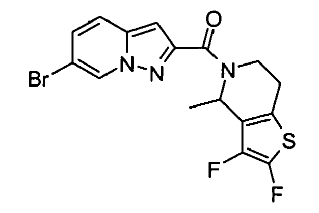

In close analogy to the procedure described in Example 1, 6-bromo-pyrazolo[1,5-a]pyridine-2-carboxylic acid is reacted with 2,3-difluoro-4-methyl-4,5,6,7-tetrahydro-thieno[3,2-c]pyridine to provide the title compound in moderate yield.
LC/MS: m/z = 413 (MH⁺)
The stereo-isomers of this compound are separated. The S-configurated compound has a different activity than the R-configurated compound.

### Example 25

### (6-Chloro-pyrazolo[1,5-a]pyridin-2-yl)-(2,3-difluoro-4-methyl-6,7-dihydro-4H-thieno[3,2-c]pyridin-5-yl)-methanone

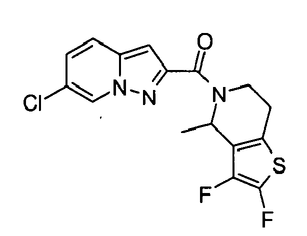

In close analogy to the procedure described in Example 1, 6-chloro-pyrazolo[1,5-a]pyridine-2-carboxylic acid is reacted with 2,3-difluoro-4-methyl-4,5,6,7-tetrahydro-thieno[3,2-c]pyridine to provide the title compound in moderate yield.
LC/MS: m/z = 369 (MH⁺)
The stereo-isomers of this compound are separated. The S-configurated compound has a different activity than the R-configurated compound.

### Example 26

### (6-Bromo-pyrazolo[1,5-a]pyridin-2-yl)-(4-methyl-2-trifluoromethyl-6,7-dihydro-4H-thieno[3,2-c]pyridin-5-yl)-methanone

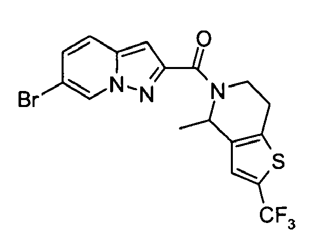

In close analogy to the procedure described in Example 1, 6-bromo-pyrazolo[1,5-a]pyridine-2-carboxylic acid is reacted with 4-methyl-2-trifluoromethyl-4,5,6,7-tetrahydro-thieno[3,2-c]pyridine to provide the title compound in moderate yield.
LC/MS: m/z = 391 (MH⁺). The stereo-isomers of this compound are separated. The S-configurated compound has a different activity than the R-configurated compound.

### Example 27

### (6-Chloro-pyrazolo[1,5-a]pyridin-2-yl)-(4-methyl-2-trifluoromethyl-6,7-dihydro-4H-thieno[3,2-c]pyridin-5-yl)-methanone

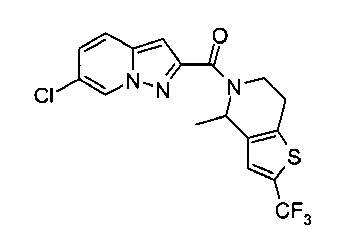

In close analogy to the procedure described in Example 1, 6-chloro-pyrazolo[1,5-a]pyridine-2-carboxylic acid is reacted with 4-methyl-2-trifluoromethyl-4,5,6,7-tetrahydro-thieno[3,2-c]pyridine to provide the title compound in moderate yield.
LC/MS: m/z = 347 (MH⁺)

### Example 28

### 6-Bromo-2-[(4-methyl-6,7-dihydrofuro[3,2-c]pyridin-5(4H)-yl)carbonyl]pyrazolo[1,5-a]pyridine

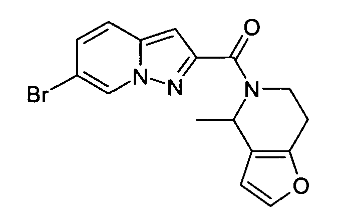

In close analogy to the procedure described in Example 1, 6-bromo-pyrazolo[1,5-a]pyridine-2-carboxylic acid is reacted with 4-methyl-4,5,6,7-tetrahydro-furo[3,2-c]pyridine to provide the title compound.
LC/MS: m/z = 361 (MH⁺)
The stereo-isomers of this compound are separated. The S-configurated compound has a different activity than the R-configurated compound.

### Example 29

### 6-Chloro-2-[(4-methyl-6,7-dihydrofuro[3,2-c]pyridin-5(4H)-yl)carbonyl]pyrazolo[1,5-a]pyridine

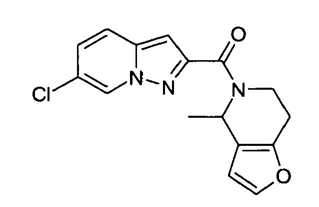

In close analogy to the procedure described in Example 1, 6-chloro-pyrazolo[1,5-a]pyridine-2-carboxylic acid is reacted with 4-methyl-4,5,6,7-tetrahydro-furo[3,2-c]pyridine to provide the title compound.
LC/MS: m/z = 316 (MH⁺)
The stereo-isomers of this compound are separated. The S-configurated compound has a different activity than the R-configurated compound.

### Example 30

### (6-Chloro-pyrazolo[1,5-a]pyridin-2-yl)-(6,7-dihydro-4H-furo[3,2-c]pyridin-5-yl)-methanone

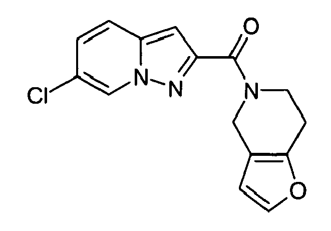

In close analogy to the procedure described in Example 1, 6-chloro-pyrazolo[1,5-a]pyridine-2-carboxylic acid is reacted with 4,5,6,7-tetrahydro-furo[3,2-c]pyridine to provide the title compound in moderate yield.
LC/MS: m/z = 303 (MH⁺)

### Example 31

### (6-Bromo-pyrazolo[1,5-a]pyridin-2-yl)-(6,7-dihydro-4H-furo[3,2-c]pyridin-5-yl)-methanone

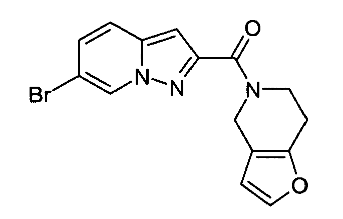

In close analogy to the procedure described in Example 1, 6-bromo-pyrazolo[1,5-a]pyridine-2-carboxylic acid is reacted with 4,5,6,7-tetrahydro-furo[3,2-c]pyridine to provide the title compound in moderate yield.
LC/MS: m/z = 347 (MH⁺)

### Example 32

### (6-Bromo-pyrazolo[1,5-a]pyridin-2-yl)-(6-methyl-6,7-dihydro-4H-furo[3,2-c]pyridin-5-yl)-methanone

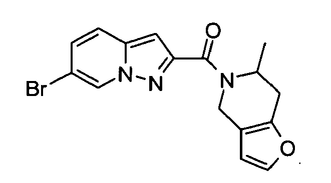

In close analogy to the procedure described in Example 1, 6-bromo-pyrazolo[1,5-a]pyridine-2-carboxylic acid is reacted with 6-methyl-4,5,6,7-tetrahydro-furo[3,2-c]pyridine to provide the title compound in moderate yield.
LC/MS: m/z = 361 (MH⁺)
The stereo-isomers of this compound are separated. The S-configurated compound has a different activity than the R-configurated compound.

### Example 33

### (6-Chloro-pyrazolo[1,5-a]pyridin-2-yl)-(6-methyl-6,7-dihydro-4H-furo[3,2-c]pyridin-5-yl)-methanone

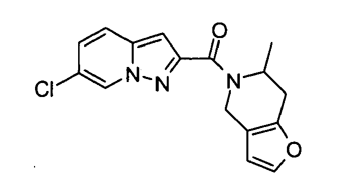

In close analogy to the procedure described in Example 1, 6-chloro-pyrazolo[1,5-a]pyridine-2-carboxylic acid is reacted with 6-methyl-4,5,6,7-tetrahydro-furo[3,2-c]pyridine to provide the title compound in moderate yield.
LC/MS: m/z = 317 (MH⁺)
The stereo-isomers of this compound are separated. The S-configurated compound has a different activity than the R-configurated compound.

### Example 34

### (6-Bromo-pyrazolo[1,5-a]pyridin-2-yl)-(1,4,6,7-tetrahydro-pyrrolo[3,2-c]pyridin-5-yl)-methanone

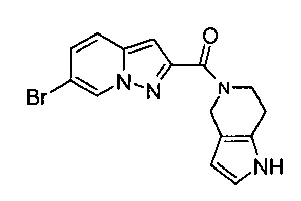

In close analogy to the procedure described in Example 1, 6-bromo-pyrazolo[1,5-a]pyridine-2-carboxylic acid is reacted with 4,5,6,7-tetrahydro-1H-pyrrolo[3,2-c]pyridine to provide the title compound in moderate yield.
LC/MS: m/z = 347 (MH⁺)
The stereo-isomers of this compound are separated. The S-configurated compound has a different activity than the R-configurated compound.

### Example 35

### (6-Chloro-pyrazolo[1,5-a]pyridin-2-yl)-(1,4,6,7-tetrahydro-pyrrolo[3,2-c]pyridin-5-yl)-methanone

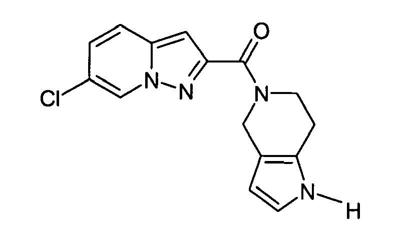

In close analogy to the procedure described in Example 1, 6-chloro-pyrazolo[1,5-a]pyridine-2-carboxylic acid is reacted with 4,5,6,7-tetrahydro-1H-pyrrolo[3,2-c]pyridine to provide the title compound in moderate yield.
LC/MS: m/z = 316 (MH⁺)
The stereo-isomers of this compound are separated. The S-configurated compound has a different activity than the R-configurated compound.

### Example 36

### (6-Chloro-pyrazolo[1,5-a]pyridin-2-yl)-(4-methyl-1,4,6,7-tetrahydro-pyrrolo[3,2-c]pyridin-5-yl)-methanone

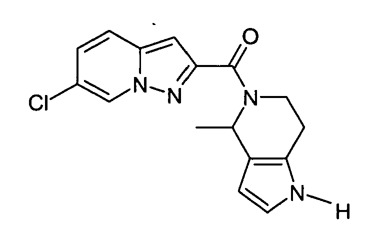

In close analogy to the procedure described in Example 1, 6-chloro-pyrazolo[1,5-a]pyridine-2-carboxylic acid is reacted with 4-methyl-4,5,6,7-tetrahydro-1H-pyrrolo[3,2-c]pyridine to provide the title compound in moderate yield.
LC/MS: m/z = 330 (MH⁺)
The stereo-isomers of this compound are separated. The S-configurated compound has a different activity than the R-configurated compound.

### Example 37

### (6-Bromo-pyrazolo[1,5-a]pyridin-2-yl)-(4-methyl-1,4,6,7-tetrahydro-pyrrolo[3,2-c]pyridin-5-yl)-methanone

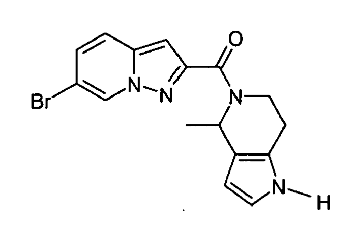

In close analogy to the procedure described in Example 1, 6-bromo-pyrazolo[1,5-a]pyridine-2-carboxylic acid is reacted with 4-methyl-4,5,6,7-tetrahydro-1H-pyrrolo[3,2-c]pyridine to provide the title compound in moderate yield.
LC/MS: m/z = 374 (MH⁺)
The stereo-isomers of this compound are separated. The S-configurated compound has a different activity than the R-configurated compound.

### Example 38

### (6-Bromo-pyrazolo[1,5-a]pyridin-2-yl)-(1-methyl-1,3,4,5-tetrahydro-2-pyrindin-2-yl)-methanone

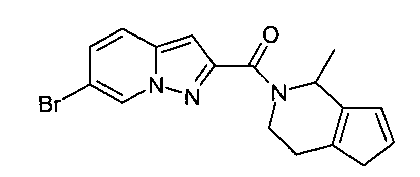

In close analogy to the procedure described in Example 1, 6-bromo-pyrazolo[1,5-a]pyridine-2-carboxylic acid is reacted with 1-methyl-2,3,4,5-tetrahydro-1H-2-pyrindine to provide the title compound in moderate yield.
LC/MS: m/z = 359 (MH⁺)
The stereo-isomers of this compound are separated. The S-configurated compound has a different activity than the R-configurated compound.

### Example 39

### (6-Chloro-pyrazolo[1,5-a]pyridin-2-yl)-(1-methyl-1,3,4,5-tetrahydro-2-pyrindin-2-yl)-methanone

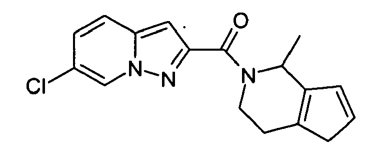

In close analogy to the procedure described in Example 1, 6-chloro-pyrazolo[1,5-a]pyridine-2-carboxylic acid is reacted with 1-methyl-2,3,4,5-tetrahydro-1H-[2]pyrindine to provide the title compound in moderate yield.
LC/MS: m/z = 315 (MH⁺).
The stereo-isomers of this compound are separated. The S-configurated compound has a different activity than the R-configurated compound.

### Example 40

### 5-[(6-Bromopyrazolo[1,5-a]pyridin-2-yl)carbonyl]-3-phenyl-4,5,6,7-tetrahydro-isoxazolo[4,5-c]pyridine

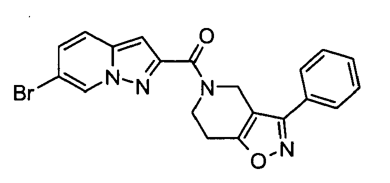

In close analogy to the procedure described in Example 1, 6-bromo-pyrazolo[1,5-a]pyridine-2-carboxylic acid is reacted with 3-phenyl-4,5,6,7-tetrahydro-isoxazolo[4,5-c]pyridine to provide the title compound.
LC/MS: m/z = 528 (MH⁺)

### Example 41

### (6-Bromo-pyrazolo[1,5-a]pyridin-2-yl)-(4-methyl-6,7-dihydro-4H-thieno[3,2-c]pyridin-5-yl)-methanone

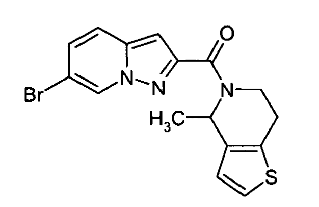

In close analogy to the procedure described in Example 1, 6-bromo-pyrazolo[1,5-a]pyridine-2-carboxylic acid is reacted with 4-methyl-4,5,6,7-tetrahydro-thieno[3,2-c]pyridine to provide the title compound.
LC/MS: m/z = 377 (MH⁺)
The stereo-isomers of this compound are separated. The S-configurated compound has a different activity than the R-configurated compound.

### Example 42

### (6-Chloro-pyrazolo[1,5-a]pyridin-2-yl)-(2,4-dimethyl-6,7-dihydro-4H-furo[3,2-c]pyridin-5-yl)-methanone

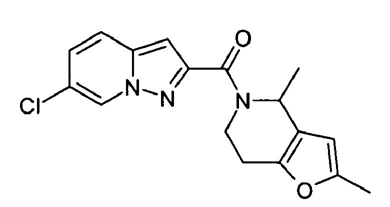

In close analogy to the procedure described in Example 11, 6-chloro-pyrazolo[1,5-a]pyridine-2-carboxylic acid is reacted with 2,4-dimethyl-4,5,6,7-tetrahydro-furo[3,2-c]pyridine to provide the title compound.
LC/MS: m/z = 330 (MH+)
The stereo-isomers of this compound are separated. The S-configurated compound has a different activity than the R-configurated compound.

### Example 43

(6-Bromo-pyrazolo[1,5-a]pyridin-2-yl)-(2,4-dimethyl-6,7-dihydro-4H-furo[3,2-c]pyridin-5-yl)-methanone

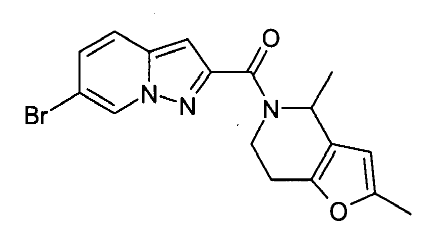

In close analogy to the procedure described in Example 1, 6-bromo-pyrazolo[1,5-a]pyridine-2-carboxylic acid is reacted with 2,4-dimethyl-4,5,6,7-tetrahydro-furo[3,2-c]pyridine to provide the title compound.
LC/MS: m/z = 376 (MH+)
The stereo-isomers of this compound are separated. The S-configurated compound has a different activity than the R-configurated compound.

### Example 44

### (6-Chloro-pyrazolo[1,5-a]pyridin-2-yl)-(3,4-dimethyl-6,7-dihydro-4H-thieno[3,2-c]pyridin-5-yl)-methanone

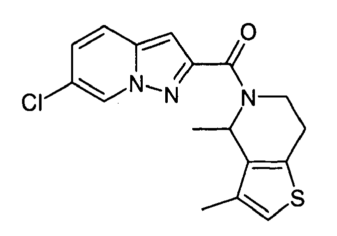

In close analogy to the procedure described in Example 1, 6-chloro-pyrazolo[1,5-a]pyridine-2-carboxylic acid is reacted with 2,4-dimethyl-4,5,6,7-tetrahydro-thieno[3,2-c]pyridine to provide the title compound.
LC/MS: m/z = 346 (MH⁺)
The stereo-isomers of this compound are separated. The S-configurated compound has a different activity than the R-configurated compound.

### Example 45

### (6-Bromo-pyrazolo[1,5-a]pyridin-2-yl)-(1,7,7-trimethyl-1,3,4,7-tetrahydro-2-pyrindin-2-yl)-methanone

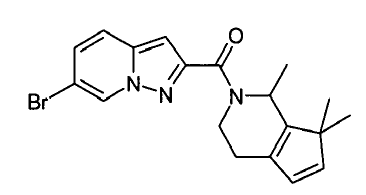

In close analogy to the procedure described in Example 1, 6-bromo-pyrazolo[1,5-a]pyridine-2-carboxylic acid is reacted with 1,7,7-trimethyl-2,3,4,7-tetrahydro-1H-2-pyrindine provide the title compound.
LC/MS: m/z = 387 (MH⁺)
The stereo-isomers of this compound are separated. The S-configurated compound has a different activity than the R-configurated compound.

### Example 46

### (6-Chloro-pyrazolo[1,5-a]pyridin-2-yl)-(1,7,7-trimethyl-1,3,4,7-tetrahydro-2-pyrindin-2-yl)-methanone

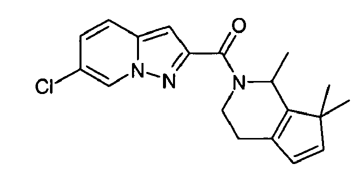

In close analogy to the procedure described in Example 1, 6-chloro-pyrazolo[1,5-a]pyridine-2-carboxylic acid is reacted with 1,7,7-trimethyl-2,3,4,7-tetrahydro-1H-[2]pyrindine to provide the title compound.
LC/MS: m/z = 342 (MH⁺)
The stereo-isomers of this compound are separated. The S-configurated compound has a different activity than the R-configurated compound.

### Example 47

### (6-Bromo-pyrazolo[1,5-a]pyridin-2-yl)-(1,6,6-trimethyl-1,3,4,6-tetrahydro-2-pyrindin-2-yl)-methanone

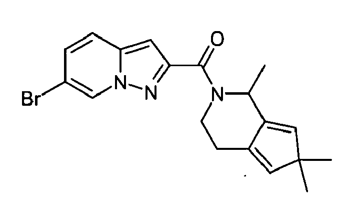

In close analogy to the procedure described in Example 1, 6-bromo-pyrazolo[1,5-a]pyridine-2-carboxylic acid is reacted with 1,6,6-trimethyl-2,3,4,6-tetrahydro-1H-2-pyrindine to provide the title compound.
LC/MS: m/z = 387 (MH⁺)
The stereo-isomers of this compound are separated. The S-configurated compound has a different activity than the R-configurated compound.

### Example 48

### (6-Chloro-pyrazolo[1,5-a]pyridin-2-yl)-(1,6,6-trimethyl-1,3,4,6-tetrahydro-2-pyrindin-2-yl)-methanone

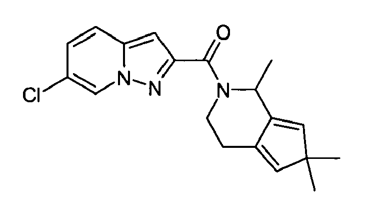

In close analogy to the procedure described in Example 1, 6-chloro-pyrazolo[1,5-a]pyridine-2-carboxylic acid is reacted with 1,6,6-trimethyl-2,3,4,6-tetrahydro-1H-2-pyrindine to provide the title compound.
LC/MS: m/z = 342 (MH⁺)
The stereo-isomers of this compound are separated. The S-configurated compound has a different activity than the R-configurated compound.

### Example 49

### (6-Bromo-pyrazolo[1,5-a]pyridin-2-yl)-(1,5,5-trimethyl-1,3,4,5-tetrahydro-2-pyrindin-2-yl)-methanone

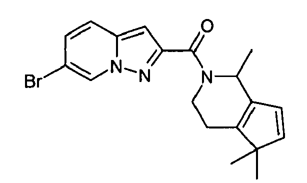

In close analogy to the procedure described in Example 1, 6-bromo-pyrazolo[1,5-a]pyridine-2-carboxylic acid is reacted with 1,5,5-trimethyl-2,3,4,5-tetrahydro-1H-2-pyrindine to provide the title compound.
LC/MS: m/z = 387 (MH⁺)
The stereo-isomers of this compound are separated. The S-configurated compound has a different activity than the R-configurated compound.

### Example 50

### (6-Chloro-pyrazolo[1,5-a]pyridin-2-yl)-(1,5,5-trimethyl-1,3,4,5-tetrahydro-2-pyrindin-2-yl)-methanone

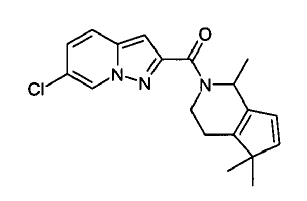

In close analogy to the procedure described in Example 1, 6-chloro-pyrazolo[1,5-a]pyridine-2-carboxylic acid is reacted with 1,5,5-trimethyl-2,3,4,5-tetrahydro-1H-2-pyrindine to provide the title compound.
LC/MS: m/z = 342 (MH⁺)
The stereo-isomers of this compound are separated. The S-configurated compound has a different activity than the R-configurated compound.

### Example 51

### (6-Bromo-pyrazolo[1,5-a]pyridin-2-yl)-[4-methyl-1-(1H-tetrazol-5-yl)-1,4,6,7-tetrahydro-pyrrolo[3,2-c]pyridin-5-yl]-methanone

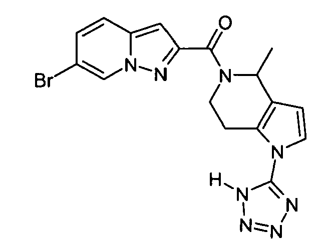

(6-Bromo-pyrazolo[1,5-a]pyridin-2-yl)-(4-methyl-1,4,6,7-tetrahydro-pyrrolo[3,2-c]pyridin-5-yl)-methanone is reacted with 1H tetrazole under Buchwald reaction conditions to provide the title compound.
The stereo-isomers of this compound are separated. The S-configurated compound has a different activity than the R-configurated compound.
LC/MS: m/z = 428 (MH⁺)
(see S. L. Buchwald, Acc. Chem. Res. 1998, 31, 805; S. L. Buchwald, J. Organomet. Chem. 1999, 576, 125,37; S. L. Buchwald, Angew. Chem. Int. Ed. Engl. 1999, 38, 2413).

### Example 52

### (6-Chloro-pyrazolo[1,5-a]pyridin-2-yl)- [4-methyl-1-(1H-tetrazol-5-yl)-1,4,6,7-tetrahydro-pyrrolo[3,2-c]pyridin-5-yl]-methanone

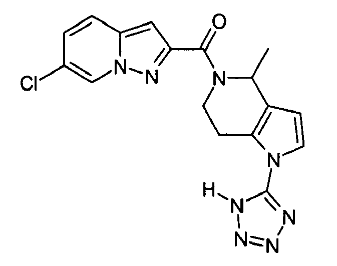

In close analogy to the procedure described in Example 51, (6-chloro-pyrazolo[1,5-a]pyridin-2-yl)-(4-methyl-1,4,6,7-tetrahydro-pyrrolo[3,2-c]pyridin-5-yl)-methanone is reacted with 1H tetrazole to provide the title compound.
LC/MS: m/z = 383 (MH⁺)
The stereo-isomers of this compound are separated. The S-configurated compound has a different activity than the R-configurated compound.

### Example 53

### (6-Bromo-pyrazolo[1,5-a]pyridin-2-yl)-[7-methyl-1-(1H-tetrazol-5-yl)-1,4,5,7-tetrahydro-pyrrolo[2,3-c]pyridin-6-yl]-methanone

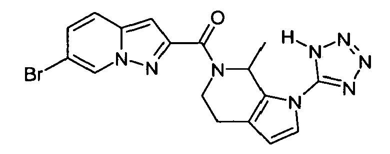

In close analogy to the procedure described in Example 51, (6-bromo-pyrazolo[1,5-a]pyridin-2-yl)-(7-methyl-1,4,5,7-tetrahydro-pyrrolo[2,3-c]pyridin-6-yl)-methanone is reacted with 1H tetrazole to provide the title compound.
LC/MS: m/z = 428 (MH⁺)
The stereo-isomers of this compound are separated. The S-configurated compound has a different activity than the R-configurated compound.

### Example 54

### (6-Chloro-pyrazolo[1,5-a]pyridin-2-yl)-[7-methyl-1-(1H-tetrazol-5-yl)-1,4,5,7-tetrahydro-pyrrolo[2,3-c]pyridin-6-yl]-methanone

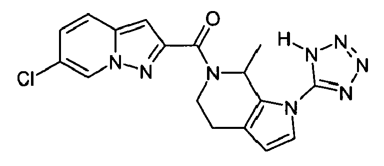

In close analogy to the procedure described in Example 51, (6-chloro-pyrazolo[1,5-a]pyridin-2-yl)-(7-methyl-1,4,5,7-tetrahydro-pyrrolo[2,3-c]pyridin-6-yl)-methanone is reacted with 1H tetrazole to provide the title compound.
LC/MS: m/z = 383 (MH⁺)
The stereo-isomers of this compound are separated. The S-configurated compound has a different activity than the R-configurated compound.

### Example 55

### (6-Bromo-pyrazolo[1,5-a]pyridin-2-yl)-[4-methyl-1-(1H-tetrazol-5-yl)-2,4,6,7-tetrahydro-pyrrolo[3,4-c]pyridin-5-yl]-methanone

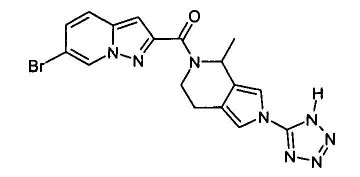

In close analogy to the procedure described in Example 51, (6-bromo-pyrazolo[1,5-a]pyridin-2-yl)-(4-methyl-2,4,6,7-tetrahydro-pyrrolo[3,4-c]pyridin-5-yl)-methanone is reacted with 1H tetrazole to provide the title compound.
LC/MS: m/z = 428 (MH⁺)
The stereo-isomers of this compound are separated. The S-configurated compound has a different activity than the R-configurated compound.

### Example 56

### (6-Chloro-pyrazolo[1,5-a]pyridin-2-yl)-[4-methyl-1-(1H-tetrazol-5-yl)-2,4,6,7-tetrahydro-pyrrolo[3,4-c]pyridin-5-yl]-methanone

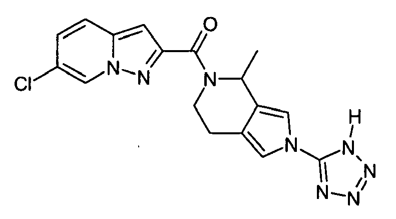

In close analogy to the procedure described in Example 51, (6-chloro-pyrazolo[1,5-a]pyridin-2-yl)-(4-methyl-2,4,6,7-tetrahydro-pyrrolo[3,4-c]pyridin-5-yl)-methanone is reacted with 1H tetrazole to provide the title compound.
LC/MS: m/z = 383 (MH⁺)
The stereo-isomers of this compound are separated. The S-configurated compound has a different activity than the R-configurated compound.

### Example 57

### (2-Bromo-7-methyl-4,7-dihydro-5H-furo[2,3-c]pyridin-6-yl)-(6-bromo-pyrazolo[1,5-a]pyridin-2-yl)-methanone

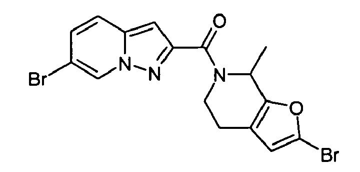

In close analogy to the procedure described in Example 1, 6-bromo-pyrazolo[1,5-a]pyridine-2-carboxylic acid is reacted with 2-bromo-7-methyl-4,5,6,7-tetrahydro-furo[2,3-c]pyridine to provide the title compound.
LC/MS: m/z = 440 (MH+)
The stereo-isomers of this compound are separated. The S-configurated compound has a different activity than the R-configurated compound.

### Example 58

### (2-Chloro-7-methyl-4,7-dihydro-5H-furo[2,3-c]pyridin-6-yl)-(6-bromo-pyrazolo[1,5-a]pyridin-2-yl)-methanone

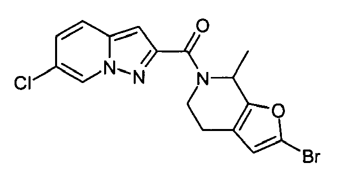

In close analogy to the procedure described in Example 1, 6-chloro-pyrazolo[1,5-a]pyridine-2-carboxylic acid is reacted with 2-bromo-7-methyl-4,5,6,7-tetrahydro-furo[2,3-c]pyridine to provide the title compound.
LC/MS: m/z = 395 (MH+)
The stereo-isomers of this compound are separated. The S-configurated compound has a different activity than the R-configurated compound.

### Example 59

### 2-Bromo-7-methyl-4,7-dihydro-5H-furo[2,3-c]pyridin-6-yl)-(6-chloro-pyrazolo[1,5-a]pyridin-2-yl)-methanone

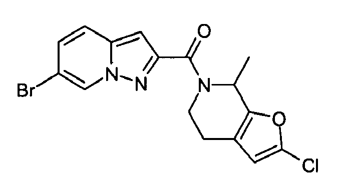

In close analogy to the procedure described in Example 1, 6-bromo-pyrazolo[1,5-a]pyridine-2-carboxylic acid is reacted with 2-chloro-7-methyl-4,5,6,7-tetrahydro-furo[2,3-c]pyridine to provide the title compound.
LC/MS: m/z = 395 (MH+)
The stereo-isomers of this compound are separated. The S-configurated compound has a different activity than the R-configurated compound.

### Example 60

### 2-Chloro-7-methyl-4,7-dihydro-5H-furo[2,3-c]pyridin-6-yl)-(6-chloro-pyrazolo[1,5-a]pyridin-2-yl)-methanone

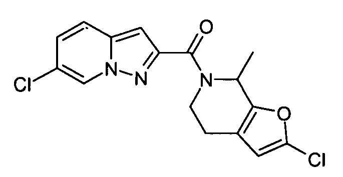

In close analogy to the procedure described in Example 1, 6-chloro-pyrazolo[1,5-a]pyridine-2-carboxylic acid is reacted with 2-chloro-7-methyl-4,5,6,7-tetrahydro-furo[2,3-c]pyridine to provide the title compound.
LC/MS: m/z = 350 (MH+)
The stereo-isomers of this compound are separated. The S-configurated compound has a different activity than the R-configurated compound.

### Example 61

### (2-Bromo-7-methyl-4,7-dihydro-5H-thieno[2,3-c]pyridin-6-yl)-(6-bromo-pyrazolo[1,5-a]pyridin-2-yl)-methanone

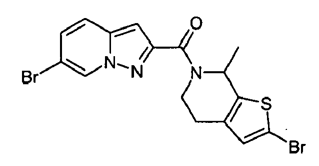

In close analogy to the procedure described in Example 1, 6-bromo-pyrazolo[1,5-a]pyridine-2-carboxylic acid is reacted with 2-bromo-7-methyl-4,5,6,7-tetrahydro-thieno[2,3-c]pyridine to provide the title compound.
LC/MS: m/z = 456 (MH+)
The stereo-isomers of this compound are separated. The S-configurated compound has a different activity than the R-configurated compound.

### Example 62

### (2-Chloro-7-methyl-4,7-dihydro-5H-thieno[2,3-c]pyridin-6-yl)-(6-bromo-pyrazolo[1,5-a]pyridin-2-yl)-methanone

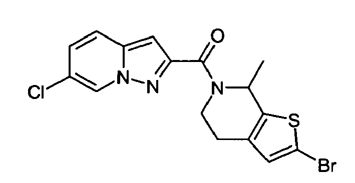

In close analogy to the procedure described in Example 1, 6-chloro-pyrazolo[1,5-a]pyridine-2-carboxylic acid is reacted with 2-bromo-7-methyl-4,5,6,7-tetrahydro-thieno[2,3-c]pyridine to provide the title compound.
LC/MS: m/z = 411 (MH+)
The stereo-isomers of this compound are separated. The S-configurated compound has a different activity than the R-configurated compound.

### Example 63

### (2-Bromo-7-methyl-4,7-dihydro-5H-thieno[2,3-c]pyridin-6-yl)-(6-chloro-pyrazolo[1,5-a]pyridin-2-yl)-methanone

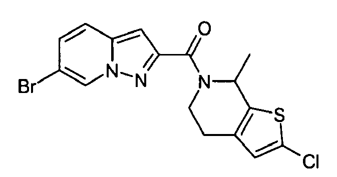

In close analogy to the procedure described in Example 1, 6-bromo-pyrazolo[1,5-a]pyridine-2-carboxylic acid is reacted with 2-chloro-7-methyl-4,5,6,7-tetrahydro-thieno[2,3-c]pyridine to provide the title compound.
LC/MS: m/z = 411 (MH+)
The stereo-isomers of this compound are separated. The S-configurated compound has a different activity than the R-configurated compound.

### Example 64

### (2-Chloro-7-methyl-4,7-dihydro-5H-thieno[2,3-c]pyridin-6-yl)-(6-chloro-pyrazolo[1,5-a]pyridin-2-yl)-methanone

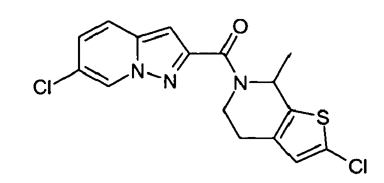

In close analogy to the procedure described in Example 1, 6-chloro-pyrazolo[1,5-a]pyridine-2-carboxylic acid is reacted with 2-chloro-7-methyl-4,5,6,7-tetrahydro-thieno[2,3-c]pyridine to provide the title compound.
LC/MS: m/z = 366 (MH+)
The stereo-isomers of this compound are separated. The S-configurated compound has a different activity than the R-configurated compound.

### Example 65

### (3-Bromo-7-methyl-4,7-dihydro-5H-furo[2,3-c]pyridin-6-yl)-(6-bromo-pyrazolo[1,5-a]pyridin-2-yl)-methanone

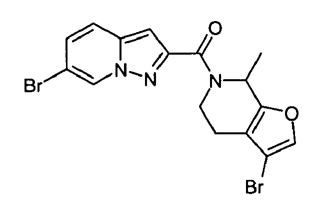

In close analogy to the procedure described in Example 1, 6-bromo-pyrazolo[1,5-a]pyridine-2-carboxylic acid is reacted with 3-bromo-7-methyl-4,5,6,7-tetrahydro-furo[2,3-c]pyridine to provide the title compound.
LC/MS: m/z = 440 (MH+)
The stereo-isomers of this compound are separated. The S-configurated compound has a different activity than the R-configurated compound.

### Example 66

### (3-Chloro-7-methyl-4,7-dihydro-5H-furo[2,3-c]pyridin-6-yl)-(6-bromo-pyrazolo[1,5-a]pyridin-2-yl)-methanone

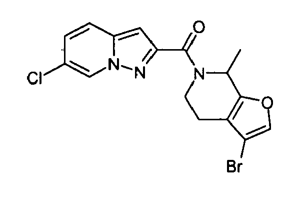

In close analogy to the procedure described in Example 1, 6-chloro-pyrazolo[1,5-a]pyridine-2-carboxylic acid is reacted with 3-bromo-7-methyl-4,5,6,7-tetrahydro-furo[2,3-c]pyridine to provide the title compound.
LC/MS: m/z = 395 (MH+)
The stereo-isomers of this compound are separated. The S-configurated compound has a different activity than the R-configurated compound.

### Example 67

### (3-Bromo-7-methyl-4,7-dihydro-5H-furo[2,3-c]pyridin-6-yl)-(6-chloro-pyrazolo[1,5-a]pyridin-2-yl)-methanone

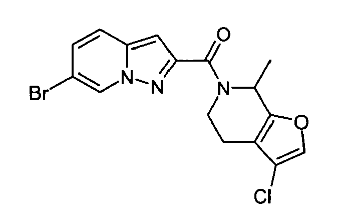

In close analogy to the procedure described in Example 1, 6-bromo-pyrazolo[1,5-a]pyridine-2-carboxylic acid is reacted with 3-chloro-7-methyl-4,5,6,7-tetrahydro-furo[2,3-c]pyridine to provide the title compound.
LC/MS: m/z = 395 (MH+)
The stereo-isomers of this compound are separated. The S-configurated compound has a different activity than the R-configurated compound.

### Example 68

### (3-Chloro-7-methyl-4,7-dihydro-5H-furo[2,3-c]pyridin-6-yl)-(6-chloro-pyrazolo[1,5-a]pyridin-2-yl)-methanone

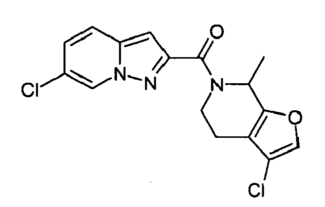

In close analogy to the procedure described in Example 1, 6-chloro-pyrazolo[1,5-a]pyridine-2-carboxylic acid is reacted with 3-chloro-7-methyl-4,5,6,7-tetrahydro-furo[2,3-c]pyridine to provide the title compound.
LC/MS: m/z = 350 (MH+)
The stereo-isomers of this compound are separated. The S-configurated compound has a different activity than the R-configurated compound.

### Example 69

### (3-Bromo-7-methyl-4,7-dihydro-5H-thieno[2,3-c]pyridin-6-yl)-(6-bromo-pyrazolo[1,5-a]pyridin-2-yl)-methanone

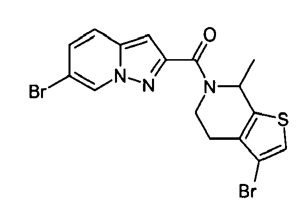

In close analogy to the procedure described in Example 1, 6-bromo-pyrazolo[1,5-a]pyridine-2-carboxylic acid is reacted with 3-bromo-7-methyl-4,5,6,7-tetrahydro-thieno[2,3-c]pyridine to provide the title compound.
LC/MS: m/z = 456 (MH+)
The stereo-isomers of this compound are separated. The S-configurated compound has a different activity than the R-configurated compound.

### Example 70

### (3-Chloro-7-methyl-4,7-dihydro-5H-thieno[2,3-c]pyridin-6-yl)-(6-bromo-pyrazolo[1,5-a]pyridin-2-yl)-methanone

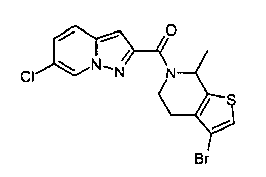

In close analogy to the procedure described in Example 1, 6-chloro-pyrazolo[1,5-a]pyridine-2-carboxylic acid is reacted with 3-bromo-7-methyl-4,5,6,7-tetrahydro-thieno[2,3-c]pyridine to provide the title compound.
LC/MS: m/z = 411 (MH+)
The stereo-isomers of this compound are separated. The S-configurated compound has a different activity than the R-configurated compound.

### Example 71

### (3-Bromo-7-methyl-4,7-dihydro-5H-thieno[2,3-c]pyridin-6-yl)-(6-chloro-pyrazolo[1,5-a]pyridin-2-yl)-methanone

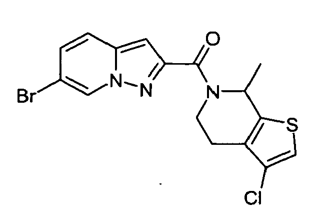

In close analogy to the procedure described in Example 1, 6-bromo-pyrazolo[1,5-a]pyridine-2-carboxylic acid is reacted with 3-chloro-7-methyl-4,5,6,7-tetrahydro-thieno[2,3-c]pyridine to provide the title compound.
LC/MS: m/z = 411 (MH+)
The stereo-isomers of this compound are separated. The S-configurated compound has a different activity than the R-configurated compound.

### Example 72

### (3-Chloro-7-methyl-4,7-dihydro-5H-thieno[2,3-c]pyridin-6-yl)-(6-chloro-pyrazolo[1,5-a]pyridin-2-yl)-methanone

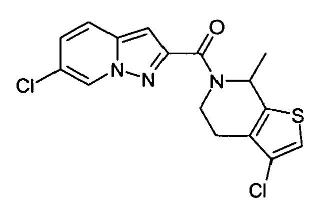

In close analogy to the procedure described in Example 1, 6-chloro-pyrazolo[1,5-a]pyridine-2-carboxylic acid is reacted with 3-chloro-7-methyl-4,5,6,7-tetrahydro-thieno[2,3-c]pyridine to provide the title compound.
LC/MS: m/z = 366 (MH+)
The stereo-isomers of this compound are separated. The S-configurated compound has a different activity than the R-configurated compound.

### Example 73

### 1-[5-(6-Bromo-pyrazolo[1,5-a]pyridine-2-carbonyl)-4-methyl-4,5,6,7-tetrahydro-pyrrolo[3,2-c]pyridin-1-yl]-ethanone

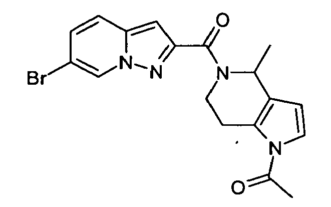

(6-Bromo-pyrazolo[1,5-a]pyridin-2-yl)-(4-methyl-1,4,6,7-tetrahydro-pyrrolo[3,2-c]pyridin-5-yl)-methanone is reacted with acetic acid to provide the title compound.
LC/MS: m/z = 402 (MH⁺)
The stereo-isomers of this compound are separated. The S-configurated compound has a different activity than the R-configurated compound.

### Example 74

### 1-[5-(6-Chloro-pyrazolo[1,5-a]pyridine-2-carbonyl)-4-methyl-4,5,6,7-tetrahydro-pyrrolo[3,2-c]pyridin-1-yl]-ethanone

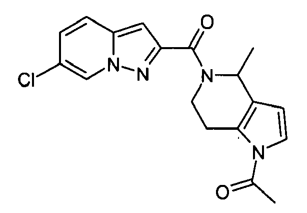

(6-Chloro-pyrazolo[1,5-a]pyridin-2-yl)-(4-methyl-1,4,6,7-tetrahydro-pyrrolo[3,2-c]pyridin-5-yl)-methanone is reacted with acetic acid to provide the title compound.
LC/MS: m/z = 357 (MH⁺)
The stereo-isomers of this compound are separated. The S-configurated compound has a different activity than the R-configurated compound.

### Example 75

### 1-[6-(6-Bromo-pyrazolo[1,5-a]pyridine-2-carbonyl)-7-methyl-4,5,6,7-tetrahydro-pyrrolo[2,3-c]pyridin-1-yl]-ethanone

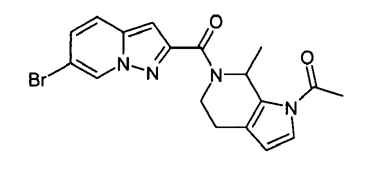

(6-Bromo-pyrazolo[1,5-a]pyridin-2-yl)-(7-methyl-1,4,5,7-tetrahydro-pyrrolo[2,3-c]pyridin-6-yl)-methanone is reacted with acetic acid to provide the title compound.
LC/MS: m/z = 402 (MH⁺)
The stereo-isomers of this compound are separated. The S-configurated compound has a different activity than the R-configurated compound.

### Example 76

### 1-[6-(6-Chloro-pyrazolo[1,5-a]pyridine-2-carbonyl)-7-methyl-4,5,6,7-tetrahydro-pyrrolo[2,3-c]pyridin-1-yl]-ethanone

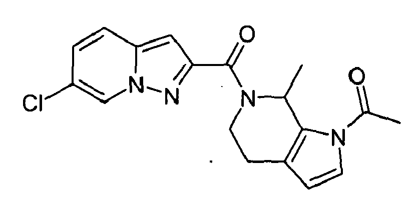

(6-Chloro-pyrazolo[1,5-a]pyridin-2-yl)-(7-methyl-1,4,5,7-tetrahydro-pyrrolo[2,3-c]pyridin-6-yl)-methanone is reacted with acetic acid to provide the title compound.
LC/MS: m/z = 357 (MH⁺)
The stereo-isomers of this compound are separated. The S-configurated compound has a different activity than the R-configurated compound.

### Example 77

### (6-Bromo-pyrazolo[1,5-a]pyridin-2-yl)-(4-methyl-6,7-dihydro-4H-oxazolo[4,5-c]pyridin-5-yl)-methanone

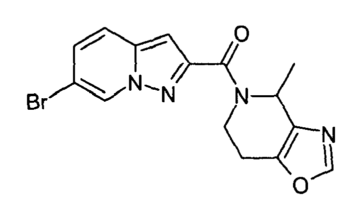

6-bromo-pyrazolo[1,5-a]pyridine-2-carboxylic acid is reacted with 4-methyl-4,5,6,7-tetrahydro-oxazolo[4,5-c]pyridine to provide the title compound.
LC/MS: m/z = 362 (MH⁺)
The stereo-isomers of this compound are separated. The S-configurated compound has a different activity than the R-configurated compound.

### Example 78

### 6-Bromo-pyrazolo[1,5-a]pyridine-2-carboxylic acid (1-phenyl-ethyl)-amide

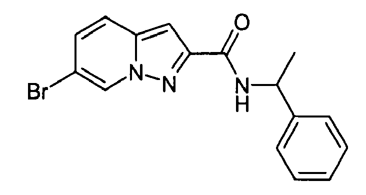

6-bromo-pyrazolo[1,5-a]pyridine-2-carboxylic acid is reacted with α-phenethylamine to provide the title compound.
LC/MS: m/z = 345 (MH⁺)
The stereo-isomers of this compound are separated. The S-configurated compound has a different activity than the R-configurated compound.

### Example 79

### 6-Chloro-pyrazolo[1,5-a]pyridine-2-carboxylic acid (1-phenyl-ethyl)-amide

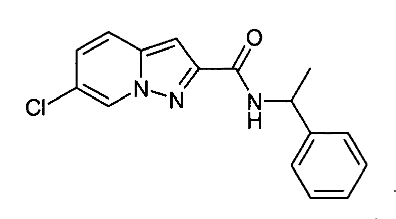

6-Chloro-pyrazolo[1,5-a]pyridine-2-carboxylic acid is reacted with α-phenethylamine to provide the title compound.
LC/MS: m/z = 300 (MH⁺)
The stereo-isomers of this compound are separated. The S-configurated compound has a different activity than the R-configurated compound.

### Example 80

### (6-Bromo-pyrazolo[1,5-a]pyridin-2-yl)-(2,4-dimethyl-6,7-dihydro-4H-oxazolo[4,5-c]pyridin-5-yl)-methanone

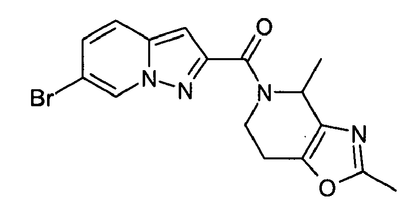

(6-Bromo-pyrazolo[1,5-a]pyridin-2-yl)-(7-methyl-1,4,5,7-tetrahydro-pyrrolo[2,3-c]pyridin-6-yl)-methanone is reacted with 2,4-dimethyl-4,5,6,7-tetrahydro-oxazolo[4,5-c]pyridine to provide the title compound.
LC/MS: m/z = 376 (MH⁺)
The stereo-isomers of this compound are separated. The S-configurated compound has a different activity than the R-configurated compound.

### Example 81

### (6-Bromo-pyrazolo[1,5-a]pyridin-2-yl)-(4-methyl-2-trifluoromethyl-6,7-dihydro-4H-oxazolo[4,5-c]pyridin-5-yl)-methanone

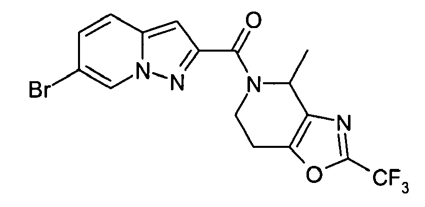

(6-Bromo-pyrazolo[1,5-a]pyridin-2-yl)-(7-methyl-1,4,5,7-tetrahydro-pyrrolo[2,3-c]pyridin-6-yl)-methanone is reacted with 4-methyl-2-trifluoromethyl-4,5,6,7-tetrahydro-oxazolo[4,5-c]pyridine to provide the title compound.
LC/MS: m/z = 430 (MH⁺)
The stereo-isomers of this compound are separated. The S-configurated compound has a different activity than the R-configurated compound.

### Example 82

### (6-Bromo-pyrazolo[1,5-a]pyridin-2-yl)-(2-methoxy-4-methyl-6,7-dihydro-4H-oxazolo[4,5-c]pyridin-5-yl)-methanone

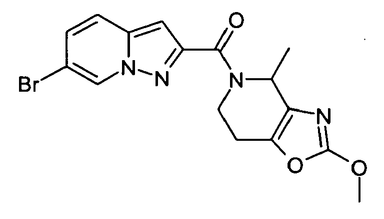

(6-Bromo-pyrazolo[1,5-a]pyridin-2-yl)-(7-methyl-1,4,5,7-tetrahydro-pyrrolo[2,3-c]pyridin-6-yl)-methanone is reacted with 2-methoxy-4-methyl-4,5,6,7-tetrahydro-oxazolo[4,5-c]pyridine to provide the title compound.
LC/MS: m/z = 392 (MH⁺).
The stereo-isomers of this compound are separated. The S-configurated compound has a different activity than the R-configurated compound.

### Example 83

### (6-Bromo-pyrazolo[1,5-a]pyridin-2-yl)-(6-hydroxy-7-methoxy-1-methyl-3,4-dihydro-1H-isoquinolin-2-yl)-methanone

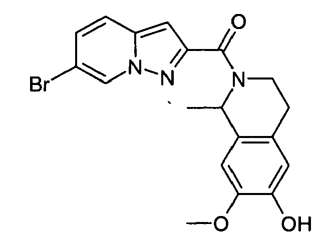

In close analogy to the procedure described in Example 1, 6-bromo-pyrazolo[1,5-a]pyridine-2-carboxylic acid is reacted with 1-methyl-1,2,3,4-tetrahydro-isoquinoline to provide the title compound.
LC/MS: m/z = 416 (MH⁺)
The stereo-isomers of this compound are separated. The S-configurated compound has a different activity than the R-configurated compound.

### Example 84

### (6-Chloro-pyrazolo[1,5-a]pyridin-2-yl)-(1-methyl-3,4-dihydro-1H-isoquinolin-2-yl)-methanone

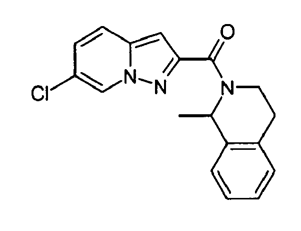

In close analogy to the procedure described in Example 1, 6-chloro-pyrazolo[1,5-a]pyridine-2-carboxylic acid is reacted with 1-methyl-1,2,3,4-tetrahydro-isoquinoline to provide the title compound.
LC/MS: m/z = 327 (MH⁺)
The stereo-isomers of this compound are separated. The S-configurated compound has a different activity than the R-configurated compound.

### Example 85

### (6-Chloro-pyrazolo[1,5-a]pyridin-2-yl)-(3-methyl-3,4-dihydro-1H-isoquinolin-2-yl)-methanone

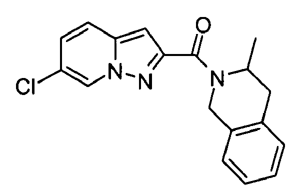

In close analogy to the procedure described in Example 1, 6-chloro-pyrazolo[1,5-a]pyridine-2-carboxylic acid is reacted with 3-methyl-1,2,3,4-tetrahydro-isoquinoline to provide the title compound in moderate yield.
LC/MS: m/z = 326 (MH⁺)
The stereoisomers of this compound are separated. The S-configurated compound has a different activity than the R-configurated compound.

### Example 86

### (6-Bromo-pyrazolo[1,5-a]pyridin-2-yl)-(3-methyl-3,4-dihydro-1H-isoquinolin-2-yl)-methanone

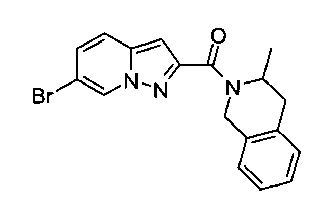

In close analogy to the procedure described in Example 1, 6-bromo-pyrazolo[1,5-a]pyridine-2-carboxylic acid is reacted with 3-methyl-1,2,3,4-tetrahydro-isoquinoline to provide the title compound in moderate yield.
LC/MS: m/z = 371 (MH⁺)
The stereoisomers of this compound are separated. The S-configurated compound has a different activity than the R-configurated compound.

### Example 87

### (6-Bromo-pyrazolo[1,5-a]pyridin-2-yl)-(3,3-dimethyl-3,4-dihydro-1H-isoquinolin-2-yl)-methanone

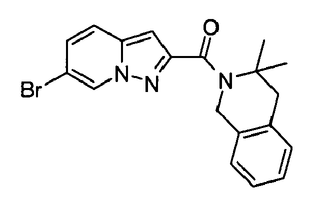

In close analogy to the procedure described in Example 1, 6-bromo-pyrazolo[1,5-a]pyridine-2-carboxylic acid is reacted with 3,3-dimethyl-1,2,3,4-tetrahydro-isoquinoline to provide the title compound in moderate yield.
LC/MS: m/z = 385 (MH⁺)

### Example 88

### (6-Chloro-pyrazolo[1,5-a]pyridin-2-yl)-(3,3-dimethyl-3,4-dihydro-1H-isoquinolin-2-yl)-methanone

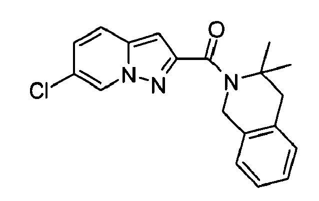

In close analogy to the procedure described in Example 1, 6-chloro-pyrazolo[1,5-a]pyridine-2-carboxylic acid is reacted with 3,3-dimethyl-1,2,3,4-tetrahydro-isoquinoline to provide the title compound in moderate yield.
LC/MS: m/z = 340 (MH⁺)

### Example 89

### (6-Chloro-pyrazolo[1,5-a]pyridin-2-yl)-(3,4-dihydro-1H-isoquinolin-2-yl)-methanone

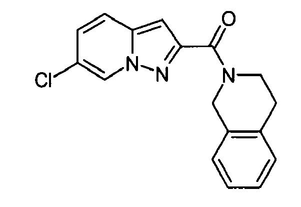

In close analogy to the procedure described in Example 1, 6-chloro-pyrazolo[1,5-a]pyridine-2-carboxylic acid is reacted with 1,2,3,4-tetrahydro-isoquinoline to provide the title compound in moderate yield.
LC/MS: m/z = 391 (MH⁺)

### Example 90

### (6-Chloro-pyrazolo[1,5-a]pyridin-2-yl)-(6,7-dimethoxy-1-methyl-3,4-dihydro-1H-isoquinolin-2-yl)-methanone

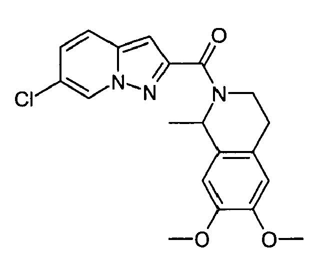

In close analogy to the procedure described in Example 1, 6-chloro-pyrazolo[1,5-a]pyridine-2-carboxylic acid is reacted with 6,7-dimethoxy-1-methyl-1,2,3,4-tetrahydro-isoquinoline to provide the title compound in moderate yield.
LC/MS: m/z = 386 (MH⁺)
The stereo-isomers of this compound are separated. The S-configurated compound has a different activity than the R-configurated compound.

### Example 91

### (6-Chloro-pyrazolo[1,5-a]pyridin-2-yl)-(6,7-dimethoxy-3,4-dihydro-1H-isoquinolin-2-yl)-methanone

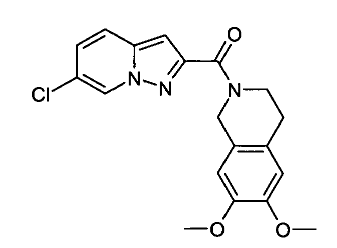

In close analogy to the procedure described in Example 1, 6-chloro-pyrazolo[1,5-a]pyridine-2-carboxylic acid is reacted with 6,7-dimethoxy-1,2,3,4-tetrahydro-isoquinoline to provide the title compound in moderate yield.
LC/MS: m/z = 372 (MH⁺)

### Example 92

### (6-Bromo-pyrazolo[1,5-a]pyridin-2-yl)-(6,7-dimethoxy-3,4-dihydro-1H-isoquinolin-2-yl)-methanone

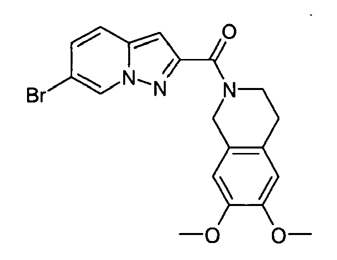

In close analogy to the procedure described in Example 1, 6-bromo-pyrazolo[1,5-a]pyridine-2-carboxylic acid is reacted with 6,7-dimethoxy-1,2,3,4-tetrahydro-isoquinoline to provide the title compound as a solid.
LC/MS: m/z = 417 (MH⁺)

### Example 93

### (6-Bromo-pyrazolo[1,5-a]pyridin-2-yl)-(6,7-dimethoxy-1-methyl-3,4-dihydro-1H-isoquinolin-2-yl)-methanone

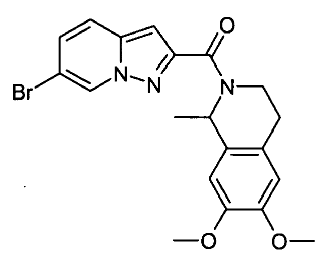

In close analogy to the procedure described in Example 1, 6-bromo-pyrazolo[1,5-a]pyridine-2-carboxylic acid is reacted with 6,7-dimethoxy-1-methyl-1,2,3,4-tetrahydro-isoquinoline to provide the title compound as a solid.
LC/MS: m/z = 431 (MH⁺)
The stereo-isomers of this compound are separated. The S-configurated compound has a different activity than the R-configurated compound.

### Example 94

### (6-Bromo-pyrazolo[1,5-a]pyridin-2-yl)-(6-hydroxy-7-methoxy-3,4-dihydro-1H-isoquinolin-2-yl)-methanone

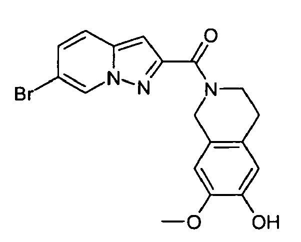

In close analogy to the procedure described in Example 1, 6-bromo-pyrazolo[1,5-a]pyridine-2-carboxylic acid is reacted with 7-methoxy-1,2,3,4-tetrahydro-isoquinolin-6-ol to provide the title compound as a solid.
LC/MS: m/z = 403 (MH⁺)

### Example 95

### (6-Chloro-pyrazolo[1,5-a]pyridin-2-yl)-(6-hydroxy-7-methoxy-3,4-dihydro-1H-isoquinolin-2-yl)-methanone

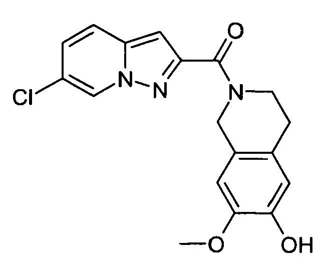

In close analogy to the procedure described in Example 1, 6-chloro-pyrazolo[1,5-a]pyridine-2-carboxylic acid is reacted with 7-methoxy-1,2,3,4-tetrahydro-isoquinolin-6-ol to provide the title compound as a solid.
LC/MS: m/z = 358 (MH⁺)

### Example 96

### (6-Chloro-pyrazolo[1,5-a]pyridin-2-yl)-(6-hydroxy-7-methoxy-1-methyl-3,4-dihydro-1H-isoquinolin-2-yl)-methanone

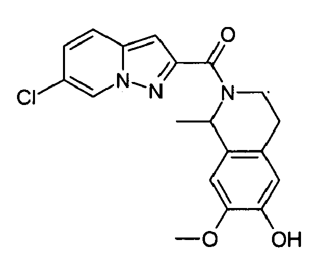

In close analogy to the procedure described in Example 1, 6-chloro-pyrazolo[1,5-a]pyridine-2-carboxylic acid is reacted with 7-methoxy-1-methyl-1,2,3,4-tetrahydro-isoquinolin-6-ol to provide the title compound as a colorless solid.
LC/MS: m/z = 372 (MH⁺)
The stereo-isomers of this compound are separated. The S-configurated compound has a different activity than the R-configurated compound.

### Example 97

### (6-Bromo-pyrazolo[1,5-a]pyridin-2-yl)-(6,7-dimethoxy-3-methyl-3,4-dihydro-1H-isoquinolin-2-yl)-methanone

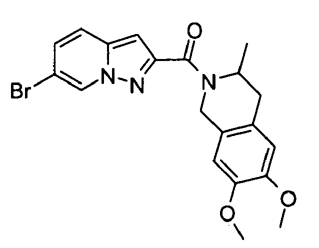

In close analogy to the procedure described in Example 1, 6-bromo-pyrazolo[1,5-a]pyridine-2-carboxylic acid is reacted with 6,7-dimethoxy-3-methyl-1,2,3,4-tetrahydro-isoquinoline to provide the title compound as a colorless solid.
LC/MS: m/z = 431 (MH⁺)
The stereoisomers of this compound are separated. The S-configurated compound has a different activity than the R-configuratedcompound.

### Example 98

### (6-Chloro-pyrazolo[1,5-a]pyridin-2-yl)-(6,7-dimethoxy-3-methyl-3,4-dihydro-1H-isoquinolin-2-yl)-methanone

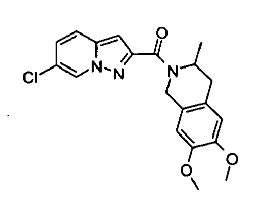

In close analogy to the procedure described in Example 1, 6-chloro-pyrazolo[1,5-a]pyridine-2-carboxylic acid is reacted with 6,7-dimethoxy-3-methyl-1,2,3,4-tetrahydro-isoquinoline to provide the title compound as a colorless solid.
LC/MS: m/z = 386 (MH⁺)
The stereoisomers of this compound are separated. The S-configurated compound has a different activity than the R-configurated compound.

### Example 99

### (6-Bromo-pyrazolo[1,5-a]pyridin-2-yl)-(6,7-dimethoxy-3,3-dimethyl-3,4-dihydro-1H-isoquinolin-2-yl)-methanone

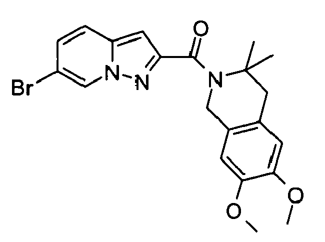

In close analogy to the procedure described in Example 1, 6-bromo-pyrazolo[1,5-a]pyridine-2-carboxylic acid is reacted with 6,7-dimethoxy-3,3-dimethyl-1,2,3,4-tetrahydro-isoquinoline to provide the title compound as a colorless solid.
LC/MS: m/z = 445 (MH⁺)

### Example 100

### (6-Chloro-pyrazolo[1,5-a]pyridin-2-yl)-(6,7-dimethoxy-3,3-dimethyl-3,4-dihydro-1H-isoquinolin-2-yl)-methanone

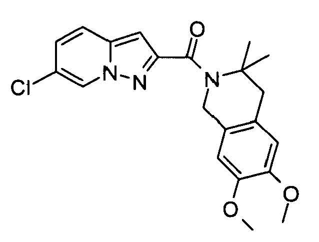

In close analogy to the procedure described in Example 1, 6-chloro-pyrazolo[1,5-a]pyridine-2-carboxylic acid is reacted with 6,7-dimethoxy-3,3-dimethyl-1,2,3,4-tetrahydro-isoquinoline to provide the title compound as a solid.
LC/MS: m/z = 400 (MH⁺)

### Example 101

### (6-Bromo-pyrazolo[1,5-a]pyridin-2-yl)-(8-methyl-5,8-dihydro-6H-[1,7]naphthyridin-7-yl)-methanone

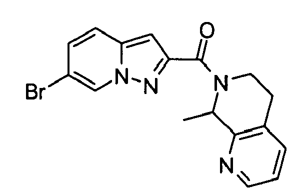

In close analogy to the procedure described in Example 1, 6-bromo-pyrazolo[1,5-a]pyridine-2-carboxylic acid is reacted with 8-methyl-5,6,7,8-tetrahydro-[1,7]naphthyridine to provide the title compound in moderate yield.
LC/MS: m/z = 372 (MH⁺)
The stereo-isomers of this compound are separated. The S-configurated compound has a different activity than the R-configurated compound.

### Example 102

### (6-Chloro-pyrazolo[1,5-a]pyridin-2-yl)-(8-methyl-5,8-dihydro-6H-[1,7]naphthyridin-7-yl)-methanone

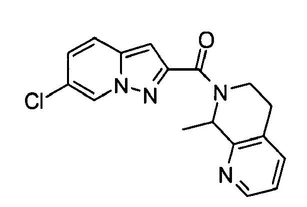

In close analogy to the procedure described in Example 1, 6-chloro-pyrazolo[1,5-a]pyridine-2-carboxylic acid is reacted with 8-methyl-5,6,7,8-tetrahydro-[1,7]naphthyridine to provide the title compound in moderate yield.
LC/MS: m/z = 327 (MH⁺)
The stereo-isomers of this compound are separated. The S-configurated compound has a different activity than the R-configurated compound.

### Example 103

### (6-Bromo-pyrazolo[1,5-a]pyridin-2-yl)-(1-methyl-3,4-dihydro-1H-[2,7]naphthyridin-2-yl)-methanone

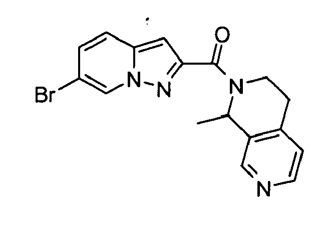

In close analogy to the procedure described in Example 1, 6-bromo-pyrazolo[1,5-a]pyridine-2-carboxylic acid is reacted with 1-methyl-1,2,3,4-tetrahydro-[2,7]naphthyridine to provide the title compound in moderate yield.
LC/MS: m/z = 372 (MH⁺)
The stereo-isomers of this compound are separated. The S-configurated compound has a different activity than the R-configurated compound.

### Example 104

### (6-Chloro-pyrazolo[1,5-a]pyridin-2-yl)-(1-methyl-3,4-dihydro-1H-[2,7]naphthyridin-2-yl)-methanone

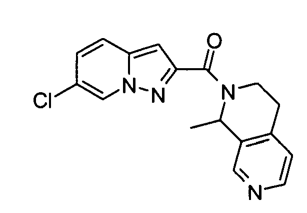

In close analogy to the procedure described in Example 1, 6-chloro-pyrazolo[1,5-a]pyridine-2-carboxylic acid is reacted with 1-methyl-1,2,3,4-tetrahydro-[2,7]naphthyridine to provide the title compound in moderate yield.
LC/MS: m/z = 327 (MH⁺)
The stereo-isomers of this compound are separated. The S-configurated compound has a different activity than the R-configurated compound.

### Example 105

### (6-Bromo-pyrazolo[1,5-a]pyridin-2-yl)-(1-methyl-3,4-dihydro-1H-[2,6]naphthyridin-2-yl)-methanone

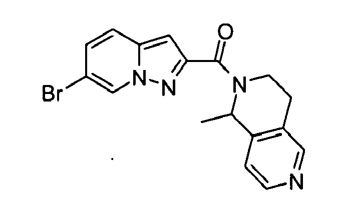

In close analogy to the procedure described in Example 1, 6-bromo-pyrazolo[1,5-a]pyridine-2-carboxylic acid is reacted with 1-methyl-1,2,3,4-tetrahydro-[2,6]naphthyridine to provide the title compound.
LC/MS: m/z = 373 (MH⁺)
The stereo-isomers of this compound are separated. The S-configurated compound has a different activity than the R-configurated compound.

### Example 106

### (6-Chloro-pyrazolo[1,5-a]pyridin-2-yl)-(1-methyl-3,4-dihydro-1H-[2,6]naphthyridin-2-yl)-methanone

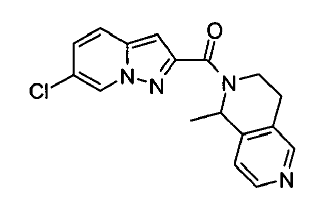

In close analogy to the procedure described in Example 1, 6-chloro-pyrazolo[1,5-a]pyridine-2-carboxylic acid is reacted with 1-methyl-1,2,3,4-tetrahydro-[2,6]naphthyridine to provide the title compound in moderate yield.
LC/MS: m/z = 327 (MH⁺)
The stereo-isomers of this compound are separated. The S-configurated compound has a different activity than the R-configurated compound.

### Example 107

### (6-Bromo-pyrazolo[1,5-a]pyridin-2-yl)-(5-methyl-7,8-dihydro-5H-[1,6]naphthyridin-6-yl)-methanone

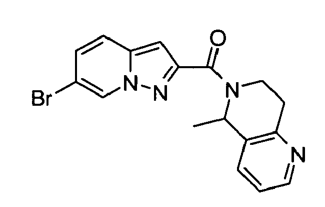

In close analogy to the procedure described in Example 1, 6-bromo-pyrazolo[1,5-a]pyridine-2-carboxylic acid is reacted with 5-methyl-5,6,7,8-tetrahydro-[1,6]naphthyridine to provide the title compound in moderate yield.
LC/MS: m/z = 372 (MH⁺)
The stereo-isomers of this compound are separated. The S-configurated compound has a different activity than the R-configurated compound.

### Example 108

### (6-Chloro-pyrazolo[1,5-a]pyridin-2-yl)-(5-methyl-7,8-dihydro-5H-[1,6]naphthyridin-6-yl)-methanone

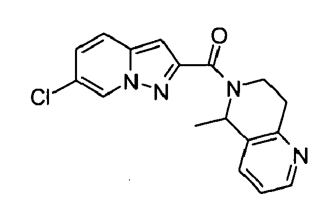

In close analogy to the procedure described in Example 1, 6-chloro-pyrazolo[1,5-a]pyridine-2-carboxylic acid is reacted with 5-methyl-5,6,7,8-tetrahydro-[1,6]naphthyridine to provide the title compound in moderate yield.
LC/MS: m/z = 327 (MH⁺)
The stereo-isomers of this compound are separated. The S-configurated compound has a different activity than the R-configurated compound.

### Example 109

### (6-Bromo-pyrazolo[1,5-a]pyridin-2-yl)-(1-methyl-3,4-dihydro-1H-isoquinolin-2-yl)-methanone

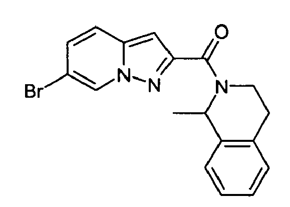

In close analogy to the procedure described in Example 1, 6-bromo-pyrazolo[1,5-a]pyridine-2-carboxylic acid is reacted with 1-methyl-1,2,3,4-tetrahydro-isoquinoline to provide the title compound in moderate yield.
LC/MS: m/z = 371 (MH⁺)
The stereo-isomers of this compound are separated. The S-configurated compound has a different activity than the R-configurated compound.

### Example 110

### (6-Bromo-pyrazolo[1,5-a]pyridin-2-yl)-(3,4-dihydro-1H-isoquinolin-2-yl)-methanone

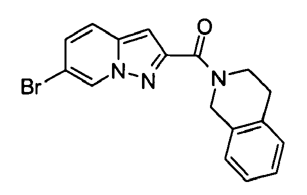

In close analogy to the procedure described in Example 1, 6-bromo-pyrazolo[1,5-a]pyridine-2-carboxylic acid is reacted with 1,2,3,4-tetrahydroisoquinoline to provide the title compound in moderate yield.
LC/MS: m/z = 357 (MH⁺)

### Example 111

### (6-Bromo-pyrazolo[1,5-a]pyridin-2-yl)-(7-fluoro-1-methyl-3,4-dihydro-1H-isoquinolin-2-yl)-methanone

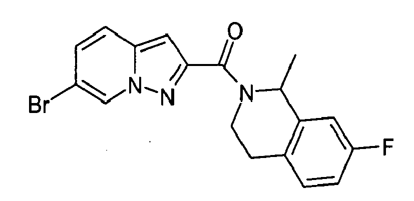

In close analogy to the procedure described in Example 1, 6-bromo-pyrazolo[1,5-a]pyridine-2-carboxylic acid is reacted with 7-fluoro-1-methyl-1,2,3,4-tetrahydro-isoquinoline to provide the title compound in moderate yield.
LC/MS: m/z = 389 (MH⁺)
The stereo-isomers of this compound are separated. The S-configurated compound has a different activity than the R-configurated compound.

### Example 112

### (6-Bromo-pyrazolo[1,5-a]pyridin-2-yl)-(8-fluoro-1-methyl-3,4-dihydro-1H-isoquinolin-2-yl)-methanone

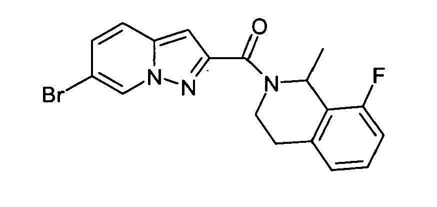

In close analogy to the procedure described in Example 1, 6-bromo-pyrazolo[1,5-a]pyridine-2-carboxylic acid is reacted with 8-fluoro-1-methyl-1,2,3,4-tetrahydro-isoquinoline to provide the title compound in moderate yield.
LC/MS: m/z = 389 (MH⁺)
The stereo-isomers of this compound are separated. The S-configurated compound has a different activity than the R-configurated compound.

### Example 113

### (6-Bromo-pyrazolo[1,5-a]pyridin-2-yl)-(6-fluoro-1-methyl-3,4-dihydro-1H-isoquinolin-2-yl)-methanone

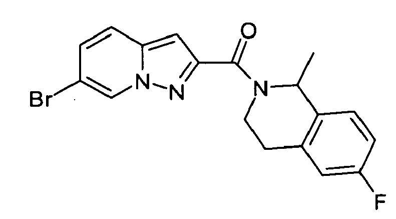

In close analogy to the procedure described in Example 1, 6-bromo-pyrazolo[1,5-a]pyridine-2-carboxylic acid is reacted with 6-fluoro-1-methyl-1,2,3,4-tetrahydro-isoquinoline to provide the title compound in moderate yield.
LC/MS: m/z = 389 (MH⁺)
The stereo-isomers of this compound are separated. The S-configurated compound has a different activity than the R-configurated compound.

### Example 114

### (6-Bromo-pyrazolo[1,5-a]pyridin-2-yl)-(5-fluoro-1-methyl-3,4-dihydro-1H-isoquinolin-2-yl)-methanone

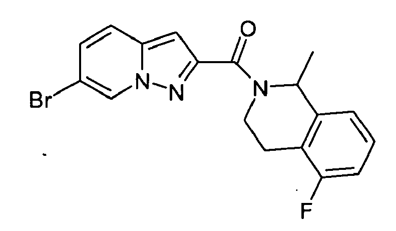

In close analogy to the procedure described in Example 1, 6-bromo-pyrazolo[1,5-a]pyridine-2-carboxylic acid is reacted with 5-fluoro-1-methyl-1,2,3,4-tetrahydro-isoquinoline to provide the title compound in moderate yield.
LC/MS: m/z = 389 (MH⁺)
The stereo-isomers of this compound are separated. The S-configurated compound has a different activity than the R-configurated compound.

### Example 115

### (6-Chloro-pyrazolo[1,5-a]pyridin-2-yl)-(7-fluoro-1-methyl-3,4-dihydro-1H-isoquinolin-2-yl)-methanone

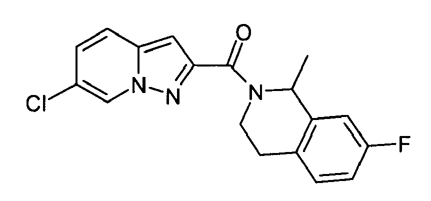

In close analogy to the procedure described in Example 1, 6-chloro-pyrazolo[1,5-a]pyridine-2-carboxylic acid is reacted with 7-fluoro-1-methyl-1,2,3,4-tetrahydro-isoquinoline to provide the title compound in moderate yield.
LC/MS: m/z = 344 (MH⁺)
The stereo-isomers of this compound are separated. The S-configurated compound has a different activity than the R-configurated compound.

### Example 116

### (6-Chloro-pyrazolo[1,5-a]pyridin-2-yl)-(8-fluoro-1-methyl-3,4-dihydro-1H-isoquinolin-2-yl)-methanone

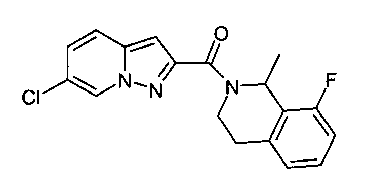

In close analogy to the procedure described in Example 1, 6-chloro-pyrazolo[1,5-a]pyridine-2-carboxylic acid is reacted with 8-fluoro-1-methyl-1,2,3,4-tetrahydro-isoquinoline to provide the title compound in moderate yield.
LC/MS: m/z = 344 (MH⁺)
The stereo-isomers of this compound are separated. The S-configurated compound has a different activity than the R-configurated compound.

### Example 117

### (6-Chloro-pyrazolo[1,5-a]pyridin-2-yl)-(6-fluoro-1-methyl-3,4-dihydro-1H-isoquinolin-2-yl)-methanone

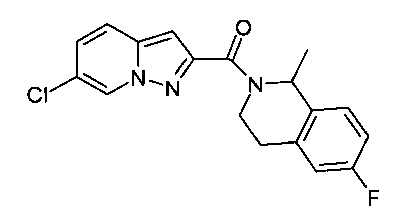

In close analogy to the procedure described in Example 1, 6-chloro-pyrazolo[1,5-a]pyridine-2-carboxylic acid is reacted with 6-fluoro-1-methyl-1,2,3,4-tetrahydro-isoquinoline to provide the title compound in moderate yield.
LC/MS: m/z = 344 (MH⁺)
The stereo-isomers of this compound are separated. The S-configurated compound has a different activity than the R-configurated compound.

### Example 118

### (6-Chloro-pyrazolo[1,5-a]pyridin-2-yl)-(5-fluoro-1-methyl-3,4-dihydro-1H-isoquinolin-2-yl)-methanone

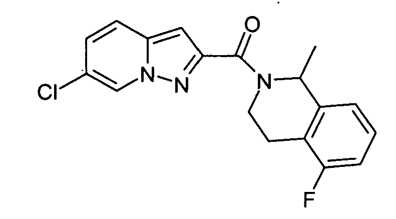

In close analogy to the procedure described in Example 1, 6-chloro-pyrazolo[1,5-a]pyridine-2-carboxylic acid is reacted with 5-fluoro-1-methyl-1,2,3,4-tetrahydro-isoquinoline to provide the title compound in moderate yield.
LC/MS: m/z = 344 (MH⁺)
The stereo-isomers of this compound are separated. The S-configurated compound has a different activity than the R-configurated compound.

### Example 119

### (6-Bromo-pyrazolo[1,5-a]pyridin-2-yl)-(8-fluoro-1-ethyl-3,4-dihydro-1H-isoquinolin-2-yl)-methanone

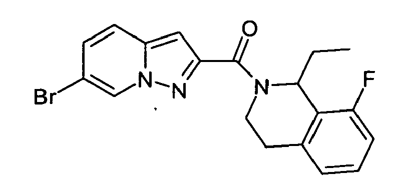

In close analogy to the procedure described in Example 1, 6-bromo-pyrazolo[1,5-a]pyridine-2-carboxylic acid is reacted with 8-fluoro-1-ethyl-1,2,3,4-tetrahydro-isoquinoline to provide the title compound in moderate yield.
LC/MS: m/z = 403 (MH⁺)
The stereoisomers of this compound are separated. The S-configurated compound has a different activity than the R-configurated compound.

### Example 120

### (6-Bromo-pyrazolo[1,5-a]pyridin-2-yl)-(7-fluoro-1-ethyl-3,4-dihydro-1H-isoquinolin-2-yl)-methanone

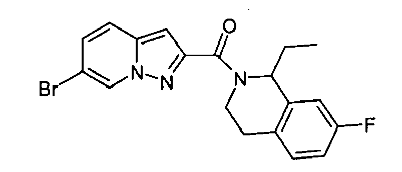

In close analogy to the procedure described in Example 1, 6-bromo-pyrazolo[1,5-a]pyridine-2-carboxylic acid is reacted with 7-fluoro-1-ethyl-1,2,3,4-tetrahydro-isoquinoline to provide the title compound in moderate yield.
LC/MS: m/z = 403 (MH⁺)
The stereoisomers of this compound are separated. The S-configurated compound has a different activity than the R-configurated compound.

### Example 121

### (6-Bromo-pyrazolo[1,5-a]pyridin-2-yl)-(6-fluoro-1-ethyl-3,4-dihydro-1H-isoquinolin-2-yl)-methanone

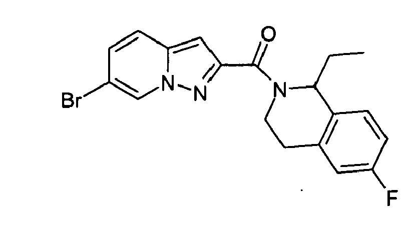

In close analogy to the procedure described in Example 1, 6-bromo-pyrazolo[1,5-a]pyridine-2-carboxylic acid is reacted with 6-fluoro-1-ethyl-1,2,3,4-tetrahydro-isoquinoline to provide the title compound in moderate yield.
LC/MS: m/z = 403 (MH⁺)
The stereoisomers of this compound are separated. The S-configurated compound has a different activity than the R-configurated compound.

### Example 122

### (6-Bromo-pyrazolo[1,5-a]pyridin-2-yl)-(5-fluoro-1-ethyl-3,4-dihydro-1H-isoquinolin-2-yl)-methanone

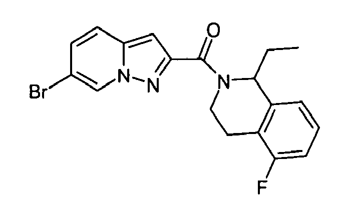

In close analogy to the procedure described in Example 1, 6-bromo-pyrazolo[1,5-a]pyridine-2-carboxylic acid is reacted with 5-fluoro-1-ethyl-1,2,3,4-tetrahydro-isoquinoline to provide the title compound in moderate yield.
LC/MS: m/z = 403 (MH⁺)
The stereoisomers of this compound are separated. The S-configurated compound has a different activity than the R-configurated compound.

### Example 123

### (6-Chloro-pyrazolo[1,5-a]pyridin-2-yl)-(8-fluoro-1-ethyl-3,4-dihydro-1H-isoquinolin-2-yl)-methanone

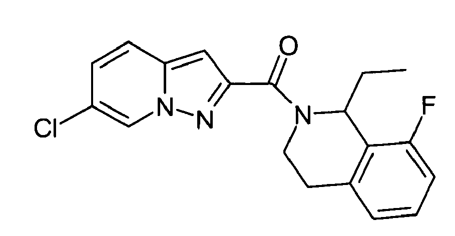

In close analogy to the procedure described in Example 1, 6-chloro-pyrazolo[1,5-a]pyridine-2-carboxylic acid is reacted with 8-fluoro-1-ethyl-1,2,3,4-tetrahydro-isoquinoline to provide the title compound in moderate yield.
LC/MS: m/z = 358 (MH⁺)
The stereoisomers of this compound are separated. The S-configurated compound has a different activity than the R-configurated compound.

### Example 124

### (6-Chloro-pyrazolo[1,5-a]pyridin-2-yl)-(7-fluoro-1-ethyl-3,4-dihydro-1H-isoquinolin-2-yl)-methanone

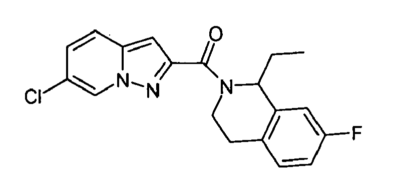

In close analogy to the procedure described in Example 1, 6-chloro-pyrazolo[1,5-a]pyridine-2-carboxylic acid is reacted with 7-fluoro-1-ethyl-1,2,3,4-tetrahydro-isoquinoline to provide the title compound in moderate yield.
LC/MS: m/z = 358 (MH⁺)
The stereoisomers of this compound are separated. The S-configurated compound has a different activity than the R-configurated compound.

### Example 125

### (6-Chloro-pyrazolo[1,5-a]pyridin-2-yl)-(6-fluoro-1-ethyl-3,4-dihydro-1H-isoquinolin-2-yl)-methanone

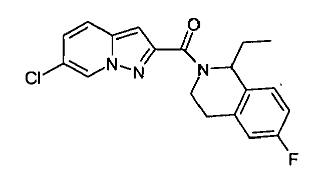

In close analogy to the procedure described in Example 1, 6-chloro-pyrazolo[1,5-a]pyridine-2-carboxylic acid is reacted with 6-fluoro-1-ethyl-1,2,3,4-tetrahydro-isoquinoline to provide the title compound in moderate yield.
LC/MS: m/z = 358 (MH⁺)
The stereoisomers of this compound are separated. The S-configurated compound has a different activity than the R-configurated compound.

### Example 126

### (6-Chloro-pyrazolo[1,5-a]pyridin-2-yl)-(5-fluoro-1-ethyl-3,4-dihydro-1H-isoquinolin-2-yl)-methanone

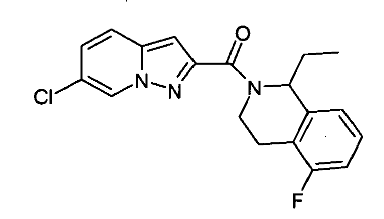

In close analogy to the procedure described in Example 1, 6-chloro-pyrazolo[1,5-a]pyridine-2-carboxylic acid is reacted with 5-fluoro-1-ethyl-1,2,3,4-tetrahydro-isoquinoline to provide the title compound in moderate yield.
LC/MS: m/z = 358 (MH⁺)
The stereoisomers of this compound are separated. The S-configurated compound has a different activity than the R-configurated compound.

### Example 127

### (6-Bromo-pyrazolo[1,5-a]pyridin-2-yl)-(8-fluoro-1-trifluoromethyl-3,4-dihydro-1H-isoquinolin-2-yl)-methanone

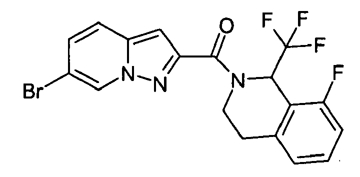

In close analogy to the procedure described in Example 1, 6-bromo-pyrazolo[1,5-a]pyridine-2-carboxylic acid is reacted with 8-fluoro-1-trifluoromethyl-1,2,3,4-tetrahydro-isoquinoline to provide the title compound in moderate yield.
LC/MS: m/z = 443 (MH⁺)
The stereoisomers of this compound are separated. The S-configurated compound has a different activity than the R-configurated compound.

### Example 128

### (6-Bromo-pyrazolo[1,5-a]pyridin-2-yl)-(7-fluoro-1-trifluoromethyl-3,4-dihydro-1H-isoquinolin-2-yl)-methanone

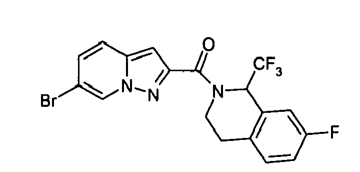

In close analogy to the procedure described in Example 1, 6-bromo-pyrazolo[1,5-a]pyridine-2-carboxylic acid is reacted with 7-fluoro-1-trifluoromethyl-1,2,3,4-tetrahydro-isoquinoline to provide the title compound in moderate yield.
LC/MS: m/z = 443 (MH⁺)
The stereoisomers of this compound are separated. The S-configurated compound has a different activity than the R-configurated compound.

### Example 129

### (6-Bromo-pyrazolo[1,5-a]pyridin-2-yl)-(6-fluoro-1-trifluoromethyl-3,4-dihydro-1H-isoquinolin-2-yl)-methanone

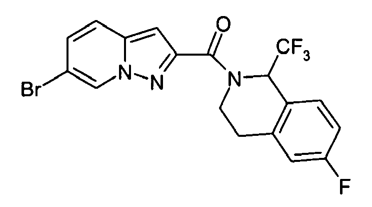

In close analogy to the procedure described in Example 1, 6-bromo-pyrazolo[1,5-a]pyridine-2-carboxylic acid is reacted with 6-fluoro-1-trifluoromethyl-1,2,3,4-tetrahydro-isoquinoline to provide the title compound in moderate yield.
LC/MS: m/z = 443 (MH⁺)
The stereoisomers of this compound are separated. The S-configurated compound has a different activity than the R-configurated compound.

### Example 130

### (6-Bromo-pyrazolo[1,5-a]pyridin-2-yl)-(5-fluoro-1-trifluoromethyl-3,4-dihydro-1H-isoquinolin-2-yl)-methanone

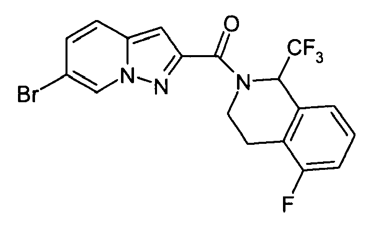

In close analogy to the procedure described in Example 1, 6-bromo-pyrazolo[1,5-a]pyridine-2-carboxylic acid is reacted with 5-fluoro-1-trifluoromethyl-1,2,3,4-tetrahydro-isoquinoline to provide the title compound in moderate yield.
LC/MS: m/z = 443 (MH⁺)
The stereoisomers of this compound are separated. The S-configurated compound has a different activity than the R-configurated compound.

### Example 131

### (6-Chloro-pyrazolo[1,5-a]pyridin-2-yl)-(8-fluoro-1-trifluoromethyl-3,4-dihydro-1H-isoquinolin-2-yl)-methanone

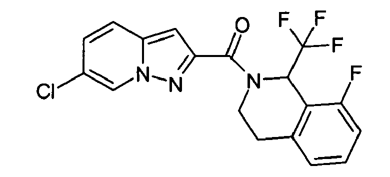

In close analogy to the procedure described in Example 1, 6-chloro-pyrazolo[1,5-a]pyridine-2-carboxylic acid is reacted with 8-fluoro-1-trifluoromethyl-1,2,3,4-tetrahydro-isoquinoline to provide the title compound in moderate yield.
LC/MS: m/z = 398 (MH⁺)
The stereoisomers of this compound are separated. The S-configurated compound has a different activity than the R-configurated compound.

### Example 132

### (6-Chloro-pyrazolo[1,5-a]pyridin-2-yl)-(7-fluoro-1-trifluoromethyl-3,4-dihydro-1H-isoquinolin-2-yl)-methanone

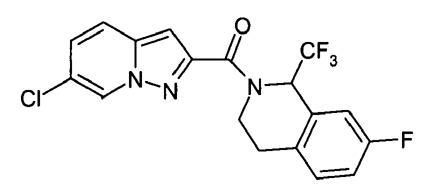

In close analogy to the procedure described in Example 1, 6-chloro-pyrazolo[1,5-a]pyridine-2-carboxylic acid is reacted with 7-fluoro-1-trifluoromethyl-1,2,3,4-tetrahydro-isoquinoline to provide the title compound in moderate yield.
LC/MS: m/z = 398 (MH⁺)
The stereoisomers of this compound are separated. The S-configurated compound has a different activity than the R-configurated compound.

### Example 133

### (6-Chloro-pyrazolo[1,5-a]pyridin-2-yl)-(6-fluoro-1-trifluoromethyl-3,4-dihydro-1H-isoquinolin-2-yl)-methanone

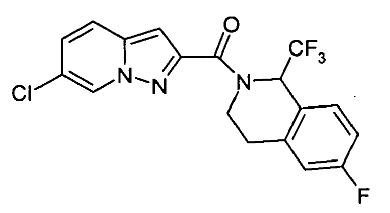

In close analogy to the procedure described in Example 1, 6-chloro-pyrazolo[1,5-a]pyridine-2-carboxylic acid is reacted with 6-fluoro-1-trifluoromethyl-1,2,3,4-tetrahydro-isoquinoline to provide the title compound in moderate yield.
LC/MS: m/z = 398 (MH⁺)
The stereoisomers of this compound are separated. The S-configurated compound has a different activity than the R-configurated compound.

### Example 134

### (6-Chloro-pyrazolo[1,5-a]pyridin-2-yl)-(5-fluoro-1-trifluoromethyl-3,4-dihydro-1H-isoquinolin-2-yl)-methanone

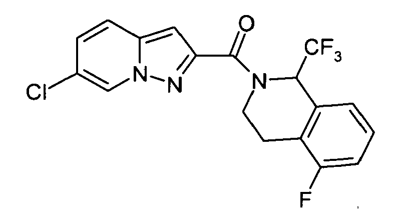

In close analogy to the procedure described in Example 1, 6-chloro-pyrazolo[1,5-a]pyridine-2-carboxylic acid is reacted with 5-fluoro-1-trifluoromethyl-1,2,3,4-tetrahydro-isoquinoline to provide the title compound in moderate yield.
LC/MS: m/z = 398 (MH⁺)
The stereoisomers of this compound are separated. The S-configurated compound has a different activity than the R-configurated compound.

### Example 135

### (6-Bromo-pyrazolo[1,5-a]pyridin-2yl)-(3-ethyl-1-methyl-3,4-dihydro-1H-isoquinolin-2-yl)methanone

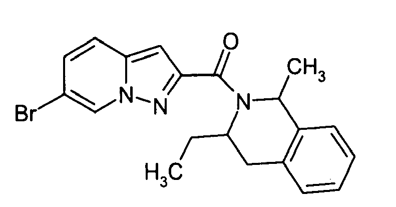

In close analogy to the procedure described in Example 1, 6-bromo-pyrazolo[1,5-a]pyridine-2-carboxylic acid is reacted with 3-ethyl-1-methyl-1,2,3,4-tetrahydro-isoquinoline to provide the title compound in moderate yield.
LC/MS: m/z = 399 (MH⁺)
The four diastereomers can be separated. They have different activities.

### Example 136

### (6-Bromo-pyrazolo[1,5-a]pyridin-2yl)-((R)-3-methyl-3,4-dihydro-1H-isoquinolin-2-yl)-methanone

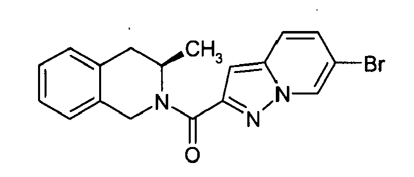

In close analogy to the procedure described in Example 1, 6-bromo-pyrazolo[1,5-a]pyridine-2-carboxylic acid is reacted with (R)-3-methyl-1,2,3,4-tetrahydro-isoquinoline to provide the title compound in moderate yield.
LC/MS: m/z = 371 (MH⁺)

### Example 137

### (6-Bromo-pyrazolo[1,5-a]pyridin-2yl)-((S)-3-methyl-3,4-dihydro-1H-isoquinolin-2-yl)-methanone

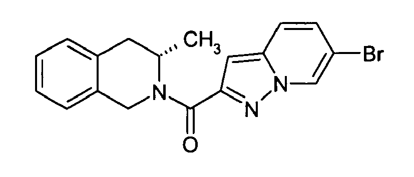

In close analogy to the procedure described in Example 1, 6-bromo-pyrazolo[1,5-a]pyridine-2-carboxylic acid is reacted with (S)-3-methyl-1,2,3,4-tetrahydro-isoquinoline to provide the title compound in moderate yield.
LC/MS: m/z = 371 (MH⁺)

### Example 138

### (6-Chloro-pyrazolo[1,5-a]pyridin-2-yl)-(1-(S)-methyl-3,4-dihydro-1H-isoquinolin-2-yl)-methanone

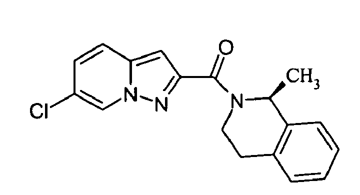

In close analogy to the procedure described in Example 1, 6-chloro-pyrazolo[1,5-a]pyridine-2-carboxylic acid is reacted with (S)-1-methyl-1,2,3,4-tetrahydro-isoquinoline to provide the title-configurated compound in moderate yield.
LC/MS: m/z = 326 (MH⁺).

### Example 139

### (7-Bromo-3,4-dihydro-1H-isoquinolin-2-yl)-(6-chloro-pyrazolo[1,5-a]pyridin-2-yl)-methanone

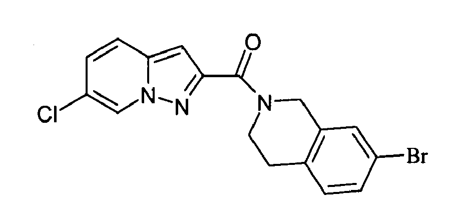

In close analogy to the procedure described in Example 1, 6-chloro-pyrazolo[1,5-a]pyridine-2-carboxylic acid is reacted with 7-bromo-1,2,3,4-tetrahydro-isoquinoline to provide the title compound in moderate yield.
LC/MS: m/z = 391 (MH⁺)

### Example 140

### (7-Bromo-3,4-dihydro-1H-isoquinolin-2-yl)-(6-bromo-pyrazolo[1,5-a]pyridin-2-yl)-methanone

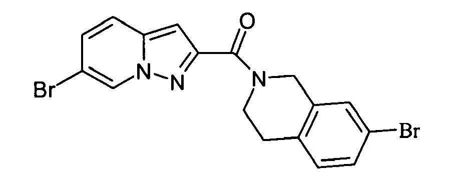

In close analogy to the procedure described in Example 1, 6-bromo-pyrazolo[1,5-a]pyridine-2-carboxylic acid is reacted with 7-bromo-1,2,3,4-tetrahydro-isoquinoline to provide the title compound in moderate yield.
LC/MS: m/z = 436 (MH⁺)

### Example 141

### (6-Chloro-pyrazolo[1,5-a]pyridin-2-yl)-(7-fluoro-1-methyl-3,4-dihydro-1H-isoquinolin-2-yl)-methanone

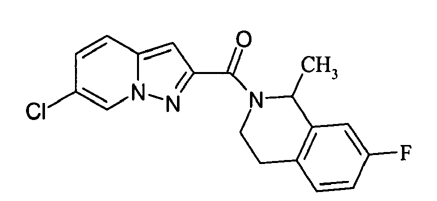

In close analogy to the procedure described in Example 1, 6-chloro-pyrazolo[1,5-a]pyridine-2-carboxylic acid is reacted with 7-fluoro-1-methyl-1,2,3,4-tetrahydro-isoquinoline to provide the title compound in moderate yield.
LC/MS: m/z = 444 (MH⁺)
The stereo-isomers of this compound are separated. The S-configurated compound has a different activity than the R-configurated compound.

### Example 142

### (6-Chloro-pyrazolo[1,5-a]pyridin-2-yl)-(1,4-dimethyl-3,4-dihydro-1H-isoquinolin-2-yl)-methanone

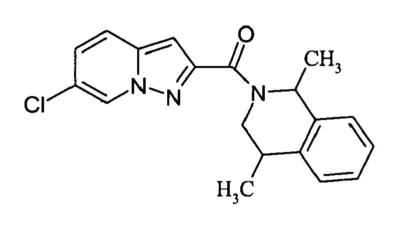

In close analogy to the procedure described in Example 1, 6-chloro-pyrazolo[1,5-a]pyridine-2-carboxylic acid is reacted with 1,4-dimethyl-1,2,3,4-tetrahydro-isoquinoline to provide the title compound in moderate yield.
LC/MS: m/z = 340 (MH⁺)
The diasteromers of this compound are separated. The different configurated compounds (R,R), (R,S), (S,R) and (S,S) have different activities.

### Example 143

### (6-Bromo-pyrazolo[1,5-a]pyridin-2-yl)-(1,4-dimethyl-3,4-dihydro-1H-isoquinolin-2-yl)-methanone

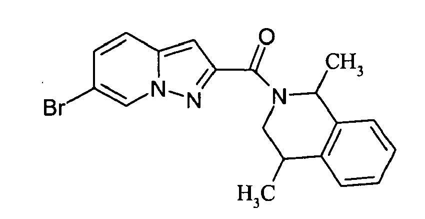

In close analogy to the procedure described in Example 1, 6-bromo-pyrazolo[1,5-a]pyridine-2-carboxylic acid is reacted with 1,4-dimethyl-1,2,3,4-tetrahydro-isoquinoline to provide the title compound in moderate yield.
LC/MS: m/z = 385 (MH⁺)
The diasteromers of this compound are separated. The different configurated compounds have different activities.

### Example 144

### (6-Bromo-pyrazolo[1,5-a]pyridin-2-yl)-((1R,4S)-1,4-dimethyl-3,4-dihydro-1H-isoquinolin-2-yl)-methanone

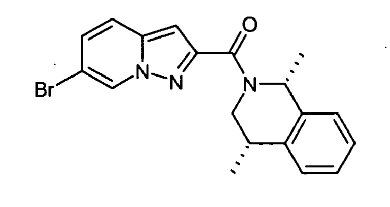

### Example 145

(6-Bromo-pyrazolo[1,5-a]pyridin-2-yl)-((1S,4S)-1,4-dimethyl-3,4-dihydro-1H-isoquinolin-2-yl)-methanone

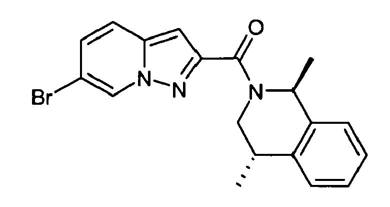

### Example 146

### (6-Bromo-pyrazolo[1,5-a]pyridin-2-yl)-((1S,4R)-1,4-dimethyl-3,4-dihydro-1H-isoquinolin-2-yl)-methanone

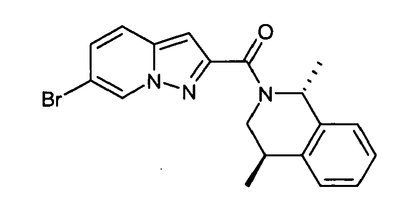

### Example 147

### (6-Bromo-pyrazolo[1,5-a]pyridin-2-yl)-((1R,4R)-1,4-dimethyl-3,4-dihydro-1H-isoquinolin-2-yl)-methanone

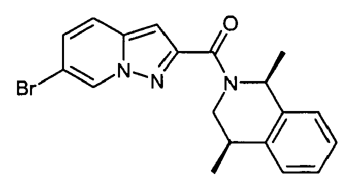

### Example 148

### (6-Bromo-pyrazolo[1,5-a]pyridin-2-yl)-(6-methoxy-1-methyl-3,4-dihydro-1H-isoquinolin-2-yl)-methanone

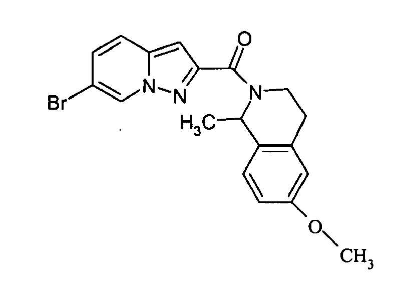

In close analogy to the procedure described in Example 1, 6-bromo-pyrazolo[1,5-a]pyridine-2-carboxylic acid is reacted with 6-methoxy-1-methyl-1,2,3,4-tetrahydro-isoquinoline to provide the title compound in moderate yield.
LC/MS: m/z = 401 (MH⁺)
The stereo-isomers of this compound are separated. The S-configurated compound has a different activity than the R-configurated compound.

### Example 149

### (6-Chloro-pyrazolo[1,5-a]pyridin-2-yl)-(6-methoxy-1-methyl-3,4-dihydro-1H-isoquinolin-2-yl)-methanone

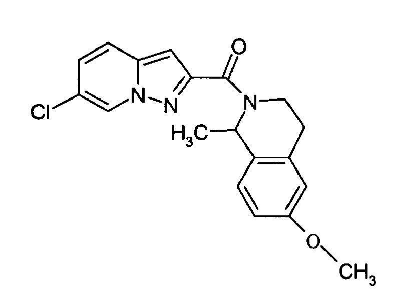

In close analogy to the procedure described in Example 1, 6-chloro-pyrazolo[1,5-a]pyridine-2-carboxylic acid is reacted with 6-methoxy-1-methyl-1,2,3,4-tetrahydro-isoquinoline to provide the title compound in moderate yield.
LC/MS: m/z = 356 (MH⁺)
The stereo-isomers of this compound are separated. The S-configurated compound has a different activity than the R-configurated compound.

### Example 150

### (6-Bromopyrazolo[1,5-a]pyridin-2yl)-(1-ethyl-3,4-dihydro-1H-isoquinolin-2-yl)methanone

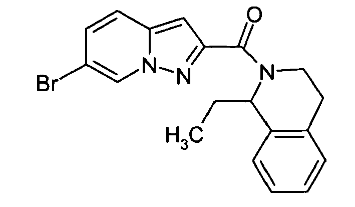

In close analogy to the procedure described in Example 1, 6-bromo-pyrazolo[1,5-a]pyridine-2-carboxylic acid is reacted with 1-ethyl-1,2,3,4-tetrahydro-isoquinoline to provide the title compound in moderate yield.
LC/MS: m/z = 384 (MH⁺)
The stereo-isomers of this compound are separated. The S-configurated compound has a different activity than the R-configurated compound.

### Example 151

### (6-Bromopyrazolo[1,5-a]pyridin-2yl)-(1-isopropyl-3,4-dihydro-1H-isoquinolin-2-yl)methanone

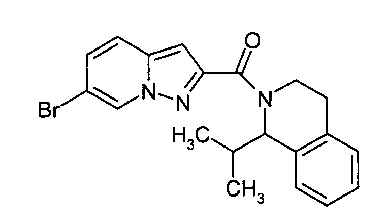

In close analogy to the procedure described in Example 1, 6-bromo-pyrazolo[1,5-a]pyridine-2-carboxylic acid is reacted with 1-isopropyl-1,2,3,4-tetrahydro-isoquinoline to provide the title compound in moderate yield.
LC/MS: m/z = 398 (MH⁺)
The stereo-isomers of this compound are separated. The S-configurated compound has a different activity than the R-configurated compound.

### Example 152

### (6-Chloro-pyrazolo[1,5-a]pyridin-2-yl)-(1-isopropyl-3,4-dihydro-1H-isoquinolin-2-yl)-methanone

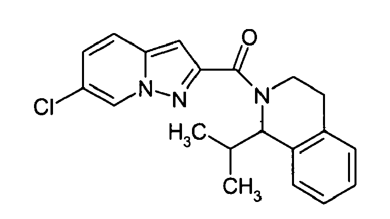

In close analogy to the procedure described in Example 1, 6-chloro-pyrazolo[1,5-a]pyridine-2-carboxylic acid is reacted with 1-isopropyl-1,2,3,4-tetrahydro-isoquinoline to provide the title compound in moderate yield.
The stereo-isomers of this compound are separated. The S-configurated compound has a different activity than the R-configurated compound.

### Example 153

### (6-Chloro-pyrazolo[1,5-a]pyridin-2-yl)-(1-ethyl-3,4-dihydro-1H-isoquinolin-2-yl)-methanone

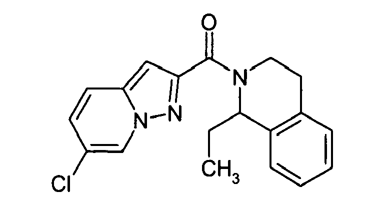

In close analogy to the procedure described in Example 1, 6-chloro-pyrazolo[1,5-a]pyridine-2-carboxylic acid is reacted with 1-ethyl-1,2,3,4-tetrahydro-isoquinoline to provide the title compound in moderate yield.
LC/MS: m/z = 340 (MH⁺)
The stereo-isomers of this compound are separated. The S-configurated compound has a different activity than the R-configurated compound.

### Example 154

### (6-Bromo-pyrazolo[1,5-a]pyridin-2-yl)-(1-trifluoromethyl-3,4-dihydro-1H-isoquinolin-2-yl)-methanone

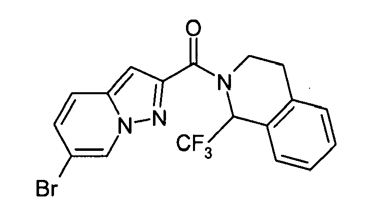

In close analogy to the procedure described in Example 1, 6-bromo-pyrazolo[1,5-a]pyridine-2-carboxylic acid is reacted with 1-trifluoromethyl-1,2,3,4-tetrahydro-isoquinoline to provide the title compound in moderate yield.
LC/MS: m/z = 424 (MH⁺)
The stereo-isomers of this compound are separated. The S-configurated compound has a different activity than the R-configurated compound.

### Example 155

### (6-Choro-pyrazolo[1,5-a]pyridin-2-yl)-(1-trifluoromethyl-3,4-dihydro-1H-isoquinolin-2-yl)-methanone

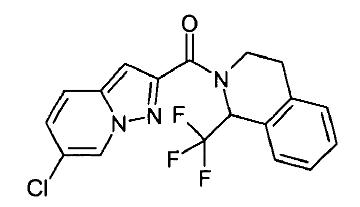

In close analogy to the procedure described in Example 1, 6-bromo-pyrazolo[1,5-a]pyridine-2-carboxylic acid is reacted with 1-trifluoromethyl-1,2,3,4-tetrahydro-isoquinoline to provide the title compound in moderate yield.
LC/MS: m/z = 380 (MH⁺)
The stereo-isomers of this compound are separated. The S-configurated compound has a different activity than the R-configurated compound.

### Example 156

### (7-Bromo-1-methyl-3,4-dihydro-1H-isoquinolin-2-yl)-(6-bromo-pyrazolo[1,5-a]pyridin-2-yl)-methanone

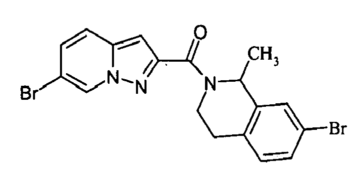

In close analogy to the procedure described in Example 1, 6-bromo-pyrazolo[1,5-a]pyridine-2-carboxylic acid is reacted with 7-bromo-1-methyl-1,2,3,4-tetrahydro-isoquinoline to provide the title compound in moderate yield.
LC/MS: m/z = 448 (MH⁺)
The stereo-isomers of this compound are separated. The S-configurated compound has a different activity than the R-configurated compound.

### Example 157

### (7-Bromo-1-methyl-3,4-dihydro-1H-isoquinolin-2-yl)-(6-chloro-pyrazolo[1,5-a]pyridin-2-yl)-methanone

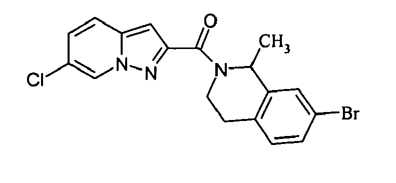

In close analogy to the procedure described in Example 1, 6-chloro-pyrazolo[1,5-a]pyridine-2-carboxylic acid is reacted with 7-bromo-1-methyl-1,2,3,4-tetrahydro-isoquinoline to provide the title compound in moderate yield.
LC/MS: m/z = 405 (MH+)
The stereo-isomers of this compound are separated. The S-configurated compound has a different activity than the R-configurated compound.

### Example 158

### (6-Bromo-pyrazolo[1,5-a]pyridin-2-yl)-(1-cyclohexyl-3,4-dihydro-1H-isoquinolin-2-yl)-methanone

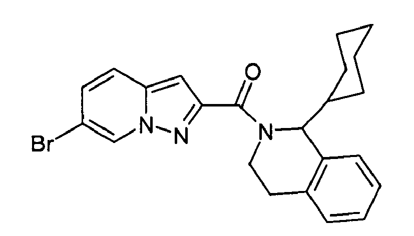

In close analogy to the procedure described in Example 1, 6-bromo-pyrazolo[1,5-a]pyridine-2-carboxylic acid is reacted with 1-cyclohexyl-1,2,3,4-tetrahydro-isoquinoline to provide the title compound in moderate yield.
LC/MS: m/z = 438 (MH⁺)
The stereo-isomers of this compound are separated. The S-configurated compound has a different activity than the R-configurated compound.

### Example 159

### (6-Chloro-pyrazolo[1,5-a]pyridin-2-yl)-(1-cyclohexyl-3,4-dihydro-1H-isoquinolin-2-yl)-methanone

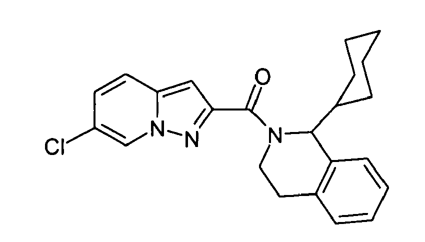

In close analogy to the procedure described in Example 1, 6-chloro-pyrazolo[1,5-a]pyridine-2-carboxylic acid is reacted with 1-cyclohexyl-1,2,3,4-tetrahydro-isoquinoline to provide the title compound in moderate yield.
LC/MS: m/z = 394 (MH⁺)
The stereo-isomers of this compound are separated. The S-configurated compound has a different activity than the R-configurated compound.

### Example 160

### (6-Bromo-pyrazolo[1,5-a]pyridin-2-yl)-(1-propyl-3,4-dihydro-1H-isoquinolin-2yl)-methanone

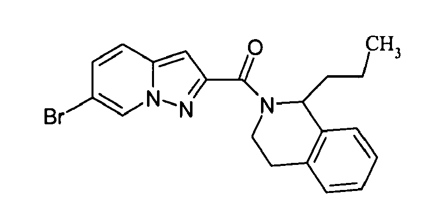

In close analogy to the procedure described in Example 1, 6-bromo-pyrazolo[1,5-a]pyridine-2-carboxylic acid is reacted with 1-propyl-1,2,3,4-tetrahydro-isoquinoline to provide the title compound in moderate yield.
LC/MS: m/z = 399 (MH⁺)
The stereo-isomers of this compound are separated. The S-configurated compound has a different activity than the R-configurated compound.

### Example 170

### (6-Bromo-pyrazolo[1,5-a]pyridin-2-yl)-((S)1-methyl-3,4-dihydro-1H-isoquinolin-2yl)-methanone

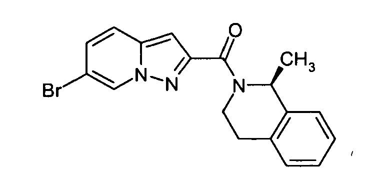

In close analogy to the procedure described in Example 1, 6-bromo-pyrazolo[1,5-a]pyridine-2-carboxylic acid is reacted with 1-(S)-methyl-1,2,3,4-tetrahydro-isoquinoline to provide the title compound in moderate yield.
LC/MS: m/z = 371 (MH⁺)

### Example 171

### (6-Bromo-pyrazolo[1,5-a]pyridin-2-yl)-((R)1-methyl-3,4-dihydro-1H-isoquinolin-2yl)-methanone

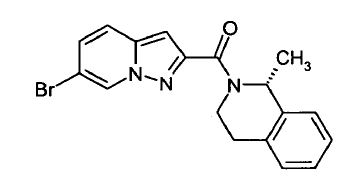

In close analogy to the procedure described in Example 1, 6-bromo-pyrazolo[1,5-a]pyridine-2-carboxylic acid is reacted with 1-(R)-methyl-1,2,3,4-tetrahydro-isoquinoline to provide the title compound in moderate yield.
LC/MS: m/z = 371 (MH⁺)

### Example 172

### (5-Bromo-1-methyl-3,4-dihydro-1H-isoquinolin-2-yl)-(6-chloro-pyrazolo[1,5-a]pyridin-2yl)-methanone

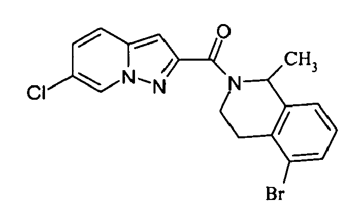

In close analogy to the procedure described in Example 1, 6-chloro-pyrazolo[1,5-a]pyridine-2-carboxylic acid is reacted with 5-bromo-1-methyl-1,2,3,4-tetrahydro-isoquinoline to provide the title compound in moderate yield.
LC/MS: m/z = 405 (MH⁺)
The stereo-isomers of this compound are separated. The S-configurated compound has a different activity than the R-configurated compound.

### Example 173

### (6-Bromo-pyrazolo[1,5-a]pyridin-2-yl)-5-methoxy-1-methyl-3,4-dihydro-1H-isoquinolin-2-yl)-methanone

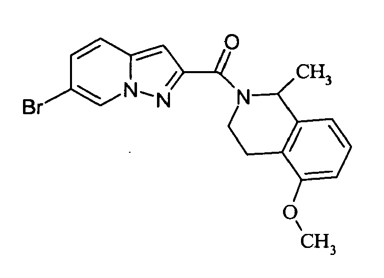

In close analogy to the procedure described in Example 1, 6-bromo-pyrazolo[1,5-a]pyridine-2-carboxylic acid is reacted with 5-methoxy-1-methyl-1,2,3,4-tetrahydro-isoquinoline to provide the title compound in moderate yield.
LC/MS: m/z = 401 (MH⁺)
The stereo-isomers of this compound are separated. The S-configurated compound has a different activity than the R-configurated compound.

### Example 174

### (5-Bromo-1-methyl-3,4-dihydro-1H-isoquinolin-2-yl)-(6-bromo-pyrazolo[1,5-a]pyridin-2-yl)-methanone

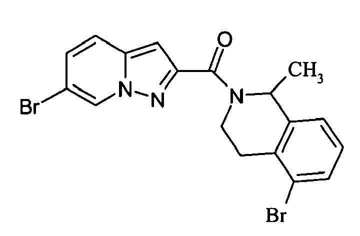

In close analogy to the procedure described in Example 1, 6-bromo-pyrazolo[1,5-a]pyridine-2-carboxylic acid is reacted with 5-bromo-1-methyl-1,2,3,4-tetrahydro-isoquinoline to provide the title compound in moderate yield.
LC/MS: m/z = 448 (MH⁺)
The stereo-isomers of this compound are separated. The S-configurated compound has a different activity than the R-configurated compound.

### Example 175

### (6-Bromo-pyrazolo[1,5-a]pyridin-2-yl)-(7-pyridin-4-yl-3,4-dihydro-1H-isoquinolin-2-yl)-methanone

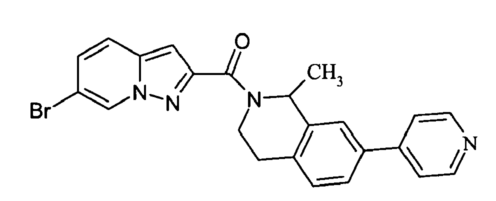

In close analogy to the procedure described in Example 1, 6-bromo-pyrazolo[1,5-a]pyridine-2-carboxylic acid is reacted with 1-methyl-7-pyridin-4-yl-1,2,3,4-tetrahydro-isoquinoline to provide the title compound in moderate yield.
LC/MS: m/z = 433 (MH⁺)
The stereo-isomers of this compound are separated. The S-configurated compound has a different activity than the R-configurated compound.

### Example 176

### (6-Bromo-pyrazolo[1,5-a]pyridin-2-yl)-(7-methoxy-1-methyl-3,4-dihydro-1H-isoquinolin-2-yl)-methanone

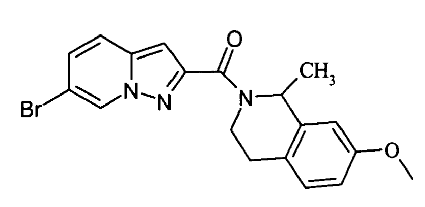

In close analogy to the procedure described in Example 1, 6-bromo-pyrazolo[1,5-a]pyridine-2-carboxylic acid is reacted with 7-methoxy-1-methyl-1,2,3,4-tetrahydro-isoquinoline to provide the title compound in moderate yield.
LC/MS: m/z = 400 (MH⁺)
The stereo-isomers of this compound are separated. The S-configurated compound has a different activity than the R-configurated compound.

### Example 177

### (6-Chloro-pyrazolo[1,5-a]pyridin-2-yl)-(7-methoxy-1-methyl-3,4-dihydro-1H-isoquinolin-2-yl)-methanone

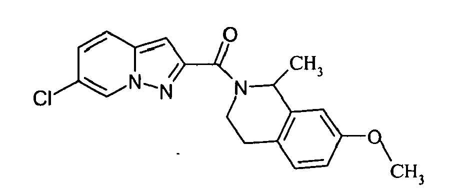

In close analogy to the procedure described in Example 1, 6-chloro-pyrazolo[1,5-a]pyridine-2-carboxylic acid is reacted with 7-methoxy-1-methyl-1,2,3,4-tetrahydro-isoquinoline to provide the title compound in moderate yield.
LC/MS: m/z = 356 (MH⁺)
The stereo-isomers of this compound are separated. The S-configurated compound has a different activity than the R-configurated compound.

### Example 178

### (6-Bromo-1-methyl-3,4-dihydro-1H-isoquinolin-2yl)-(6-bromo-pyrazolo[1,5-a]pyridin-2-yl)-methanone

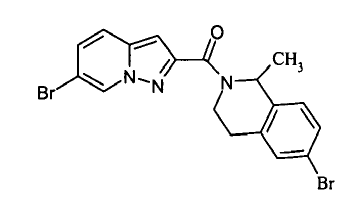

In close analogy to the procedure described in Example 1, 6-bromo-pyrazolo[1,5-a]pyridine-2-carboxylic acid is reacted with 6-bromo-1-methyl-1,2,3,4-tetrahydro-isoquinoline to provide the title compound in moderate yield.
LC/MS: m/z = 448 (MH⁺)
The stereo-isomers of this compound are separated. The S-configurated compound has a different activity than the R-configurated compound.

### Example 179

### (6-Bromo-pyrazolo[1,5-a]pyridin-2-yl)-(5,6-dimethoxy-1-methyl-3,4-dihydro-1H-isoquinolin-2-yl)-methanone

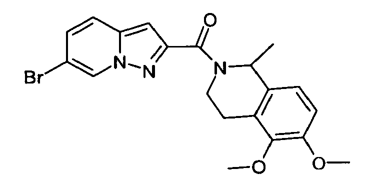

In close analogy to the procedure described in Example 1, 6-bromo-pyrazolo[1,5-a]pyridine-2-carboxylic acid is reacted with 5,6-dimethoxy-1-methyl-1,2,3,4-tetrahydro-isoquinoline to provide the title compound in moderate yield.
LC/MS: m/z = 431 (MH⁺)
The stereo-isomers of this compound are separated. The S-configurated compound has a different activity than the R-configurated compound.

### Example 180

### (6-Bromo-pyrazolo[1,5-a]pyridin-2-yl)-(5,8-difluoro-1-methyl-3,4-dihydro-1H-isoquinolin-2-yl)-methanone

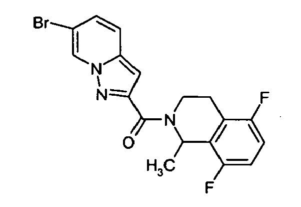

In close analogy to the procedure described in Example 1, 6-bromo-pyrazolo[1,5-a]pyridine-2-carboxylic acid is reacted with 5,8-difluoro-1-methyl-1,2,3,4-tetrahydro-isoquinoline to provide the title compound in moderate yield.
LC/MS: m/z = 407 (MH⁺)
The stereo-isomers of this compound are separated. The S-configurated compound has a different activity than the R-configurated compound.

### Example 181

### (6-Chloro-pyrazolo[1,5-a]pyridin-2-yl)-(5,8-difluoro-1-methyl-3,4-dihydro-1H-isoquinolin-2-yl)-methanone

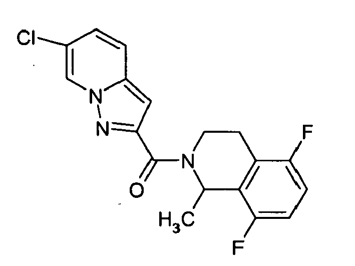

In close analogy to the procedure described in Example 1, 6-chloro-pyrazolo[1,5-a]pyridine-2-carboxylic acid is reacted with 5,8-difluoro-1-methyl-1,2,3,4-tetrahydro-isoquinoline to provide the title compound in moderate yield.
LC/MS: m/z = 362 (MH⁺)
The stereo-isomers of this compound are separated. The S-configurated compound has a different activity than the R-configurated compound.

### Example 182

### (6-Bromopyrazolo[1,5-a]pyridin-2-yl)-(1-methyl-5-trifluoromethyl-3,4-dihydro-1H-isoquinolin-2-yl)-methanone

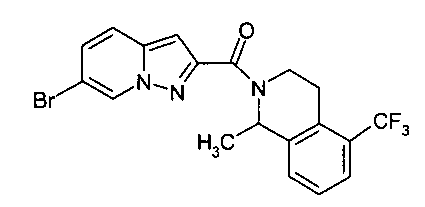

In close analogy to the procedure described in Example 1, 6-bromo-pyrazolo[1,5-a]pyridine-2-carboxylic acid is reacted with 1,5-dimethyl-1,2,3,4-tetrahydro-isoquinoline to provide the title compound in moderate yield.
LC/MS: m/z = 438 (MH⁺)
The stereo-isomers of this compound are separated. The S-configurated compound has a different activity than the R-configurated compound.

### Example 183

### (6-Chloro-pyrazolo[1,5-a]pyridin-2-yl)-(1-methyl-5-trifluoromethyl-3,4-dihydro-1H-isoquinolin-2-yl)-methanone

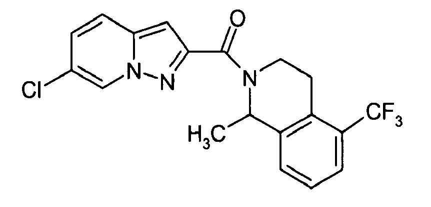

In close analogy to the procedure described in Example 1, 6-chloro-pyrazolo[1,5-a]pyridine-2-carboxylic acid is reacted with 1,5-dimethyl-1,2,3,4-tetrahydro-isoquinoline to provide the title compound in moderate yield.
LC/MS: m/z = 394 (MH⁺)
The stereo-isomers of this compound are separated. The S-configurated compound has a different activity than the R-configurated compound.

### Example 184

### (6-Chloro-pyrazolo[1,5-a]pyridin-2-yl)-(5-methyl-7,8-dihydro-5H-[1,6]naphthyridin-6-yl)-methanone

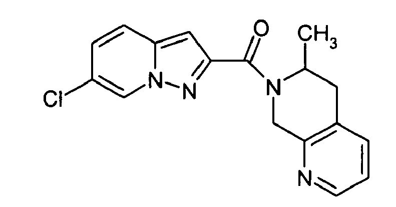

In close analogy to the procedure described in Example 1, 6-chloro-pyrazolo[1,5-a]pyridine-2-carboxylic acid is reacted with 5-methyl-5,6,7,8-tetrahydro-[1,6]naphthyridine to provide the title compound in moderate yield.
LC/MS: m/z = 327 (MH⁺)
The stereo-isomers of this compound are separated. The S-configurated compound has a different activity than the R-configurated compound.

### Example 185

### (3-Bromo-7,8-dihydro-5H-[1,6]naphthyridin-6-yl)-(6-chloro-pyrazolo[1,5-a]pyridin-2-yl)-methanone

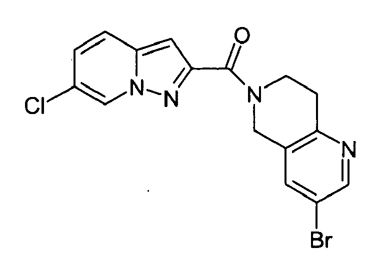

In close analogy to the procedure described in Example 1, 6-chloro-pyrazolo[1,5-a]pyridine-2-carboxylic acid is reacted with 3-bromo-5,6,7,8-tetrahydro-[1,6]naphthyridine to provide the title compound..
LC/MS: m/z = 393 (MH⁺)

### Example 186

### (6-Bromo-pyrazolo[1,5-a]pyridin-2-yl)-(1-methyl-6-pyridin-3-yl-3,4-dihydro-1H-isoquinolin-2-yl)-methanone

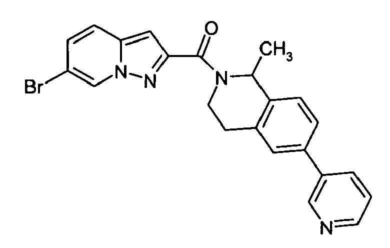

In close analogy to the procedure described in Example 1, 6-bromo-pyrazolo[1,5-a]pyridine-2-carboxylic acid is reacted with 1-methyl-6-pyridin-3-yl-1,2,3,4-tetrahydro-isoquinoline to provide the title compound in moderate yield.
LC/MS: m/z = 448 (MH⁺)
The stereo-isomers of this compound are separated. The S-configurated compound has a different activity than the R-configurated compound.

### Example 187

### (3-Bromo-7,8-dihydro-5H-[1,6]naphthyridin-6-yl)-(6-bromo-pyrazolo[1,5-a]pyridin-2-yl)-methanone

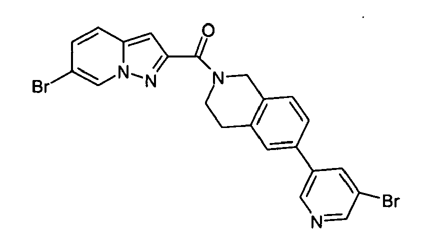

In close analogy to the procedure described in Example 1, 6-bromo-pyrazolo[1,5-a]pyridine-2-carboxylic acid is reacted with 6-(5-bromo-pyridin-3-yl)-1,2,3,4-tetrahydro-isoquinoline to provide the title compound in moderate yield.
LC/MS: m/z = 513 (MH⁺)

### Example 188

### (6-Bromo-pyrazolo[1,5-a]pyridin-2-yl)-(1-methyl-7-pyridin-3-yl-3,4-dihydro-1H-isoquinolin-2-yl)-methanone

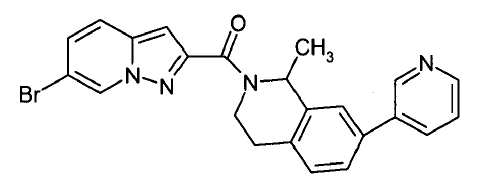

In close analogy to the procedure described in Example 1, 6-bromo-pyrazolo[1,5-a]pyridine-2-carboxylic acid is reacted with 1-methyl-7-pyridin-3-yl-1,2,3,4-tetrahydro-isoquinoline to provide the title compound in moderate yield.
LC/MS: m/z = 448 (MH⁺)
The stereo-isomers of this compound are separated. The S-configurated compound has a different activity than the R-configurated compound.

### Example 189

### 2-(6-Bromo-pyrazolo[1,5-a]pyridine-2-carbonyl-)1,2,3,4-tetrahydro-isoquinoline-7-carbonitrile

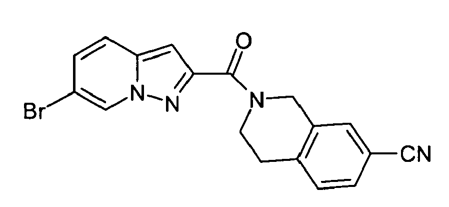

In close analogy to the procedure described in Example 1, 6-bromo-pyrazolo[1,5-a]pyridine-2-carboxylic acid is reacted with 1,2,3,4-tetrahydro-isoquinoline-7-carbonitrile to provide the title compound in moderate yield.
LC/MS: m/z = 382 (MH⁺)

### Example 190

### (6-Bromo-pyrazolo[1,5-a]pyridin-2-yl)-(1-methyl-7-pyridin-4-yl-3,4-dihydro-1H-isoquinolin-2-yl)-methanone

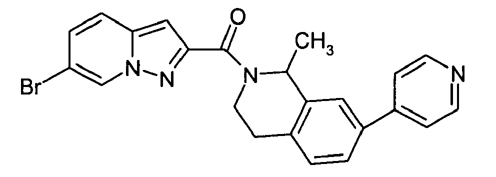

In close analogy to the procedure described in Example 1, 6-bromo-pyrazolo[1,5-a]pyridine-2-carboxylic acid is reacted with 1-methyl-7-pyridin-4-yl-1,2,3,4-tetrahydro-isoquinoline to provide the title compound in moderate yield.
LC/MS: m/z = 448 (MH⁺)
The stereo-isomers of this compound are separated. The S-configurated compound has a different activity than the R-configurated compound.

### Example 191

### (6-Bromo-pyrazolo[1,5-a]pyridin-2-yl)-(5-nitro-3,4-dihydro-1H-isoquinolin-2-yl)-methanone

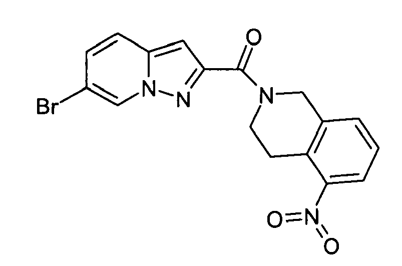

In close analogy to the procedure described in Example 1, 6-bromo-pyrazolo[1,5-a]pyridine-2-carboxylic acid is reacted with 5-nitro-1,2,3,4-tetrahydro-isoquinoline to provide the title compound in moderate yield.
LC/MS: m/z = 401 (MH⁺)

### Example 192

### (6-Chloro-pyrazolo[1,5-a]pyridin-2-yl)-(5-nitro-3,4-dihydro-1H-isoquinolin-2-yl)-methanone

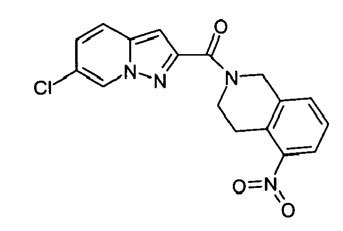

In close analogy to the procedure described in Example 1, 6-chloro-pyrazolo[1,5-a]pyridine-2-carboxylic acid is reacted with 5-nitro-1,2,3,4-tetrahydro-isoquinoline to provide the title compound in moderate yield.
LC/MS: m/z = 357 (MH⁺)

### Example 193

### (6-Bromo-pyrazolo[1,5-a]pyridin-2-yl)-(1-methyl-7-pyrimidin-5-yl-3,4-dihydro-1H-isoquinolin-2-yl)-methanone

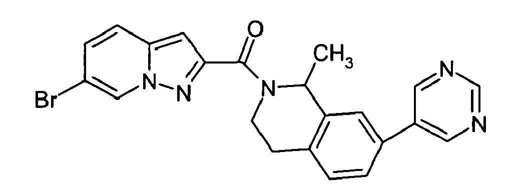

In close analogy to the procedure described in Example 1, 6-bromo-pyrazolo[1,5-a]pyridine-2-carboxylic acid is reacted with 1-methyl-7-pyridin-5-yl-1,2,3,4-tetrahydro-isoquinoline to provide the title compound in moderate yield.
LC/MS: m/z = 449 (MH⁺)
The stereo-isomers of this compound are separated. The S-configurated compound has a different activity than the R-configurated compound.

### Example 194

### (6-Bromo-pyrazolo[1,5-a]pyridin-2-yl)-(7-morpholin-4-yl-3,4-dihydro-1H-isoquinolin-2-yl)-methanone

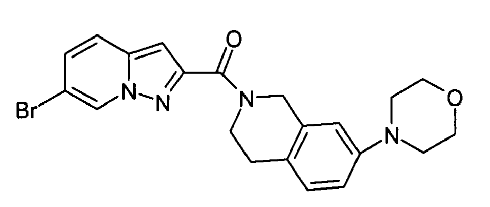

In close analogy to the procedure described in Example 1, 6-bromo-pyrazolo[1,5-a]pyridine-2-carboxylic acid is reacted with 7-morpholin-4-yl-1,2,3,4-tetrahydro-isoquinoline to provide the title compound in moderate yield.
LC/MS: m/z = 442 (MH⁺)

### Example 195

### (6-Chloro-pyrazolo[1,5-a]pyridin-2-yl)-(7-morpholin-4-yl-3,4-dihydro-1H-isoquinolin-2-yl)-methanone

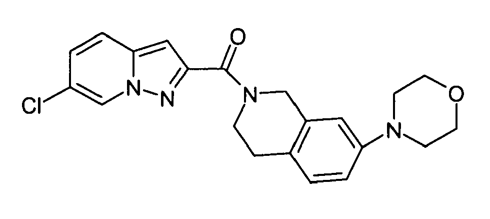

In close analogy to the procedure described in Example 1, 6-chloro-pyrazolo[1,5-a]pyridine-2-carboxylic acid is reacted with 7-morpholin-4-yl-1,2,3,4-tetrahydro-isoquinoline to provide the title compound in moderate yield.
LC/MS: m/z = 397 (MH⁺)

### Example 196

### (6-Bromo-pyrazolo[1,5-a]pyridin-2-yl)-(8-morpholin-4-yl-3,4-dihydro-1H-isoquinolin-2-yl)-methanone

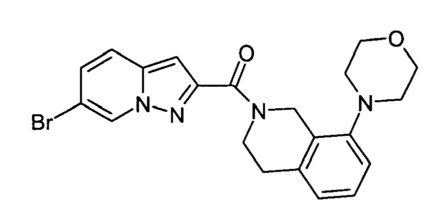

In close analogy to the procedure described in Example 1, 6-bromo-pyrazolo[1,5-a]pyridine-2-carboxylic acid is reacted with 8-morpholin-4-yl-1,2,3,4-tetrahydro-isoquinoline to provide the title compound in moderate yield.
LC/MS: m/z = 442 (MH⁺)

### Example 197

### (6-Chloro-pyrazolo[1,5-a]pyridin-2-yl)-(8-morpholin-4-yl-3,4-dihydro-1H-isoquinolin-2-yl)-methanone

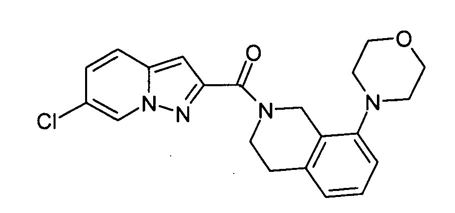

In close analogy to the procedure described in Example 1, 6-chloro-pyrazolo[1,5-a]pyridine-2-carboxylic acid is reacted with 8-morpholin-4-yl-1,2,3,4-tetrahydro-isoquinoline to provide the title compound in moderate yield.
LC/MS: m/z = 397 (MH⁺)

### Example 198

### (6-Bromo-pyrazolo[1,5-a]pyridin-2-yl)-(6-morpholin-4-yl-3,4-dihydro-1H-isoquinolin-2-yl)-methanone

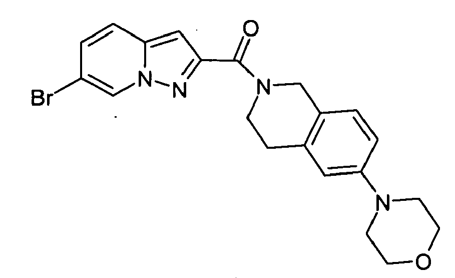

In close analogy to the procedure described in Example 1, 6-bromo-pyrazolo[1,5-a]pyridine-2-carboxylic acid is reacted with 6-morpholin-4-yl-1,2,3,4-tetrahydro-isoquinoline to provide the title compound in moderate yield.
LC/MS: m/z = 442 (MH⁺)

### Example 199

### (6-Chloro-pyrazolo[1,5-a]pyridin-2-yl)-(6-morpholin-4-yl-3,4-dihydro-1H-isoquinolin-2-yl)-methanone

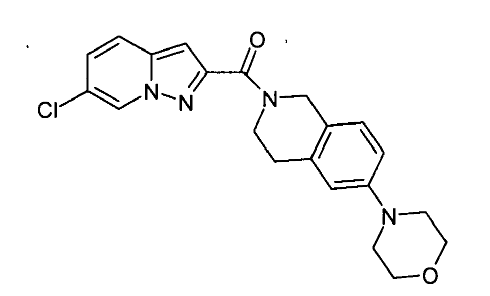

In close analogy to the procedure described in Example 1, 6-chloro-pyrazolo[1,5-a]pyridine-2-carboxylic acid is reacted with 6-morpholin-4-yl-1,2,3,4-tetrahydro-isoquinoline to provide the title compound in moderate yield.
LC/MS: m/z = 397 (MH⁺)

### Example 200

### (6-Bromo-pyrazolo[1,5-a]pyridin-2-yl)-(5-morpholin-4-yl-3,4-dihydro-1H-isoquinolin-2-yl)-methanone

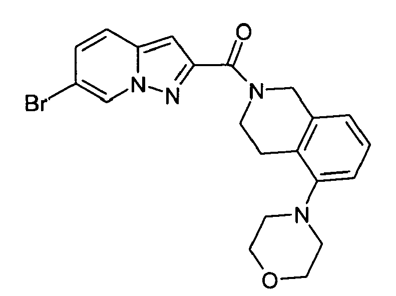

In close analogy to the procedure described in Example 1, 6-bromo-pyrazolo[1,5-a]pyridine-2-carboxylic acid is reacted with 5-morpholin-4-yl-1,2,3,4-tetrahydro-isoquinoline to provide the title compound in moderate yield.
LC/MS: m/z = 442 (MH⁺)

### Example 201

### (6-Chloro-pyrazolo[1,5-a]pyridin-2-yl)-(5-morpholin-4-yl-3,4-dihydro-1H-isoquinolin-2-yl)-methanone

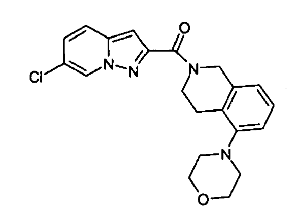

In close analogy to the procedure described in Example 1, 6-chloro-pyrazolo[1,5-a]pyridine-2-carboxylic acid is reacted with 5-morpholin-4-yl-1,2,3,4-tetrahydro-isoquinoline to provide the title compound in moderate yield.
LC/MS: m/z = 397 (MH⁺)

### Example 202

### (6-Bromo-pyrazolo[1,5-a]pyridin-2-yl)-(1-methyl-6-pyrimidin-5-yl-3,4-dihydro-1H-isoquinolin-2-yl)-methanone

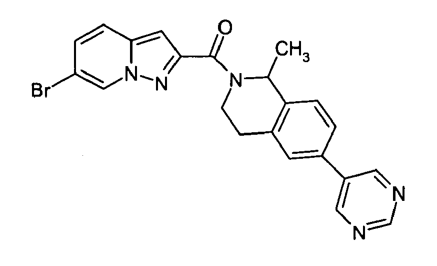

In close analogy to the procedure described in Example 1, 6-bromo-pyrazolo[1,5-a]pyridine-2-carboxylic acid is reacted with 1-methyl-6-pyrimidin-5-yl-1,2,3,4-tetrahydro-isoquinoline to provide the title compound in moderate yield.
LC/MS: m/z = 449 (MH⁺)
The stereo-isomers of this compound are separated. The S-configurated compound has a different activity than the R-configurated compound.

### Example 203

### (6-Bromo-pyrazolo[1,5-a]pyridin-2-yl)-(1-methyl-5-pyrimidin-5-yl-3,4-dihydro-1H-isoquinolin-2-yl)-methanone

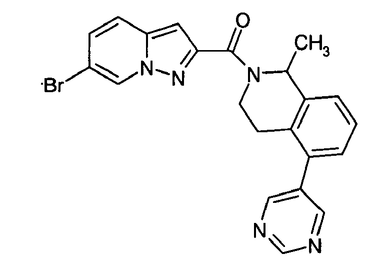

In close analogy to the procedure described in Example 1, 6-bromo-pyrazolo[1,5-a]pyridine-2-carboxylic acid is reacted with 1-methyl-5-pyrimidin-5-yl-1,2,3,4-tetrahydro-isoquinoline to provide the title compound in moderate yield.
LC/MS: m/z = 449 (MH⁺)
The stereo-isomers of this compound are separated. The S-configurated compound has a different activity than the R-configurated compound.

### Example 204

### (6-Bromo-pyrazolo[1,5-a]pyridin-2-yl)-(1-methyl-5-pyridin-3-yl-3,4-dihydro-1H-isoquinolin-2-yl)-methanone

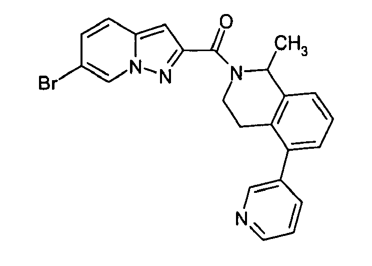

In close analogy to the procedure described in Example 1, 6-bromo-pyrazolo[1,5-a]pyridine-2-carboxylic acid is reacted with 1-methyl-5-pyridin-3-yl-1,2,3,4-tetrahydro-isoquinoline to provide the title compound in moderate yield.
LC/MS: m/z = 449 (MH⁺)
The stereo-isomers of this compound are separated. The S-configurated compound has a different activity than the R-configurated compound.

### Example 205

### (3-Bromo-7,8-dihydro-5H-[1,6]naphthyridin-6-yl)-(6-bromo-pyrazolo[1,5-a]pyridin-2-yl)-methanone

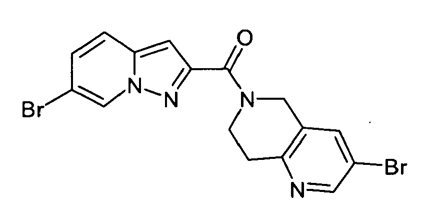

In close analogy to the procedure described in Example 1, 6-bromo-pyrazolo[1,5-a]pyridine-2-carboxylic acid is reacted with 3-bromo-5,6,7,8-tetrahydro-[1,6]naphthyridine to provide the title compound.
LC/MS: m/z = 439 (MH⁺)

### Example 206

### (6-Chloro-pyrazolo[1,5-a]pyridin-2-yl)-(1-methyl-7-trifluoromethyl-3,4-dihydro-1H-isoquinolin-2-yl)-methanone

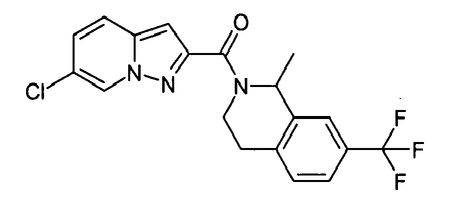

In close analogy to the procedure described in Example 1, 6-chloro-pyrazolo[1,5-a]pyridine-2-carboxylic acid is reacted with 1-methyl-7-trifluoromethyl-1,2,3,4-tetrahydro-isoquinoline to provide the title compound.
LC/MS: m/z = 395 (MH⁺)
The stereo-isomers of this compound are separated. The S-configurated compound has a different activity than the R-configurated compound.

### Example 207

### (6-Bromo-pyrazolo[1,5-a]pyridin-2-yl)-(1-methyl-7-trifluoromethyl-3,4-dihydro-1H-isoquinolin-2-yl)-methanone

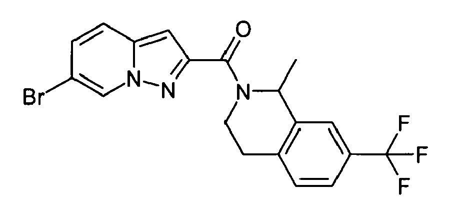

In close analogy to the procedure described in Example 1, 6-bromo-pyrazolo[1,5-a]pyridine-2-carboxylic acid is reacted with 1-methyl-7-trifluoromethyl-1,2,3,4-tetrahydro-isoquinoline to provide the title compound.
LC/MS: m/z = 439 (MH⁺)
The stereo-isomers of this compound are separated. The S-configurated compound has a different activity than the R-configurated compound.

### Example 208

### (6-Chloro-pyrazolo[1,5-a]pyridin-2-yl)-(1,5-dimethyl-3,4-dihydro-1H-isoquinolin-2-yl)-methanone

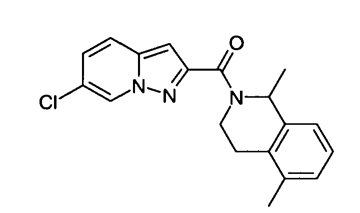

In close analogy to the procedure described in Example 1, 6-chloro-pyrazolo[1,5-a]pyridine-2-carboxylic acid is reacted with 1,5-dimethyl-1,2,3,4-tetrahydro-isoquinoline to provide the title compound.
LC/MS: m/z = 340 (MH⁺)
The stereo-isomers of this compound are separated. The S-configurated compound has a different activity than the R-configurated compound.

### Example 209

### (6-Chloro-pyrazolo[1,5-a]pyridin-2-yl)-(1,7-dimethyl-3,4-dihydro-1H-isoquinolin-2-yl)-methanone

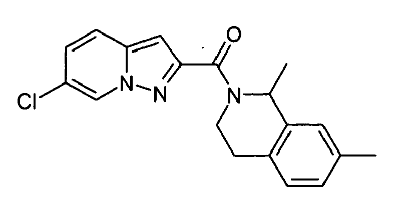

In close analogy to the procedure described in Example 1, 6-chloro-pyrazolo[1,5-a]pyridine-2-carboxylic acid is reacted with 1,7-dimethyl-1,2,3,4-tetrahydro-isoquinoline to provide the title compound.
LC/MS: m/z = 340 (MH⁺)
The stereo-isomers of this compound are separated. The S-configurated compound has a different activity than the R-configurated compound.

### Example 210

### (6-Bromo-pyrazolo[1,5-a]pyridin-2-yl)-(1,7-dimethyl-3,4-dihydro-1H-isoquinolin-2-yl)-methanone

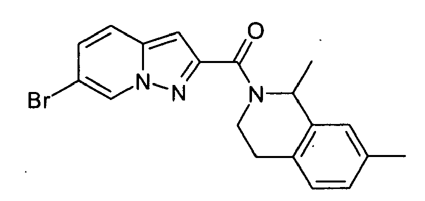

In close analogy to the procedure described in Example 1, 6-bromo-pyrazolo[1,5-a]pyridine-2-carboxylic acid is reacted with 1,7-dimethyl-1,2,3,4-tetrahydro-isoquinoline to provide the title compound.
LC/MS: m/z = 385 (MH⁺)
The stereo-isomers of this compound are separated. The S-configurated compound has a different activity than the R-configurated compound.

### Example 211

### (6-Bromo-pyrazolo[1,5-a]pyridin-2-yl)-(1,5-dimethyl-3,4-dihydro-1H-isoquinolin-2-yl)-methanone

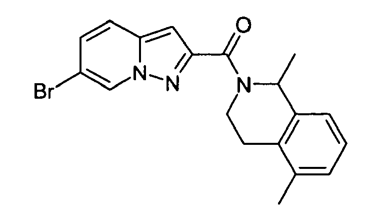

In close analogy to the procedure described in Example 1, 6-bromo-pyrazolo[1,5-a]pyridine-2-carboxylic acid is reacted with 1,5-dimethyl-1,2,3,4-tetrahydro-isoquinoline to provide the title compound.
LC/MS: m/z = 384 (MH⁺)
The stereo-isomers of this compound are separated. The S-configurated compound has a different activity than the R-configurated compound.

### Example 212

### (7-Chloro-1-methyl-3,4-dihydro-1H-isoquinolin-2-yl)-(6-chloro-pyrazolo[1,5-a]pyridin-2-yl)-methanone

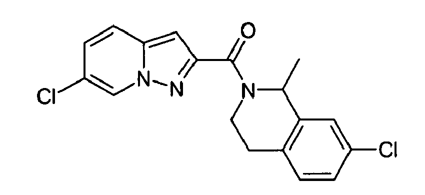

In close analogy to the procedure described in Example 1, 6-chloro-pyrazolo[1,5-a]pyridine-2-carboxylic acid is reacted with 7-chloro-1-methyl-1,2,3,4-tetrahydro-isoquinoline to provide the title compound.
LC/MS: m/z = 362 (MH⁺)
The stereo-isomers of this compound are separated. The S-configurated compound has a different activity than the R-configurated compound.

### Example 213

### (6-Bromo-pyrazolo[1,5-a]pyridin-2-yl)-(7-chloro-1-methyl-3,4-dihydro-1H-isoquinolin-2-yl)-methanone

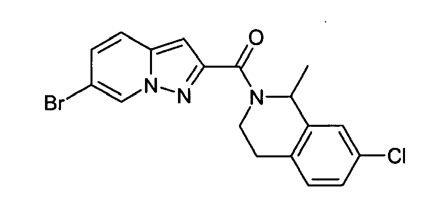

In close analogy to the procedure described in Example 1, 6-bromo-pyrazolo[1,5-a]pyridine-2-carboxylic acid is reacted with 7-chloro-1-methyl-1,2,3,4-tetrahydro-isoquinoline to provide the title compound.
LC/MS: m/z = 406 (MH⁺)
The stereo-isomers of this compound are separated. The S-configurated compound has a different activity than the R-configurated compound.

### Example 214

### (5-Chloro-1-methyl-3,4-dihydro-1H-isoquinolin-2-yl)-(6-chloro-pyrazolo[1,5-a]pyridin-2-yl)-methanone

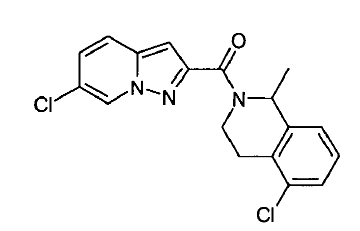

In close analogy to the procedure described in Example 1, 6-chloro-pyrazolo[1,5-a]pyridine-2-carboxylic acid is reacted with 5-chloro-1-methyl-1,2,3,4-tetrahydro-isoquinoline to provide the title compound.
LC/MS: m/z = 362 (MH⁺)
The stereo-isomers of this compound are separated. The S-configurated compound has a different activity than the R-configurated compound.

### Example 215

### N-[2-(6-Chloro-pyrazolo[1,5-a]pyridine-2-carbonyl)-1,2,3,4-tetrahydro-isoquinolin-5-yl]-acetamide

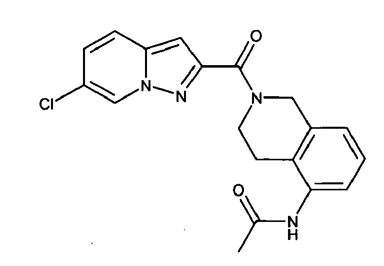

In close analogy to the procedure described in Example 1, 6-chloro-pyrazolo[1,5-a]pyridine-2-carboxylic acid is reacted with N-(1,2,3,4-tetrahydro-isoquinolin-5-yl)-acetamide to provide the title compound in moderate yield.
LC/MS: m/z = 369 (MH⁺)

### Example 216

### N-[2-(6-Bromo-pyrazolo[1,5-a]pyridine-2-carbonyl)-1,2,3,4-tetrahydro-isoquinolin-5-yl]-acetamide

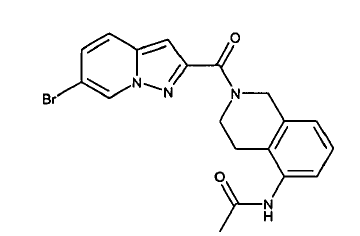

In close analogy to the procedure described in Example 1, 6-bromo-pyrazolo[1,5-a]pyridine-2-carboxylic acid is reacted with N-(1,2,3,4-tetrahydro-isoquinolin-5-yl)-acetamide to provide the title compound in moderate yield.
LC/MS: m/z = 413 (MH⁺)

### Example 217

### N-[2-(6-Chloro-pyrazolo[1,5-a]pyridine-2-carbonyl)-1-methyl-1,2,3,4-tetrahydro-isoquinolin-5-yl]-acetamide

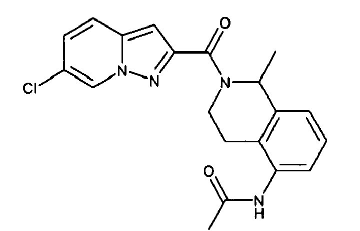

In close analogy to the procedure described in Example 1, 6-chloro-pyrazolo[1,5-a]pyridine-2-carboxylic acid is reacted with N-(1-methyl-1,2,3,4-tetrahydro-isoquinolin-5-yl)-acetamide to provide the title compound in moderate yield.
LC/MS: m/z = 383 (MH⁺)
The stereo-isomers of this compound are separated. The S-configurated compound has a different activity than the R-configurated compound.

### Example 218

### N-[2-(6-Bromo-pyrazolo[1,5-a]pyridine-2-carbonyl)-1-methyl-1,2,3,4-tetrahydro-isoquinolin-5-yl]-acetamide

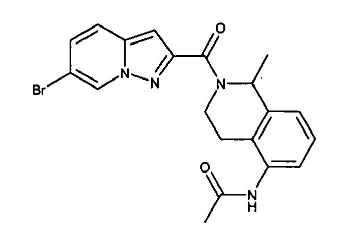

In close analogy to the procedure described in Example 1, 6-bromo-pyrazolo[1,5-a]pyridine-2-carboxylic acid is reacted with N-(1-methyl-1,2,3,4-tetrahydro-isoquinolin-5-yl)-acetamide to provide the title compound in moderate yield.
LC/MS: m/z = 428 (MH⁺)
The stereo-isomers of this compound are separated. The S-configurated compound has a different activity than the R-configurated compound.

### Example 219

### N-[2-(6-Chloro-pyrazolo[1,5-a]pyridine-2-carbonyl)-1-methyl-1,2,3,4-tetrahydro-isoquinolin-5-yl]-acetamide

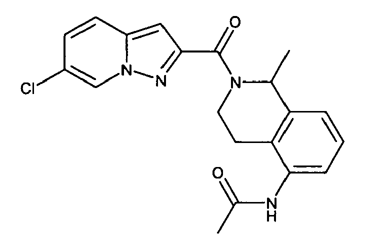

In close analogy to the procedure described in Example 1, 6-chloro-pyrazolo[1,5-a]pyridine-2-carboxylic acid is reacted with N-(1-methyl-1,2,3,4-tetrahydro-isoquinolin-5-yl)-acetamide to provide the title compound in moderate yield.
LC/MS: m/z = 383 (MH⁺)
The stereo-isomers of this compound are separated. The S-configurated compound has a different activity than the R-configurated compound.

### Example 220

### 2-(6-Bromo-pyrazolo[1,5-a]pyridine-2-carbonyl)-1-methyl-1,2,3,4-tetrahydro-isoquinoline-5-carboxylic acid dimethylamide

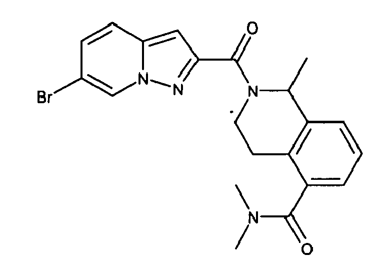

In close analogy to the procedure described in Example 1, 6-bromo-pyrazolo[1,5-a]pyridine-2-carboxylic acid is reacted with 1-methyl-1,2,3,4-tetrahydro-isoquinoline-5-carboxylic acid dimethylamide to provide the title compound in moderate yield.
LC/MS: m/z = 442 (MH⁺)
The stereo-isomers of this compound are separated. The S-configurated compound has a different activity than the R-configurated compound.

### Example 221

### 2-(6-Chloro-pyrazolo[1,5-a]pyridine-2-carbonyl)-1-methyl-1,2,3,4-tetrahydro-isoquinoline-5-carboxylic acid dimethylamide

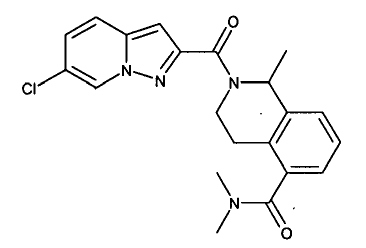

In close analogy to the procedure described in Example 1, 6-chloro-pyrazolo[1,5-a]pyridine-2-carboxylic acid is reacted with 1-methyl-1,2,3,4-tetrahydro-isoquinoline-5-carboxylic acid dimethylamide to provide the title compound in moderate yield.
LC/MS: m/z = 397 (MH⁺)
The stereo-isomers of this compound are separated. The S-configurated compound has a different activity than the R-configurated compound.

### Example 222

### (6-Bromo-pyrazolo[1,5-a]pyridin-2-yl)-(8-methyl-2,3,5,8-tetrahydro-6H-1,4-dioxa-7-azaphenanthren-7-yl)-methanone

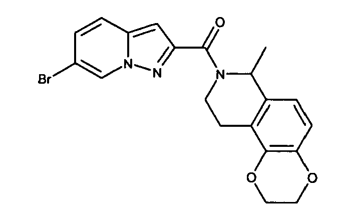

In close analogy to the procedure described in Example 1, 6-bromo-pyrazolo[1,5-a]pyridine-2-carboxylic acid is reacted with 8-methyl-2,3,5,6,7,8-hexahydro-1,4-dioxa-7-aza-phenanthrene to provide the title compound in moderate yield.
LC/MS: m/z = 428 (MH⁺)
The stereo-isomers of this compound are separated. The S-configurated compound has a different activity than the R-configurated compound.

### Example 223

### 2-(6-Bromo-pyrazolo[1,5-a]pyridine-2-carbonyl)-1,2,3,4-tetrahydro-isoquinoline-5-carbonitrile

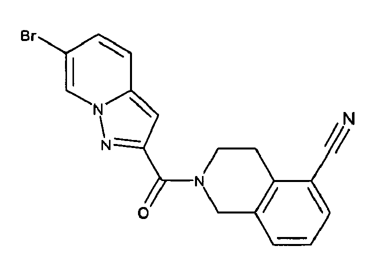

In close analogy to the procedure described in Example 1, 6-bromo-pyrazolo[1,5-a]pyridine-2-carboxylic acid is reacted with 1,2,3,4-tetrahydro-isoquinoline-5-carbonitrile to provide the title compound in moderate yield.
LC/MS: m/z = 382 (MH⁺)

### Example 224

### 2-(6-Bromo-pyrazolo[1,5-a]pyridine-2-carbonyl)-1,2,3,4-tetrahydro-isoquinoline-7-carbonitrile

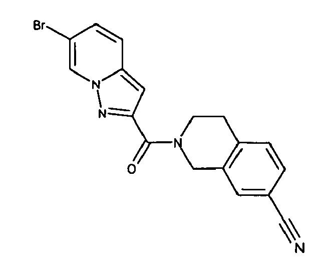

In close analogy to the procedure described in Example 1, 6-bromo-pyrazolo[1,5-a]pyridine-2-carboxylic acid is reacted with 1,2,3,4-tetrahydro-isoquinoline-7-carbonitrile to provide the title compound in moderate yield.
LC/MS: m/z = 382 (MH⁺)

### Example 225

### (6-Bromo-pyrazolo[1,5-a]pyridin-2-yl)-(6-pyridin-4-yl-3,4-dihydro-1H-isoquinolin-2-yl)-methanone

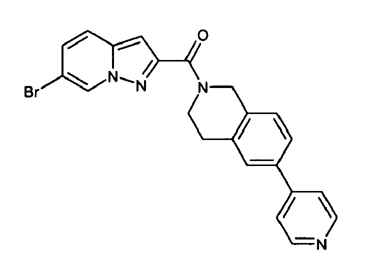

In close analogy to the procedure described in Example 1, 6-bromo-pyrazolo[1,5-a]pyridine-2-carboxylic acid is reacted with 6-pyridin-4-yl-1,2,3,4-tetrahydro-isoquinoline to provide the title compound in moderate yield.
LC/MS: m/z = 433 (MH⁺)

### Example 226

### (6-Chloro-pyrazolo[1,5-a]pyridin-2-yl)-(6-pyridin-4-yl-3,4-dihydro-1H-isoquinolin-2-yl)-methanone

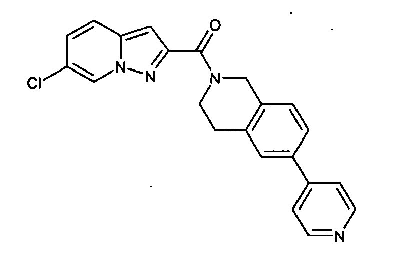

In close analogy to the procedure described in Example 1, 6-chloro-pyrazolo[1,5-a]pyridine-2-carboxylic acid is reacted with 6-pyridin-4-yl-1,2,3,4-tetrahydro-isoquinoline to provide the title compound in moderate yield.
LC/MS: m/z = 389 (MH⁺)

### Example 227

### (6-Chloro-pyrazolo[1,5-a]pyridin-2-yl)-(1-methyl-6-pyridin-4-yl-3,4-dihydro-1H-isoquinolin-2-yl)-methanone

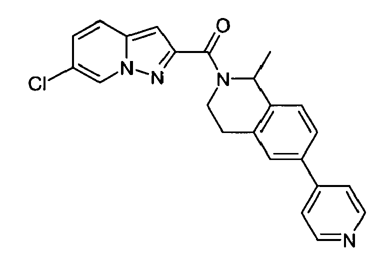

In close analogy to the procedure described in Example 1, 6-chloro-pyrazolo[1,5-a]pyridine-2-carboxylic acid is reacted with 1-methyl-6-pyridin-4-yl-1,2,3,4-tetrahydro-isoquinoline to provide the title compound in moderate yield.
LC/MS: m/z = 404 (MH⁺)
The stereo-isomers of this compound are separated. The S-configurated compound has a different activity than the R-configurated compound.

### Example 228

### (6-Bromo-pyrazolo[1,5-a]pyridin-2-yl)-(1-methyl-6-pyridin-4-yl-3,4-dihydro-1H-isoquinolin-2-yl)-methanone

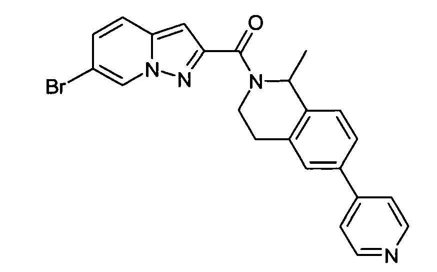

In close analogy to the procedure described in Example 1, 6-bromo-pyrazolo[1,5-a]pyridine-2-carboxylic acid is reacted with 1-methyl-6-pyridin-4-yl-1,2,3,4-tetrahydro-isoquinoline to provide the title compound in moderate yield.
LC/MS: m/z = 448 (MH⁺)
The stereo-isomers of this compound are separated. The S-configurated compound has a different activity than the R-configurated compound.

### Example 229

### (6-Bromo-pyrazolo[1,5-a]pyridin-2-yl)-(6-methyl-8,9-dihydro-6H-[1,3]dioxolo[4,5-f]isoquinolin-7-yl)-methanone

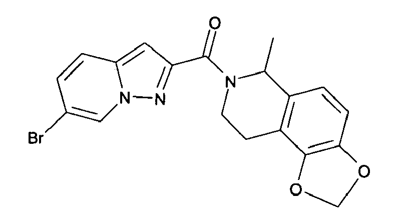

In close analogy to the procedure described in Example 1, 6-bromo-pyrazolo[1,5-a]pyridine-2-carboxylic acid is reacted with 6-methyl-6,7,8,9-tetrahydro-1,3-dioxa-7-azacyclopenta[a]naphthalene to provide the title compound in moderate yield.
LC/MS: m/z = 415 (MH⁺)
The stereo-isomers of this compound are separated. The S-configurated compound has a different activity than the R-configurated compound.

### Example 230

### (6-Bromo-pyrazolo[1,5-a]pyridin-2-yl)-(1-methyl-5-pyridin-4-yl-3,4-dihydro-1H-isoquinolin-2-yl)-methanone

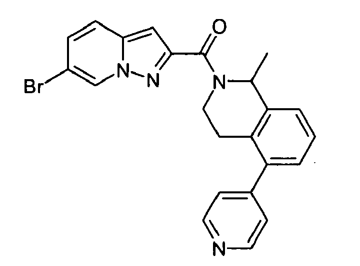

In close analogy to the procedure described in Example 1, 6-bromo-pyrazolo[1,5-a]pyridine-2-carboxylic acid is reacted with 1-methyl-5-pyridin-4-yl-1,2,3,4-tetrahydro-isoquinoline to provide the title compound in moderate yield.
LC/MS: m/z = 448 (MH⁺)
The stereo-isomers of this compound are separated. The S-configurated compound has a different activity than the R-configurated compound.

### Example 231

### (6-Chloro-pyrazolo[1,5-a]pyridin-2-yl)-(1-methyl-5-pyridin-4-yl-3,4-dihydro-1H-isoquinolin-2-yl)-methanone

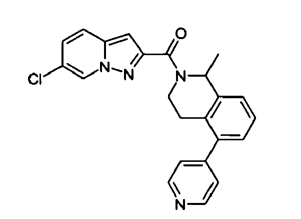

In close analogy to the procedure described in Example 1, 6-chloro-pyrazolo[1,5-a]pyridine-2-carboxylic acid is reacted with 1-methyl-5-pyridin-4-yl-1,2,3,4-tetrahydro-isoquinoline to provide the title compound in moderate yield.
LC/MS: m/z = 404 (MH⁺)
The stereo-isomers of this compound are separated. The S-configurated compound has a different activity than the R-configurated compound.

### Example 232

### (6-Bromo-3,4-dihydro-1H-isoquinolin-2-yl)-(6-bromo-pyrazolo[1,5-a]pyridin-2-yl)-methanone

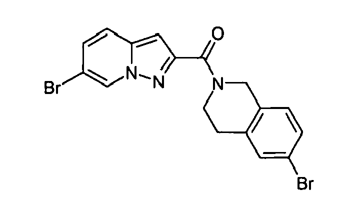

In close analogy to the procedure described in Example 1, 6-chloro-pyrazolo[1,5-a]pyridine-2-carboxylic acid is reacted with 6-bromo-1,2,3,4-tetrahydro-isoquinoline to provide the title compound in moderate yield.
LC/MS: m/z = 436 (MH⁺)

### Example 233

### (6-Bromo-pyrazolo[1,5-a]pyridin-2-yl)-(6-pyridin-2-yl-3,4-dihydro-1H-isoquinolin-2-yl)-methanone

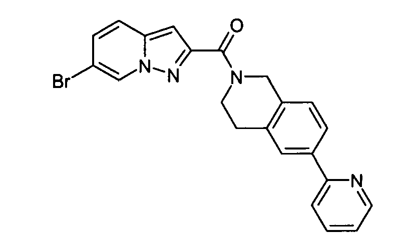

In close analogy to the procedure described in Example 1, 6-bromo-pyrazolo[1,5-a]pyridine-2-carboxylic acid is reacted with 6-pyridin-2-yl-1,2,3,4-tetrahydro-isoquinoline to provide the title compound in moderate yield.
LC/MS: m/z = 434 (MH⁺)

### Example 234

### (6-Bromo-pyrazolo[1,5-a]pyridin-2-yl)-(1-methyl-6-pyridin-2-yl-3,4-dihydro-1H-isoquinolin-2-yl)-methanone

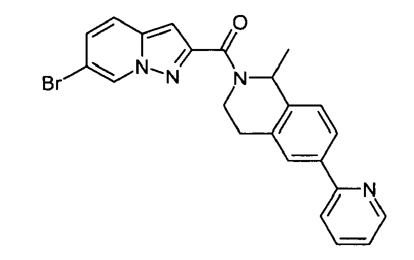

In close analogy to the procedure described in Example 1, 6-bromo-pyrazolo[1,5-a]pyridine-2-carboxylic acid is reacted with 1-methyl-6-pyridin-2-yl-1,2,3,4-tetrahydro-isoquinoline to provide the title compound in moderate yield.
LC/MS: m/z = 448 (MH⁺)
The stereo-isomers of this compound are separated. The S-configurated compound has a different activity than the R-configurated compound.

### Example 235

### (6-Chloro-pyrazolo[1,5-a]pyridin-2-yl)-(1-methyl-6-pyridin-2-yl-3,4-dihydro-1H-isoquinolin-2-yl)-methanone

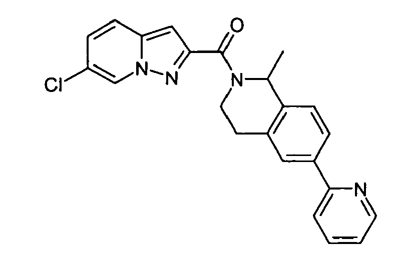

In close analogy to the procedure described in Example 1, 6-chloro-pyrazolo[1,5-a]pyridine-2-carboxylic acid is reacted with 1-methyl-6-pyridin-2-yl-1,2,3,4-tetrahydro-isoquinoline to provide the title compound in moderate yield.
LC/MS: m/z = 404 (MH⁺)
The stereo-isomers of this compound are separated. The S-configurated compound has a different activity than the R-configurated compound.

### Example 236

### (6-Chloro-pyrazolo[1,5-a]pyridin-2-yl)-(1-methyl-7-pyridin-2-yl-3,4-dihydro-1H-isoquinolin-2-yl)-methanone

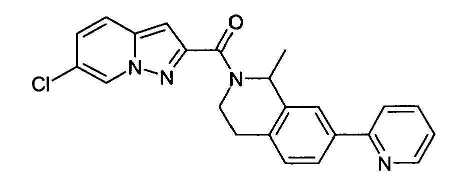

In close analogy to the procedure described in Example 1, 6-chloro-pyrazolo[1,5-a]pyridine-2-carboxylic acid is reacted with 1-methyl-7-pyridin-2-yl-1,2,3,4-tetrahydro-isoquinoline to provide the title compound in moderate yield.
LC/MS: m/z = 404 (MH⁺)
The stereo-isomers of this compound are separated. The S-configurated compound has a different activity than the R-configurated compound.

### Example 237

### (5-Bromo-3,4-dihydro-1H-isoquinolin-2-yl)-(6-bromo-pyrazolo[1,5-a]pyridin-2-yl)-methanone

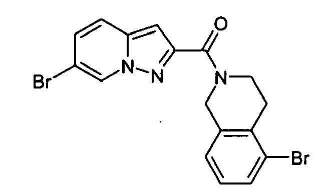

In close analogy to the procedure described in Example 1, 6-bromo-pyrazolo[1,5-a]pyridine-2-carboxylic acid is reacted with 5-bromo-1,2,3,4-tetrahydro-isoquinoline to provide the title compound in moderate yield.
LC/MS: m/z = 436 (MH⁺)

### Example 238

### (5-Bromo-3,4-dihydro-1H-isoquinolin-2-yl)-(6-chloro-pyrazolo[1,5-a]pyridin-2-yl)-methanone

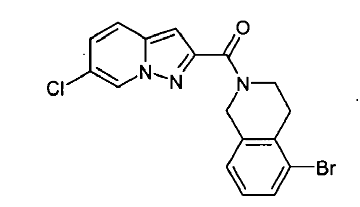

In close analogy to the procedure described in Example 1, 6-chloro-pyrazolo[1,5-a]pyridine-2-carboxylic acid is reacted with 5-bromo-1,2,3,4-tetrahydro-isoquinoline to provide the title compound in moderate yield.
LC/MS: m/z = 391 (MH⁺)

### Example 239

### (6-Bromo-pyrazolo[1,5-a]pyridin-2-yl)-(5-pyridin-2-yl-3,4-dihydro-1H-isoquinolin-2-yl)-methanone

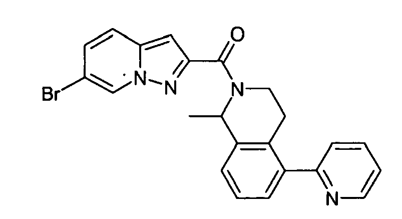

In close analogy to the procedure described in Example 1, 6-bromo-pyrazolo[1,5-a]pyridine-2-carboxylic acid is reacted with 1-methyl-5-pyridin-2-yl-1,2,3,4-tetrahydro-isoquinoline to provide the title compound in moderate yield.
LC/MS: m/z = 434 (MH⁺)
The stereo-isomers of this compound are separated. The S-configurated compound has a different activity than the R-configurated compound.

### Example 240

### (6-Bromo-pyrazolo[1,5-a]pyridin-2-yl)-[5-(2,4-dimethoxy-pyrimidin-5-yl)-1-methyl-3,4-dihydro-1H-isoquinolin-2-yl]-methanone

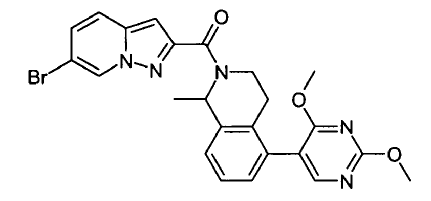

In close analogy to the procedure described in Example 1, 6-bromo-pyrazolo[1,5-a]pyridine-2-carboxylic acid is reacted with 5-(2,4-dimethoxy-pyrimidin-5-yl)-1-methyl-1,2,3,4-tetrahydro-isoquinoline to provide the title compound in moderate yield.
LC/MS: m/z = 509 (MH⁺)
The stereo-isomers of this compound are separated. The S-configurated compound has a different activity than the R-configurated compound.

### Example 241

### (6-Chloro-pyrazolo[1,5-a]pyridin-2-yl)-[5-(2,4-dimethoxy-pyrimidin-5-yl)-1-methyl-3,4-dihydro-1H-isoquinolin-2-yl]-methanone

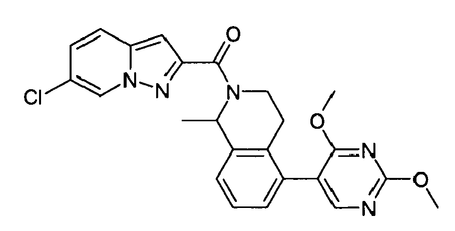

In close analogy to the procedure described in Example 1, 6-chloro-pyrazolo[1,5-a]pyridine-2-carboxylic acid is reacted with 5-(2,4-dimethoxy-pyrimidin-5-yl)-1-methyl-1,2,3,4-tetrahydro-isoquinoline to provide the title compound in moderate yield.
LC/MS: m/z = 464 (MH⁺)
The stereo-isomers of this compound are separated. The S-configurated compound has a different activity than the R-configurated compound.

### Example 242

### (6-Bromo-pyrazolo[1,5-a]pyridin-2-yl)-[5-(2,6-dimethoxy-pyridin-3-yl)-1-methyl-3,4-dihydro-1H-isoquinolin-2-yl]-methanone

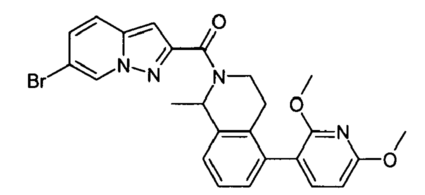

In close analogy to the procedure described in Example 1, 6-bromo-pyrazolo[1,5-a]pyridine-2-carboxylic acid is reacted with 5-(2,6-dimethoxy-pyridin-3-yl)-1-methyl-1,2,3,4-tetrahydro-isoquinoline to provide the title compound in moderate yield.
LC/MS: m/z = 508 (MH⁺)
The stereo-isomers of this compound are separated. The S-configurated compound has a different activity than the R-configurated compound.

### Example 243

### (6-Chloro-pyrazolo[1,5-a]pyridin-2-yl)-[5-(2,6-dimethoxy-pyridin-3-yl)-1-methyl-3,4-dihydro-1H-isoquinolin-2-yl]-methanone

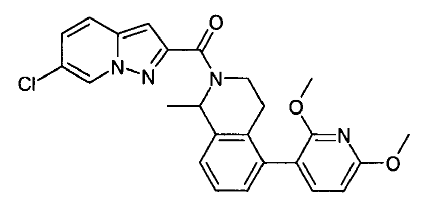

In close analogy to the procedure described in Example 1, 6-chloro-pyrazolo[1,5-a]pyridine-2-carboxylic acid is reacted with 5-(2,6-dimethoxy-pyridin-3-yl)-1-methyl-1,2,3,4-tetrahydro-isoquinoline to provide the title compound in moderate yield.
LC/MS: m/z = 464 (MH⁺)
The stereo-isomers of this compound are separated. The S-configurated compound has a different activity than the R-configurated compound.

### Example 244

### (6-Bromo-pyrazolo[1,5-a]pyridin-2-yl)-[5-(6-fluoro-pyridin-3-yl)-1-methyl-3,4-dihydro-1 H-isoquinolin-2-yl]-methanone

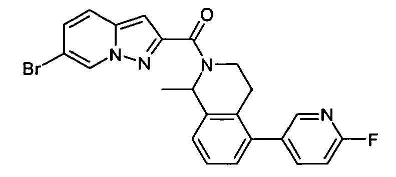

In close analogy to the procedure described in Example 1, 6-bromo-pyrazolo[1,5-a]pyridine-2-carboxylic acid is reacted with 5-(6-fluoro-pyridin-3-yl)-1-methyl-1,2,3,4-tetrahydro-isoquinoline to provide the title compound in moderate yield.
LC/MS: m/z = 466 (MH⁺)
The stereo-isomers of this compound are separated. The S-configurated compound has a different activity than the R-configurated compound.

### Example 245

### (6-Chloro-pyrazolo[1,5-a]pyridin-2-yl)-[5-(6-fluoro-pyridin-3-yl)-1-methyl-3,4-dihydro-1 H-isoquinolin-2-yl]-methanone

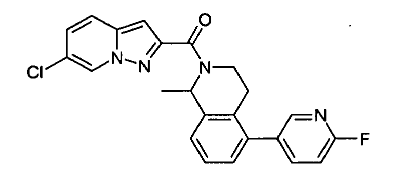

In close analogy to the procedure described in Example 1, 6-chloro-pyrazolo[1,5-a]pyridine-2-carboxylic acid is reacted with 5-(6-fluoro-pyridin-3-yl)-1-methyl-1,2,3,4-tetrahydro-isoquinoline to provide the title compound in moderate yield.
LC/MS: m/z = 421 (MH⁺)
The stereo-isomers of this compound are separated. The S-configurated compound has a different activity than the R-configurated compound.

### Example 246

### (6-Bromo-pyrazolo[1,5-a]pyridin-2-yl)-[5-(2-methoxy-pyridin-3-yl)-1-methyl-3,4-dihydro-1 H-isoquinolin-2-yl]-methanone

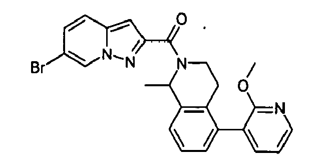

In close analogy to the procedure described in Example 1, 6-bromo-pyrazolo[1,5-a]pyridine-2-carboxylic acid is reacted with 5-(2-methoxy-pyridin-3-yl)-1-methyl-1,2,3,4-tetrahydro-isoquinoline to provide the title compound in moderate yield.
LC/MS: m/z = 478 (MH⁺)
The stereo-isomers of this compound are separated. The S-configurated compound has a different activity than the R-configurated compound.

### Example 247

### (6-Chloro-pyrazolo[1,5-a]pyridin-2-yl)-[5-(2-methoxy-pyridin-3-yl)-1-methyl-3,4-dihydro-1 H-isoquinolin-2-yl]-methanone

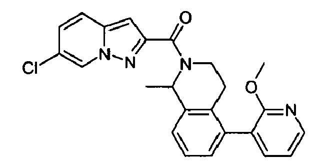

In close analogy to the procedure described in Example 1, 6-chloro-pyrazolo[1,5-a]pyridine-2-carboxylic acid is reacted with 5-(2-methoxy-pyridin-3-yl)-1-methyl-1,2,3,4-tetrahydro-isoquinoline to provide the title compound in moderate yield.
LC/MS: m/z = 433 (MH⁺)
The stereo-isomers of this compound are separated. The S-configurated compound has a different activity than the R-configurated compound.

### Example 248

### (6-Chloro-pyrazolo[1,5-a]pyridin-2-yl)-[5-(6-methoxy-pyridin-3-yl)-1-methyl-3,4-dihydro-1 H-isoquinolin-2-yl]-methanone

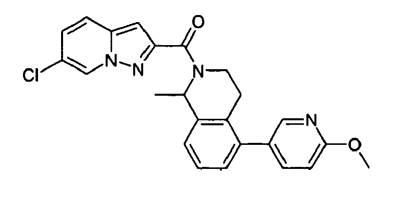

In close analogy to the procedure described in Example 1, 6-chloro-pyrazolo[1,5-a]pyridine-2-carboxylic acid is reacted with 5-(6-methoxy-pyridin-3-yl)-1-methyl-1,2,3,4-tetrahydro-isoquinoline to provide the title compound in moderate yield.
LC/MS: m/z = 433 (MH⁺)
The stereo-isomers of this compound are separated. The S-configurated compound has a different activity than the R-configurated compound.

### Example 249

### (6-Bromo-pyrazolo[1,5-a]pyridin-2-yl)-[5-(6-methoxy-pyridin-3-yl)-1-methyl-3,4-dihydro-1H-isoquinolin-2-yl]-methanone

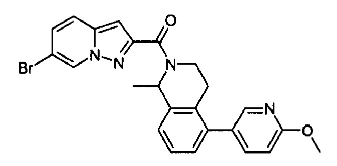

In close analogy to the procedure described in Example 1, 6-bromo-pyrazolo[1,5-a]pyridine-2-carboxylic acid is reacted with 5-(6-methoxy-pyridin-3-yl)-1-methyl-1,2,3,4-tetrahydro-isoquinoline to provide the title compound in moderate yield.
LC/MS: m/z = 477 (MH⁺)
The stereo-isomers of this compound are separated. The S-configurated compound has a different activity than the R-configurated compound.

### Example 250

### (6-Bromo-pyrazolo[1,5-a]pyridin-2-yl)-[6-(2-methoxy-pyridin-3-yl)-1-methyl-3,4-dihydro-1H-isoquinolin-2-yl]-methanone

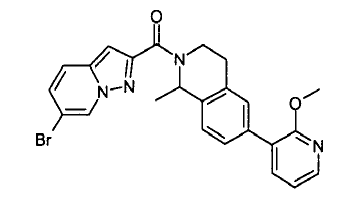

In close analogy to the procedure described in Example 1, 6-bromo-pyrazolo[1,5-a]pyridine-2-carboxylic acid is reacted with 6-(2-methoxy-pyridin-3-yl)-1-methyl-1,2,3,4-tetrahydro-isoquinoline to provide the title compound in moderate yield.
LC/MS: m/z = 478 (MH⁺)
The stereo-isomers of this compound are separated. The S-configurated compound has a different activity than the R-configurated compound.

### Example 251

### (6-Chloro-pyrazolo[1,5-a]pyridin-2-yl)-[6-(2-methoxy-pyridin-3-yl)-1-methyl-3,4-dihydro-1H-isoquinolin-2-yl]-methanone

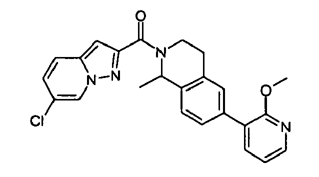

In close analogy to the procedure described in Example 1, 6-chloro-pyrazolo[1,5-a]pyridine-2-carboxylic acid is reacted with 6-(2-methoxy-pyridin-3-yl)-1-methyl-1,2,3,4-tetrahydro-isoquinoline to provide the title compound in moderate yield.
LC/MS: m/z = 433 (MH⁺)
The stereo-isomers of this compound are separated. The S-configurated compound has a different activity than the R-configurated compound.

### Example 252

### (6-Bromo-pyrazolo[1,5-a]pyridin-2-yl)-[6-(2-fluoro-pyridin-3-yl)-1-methyl-3,4-dihydro-1H-isoquinolin-2-yl]-methanone

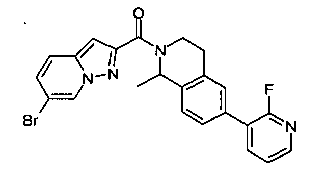

In close analogy to the procedure described in Example 1, 6-bromo-pyrazolo[1,5-a]pyridine-2-carboxylic acid is reacted with 6-(2-fluoro-pyridin-3-yl)-l-methyl-1,2,3,4-tetrahydro-isoquinoline to provide the title compound in moderate yield.
LC/MS: m/z = 466 (MH⁺)
The stereo-isomers of this compound are separated. The S-configurated compound has a different activity than the R-configurated compound.

### Example 253

### (6-Chloro-pyrazolo[1,5-a]pyridin-2-yl)-[6-(2-fluoro-pyridin-3-yl)-1-methyl-3,4-dihydro-1 H-isoquinolin-2-yl]-methanone

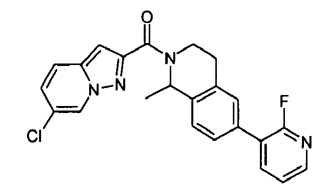

In close analogy to the procedure described in Example 1, 6-chloro-pyrazolo[1,5-a]pyridine-2-carboxylic acid is reacted with 6-(2-fluoro-pyridin-3-yl)-1-methyl-1,2,3,4-tetrahydro-isoquinoline to provide the title compound in moderate yield.
LC/MS: m/z = 421 (MH⁺)
The stereo-isomers of this compound are separated. The S-configurated compound has a different activity than the R-configurated compound.

### Example 254

### (6-Bromo-pyrazolo[1,5-a]pyridin-2-yl)-[6-(6-fluoro-pyridin-3-yl)-1-methyl-3,4-dihydro-1H-isoquinolin-2-yl]-methanone

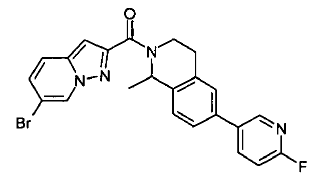

In close analogy to the procedure described in Example 1, 6-bromo-pyrazolo[1,5-a]pyridine-2-carboxylic acid is reacted with 6-(6-fluoro-pyridin-3-yl)-l-methyl-1,2,3,4-tetrahydro-isoquinoline to provide the title compound in moderate yield.
LC/MS: m/z = 466 (MH⁺)
The stereo-isomers of this compound are separated. The S-configurated compound has a different activity than the R-configurated compound.

### Example 255

### (6-Chloro-pyrazolo[1,5-a]pyridin-2-yl)-[6-(6-fluoro-pyridin-3-yl)-1-methyl-3,4-dihydro-1 H-isoquinolin-2-yl]-methanone

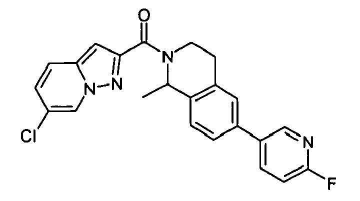

In close analogy to the procedure described in Example 1, 6-chloro-pyrazolo[1,5-a]pyridine-2-carboxylic acid is reacted with 6-(6-fluoro-pyridin-3-yl)-l-methyl-1,2,3,4-tetrahydro-isoquinoline to provide the title compound in moderate yield.
LC/MS: m/z = 421 (MH⁺)
The stereo-isomers of this compound are separated. The S-configurated compound has a different activity than the R-configurated compound.

### Example 256

### (6-Bromo-pyrazolo[1,5-a]pyridin-2-yl)-[6-(2,6-dimethoxy-pyridin-3-yl)-1-methyl-3,4-dihydro-1H-isoquinolin-2-yl]-methanone

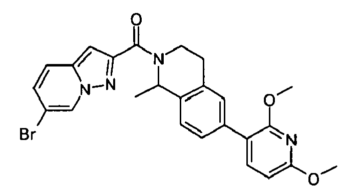

In close analogy to the procedure described in Example 1, 6-bromo-pyrazolo[1,5-a]pyridine-2-carboxylic acid is reacted with 6-(2,6-dimethoxy-pyridin-3-yl)-l-methyl-1,2,3,4-tetrahydro-isoquinoline to provide the title compound in moderate yield.
LC/MS: m/z = 508 (MH⁺)
The stereo-isomers of this compound are separated. The S-configurated compound has a different activity than the R-configurated compound.

### Example 257

### (6-Chloro-pyrazolo[1,5-a]pyridin-2-yl)-[6-(2,6-dimethoxy-pyridin-3-yl)-1-methyl-3,4-dihydro-1H-isoquinolin-2-yl]-methanone

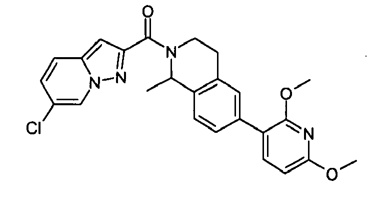

In close analogy to the procedure described in Example 1, 6-chloro-pyrazolo[1,5-a]pyridine-2-carboxylic acid is reacted with 6-(2,6-dimethoxy-pyridin-3-yl)-1-methyl-1,2,3,4-tetrahydro-isoquinoline to provide the title compound in moderate yield.
LC/MS: m/z = 463 (MH⁺)
The stereo-isomers of this compound are separated. The S-configurated compound has a different activity than the R-configurated compound.

### Example 258

### (6-Bromo-pyrazolo[1,5-a]pyridin-2-yl)-[6-(2,4-dimethoxy-pyrimidin-5-yl)-1-methyl-3,4-dihydro-1H-isoquinolin-2-yl]-methanone

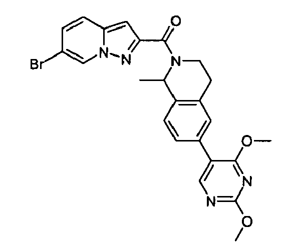

In close analogy to the procedure described in Example 1, 6-bromo-pyrazolo[1,5-a]pyridine-2-carboxylic acid is reacted with 6-(2,4-dimethoxy-pyrimidin-5-yl)-1-methyl-1,2,3,4-tetrahydro-isoquinoline to provide the title compound in moderate yield.
LC/MS: m/z = 508 (MH⁺)
The stereo-isomers of this compound are separated. The S-configurated compound has a different activity than the R-configurated compound.

### Example 259

### (6-Chloro-pyrazolo[1,5-a]pyridin-2-yl)-[6-(2,4-dimethoxy-pyrimidin-5-yl)-1-methyl-3,4-dihydro-1H-isoquinolin-2-yl]-methanone

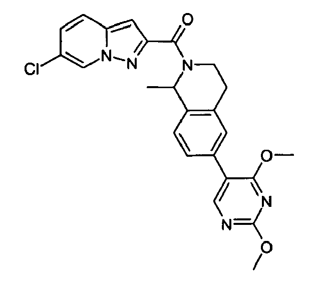

In close analogy to the procedure described in Example 1, 6-chloro-pyrazolo[1,5-a]pyridine-2-carboxylic acid is reacted with 6-(2,4-dimethoxy-pyrimidin-5-yl)-1-methyl-1,2,3,4-tetrahydro-isoquinoline to provide the title compound in moderate yield.
LC/MS: m/z = 463 (MH⁺)
The stereo-isomers of this compound are separated. The S-configurated compound has a different activity than the R-configurated compound.

### Example 260

### (6-Chloro-pyrazolo[1,5-a]pyridin-2-yl)-[7-(6-methoxy-pyridin-3-yl)-1-methyl-3,4-dihydro-1 H-isoquinolin-2-yl]-methanone

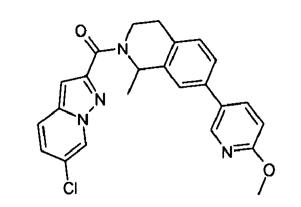

In close analogy to the procedure described in Example 1, 6-chloro-pyrazolo[1,5-a]pyridine-2-carboxylic acid is reacted with 7-(6-methoxy-pyridin-3-yl)-1-methyl-1,2,3,4-tetrahydro-isoquinoline to provide the title compound in moderate yield.
LC/MS: m/z = 433 (MH⁺)
The stereo-isomers of this compound are separated. The S-configurated compound has a different activity than the R-configurated compound.

### Example 261

### (6-Chloro-pyrazolo[1,5-a]pyridin-2-yl)-[7-(2-methoxy-pyridin-3-yl)-1-methyl-3,4-dihydro-1H-isoquinolin-2-yl]-methanone

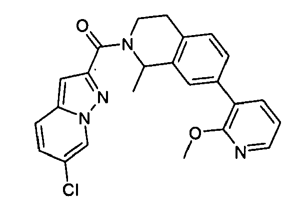

In close analogy to the procedure described in Example 1, 6-chloro-pyrazolo[1,5-a]pyridine-2-carboxylic acid is reacted with 7-(2-methoxy-pyridin-3-yl)-1-methyl-1,2,3,4-tetrahydro-isoquinoline to provide the title compound in moderate yield.
LC/MS: m/z = 433 (MH⁺)
The stereo-isomers of this compound are separated. The S-configurated compound has a different activity than the R-configurated compound.

### Example 262

### (6-Bromo-pyrazolo[1,5-a]pyridin-2-yl)-[7-(2,4-dimethoxy-pyrimidin-5-yl)-1-methyl-3,4-dihydro-1H-isoquinolin-2-yl]-methanone

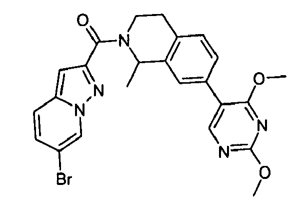

In close analogy to the procedure described in Example 1, 6-bromo-pyrazolo[1,5-a]pyridine-2-carboxylic acid is reacted with 7-(2,4-dimethoxy-pyrimidin-5-yl)-1-methyl-1,2,3,4-tetrahydro-isoquinoline to provide the title compound in moderate yield.
LC/MS: m/z = 509 (MH⁺)
The stereo-isomers of this compound are separated. The S-configurated compound has a different activity than the R-configurated compound.

### Example 263

### (6-Chloro-pyrazolo[1,5-a]pyridin-2-yl)-[7-(2,4-dimethoxy-pyrimidin-5-yl)-1-methyl-3,4-dihydro-1H-isoquinolin-2-yl]-methanone

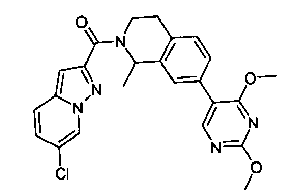

In close analogy to the procedure described in Example 1, 6-chloro-pyrazolo[1,5-a]pyridine-2-carboxylic acid is reacted with 7-(2,4-dimethoxy-pyrimidin-5-yl)-1-methyl-1,2,3,4-tetrahydro-isoquinoline to provide the title compound in moderate yield.
LC/MS: m/z = 464 (MH⁺)
The stereo-isomers of this compound are separated. The S-configurated compound has a different activity than the R-configurated compound.

### Example 264

### (6-Bromo-pyrazolo[1,5-a]pyridin-2-yl)-[7-(2-fluoro-pyridin-3-yl)-1-methyl-3,4-dihydro-1H-isoquinolin-2-yl]-methanone

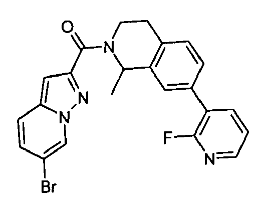

In close analogy to the procedure described in Example 1, 6-bromo-pyrazolo[1,5-a]pyridine-2-carboxylic acid is reacted with 7-(2-fluoro-pyridin-3-yl)-1-methyl-1,2,3,4-tetrahydro-isoquinoline to provide the title compound in moderate yield.
LC/MS: m/z = 466 (MH⁺)
The stereo-isomers of this compound are separated. The S-configurated compound has a different activity than the R-configurated compound.

### Example 265

### (6-Chloro-pyrazolo[1,5-a]pyridin-2-yl)-[7-(2-fluoro-pyridin-3-yl)-1-methyl-3,4-dihydro-1H-isoquinolin-2-yl]-methanone

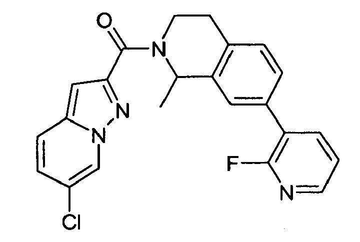

In close analogy to the procedure described in Example 1, 6-chloro-pyrazolo[1,5-a]pyridine-2-carboxylic acid is reacted with 7-(2-fluoro-pyridin-3-yl)-1-methyl-1,2,3,4-tetrahydro-isoquinoline to provide the title compound in moderate yield.
LC/MS: m/z = 421 (MH⁺)
The stereo-isomers of this compound are separated. The S-configurated compound has a different activity than the R-configurated compound.

### Example 266

### (6-Bromo-pyrazolo[1,5-a]pyridin-2-yl)-[5-(2-fluoro-pyridin-3-yl)-1-methyl-3,4-dihydro-1 H-isoquinolin-2-yl]-methanone

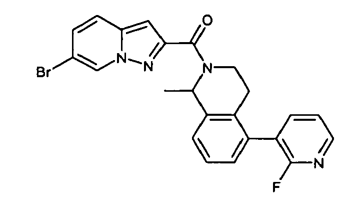

In close analogy to the procedure described in Example 1, 6-bromo-pyrazolo[1,5-a]pyridine-2-carboxylic acid is reacted with 5-(2-fluoro-pyridin-3-yl)-1-methyl-1,2,3,4-tetrahydro-isoquinoline to provide the title compound in moderate yield.
LC/MS: m/z = 466 (MH⁺)
The stereo-isomers of this compound are separated. The S-configurated compound has a different activity than the R-configurated compound.

### Example 267

### (6-Chloro-pyrazolo[1,5-a]pyridin-2-yl)-[5-(2-fluoro-pyridin-3-yl)-1-methyl-3,4-dihydro-1 H-isoquinolin-2-yl]-methanone

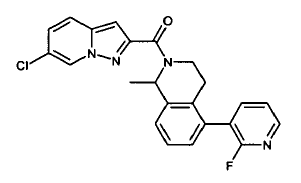

In close analogy to the procedure described in Example 1, 6-chloro-pyrazolo[1,5-a]pyridine-2-carboxylic acid is reacted with 5-(2-fluoro-pyridin-3-yl)-1-methyl-1,2,3,4-tetrahydro-isoquinoline to provide the title compound in moderate yield.
LC/MS: m/z = 421 (MH⁺)
The stereo-isomers of this compound are separated. The S-configurated compound has a different activity than the R-configurated compound.

### Example 268

### (6-Bromo-pyrazolo[1,5-a]pyridin-2-yl)-(8-furan-2-yl-1-methyl-3,4-dihydro-1H-isoquinolin-2-yl)-methanone

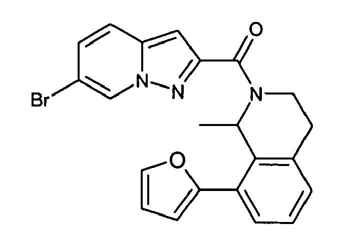

In close analogy to the procedure described in Example 1, 6-bromo-pyrazolo[1,5-a]pyridine-2-carboxylic acid is reacted with 8-furan-2-yl-1-methyl-1,2,3,4-tetrahydro-isoquinoline to provide the title compound in moderate yield.
LC/MS: m/z = 437 (MH⁺)
The stereo-isomers of this compound are separated. The S-configurated compound has a different activity than the R-configurated compound.

### Example 269

### (6-Chloro-pyrazolo[1,5-a]pyridin-2-yl)-(8-furan-2-yl-1-methyl-3,4-dihydro-1H-isoquinolin-2-yl)-methanone is synthesized in a similar way.

The stereo-isomers of this compound are separated. The S-configurated compound has a different activity than the R-configurated compound.

### Example 270

### (6-Bromo-pyrazolo[1,5-a]pyridin-2-yl)-(7-furan-2-yl-1-methyl-3,4-dihydro-1H-isoquinolin-2-yl)-methanone

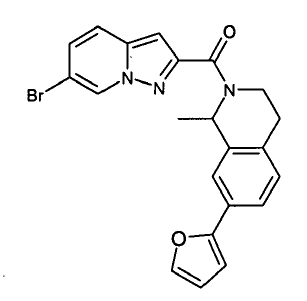

In close analogy to the procedure described in Example 1, 6-bromo-pyrazolo[1,5-a]pyridine-2-carboxylic acid is reacted with 7-furan-2-yl-1-methyl-1,2,3,4-tetrahydro-isoquinoline to provide the title compound in moderate yield.
LC/MS: m/z = 437 (MH⁺)
The stereo-isomers of this compound are separated. The S-configurated compound has a different activity than the R-configurated compound.

### Example 271

### (6-Chloro-pyrazolo[1,5-a]pyridin-2-yl)-(7-furan-2-yl-1-methyl-3,4-dihydro-1H-isoquinolin-2-yl)-methanone is synthesized in a similar way.

The stereo-isomers of this compound are separated. The S-configurated compound has a different activity than the R-configurated compound.

### Example 272

### (6-Bromo-pyrazolo[1,5-a]pyridin-2-yl)-(6-furan-2-yl-1-methyl-3,4-dihydro-1H-isoquinolin-2-yl)-methanone

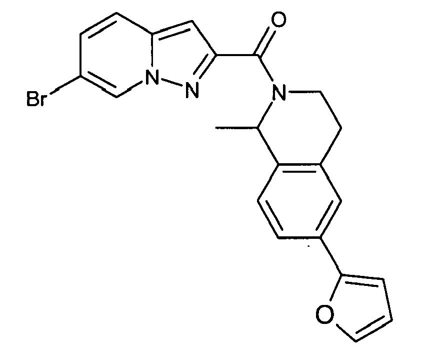

In close analogy to the procedure described in Example 1, 6-bromo-pyrazolo[1,5-a]pyridine-2-carboxylic acid is reacted with 6-furan-2-yl-1-methyl-1,2,3,4-tetrahydro-isoquinoline to provide the title compound in moderate yield.
LC/MS: m/z = 437 (MH⁺)
The stereo-isomers of this compound are separated. The S-configurated compound has a different activity than the R-configurated compound.

### Example 273

### (6-Chloro-pyrazolo[1,5-a]pyridin-2-yl)-(6-furan-2-yl-1-methyl-3,4-dihydro-1H-isoquinolin-2-yl)-methanone is synthesized in a similar way.

The stereo-isomers of this compound are separated. The S-configurated compound has a different activity than the R-configurated compound.

### Example 274

### (6-Bromo-pyrazolo[1,5-a]pyridin-2-yl)-(5-furan-2-yl-1-methyl-3,4-dihydro-1H-isoquinolin-2-yl)-methanone

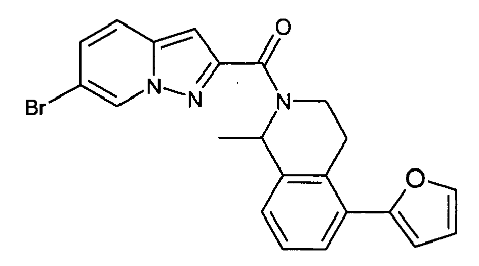

In close analogy to the procedure described in Example 1, 6-bromo-pyrazolo[1,5-a]pyridine-2-carboxylic acid is reacted with 5-furan-2-yl-1-methyl-1,2,3,4-tetrahydro-isoquinoline to provide the title compound in moderate yield.
LC/MS: m/z = 437 (MH⁺)
The stereo-isomers of this compound are separated. The S-configurated compound has a different activity than the R-configurated compound.

### Example 275

### (6-Chloro-pyrazolo[1,5-a]pyridin-2-yl)-(5-furan-2-yl-1-methyl-3,4-dihydro-1H-isoquinolin-2-yl)-methanone is synthesized in a similar way.

The stereo-isomers of this compound are separated. The S-configurated compound has a different activity than the R-configurated compound.

### Example 276

### (6-Bromo-pyrazolo[1,5-a]pyridin-2-yl)-[1-methyl-8-(5-methyl-furan-2-yl)-3,4-dihydro-1H-isoquinolin-2-yl]-methanone

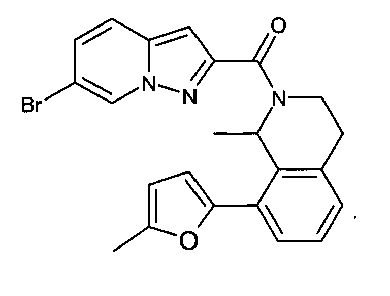

In close analogy to the procedure described in Example 1, 6-bromo-pyrazolo[1,5-a]pyridine-2-carboxylic acid is reacted with 1-methyl-8-(5-methyl-furan-2-yl)-1,2,3,4-tetrahydro-isoquinoline to provide the title compound in moderate yield.
LC/MS: m/z = 451 (MH⁺)
The stereo-isomers of this compound are separated. The S-configurated compound has a different activity than the R-configurated compound.

### Example 277

### (6-Chloro-pyrazolo[1,5-a]pyridin-2-yl)-[1-methyl-8-(5-methyl-furan-2-yl)-3,4-dihydro-1H-isoquinolin-2-yl]-methanone is synthesized in a similar way.

The stereo-isomers of this compound are separated. The S-configurated compound has a different activity than the R-configurated compound.

### Example 278

### (6-Bromo-pyrazolo[1,5-a]pyridin-2-yl)-[1-methyl-7-(5-methyl-furan-2-yl)-3,4-dihydro-1H-isoquinolin-2-yl]-methanone

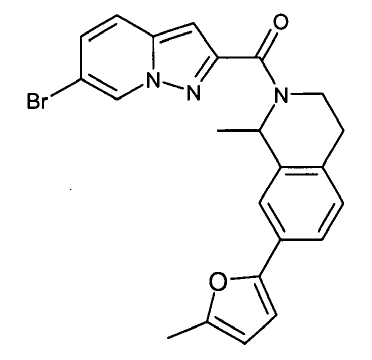

In close analogy to the procedure described in Example 1, 6-bromo-pyrazolo[1,5-a]pyridine-2-carboxylic acid is reacted with 1-methyl-7-(5-methyl-furan-2-yl)-1,2,3,4-tetrahydro-isoquinoline to provide the title compound in moderate yield.
LC/MS: m/z = 451 (MH⁺)
The stereo-isomers of this compound are separated. The S-configurated compound has a different activity than the R-configurated compound.

### Example 279

### (6-Chloro-pyrazolo[1,5-a]pyridin-2-yl)-[1-methyl-7-(5-methyl-furan-2-yl)-3,4-dihydro-1H-isoquinolin-2-yl]-methanone is synthesized in a similar way.

The stereo-isomers of this compound are separated. The S-configurated compound has a different activity than the R-configurated compound.

### Example 280

### (6-Bromo-pyrazolo[1,5-a]pyridin-2-yl)-[1-methyl-6-(5-methyl-furan-2-yl)-3,4-dihydro-1H-isoquinolin-2-yl]-methanone

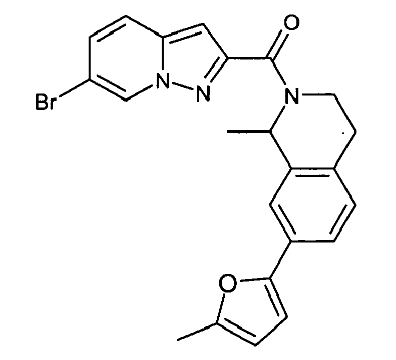

In close analogy to the procedure described in Example 1, 6-bromo-pyrazolo[1,5-a]pyridine-2-carboxylic acid is reacted with 1-methyl-6-(5-methyl-furan-2-yl)-1,2,3,4-tetrahydro-isoquinoline to provide the title compound in moderate yield.
LC/MS: m/z = 451 (MH⁺)
The stereo-isomers of this compound are separated. The S-configurated compound has a different activity than the R-configurated compound.

### Example 281

### (6-Chloro-pyrazolo[1,5-a]pyridin-2-yl)-[1-methyl-6-(5-methyl-furan-2-yl)-3,4-dihydro-1H-isoquinolin-2-yl]-methanone is synthesized in a similar way.

The stereo-isomers of this compound are separated. The S-configurated compound has a different activity than the R-configurated compound.

### Example 282

### (6-Bromo-pyrazolo[1,5-a]pyridin-2-yl)-[1-methyl-5-(5-methyl-furan-2-yl)-3,4-dihydro-1 H-isoquinolin-2-yl]-methanone

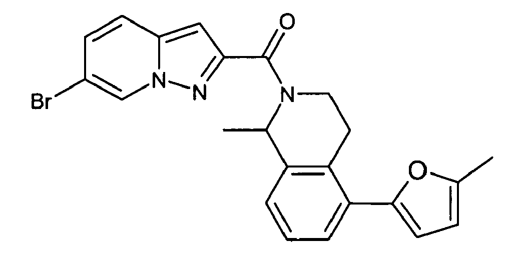

In close analogy to the procedure described in Example 1, 6-bromo-pyrazolo[1,5-a]pyridine-2-carboxylic acid is reacted with 1-methyl-5-(5-methyl-furan-2-yl)-1,2,3,4-tetrahydro-isoquinoline to provide the title compound in moderate yield.
LC/MS: m/z = 451 (MH⁺)
The stereo-isomers of this compound are separated. The S-configurated compound has a different activity than the R-configurated compound.

### Example 283

### (6-Chloro-pyrazolo[1,5-a]pyridin-2-yl)-[1-methyl-5-(5-methyl-furan-2-yl)-3,4-dihydro-1H-isoquinolin-2-yl]-methanone is synthesized in a similar way.

The stereo-isomers of this compound are separated. The S-configurated compound has a different activity than the R-configurated compound.

### Example 284

### (6-Bromo-pyrazolo[1,5-a]pyridin-2-yl)-(8-furan-3-yl-1-methyl-3,4-dihydro-1H-isoquinolin-2-yl)-methanone

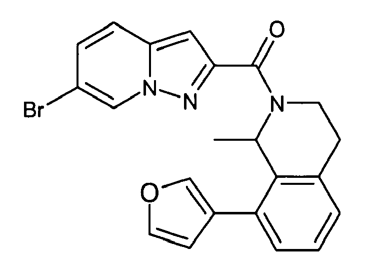

In close analogy to the procedure described in Example 1, 6-bromo-pyrazolo[1,5-a]pyridine-2-carboxylic acid is reacted with 8-furan-3-yl-1-methyl-1,2,3,4-tetrahydro-isoquinoline to provide the title compound in moderate yield.
LC/MS: m/z = 437 (MH⁺)
The stereo-isomers of this compound are separated. The S-configurated compound has a different activity than the R-configurated compound.

### Example 285

### (6-Chloro-pyrazolo[1,5-a]pyridin-2-yl)-(8-furan-3-yl-1-methyl-3,4-dihydro-1H-isoquinolin-2-yl)-methanone is synthesized in a similar way.

The stereo-isomers of this compound are separated. The S-configurated compound has a different activity than the R-configurated compound.

### Example 286

### (6-Bromo-pyrazolo[1,5-a]pyridin-2-yl)-(7-furan-3-yl-1-methyl-3,4-dihydro-1H-isoquinolin-2-yl)-methanone

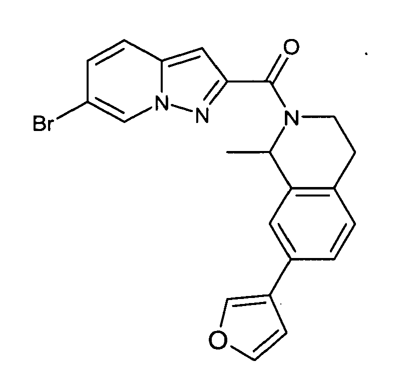

In close analogy to the procedure described in Example 1, 6-bromo-pyrazolo[1,5-a]pyridine-2-carboxylic acid is reacted with 7-furan-3-yl-1-methyl-1,2,3,4-tetrahydro-isoquinoline to provide the title compound in moderate yield.
LC/MS: m/z = 437 (MH⁺)
The stereo-isomers of this compound are separated. The S-configurated compound has a different activity than the R-configurated compound.

### Example 287

### (6-Chloro-pyrazolo[1,5-a]pyridin-2-yl)-(7-furan-3-yl-1-mdthyl-3,4-dihydro-1H-isoquinolin-2-yl)-methanone is synthesized in a similar way.

The stereo-isomers of this compound are separated. The S-configurated compound has a different activity than the R-configurated compound.

### Example 288

### (6-Bromo-pyrazolo[1,5-a]pyridin-2-yl)-(6-furan-3-yl-1-methyl-3,4-dihydro-1H-isoquinolin-2-yl)-methanone

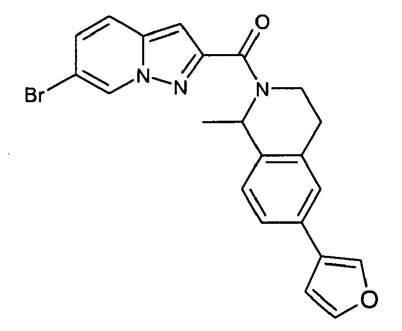

In close analogy to the procedure described in Example 1, 6-bromo-pyrazolo[1,5-a]pyridine-2-carboxylic acid is reacted with 6-furan-3-yl-1-methyl-1,2,3,4-tetrahydro-isoquinoline to provide the title compound in moderate yield.
LC/MS: m/z = 437 (MH⁺)
The stereo-isomers of this compound are separated. The S-configurated compound has a different activity than the R-configurated compound.

### Example 289

### (6-Chloro-pyrazolo[1,5-a]pyridin-2-yl)-(6-furan-3-yl-1-methyl-3,4-dihydro-1H-isoquinolin-2-yl)-methanone is synthesized in a similar way.

The stereo-isomers of this compound are separated. The S-configurated compound has a different activity than the R-configurated compound.

### Example 290

### (6-Bromo-pyrazolo[1,5-a]pyridin-2-yl)-(5-furan-3-yl-1-methyl-3,4-dihydro-1H-isoquinolin-2-yl)-methanone

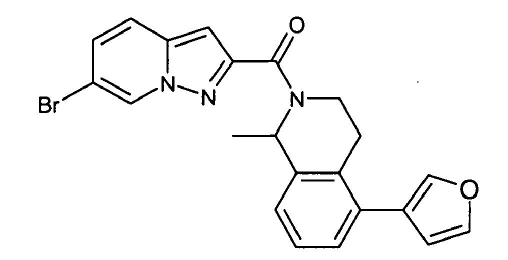

In close analogy to the procedure described in Example 1, 6-bromo-pyrazolo[1,5-a]pyridine-2-carboxylic acid is reacted with 5-furan-3-yl-1-methyl-1,2,3,4-tetrahydro-isoquinoline to provide the title compound in moderate yield.
LC/MS: m/z = 437 (MH⁺)
The stereo-isomers of this compound are separated. The S-configurated compound has a different activity than the R-configurated compound.

### Example 291

### (6-Chloro-pyrazolo[1,5-a]pyridin-2-yl)-(5-furan-3-yl-1-methyl-3,4-dihydro-1H-isoquinolin-2-yl)-methanone is synthesized in a similar way.

The stereo-isomers of this compound are separated. The S-configurated compound has a different activity than the R-configurated compound.

### Example 292

### (6-Bromo-pyrazolo[1,5-a]pyridin-2-yl)-[1-methyl-5-(1H-pyrazol-4-yl)-3,4-dihydro-1H-isoquinolin-2-yl]-methanone

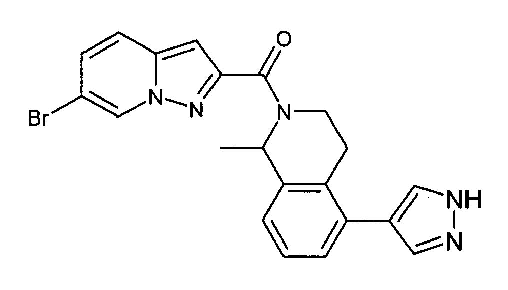

In close analogy to the procedure described in Example 1, 6-bromo-pyrazolo[1,5-a]pyridine-2-carboxylic acid is reacted with 1-methyl-5-(1H-pyrazol-4-yl)-1,2,3,4-tetrahydro-isoquinolineto provide the title compound in moderate yield.
LC/MS: m/z = 466 (MH⁺)
The stereo-isomers of this compound are separated. The S-configurated compound has a different activity than the R-configurated compound.

### Example 293

### (6-Chloro-pyrazolo[1,5-a]pyridin-2-yl)-[1-methyl-5-(1H-pyrazol-4-yl)-3,4-dihydro-1H-isoquinolin-2-yl]-methanone is synthesized in a similar way.

The stereo-isomers of this compound are separated. The S-configurated compound has a different activity than the R-configurated compound.

### Example 294

### (6-Bromo-pyrazolo[1,5-a]pyridin-2-yl)-[7-(3,5-dimethyl-isoxazol-4-yl)-1-methyl-3,4-dihydro-1H-isoquinolin-2-yl]-methanone

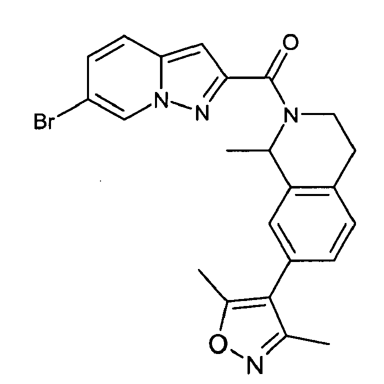

In close analogy to the procedure described in Example 1, 6-bromo-pyrazolo[1,5-a]pyridine-2-carboxylic acid is reacted with 7-(3,5-dimethyl-isoxazol-4-yl)-1-methyl-1,2,3,4-tetrahydro-isoquinoline to provide the title compound in moderate yield.
LC/MS: m/z = 466 (MH⁺)
The stereo-isomers of this compound are separated. The S-configurated compound has a different activity than the R-configurated compound.

### Example 295

### (6-Chloro-pyrazolo[1,5-a]pyridin-2-yl)-[7-(3,5-dimethyl-isoxazol-4-yl)-1-methyl-3,4-dihydro-1H-isoquinolin-2-yl]-methanone is synthesized in a similar way.

The stereo-isomers of this compound are separated. The S-configurated compound has a different activity than the R-configurated compound.

### Example 296

### (6-Bromo-pyrazolo[1,5-a]pyridin-2-yl)-[6-(3,5-dimethyl-isoxazol-4-yl)-1-methyl-3,4-dihydro-1H-isoquinolin-2-yl]-methanone

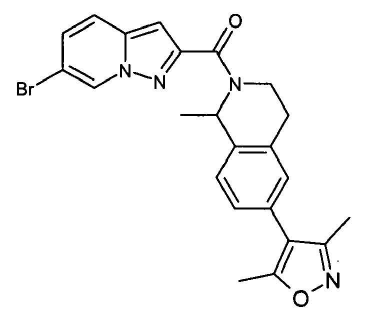

In close analogy to the procedure described in Example 1, 6-bromo-pyrazolo[1,5-a]pyridine-2-carboxylic acid is reacted with 6-(3,5-dimethyl-isoxazol-4-yl)-1-methyl-1,2,3,4-tetrahydro-isoquinoline to provide the title compound in moderate yield.
LC/MS: m/z = 466 (MH⁺)
The stereo-isomers of this compound are separated. The S-configurated compound has a different activity than the R-configurated compound.

### Example 297

### (6-Chloro-pyrazolo[1,5-a]pyridin-2-yl)-[6-(3,5-dimethyl-isoxazol-4-yl)-1-methyl-3,4-dihydro-1H-isoquinolin-2-yl]-methanone is synthesized in a similar way.

The stereo-isomers of this compound are separated. The S-configurated compound has a different activity than the R-configurated compound.

### Example 298

### (6-Bromo-pyrazolo[1,5-a]pyridin-2-yl)-[5-(3,5-dimethyl-isoxazol-4-yl)-1-methyl-3,4-dihydro-1H-isoquinolin-2-yl]-methanone

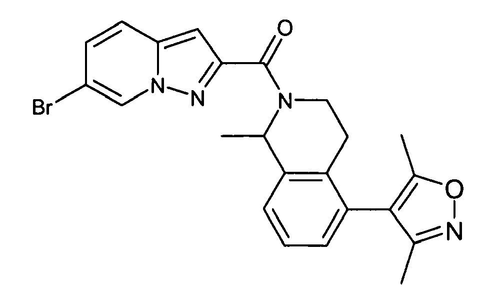

In close analogy to the procedure described in Example 1, 6-bromo-pyrazolo[1,5-a]pyridine-2-carboxylic acid is reacted with 5-(3,5-dimethyl-isoxazol-4-yl)-1-methyl-1,2,3,4-tetrahydro-isoquinoline to provide the title compound in moderate yield.
LC/MS: m/z = 466 (MH⁺)
The stereo-isomers of this compound are separated. The S-configurated compound has a different activity than the R-configurated compound.

### Example 299

### (6-Chloro-pyrazolo[1,5-a]pyridin-2-yl)-[5-(3,5-dimethyl-isoxazol-4-yl)-1-methyl-3,4-dihydro-1H-isoquinolin-2-yl]-methanone is synthesized in a similar way.

The stereo-isomers of this compound are separated. The S-configurated compound has a different activity than the R-configurated compound.

### Example 300

### (6-Bromo-pyrazolo[1,5-a]pyridin-2-yl)-[1-methyl-8-(1H-pyrazol-4-yl)-3,4-dihydro-1H-isoquinolin-2-yl]-methanone

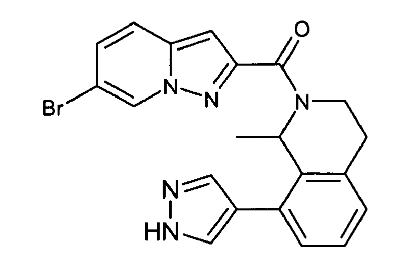

In close analogy to the procedure described in Example 1, 6-bromo-pyrazolo[1,5-a]pyridine-2-carboxylic acid is reacted with 1-methyl-8-(1H-pyrazol-4-yl)-1,2,3,4-tetrahydro-isoquinoline to provide the title compound in moderate yield.
LC/MS: m/z = 437 (MH⁺)
The stereo-isomers of this compound are separated. The S-configurated compound has a different activity than the R-configurated compound.

### Example 301

### (6-Chloro-pyrazolo[1,5-a]pyridin-2-yl)-[1-methyl-8-(1H-pyrazol-4-yl)-3,4-dihydro-1H-isoquinolin-2-yl]-methanone is synthesized in a similar way.

The stereo-isomers of this compound are separated. The S-configurated compound has a different activity than the R-configurated compound.

### Example 302

### (6-Bromo-pyrazolo[1,5-a]pyridin-2-yl)-[1-methyl-7-(1H-pyrazol-4-yl)-3,4-dihydro-1H-isoquinolin-2-yl]-methanone

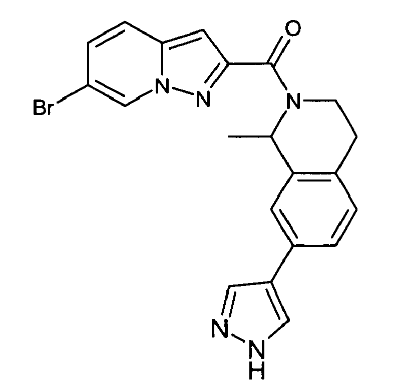

In close analogy to the procedure described in Example 1, 6-bromo-pyrazolo[1,5-a]pyridine-2-carboxylic acid is reacted with 1-methyl-7-(IH-pyrazol-4-yl)-1,2,3,4-tetrahydro-isoquinoline to provide the title compound in moderate yield.
LC/MS: m/z = 437 (MH⁺)
The stereo-isomers of this compound are separated. The S-configurated compound has a different activity than the R-configurated compound.

### Example 303

### (6-Chloro-pyrazolo[1,5-a]pyridin-2-yl)-[1-methyl-7-(1H-pyrazol-4-yl)-3,4-dihydro-1H-isoquinolin-2-yl]-methanone is synthesized in a similar way.

The stereo-isomers of this compound are separated. The S-configurated compound has a different activity than the R-configurated compound.

### Example 304

### (6-Bromo-pyrazolo[1,5-a]pyridin-2-yl)-[1-methyl-6-(1H-pyrazol-4-yl)-3,4-dihydro-1H-isoquinolin-2-yl]-methanone

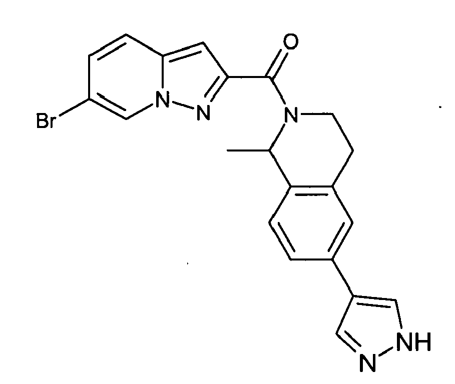

In close analogy to the procedure described in Example 1, 6-bromo-pyrazolo[1,5-a]pyridine-2-carboxylic acid is reacted with 1-methyl-6-(1H-pyrazol-4-yl)-1,2,3,4-tetrahydro-isoquinoline to provide the title compound in moderate yield.
LC/MS: m/z = 437 (MH⁺)
The stereo-isomers of this compound are separated. The S-configurated compound has a different activity than the R-configurated compound.

### Example 305

### (6-Chloro-pyrazolo[1,5-a]pyridin-2-yl)-[1-methyl-6-(1H-pyrazol-4-yl)-3,4-dihydro-1H-isoquinolin-2-yl]-methanone is synthesized in a similar way.

The stereo-isomers of this compound are separated. The S-configurated compound has a different activity than the R-configurated compound.

### Example 306

### 6-Bromo-pyrazolo[1,5-a]pyridine-2-carboxylic acid phenethyl-amide

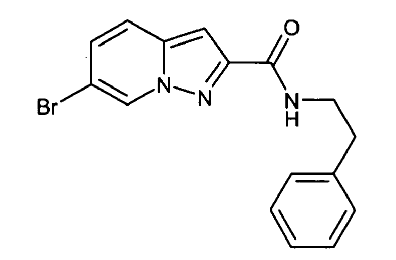

In close analogy to the procedure described in Example 1, 6-bromo-pyrazolo[1,5-a]pyridine-2-carboxylic acid is reacted with phenethylamine to provide the title compound in moderate yield.
LC/MS: m/z = 345 (MH⁺)

### Example 307

### 6-Chloro-pyrazolo[1,5-a]pyridine-2-carboxylic acid phenethyl-amide

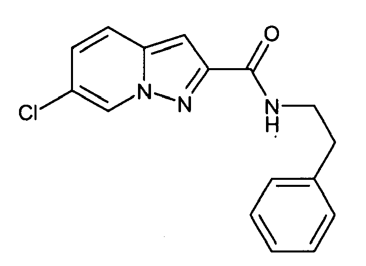

In close analogy to the procedure described in Example 1, 6-chloro-pyrazolo[1,5-a]pyridine-2-carboxylic acid is reacted with phenethylamine to provide the title compound in moderate yield.
LC/MS: m/z = 300 (MH⁺)

### Example 308

### (6-Bromo-pyrazolo[1,5-a]pyridin-2-yl)-[1-methyl-8-(1-methyl-1H-pyrazol-4-yl)-3,4-dihydro-1H-isoquinolin-2-yl]-methanone

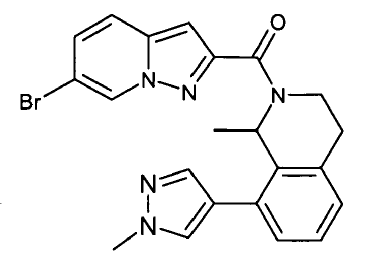

In close analogy to the procedure described in Example 1, 6-bromo-pyrazolo[1,5-a]pyridine-2-carboxylic acid is reacted with 1-methyl-8-(1-methyl-1H-pyrazol-4-yl)-1,2,3,4-tetrahydro-isoquinoline to provide the title compound in moderate yield.
LC/MS: m/z = 451 (MH⁺)
The stereo-isomers of this compound are separated. The S-configurated compound has a different activity than the R-configurated compound.

### Example 309

### (6-Chloro-pyrazolo[1,5-a]pyridin-2-yl)-[1-methyl-8-(1-methyl-1H-pyrazol-4-yl)-3,4-dihydro-1H-isoquinolin-2-yl]-methanone is synthesized in a similar way.

The stereo-isomers of this compound are separated. The S-configurated compound has a different activity than the R-configurated compound.

### Example 310

### (6-Bromo-pyrazolo[1,5-a]pyridin-2-yl)-[1-methyl-7-(1-methyl-1H-pyrazol-4-yl)-3,4-dihydro-1H-isoquinolin-2-yl]-methanone

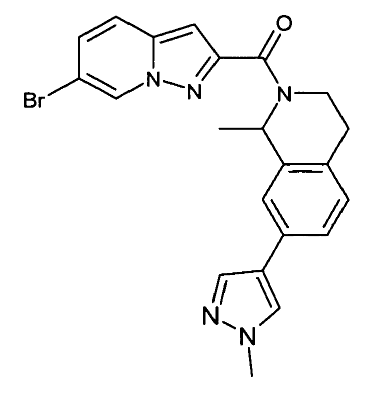

In close analogy to the procedure described in Example 1, 6-bromo-pyrazolo[1,5-a]pyridine-2-carboxylic acid is reacted with 1-methyl-7-(1-methyl-1H-pyrazol-4-yl)-1,2,3,4-tetrahydro-isoquinoline to provide the title compound in moderate yield.
LC/MS: m/z = 451 (MH⁺)
The stereo-isomers of this compound are separated. The S-configurated compound has a different activity than the R-configurated compound.

### Example 311

### (6-Chloro-pyrazolo[1,5-a]pyridin-2-yl)-[1-methyl-7-(-methyl-1H-pyrazol-4-yl)-3,4-dihydro-1H-isoquinolin-2-yl]-methanone is synthesized in a similar way.

The stereo-isomers of this compound are separated. The S-configurated compound has a different activity than the R-configurated compound.

### Example 312

### (6-Bromo-pyrazolo[1,5-a]pyridin-2-yl)-[1-methyl-6-(1-methyl-1H-pyrazol-4-yl)-3,4-dihydro-1H-isoquinolin-2-yl]-methanone

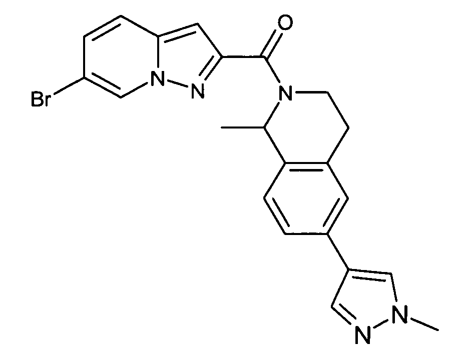

In close analogy to the procedure described in Example 1, 6-bromo-pyrazolo[1,5-a]pyridine-2-carboxylic acid is reacted with 1-methyl-6-(1-methyl-1H-pyrazol-4-yl)-1,2,3,4-tetrahydro-isoquinoline to provide the title compound in moderate yield.
LC/MS: m/z = 451 (MH⁺)
The stereo-isomers of this compound are separated. The S-configurated compound has a different activity than the R-configurated compound.

### Example 313

### (6-Chloro-pyrazolo[1,5-a]pyridin-2-yl)-[1-methyl-6-(1-methyl-1H-pyrazol-4-yl)-3,4-dihydro-1H-isoquinolin-2-yl]-methanone is synthesized in a similar way.

The stereo-isomers of this compound are separated. The S-configurated compound has a different activity than the R-configurated compound.

### Example 314

### (6-Bromo-pyrazolo[1,5-a]pyridin-2-yl)-[1-methyl-5-(1-methyl-1H-pyrazol-4-yl)-3,4-dihydro-1H-isoquinolin-2-yl]-methanone

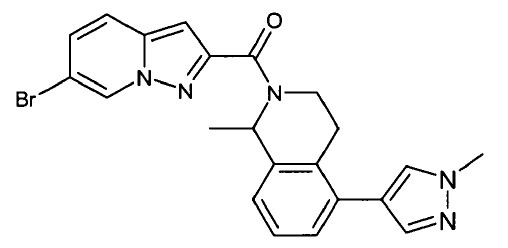

In close analogy to the procedure described in Example 1, 6-bromo-pyrazolo[1,5-a]pyridine-2-carboxylic acid is reacted with 1-methyl-5-(1-methyl-1H-pyrazol-4-yl)-1,2,3,4-tetrahydro-isoquinoline to provide the title compound in moderate yield.
LC/MS: m/z = 451 (MH⁺)
The stereo-isomers of this compound are separated. The S-configurated compound has a different activity than the R-configurated compound.

### Example 315

### (6-Chloro-pyrazolo[1,5-a]pyridin-2-yl)-[1-methyl-5-(1-methyl-1H-pyrazol-4-yl)-3,4-dihydro-1H-isoquinolin-2-yl]-methanone is synthesized in a similar way.

The stereo-isomers of this compound are separated. The S-configurated compound has a different activity than the R-configurated compound.

### Example 316

### (6-Bromo-pyrazolo[1,5-a]pyridin-2-yl)-[1-methyl-8-(3-methyl-1H-pyrazol-4-yl)-3,4-dihydro-1H-isoquinolin-2-yl]-methanone

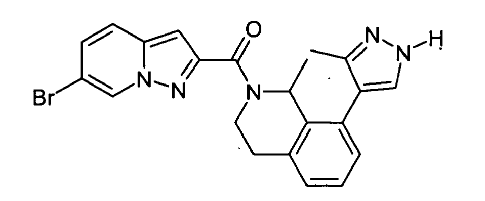

In close analogy to the procedure described in Example 1, 6-bromo-pyrazolo[1,5-a]pyridine-2-carboxylic acid is reacted with 1-methyl-8-(3-methyl-1H-pyrazol-4-yl)-1,2,3,4-tetrahydro-isoquinoline to provide the title compound in moderate yield.
LC/MS: m/z = 451 (MH⁺)
The stereo-isomers of this compound are separated. The S-configurated compound has a different activity than the R-configurated compound.

### Example 317

### (6-Chloro-pyrazolo[1,5-a]pyridin-2-yl)-[1-methyl-8-(3-methyl-1H-pyrazol-4-yl)-3,4-dihydro-1H-isoquinolin-2-yl]-methanone is synthesized in a similar way.

The stereo-isomers of this compound are separated. The S-configurated compound has a different activity than the R-configurated compound.

### Example 318

### (6-Bromo-pyrazolo[1,5-a]pyridin-2-yl)-[1-methyl-7-(3-methyl-1H-pyrazol-4-yl)-3,4-dihydro-1H-isoquinolin-2-yl]-methanone

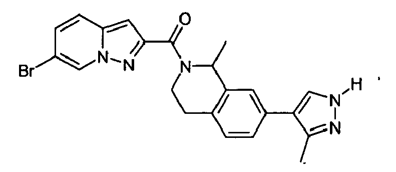

In close analogy to the procedure described in Example 1, 6-bromo-pyrazolo[1,5-a]pyridine-2-carboxylic acid is reacted with 1-methyl-7-(3-methyl-1H-pyrazol-4-yl)-1,2,3,4-tetrahydro-isoquinoline to provide the title compound in moderate yield.
LC/MS: m/z = 451 (MH⁺)
The stereo-isomers of this compound are separated. The S-configurated compound has a different activity than the R-configurated compound.

### Example 319

### (6-Chloro-pyrazolo[1,5-a]pyridin-2-yl)-[1-methyl-7-(3-methyl-1H-pyrazol-4-yl)-3,4-dihydro-1H-isoquinolin-2-yl]-methanone is synthesized in a similar way.

The stereo-isomers of this compound are separated. The S-configurated compound has a different activity than the R-configurated compound.

### Example 320

### (6-Bromo-pyrazolo[1,5-a]pyridin-2-yl)-[1-methyl-6-(3-methyl-1H-pyrazol-4-yl)-3,4-dihydro-1H-isoquinolin-2-yl]-methanone

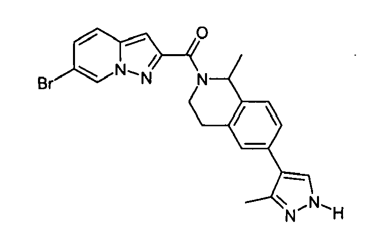

In close analogy to the procedure described in Example 1, 6-bromo-pyrazolo[1,5-a]pyridine-2-carboxylic acid is reacted with 1-methyl-6-(3-methyl-1H-pyrazol-4-yl)-1,2,3,4-tetrahydro-isoquinoline to provide the title compound in moderate yield.
LC/MS: m/z = 451 (MH⁺)
The stereo-isomers of this compound are separated. The S-configurated compound has a different activity than the R-configurated compound.

### Example 321

### (6-Chloro-pyrazolo[1,5-a]pyridin-2-yl)-[1-methyl-6-(3-methyl-1H-pyrazol-4-yl)-3,4-dihydro-1H-isoquinolin-2-yl]-methanone is synthesized in a similar way.

The stereo-isomers of this compound are separated. The S-configurated compound has a different activity than the R-configurated compound.

### Example 322

### (6-Bromo-pyrazolo[1,5-a]pyridin-2-yl)-[1-methyl-5-(3-methyl-1H-pyrazol-4-yl)-3,4-dihydro-1H-isoquinolin-2-yl]-methanone

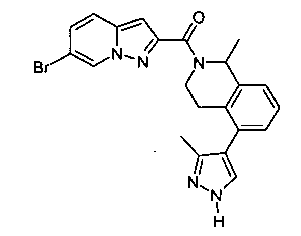

In close analogy to the procedure described in Example 1, 6-bromo-pyrazolo[1,5-a]pyridine-2-carboxylic acid is reacted with 1-methyl-5-(3-methyl-1H-pyrazol-4-yl)-1,2,3,4-tetrahydro-isoquinoline to provide the title compound in moderate yield.
LC/MS: m/z = 451 (MH⁺)
The stereo-isomers of this compound are separated. The S-configurated compound has a different activity than the R-configurated compound.

### Example 323

### (6-Chloro-pyrazolo[1,5-a]pyridin-2-yl)-[1-methyl-5-(3-methyl-1H-pyrazol-4-yl)-3,4-dihydro-1H-isoquinolin-2-yl]-methanone is synthesized in a similar way.

The stereo-isomers of this compound are separated. The S-configurated compound has a different activity than the R-configurated compound.

### Example 324

### (6-Bromo-pyrazolo[1,5-a]pyridin-2-yl)-[1-methyl-8-(2H-tetrazol-5-yl)-3,4-dihydro-1H-isoquinolin-2-yl]-methanone

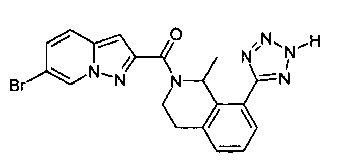

In close analogy to the procedure described in Example 1, 6-bromo-pyrazolo[1,5-a]pyridine-2-carboxylic acid is reacted with 1-methyl-8-(2H-tetrazol-5-yl)-1,2,3,4-tetrahydro-isoquinoline to provide the title compound in moderate yield.
LC/MS: m/z = 439 (MH⁺)
The stereo-isomers of this compound are separated. The S-configurated compound has a different activity than the R-configurated compound.

### Example 325

### (6-Chloro-pyrazolo[1,5-a]pyridin-2-yl)-[1-methyl-8-(2H-tetrazol-5-yl)-3,4-dihydro-1H-isoquinolin-2-yl]-methanone is synthesized in a similar way.

The stereo-isomers of this compound are separated. The S-configurated compound has a different activity than the R-configurated compound.

### Example 326

### (6-Bromo-pyrazolo[1,5-a]pyridin-2-yl)-[I-methyl-7-(2H-tetrazol-5-yl)-3,4-dihydro-1H-isoquinolin-2-yl]-methanone

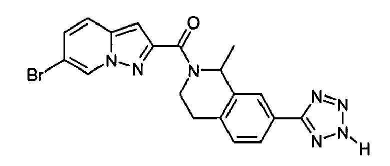

In close analogy to the procedure described in Example 1, 6-bromo-pyrazolo[1,5-a]pyridine-2-carboxylic acid is reacted with 1-methyl-7-(2H-tetrazol-5-yl)-1,2,3,4-tetrahydro-isoquinoline to provide the title compound in moderate yield.
LC/MS: m/z = 439 (MH⁺)
The stereo-isomers of this compound are separated. The S-configurated compound has a different activity than the R-configurated compound.

### Example 327

### (6-Chloro-pyrazolo[1,5-a]pyridin-2-yl)-[1-methyl-7-(2H-tetrazol-5-yl)-3,4-dihydro-1H-isoquinolin-2-yl]-methanone is synthesized in a similar way.

The stereo-isomers of this compound are separated. The S-configurated compound has a different activity than the R-configurated compound.

### Example 328

### (6-Bromo-pyrazolo[1,5-a]pyridin-2-yl)-[1-methyl-6-(2H-tetrazol-5-yl)-3,4-dihydro-1H-isoquinolin-2-yl]-methanone

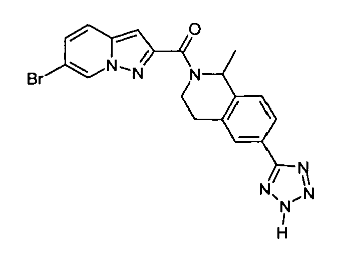

In close analogy to the procedure described in Example 1, 6-bromo-pyrazolo[1,5-a]pyridine-2-carboxylic acid is reacted with 1-methyl-6-(2H-tetrazol-5-yl)-1,2,3,4-tetrahydro-isoquinoline to provide the title compound in moderate yield.
LC/MS: m/z = 439 (MH⁺)
The stereo-isomers of this compound are separated. The S-configurated compound has a different activity than the R-configurated compound.

### Example 329

### (6-Chloro-pyrazolo[1,5-a]pyridin-2-yl)-[1-methyl-6-(2H-tetrazol-5-yl)-3,4-dihydro-1H-isoquinolin-2-yl]-methanone is synthesized in a similar way.

The stereo-isomers of this compound are separated. The S-configurated compound has a different activity than the R-configurated compound.

### Example 330

### (6-Bromo-pyrazolo[1,5-a]pyridin-2-yl)-(I-methyl-5-nitro-3,4-dihydro-IH-2,7-naphthyridin-2-yl)-methanone

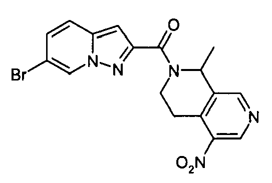

In close analogy to the procedure described in Example 1, 6-bromo-pyrazolo[1,5-a]pyridine-2-carboxylic acid is reacted with 1-methyl-5-nitro-1,2,3,4-tetrahydro-2,7-naphthyridineto provide the title compound in moderate yield.
LC/MS: m/z = 439 (MH⁺)
The stereo-isomers of this compound are separated. The S-configurated compound has a different activity than the R-configurated compound.

### Example 331

### (6-Chloro-pyrazolo[1,5-a]pyridin-2-yl)-(1-methyl-5-nitro-3,4-dihydro-1H-2,7-naphthyridin-2-yl)-methanone is synthesized in a similar way.

The stereo-isomers of this compound are separated. The S-configurated compound has a different activity than the R-configurated compound.

### Example 332

### 6-Bromo-pyrazolo[1,5-a]pyridin-2-yl)-(1-methyl-3,4-dihydro-1H-[2,7]naphthyridin-2-yl)-methanone

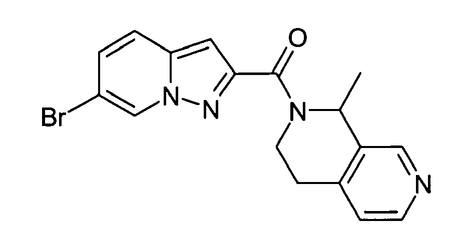

In close analogy to the procedure described in Example 1, 6-bromo-pyrazolo[1,5-a]pyridine-2-carboxylic acid is reacted with 1-methyl-1,2,3,4-tetrahydro-[2,7]naphthyridine to provide the title compound in moderate yield.
LC/MS: m/z = 372 (MH⁺)
The stereo-isomers of this compound are separated. The S-configurated compound has a different activity than the R-configurated compound.

### Example 333

### 6-Chloro-pyrazolo[1,5-a]pyridin-2-yl)-(1-methyl-3,4-dihydro-1H-[2,7]naphthyridin-2-yl)-methanone is synthesized in a similar way.

The stereo-isomers of this compound are separated. The S-configurated compound has a different activity than the R-configurated compound.

### Example 334

### (6-Bromo-pyrazolo[1,5-a]pyridin-2-yl)-(1-methyl-3,4-dihydro-1H-[2,6]naphthyridin-2-yl)-methanone

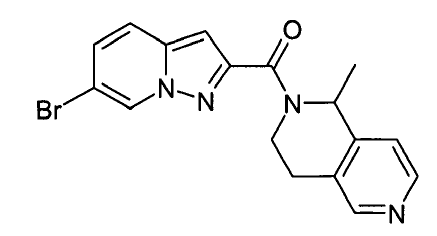

In close analogy to the procedure described in Example 1, 6-bromo-pyrazolo[1,5-a]pyridine-2-carboxylic acid is reacted with 1-methyl-1,2,3,4-tetrahydro-[2,6]naphthyridine to provide the title compound in moderate yield.
LC/MS: m/z = 372 (MH⁺)
The stereo-isomers of this compound are separated. The S-configurated compound has a different activity than the R-configurated compound.

### Example 335

### (6-Chloro-pyrazolo[1,5-a]pyridin-2-yl)-(1-methyl-3,4-dihydro-1H-[2,6]naphthyridin-2-yl)-methanone is synthesized in a similar way.

The stereo-isomers of this compound are separated. The S-configurated compound has a different activity than the R-configurated compound.

### Example 336

### (6-Bromo-pyrazolo[1,5-a]pyridin-2-yl)-(5-methyl-7,8-dihydro-5H-pyrido[4,3-d]pyrimidin-6-yl)-methanone

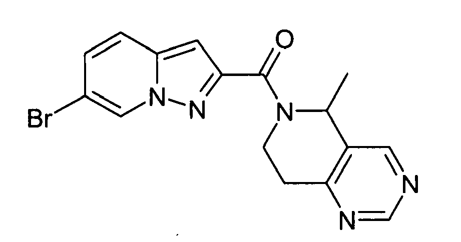

In close analogy to the procedure described in Example 1, 6-bromo-pyrazolo[1,5-a]pyridine-2-carboxylic acid is reacted with 5-methyl-5,6,7,8-tetrahydro-pyrido[4,3-d]pyrimidine to provide the title compound in moderate yield.
LC/MS: m/z = 373 (MH⁺)
The stereo-isomers of this compound are separated. The S-configurated compound has a different activity than the R-configurated compound.

### Example 337

### (6-Chloro-pyrazolo[1,5-a]pyridin-2-yl)-(5-methyl-7,8-dihydro-5H-pyrido[4,3-d]pyrimidin-6-yl)-methanone is synthesized in a similar way.

The stereo-isomers of this compound are separated. The S-configurated compound has a different activity than the R-configurated compound.

### Example 338

### (6-Bromo-pyrazolo[1,5-a]pyridin-2-yl)-(8-methyl-5,8-dihydro-6H-pyrido[3,4-d]pyrimidin-7-yl)-methanone

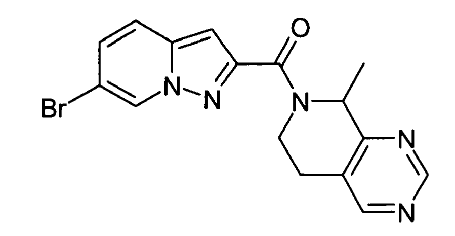

In close analogy to the procedure described in Example 1, 6-bromo-pyrazolo[1,5-a]pyridine-2-carboxylic acid is reacted with 8-methyl-5,6,7,8-tetrahydro-pyrido[3,4-d]pyrimidine to provide the title compound in moderate yield.
LC/MS: m/z = 373 (MH⁺).
The stereo-isomers of this compound are separated. The S-configurated compound has a different activity than the R-configurated compound.

### Example 339

### (6-Chloro-pyrazolo[1,5-a]pyridin-2-yl)-(8-methyl-5,8-dihydro-6H-pyrido[3,4-d]pyrimidin-7-yl)-methanone is synthesized in a similar way.

The stereo-isomers of this compound are separated. The S-configurated compound has a different activity than the R-configurated compound.

### Example 340

### (6-Bromo-pyrazolo[1,5-a]pyridin-2-yl)-(1-methyl-5-nitro-3,4-dihydro-1H-isoquinolin-2-yl)-methanone

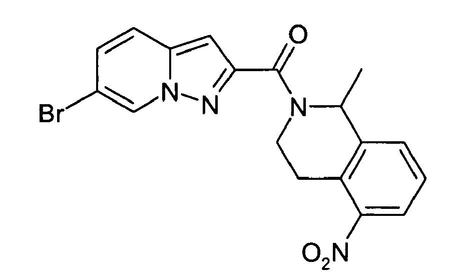

In close analogy to the procedure described in Example 1, 6-bromo-pyrazolo[1,5-a]pyridine-2-carboxylic acid is reacted with 1-methyl-5-nitro-1,2,3,4-tetrahydro-isoquinoline to provide the title compound in moderate yield.
LC/MS: m/z = 416 (MH⁺)
The stereo-isomers of this compound are separated. The S-configurated compound has a different activity than the R-configurated compound.

### Example 341

### (6-Chloro-pyrazolo[1,5-a]pyridin-2-yl)-(1-methyl-5-nitro-3,4-dihydro-1H-isoquinolin-2-yl)-methanone is synthesized in a similar way.

The stereo-isomers of this compound are separated. The S-configurated compound has a different activity than the R-configurated compound.

### Example 342

### (6-Bromo-pyrazolo[1,5-a]pyridin-2-yl)-(1-methyl-6-nitro-3,4-dihydro-1H-isoquinolin-2-yl)-methanone

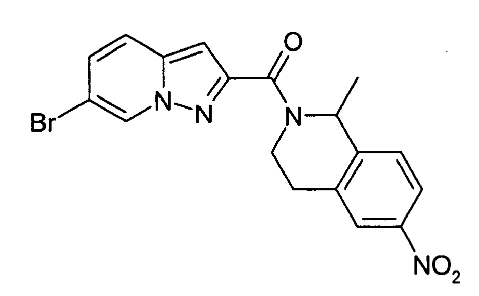

In close analogy to the procedure described in Example 1, 6-bromo-pyrazolo[1,5-a]pyridine-2-carboxylic acid is reacted with 1-methyl-6-nitro-1,2,3,4-tetrahydro-isoquinoline to provide the title compound in moderate yield.
LC/MS: m/z = 416 (MH⁺)
The stereo-isomers of this compound are separated. The S-configurated compound has a different activity than the R-configurated compound.

### Example 343

### (6-Chloro-pyrazolo[1,5-a]pyridin-2-yl)-(1-methyl-6-nitro-3,4-dihydro-1H-isoquinolin-2-yl)-methanone is synthesized in a similar way.

The stereo-isomers of this compound are separated. The S-configurated compound has a different activity than the R-configurated compound.

### Example 344

### (6-Bromo-pyrazolo[1,5-a]pyridin-2-yl)-(1-methyl-7-nitro-3,4-dihydro-1H-isoquinolin-2-yl)-methanone

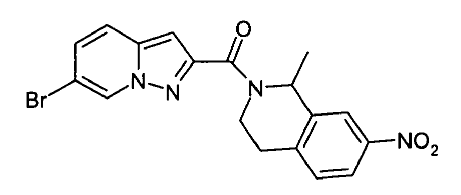

In close analogy to the procedure described in Example 1, 6-bromo-pyrazolo[1,5-a]pyridine-2-carboxylic acid is reacted with 1-methyl-7-nitro-1,2,3,4-tetrahydro-isoquinoline to provide the title compound in moderate yield.
LC/MS: m/z = 416 (MH⁺)
The stereo-isomers of this compound are separated. The S-configurated compound has a different activity than the R-configurated compound.

### Example 345

### (6-Chloro-pyrazolo[1,5-a]pyridin-2-yl)-(1-methyl-7-nitro-3,4-dihydro-1H-isoquinolin-2-yl)-methanone is synthesized in a similar way.

The stereo-isomers of this compound are separated. The S-configurated compound has a different activity than the R-configurated compound.

### Example 346

### 6-Bromo-pyrazolo[1,5-a]pyridine-2-carboxylic acid cycloheptylamide

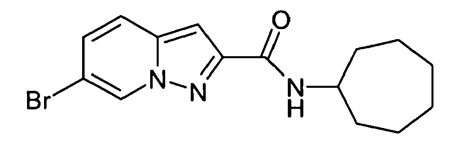

In close analogy to the procedure described in Example 1, 6-bromo-pyrazolo[1,5-a]pyridine-2-carboxylic acid is reacted with cycloheptylamine to provide the title compound in moderate yield.
LC/MS: m/z = 337 (MH⁺)

### Example 347

### 6-Chloro-pyrazolo[1,5-a]pyridine-2-carboxylic acid cycloheptylamide

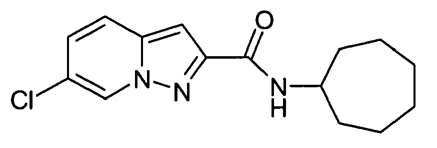

In close analogy to the procedure described in Example 1, 6-chloro-pyrazolo[1,5-a]pyridine-2-carboxylic acid is reacted with cycloheptylamine to provide the title compound in moderate yield.
LC/MS: m/z = 292 (MH⁺)

### Example 348

### 6-Bromo-pyrazolo[1,5-a]pyridine-2-carboxylic acid cyclohexylamide

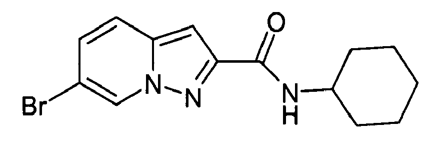

In close analogy to the procedure described in Example 1, 6-bromo-pyrazolo[1,5-a]pyridine-2-carboxylic acid is reacted with cyclohexylamine to provide the title compound in moderate yield.
LC/MS: m/z = 323 (MH⁺)

### Example 349

### 6-Chloro-pyrazolo[1,5-a]pyridine-2-carboxylic acid cyclohexylamide

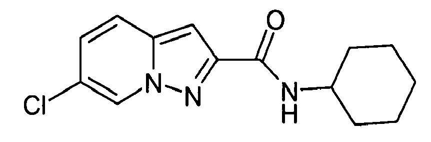

In close analogy to the procedure described in Example 1, 6-chloro-pyrazolo[1,5-a]pyridine-2-carboxylic acid is reacted with cyclohexylamine to provide the title compound in moderate yield.
LC/MS: m/z = 278 (MH⁺)

### Example 350

### 2-(6-Bromo-pyrazolo[1,5-a]pyridine-2-carbonyl)-1-methyl-1,2,3,4-tetrahydro-isoquinoline-5-carbonitrile

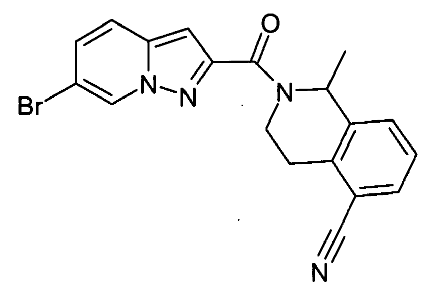

In close analogy to the procedure described in Example 1, 6-bromo-pyrazolo[1,5-a]pyridine-2-carboxylic acid is reacted with 1-methyl-1,2,3,4-tetrahydro-isoquinoline-5-carbonitrile to provide the title compound in moderate yield.
LC/MS: m/z = 396 (MH⁺)
The stereo-isomers of this compound are separated. The S-configurated compound has a different activity than the R-configurated compound.

### Example 351

### 2-(6-Chloro-pyrazolo[1,5-a]pyridine-2-carbonyl)-1-methyl-1,2,3,4-tetrahydro-isoquinoline-5-carbonitrile is synthesized in a similar way.

The stereo-isomers of this compound are separated. The S-configurated compound has a different activity than the R-configurated compound.

### Example 352

### 2-(6-Bromo-pyrazolo[1,5-a]pyridine-2-carbonyl)-1-methyl-1,2,3,4-tetrahydro-isoquinoline-5-carboxylic acid methyl ester

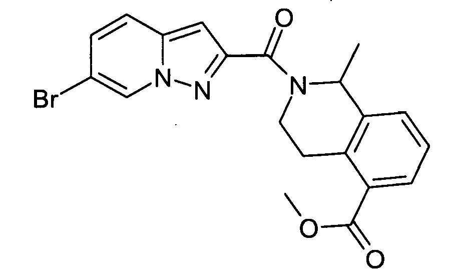

In close analogy to the procedure described in Example 1, 6-bromo-pyrazolo[1,5-a]pyridine-2-carboxylic acid is reacted with 1-methyl-1,2,3,4-tetrahydro-isoquinoline-5-carboxylic acid methyl ester to provide the title compound in moderate yield.
LC/MS: m/z = 429 (MH⁺)
The stereo-isomers of this compound are separated. The S-configurated compound has a different activity than the R-configurated compound.

### Example 353

### 2-(6-Chloro-pyrazolo[1,5-a]pyridine-2-carbonyl)-1-methyl-1,2,3,4-tetrahydro-isoquinoline-5-carboxylic acid methyl ester is synthesized in a similar way.

The stereo-isomers of this compound are separated. The S-configurated compound has a different activity than the R-configurated compound.

### Example 354

### (6-Bromo-pyrazolo[1,5-a]pyridin-2-yl)-(2,6-dimethyl-8,9-dihydro-6H-3-oxa-1,7-diazacyclopenta[a]naphthalen-7-yl)-methanone

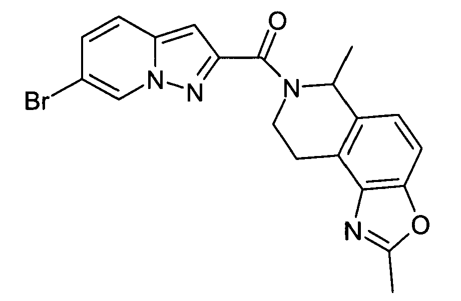

In close analogy to the procedure described in Example 1, 6-bromo-pyrazolo[1,5-a]pyridine-2-carboxylic acid is reacted with 2,6-dimethyl-6,7,8,9-tetrahydro-3-oxa-1,7-diaza-cyclopenta[a]naphthalene to provide the title compound in moderate yield.
LC/MS: m/z = 426 (MH⁺)
The stereo-isomers of this compound are separated. The S-configurated compound has a different activity than the R-configurated compound.

### Example 355

### (6-Chloro-pyrazolo[1,5-a]pyridin-2-yl)-(2,6-dimethyl-8,9-dihydro-6H-3-oxa-1,7-diazacyclopenta[a]naphthalen-7-yl)-methanone is synthesized in a similar way.

The stereo-isomers of this compound are separated. The S-configurated compound has a different activity than the R-configurated compound.

### Example 356

### (6-Bromo-pyrazolo[1,5-a]pyridin-2-yl)-(4-methyl-1,4-dihydro-2H-[3,7]phenanthrolin-3-yl)-methanone

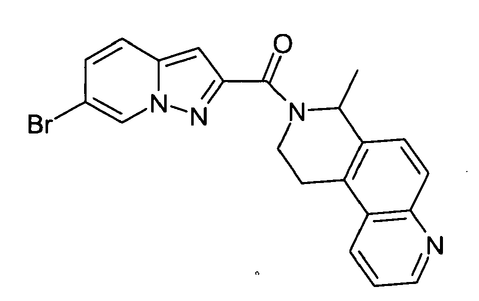

In close analogy to the procedure described in Example 1, 6-bromo-pyrazolo[1,5-a]pyridine-2-carboxylic acid is reacted with 4-methyl-1,2,3,4-tetrahydro-[3,7]phenanthroline to provide the title compound in moderate yield.
LC/MS: m/z = 422 (MH⁺)
The stereo-isomers of this compound are separated. The S-configurated compound has a different activity than the R-configurated compound.

### Example 357

### (6-Chloro-pyrazolo[1,5-a]pyridin-2-yl)-(4-methyl-1,4-dihydro-2H-[3,7]phenanthrolin-3-yl)-methanone is synthesized in a similar way.

The stereo-isomers of this compound are separated. The S-configurated compound has a different activity than the R-configurated compound.

### Example 358

### (6-Bromo-pyrazolo[1,5-a]pyridin-2-yl)-(2,6-dimethyl-8,9-dihydro-6H-thiazolo[4,5-f]isoquinolin-7-yl)-methanone

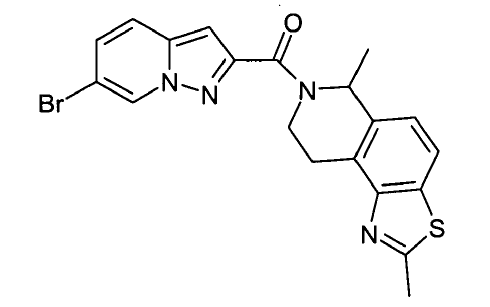

In close analogy to the procedure described in Example 1, 6-bromo-pyrazolo[1,5-a]pyridine-2-carboxylic acid is reacted with 2,6-dimethyl-6,7,8,9-tetrahydro-thiazolo[4,5-f]isoquinoline to provide the title compound in moderate yield.
LC/MS: m/z = 442 (MH⁺)
The stereo-isomers of this compound are separated. The S-configurated compound has a different activity than the R-configurated compound.

### Example 359

### (6-Chloro-pyrazolo[1,5-a]pyridin-2-yl)-(2,6-dimethyl-8,9-dihydro-6H-thiazolo[4,5-f]isoquinolin-7-yl)-methanone is synthesized in a similar way.

The stereo-isomers of this compound are separated. The S-configurated compound has a different activity than the R-configurated compound.

### Example 360

### (6-Bromo-pyrazolo[1,5-a]pyridin-2-yl)-(5-methanesulfonyl-1-methyl-3,4-dihydro-1H-isoquinolin-2-yl)-methanone

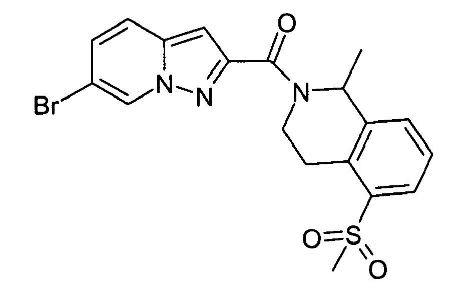

In close analogy to the procedure described in Example 1, 6-bromo-pyrazolo[1,5-a]pyridine-2-carboxylic acid is reacted with 5-methanesulfonyl-l-methyl-1,2,3,4-tetrahydro-isoquinoline to provide the title compound in moderate yield.
LC/MS: m/z = 449 (MH⁺)
The stereo-isomers of this compound are separated. The S-configurated compound has a different activity than the R-configurated compound.

### Example 361

### (6-Chloro-pyrazolo[1,5-a]pyridin-2-yl)-(5-methanesulfonyl-1-methyl-3,4-dihydro-1H-isoquinolin-2-yl)-methanone is synthesized in a similar way.

The stereo-isomers of this compound are separated. The S-configurated compound has a different activity than the R-configurated compound.

### Example 362

### (6-Bromo-pyrazolo[1,5-a]pyridin-2-yl)-(6-methanesulfonyl-1-methyl-3,4-dihydro-1H-isoquinolin-2-yl)-methanone

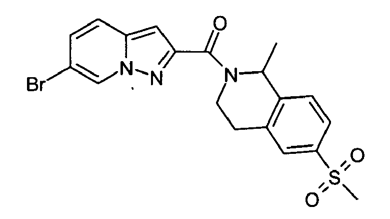

In close analogy to the procedure described in Example 1, 6-bromo-pyrazolo[1,5-a]pyridine-2-carboxylic acid is reacted with 6-methanesulfonyl-1-methyl-1,2,3,4-tetrahydro-isoquinoline to provide the title compound in moderate yield.
LC/MS: m/z = 449 (MH⁺)
The stereo-isomers of this compound are separated. The S-configurated compound has a different activity than the R-configurated compound.

### Example 363

### (6-Chloro-pyrazolo[1,5-a]pyridin-2-yl)-(6-methanesulfonyl-1-methyl-3,4-dihydro-1H-isoquinolin-2-yl)-methanone is synthesized in a similar way.

The stereo-isomers of this compound are separated. The S-configurated compound has a different activity than the R-configurated compound.

### Example 364

### (6-Bromo-pyrazolo[1,5-a]pyridin-2-yl)-(7-methanesulfonyl-1-methyl-3,4-dihydro-1H-isoquinolin-2-yl)-methanone

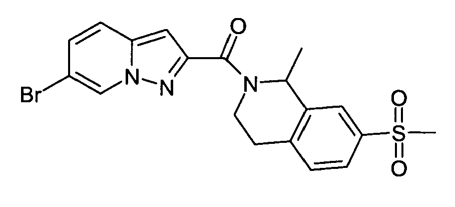

In close analogy to the procedure described in Example 1, 6-bromo-pyrazolo[1,5-a]pyridine-2-carboxylic acid is reacted with 7-methanesulfonyl-1-methyl-1,2,3,4-tetrahydro-isoquinoline to provide the title compound in moderate yield.
LC/MS: m/z = 449 (MH⁺)
The stereo-isomers of this compound are separated. The S-configurated compound has a different activity than the R-configurated compound.

### Example 365

### (6-Chloro-pyrazolo[1,5-a]pyridin-2-yl)-(7-methanesulfonyl-1-methyl-3,4-dihydro-1H-isoquinolin-2-yl)-methanone is synthesized in a similar way.

The stereo-isomers of this compound are separated. The S-configurated compound has a different activity than the R-configurated compound.

### Example 366

### 2-(6-Bromo-pyrazolo[1,5-a]pyridine-2-carbonyl)-1-methyl-1,2,3,4-tetrahydro-isoquinoline-6-carboxylic acid methyl ester

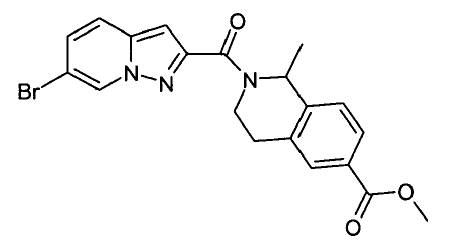

In close analogy to the procedure described in Example 1, 6-bromo-pyrazolo[1,5-a]pyridine-2-carboxylic acid is reacted with 1-methyl-1,2,3,4-tetrahydro-isoquinoline-6-carboxylic acid methyl ester provide the title compound in moderate yield.
LC/MS: m/z = 449 (MH⁺)
The stereo-isomers of this compound are separated. The S-configurated compound has a different activity than the R-configurated compound.

### Example 367

### 2-(6-Chloro-pyrazolo[1,5-a]pyridine-2-carbonyl)-1-methyl-1,2,3,4-tetrahydro-isoquinoline-6-carboxylic acid methyl ester is synthesized in a similar way.

The stereo-isomers of this compound are separated. The S-configurated compound has a different activity than the R-configurated compound.

### Example 368

### (6-Bromo-pyrazolo[1,5-a]pyridin-2-yl)-(4,7-dimethyl-1,4,7,8,9,10-hexahydro-2H-[3,7]phenanthrolin-3-yl)-methanone

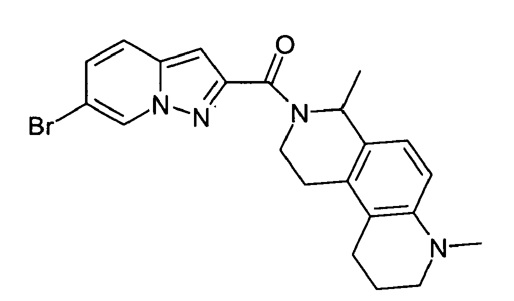

In close analogy to the procedure described in Example 1, 6-bromo-pyrazolo[1,5-a]pyridine-2-carboxylic acid is reacted with 4,7-dimethyl-1,2,3,4,7,8,9,10-octahydro-[3,7]phenanthroline to provide the title compound in moderate yield.
LC/MS: m/z = 440 (MH⁺)
The stereo-isomers of this compound are separated. The S-configurated compound has a different activity than the R-configurated compound.

### Example 369

### (6-Chloro-pyrazolo[1,5-a]pyridin-2-yl)-(4,7-dimethyl-1,4,7,8,9,10-hexahydro-2H-[3,7]phenanthrolin-3-yl)-methanone is synthesized in a similar way.

The stereo-isomers of this compound are separated. The S-configurated compound has a different activity than the R-configurated compound.

### Example 370

### 1-[2-(6-Bromo-pyrazolo[1,5-a]pyridine-2-carbonyl)-1-methyl-1,2,3,4-tetrahydro-isoquinolin-5-yl]-ethanone

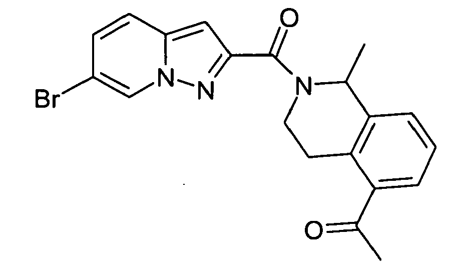

In close analogy to the procedure described in Example 1, 6-bromo-pyrazolo[1,5-a]pyridine-2-carboxylic acid is reacted with 1-(1-methyl-1,2,3,4-tetrahydro-isoquinolin-5-yl)-ethanone to provide the title compound in moderate yield.
LC/MS: m/z = 413 (MH⁺)
The stereo-isomers of this compound are separated. The S-configurated compound has a different activity than the R-configurated compound.

### Example 371

### 1-[2-(6-Chloro-pyrazolo[1,5-a]pyridine-2-carbonyl)-1-methyl-1,2,3,4-tetrahydro-isoquinolin-5-yl]-ethanone is synthesized in a similar way.

The stereo-isomers of this compound are separated. The S-configurated compound has a different activity than the R-configurated compound.

### Example 372

### 6-Bromo-pyrazolo[1,5-a]pyridine-2-carboxylic acid adamantyl-1 amide

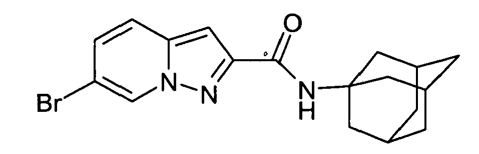

In close analogy to the procedure described in Example 1, 6-bromo-pyrazolo[1,5-a]pyridine-2-carboxylic acid is reacted with adamantyl-1 amineto provide the title compound in moderate yield.
LC/MS: m/z = 375 (MH⁺)

### Example 373

### 6-Chloro-pyrazolo[1,5-a]pyridine-2-carboxylic acid adamantyl-1 amide

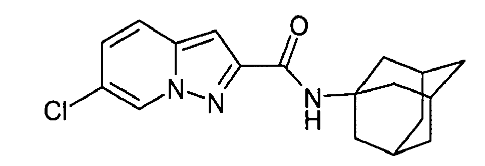

In close analogy to the procedure described in Example 1, 6-chloro-pyrazolo[1,5-a]pyridine-2-carboxylic acid is reacted with adamantyl-1 amine to provide the title compound in moderate yield.
LC/MS: m/z = 330 (MH⁺)

### Example 374

### 2-(6-Bromo-pyrazolo[1,5-a]pyridine-2-carbonyl)-1-methyl-1,2,3,4-tetrahydro-isoquinoline-7-carboxylic acid methyl ester

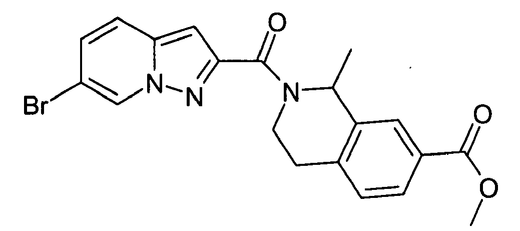

In close analogy to the procedure described in Example 1, 6-bromo-pyrazolo[1,5-a]pyridine-2-carboxylic acid is reacted with 1-methyl-1,2,3,4-tetrahydro-isoquinoline-7-carboxylic acid methyl ester to provide the title compound in moderate yield.
LC/MS: m/z = 429 (MH⁺)
The stereo-isomers of this compound are separated. The S-configurated compound has a different activity than the R-configurated compound.

### Example 375

### 2-(6-Chloro-pyrazolo[1,5-a]pyridine-2-carbonyl)-1-methyl-1,2,3,4-tetrahydro-isoquinoline-7-carboxylic acid methyl ester is synthesized in a similar way.

The stereo-isomers of this compound are separated. The S-configurated compound has a different activity than the R-configurated compound.

### Example 376

### (6-Bromo-pyrazolo[1,5-a]pyridin-2-yl)-[1-methyl-5-(morpholine-4-carbonyl)-3,4-dihydro-1H-isoquinolin-2-yl]-methanone

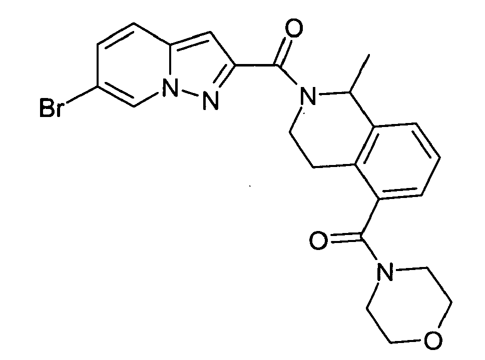

In close analogy to the procedure described in Example 1, 6-bromo-pyrazolo[1,5-a]pyridine-2-carboxylic acid is reacted with (1-methyl-1,2,3,4-tetrahydro-isoquinolin-5-yl)-morpholin-4-yl-methanone to provide the title compound in moderate yield.
LC/MS: m/z = 484 (MH⁺)
The stereo-isomers of this compound are separated. The S-configurated compound has a different activity than the R-configurated compound.

### Example 377

### ((6-Chloro-pyrazolo[1,5-a]pyridin-2-yl)-[1-methyl-5-(morpholine-4-carbonyl)-3,4-dihydro-1H-isoquinolin-2-yl]-methanone is synthesized in a similar way.

The stereo-isomers of this compound are separated. The S-configurated compound has a different activity than the R-configurated compound.

### Example 378

### (6-Bromo-pyrazolo[1,5-a]pyridin-2-yl)-[1-methyl-7-(morpholine-4-carbonyl)-3,4-dihydro-1 H-isoquinolin-2-yl]-methanone

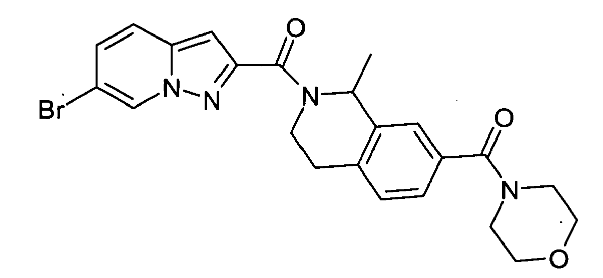

In close analogy to the procedure described in Example 1, 6-bromo-pyrazolo[1,5-a]pyridine-2-carboxylic acid is reacted with (1-methyl-1,2,3,4-tetrahydro-isoquinolin-7-yl)-morpholin-4-yl-methanone to provide the title compound in moderate yield.
LC/MS: m/z = 484 (MH⁺)
The stereo-isomers of this compound are separated. The S-configurated compound has a different activity than the R-configurated compound.

### Example 379

### (6-Chloro-pyrazolo[1,5-a]pyridin-2-yl)-[1-methyl-7-(morpholine-4-carbonyl)-3,4-dihydro-1H-isoquinolin-2-yl]-methanone is synthesized in a similar way.

The stereo-isomers of this compound are separated. The S-configurated compound has a different activity than the R-configurated compound.

### Example 380

### (6-Bromo-pyrazolo[1,5-a]pyridin-2-yl)-[1-methyl-6-(morpholine-4-carbonyl)-3,4-dihydro-1 H-isoquinolin-2-yl]-methanone

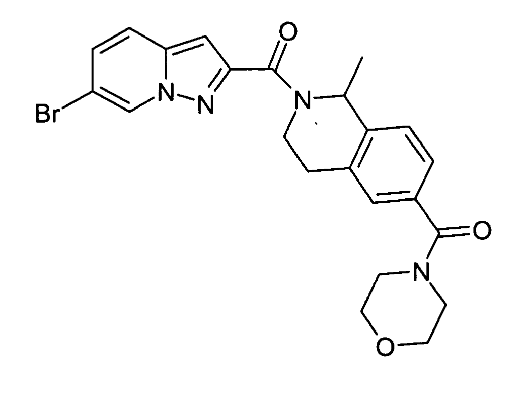

In close analogy to the procedure described in Example 1, 6-bromo-pyrazolo[1,5-a]pyridine-2-carboxylic acid is reacted with (1-methyl-1,2,3,4-tetrahydro-isoquinolin-6-yl)-morpholin-4-yl-methanone to provide the title compound in moderate yield.
LC/MS: m/z = 484 (MH⁺)
The stereo-isomers of this compound are separated. The S-configurated compound has a different activity than the R-configurated compound.

### Example 381

### (6-Chloro-pyrazolo[1,5-a]pyridin-2-yl)-[1-methyl-6-(morpholine-4-carbonyl)-3,4-dihydro-1H-isoquinolin-2-yl]-methanone is synthesized in a similar way.

The stereo-isomers of this compound are separated. The S-configurated compound has a different activity than the R-configurated compound.

### Example 382

### (6-Bromo-pyrazolo[1,5-a]pyridin-2-yl)-(6-methyl-8,9-dihydro-6H-furo[3,2-f]isoquinolin-7-yl)-methanone

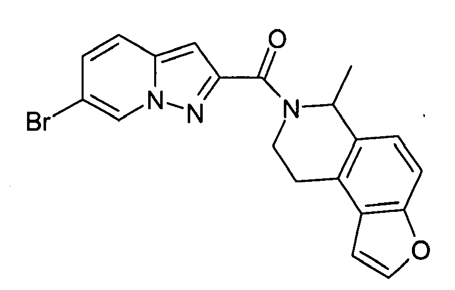

In close analogy to the procedure described in Example 1, 6-bromo-pyrazolo[1,5-a]pyridine-2-carboxylic acid is reacted with 6-methyl-6,7,8,9-tetrahydro-furo[3,2-flisoquinoline to provide the title compound in moderate yield.
LC/MS: m/z = 411 (MH⁺)
The stereo-isomers of this compound are separated. The S-configurated compound has a different activity than the R-configurated compound.

### Example 383

(6-Chloro-pyrazolo[1,5-a]pyridin-2-yl)-(6-methyl-8,9-dihydro-6H-furo[3,2-f]isoquinolin-7-yl)-methanone is synthesized in a similar way.

The stereo-isomers of this compound are separated. The S-configurated compound has a different activity than the R-configurated compound.

### Example 384

### (6-Bromo-pyrazolo[1,5-a]pyridin-2-yl)-(3,6-dimethyl-3,6,8,9-tetrahydro-pyrrolo[3,2-f]isoqumolm-7-yl)-methanone

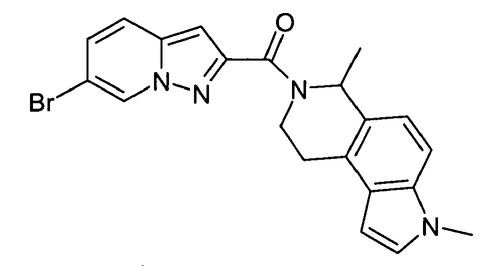

In close analogy to the procedure described in Example 1, 6-bromo-pyrazolo[1,5-a]pyridine-2-carboxylic acid is reacted with 3,6-dimethyl-6,7,8,9-tetrahydro-3H-pyrrolo[3,2-f]isoquinoline to provide the title compound in moderate yield.
LC/MS: m/z = 424 (MH⁺)
The stereo-isomers of this compound are separated. The S-configurated compound has a different activity than the R-configurated compound.

### Example 385

### (6-Chloro-pyrazolo[1,5-a]pyridin-2-yl)-(3,6-dimethyl-3,6,8,9-tetrahydro-pyrrolo[3,2-f]isoquinolin-7-yl)-methanone is synthesized in a similar way.

The stereo-isomers of this compound are separated. The S-configurated compound has a different activity than the R-configurated compound.

### Example 386

(6-Bromo-pyrazolo[1,5-a]pyridin-2-yl)-(8-methyl-5,8-dihydro-6H-furo[3,2-g]isoquinolin-7-yl)-methanone

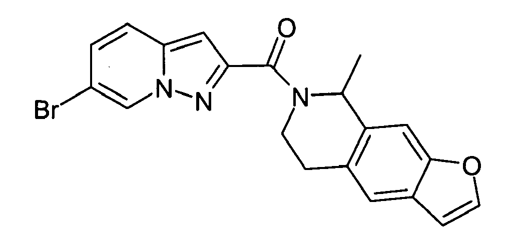

In close analogy to the procedure described in Example 1, 6-bromo-pyrazolo[1,5-a]pyridine-2-carboxylic acid is reacted with 8-methyl-5,6,7,8-tetrahydro-furo[3,2-g]isoquinoline to provide the title compound in moderate yield.
LC/MS: m/z = 411 (MH⁺)
The stereo-isomers of this compound are separated. The S-configurated compound has a different activity than the R-configurated compound.

### Example 387

### (6-Chloro-pyrazolo[1,5-a]pyridin-2-yl)-(8-methyl-5,8-dihydro-6H-furo[3,2-g]isoquinolin-7-yl)-methanone is synthesized in a similar way.

The stereo-isomers of this compound are separated. The S-configurated compound has a different activity than the R-configurated compound.

### Example 388

### 7-(6-Bromo-pyrazolo[1,5-a]pyridine-2-carbonyl)-2,6-dimethyl-1,2,6,7,8,9-hexahydro-pyrrolo[3,4-f]isoquinolin-3-one

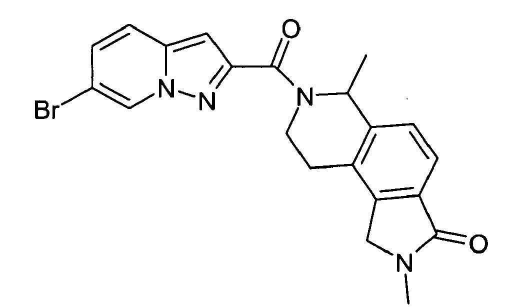

In close analogy to the procedure described in Example 1, 6-bromo-pyrazolo[1,5-a]pyridine-2-carboxylic acid is reacted with 2,6-dimethyl-1,2,6,7,8,9-hexahydro-pyrrolo[3,4-f]isoquinolin-3-one to provide the title compound in moderate yield.
LC/MS: m/z = 440 (MH⁺)
The stereo-isomers of this compound are separated. The S-configurated compound has a different activity than the R-configurated compound.

### Example 389

### 7-(6-Chloro-pyrazolo[1,5-a]pyridine-2-carbonyl)-2,6-dimethyl-1,2,6,7,8,9-hexahydro-pyrrolo[3,4-f]isoquinolin-3-one is synthesized in a similar way.

The stereo-isomers of this compound are separated. The S-configurated compound has a different activity than the R-configurated compound.

### Example 390

### 7-(6-Bromo-pyrazolo[1,5-a]pyridine-2-carbonyl)-2,6-dimethyl-2,3,6,7,8,9-hexahydro-pyrrolo[3,4-f]isoquinolin-1-one

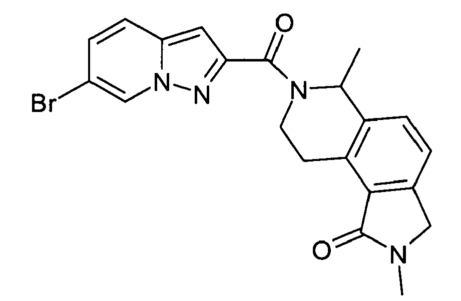

In close analogy to the procedure described in Example 1, 6-bromo-pyrazolo[1,5-a]pyridine-2-carboxylic acid is reacted with 2,6-dimethyl-2,3,6,7,8,9-hexahydro-pyrrolo[3,4-f]isoquinolin-1-one to provide the title compound in moderate yield.
LC/MS: m/z = 440 (MH⁺)
The stereo-isomers of this compound are separated. The S-configurated compound has a different activity than the R-configurated compound.

### Example 391

### 7-(6-Chloro-pyrazolo[1,5-a]pyridine-2-carbonyl)-2,6-dimethyl-2,3,6,7,8,9-hexahydro-pyrrolo[3,4-f]isoquinolin-1-one is synthesized in a similar way.

The stereo-isomers of this compound are separated. The S-configurated compound has a different activity than the R-configurated compound.

### Example 392

### (6-Bromo-pyrazolo[1,5-a]pyridin-2-yl)-(6-diethylamino-1-methyl-3,4-dihydro-1H-isoquinolin-2-yl)-methanone

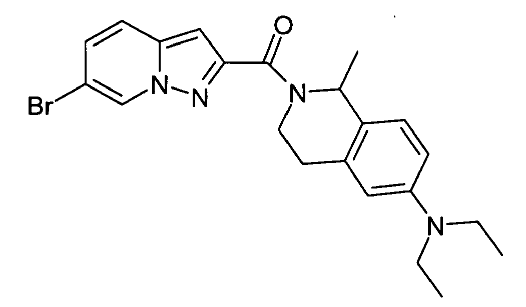

In close analogy to the procedure described in Example 1, 6-bromo-pyrazolo[1,5-a]pyridine-2-carboxylic acid is reacted with N,N-diethyl-(1-methyl-1,2,3,4-tetrahydro-isoquinolin-6-yl)-amine to provide the title compound in moderate yield.
LC/MS: m/z = 442 (MH⁺)
The stereo-isomers of this compound are separated. The S-configurated compound has a different activity than the R-configurated compound.

### Example 393

### (6-Chloro-pyrazolo[1,5-a]pyridin-2-yl)-(6-diethylamino-1-methyl-3,4-dihydro-1H-isoquinolin-2-yl)-methanone is synthesized in a similar way.

The stereo-isomers of this compound are separated. The S-configurated compound has a different activity than the R-configurated compound.

### Example 394

### (6-Bromo-pyrazolo[1,5-a]pyridin-2-yl)-(5-diethylamino-1-methyl-3,4-dihydro-1H-isoquinolin-2-yl)-methanone

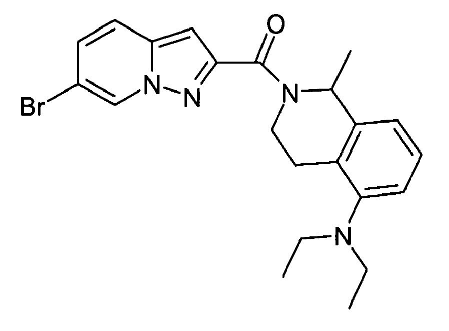

In close analogy to the procedure described in Example 1, 6-bromo-pyrazolo[1,5-a]pyridine-2-carboxylic acid is reacted with N,N-diethyl-(1-methyl-1,2,3,4-tetrahydro-isoquinolin-5-yl)-amine to provide the title compound in moderate yield.
LC/MS: m/z = 442 (MH⁺)
The stereo-isomers of this compound are separated. The S-configurated compound has a different activity than the R-configurated compound.

### Example 395

### (6-Chloro-pyrazolo[1,5-a]pyridin-2-yl)-(5-diethylamino-1-methyl-3,4-dihydro-1H-isoquinolin-2-yl)-methanone is synthesized in a similar way.

The stereo-isomers of this compound are separated. The S-configurated compound has a different activity than the R-configurated compound.

### Example 396

### (6-Bromo-pyrazolo[1,5-a]pyridin-2-yl)-(8-diethylamino-1-methyl-3,4-dihydro-1H-isoquinolin-2-yl)-methanone

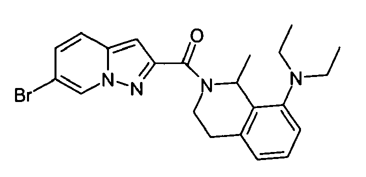

In close analogy to the procedure described in Example 1, 6-bromo-pyrazolo[1,5-a]pyridine-2-carboxylic acid is reacted with N,N-diethyl-(1-methyl-1,2,3,4-tetrahydro-isoquinolin-8-yl)-amine to provide the title compound in moderate yield.
LC/MS: m/z = 442 (MH⁺)
The stereo-isomers of this compound are separated. The S-configurated compound has a different activity than the R-configurated compound.

### Example 397

### (6-Chloro-pyrazolo[1,5-a]pyridin-2-yl)-(8-diethylamino-1-methyl-3,4-dihydro-1H-isoquinolin-2-yl)-methanone is synthesized in a similar way.

The stereo-isomers of this compound are separated. The S-configurated compound has a different activity than the R-configurated compound.

### Example 398

### (6-Bromo-pyrazolo[1,5-a]pyridin-2-yl)-(7-diethylamino-1-methyl-3,4-dihydro-1H-isoquinolin-2-yl)-methanone

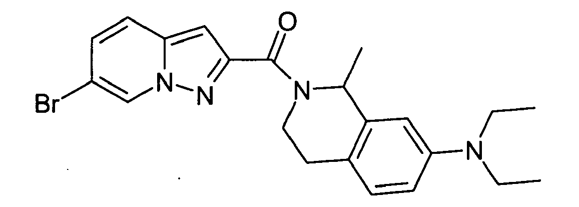

In close analogy to the procedure described in Example 1, 6-bromo-pyrazolo[1,5-a]pyridine-2-carboxylic acid is reacted with N,N-diethyl-(1-methyl-1,2,3,4-tetrahydro-isoquinolin-7-yl)-amine to provide the title compound in moderate yield.
LC/MS: m/z = 442 (MH⁺)
The stereo-isomers of this compound are separated. The S-configurated compound has a different activity than the R-configurated compound.

### Example 399

### (6-Chloro-pyrazolo[1,5-a]pyridin-2-yl)-(7-diethylamino-1-methyl-3,4-dihydro-1H-isoquinolin-2-yl)-methanone is synthesized in a similar way.

The stereo-isomers of this compound are separated. The S-configurated compound has a different activity than the R-configurated compound.

### Example 400

### (6-Bromo-pyrazolo[1,5-a]pyridin-2-yl)-(6,7-difluoro-1-methyl-3,4-dihydro-1H-isoquinolin-2-yl)-methanone

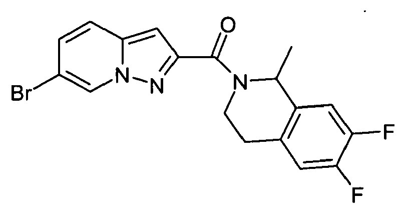

In close analogy to the procedure described in Example 1, 6-bromo-pyrazolo[1,5-a]pyridine-2-carboxylic acid is reacted with 6,7-difluoro-1-methyl-1,2,3,4-tetrahydro-isoquinoline to provide the title compound in moderate yield.
LC/MS: m/z = 409 (MH⁺)
The stereo-isomers of this compound are separated. The S-configurated compound has a different activity than the R-configurated compound.

### Example 401

### (6-Chloro-pyrazolo[1,5-a]pyridin-2-yl)-(6,7-difluoro-1-methyl-3,4-dihydro-1H-isoquinolin-2-yl)-methanone

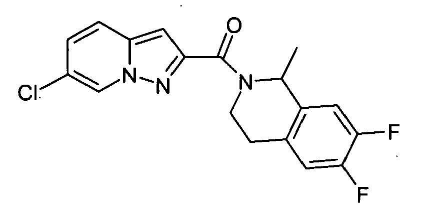

In close analogy to the procedure described in Example 1, 6-chloro-pyrazolo[1,5-a]pyridine-2-carboxylic acid is reacted with 6,7-difluoro-1-methyl-1,2,3,4-tetrahydro-isoquinoline to provide the title compound in moderate yield.
LC/MS: m/z = 362 (MH⁺)
The stereo-isomers of this compound are separated. The S-configurated compound has a different activity than the R-configurated compound.

### Example 402

### (6-Bromo-pyrazolo[1,5-a]pyridin-2-yl)-(6,7-dichloro-1-methyl-3,4-dihydro-1H-isoquinolin-2-yl)-methanone

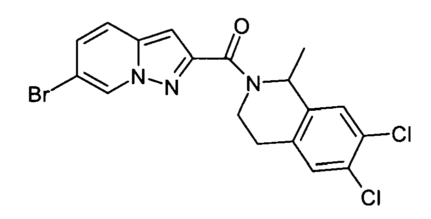

In close analogy to the procedure described in Example 1, 6-bromo-pyrazolo[1,5-a]pyridine-2-carboxylic acid is reacted with 6,7-dichloro-1-methyl-1,2,3,4-tetrahydro-isoquinoline to provide the title compound in moderate yield.
LC/MS: m/z = 440 (MH⁺)
The stereo-isomers of this compound are separated. The S-configurated compound has a different activity than the R-configurated compound.

### Example 403

### (6-Chloro-pyrazolo[1,5-a]pyridin-2-yl)-(6,7-dichloro-1-methyl-3,4-dihydro-1H-isoquinolin-2-yl)-methanone

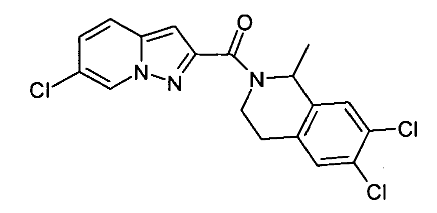

In close analogy to the procedure described in Example 1, 6-chloro-pyrazolo[1,5-a]pyridine-2-carboxylic acid is reacted with 6,7-dichloro-1-methyl-1,2,3,4-tetrahydro-isoquinoline to provide the title compound in moderate yield.
LC/MS: m/z = 394 (MH⁺)
The stereo-isomers of this compound are separated. The S-configurated compound has a different activity than the R-configurated compound.

### Example 404

### (6-Bromo-pyrazolo[1,5-a]pyridin-2-yl)-(6,7-dibromo-1-methyl-3,4-dihydro-1H-isoquinolin-2-yl)-methanone

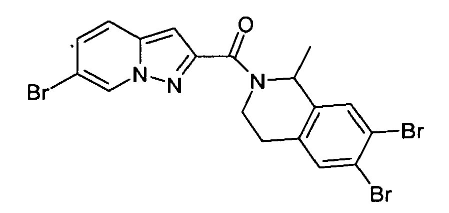

In close analogy to the procedure described in Example 1, 6-bromo-pyrazolo[1,5-a]pyridine-2-carboxylic acid is reacted with 6,7-dibromo-1-methyl-1,2,3,4-tetrahydro-isoquinoline to provide the title compound in moderate yield.
LC/MS: m/z = 529 (MH⁺)
The stereo-isomers of this compound are separated. The S-configurated compound has a different activity than the R-configurated compound.

### Example 405

### (6-Chloro-pyrazolo[1,5-a]pyridin-2-yl)-(6,7-dibromo-1-methyl-3,4-dihydro-1 H-isoquinolin-2-yl)-methanone

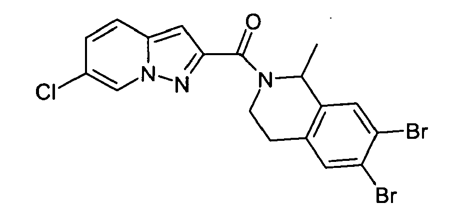

In close analogy to the procedure described in Example 1, 6-chloro-pyrazolo[1,5-a]pyridine-2-carboxylic acid is reacted with 6,7-dibromo-1-methyl-1,2,3,4-tetrahydro-isoquinoline to provide the title compound in moderate yield.
LC/MS: m/z = 484 (MH⁺)
The stereo-isomers of this compound are separated. The S-configurated compound has a different activity than the R-configurated compound.

### Example 406

### (6-Bromo-pyrazolo[1,5-a]pyridin-2-yl)-(1-methyl-7-morpholin-4-yl-3,4-dihydro-1H-isoquinolin-2-yl)-methanone

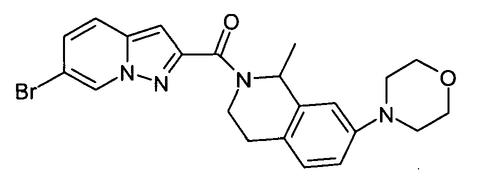

In close analogy to the procedure described in Example 1, 6-bromo-pyrazolo[1,5-a]pyridine-2-carboxylic acid is reacted with 1-methyl-7-morpholin-4-yl-1,2,3,4-tetrahydro-isoquinoline to provide the title compound in moderate yield.
LC/MS: m/z = 456 (MH⁺)
The stereo-isomers of this compound are separated. The S-configurated compound has a different activity than the R-configurated compound.

### Example 407

### (6-Chloro-pyrazolo[1,5-a]pyridin-2-yl)-(1-methyl-7-morpholin-4-yl-3,4-dihydro-1H-isoquinolin-2-yl)-methanone

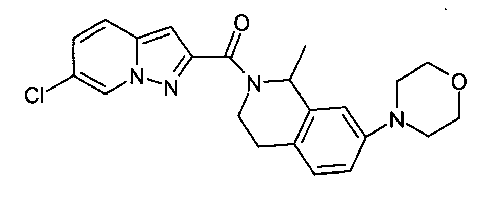

In close analogy to the procedure described in Example 1, 6-chloro-pyrazolo[1,5-a]pyridine-2-carboxylic acid is reacted with 1-methyl-7-morpholin-4-yl-1,2,3,4-tetrahydro-isoquinoline to provide the title compound in moderate yield.
LC/MS: m/z = 411 (MH⁺)
The stereo-isomers of this compound are separated. The S-configurated compound has a different activity than the R-configurated compound.

### Example 408

### (6-Bromo-pyrazolo[1,5-a]pyridin-2-yl)-(1-methyl-8-morpholin-4-yl-3,4-dihydro-1H-isoquinolin-2-yl)-methanone

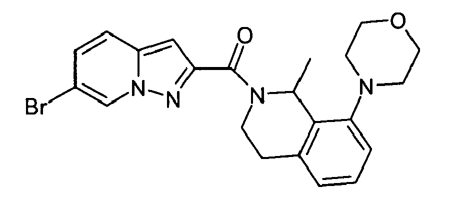

In close analogy to the procedure described in Example 1, 6-bromo-pyrazolo[1,5-a]pyridine-2-carboxylic acid is reacted with 1-methyl-8-morpholin-4-yl-1,2,3,4-tetrahydro-isoquinoline to provide the title compound in moderate yield.
LC/MS: m/z = 456 (MH⁺)
The stereo-isomers of this compound are separated. The S-configurated compound has a different activity than the R-configurated compound.

### Example 409

### (6-Chloro-pyrazolo[1,5-a]pyridin-2-yl)-(1-methyl-8-morpholin-4-yl-3,4-dihydro-1H-isoquinolin-2-yl)-methanone

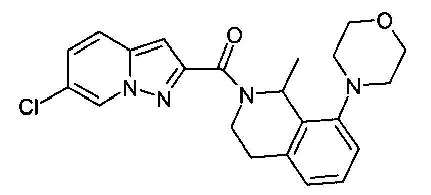

In close analogy to the procedure described in Example 1, 6-chloro-pyrazolo[1,5-a]pyridine-2-carboxylic acid is reacted with 1-methyl-8-morpholin-4-yl-1,2,3,4-tetrahydro-isoquinoline to provide the title compound in moderate yield.
LC/MS: m/z = 411 (MH⁺)
The stereo-isomers of this compound are separated. The S-configurated compound has a different activity than the R-configurated compound.

### Example 410

### (6-Bromo-pyrazolo[1,5-a]pyridin-2-yl)-(1-methyl-6-morpholin-4-yl-3,4-dihydro-1H-isoquinolin-2-yl)-methanone

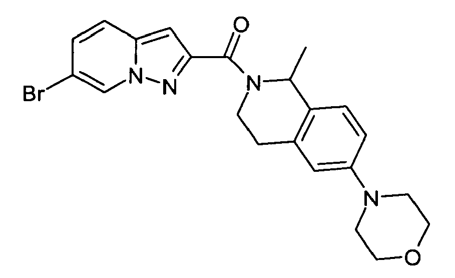

In close analogy to the procedure described in Example 1, 6-bromo-pyrazolo[1,5-a]pyridine-2-carboxylic acid is reacted with 1-methyl-6-morpholin-4-yl-1,2,3,4-tetrahydro-isoquinoline to provide the title compound in moderate yield.
LC/MS: m/z = 456 (MH⁺)
The stereo-isomers of this compound are separated. The S-configurated compound has a different activity than the R-configurated compound.

### Example 411

### (6-Chloro-pyrazolo[1,5-a]pyridin-2-yl)-(1-methyl-6-morpholin-4-yl-3,4-dihydro-1H-isoquinolin-2-yl)-methanone

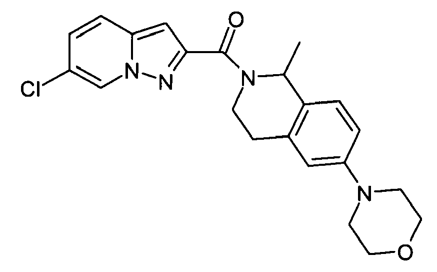

In close analogy to the procedure described in Example 1, 6-chloro-pyrazolo[1,5-a]pyridine-2-carboxylic acid is reacted with 1-methyl-6-morpholin-4-yl-1,2,3,4-tetrahydro-isoquinoline to provide the title compound in moderate yield.
LC/MS: m/z = 411 (MH⁺)
The stereo-isomers of this compound are separated. The S-configurated compound has a different activity than the R-configurated compound.

### Example 412

### (6-Bromo-pyrazolo[1,5-a]pyridin-2-yl)-(1-methyl-5-morpholin-4-yl-3,4-dihydro-1H-isoquinolin-2-yl)-methanone

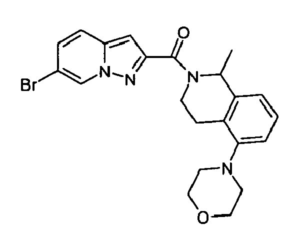

In close analogy to the procedure described in Example 1, 6-bromo-pyrazolo[1,5-a]pyridine-2-carboxylic acid is reacted with 1-methyl-5-morpholin-4-yl-1,2,3,4-tetrahydro-isoquinoline to provide the title compound in moderate yield.
LC/MS: m/z = 456 (MH⁺)
The stereo-isomers of this compound are separated. The S-configurated compound has a different activity than the R-configurated compound.

### Example 413

### (6-Chloro-pyrazolo[1,5-a]pyridin-2-yl)-(1-methyl-5-morpholin-4-yl-3,4-dihydro-1H-isoquinolin-2-yl)-methanone

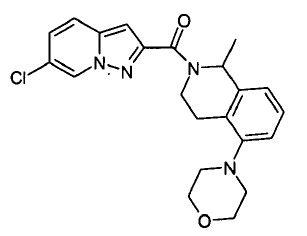

In close analogy to the procedure described in Example 1, 6-chloro-pyrazolo[1,5-a]pyridine-2-carboxylic acid is reacted with 1-methyl-5-morpholin-4-yl-1,2,3,4-tetrahydro-isoquinoline to provide the title compound in moderate yield.
LC/MS: m/z = 411 (MH⁺)
The stereo-isomers of this compound are separated. The S-configurated compound has a different activity than the R-configurated compound.

### Example 414

### (6-Bromo-pyrazolo[1,5-a]pyridin-2-yl)-(1-methyl-7-piperidin-1-yl-3,4-dihydro-1H-isoquinolin-2-yl)-methanone

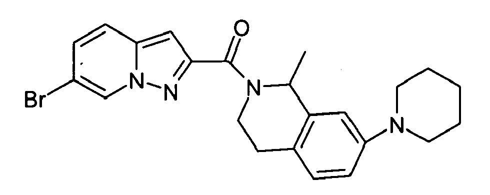

In close analogy to the procedure described in Example 1, 6-bromo-pyrazolo[1,5-a]pyridine-2-carboxylic acid is reacted 1-methyl-7-piperidin-1-yl-1,2,3,4-tetrahydro-isoquinoline to provide the title compound in moderate yield.
LC/MS: m/z = 455 (MH⁺)
The stereo-isomers of this compound are separated. The S-configurated compound has a different activity than the R-configurated compound.

### Example 415

### (6-Chloro-pyrazolo[1,5-a]pyridin-2-yl)-(1-methyl-7-piperidin-1-yl-3,4-dihydro-1H-isoquinolin-2-yl)-methanone

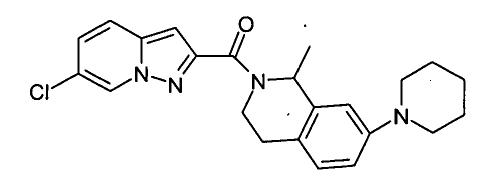

In close analogy to the procedure described in Example 1, 6-bromo-pyrazolo[1,5-a]pyridine-2-carboxylic acid is reacted 1-methyl-7-piperidin-1-yl-1,2,3,4-tetrahydro-isoquinoline to provide the title compound in moderate yield.
LC/MS: m/z = 409 (MH⁺)
The stereo-isomers of this compound are separated. The S-configurated compound has a different activity than the R-configurated compound.

### Example 416

### (6-Bromo-pyrazolo[1,5-a]pyridin-2-yl)-(1-methyl-7-pyrrolidin-1-yl-3,4-dihydro-1H-isoquinolin-2-yl)-methanone

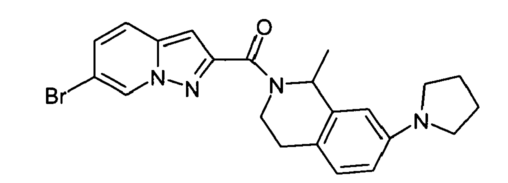

In close analogy to the procedure described in Example 1, 6-bromo-pyrazolo[1,5-a]pyridine-2-carboxylic acid is reacted 1-methyl-7-pyrrolidin-1-yl-1,2,3,4-tetrahydro-isoquinoline to provide the title compound in moderate yield.
LC/MS: m/z = 440 (MH⁺)
The stereo-isomers of this compound are separated. The S-configurated compound has a different activity than the R-configurated compound.

### Example 417

### (6-Chloro-pyrazolo[1,5-a]pyridin-2-yl)-(1-methyl-7-pyrrolidin-1-yl-3,4-dihydro-1H-isoquinolin-2-yl)-methanone

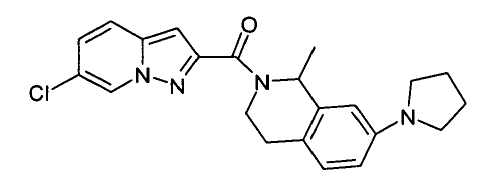

In close analogy to the procedure described in Example 1, 6-chloro-pyrazolo[1,5-a]pyridine-2-carboxylic acid is reacted 1-methyl-7-pyrrolidin-1-yl-1,2,3,4-tetrahydro-isoquinoline to provide the title compound in moderate yield.
LC/MS: m/z = 395 (MH⁺)
The stereo-isomers of this compound are separated. The S-configurated compound has a different activity than the R-configurated compound.

### Example 418

### (6-Chloro-pyrazolo[1,5-a]pyridin-2-yl)-(1-methyl-3,4-dihydro-1H-pyrrolo[1,2-a]pyrazin-2-yl)-methanone

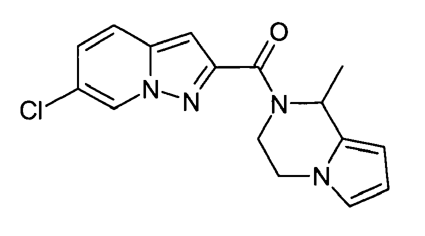

In close analogy to the procedure described in Example 1, 6-chloro-pyrazolo[1,5-a]pyridine-2-carboxylic acid is reacted with 1-methyl-1,2,3,4-tetrahydro-pyrrolo[1,2-a]pyrazine to provide the title compound in good to moderate yield in good to moderate yield.
LC/MS: m/z = 316 (MH⁺)

The stereo-isomers of this compound are separated. The S-configurated compound has a different activity than the R-configurated compound.

### Example 419

### (6-Chloro-pyrazolo[1,5-a]pyridin-2-yl)-(3,4-dihydro-1H-pyrrolo[1,2-a]pyrazin-2-yl)-methanone

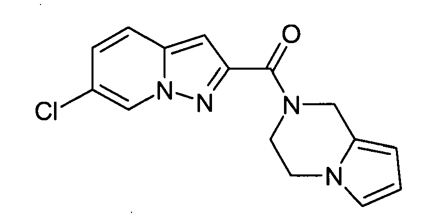

In close analogy to the procedure described in Example 1, 6-chloro-pyrazolo[1,5-a]pyridine-2-carboxylic acid is reacted with 1,2,3,4-tetrahydro-pyrrolo[1,2-a]pyrazine to provide the title compound in good to moderate yield in good to moderate yield.
LC/MS: m/z = 302 (MH⁺)

### Example 420

### (6-Bromo-pyrazolo[1,5-a]pyridin-2-yl)-(1-methyl-3,4-dihydro-1H-pyrrolo[1,2-a]pyrazin-2-yl)-methanone

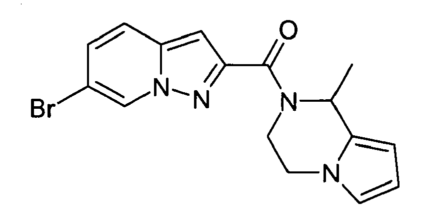

In close analogy to the procedure described in Example 1, 6-bromo-pyrazolo[1,5-a]pyridine-2-carboxylic acid is reacted with 1-methyl-1,2,3,4-tetrahydro-pyrrolo[1,2-a]pyrazine to provide the title compound in good to moderate yield in good to moderate yield.
LC/MS: m/z = 360 (MH⁺)

The stereo-isomers of this compound are separated. The S-configurated compound has a different activity than the R-configurated compound.

### Example 421

### (6-Bromo-pyrazolo[1,5-a]pyridin-2-yl)-(3,4-dihydro-1H-pyrrolo[1,2-a]pyrazin-2-yl)-methanone

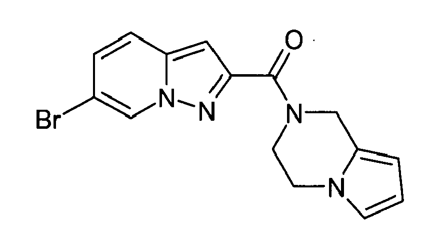

In close analogy to the procedure described in Example 1, 6-bromo-pyrazolo[1,5-a]pyridine-2-carboxylic acid is reacted with 1,2,3,4-tetrahydro-pyrrolo[1,2-a]pyrazine to provide the title compound in good to moderate yield in good to moderate yield.
LC/MS: m/z = 346 (MH⁺)

### Example 422

### (6-Chloro-pyrazolo[1,5-a]pyridin-2-yl)-(1,6-dimethyl-3,4-dihydro-1H-pymolo[1,2-a]pyrazin-2-yl)-methanone

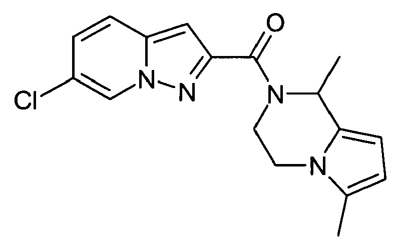

In close analogy to the procedure described in Example 1, 6-chloro-pyrazolo[1,5-a]pyridine-2-carboxylic acid is reacted with 1,6-dimethyl-1,2,3,4-tetrahydro-pyrrolo[1,2-a]pyrazine to provide the title compound in good to moderate yield in good to moderate yield.
LC/MS: m/z = 330 (MH⁺)
The stereo-isomers of this compound are separated. The S-configurated compound has a different activity than the R-configurated compound.

### Example 423

### (6-Chloro-pyrazolo[1,5-a]pyridin-2-yl)-(6-methyl-3,4-dihydro-1H-pyrrolo[1,2-a]pyrazin-2-yl)-methanone

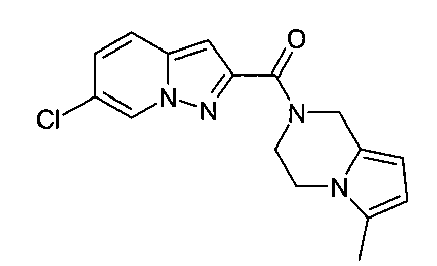

In close analogy to the procedure described in Example 1, 6-chloro-pyrazolo[1,5-a]pyridine-2-carboxylic acid is reacted with 6-methyl-1,2,3,4-tetrahydro-pyrrolo[1,2-a]pyrazine to provide the title compound in good to moderate yield in good to moderate yield.
LC/MS: m/z = 316 (MH⁺)

### Example 424

### (6-Bromo-pyrazolo[1,5-a]pyridin-2-yl)-(1,6-dimethyl-3,4-dihydro-1H-pyrrolo[1,2-a]pyrazin-2-yl)-methanone

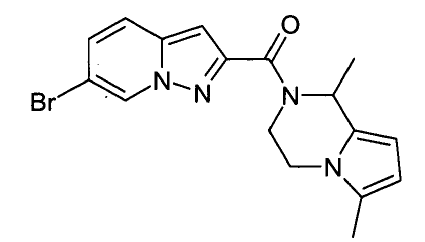

In close analogy to the procedure described in Example 1, 6-bromo-pyrazolo[1,5-a]pyridine-2-carboxylic acid is reacted with 1,6-dimethyl-1,2,3,4-tetrahydro-pyrrolo[1,2-a]pyrazine to provide the title compound in good to moderate yield in good to moderate yield.
LC/MS: m/z = 375 (MH⁺)
The stereo-isomers of this compound are separated. The S-configurated compound has a different activity than the R-configurated compound.

### Example 425

### (6-Bromo-pyrazolo[1,5-a]pyridin-2-yl)-(6-methyl-3,4-dihydro-1H-pyrrolo[1,2-a]pyrazin-2-yl)-methanone

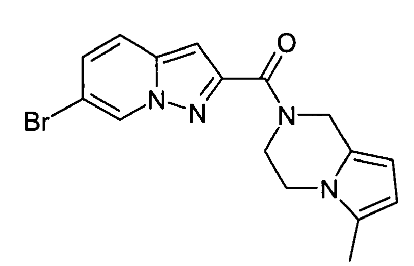

In close analogy to the procedure described in Example 1, 6-bromo-pyrazolo[1,5-a]pyridine-2-carboxylic acid is reacted with 6-methyl-1,2,3,4-tetrahydro-pyrrolo[1,2-a]pyrazine to provide the title compound in good to moderate yield in good to moderate yield.
LC/MS: m/z = 331 (MH⁺)

### Example 426

### (6-Chloro-pyrazolo[1,5-a]pyridin-2-yl)-(4-methyl-6,7-dihydro-4H-[1,2,3]triazolo[1,5-a]pyrazin-5-yl)-methanone

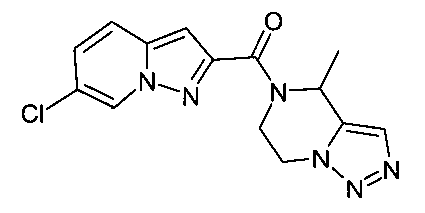

In close analogy to the procedure described in Example 1, 6-chloro-pyrazolo[1,5-a]pyridine-2-carboxylic acid is reacted with 4-methyl-4,5,6,7-tetrahydro-[1,2,3]triazolo[1,5-a]pyrazine to provide the title compound in good to moderate yield in good to moderate yield.
LC/MS: m/z = 318 (MH⁺)
The stereo-isomers of this compound are separated. The S-configurated compound has a different activity than the R-configurated compound.

### Example 427

### (6-Chloro-pyrazolo[1,5-a]pyridin-2-yl)-(6,7-dihydro-4H-[1,2,3]triazolo[1,5-a]pyrazin-5-yl)-methanone

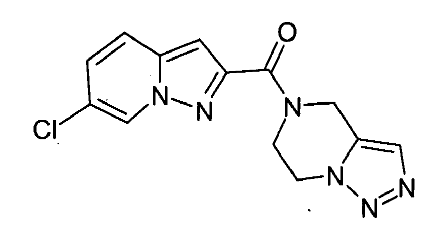

In close analogy to the procedure described in Example 1, 6-chloro-pyrazolo[1,5-a]pyridine-2-carboxylic acid is reacted with 4,5,6,7-tetrahydro-[1,2,3]triazolo[1,5-a]pyrazine to provide the title compound in good to moderate yield in good to moderate yield.
LC/MS: m/z = 304 (MH⁺)

### Example 428

### (6-Bromo-pyrazolo[1,5-a]pyridin-2-yl)-(4-methyl-6,7-dihydro-4H-[1,2,3]triazolo[1,5-a]pyrazin-5-yl)-methanone

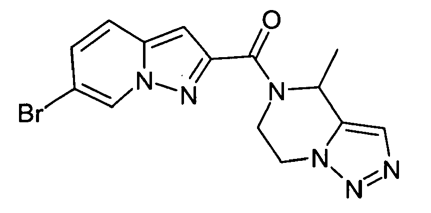

In close analogy to the procedure described in Example 1, 6-bromo-pyrazolo[1,5-a]pyridine-2-carboxylic acid is reacted with 4-methyl-4,5,6,7-tetrahydro-[1,2,3]triazolo[1,5-a]pyrazine to provide the title compound in good to moderate yield in good to moderate yield.
LC/MS: m/z = 362 (MH⁺)
The stereo-isomers of this compound are separated. The S-configurated compound has a different activity than the R-configurated compound.

### Example 429

### (6-Bromo-pyrazolo[1,5-a]pyridin-2-yl)-(6,7-dihydro-4H-[1,2,3]triazolo[1,5-a]pyrazin-5-yl)-methanone

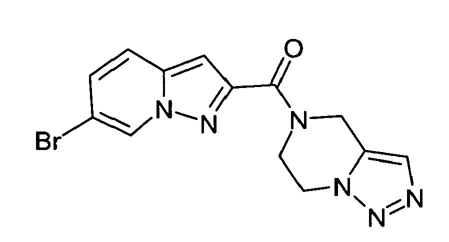

In close analogy to the procedure described in Example 1, 6-bromo-pyrazolo[1,5-a]pyridine-2-carboxylic acid is reacted with 4,5,6,7-tetrahydro-[1,2,3]triazolo[1,5-a]pyrazine to provide the title compound in good to moderate yield in good to moderate yield.
LC/MS: m/z = 348 (MH⁺)

### Example 430

### 7-(6-Chloro-pyrazolo[1,5-a]pyridine-2-carbonyl)-8-methyl-5,6,7,8-tetrahydro-imidazo[1,2-a]pyrazine-2-carboxylic acid ethyl ester

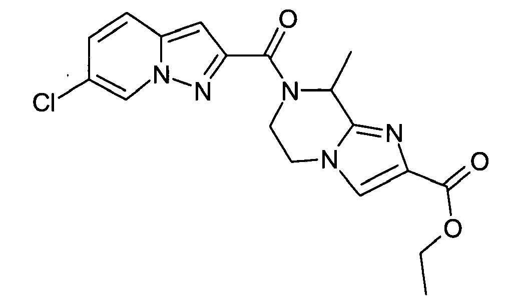

In close analogy to the procedure described in Example 1, 6-chloro-pyrazolo[1,5-a]pyridine-2-carboxylic acid is reacted with 8-methyl-5,6,7,8-tetrahydro-imidazo[1,2-a]pyrazine-2-carboxylic acid ethyl ester to provide the title compound in good to moderate yield in good to moderate yield.
LC/MS: m/z = 389 (MH⁺)
The stereo-isomers of this compound are separated. The S-configurated compound has a different activity than the R-configurated compound.

### Example 431

### 7-(6-Chloro-pyrazolo[1,5-a]pyridine-2-carbonyl)-5,6,7,8-tetrahydro-imidazo[1,2-a]pyrazine-2-carboxylic acid ethyl ester

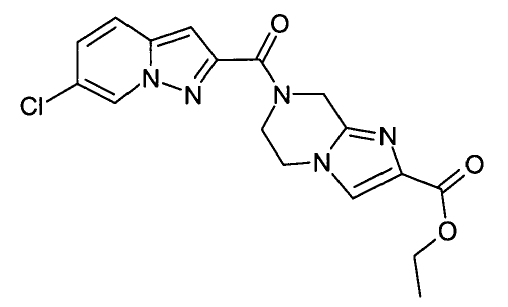

In close analogy to the procedure described in Example 1, 6-chloro-pyrazolo[1,5-a]pyridine-2-carboxylic acid is reacted with 5,6,7,8-tetrahydro-imidazo[1,2-a]pyrazine-2-carboxylic acid ethyl ester to provide the title compound in good to moderate yield in good to moderate yield.
LC/MS: m/z = 375 (MH⁺)

### Example 432

### 7-(6-Bromo-pyrazolo[1,5-a]pyridine-2-carbonyl)-8-methyl-5,6,7,8-tetrahydro-imidazo[1,2-a]pyrazine-2-carboxylic acid ethyl ester

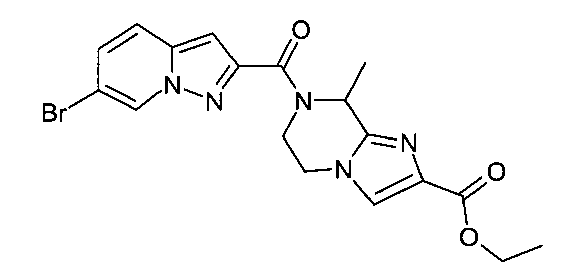

In close analogy to the procedure described in Example 1, 6-bromo-pyrazolo[1,5-a]pyridine-2-carboxylic acid is reacted with 8-methyl-5,6,7,8-tetrahydro-imidazo[1,2-a]pyrazine-2-carboxylic acid ethyl ester to provide the title compound in good to moderate yield in good to moderate yield.
LC/MS: m/z = 433 (MH⁺)
The stereo-isomers of this compound are separated. The S-configurated compound has a different activity than the R-configurated compound.

### Example 433

### 7-(6-Bromo-pyrazolo[1,5-a]pyridine-2-carbonyl)-5,6,7,8-tetrahydro-imidazo[1,2-a]pyrazine-2-carboxylic acid ethyl ester

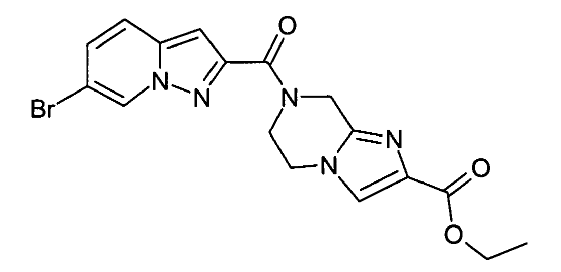

In close analogy to the procedure described in Example 1, 6-bromo-pyrazolo[1,5-a]pyridine-2-carboxylic acid is reacted with 5,6,7,8-tetrahydro-imidazo[1,2-a]pyrazine-2-carboxylic acid ethyl ester to provide the title compound in good to moderate yield.
LC/MS: m/z = 319 (MH⁺)

### Example 434

### 7-(6-Chloro-pyrazolo[1,5-a]pyridine-2-carbonyl)-8-methyl-5,6,7,8-tetrahydro-[1,2,4]triazolo[4,3-a]pyrazine-3-carboxylic acid ethyl ester

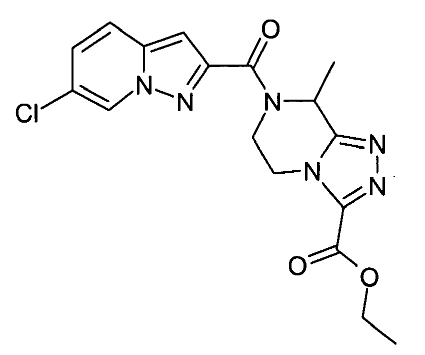

In close analogy to the procedure described in Example 1, 6-chloro-pyrazolo[1,5-a]pyridine-2-carboxylic acid is reacted with 8-methyl-5,6,7,8-tetrahydro-[1,2,4]triazolo[4,3-a]pyrazine-3-carboxylic acid ethyl ester to provide the title compound in good to moderate yield.
LC/MS: m/z = 390 (MH⁺)
The stereo-isomers of this compound are separated. The S-configurated compound has a different activity than the R-configurated compound.

### Example 435

### 7-(6-Chloro-pyrazolo[1,5-a]pyridine-2-carbonyl)-5,6,7,8-tetrahydro-[1,2,4]triazolo[4,3-a]pyrazine-3-carboxylic acid ethyl ester

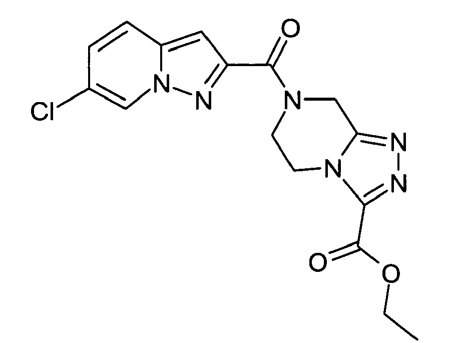

In close analogy to the procedure described in Example 1, 6-chloro-pyrazolo[1,5-a]pyridine-2-carboxylic acid is reacted with 5,6,7,8-tetrahydro-[1,2,4]triazolo[4,3-a]pyrazine-3-carboxylic acid ethyl ester to provide the title compound in good to moderate yield.
LC/MS: m/z = 376 (MH⁺)

### Example 436

### 7-(6-Bromo-pyrazolo[1,5-a]pyridine-2-carbonyl)-8-methyl-5,6,7,8-tetrahydro-[1,2,4]triazolo[4,3-a]pyrazine-3-carboxylic acid ethyl ester

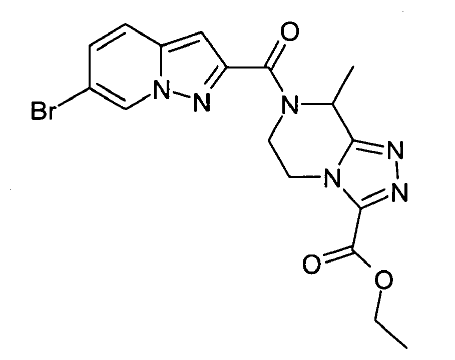

In close analogy to the procedure described in Example 1, 6-bromo-pyrazolo[1,5-a]pyridine-2-carboxylic acid is reacted with 8-methyl-5,6,7,8-tetrahydro-[1,2,4]triazolo[4,3-a]pyrazine-3-carboxylic acid ethyl ester to provide the title compound in good to moderate yield.
LC/MS: m/z = 434 (MH⁺)
The stereo-isomers of this compound are separated. The S-configurated compound has a different activity than the R-configurated compound.

### Example 437

### 7-(6-Bromo-pyrazolo[1,5-a]pyridine-2-carbonyl)-5,6,7,8-tetrahydro-[1,2,4]triazolo[4,3-a]pyrazine-3-carboxylic acid ethyl ester

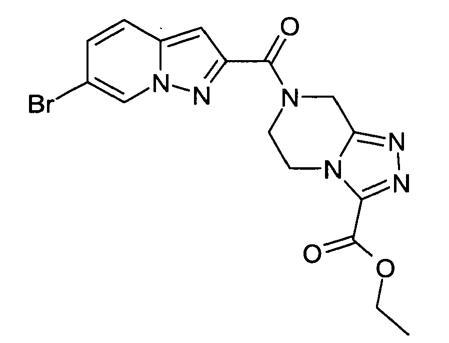

In close analogy to the procedure described in Example 1, 6-bromo-pyrazolo[1,5-a]pyridine-2-carboxylic acid is reacted with 5,6,7,8-tetrahydro-[1,2,4]triazolo[4,3-a]pyrazine-3-carboxylic acid ethyl ester to provide the title compound in good to moderate yield.
LC/MS: m/z = 320 (MH⁺)

### Example 438

### (3-Bromo-8-methyl-5,6-dihydro-8H-[1,2,4]triazolo[4,3-a]pyrazin-7-yl)-(6-chloro-pyrazolo[1,5-a]pyridin-2-yl)-methanone

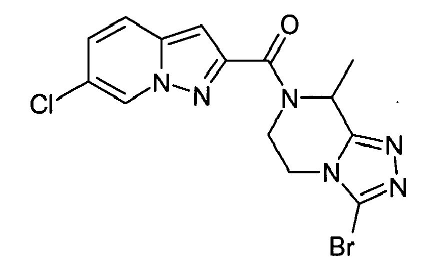

In close analogy to the procedure described in Example 1, 6-chloro-pyrazolo[1,5-a]pyridine-2-carboxylic acid is reacted with 3-bromo-8-methyl-5,6,7,8-tetrahydro-[1,2,4]triazolo[4,3-a]pyrazine to provide the title compound in good to moderate yield.
LC/MS: m/z = 396 (MH⁺)
The stereo-isomers of this compound are separated. The S-configurated compound has a different activity than the R-configurated compound.

### Example 439

### (3-Bromo-5,6-dihydro-8H-[1,2,4]triazolo[4,3-a]pyrazin-7-yl)-(6-chloro-pyrazolo[1,5-a]pyridin-2-yl)-methanone

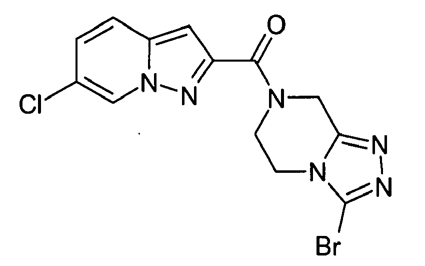

In close analogy to the procedure described in Example 1, 6-chloro-pyrazolo[1,5-a]pyridine-2-carboxylic acid is reacted with 3-bromo-5,6,7,8-tetrahydro-[1,2,4]triazolo[4,3-a]pyrazine to provide the title compound in good to moderate yield.
LC/MS: m/z = 382 (MH⁺)

### Example 440

### (3-Bromo-8-methyl-5,6-dihydro-8H-[1,2,4]triazolo[4,3-a]pyrazin-7-yl)-(6-bromo-pyrazolo[1,5-a]pyridin-2-yl)-methanone

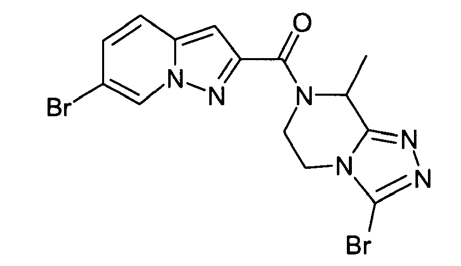

In close analogy to the procedure described in Example 1, 6-bromo-pyrazolo[1,5-a]pyridine-2-carboxylic acid is reacted with 3-bromo-8-methyl-5,6,7,8-tetrahydro-[1,2,4]triazolo[4,3-a]pyrazine to provide the title compound in good to moderate yield.
LC/MS: m/z = 441 (MH⁺)
The stereo-isomers of this compound are separated. The S-configurated compound has a different activity than the R-configurated compound.

### Example 441

### (3-Bromo-5,6-dihydro-8H-[1,2,4]triazolo[4,3-a]pyrazin-7-yl)-(6-bromo-pyrazolo[1,5-a]pyridin-2-yl)-methanone

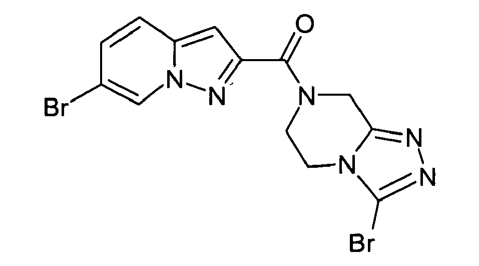

In close analogy to the procedure described in Example 1, 6-bromo-pyrazolo[1,5-a]pyridine-2-carboxylic acid is reacted with 3-bromo-5,6,7,8-tetrahydro-[1,2,4]triazolo[4,3-a]pyrazine to provide the title compound in good to moderate yield.
LC/MS: m/z = 427 (MH⁺)

### Example 442

### (6-Chloro-pyrazolo[1,5-a]pyridin-2-yl)-(8-methyl-5,6-dihydro-8H-[1,2,4]triazolo[4,3-a]pyrazin-7-yl)-methanone

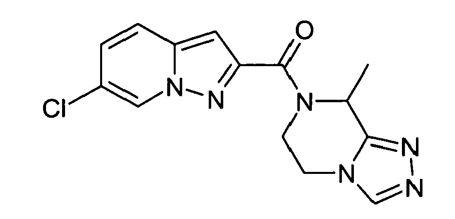

In close analogy to the procedure described in Example 1, 6-chloro-pyrazolo[1,5-a]pyridine-2-carboxylic acid is reacted with 8-methyl-5,6,7,8-tetrahydro-[1,2,4]triazolo[4,3-a]pyrazine to provide the title compound in good to moderate yield.
LC/MS: m/z = 318 (MH⁺)
The stereo-isomers of this compound are separated. The S-configurated compound has a different activity than the R-configurated compound.

### Example 443

### (6-Chloro-pyrazolo[1,5-a]pyridin-2-yl)-(5,6-dihydro-8H-[1,2,4]triazolo[4,3-a]pyrazin-7-yl)-methanone

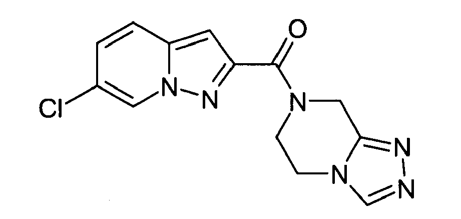

In close analogy to the procedure described in Example 1, 6-chloro-pyrazolo[1,5-a]pyridine-2-carboxylic acid is reacted with 5,6,7,8-tetrahydro-[1,2,4]triazolo[4,3-a]pyrazine to provide the title compound in good to moderate yield.
LC/MS: m/z = 304 (MH⁺)

### Example 444

### (6-Bromo-pyrazolo[1,5-a]pyridin-2-yl)-(8-methyl-5,6-dihydro-8H-[1,2,4]triazolo[4,3-a]pyrazin-7-yl)-methanone

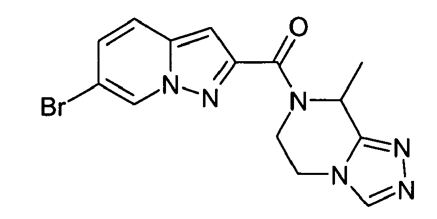

In close analogy to the procedure described in Example 1, 6-bromo-pyrazolo[1,5-a]pyridine-2-carboxylic acid is reacted with 8-methyl-5,6,7,8-tetrahydro-[1,2,4]triazolo[4,3-a]pyrazine to provide the title compound in good to moderate yield.
LC/MS: m/z = 362 (MH⁺)
The stereo-isomers of this compound are separated. The S-configurated compound has a different activity than the R-configurated compound.

### Example 445

### (6-Bromo-pyrazolo[1,5-a]pyridin-2-yl)-(5,6-dihydro-8H-[1,2,4]triazolo[4,3-a]pyrazin-7-yl)-methanone

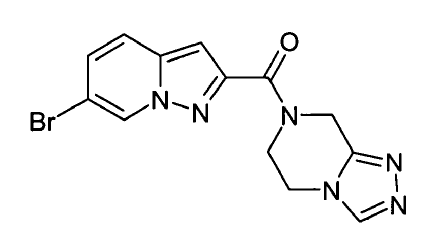

In close analogy to the procedure described in Example 1, 6-bromo-pyrazolo[1,5-a]pyridine-2-carboxylic acid is reacted with 5,6,7,8-tetrahydro-[1,2,4]triazolo[4,3-a]pyrazine to provide the title compound in good to moderate yield.
LC/MS: m/z = 348 (MH⁺)

### Example 446

### (6-Chloro-1-methyl-3,4-dihydro-1H-pyrrolo[1,2-a]pyrazin-2-yl)-(6-chloro-pyrazolo[1,5-a]pyridin-2-yl)-methanone

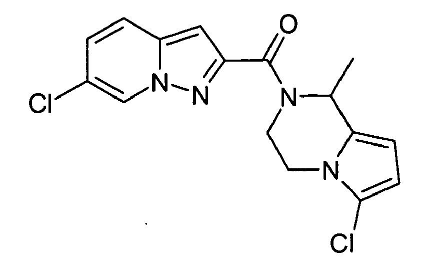

In close analogy to the procedure described in Example 1, 6-chloro-pyrazolo[1,5-a]pyridine-2-carboxylic acid is reacted with 6-chloro-1-methyl-1,2,3,4-tetrahydro-pyrrolo[1,2-a]pyrazine to provide the title compound in good to moderate yield.
LC/MS: m/z = 350 (MH⁺)
The stereo-isomers of this compound are separated. The S-configurated compound has a different activity than the R-configurated compound.

### Example 447

### (6-Chloro-3,4-dihydro-1H-pyrrolo[1,2-a]pyrazin-2-yl)-(6-chloro-pyrazolo[1,5-a]pyridin-2-yl)-methanone

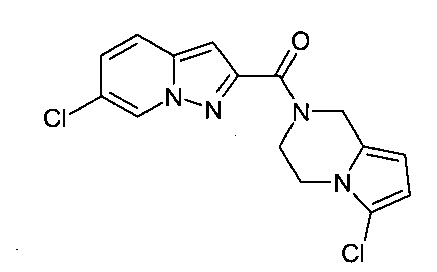

In close analogy to the procedure described in Example 1, 6-chloro-pyrazolo[1,5-a]pyridine-2-carboxylic acid is reacted with 6-chloro-1,2,3,4-tetrahydro-pyrrolo[1,2-a]pyrazine to provide the title compound in good to moderate yield.
LC/MS: m/z = 336 (MH⁺)

### Example 448

### (6-Chloro-1-methyl-3,4-dihydro-1H-pyrrolo[1,2-a]pyrazin-2-yl)-(6-bromo-pyrazolo[1,5-a]pyridin-2-yl)-methanone

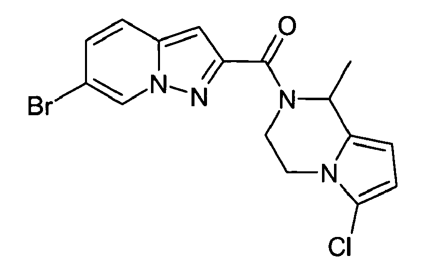

In close analogy to the procedure described in Example 1, 6-bromo-pyrazolo[1,5-a]pyridine-2-carboxylic acid is reacted with 6-chloro-1-methyl-1,2,3,4-tetrahydro-pyrrolo[1,2-a]pyrazine to provide the title compound in good to moderate yield.
LC/MS: m/z = 395 (MH⁺)
The stereo-isomers of this compound are separated. The S-configurated compound has a different activity than the R-configurated compound.

### Example 449

### (6-Chloro-3,4-dihydro-1H-pyrrolo[1,2-a]pyrazin-2-yl)-(6-bromo-pyrazolo[1,5-a]pyridin-2-yl)-methanone

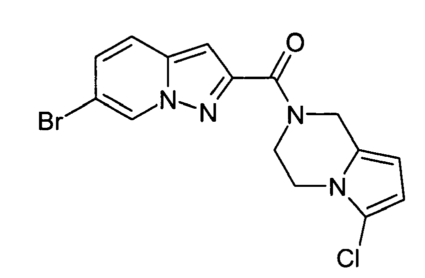

In close analogy to the procedure described in Example 1, 6-bromo-pyrazolo[1,5-a]pyridine-2-carboxylic acid is reacted with 6-chloro-1,2,3,4-tetrahydro-pyrrolo[1,2-a]pyrazine to provide the title compound in good to moderate yield.
LC/MS: m/z = 381 (MH⁺)

### Example 450

### (6-Chloro-pyrazolo[1,5-a]pyridin-2-yl)-(6,7-dibromo-1-methyl-3,4-dihydro-1H-pyrrolo[1,2-a]pyrazin-2-yl)-methanone

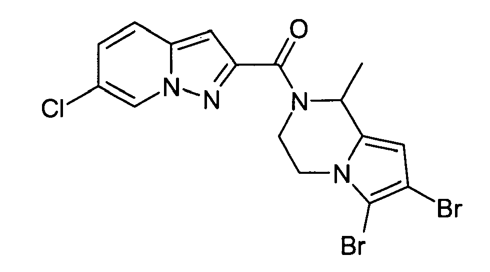

In close analogy to the procedure described in Example 1, 6-chloro-pyrazolo[1,5-a]pyridine-2-carboxylic acid is reacted with 6,7-dibromo-1-methyl-1,2,3,4-tetrahydro-pyrrolo[1,2-a]pyrazine to provide the title compound in good to moderate yield.
LC/MS: m/z = 473 (MH⁺)
The stereo-isomers of this compound are separated. The S-configurated compound has a different activity than the R-configurated compound.

### Example 451

### (6-Chloro-pyrazolo[1,5-a]pyridin-2-yl)-(6,7-dibromo-3,4-dihydro-1H-pyrrolo[1,2-a]pyrazin-2-yl)-methanone

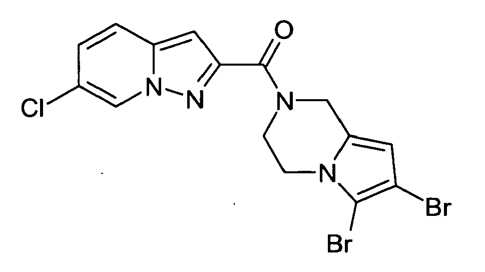

In close analogy to the procedure described in Example 1, 6-chloro-pyrazolo[1,5-a]pyridine-2-carboxylic acid is reacted with 6,7-dibromo-1,2,3,4-tetrahydro-pyrrolo[1,2-a]pyrazine to provide the title compound in good to moderate yield.
LC/MS: m/z = 459 (MH⁺)

### Example 452

### (6-Bromo-pyrazolo[1,5-a]pyridin-2-yl)-(6,7-dibromo-1-methyl-3,4-dihydro-1H-pyrrolo[1,2-a]pyrazin-2-yl)-methanone

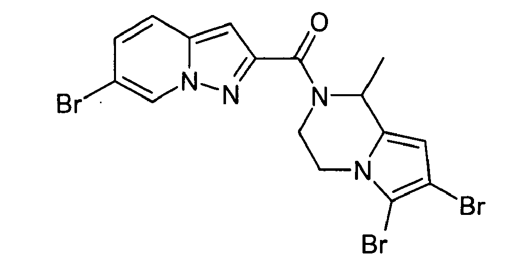

In close analogy to the procedure described in Example 1, 6-bromo-pyrazolo[1,5-a]pyridine-2-carboxylic acid is reacted with 6,7-dibromo-1-methyl-1,2,3,4-tetrahydro-pyrrolo[1,2-a]pyrazine to provide the title compound in good to moderate yield.
LC/MS: m/z = 518 (MH⁺)
The stereo-isomers of this compound are separated. The S-configurated compound has a different activity than the R-configurated compound.

### Example 453

### (6-Bromo-pyrazolo[1,5-a]pyridin-2-yl)-(6,7-dibromo-3,4-dihydro-1H-pyrrolo[1,2-a]pyrazin-2-yl)-methanone

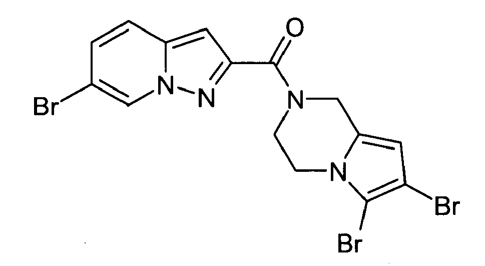

In close analogy to the procedure described in Example 1, 6-bromo-pyrazolo[1,5-a]pyridine-2-carboxylic acid is reacted with 6,7-dibromo-1,2,3,4-tetrahydro-pyrrolo[1,2-a]pyrazine to provide the title compound in good to moderate yield.
LC/MS: m/z = 503 (MH⁺)

### Example 454

### (6-Bromo-1-methyl-3,4-dihydro-1H-pyrrolo[1,2-a]pyrazin-2-yl)-(6-chloro-pyrazolo[1,5-a]pyridin-2-yl)-methanone

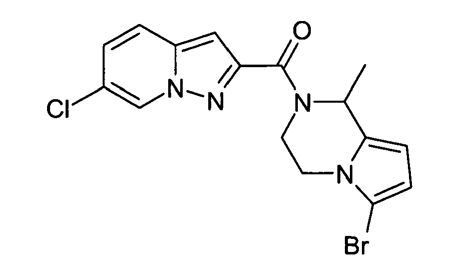

In close analogy to the procedure described in Example 1, 6-chloro-pyrazolo[1,5-a]pyridine-2-carboxylic acid is reacted with 6-bromo-1-methyl-1,2,3,4-tetrahydro-pyrrolo[1,2-a]pyrazine to provide the title compound in good to moderate yield.
LC/MS: m/z = 395 (MH⁺)
The stereo-isomers of this compound are separated. The S-configurated compound has a different activity than the R-configurated compound.

### Example 455

### (6-Bromo-3,4-dihydro-1H-pyrrolo[1,2-a]pyrazin-2-yl)-(6-chloro-pyrazolo[1,5-a]pyridin-2-yl)-methanone

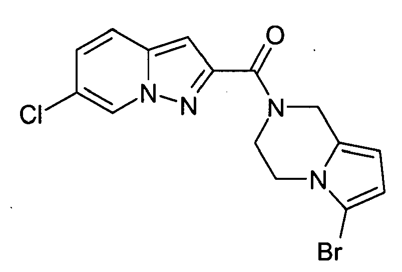

In close analogy to the procedure described in Example 1, 6-chloro-pyrazolo[1,5-a]pyridine-2-carboxylic acid is reacted with 6-bromo-1,2,3,4-tetrahydro-pyrrolo[1,2-a]pyrazine to provide the title compound in good to moderate yield.
LC/MS: m/z = 381 (MH⁺)

### Example 456

### (6-Bromo-1-methyl-3,4-dihydro-1H-pyrrolo[1,2-a]pyrazin-2-yl)-(6-bromo-pyrazolo[1,5-a]pyridin-2-yl)-methanone

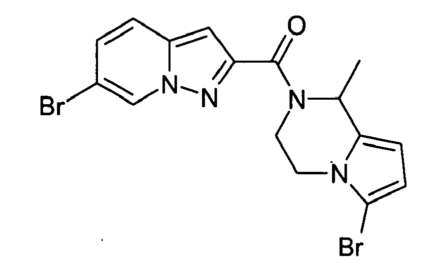

In close analogy to the procedure described in Example 1, 6-bromo-pyrazolo[1,5-a]pyridine-2-carboxylic acid is reacted with 6-bromo-1-methyl-1,2,3,4-tetrahydro-pyrrolo[1,2-a]pyrazine to provide the title compound in good to moderate yield.
LC/MS: m/z = 439 (MH⁺)
The stereo-isomers of this compound are separated. The S-configurated compound has a different activity than the R-configurated compound.

### Example 457

### (6-Bromo-3,4-dihydro-1H-pyrrolo[1,2-a]pyrazin-2-yl)-(6-bromo-pyrazolo[1,5-a]pyridin-2-yl)-methanone

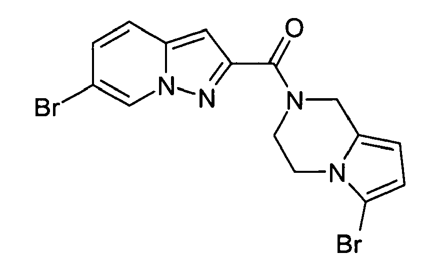

In close analogy to the procedure described in Example 1, 6-bromo-pyrazolo[1,5-a]pyridine-2-carboxylic acid is reacted with 6-bromo-1,2,3,4-tetrahydro-pyrrolo[1,2-a]pyrazine to provide the title compound in good to moderate yield.
LC/MS: m/z = 425 (MH⁺)

### Example 458

### (6-Chloro-pyrazolo[1,5-a]pyridin-2-yl)-(4-methyl-6,7-dihydro-4H-pyrazolo[1,5-a]pyrazin-5-yl)-methanone

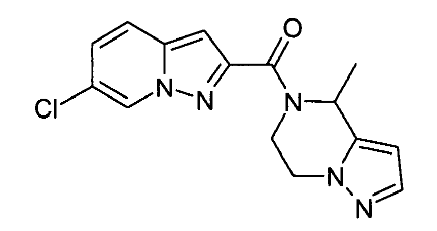

In close analogy to the procedure described in Example 1, 6-chloro-pyrazolo[1,5-a]pyridine-2-carboxylic acid is reacted with 4-methyl-4,5,6,7-tetrahydro-pyrazolo[1,5-a]pyrazine to provide the title compound in good to moderate yield.
LC/MS: m/z = 317 (MH⁺)
The stereo-isomers of this compound are separated. The S-configurated compound has a different activity than the R-configurated compound.

### Example 459

### (6-Chloro-pyrazolo[1,5-a]pyridin-2-yl)-(6,7-dihydro-4H-pyrazolo[1,5-a]pyrazin-5-yl)-methanone

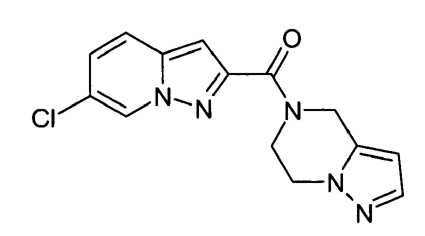

In close analogy to the procedure described in Example 1, 6-chloro-pyrazolo[1,5-a]pyridine-2-carboxylic acid is reacted with 4,5,6,7-tetrahydro-pyrazolo[1,5-a]pyrazine to provide the title compound in good to moderate yield.
LC/MS: m/z = 303 (MH⁺)

### Example 460

### (6-Bromo-pyrazolo[1,5-a]pyridin-2-yl)-(4-methyl-6,7-dihydro-4H-pyrazolo[1,5-a]pyrazin-5-yl)-methanone

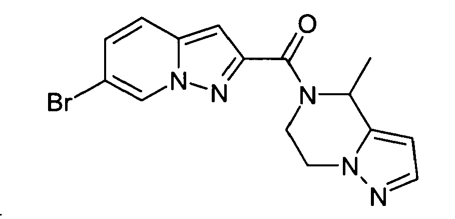

In close analogy to the procedure described in Example 1, 6-bromo-pyrazolo[1,5-a]pyridine-2-carboxylic acid is reacted with 4-methyl-4,5,6,7-tetrahydro-pyrazolo[1,5-a]pyrazine to provide the title compound in good to moderate yield.
LC/MS: m/z = 361 (MH⁺)
The stereo-isomers of this compound are separated. The S-configurated compound has a different activity than the R-configurated compound.

### Example 461

### (6-Bromo-pyrazolo[1,5-a]pyridin-2-yl)-(6,7-dihydro-4H-pyrazolo[1,5-a]pyrazin-5-yl)-methanone

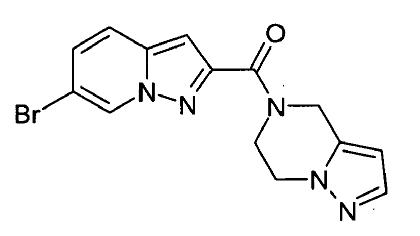

In close analogy to the procedure described in Example 1, 6-bromo-pyrazolo[1,5-a]pyridine-2-carboxylic acid is reacted with 4,5,6,7-tetrahydro-pyrazolo[1,5-a]pyrazine to provide the title compound in good to moderate yield.
LC/MS: m/z = 347 (MH⁺)

### Example 462

### (6-Chloro-pyrazolo[1,5-a]pyridin-2-yl)-(8-methyl-5,6-dihydro-8H-imidazo[1,2-a]pyrazin-7-yl)-methanone

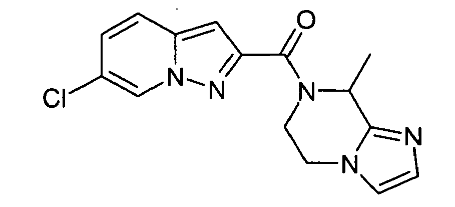

In close analogy to the procedure described in Example 1, 6-chloro-pyrazolo[1,5-a]pyridine-2-carboxylic acid is reacted with 8-methyl-5,6,7,8-tetrahydro-imidazo[1,2-a]pyrazine to provide the title compound in good to moderate yield.
LC/MS: m/z = 317 (MH⁺)
The stereo-isomers of this compound are separated. The S-configurated compound has a different activity than the R-configurated compound.

### Example 463

### (6-Chloro-pyrazolo[1,5-a]pyridin-2-yl)-(5,6-dihydro-8H-imidazo[1,2-a]pyrazin-7-yl)-methanone

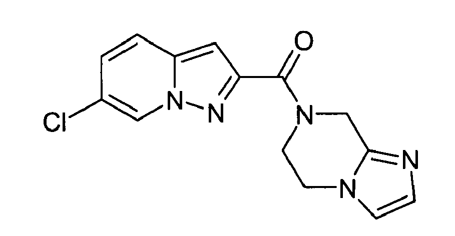

In close analogy to the procedure described in Example 1, 6-chloro-pyrazolo[1,5-a]pyridine-2-carboxylic acid is reacted with 5,6,7,8-tetrahydro-imidazo[1,2-a]pyrazine to provide the title compound in good to moderate yield.
LC/MS: m/z = 303 (MH⁺)

### Example 464

### (6-Bromo-pyrazolo[1,5-a]pyridin-2-yl)-(8-methyl-5,6-dihydro-8H-imidazo[1,2-a]pyrazin-7-yl)-methanone

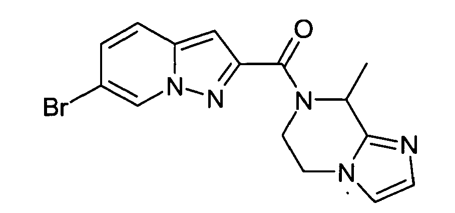

In close analogy to the procedure described in Example 1, 6-bromo-pyrazolo[1,5-a]pyridine-2-carboxylic acid is reacted with 8-methyl-5,6,7,8-tetrahydro-imidazo[1,2-a]pyrazine to provide the title compound in good to moderate yield.
LC/MS: m/z = 361 (MH⁺)
The stereo-isomers of this compound are separated. The S-configurated compound has a different activity than the R-configurated compound.

### Example 465

### (6-Bromo-pyrazolo[1,5-a]pyridin-2-yl)-(5,6-dihydro-8H-imidazo[1,2-a]pyrazin-7-yl)-methanone

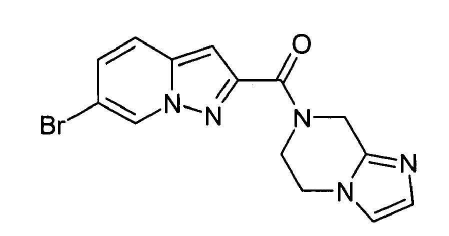

In close analogy to the procedure described in Example 1, 6-bromo-pyrazolo[1,5-a]pyridine-2-carboxylic acid is reacted with 5,6,7,8-tetrahydro-imidazo[1,2-a]pyrazine to provide the title compound in good to moderate yield.
LC/MS: m/z = 347 (MH⁺)

### Example 466

### (6-Chloro-pyrazolo[1,5-a]pyridin-2-yl)-(2,8-dimethyl-5,6-dihydro-8H-imidazo[1,2-a]pyrazin-7-yl)-methanone

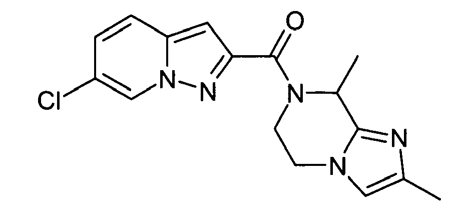

In close analogy to the procedure described in Example 1, 6-chloro-pyrazolo[1,5-a]pyridine-2-carboxylic acid is reacted with 2,8-dimethyl-5,6,7,8-tetrahydro-imidazo[1,2-a]pyrazine to provide the title compound in good to moderate yield.
LC/MS: m/z = 331 (MH⁺)
The stereo-isomers of this compound are separated. The S-configurated compound has a different activity than the R-configurated compound.

### Example 467

### (6-Chloro-pyrazolo[1,5-a]pyridin-2-yl)-(2-methyl-5,6-dihydro-8H-imidazo[1,2-a]pyrazin-7-yl)-methanone

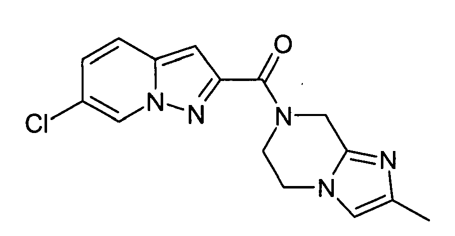

In close analogy to the procedure described in Example 1, 6-chloro-pyrazolo[1,5-a]pyridine-2-carboxylic acid is reacted with 2-methyl-5,6,7,8-tetrahydro-imidazo[1,2-a]pyrazine to provide the title compound in good to moderate yield.
LC/MS: m/z = 317 (MH⁺)

### Example 468

### (6-Bromo-pyrazolo[1,5-a]pyridin-2-yl)-(2,8-dimethyl-5,6-dihydro-8H-imidazo[1,2-a]pyrazin-7-yl)-methanone

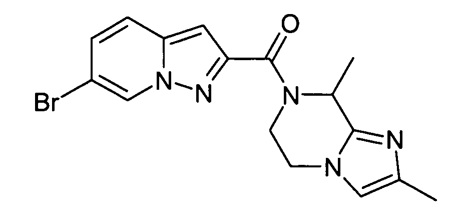

In close analogy to the procedure described in Example 1, 6-bromo-pyrazolo[1,5-a]pyridine-2-carboxylic acid is reacted with 2,8-dimethyl-5,6,7,8-tetrahydro-imidazo[1,2-a]pyrazine to provide the title compound in good to moderate yield.
LC/MS: m/z = 375 (MH⁺)
The stereo-isomers of this compound are separated. The S-configurated compound has a different activity than the R-configurated compound.

### Example 469

### (6-Bromo-pyrazolo[1,5-a]pyridin-2-yl)-(2-methyl-5,6-dihydro-8H-imidazo[1,2-a]pyrazin-7-yl)-methanone

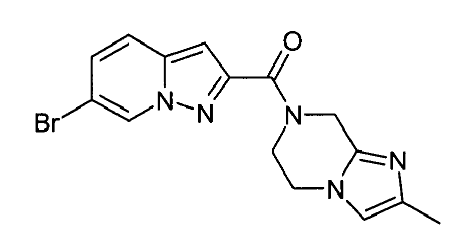

In close analogy to the procedure described in Example 1, 6-bromo-pyrazolo[1,5-a]pyridine-2-carboxylic acid is reacted with 2-methyl-5,6,7,8-tetrahydro-imidazo[1,2-a]pyrazine to provide the title compound in good to moderate yield.
LC/MS: m/z = 361 (MH⁺)

### Example 470

### (6-Chloro-pyrazolo[1,5-a]pyridin-2-yl)-(3,8-dimethyl-5,6-dihydro-8H-imidazo[1,2-a]pyrazin-7-yl)-methanone

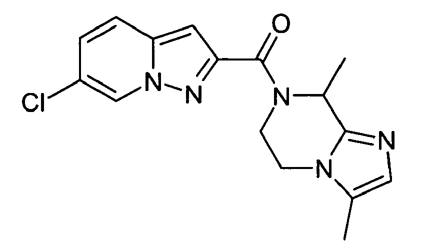

In close analogy to the procedure described in Example 1, 6-chloro-pyrazolo[1,5-a]pyridine-2-carboxylic acid is reacted with 3,8-dimethyl-5,6,7,8-tetrahydro-imidazo[1,2-a]pyrazine to provide the title compound in good to moderate yield.
LC/MS: m/z = 331 (MH⁺)
The stereo-isomers of this compound are separated. The S-configurated compound has a different activity than the R-configurated compound.

### Example 471

### (6-Chloro-pyrazolo[1,5-a]pyridin-2-yl)-(3-methyl-5,6-dihydro-8H-imidazo[1,2-a]pyrazin-7-yl)-methanone

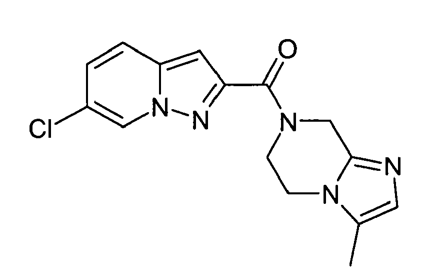

In close analogy to the procedure described in Example 1, 6-chloro-pyrazolo[1,5-a]pyridine-2-carboxylic acid is reacted with 3-methyl-5,6,7,8-tetrahydro-imidazo[1,2-a]pyrazine to provide the title compound in good to moderate yield.
LC/MS: m/z = 317 (MH⁺)

### Example 472

### (6-Bromo-pyrazolo[1,5-a]pyridin-2-yl)-(3,8-dimethyl-5,6-dihydro-8H-imidazo[1,2-a]pyrazin-7-yl)-methanone

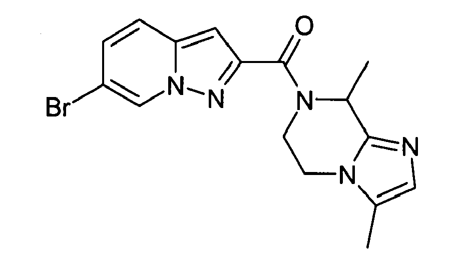

In close analogy to the procedure described in Example 1, 6-bromo-pyrazolo[1,5-a]pyridine-2-carboxylic acid is reacted with 3,8-dimethyl-5,6,7,8-tetrahydro-imidazo[1,2-a]pyrazine to provide the title compound in good to moderate yield.
LC/MS: m/z = 375 (MH⁺)
The stereo-isomers of this compound are separated. The S-configurated compound has a different activity than the R-configurated compound.

### Example 473

### (6-Bromo-pyrazolo[1,5-a]pyridin-2-yl)-(3-methyl-5,6-dihydro-8H-imidazo[1,2-a]pyrazin-7-yl)-methanone

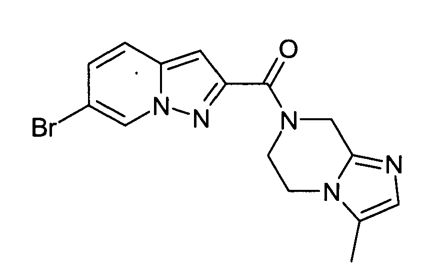

In close analogy to the procedure described in Example 1, 6-bromo-pyrazolo[1,5-a]pyridine-2-carboxylic acid is reacted with 3-methyl-5,6,7,8-tetrahydro-imidazo[1,2-a]pyrazine to provide the title compound in good to moderate yield.
LC/MS: m/z = 361 (MH⁺)

### Example 474

### (6-Chloro-pyrazolo[1,5-a]pyridin-2-yl)-(3,8-dimethyl-5,6-dihydro-8H-[1,2,4]triazolo[4,3-a]pyrazin-7-yl)-methanone

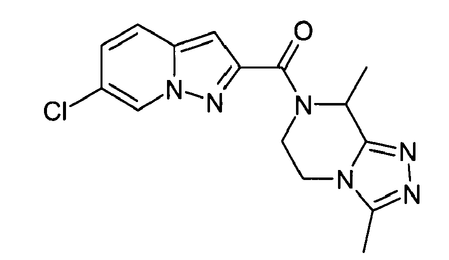

In close analogy to the procedure described in Example 1, 6-chloro-pyrazolo[1,5-a]pyridine-2-carboxylic acid is reacted with 3,8-dimethyl-5,6,7,8-tetrahydro-[1,2,4]triazolo[4,3-a]pyrazine to provide the title compound in good to moderate yield.
LC/MS: m/z = 332 (MH⁺)
The stereo-isomers of this compound are separated. The S-configurated compound has a different activity than the R-configurated compound.

### Example 475

### (6-Chloro-pyrazolo[1,5-a]pyridin-2-yl)-(3-methyl-5,6-dihydro-8H-[1,2,4]triazolo[4,3-a]pyrazin-7-yl)-methanone

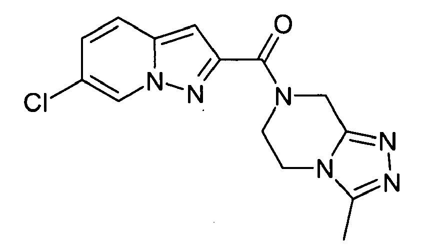

In close analogy to the procedure described in Example 1, 6-chloro-pyrazolo[1,5-a]pyridine-2-carboxylic acid is reacted with 3-methyl-5,6,7,8-tetrahydro-[1,2,4]triazolo[4,3-a]pyrazine to provide the title compound in good to moderate yield.
LC/MS: m/z = 318 (MH⁺)

### Example 476

### (6-Bromo-pyrazolo[1,5-a]pyridin-2-yl)-(3,8-dimethyl-5,6-dihydro-8H-[1,2,4]triazolo[4,3-a]pyrazin-7-yl)-methanone

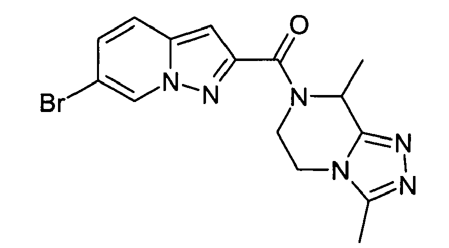

In close analogy to the procedure described in Example 1, 6-bromo-pyrazolo[1,5-a]pyridine-2-carboxylic acid is reacted with 3,8-dimethyl-5,6,7,8-tetrahydro-[1,2,4]triazolo[4,3-a]pyrazine to provide the title compound in good to moderate yield.
LC/MS: m/z = 376 (MH⁺)
The stereo-isomers of this compound are separated. The S-configurated compound has a different activity than the R-configurated compound.

### Example 477

### (6-Bromo-pyrazolo[1,5-a]pyridin-2-yl)-(3-methyl-5,6-dihydro-8H-[1,2,4]triazolo[4,3-a]pyrazin-7-yl)-methanone

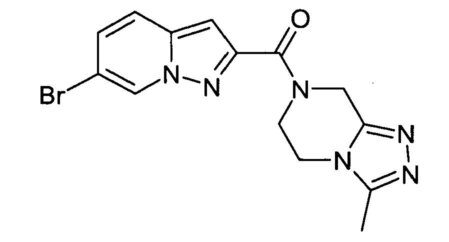

In close analogy to the procedure described in Example 1, 6-bromo-pyrazolo[1,5-a]pyridine-2-carboxylic acid is reacted with 3-methyl-5,6,7,8-tetrahydro-[1,2,4]triazolo[4,3-a]pyrazine to provide the title compound in good to moderate yield.
LC/MS: m/z = 362 (MH⁺)

### Example 478

### 2-(6-Chloro-pyrazolo[1,5-a]pyridine-2-carbonyl)-1-methyl-1,2,3,4-tetrahydro-pyrrolo[1,2-a]pyrazine-6-carbonitrile

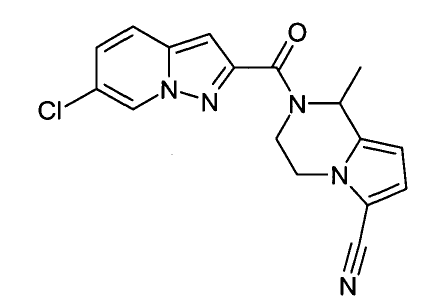

In close analogy to the procedure described in Example 1, 6-chloro-pyrazolo[1,5-a]pyridine-2-carboxylic acid is reacted with 1-methyl-1,2,3,4-tetrahydro-pyrrolo[1,2-a]pyrazine-6-carbonitrile to provide the title compound in good to moderate yield.
LC/MS: m/z = 341 (MH⁺)
The stereo-isomers of this compound are separated. The S-configurated compound has a different activity than the R-configurated compound.

### Example 479

### 2-(6-Chloro-pyrazolo[1,5-a]pyridine-2-carbonyl)-1,2,3,4-tetrahydro-pyrrolo[1,2-a]pyrazine-6-carbonitrile

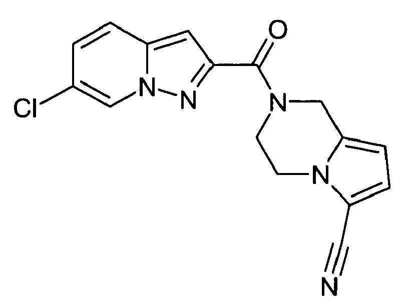

In close analogy to the procedure described in Example 1, 6-chloro-pyrazolo[1,5-a]pyridine-2-carboxylic acid is reacted with 1,2,3,4-tetrahydro-pyrrolo[1,2-a]pyrazine-6-carbonitrile to provide the title compound in good to moderate yield.
LC/MS: m/z = 327 (MH⁺)

### Example 480

### 2-(6-Bromo-pyrazolo[1,5-a]pyridine-2-carbonyl)-1-methyl-1,2,3,4-tetrahydro-pyrrolo[1,2-a]pyrazine-6-carbonitrile

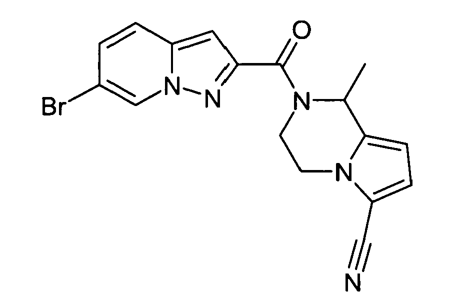

In close analogy to the procedure described in Example 1, 6-bromo-pyrazolo[1,5-a]pyridine-2-carboxylic acid is reacted with 1-methyl-1,2,3,4-tetrahydro-pyrrolo[1,2-a]pyrazine-6-carbonitrile to provide the title compound in good to moderate yield.
LC/MS: m/z = 385 (MH⁺)
The stereo-isomers of this compound are separated. The S-configurated compound has a different activity than the R-configurated compound.

### Example 481

### 2-(6-Bromo-pyrazolo[1,5-a]pyridine-2-carbonyl)-1,2,3,4-tetrahydro-pyrrolo[ 1,2-a]pyrazine-6-carbonitrile

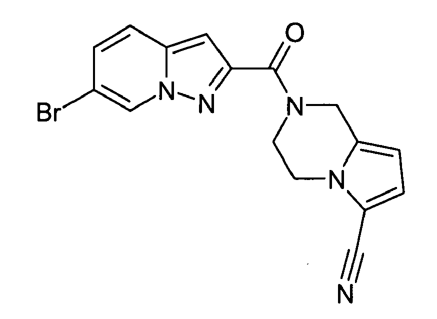

In close analogy to the procedure described in Example 1, 6-bromo-pyrazolo[1,5-a]pyridine-2-carboxylic acid is reacted with 1,2,3,4-tetrahydro-pyrrolo[1,2-a]pyrazine-6-carbonitrile to provide the title compound in good to moderate yield.
LC/MS: m/z = 371 (MH⁺)

### Example 482

### 2-(6-Chloro-pyrazolo[1,5-a]pyridine-2-carbonyl)-1-methyl-1,2,3,4-tetrahydro-pyrrolo[1,2-a]pyrazine-6-carboxylic acid methylamide

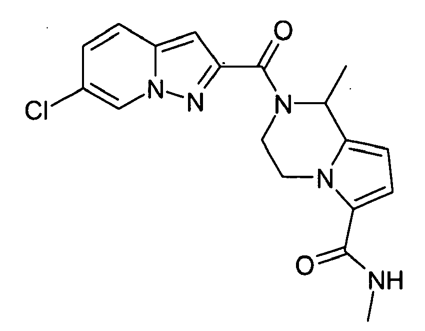

In close analogy to the procedure described in Example 1, 6-chloro-pyrazolo[1,5-a]pyridine-2-carboxylic acid is reacted with 1-methyl-1,2,3,4-tetrahydro-pyrrolo[1,2-a]pyrazine-6-carboxylic acid methylamide to provide the title compound in good to moderate yield.
LC/MS: m/z = 373 (MH⁺)
The stereo-isomers of this compound are separated. The S-configurated compound has a different activity than the R-configurated compound.

### Example 483

### 2-(6-Chloro-pyrazolo[1,5-a]pyridine-2-carbonyl)-1,2,3,4-tetrahydro-pyrrolo[1,2-a]pyrazine-6-carboxylic acid methylamide

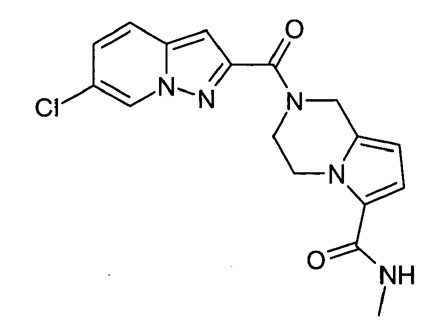

In close analogy to the procedure described in Example 1, 6-chloro-pyrazolo[1,5-a]pyridine-2-carboxylic acid is reacted with 1,2,3,4-tetrahydro-pyrrolo[1,2-a]pyrazine-6-carboxylic acid methylamide to provide the title compound in good to moderate yield.
LC/MS: m/z = 359 (MH⁺)

### Example 484

### 2-(6-Bromo-pyrazolo[1,5-a]pyridine-2-carbonyl)-1-methyl-1,2,3,4-tetrahydro-pyrrolo[1,2-a]pyrazine-6-carboxylic acid methylamide

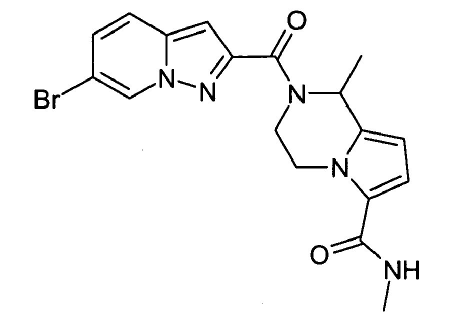

In close analogy to the procedure described in Example 1, 6-bromo-pyrazolo[1,5-a]pyridine-2-carboxylic acid is reacted with 1-methyl-1,2,3,4-tetrahydro-pyrrolo[1,2-a]pyrazine-6-carboxylic acid methylamide to provide the title compound in good to moderate yield.
LC/MS: m/z = 417 (MH⁺)

The stereo-isomers of this compound are separated. The S-configurated compound has a different activity than the R-configurated compound.

### Example 485

### 2-(6-Bromo-pyrazolo[1,5-a]pyridine-2-carbonyl)-1,2,3,4-tetrahydro-pyrrolo[1,2-a]pyrazine-6-carboxylic acid methylamide

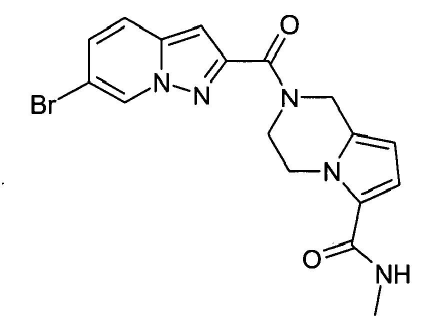

In close analogy to the procedure described in Example 1, 6-bromo-pyrazolo[1,5-a]pyridine-2-carboxylic acid is reacted with 1,2,3,4-tetrahydro-pyrrolo[1,2-a]pyrazine-6-carboxylic acid methylamide to provide the title compound in good to moderate yield.
LC/MS: m/z = 403 (MH⁺)

### Example 486

### 2-(6-Chloro-pyrazolo[1,5-a]pyridine-2-carbonyl)-1-methyl-1,2,3,4-tetrahydro-pyrrolo[1,2-a]pyrazine-6-carboxylic acid cyclopropylamide

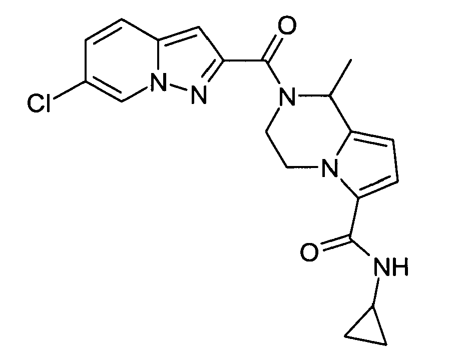

In close analogy to the procedure described in Example 1, 6-chloro-pyrazolo[1,5-a]pyridine-2-carboxylic acid is reacted with 1-methyl-1,2,3,4-tetrahydro-pyrrolo[1,2-a]pyrazine-6-carboxylic acid cyclopropylamide to provide the title compound in good to moderate yield.
LC/MS: m/z = 400 (MH⁺)
The stereo-isomers of this compound are separated. The S-configurated compound has a different activity than the R-configurated compound.

### Example 487

### 2-(6-Chloro-pyrazolo[1,5-a]pyridine-2-carbonyl)-1,2,3,4-tetrahydro-pyrrolo[1,2-a]pyrazine-6-carboxylic acid cyclopropylamide

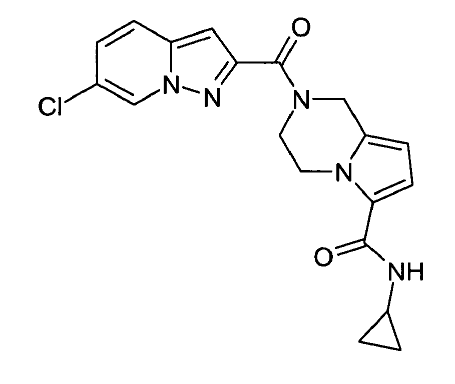

In close analogy to the procedure described in Example 1, 6-chloro-pyrazolo[1,5-a]pyridine-2-carboxylic acid is reacted with 1,2,3,4-tetrahydro-pyrrolo[1,2-a]pyrazine-6-carboxylic acid cyclopropylamide to provide the title compound in good to moderate yield.
LC/MS: m/z = 386 (MH⁺)

### Example 488

### 2-(6-Bromo-pyrazolo[1,5-a]pyridine-2-carbonyl)-1-methyl-1,2,3,4-tetrahydro-pyrrolo[1,2-a]pyrazine-6-carboxylic acid cyclopropylamide

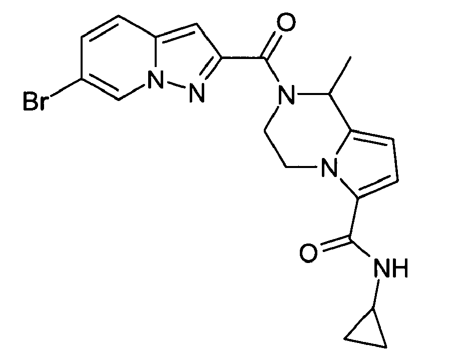

In close analogy to the procedure described in Example 1, 6-bromo-pyrazolo[1,5-a]pyridine-2-carboxylic acid is reacted with 1-methyl-1,2,3,4-tetrahydro-pyrrolo[1,2-a]pyrazine-6-carboxylic acid cyclopropylamide to provide the title compound in good to moderate yield.
LC/MS: m/z = 443 (MH⁺)
The stereo-isomers of this compound are separated. The S-configurated compound has a different activity than the R-configurated compound.

### Example 489

### 2-(6-Bromo-pyrazolo[1,5-a]pyridine-2-carbonyl)-1,2,3,4-tetrahydro-pyrrolo[1,2-a]pyrazine-6-carboxylic acid cyclopropylamide

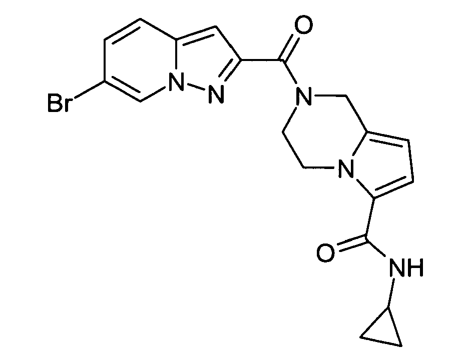

In close analogy to the procedure described in Example 1, 6-bromo-pyrazolo[1,5-a]pyridine-2-carboxylic acid is reacted with 1,2,3,4-tetrahydro-pyrrolo[1,2-a]pyrazine-6-carboxylic acid cyclopropylamide to provide the title compound in good to moderate yield.
LC/MS: m/z = 429 (MH⁺)

### Example 490

### 2-(6-Chloro-pyrazolo[1,5-a]pyridine-2-carbonyl)-1-methyl-1,2,3,4-tetrahydro-pyrrolo[ 1,2-a]pyrazine-6-carboxylic acid ethylamide

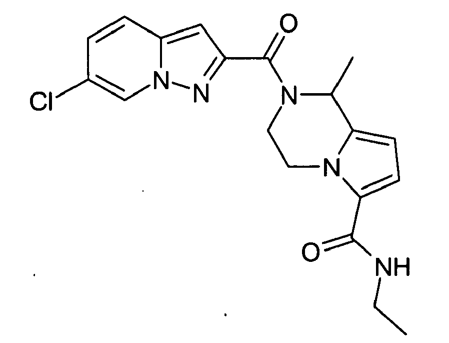

In close analogy to the procedure described in Example 1, 6-chloro-pyrazolo[1,5-a]pyridine-2-carboxylic acid is reacted with 1-methyl-1,2,3,4-tetrahydro-pyrrolo[1,2-a]pyrazine-6-carboxylic acid ethylamide to provide the title compound in good to moderate yield.
LC/MS: m/z = 387 (MH⁺)

The stereo-isomers of this compound are separated. The S-configurated compound has a different activity than the R-configurated compound.

### Example 491

### 2-(6-Chloro-pyrazolo[1,5-a]pyridine-2-carbonyl)-1,2,3,4-tetrahydro-pyrrolo[1,2-a]pyrazine-6-carboxylic acid ethylamide

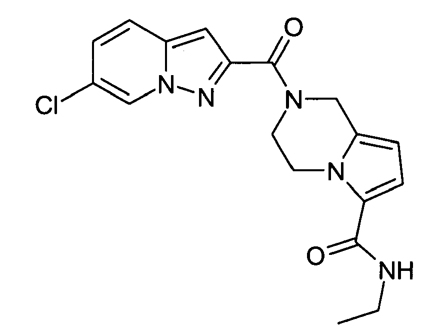

In close analogy to the procedure described in Example 1, 6-chloro-pyrazolo[1,5-a]pyridine-2-carboxylic acid is reacted with 1,2,3,4-tetrahydro-pyrrolo[1,2-a]pyrazine-6-carboxylic acid ethylamide to provide the title compound in good to moderate yield.
LC/MS: m/z = 373 (MH⁺)

### Example 492

### 2-(6-Bromo-pyrazolo[1,5-a]pyridine-2-carbonyl)-1-methyl-1,2,3,4-tetrahydro-pyrrolo[1,2-a]pyrazine-6-carboxylic acid ethylamide

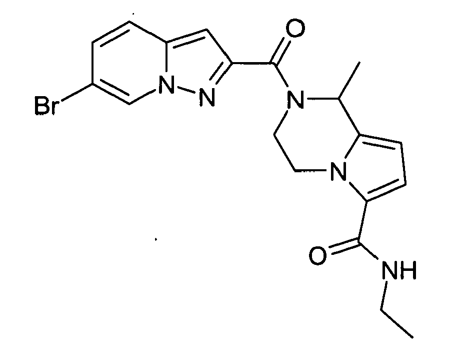

In close analogy to the procedure described in Example 1, 6-bromo-pyrazolo[1,5-a]pyridine-2-carboxylic acid is reacted with 1-methyl-1,2,3,4-tetrahydro-pyrrolo[1,2-a]pyrazine-6-carboxylic acid ethylamide to provide the title compound in good to moderate yield.
LC/MS: m/z = 431 (MH⁺)
The stereo-isomers of this compound are separated. The S-configurated compound has a different activity than the R-configurated compound.

### Example 493

### 2-(6-Bromo-pyrazolo[1,5-a]pyridine-2-carbonyl)-1,2,3,4-tetrahydro-pyrrolo[1,2-a]pyrazine-6-carboxylic acid ethylamide

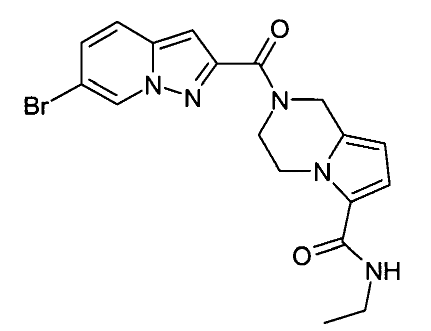

In close analogy to the procedure described in Example 1, 6-bromo-pyrazolo[1,5-a]pyridine-2-carboxylic acid is reacted with 1,2,3,4-tetrahydro-pyrrolo[1,2-a]pyrazine-6-carboxylic acid ethylamide to provide the title compound in good to moderate yield.
LC/MS: m/z = 417 (MH⁺)

### Example 494

### 2-(6-Chloro-pyrazolo[1,5-a]pyridine-2-carbonyl)-1-methyl-1,2,3,4-tetrahydro-pyrrolo[1,2-a]pyrazine-6-carboxylic acid isopropylamide

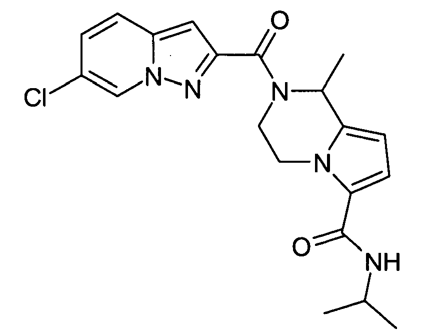

In close analogy to the procedure described in Example 1, 6-chloro-pyrazolo[1,5-a]pyridine-2-carboxylic acid is reacted with 1-methyl-1,2,3,4-tetrahydro-pyrrolo[1,2-a]pyrazine-6-carboxylic acid isopropylamide to provide the title compound in good to moderate yield.
LC/MS: m/z = 401 (MH⁺)
The stereo-isomers of this compound are separated. The S-configurated compound has a different activity than the R-configurated compound.

### Example 495

### 2-(6-Chloro-pyrazolo[1,5-a]pyridine-2-carbonyl)-1,2,3,4-tetrahydro-pyrrolo[1,2-a]pyrazine-6-carboxylic acid isopropylamide

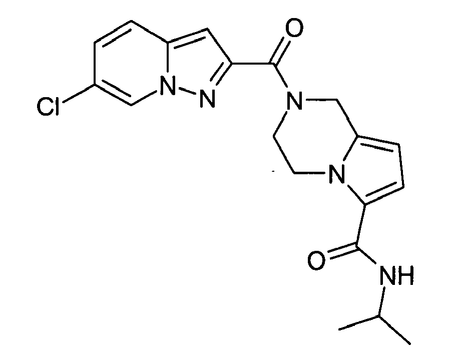

In close analogy to the procedure described in Example 1, 6-chloro-pyrazolo[1,5-a]pyridine-2-carboxylic acid is reacted with 1,2,3,4-tetrahydro-pyrrolo[1,2-a]pyrazine-6-carboxylic acid isopropylamide to provide the title compound in good to moderate yield.
LC/MS: m/z = 387 (MH⁺)

### Example 496

### 2-(6-Bromo-pyrazolo[1,5-a]pyridine-2-carbonyl)-1-methyl-1,2,3,4-tetrahydro-pyrrolo[1,2-a]pyrazine-6-carboxylic acid isopropylamide

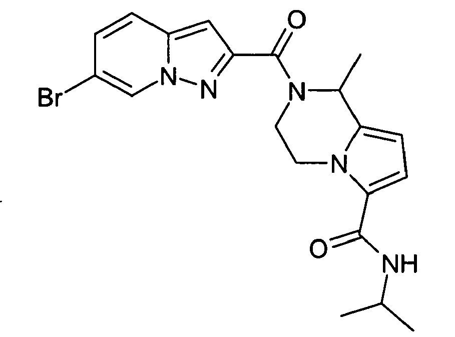

In close analogy to the procedure described in Example 1, 6-bromo-pyrazolo[1,5-a]pyridine-2-carboxylic acid is reacted with 1-methyl-1,2,3,4-tetrahydro-pyrrolo[1,2-a]pyrazine-6-carboxylic acid isopropylamide to provide the title compound in good to moderate yield.
LC/MS: m/z = 445 (MH⁺)
The stereo-isomers of this compound are separated. The S-configurated compound has a different activity than the R-configurated compound.

### Example 497

### 2-(6-Bromo-pyrazolo[1,5-a]pyridine-2-carbonyl)-1,2,3,4-tetrahydro-pyrrolo[1,2-a]pyrazine-6-carboxylic acid isopropylamide

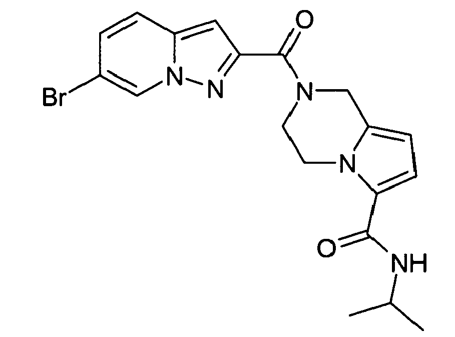

In close analogy to the procedure described in Example 1, 6-bromo-pyrazolo[1,5-a]pyridine-2-carboxylic acid is reacted with 1,2,3,4-tetrahydro-pyrrolo[1,2-a]pyrazine-6-carboxylic acid isopropylamide to provide the title compound in good to moderate yield.
LC/MS: m/z = 431 (MH⁺)

### Example 498

### (6-Chloro-pyrazolo[1,5-a]pyridin-2-yl)-[1-methyl-6-(1H-tetrazol-5-yl)-3,4-dihydro-1H-pyrrolo[1,2-a]pyrazin-2-yl]-methanone

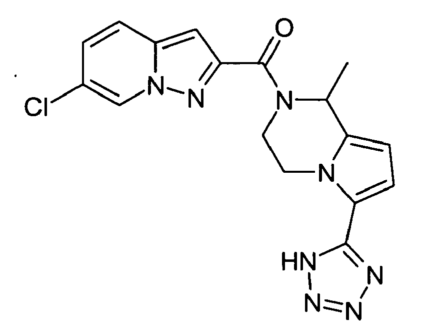

In close analogy to the procedure described in Example 1, 6-chloro-pyrazolo[1,5-a]pyridine-2-carboxylic acid is reacted with 1-methyl-6-(1H-tetrazol-5-yl)-1,2,3,4-tetrahydro-pyrrolo[1,2-a]pyrazine to provide the title compound in good to moderate yield.
LC/MS: m/z = 384 (MH⁺)
The stereo-isomers of this compound are separated. The S-configurated compound has a different activity than the R-configurated compound.

### Example 499

### (6-Chloro-pyrazolo[1,5-a]pyridin-2-yl)-[6-(1H-tetrazol-5-yl)-3,4-dihydro-1H-pyrrolo[1,2-a]pyrazin-2-yl]-methanone

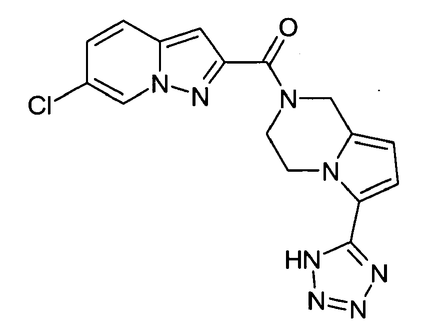

In close analogy to the procedure described in Example 1, 6-chloro-pyrazolo[1,5-a]pyridine-2-carboxylic acid is reacted with 6-(1H-tetrazol-5-yl)-1,2,3,4-tetrahydro-pyrrolo[1,2-a]pyrazine to provide the title compound in good to moderate yield.
LC/MS: m/z = 470 (MH⁺)

### Example 500

### (6-Bromo-pyrazolo[1,5-a]pyridin-2-yl)-[1-methyl-6-(H-tetrazol-5-yl)-3,4-dihydro-1H-pyrrolo[1,2-a]pyrazin-2-yl]-methanone

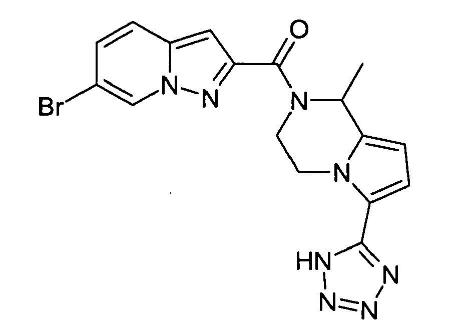

In close analogy to the procedure described in Example 1, 6-bromo-pyrazolo[1,5-a]pyridine-2-carboxylic acid is reacted with 1-methyl-6-(1H-tetrazol-5-yl)-1,2,3,4-tetrahydro-pyrrolo[1,2-a]pyrazine to provide the title compound in good to moderate yield.
LC/MS: m/z = 428 (MH⁺)
The stereo-isomers of this compound are separated. The S-configurated compound has a different activity than the R-configurated compound.

### Example 501

### (6-Bromo-pyrazolo[1,5-a]pyridin-2-yl)-[6-(H-tetrazol-5-yl)-3,4-dihydro-H-pyrrolo[1,2-a]pyrazin-2-yl]-methanone

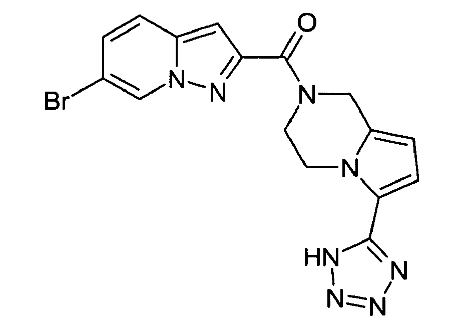

In close analogy to the procedure described in Example 1, 6-bromo-pyrazolo[1,5-a]pyridine-2-carboxylic acid is reacted with 6-(1H-tetrazol-5-yl)-1,2,3,4-tetrahydro-pyrrolo[1,2-a]pyrazine to provide the title compound in good to moderate yield.
LC/MS: m/z = 414 (MH⁺)

### Example 502

### (6-Chloro-pyrazolo[1,5-a]pyridin-2-yl)-(1-methyl-6-pyridin-2-yl-3,4-dihydro-1H-pyrrolo[1,2-a]pyrazin-2-yl)-methanone

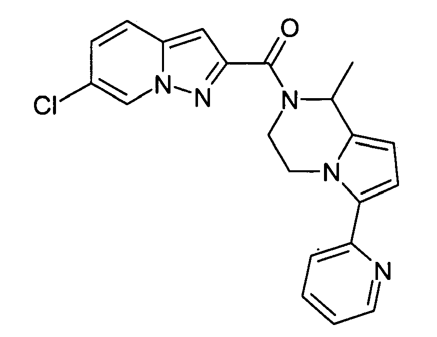

In close analogy to the procedure described in Example 1, 6-chloro-pyrazolo[1,5-a]pyridine-2-carboxylic acid is reacted with 1-methyl-6-pyridin-2-yl-1,2,3,4-tetrahydro-pyrrolo[1,2-a]pyrazine to provide the title compound in good to moderate yield.
LC/MS: m/z = 393 (MH⁺)
The stereo-isomers of this compound are separated. The S-configurated compound has a different activity than the R-configurated compound.

### Example 503

### (6-Chloro-pyrazolo[1,5-a]pyridin-2-yl)-(6-pyridin-2-yl-3,4-dihydro-1H-pyrrolo[1,2-a]pyrazin-2-yl)-methanone

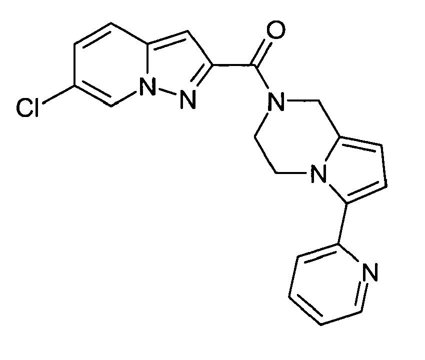

In close analogy to the procedure described in Example 1, 6-chloro-pyrazolo[1,5-a]pyridine-2-carboxylic acid is reacted with 6-pyridin-2-yl-1,2,3,4-tetrahydro-pyrrolo[1,2-a]pyrazine to provide the title compound in good to moderate yield.
LC/MS: m/z = 379 (MH⁺)

### Example 504

(6-Bromo-pyrazolo[1,5-a]pyridin-2-yl)-(1-methyl-6-pyridin-2-yl-3,4-dihydro-1H-pyrrolo[1,2-a]pyrazin-2-yl)-methanone

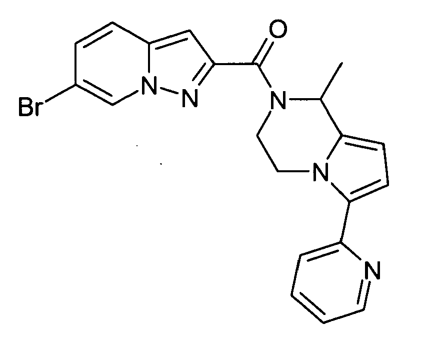

In close analogy to the procedure described in Example 1, 6-bromo-pyrazolo[1,5-a]pyridine-2-carboxylic acid is reacted with 1-methyl-6-pyridin-2-yl-1,2,3,4-tetrahydro-pyrrolo[1,2-a]pyrazine to provide the title compound in good to moderate yield.
LC/MS: m/z = 437 (MH⁺)
The stereo-isomers of this compound are separated. The S-configurated compound has a different activity than the R-configurated compound.

### Example 505

### (6-Bromo-pyrazolo[1,5-a]pyridin-2-yl)-(6-pyridin-2-yl-3,4-dihydro-1H-pyrrolo[1,2-a]pyrazin-2-yl)-methanone

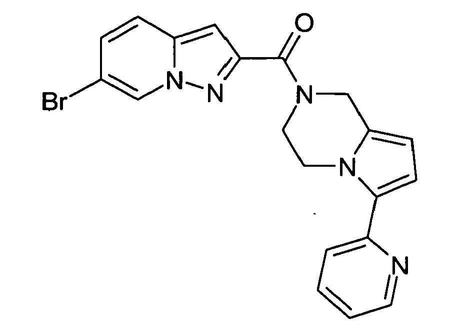

In close analogy to the procedure described in Example 1, 6-bromo-pyrazolo[1,5-a]pyridine-2-carboxylic acid is reacted with 6-pyridin-2-yl-1,2,3,4-tetrahydro-pyrrolo[1,2-a]pyrazine to provide the title compound in good to moderate yield.
LC/MS: m/z = 423 (MH⁺)

### Example 506

### (6-Chloro-pyrazolo[1,5-a]pyridin-2-yl)-(1-methyl-6-pyridin-3-yl-3,4-dihydro-1H-pyrrolo[1,2-a]pyrazin-2-yl)-methanone

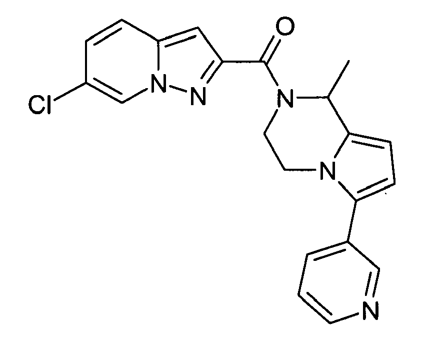

In close analogy to the procedure described in Example 1, 6-chloro-pyrazolo[1,5-a]pyridine-2-carboxylic acid is reacted with 1-methyl-6-pyridin-3-yl-1,2,3,4-tetrahydro-pyrrolo[1,2-a]pyrazine to provide the title compound in good to moderate yield.
LC/MS: m/z = 393 (MH⁺)

The stereo-isomers of this compound are separated. The S-configurated compound has a different activity than the R-configurated compound.

### Example 507

### (6-Chloro-pyrazolo[1,5-a]pyridin-2-yl)-(6-pyridin-3-yl-3,4-dihydro-1H-pyrrolo[ 1,2-a]pyrazin-2-yl)-methanone

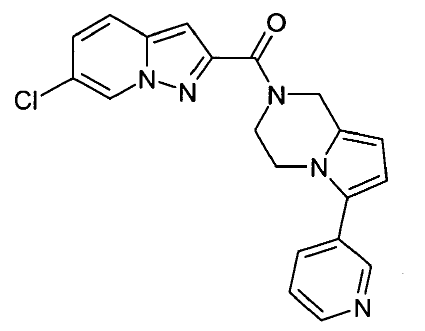

In close analogy to the procedure described in Example 1, 6-chloro-pyrazolo[1,5-a]pyridine-2-carboxylic acid is reacted with 6-pyridin-3-yl-1,2,3,4-tetrahydro-pyrrolo[1,2-a]pyrazine to provide the title compound in good to moderate yield.
LC/MS: m/z = 379 (MH⁺)

### Example 508

### (6-Bromo-pyrazolo[1,5-a]pyridin-2-yl)-(1-methyl-6-pyridin-3-yl-3,4-dihydro-1H-pyrrolo[1,2-a]pyrazin-2-yl)-methanone

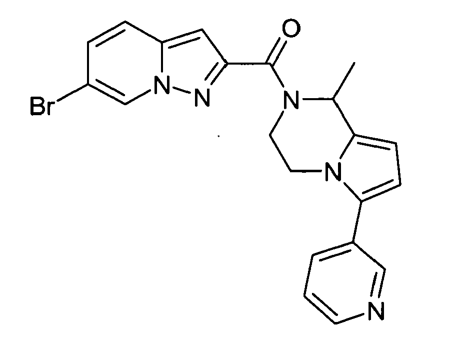

In close analogy to the procedure described in Example 1, 6-bromo-pyrazolo[1,5-a]pyridine-2-carboxylic acid is reacted with 1-methyl-6-pyridin-3-yl-1,2,3,4-tetrahydro-pyrrolo[1,2-a]pyrazine to provide the title compound in good to moderate yield.
LC/MS: m/z = 437 (MH⁺)

The stereo-isomers of this compound are separated. The S-configurated compound has a different activity than the R-configurated compound.

### Example 509

### (6-Bromo-pyrazolo[1,5-a]pyridin-2-yl)-(6-pyridin-3-yl-3,4-dihydro-1H-pyrrolo[1,2-a]pyrazin-2-yl)-methanone

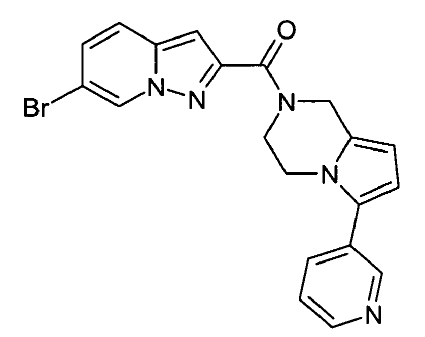

In close analogy to the procedure described in Example 1, 6-bromo-pyrazolo[1,5-a]pyridine-2-carboxylic acid is reacted with 6-pyridin-3-yl-1,2,3,4-tetrahydro-pyrrolo[1,2-a]pyrazine to provide the title compound in good to moderate yield.
LC/MS: m/z = 423 (MH⁺)

### Example 510

### (6-Chloro-pyrazolo[1,5-a]pyridin-2-yl)-(1-methyl-6-pyridin-4-yl-3,4-dihydro-1H-pyrrolo[1,2-a]pyrazin-2-yl)-methanone

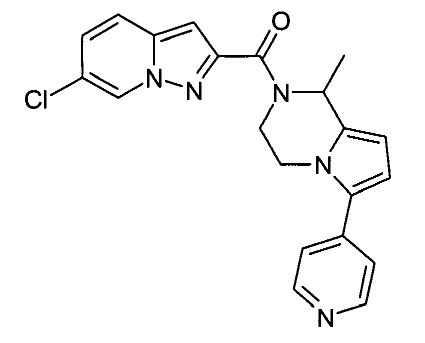

In close analogy to the procedure described in Example 1, 6-chloro-pyrazolo[1,5-a]pyridine-2-carboxylic acid is reacted with 1-methyl-6-pyridin-4-yl-1,2,3,4-tetrahydro-pyrrolo[1,2-a]pyrazine to provide the title compound in good to moderate yield.
LC/MS: m/z = 493 (MH⁺)
The stereo-isomers of this compound are separated. The S-configurated compound has a different activity than the R-configurated compound.

### Example 511

### (6-Chloro-pyrazolo[1,5-a]pyridin-2-yl)-(6-pyridin-4-yl-3,4-dihydro-1H-pyrrolo[1,2-a]pyrazin-2-yl)-methanone

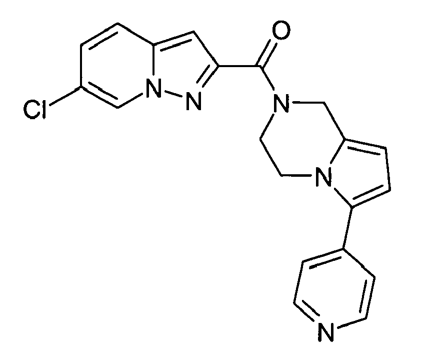

In close analogy to the procedure described in Example 1, 6-chloro-pyrazolo[1,5-a]pyridine-2-carboxylic acid is reacted with 6-pyridin-3-yl-1,2,3,4-tetrahydro-pyrrolo[1,2-a]pyrazine to provide the title compound in good to moderate yield.
LC/MS: m/z = 484 (MH⁺)

### Example 512

### (6-Bromo-pyrazolo[1,5-a]pyridin-2-yl)-(1-methyl-6-pyridin-4-yl-3,4-dihydro-1H-pyrrolo[1,2-a]pyrazin-2-yl)-methanone

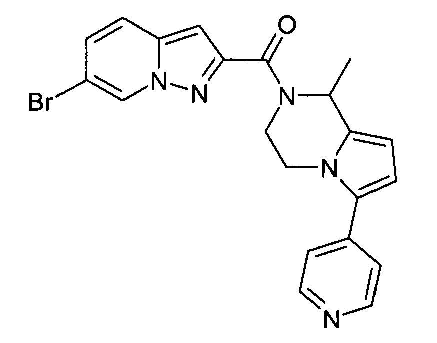

In close analogy to the procedure described in Example 1, 6-bromo-pyrazolo[1,5-a]pyridine-2-carboxylic acid is reacted with 1-methyl-6-pyridin-4-yl-1,2,3,4-tetrahydro-pyrrolo[1,2-a]pyrazine to provide the title compound in good to moderate yield.
LC/MS: m/z = 437 (MH⁺)

The stereo-isomers of this compound are separated. The S-configurated compound has a different activity than the R-configurated compound.

### Example 513

### (6-Bromo-pyrazolo[1,5-a]pyridin-2-yl)-(6-pyridin-4-yl-3,4-dihydro-1H-pyrrolo[1,2-a]pyrazin-2-yl)-methanone

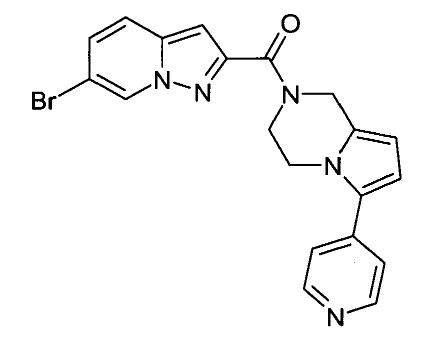

In close analogy to the procedure described in Example 1, 6-bromo-pyrazolo[1,5-a]pyridine-2-carboxylic acid is reacted with 6-pyridin-4-yl-1,2,3,4-tetrahydro-pyrrolo[1,2-a]pyrazine to provide the title compound in good to moderate yield.
LC/MS: m/z = 423 (MH⁺)

### Example 514

### (6-Chloro-pyrazolo[1,5-a]pyridin-2-yl)-(1-methyl-6-thiophen-2-yl-3,4-dihydro-1H-pyrrolo[1,2-a]pyrazin-2-yl)-methanone

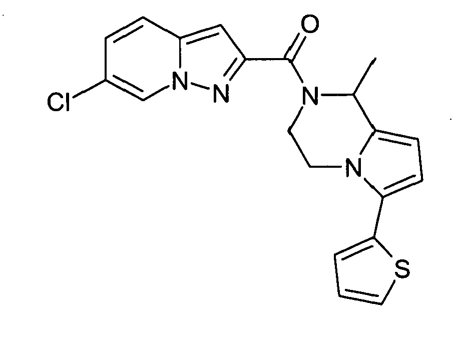

In close analogy to the procedure described in Example 1, 6-chloro-pyrazolo[1,5-a]pyridine-2-carboxylic acid is reacted with 1-methyl-6-thiophen-2-yl-1,2,3,4-tetrahydro-pyrrolo[1,2-a]pyrazine to provide the title compound in good to moderate yield.
LC/MS: m/z = 398 (MH⁺)

The stereo-isomers of this compound are separated. The S-configurated compound has a different activity than the R-configurated compound.

### Example 515

### (6-Chloro-pyrazolo[1,5-a]pyridin-2-yl)-(6-thiophen-2-yl-3,4-dihydro-1H-pyrrolo[1,2-a]pyrazin-2-yl)-methanone

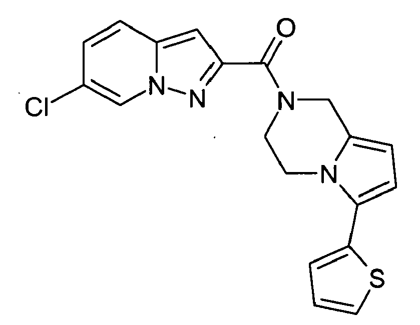

In close analogy to the procedure described in Example 1, 6-chloro-pyrazolo[1,5-a]pyridine-2-carboxylic acid is reacted with 6-thiophen-2-yl-1,2,3,4-tetrahydro-pyrrolo[1,2-a]pyrazine to provide the title compound in good to moderate yield.
LC/MS: m/z = 383 (MH⁺)

### Example 516

### (6-Bromo-pyrazolo[1,5-a]pyridin-2-yl)-(1-methyl-6-thiophen-2-yl-3,4-dihydro-1H-pyrrolo[1,2-a]pyrazin-2-yl)-methanone

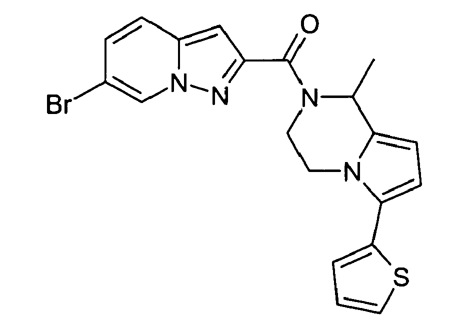

In close analogy to the procedure described in Example 1, 6-bromo-pyrazolo[1,5-a]pyridine-2-carboxylic acid is reacted with 1-methyl-6-thiophen-2-yl-1,2,3,4-tetrahydro-pyrrolo[1,2-a]pyrazine to provide the title compound in good to moderate yield.
LC/MS: m/z = 442 (MH⁺)

The stereo-isomers of this compound are separated. The S-configurated compound has a different activity than the R-configurated compound.

### Example 517

### (6-Bromo-pyrazolo[1,5-a]pyridin-2-yl)-(6-thiophen-2-yl-3,4-dihydro-1H-pyrrolo[1,2-a]pyrazin-2-yl)-methanone

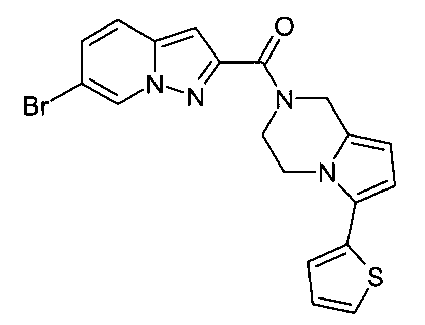

In close analogy to the procedure described in Example 1, 6-bromo-pyrazolo[1,5-a]pyridine-2-carboxylic acid is reacted with 6-thiophen-2-yl-1,2,3,4-tetrahydro-pyrrolo[1,2-a]pyrazine to provide the title compound in good to moderate yield.
LC/MS: m/z = 428 (MH⁺)

### Example 518

### (6-Chloro-pyrazolo[1,5-a]pyndin-2-yl)-(6-furan-2-yl-1-methyl-3,4-dihydro-1H-pyrrolo[1,2-a]pyrazin-2-yl)-methanone

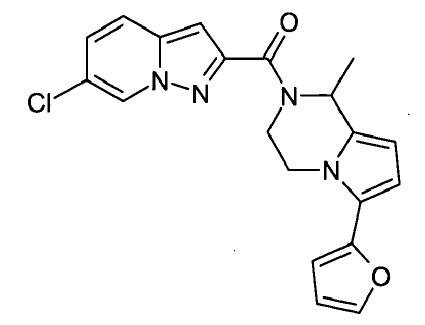

In close analogy to the procedure described in Example 1, 6-chloro-pyrazolo[1,5-a]pyridine-2-carboxylic acid is reacted with 6-furan-2-yl-1-methyl-1,2,3,4-tetrahydro-pyrrolo[1,2-a]pyrazine to provide the title compound in good to moderate yield.
LC/MS: m/z = 382 (MH⁺)

The stereo-isomers of this compound are separated. The S-configurated compound has a different activity than the R-configurated compound.

### Example 519

### (6-Chloro-pyrazolo[1,5-a]pyridin-2-yl)-(6-furan-2-yl-3,4-dihydro-1H-pyrrolo[1,2-a]pyrazin-2-yl)-methanone

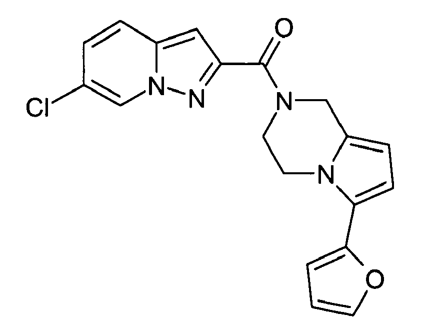

In close analogy to the procedure described in Example 1, 6-chloro-pyrazolo[1,5-a]pyridine-2-carboxylic acid is reacted with 6-furan-2-yl-1,2,3,4-tetrahydro-pyrrolo[1,2-a]pyrazine to provide the title compound in good to moderate yield.
LC/MS: m/z = 368 (MH⁺)

### Example 520

### (6-Bromo-pyrazolo[1,5-a]pyridin-2-yl)-(6-furan-2-yl-1-methyl-3,4-dihydro-1H-pyrrolo[1,2-a]pyrazin-2-yl)-methanone

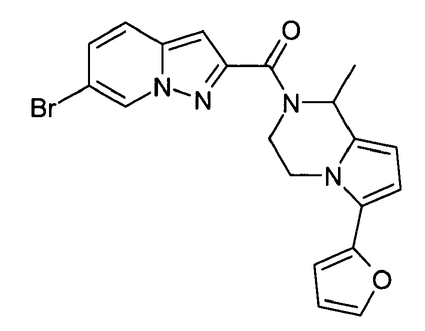

In close analogy to the procedure described in Example 1, 6-bromo-pyrazolo[1,5-a]pyridine-2-carboxylic acid is reacted with 6-furan-2-yl-1-methyl-1,2,3,4-tetrahydro-pyrrolo[1,2-a]pyrazine to provide the title compound in good to moderate yield.
LC/MS: m/z = 426 (MH⁺)

The stereo-isomers of this compound are separated. The S-configurated compound has a different activity than the R-configurated compound.

### Example 521

### (6-Bromo-pyrazolo[1,5-a]pyridin-2-yl)-(6-furan-2-yl-3,4-dihydro-1H-pyrrolo[1,2-a]pyrazin-2-yl)-methanone

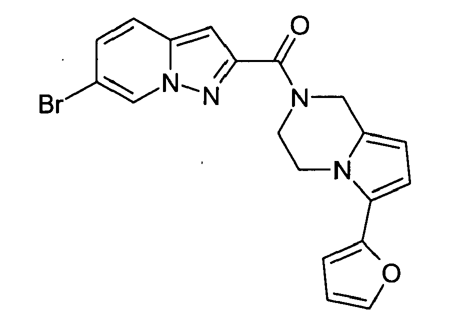

In close analogy to the procedure described in Example 1, 6-bromo-pyrazolo[1,5-a]pyridine-2-carboxylic acid is reacted with 6-furan-2-yl-1,2,3,4-tetrahydro-pyrrolo[1,2-a]pyrazine to provide the title compound in good to moderate yield.
LC/MS: m/z = 412 (MH⁺)

### Example 522

### (6-Chloro-pyrazolo[1,5-a]pyridin-2-yl)-(l-methyl-6-thiophen-3-yl-3,4-dihydro-1H-pyrrolo[1,2-a]pyrazin-2-yl)-methanone

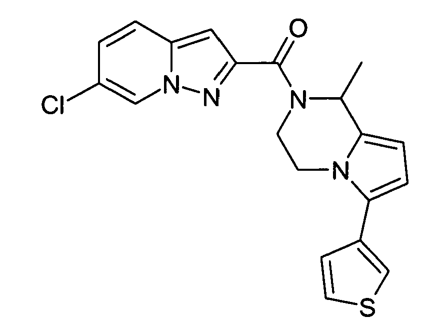

In close analogy to the procedure described in Example 1, 6-chloro-pyrazolo[1,5-a]pyridine-2-carboxylic acid is reacted with 1-methyl-6-thiophen-3-yl-1,2,3,4-tetrahydro-pyrrolo[1,2-a]pyrazine to provide the title compound in good to moderate yield.
LC/MS: m/z = 398 (MH⁺)

The stereo-isomers of this compound are separated. The S-configurated compound has a different activity than the R-configurated compound.

### Example 523

### (6-Chloro-pyrazolo[1,5-a]pyridin-2-yl)-(6-thiophen-3-yl-3,4-dihydro-1H-pyrrolo[1,2-a]pyrazin-2-yl)-methanone

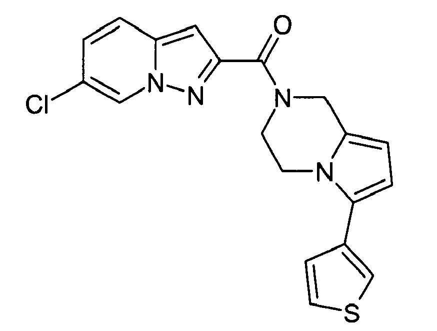

In close analogy to the procedure described in Example 1, 6-chloro-pyrazolo[1,5-a]pyridine-2-carboxylic acid is reacted with 6-thiophen-3-yl-1,2,3,4-tetrahydro-pyrrolo[1,2-a]pyrazine to provide the title compound in good to moderate yield.
LC/MS: m/z = 384 (MH⁺)

### Example 524

### (6-Bromo-pyrazolo[1,5-a]pyridin-2-yl)-(1-methyl-6-thiophen-3-yl-3,4-dihydro-1H-pyrrolo[1,2-a]pyrazin-2-yl)-methanone

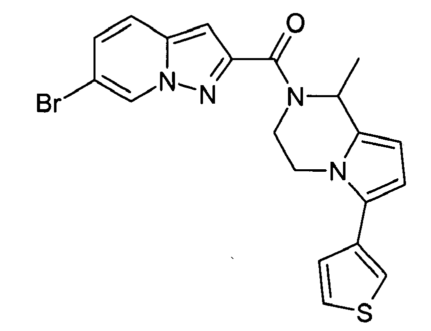

In close analogy to the procedure described in Example 1, 6-bromo-pyrazolo[1,5-a]pyridine-2-carboxylic acid is reacted with 1-methyl-6-thiophen-3-yl-1,2,3,4-tetrahydro-pyrrolo[1,2-a]pyrazine to provide the title compound in good to moderate yield.
LC/MS: m/z = 442 (MH⁺)

The stereo-isomers of this compound are separated. The S-configurated compound has a different activity than the R-configurated compound.

### Example 525

### (6-Bromo-pyrazolo[1,5-a]pyridin-2-yl)-(6-thiophen-3-yl-3,4-dihydro-1H-pyrrolo[1,2-a]pyrazin-2-yl)-methanone

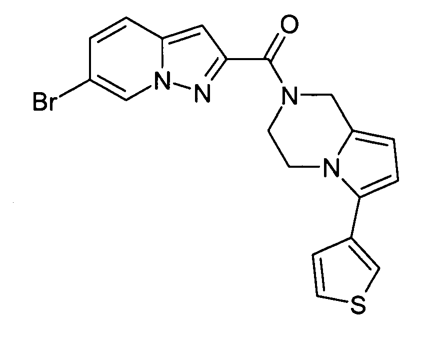

In close analogy to the procedure described in Example 1, 6-bromo-pyrazolo[1,5-a]pyridine-2-carboxylic acid is reacted with 6-thiophen-3-yl-1,2,3,4-tetrahydro-pyrrolo[1,2-a]pyrazine to provide the title compound in good to moderate yield.
LC/MS: m/z = 428 (MH⁺)

### Example 526

### (6-Chloro-pyrazolo[1,5-a]pyridin-2-yl)-(6-furan-3-yl-1-methyl-3,4-dihydro-1H-pyrrolo[1,2-a]pyrazin-2-yl)-methanone

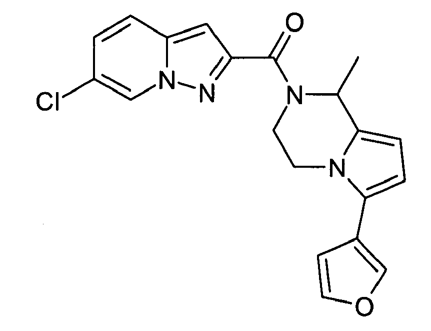

In close analogy to the procedure described in Example 1, 6-chloro-pyrazolo[1,5-a]pyridine-2-carboxylic acid is reacted with 6-furan-3-yl-1-methyl-1,2,3,4-tetrahydro-pyrrolo[1,2-a]pyrazine to provide the title compound in good to moderate yield.
LC/MS: m/z = 382 (MH⁺)

The stereo-isomers of this compound are separated. The S-configurated compound has a different activity than the R-configurated compound.

### Example 527

### (6-Chloro-pyrazolo[1,5-a]pyridin-2-yl)-(6-furan-3-yl-3,4-dihydro-1H-pyrrolo[1,2-a]pyrazin-2-yl)-methanone

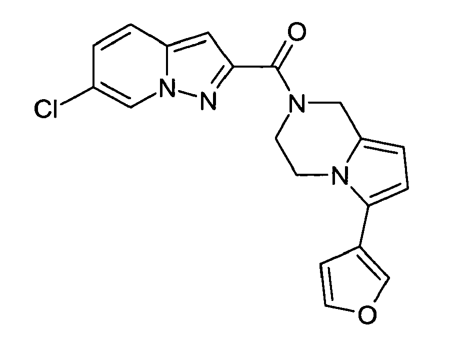

In close analogy to the procedure described in Example 1, 6-chloro-pyrazolo[1,5-a]pyridine-2-carboxylic acid is reacted with 6-furan-3-yl-1,2,3,4-tetrahydro-pyrrolo[1,2-a]pyrazine to provide the title compound in good to moderate yield.
LC/MS: m/z = 368 (MH⁺)

### Example 528

### (6-Bromo-pyrazolo[1,5-a]pyridin-2-yl)-(6-furan-3-yl-1-methyl-3,4-dihydro-1H-pyrrolo[1,2-a]pyrazin-2-yl)-methanone

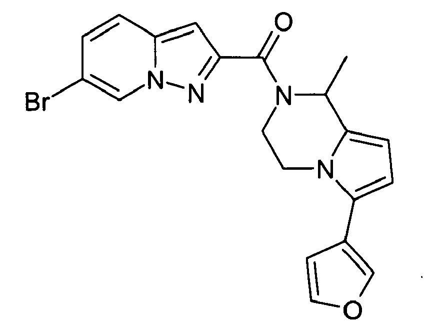

In close analogy to the procedure described in Example 1, 6-bromo-pyrazolo[1,5-a]pyridine-2-carboxylic acid is reacted with 6-furan-3-yl-1-methyl-1,2,3,4-tetrahydro-pyrrolo[1,2-a]pyrazine to provide the title compound in good to moderate yield.
LC/MS: m/z = 426 (MH⁺)

The stereo-isomers of this compound are separated. The S-configurated compound has a different activity than the R-configurated compound.

### Example 529

### (6-Bromo-pyrazolo[1,5-a]pyridin-2-yl)-(6-furan-3-yl-3,4-dihydro-1H-pyrrolo[1,2-a]pyrazin-2-yl)-methanone

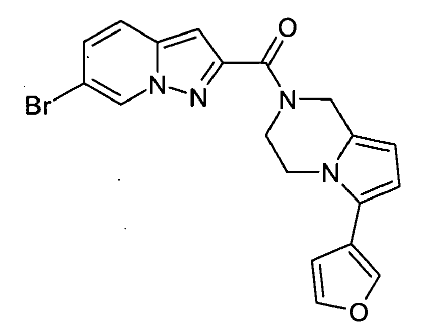

In close analogy to the procedure described in Example 1, 6-bromo-pyrazolo[1,5-a]pyridine-2-carboxylic acid is reacted with 6-furan-3-yl-1,2,3,4-tetrahydro-pyrrolo[1,2-a]pyrazine to provide the title compound in good to moderate yield.
LC/MS: m/z = 412 (MH⁺)

### Example 530

### (6-Chloro-pyrazolo[1,5-a]pyridin-2-yl)-(1-methyl-6-pyrimidin-4-yl-3,4-dihydro-1H-pyrrolo[1,2-a]pyrazin-2-yl)-methanone

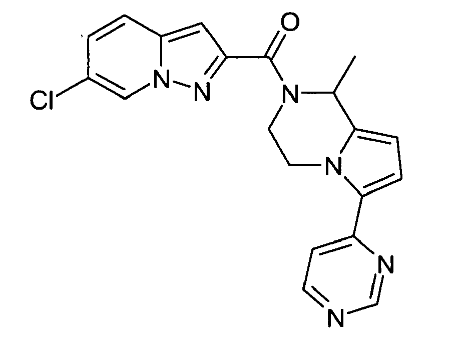

In close analogy to the procedure described in Example 1, 6-chloro-pyrazolo[1,5-a]pyridine-2-carboxylic acid is reacted with 1-methyl-6-pyrimidin-4-yl-1,2,3,4-tetrahydro-pyrrolo[1,2-a]pyrazine to provide the title compound in good to moderate yield.
LC/MS: m/z = 394 (MH⁺)

The stereo-isomers of this compound are separated. The S-configurated compound has a different activity than the R-configurated compound.

### Example 531

### (6-Chloro-pyrazolo[1,5-a]pyridin-2-yl)-(6-pyrimidin-4-yl-3,4-dihydro-1H-pyrrolo[1,2-a]pyrazin-2-yl)-methanone

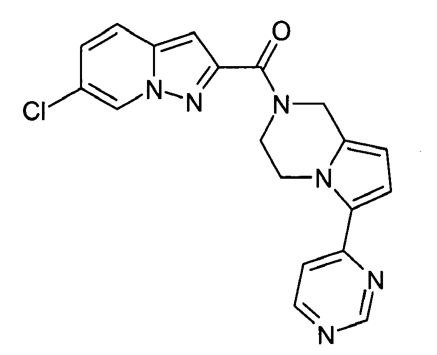

In close analogy to the procedure described in Example 1, 6-chloro-pyrazolo[1,5-a]pyridine-2-carboxylic acid is reacted with 6-pyrimidin-4-yl-1,2,3,4-tetrahydro-pyrrolo[1,2-a]pyrazine to provide the title compound in good to moderate yield.
LC/MS: m/z = 380 (MH⁺)

### Example 532

### (6-Bromo-pyrazolo[1,5-a]pyridin-2-yl)-(1-methyl-6-pyrimidin-4-yl-3,4-dihydro-1H-pyrrolo[1,2-a]pyrazin-2-yl)-methanone

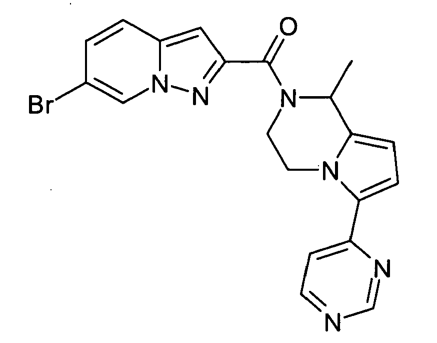

In close analogy to the procedure described in Example 1, 6-bromo-pyrazolo[1,5-a]pyridine-2-carboxylic acid is reacted with 1-methyl-6-pyrimidin-4-yl-1,2,3,4-tetrahydro-pyrrolo[1,2-a]pyrazine to provide the title compound in good to moderate yield.
LC/MS: m/z = 438 (MH⁺)
The stereo-isomers of this compound are separated. The S-configurated compound has a different activity than the R-configurated compound.

### Example 533

### (6-Bromo-pyrazolo[1,5-a]pyridin-2-yl)-(6-pyrimidin-4-yl-3,4-dihydro-1H-pyrrolo[1,2-a]pyrazin-2-yl)-methanone

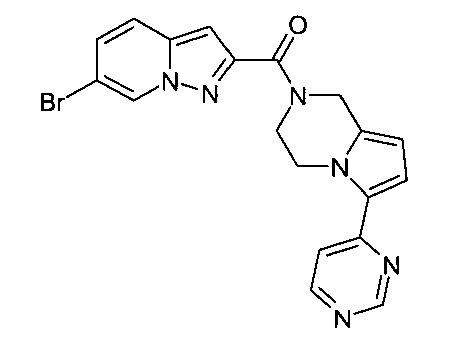

In close analogy to the procedure described in Example 1, 6-bromo-pyrazolo[1,5-a]pyridine-2-carboxylic acid is reacted with 6-pyrimidin-4-yl-1,2,3,4-tetrahydro-pyrrolo[1,2-a]pyrazine to provide the title compound in good to moderate yield.
LC/MS: m/z = 424 (MH⁺)

### Example 534

### (6-Chloro-pyrazolo[1,5-a]pyridin-2-yl)-(1-methyl-6-pyrimidin-5-yl-3,4-dihydro-1H-pyrrolo[1,2-a]pyrazin-2-yl)-methanone

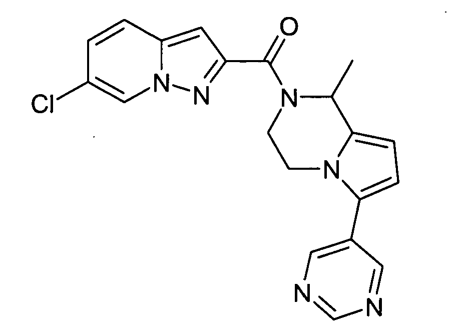

In close analogy to the procedure described in Example 1, 6-chloro-pyrazolo[1,5-a]pyridine-2-carboxylic acid is reacted with 1-methyl-6-pyrimidin-5-yl-1,2,3,4-tetrahydro-pyrrolo[1,2-a]pyrazine to provide the title compound in good to moderate yield.
LC/MS: m/z = 394 (MH⁺)

The stereo-isomers of this compound are separated. The S-configurated compound has a different activity than the R-configurated compound.

### Example 535

### (6-Chloro-pyrazolo[1,5-a]pyridin-2-yl)-(6-pyrimidin-5-yl-3,4-dihydro-1H-pyrrolo[1,2-a]pyrazin-2-yl)-methanone

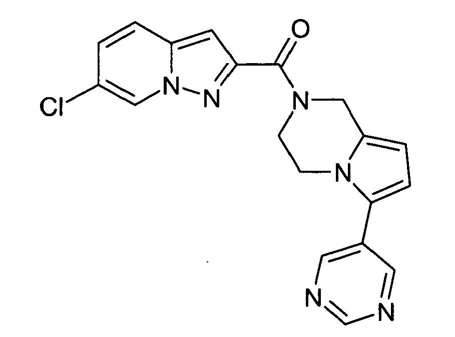

In close analogy to the procedure described in Example 1, 6-chloro-pyrazolo[1,5-a]pyridine-2-carboxylic acid is reacted with 6-pyrimidin-5-yl-1,2,3,4-tetrahydro-pyrrolo[1,2-a]pyrazine to provide the title compound in good to moderate yield.
LC/MS: m/z = 380 (MH⁺)

### Example 536

### (6-Bromo-pyrazolo[1,5-a]pyridin-2-yl)-(1-methyl-6-pyrimidin-5-yl-3,4-dihydro-1H-pyrrolo[1,2-a]pyrazin-2-yl)-methanone

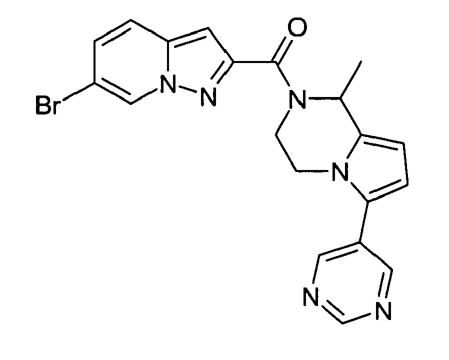

In close analogy to the procedure described in Example 1, 6-bromo-pyrazolo[1,5-a]pyridine-2-carboxylic acid is reacted with 1-methyl-6-pyrimidin-5-yl-1,2,3,4-tetrahydro-pyrrolo[1,2-a]pyrazine to provide the title compound in good to moderate yield.
LC/MS: m/z = 438 (MH⁺)

The stereo-isomers of this compound are separated. The S-configurated compound has a different activity than the R-configurated compound.

### Example 537

### (6-Bromo-pyrazolo[1,5-a]pyridin-2-yl)-(6-pyrimidin-5-yl-3,4-dihydro-1H-pyrrolo[1,2-a]pyrazin-2-yl)-methanone

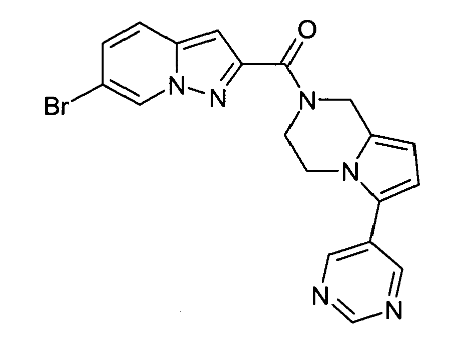

In close analogy to the procedure described in Example 1, 6-bromo-pyrazolo[1,5-a]pyridine-2-carboxylic acid is reacted with 6-pyrimidin-5-yl-1,2,3,4-tetrahydro-pyrrolo[1,2-a]pyrazine to provide the title compound in good to moderate yield.
LC/MS: m/z = 424 (MH⁺)

### Example 538

### (6-Chloro-pyrazolo[1,5-a]pyridin-2-yl)-(8-methyl-3-pyridin-2-yl-5,6-dihydro-8H-[1,2,4]triazolo[4,3-a]pyrazin-7-yl)-methanone

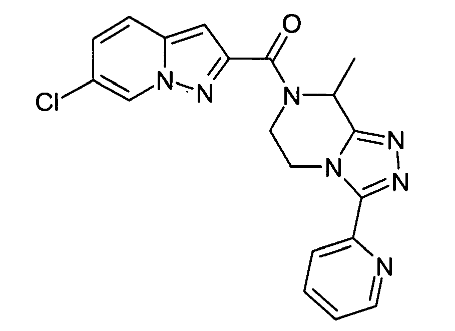

In close analogy to the procedure described in Example 1, 6-chloro-pyrazolo[1,5-a]pyridine-2-carboxylic acid is reacted with 8-methyl-3-pyridin-2-yl-5,6,7,8-tetrahydro-[1,2,4]triazolo[4,3-a]pyrazine to provide the title compound in good to moderate yield.
LC/MS: m/z = 395 (MH⁺)

The stereo-isomers of this compound are separated. The S-configurated compound has a different activity than the R-configurated compound.

### Example 539

### (6-Chloro-pyrazolo[1,5-a]pyridin-2-yl)-(3-pyridin-2-yl-5,6-dihydro-8H-[1,2,4]triazolo[4,3-a]pyrazin-7-yl)-methanone

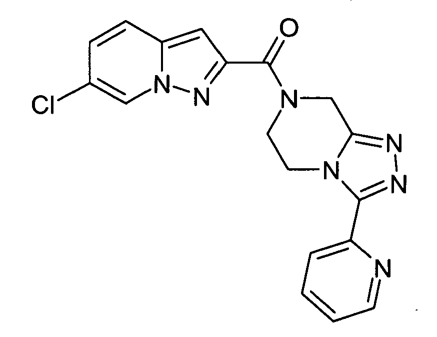

In close analogy to the procedure described in Example 1, 6-chloro-pyrazolo[1,5-a]pyridine-2-carboxylic acid is reacted with 3-pyridin-2-yl-5,6,7,8-tetrahydro-[1,2,4]triazolo[4,3-a]pyrazine to provide the title compound in good to moderate yield.
LC/MS: m/z = 381 (MH⁺)

### Example 540

### (6-Bromo-pyrazolo[1,5-a]pyridin-2-yl)-(8-methyl-3-pyridin-2-yl-5,6-dihydro-8H-[1,2,4]triazolo[4,3-a]pyrazin-7-yl)-methanone

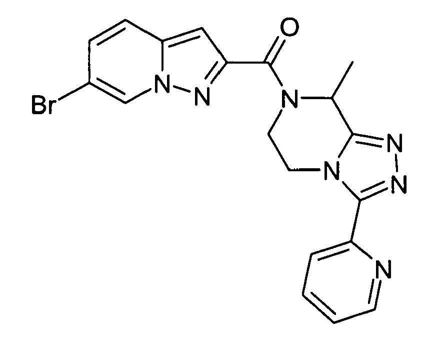

In close analogy to the procedure described in Example 1, 6-bromo-pyrazolo[1,5-a]pyridine-2-carboxylic acid is reacted with 8-methyl-3-pyridin-2-yl-5,6,7,8-tetrahydro-[1,2,4]triazolo[4,3-a]pyrazine to provide the title compound in good to moderate yield.
LC/MS: m/z = 439 (MH⁺)

The stereo-isomers of this compound are separated. The S-configurated compound has a different activity than the R-configurated compound.

### Example 541

### (6-Bromo-pyrazolo[1,5-a]pyridin-2-yl)-(3-pyridin-2-yl-5,6-dihydro-8H-[1,2,4]triazolo[4,3-a]pyrazin-7-yl)-methanone

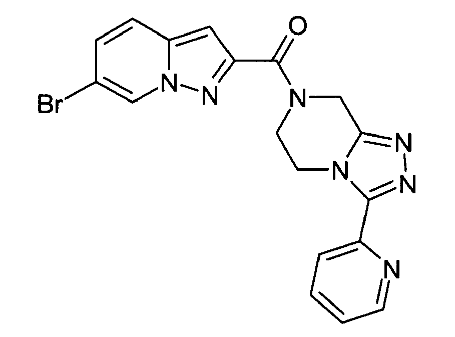

In close analogy to the procedure described in Example 1, 6-bromo-pyrazolo[1,5-a]pyridine-2-carboxylic acid is reacted with 3-pyridin-2-yl-5,6,7,8-tetrahydro-[1,2,4]triazolo[4,3-a]pyrazine to provide the title compound in good to moderate yield.
LC/MS: m/z = 425 (MH⁺)

### Example 542

### (6-Chloro-pyrazolo[1,5-a]pyridin-2-yl)-(8-methyl-3-pyridin-3-yl-5,6-dihydro-8H-[1,2,4]triazolo[4,3-a]pyrazin-7-yl)-methanone

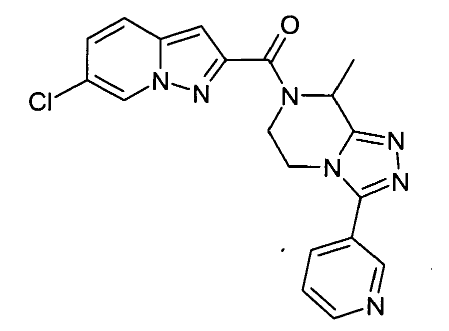

In close analogy to the procedure described in Example 1, 6-chloro-pyrazolo[1,5-a]pyridine-2-carboxylic acid is reacted with 8-methyl-3-pyridin-3-yl-5,6,7,8-tetrahydro-[1,2,4]triazolo[4,3-a]pyrazine to provide the title compound in good to moderate yield.
LC/MS: m/z = 395 (MH⁺)

The stereo-isomers of this compound are separated. The S-configurated compound has a different activity than the R-configurated compound.

### Example 543

### (6-Chloro-pyrazolo[1,5-a]pyridin-2-yl)-(3-pyridin-3-yl-5,6-dihydro-8H-[1,2,4]triazolo[4,3-a]pyrazin-7-yl)-methanone

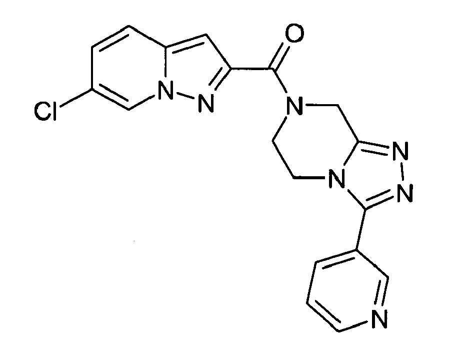

In close analogy to the procedure described in Example 1, 6-chloro-pyrazolo[1,5-a]pyridine-2-carboxylic acid is reacted with 3-pyridin-3-yl-5,6,7,8-tetrahydro-[1,2,4]triazolo[4,3-a]pyrazine to provide the title compound in good to moderate yield.
LC/MS: m/z = 381 (MH⁺)

### Example 544

### (6-Bromo-pyrazolo[1,5-a]pyridin-2-yl)-(8-methyl-3-pyridin-3-yl-5,6-dihydro-8H-[1,2,4]triazolo[4,3-a]pyrazin-7-yl)-methanone

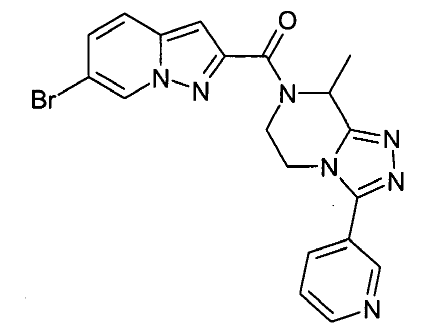

In close analogy to the procedure described in Example 1, 6-bromo-pyrazolo[1,5-a]pyridine-2-carboxylic acid is reacted with 8-methyl-3-pyridin-3-yl-5,6,7,8-tetrahydro-[1,2,4]triazolo[4,3-a]pyrazine to provide the title compound in good to moderate yield.
LC/MS: m/z = 439 (MH⁺)

The stereo-isomers of this compound are separated. The S-configurated compound has a different activity than the R-configurated compound.

### Example 545

### (6-Bromo-pyrazolo[1,5-a]pyridin-2-yl)-(3-pyridin-3-yl-5,6-dihydro-8H-[1,2,4]triazolo[4,3-a]pyrazin-7-yl)-methanone

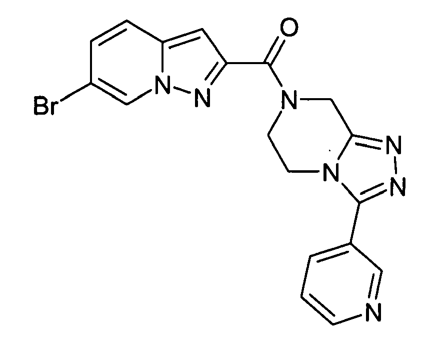

In close analogy to the procedure described in Example 1, 6-bromo-pyrazolo[1,5-a]pyridine-2-carboxylic acid is reacted with 3-pyridin-3-yl-5,6,7,8-tetrahydro-[1,2,4]triazolo[4,3-a]pyrazine to provide the title compound in good to moderate yield.
LC/MS: m/z = 425 (MH⁺)

### Example 546

### (6-Chloro-pyrazolo[1,5-a]pyridin-2-yl)-(8-methyl-3-pyridin-4-yl-5,6-dihydro-8H-[1,2,4]triazolo[4,3-a]pyrazin-7-yl)-methanone

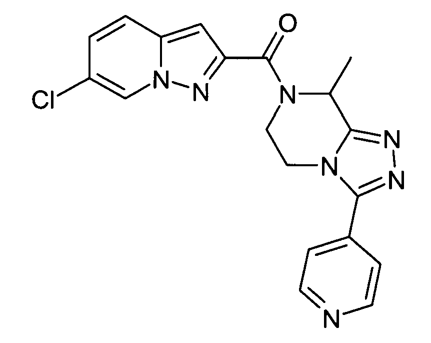

In close analogy to the procedure described in Example 1, 6-chloro-pyrazolo[1,5-a]pyridine-2-carboxylic acid is reacted with 8-methyl-3-pyridin-4-yl-5,6,7,8-tetrahydro-[1,2,4]triazolo[4,3-a]pyrazine to provide the title compound in good to moderate yield.
LC/MS: m/z = 395 (MH⁺)

The stereo-isomers of this compound are separated. The S-configurated compound has a different activity than the R-configurated compound.

### Example 547

### (6-Chloro-pyrazolo[1,5-a]pyridin-2-yl)-(3-pyridin-4-yl-5,6-dihydro-8H-[1,2,4]triazolo[4,3-a]pyrazin-7-yl)-methanone

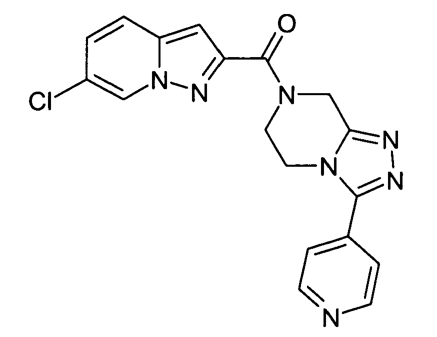

In close analogy to the procedure described in Example 1, 6-chloro-pyrazolo[1,5-a]pyridine-2-carboxylic acid is reacted with 3-pyridin-4-yl-5,6,7,8-tetrahydro-[1,2,4]triazolo[4,3-a]pyrazine to provide the title compound in good to moderate yield.
LC/MS: m/z = 381 (MH⁺)

### Example 548

### (6-Bromo-pyrazolo[1,5-a]pyridin-2-yl)-(8-methyl-3-pyridin-4-yl-5,6-dihydro-8H-[1,2,4]triazolo[4,3-a]pyrazin-7-yl)-methanone

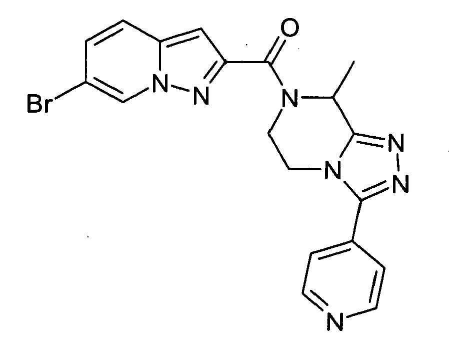

In close analogy to the procedure described in Example 1, 6-bromo-pyrazolo[1,5-a]pyridine-2-carboxylic acid is reacted with 8-methyl-3-pyridin-4-yl-5,6,7,8-tetrahydro-[1,2,4]triazolo[4,3-a]pyrazine to provide the title compound in good to moderate yield.
LC/MS: m/z = 439 (MH⁺)

The stereo-isomers of this compound are separated. The S-configurated compound has a different activity than the R-configurated compound.

### Example 549

### (6-Bromo-pyrazolo[1,5-a]pyridin-2-yl)-(3-pyridin-4-yl-5,6-dihydro-8H-[1,2,4]triazolo[4,3-a]pyrazin-7-yl)-methanone

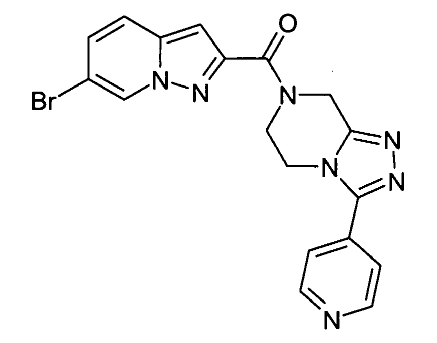

In close analogy to the procedure described in Example 1, 6-bromo-pyrazolo[1,5-a]pyridine-2-carboxylic acid is reacted with 3-pyridin-4-yl-5,6,7,8-tetrahydro-[1,2,4]triazolo[4,3-a]pyrazine to provide the title compound in good to moderate yield.
LC/MS: m/z = 425 (MH⁺)

### Example 550

### (6-Chloro-pyrazolo[1,5-a]pyridin-2-yl)-(8-methyl-3-thiophen-2-yl-5,6-dihydro-8H-[1,2,4]triazolo[4,3-a]pyrazin-7-yl)-methanone

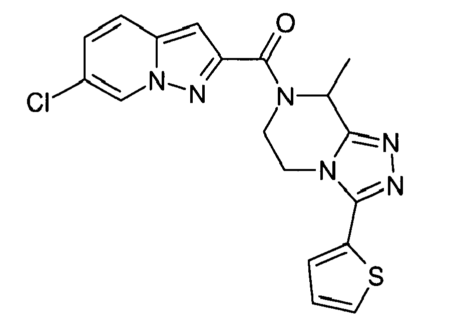

In close analogy to the procedure described in Example 1, 6-chloro-pyrazolo[1,5-a]pyridine-2-carboxylic acid is reacted with 8-methyl-3-thiophen-2-yl-5,6,7,8-tetrahydro-[1,2,4]triazolo[4,3-a]pyrazine to provide the title compound in good to moderate yield.
LC/MS: m/z = 400 (MH⁺)

The stereo-isomers of this compound are separated. The S-configurated compound has a different activity than the R-configurated compound.

### Example 551

### (6-Chloro-pyrazolo[1,5-a]pyridin-2-yl)-(3-thiophen-2-yl-5,6-dihydro-8H-[1,2,4]triazolo[4,3-a]pyrazin-7-yl)-methanone

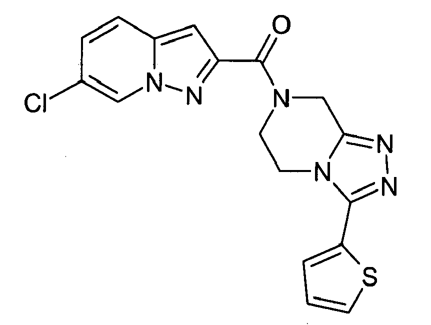

In close analogy to the procedure described in Example 1, 6-chloro-pyrazolo[1,5-a]pyridine-2-carboxylic acid is reacted with 3-thiophen-2-yl-5,6,7,8-tetrahydro-[1,2,4]triazolo[4,3-a]pyrazine to provide the title compound in good to moderate yield.
LC/MS: m/z = 386 (MH⁺)

### Example 552

### (6-Bromo-pyrazolo[1,5-a]pyridin-2-yl)-(8-methyl-3-thiophen-2-yl-5,6-dihydro-8H-[1,2,4]triazolo[4,3-a]pyrazin-7-yl)-methanone

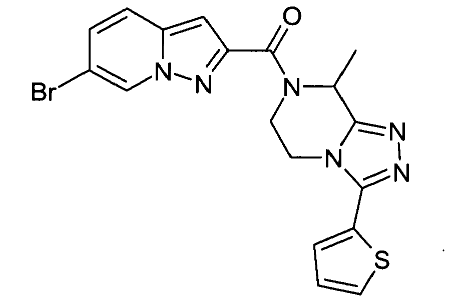

In close analogy to the procedure described in Example 1, 6-bromo-pyrazolo[1,5-a]pyridine-2-carboxylic acid is reacted with 8-methyl-3-thiophen-2-yl-5,6,7,8-tetrahydro-[1,2,4]triazolo[4,3-a]pyrazine to provide the title compound in good to moderate yield.
LC/MS: m/z = 444 (MH⁺)

The stereo-isomers of this compound are separated. The S-configurated compound has a different activity than the R-configurated compound.

### Example 553

### (6-Bromo-pyrazolo[1,5-a]pyridin-2-yl)-(3-thiophen-2-yl-5,6-dihydro-8H-[1,2,4]triazolo[4,3-a]pyrazin-7-yl)-methanone

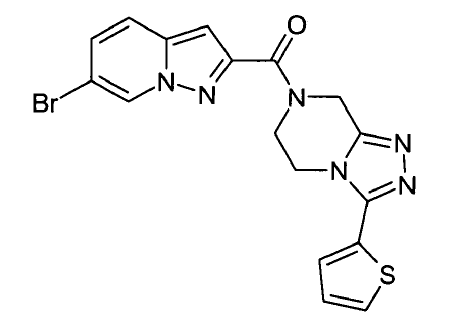

In close analogy to the procedure described in Example 1, 6-bromo-pyrazolo[1,5-a]pyridine-2-carboxylic acid is reacted with 3-thiophen-2-yl-5,6,7,8-tetrahydro-[1,2,4]triazolo[4,3-a]pyrazine to provide the title compound in good to moderate yield.
LC/MS: m/z = 430 (MH⁺)

### Example 554

### (6-Chloro-pyrazolo[1,5-a]pyridin-2-yl)-(3-furan-2-yl-8-methyl-5,6-dihydro-8H-[1,2,4]triazolo[4,3-a]pyrazin-7-yl)-methanone

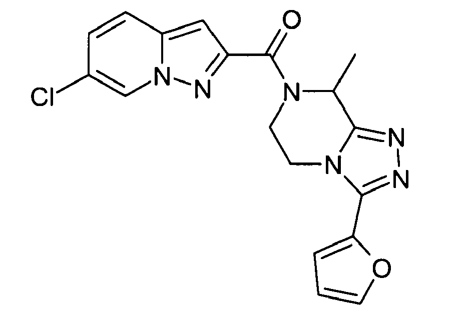

In close analogy to the procedure described in Example 1, 6-chloro-pyrazolo[1,5-a]pyridine-2-carboxylic acid is reacted with 3-furan-2-yl-8-methyl-5,6,7,8-tetrahydro-[1,2,4]triazolo[4,3-a]pyrazine to provide the title compound in good to moderate yield.
LC/MS: m/z = 384 (MH⁺)

The stereo-isomers of this compound are separated. The S-configurated compound has a different activity than the R-configurated compound.

### Example 555

### (6-Chloro-pyrazolo[1,5-a]pyridin-2-yl)-(3-furan-2-yl-5,6-dihydro-8H-[1,2,4]triazolo[4,3-a]pyrazin-7-yl)-methanone

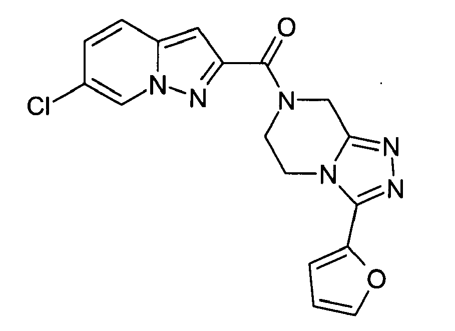

In close analogy to the procedure described in Example 1, 6-chloro-pyrazolo[1,5-a]pyridine-2-carboxylic acid is reacted with 3-furan-2-yl-5,6,7,8-tetrahydro-[1,2,4]triazolo[4,3-a]pyrazine to provide the title compound in good to moderate yield.
LC/MS: m/z = 370 (MH⁺)

### Example 556

### (6-Bromo-pyrazolo[1,5-a]pyridin-2-yl)-(3-furan-2-yl-8-methyl-5,6-dihydro-8H-[1,2,4]triazolo[4,3-a]pyrazin-7-yl)-methanone

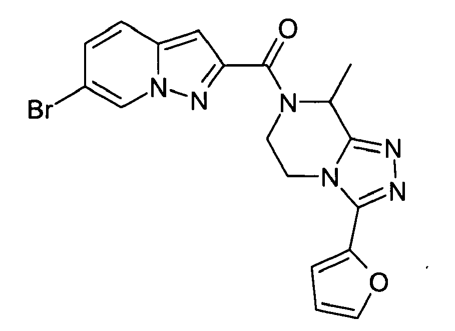

In close analogy to the procedure described in Example 1, 6-bromo-pyrazolo[1,5-a]pyridine-2-carboxylic acid is reacted with 3-furan-2-yl-8-methyl-5,6,7,8-tetrahydro-[1,2,4]triazolo[4,3-a]pyrazine to provide the title compound in good to moderate yield.
LC/MS: m/z = 428 (MH⁺)

The stereo-isomers of this compound are separated. The S-configurated compound has a different activity than the R-configurated compound.

### Example 557

### (6-Bromo-pyrazolo[1,5-a]pyridin-2-yl)-(3-furan-2-yl-5,6-dihydro-8H-[1,2,4]triazolo[4,3-a]pyrazin-7-yl)-methanone

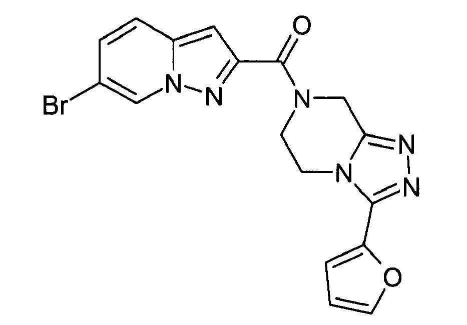

In close analogy to the procedure described in Example 1, 6-bromo-pyrazolo[1,5-a]pyridine-2-carboxylic acid is reacted with 3-furan-2-yl-5,6,7,8-tetrahydro-[1,2,4]triazolo[4,3-a]pyrazine to provide the title compound in good to moderate yield.
LC/MS: m/z = 414 (MH⁺)

### Example 558

### (6-Chloro-pyrazolo[1,5-a]pyridin-2-yl)-(8-methyl-3-thiophen-3-yl-5,6-dihydro-8H-[1,2,4]triazolo[4,3-a]pyrazin-7-yl)-methanone

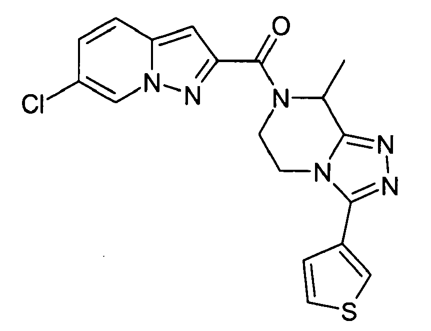

In close analogy to the procedure described in Example 1, 6-chloro-pyrazolo[1,5-a]pyridine-2-carboxylic acid is reacted with 8-methyl-3-thiophen-3-yl-5,6,7,8-tetrahydro-[1,2,4]triazolo[4,3-a]pyrazine to provide the title compound in good to moderate yield.
LC/MS: m/z = 400 (MH⁺)

The stereo-isomers of this compound are separated. The S-configurated compound has a different activity than the R-configurated compound.

### Example 559

### (6-Chloro-pyrazolo[1,5-a]pyridin-2-yl)-(3-thiophen-3-yl-5,6-dihydro-8H-[1,2,4]triazolo[4,3-a]pyrazin-7-yl)-methanone

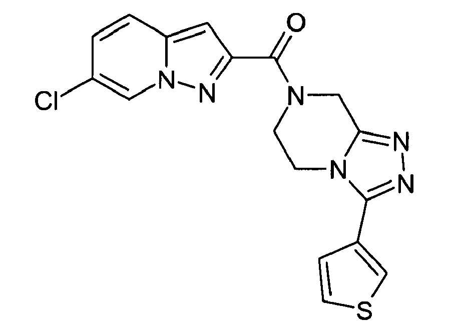

In close analogy to the procedure described in Example 1, 6-chloro-pyrazolo[1,5-a]pyridine-2-carboxylic acid is reacted with 3-thiophen-3-yl-5,6,7,8-tetrahydro-[1,2,4]triazolo[4,3-a]pyrazine to provide the title compound in good to moderate yield.
LC/MS: m/z = 386 (MH⁺)

### Example 560

### (6-Bromo-pyrazolo[1,5-a]pyridin-2-yl)-(8-methyl-3-thiophen-3-yl-5,6-dihydro-8H-[1,2,4]triazolo[4,3-a]pyrazin-7-yl)-methanone

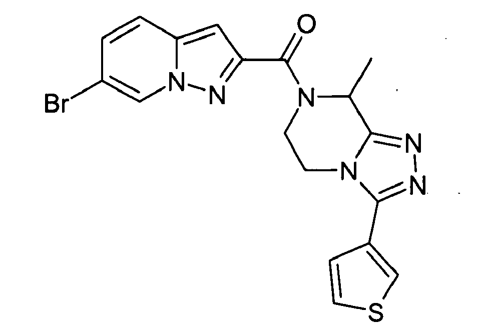

In close analogy to the procedure described in Example 1, 6-bromo-pyrazolo[1,5-a]pyridine-2-carboxylic acid is reacted with 8-methyl-3-thiophen-3-yl-5,6,7,8-tetrahydro-[1,2,4]triazolo[4,3-a]pyrazine to provide the title compound in good to moderate yield.
LC/MS: m/z = 444 (MH⁺)

The stereo-isomers of this compound are separated. The S-configurated compound has a different activity than the R-configurated compound.

### Example 561

### (6-Bromo-pyrazolo[1,5-a]pyridin-2-yl)-(3-thiophen-3-yl-5,6-dihydro-8H-[1,2,4]triazolo[4,3-a]pyrazin-7-yl)-methanone

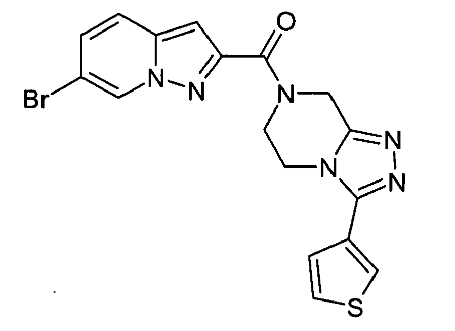

In close analogy to the procedure described in Example 1, 6-bromo-pyrazolo[1,5-a]pyridine-2-carboxylic acid is reacted with 3-thiophen-3-yl-5,6,7,8-tetrahydro-[1,2,4]triazolo[4,3-a]pyrazine to provide the title compound in good to moderate yield.
LC/MS: m/z = 430 (MH⁺)

### Example 562

### (6-Chloro-pyrazolo[1,5-a]pyridin-2-yl)-(3-furan-3-yl-8-methyl-5,6-dihydro-8H-[1,2,4]triazolo[4,3-a]pyrazin-7-yl)-methanone

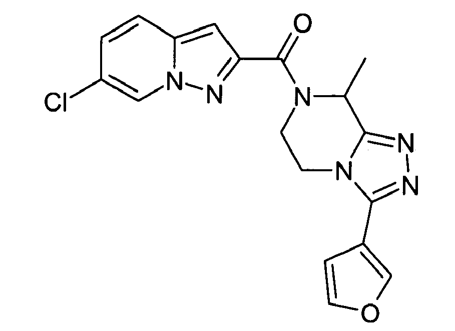

In close analogy to the procedure described in Example 1, 6-chloro-pyrazolo[1,5-a]pyridine-2-carboxylic acid is reacted with 3-furan-3-yl-8-methyl-5,6,7,8-tetrahydro-[1,2,4]triazolo[4,3-a]pyrazine to provide the title compound in good to moderate yield.
LC/MS: m/z = 384 (MH⁺)

The stereo-isomers of this compound are separated. The S-configurated compound has a different activity than the R-configurated compound.

### Example 563

### (6-Chloro-pyrazolo[1,5-a]pyridin-2-yl)-(3-furan-3-yl-5,6-dihydro-8H-[1,2,4]triazolo[4,3-a]pyrazin-7-yl)-methanone

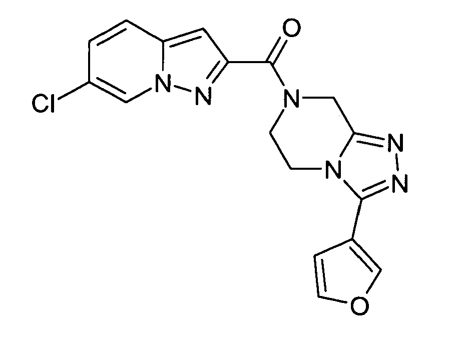

In close analogy to the procedure described in Example 1, 6-chloro-pyrazolo[1,5-a]pyridine-2-carboxylic acid is reacted with 3-furan-3-yl-5,6,7,8-tetrahydro-[1,2,4]triazolo[4,3-a]pyrazine to provide the title compound in good to moderate yield.
LC/MS: m/z = 370 (MH⁺)

### Example 564

### (6-Bromo-pyrazolo[1,5-a]pyridin-2-yl)-(3-furan-3-yl-8-methyl-5,6-dihydro-8H-[1,2,4]triazolo[4,3-a]pyrazin-7-yl)-methanone

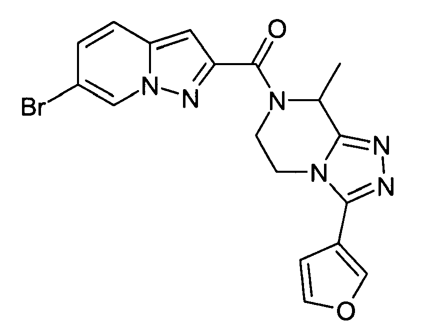

In close analogy to the procedure described in Example 1, 6-bromo-pyrazolo[1,5-a]pyridine-2-carboxylic acid is reacted with 3-furan-3-yl-8-methyl-5,6,7,8-tetrahydro-[1,2,4]triazolo[4,3-a]pyrazine to provide the title compound in good to moderate yield.
LC/MS: m/z = 428 (MH⁺)

The stereo-isomers of this compound are separated. The S-configurated compound has a different activity than the R-configurated compound.

### Example 565

### (6-Bromo-pyrazolo[1,5-a]pyridin-2-yl)-(3-furan-3-yl-5,6-dihydro-8H-[1,2,4]triazolo[4,3-a]pyrazin-7-yl)-methanone

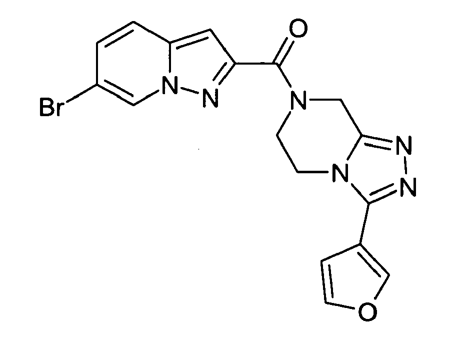

In close analogy to the procedure described in Example 1, 6-bromo-pyrazolo[1,5-a]pyridine-2-carboxylic acid is reacted with 3-furan-3-yl-5,6,7,8-tetrahydro-[1,2,4]triazolo[4,3-a]pyrazine to provide the title compound in good to moderate yield.
LC/MS: m/z = 414 (MH⁺)

### Example 566

### (6-Chloro-pyrazolo[1,5-a]pyridin-2-yl)-(8-methyl-3-pyrimidin-4-yl-5,6-dihydro-8H-[1,2,4]triazolo[4,3-a]pyrazin-7-yl)-methanone

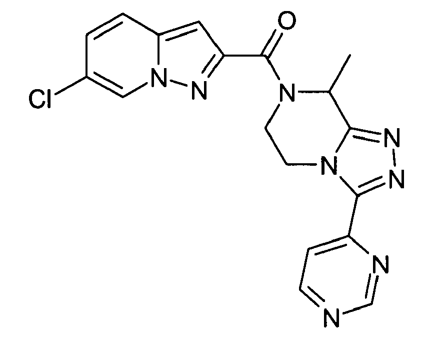

In close analogy to the procedure described in Example 1, 6-chloro-pyrazolo[1,5-a]pyridine-2-carboxylic acid is reacted with 8-methyl-3-pyrimidin-4-yl-5,6,7,8-tetrahydro-[1,2,4]triazolo[4,3-a]pyrazine to provide the title compound in good to moderate yield.
LC/MS: m/z = 396 (MH⁺)

The stereo-isomers of this compound are separated. The S-configurated compound has a different activity than the R-configurated compound.

### Example 567

### (6-Chloro-pyrazolo[1,5-a]pyridin-2-yl)-(3-pyrimidin-4-yl-5,6-dihydro-8H-[1,2,4]triazolo[4,3-a]pyrazin-7-yl)-methanone

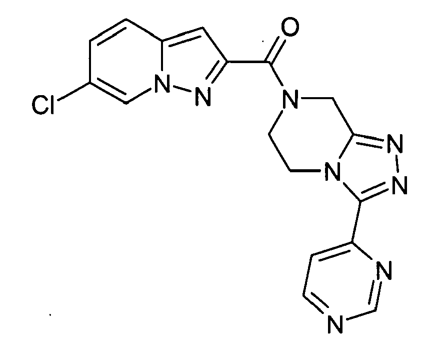

In close analogy to the procedure described in Example 1, 6-chloro-pyrazolo[1,5-a]pyridine-2-carboxylic acid is reacted with 3-pyrimidin-4-yl-5,6,7,8-tetrahydro-[1,2,4]triazolo[4,3-a]pyrazine to provide the title compound in good to moderate yield.
LC/MS: m/z = 382 (MH⁺)

### Example 568

### (6-Bromo-pyrazolo[1,5-a]pyridin-2-yl)-(8-methyl-3-pyrimidin-4-yl-5,6-dihydro-8H-[1,2,4]triazolo[4,3-a]pyrazin-7-yl)-methanone

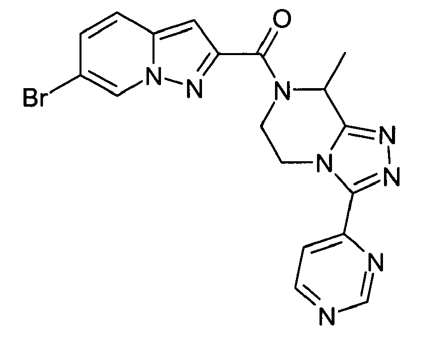

In close analogy to the procedure described in Example 1, 6-bromo-pyrazolo[1,5-a]pyridine-2-carboxylic acid is reacted with 8-methyl-3-pyrimidin-4-yl-5,6,7,8-tetrahydro-[1,2,4]triazolo[4,3-a]pyrazine to provide the title compound in good to moderate yield.
LC/MS: m/z = 440 (MH⁺)

The stereo-isomers of this compound are separated. The S-configurated compound has a different activity than the R-configurated compound.

### Example 569

### (6-Bromo-pyrazolo[1,5-a]pyridin-2-yl)-(3-pyrimidin-4-yl-5,6-dihydro-8H-[1,2,4]triazolo[4,3-a]pyrazin-7-yl)-methanone

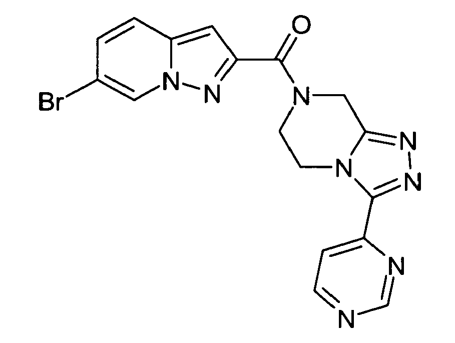

In close analogy to the procedure described in Example 1, 6-bromo-pyrazolo[1,5-a]pyridine-2-carboxylic acid is reacted with 3-Pyrimidin-4-yl-5,6,7,8-tetrahydro-[1,2,4]triazolo[4,3-a]pyrazine to provide the title compound in good to moderate yield.
LC/MS: m/z = 426 (MH⁺)

### Example 570

### (6-Chloro-pyrazolo[1,5-a]pyridin-2-yl)-(8-methyl-3-pyrimidin-5-yl-5,6-dihydro-8H-[1,2,4]triazolo[4,3-a]pyrazin-7-yl)-methanone

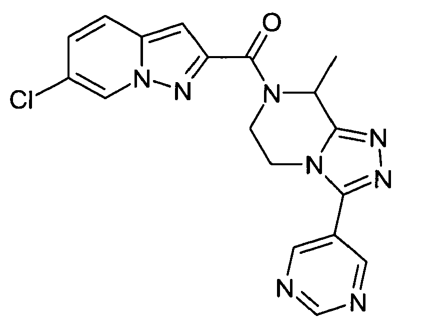

In close analogy to the procedure described in Example 1, 6-chloro-pyrazolo[1,5-a]pyridine-2-carboxylic acid is reacted with 8-methyl-3-pyrimidin-5-yl-5,6,7,8-tetrahydro-[1,2,4]triazolo[4,3-a]pyrazine to provide the title compound in good to moderate yield.
LC/MS: m/z = 396 (MH⁺)

The stereo-isomers of this compound are separated. The S-configurated compound has a different activity than the R-configurated compound.

### Example 571

### (6-Chloro-pyrazolo[1,5-a]pyridin-2-yl)-(3-pyrimidin-5-yl-5,6-dihydro-8H-[1,2,4]triazolo[4,3-a]pyrazin-7-yl)-methanone

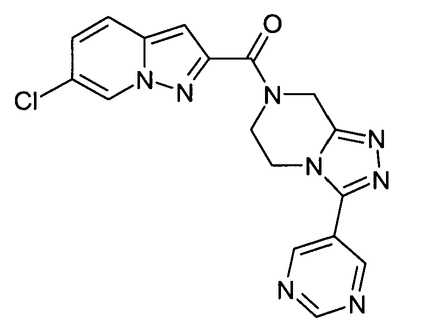

In close analogy to the procedure described in Example 1, 6-chloro-pyrazolo[1,5-a]pyridine-2-carboxylic acid is reacted with 3-pyrimidin-5-yl-5,6,7,8-tetrahydro-[1,2,4]triazolo[4,3-a]pyrazine to provide the title compound in good to moderate yield.
LC/MS: m/z = 382 (MH⁺)

### Example 572

### (6-Bromo-pyrazolo[1,5-a]pyridin-2-yl)-(8-methyl-3-pyrimidin-5-yl-5,6-dihydro-8H-[1,2,4]triazolo[4,3-a]pyrazin-7-yl)-methanone

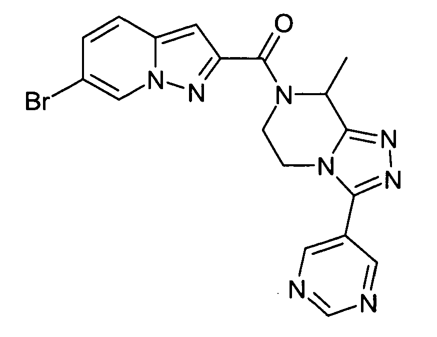

In close analogy to the procedure described in Example 1, 6-bromo-pyrazolo[1,5-a]pyridine-2-carboxylic acid is reacted with 8-methyl-3-pyrimidin-5-yl-5,6,7,8-tetrahydro-[1,2,4]triazolo[4,3-a]pyrazine to provide the title compound in good to moderate yield.
LC/MS: m/z = 440(MH⁺)

The stereo-isomers of this compound are separated. The S-configurated compound has a different activity than the R-configurated compound.

### Example 573

### (6-Bromo-pyrazolo[1,5-a]pyridin-2-yl)-(3-pyrimidin-5-yl-5,6-dihydro-8H-[1,2,4]triazolo[4,3-a]pyrazin-7-yl)-methanone

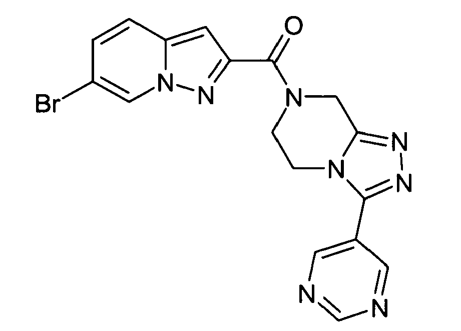

In close analogy to the procedure described in Example 1, 6-bromo-pyrazolo[1,5-a]pyridine-2-carboxylic acid is reacted with 3-pyrimidin-5-yl-5,6,7,8-tetrahydro-[1,2,4]triazolo[4,3-a]pyrazine to provide the title compound in good to moderate yield.
LC/MS: m/z = 426 (MH⁺)

### Example 574

### (6-Chloro-pyrazolo[1,5-a]pyridin-2-yl)-[3-(6-fluoro-pyridin-3-yl)-8-methyl-5,6-dihydro-8H-imidazo[1,2-a]pyrazin-7-yl]-methanone

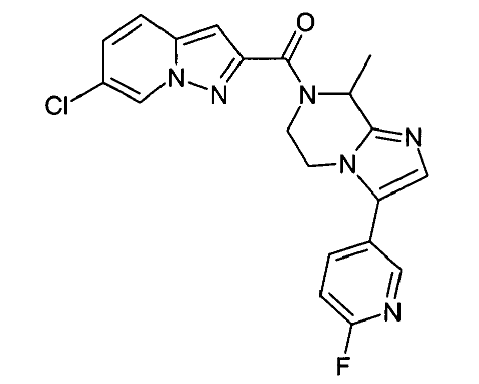

In close analogy to the procedure described in Example 1, 6-chloro-pyrazolo[1,5-a]pyridine-2-carboxylic acid is reacted with 3-(6-fluoro-pyridin-3-yl)-8-methyl-5,6,7,8-tetrahydro-imidazo[1,2-a]pyrazine to provide the title compound in good to moderate yield.
LC/MS: m/z = 412 (MH⁺)

The stereo-isomers of this compound are separated. The S-configurated compound has a different activity than the R-configurated compound.

### Example 575

### (6-Chloro-pyrazolo[1,5-a]pyridin-2-yl)-[3-(6-fluoro-pyridin-3-yl)-5,6-dihydro-8H-imidazo[1,2-a]pyrazin-7-yl]-methanone

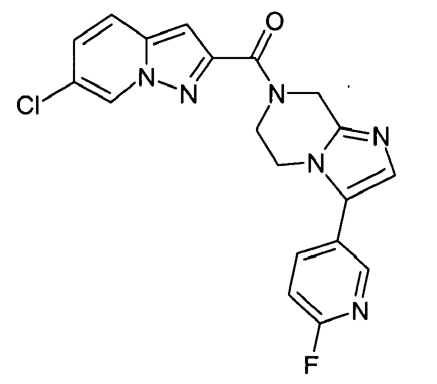

In close analogy to the procedure described in Example 1, 6-chloro-pyrazolo[1,5-a]pyridine-2-carboxylic acid is reacted with 3-(6-fluoro-pyridin-3-yl)-5,6,7,8-tetrahydro-imidazo[1,2-a]pyrazine to provide the title compound in good to moderate yield.
LC/MS: m/z = 398 (MH⁺)

### Example 576

### (6-Bromo-pyrazolo[1,5-a]pyridin-2-yl)-[3-(6-fluoro-pyridin-3-yl)-8-methyl-5,6-dihydro-8H-imidazo[1,2-a]pyrazin-7-yl]-methanone

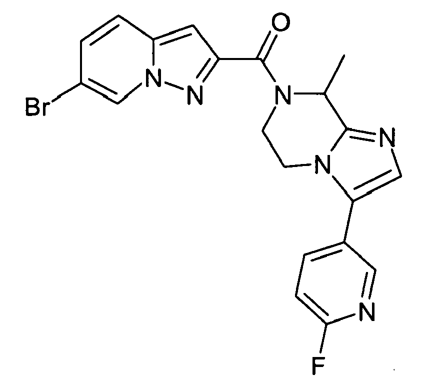

In close analogy to the procedure described in Example 1, 6-bromo-pyrazolo[1,5-a]pyridine-2-carboxylic acid is reacted with 3-(6-fluoro-pyridin-3-yl)-8-methyl-5,6,7,8-tetrahydro-imidazo[1,2-a]pyrazine to provide the title compound in good to moderate yield.
LC/MS: m/z = 456 (MH⁺)

The stereo-isomers of this compound are separated. The S-configurated compound has a different activity than the R-configurated compound.

### Example 577

### (6-Bromo-pyrazolo[1,5-a]pyridin-2-yl)-[3-(6-fluoro-pyridin-3-yl)-5,6-dihydro-8H-imidazo[1,2-a]pyrazin-7-yl]-methanone

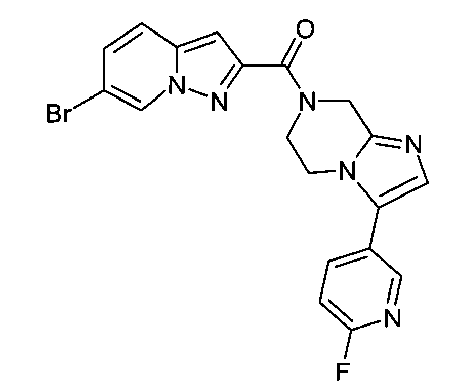

In close analogy to the procedure described in Example 1, 6-bromo-pyrazolo[1,5-a]pyridine-2-carboxylic acid is reacted with 3-(6-fluoro-pyridin-3-yl)-5,6,7,8-tetrahydro-imidazo[1,2-a]pyrazine to provide the title compound in good to moderate yield.
LC/MS: m/z = 442 (MH⁺)

### Example 578

### (6-Chloro-pyrazolo[1,5-a]pyridin-2-yl)-[3-(2-fluoro-pyridin-3-yl)-8-methyl-5,6-dihydro-8H-imidazo[1,2-a]pyrazin-7-yl]-methanone

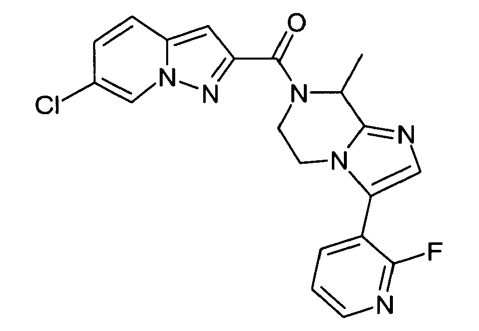

In close analogy to the procedure described in Example 1, 6-chloro-pyrazolo[1,5-a]pyridine-2-carboxylic acid is reacted with 3-(2-fluoro-pyridin-3-yl)-8-methyl-5,6,7,8-tetrahydro-imidazo[1,2-a]pyrazine to provide the title compound in good to moderate yield.
LC/MS: m/z = 412 (MH⁺)

The stereo-isomers of this compound are separated. The S-configurated compound has a different activity than the R-configurated compound.

### Example 579

### (6-Chloro-pyrazolo[1,5-a]pyridin-2-yl)-[3-(2-fluoro-pyridin-3-yl)-5,6-dihydro-8H-imidazo[1,2-a]pyrazin-7-yl]-methanone

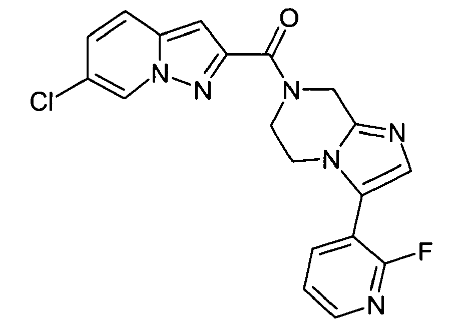

In close analogy to the procedure described in Example 1, 6-chloro-pyrazolo[1,5-a]pyridine-2-carboxylic acid is reacted with 3-(2-fluoro-pyridin-3-yl)-5,6,7,8-tetrahydro-imidazo[1,2-a]pyrazine to provide the title compound in good to moderate yield.
LC/MS: m/z = 398 (MH⁺)

### Example 580

### (6-Bromo-pyrazolo[1,5-a]pyridin-2-yl)-[3-(2-fluoro-pyridin-3-yl)-8-methyl-5,6-dihydro-8H-imidazo[1,2-a]pyrazin-7-yl]-methanone

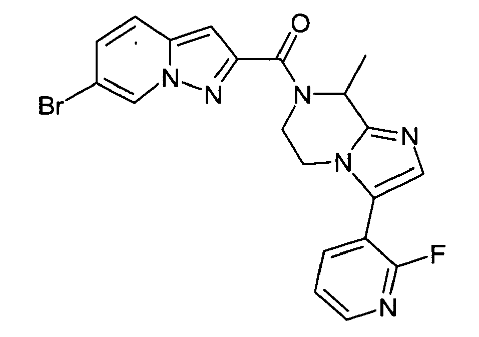

In close analogy to the procedure described in Example 1, 6-bromo-pyrazolo[1,5-a]pyridine-2-carboxylic acid is reacted with 3-(2-fluoro-pyridin-3-yl)-8-methyl-5,6,7,8-tetrahydro-imidazo[1,2-a]pyrazine to provide the title compound in good to moderate yield.
LC/MS: m/z = 456 (MH⁺)

The stereo-isomers of this compound are separated. The S-configurated compound has a different activity than the R-configurated compound.

### Example 581

### (6-Bromo-pyrazolo[ 1,5-a]pyridin-2-yl)-[3-(2-fluoro-pyridin-3-yl)-5,6-dihydro-8H-imidazo[1,2-a]pyrazin-7-yl]-methanone

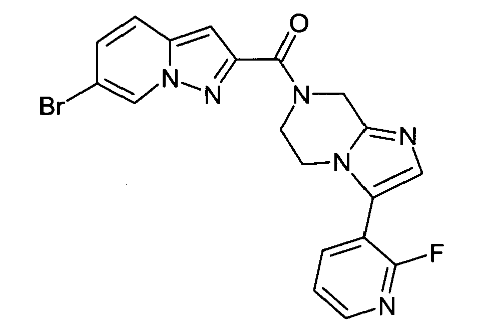

In close analogy to the procedure described in Example 1, 6-bromo-pyrazolo[1,5-a]pyridine-2-carboxylic acid is reacted with 3-(2-fluoro-pyridin-3-yl)-5,6,7,8-tetrahydro-imidazo[1,2-a]pyrazine to provide the title compound in good to moderate yield.
LC/MS: m/z = 442 (MH⁺)

### Example 582

### (6-Chloro-pyrazolo[1,5-a]pyridin-2-yl)-[2-(6-fluoro-pyridin-3-yl)-8-methyl-5,6-dihydro-8H-imidazo[1,2-a]pyrazin-7-yl]-methanone

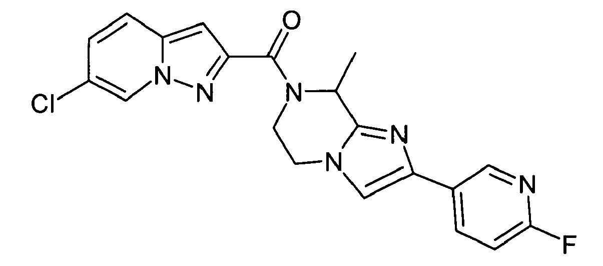

In close analogy to the procedure described in Example 1, 6-chloro-pyrazolo[1,5-a]pyridine-2-carboxylic acid is reacted with 2-(6-fluoro-pyridin-3-yl)-8-methyl-5,6,7,8-tetrahydro-imidazo[1,2-a]pyrazine to provide the title compound in good to moderate yield.
LC/MS: m/z = 412 (MH⁺)

The stereo-isomers of this compound are separated. The S-configurated compound has a different activity than the R-configurated compound.

### Example 583

### (6-Chloro-pyrazolo[1,5-a]pyridin-2-yl)-[2-(6-fluoro-pyridin-3-yl)-5,6-dihydro-8H-imidazo[1,2-a]pyrazin-7-yl]-methanone

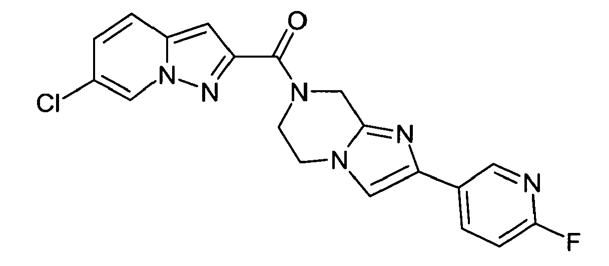

In close analogy to the procedure described in Example 1, 6-chloro-pyrazolo[1,5-a]pyridine-2-carboxylic acid is reacted with 2-(6-fluoro-pyridin-3-yl)-5,6,7,8-tetrahydro-imidazo[1,2-a]pyrazine to provide the title compound in good to moderate yield.
LC/MS: m/z = 398 (MH⁺)

### Example 584

### (6-Bromo-pyrazolo[1,5-a]pyridin-2-yl)-[2-(6-fluoro-pyridin-3-yl)-8-methyl-5,6-dihydro-8H-imidazo[1,2-a]pyrazin-7-yl]-methanone

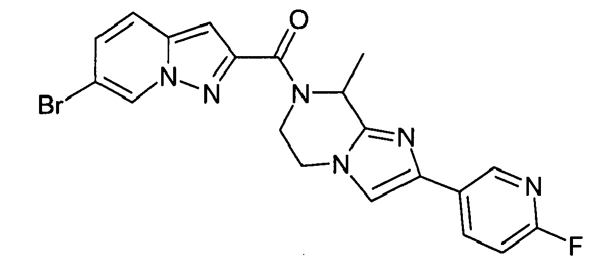

In close analogy to the procedure described in Example 1, 6-bromo-pyrazolo[1,5-a]pyridine-2-carboxylic acid is reacted with 2-(6-fluoro-pyridin-3-yl)-8-methyl-5,6,7,8-tetrahydro-imidazo[1,2-a]pyrazine to provide the title compound in good to moderate yield.
LC/MS: m/z = 456 (MH⁺)

The stereo-isomers of this compound are separated. The S-configurated compound has a different activity than the R-configurated compound.

### Example 585

### (6-Bromo-pyrazolo[1,5-a]pyridin-2-yl)-[2-(6-fluoro-pyridin-3-yl)-5,6-dihydro-8H-imidazo[1,2-a]pyrazin-7-yl]-methanone

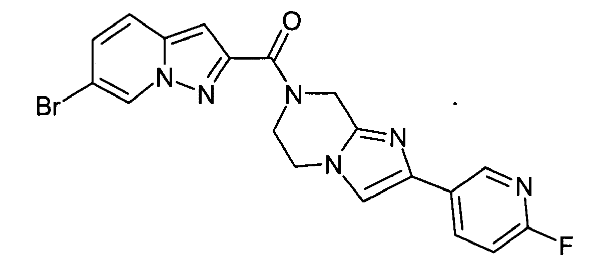

In close analogy to the procedure described in Example 1, 6-bromo-pyrazolo[1,5-a]pyridine-2-carboxylic acid is reacted with 2-(6-fluoro-pyridin-3-yl)-5,6,7,8-tetrahydro-imidazo[1,2-a]pyrazine to provide the title compound in good to moderate yield.
LC/MS: m/z = 442 (MH⁺)

### Example 586

### (6-Chloro-pyrazolo[1,5-a]pyridin-2-yl)-[2-(2-fluoro-pyridin-3-yl)-8-methyl-5,6-dihydro-8H-imidazo[1,2-a]pyrazin-7-yl]-methanone

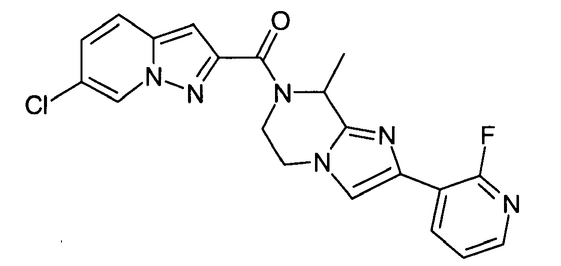

In close analogy to the procedure described in Example 1, 6-chloro-pyrazolo[1,5-a]pyridine-2-carboxylic acid is reacted with 2-(2-fluoro-pyridin-3-yl)-8-methyl-5,6,7,8-tetrahydro-imidazo[1,2-a]pyrazine to provide the title compound in good to moderate yield.
LC/MS: m/z = 412 (MH⁺)

The stereo-isomers of this compound are separated. The S-configurated compound has a different activity than the R-configurated compound.

### Example 587

### (6-Chloro-pyrazolo[1,5-a]pyridin-2-yl)-[2-(2-fluoro-pyridin-3-yl)-5,6-dihydro-8H-imidazo[1,2-a]pyrazin-7-yl]-methanone

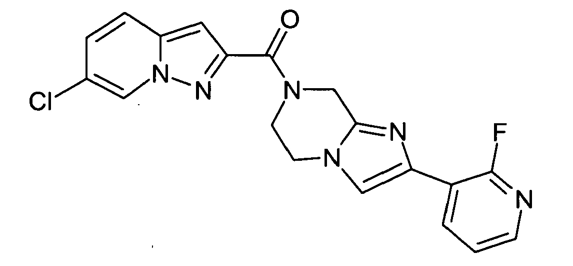

In close analogy to the procedure described in Example 1, 6-chloro-pyrazolo[1,5-a]pyridine-2-carboxylic acid is reacted with 2-(2-fluoro-pyridin-3-yl)-5,6,7,8-tetrahydro-imidazo[1,2-a]pyrazine to provide the title compound in good to moderate yield.
LC/MS: m/z = 398 (MH⁺)

### Example 588

### (6-Bromo-pyrazolo[1,5-a]pyridin-2-yl)-[2-(2-fluoro-pyridin-3-yl)-8-methyl-5,6-dihydro-8H-imidazo[1,2-a]pyrazin-7-yl]-methanone

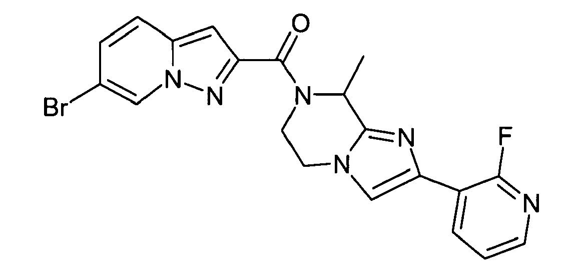

In close analogy to the procedure described in Example 1, 6-bromo-pyrazolo[1,5-a]pyridine-2-carboxylic acid is reacted with 2-(2-fluoro-pyridin-3-yl)-8-methyl-5,6,7,8-tetrahydro-imidazo[1,2-a]pyrazine to provide the title compound in good to moderate yield.
LC/MS: m/z = 456 (MH⁺)

The stereo-isomers of this compound are separated. The S-configurated compound has a different activity than the R-configurated compound.

### Example 589

### (6-Bromo-pyrazolo[1,5-a]pyridin-2-yl)-[2-(2-fluoro-pyridin-3-yl)-5,6-dihydro-8H-imidazo[1,2-a]pyrazin-7-yl]-methanone

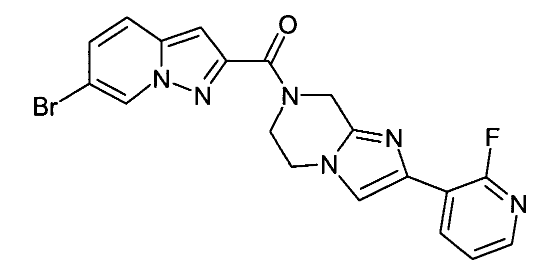

In close analogy to the procedure described in Example 1, 6-bromo-pyrazolo[1,5-a]pyridine-2-carboxylic acid is reacted with 2-(2-fluoro-pyridin-3-yl)-5,6,7,8-tetrahydro-imidazo[1,2-a]pyrazine to provide the title compound in good to moderate yield.
LC/MS: m/z = 442 (MH⁺)

### Example 590

### (6-Chloro-pyrazolo[1,5-a]pyridin-2-yl)-[3-(6-fluoro-pyridin-3-yl)-8-methyl-5,6-dihydro-8H-[1,2,4]triazolo[4,3-a]pyrazin-7-yl]-methanone

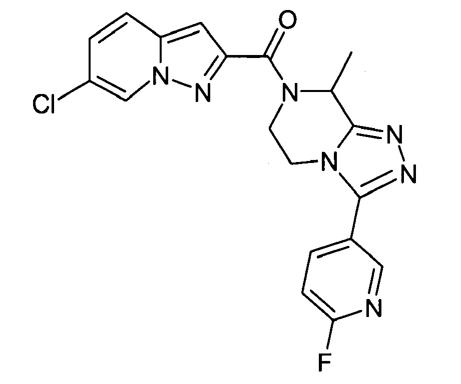

In close analogy to the procedure described in Example 1, 6-chloro-pyrazolo[1,5-a]pyridine-2-carboxylic acid is reacted with 3-(6-fluoro-pyridin-3-yl)-8-methyl-5,6,7,8-tetrahydro-[1,2,4]triazolo[4,3-a]pyrazine to provide the title compound in good to moderate yield.
LC/MS: m/z = 412 (MH⁺)

The stereo-isomers of this compound are separated. The S-configurated compound has a different activity than the R-configurated compound.

### Example 591

### (6-Chloro-pyrazolo[1,5-a]pyridin-2-yl)-[3-(6-fluoro-pyridin-3-yl)-5,6-dihydro-8H-[1,2,4]triazolo[4,3-a]pyrazin-7-yl]-methanone

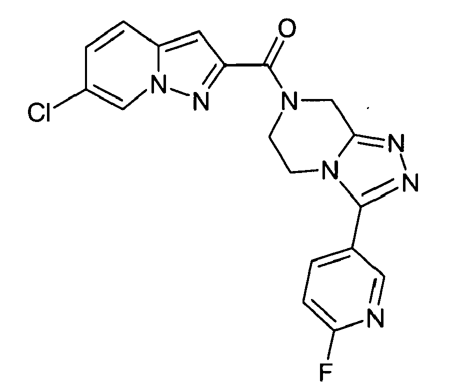

In close analogy to the procedure described in Example 1, 6-chloro-pyrazolo[1,5-a]pyridine-2-carboxylic acid is reacted with 3-(6-fluoro-pyridin-3-yl)-5,6,7,8-tetrahydro-[1,2,4]triazolo[4,3-a]pyrazine to provide the title compound in good to moderate yield.
LC/MS: m/z = 398 (MH⁺)

### Example 592

### (6-Bromo-pyrazolo[1,5-a]pyridin-2-yl)-[3-(6-fluoro-pyridin-3-yl)-8-methyl-5,6-dihydro-8H-[1,2,4]triazolo[4,3-a]pyrazin-7-yl]-methanone

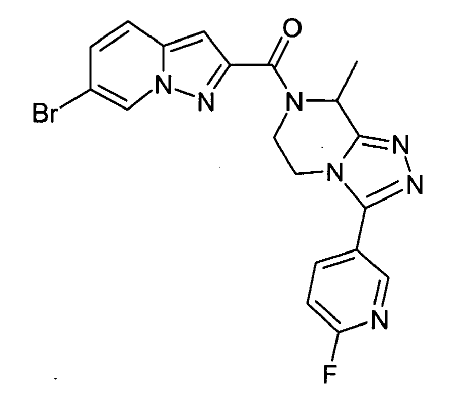

In close analogy to the procedure described in Example 1, 6-bromo-pyrazolo[1,5-a]pyridine-2-carboxylic acid is reacted with 3-(6-fluoro-pyridin-3-yl)-8-methyl-5,6,7,8-tetrahydro-[1,2,4]triazolo[4,3-a]pyrazine to provide the title compound in good to moderate yield.
LC/MS: m/z = 457 (MH⁺)

The stereo-isomers of this compound are separated. The S-configurated compound has a different activity than the R-configurated compound.

### Example 593

### (6-Bromo-pyrazolo[1,5-a]pyridin-2-yl)-[3-(6-fluoro-pyridin-3-yl)-5,6-dihydro-8H-[1,2,4]triazolo[4,3-a]pyrazin-7-yl]-methanone

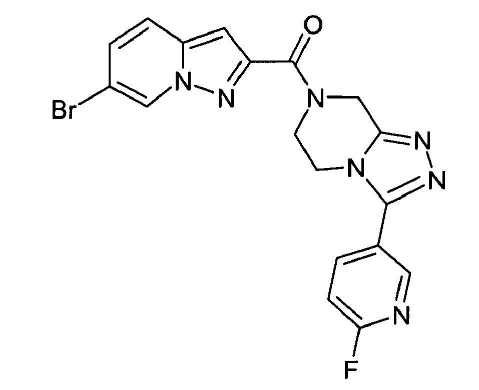

In close analogy to the procedure described in Example 1, 6-bromo-pyrazolo[1,5-a]pyridine-2-carboxylic acid is reacted with 3-(6-fluoro-pyridin-3-yl)-5,6,7,8-tetrahydro-[1,2,4]triazolo[4,3-a]pyrazine to provide the title compound in good to moderate yield.
LC/MS: m/z = 443 (MH⁺)

### Example 594

### (6-Chloro-pyrazolo[1,5-a]pyridin-2-yl)-[3-(2-fluoro-pyridin-3-yl)-8-methyl-5,6-dihydro-8H-[1,2,4]triazolo[4,3-a]pyrazin-7-yl]-methanone

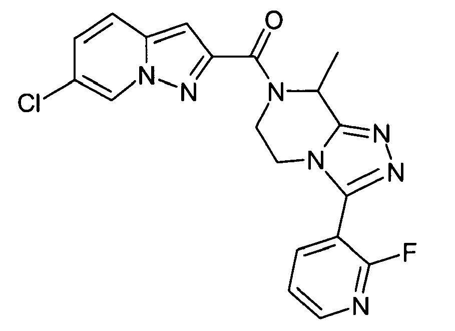

In close analogy to the procedure described in Example 1, 6-chloro-pyrazolo[1,5-a]pyridine-2-carboxylic acid is reacted with 3-(2-fluoro-pyridin-3-yl)-8-methyl-5,6,7,8-tetrahydro-[1,2,4]triazolo[4,3-a]pyrazine to provide the title compound in good to moderate yield.
LC/MS: m/z = 413 (MH⁺)

The stereo-isomers of this compound are separated. The S-configurated compound has a different activity than the R-configurated compound.

### Example 595

### (6-Chloro-pyrazolo[1,5-a]pyridin-2-yl)-[3-(2-fluoro-pyridin-3-yl)-5,6-dihydro-8H-[1,2,4]triazolo[4,3-a]pyrazin-7-yl]-methanone

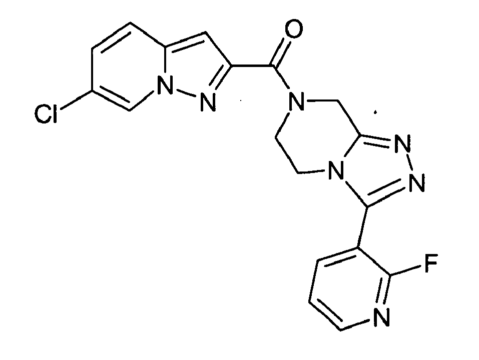

In close analogy to the procedure described in Example 1, 6-chloro-pyrazolo[1,5-a]pyridine-2-carboxylic acid is reacted with 3-(2-fluoro-pyridin-3-yl)-5,6,7,8-tetrahydro-[1,2,4]triazolo[4,3-a]pyrazine to provide the title compound in good to moderate yield.
LC/MS: m/z = 400 (MH⁺)

### Example 596

### (6-Bromo-pyrazolo[1,5-a]pyridin-2-yl)-[3-(2-fluoro-pyridin-3-yl)-8-methyl-5,6-dihydro-8H-[1,2,4]triazolo[4,3-a]pyrazin-7-yl]-methanone

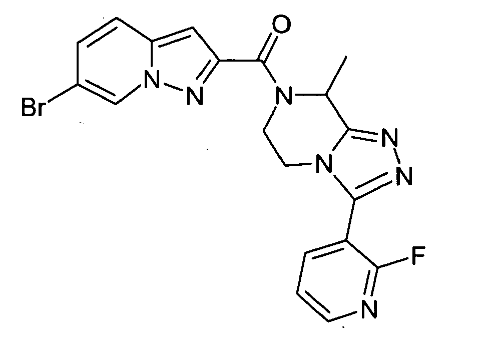

In close analogy to the procedure described in Example 1, 6-bromo-pyrazolo[1,5-a]pyridine-2-carboxylic acid is reacted with 3-(2-fluoro-pyridin-3-yl)-8-methyl-5,6,7,8-tetrahydro-[1,2,4]triazolo[4,3-a]pyrazine to provide the title compound in good to moderate yield.
LC/MS: m/z = 458 (MH⁺)

The stereo-isomers of this compound are separated. The S-configurated compound has a different activity than the R-configurated compound.

### Example 597

### (6-Bromo-pyrazolo[1,5-a]pyridin-2-yl)-[3-(2-fluoro-pyridin-3-yl)-5,6-dihydro-8H-[1,2,4]triazolo[4,3-a]pyrazin-7-yl]-methanone

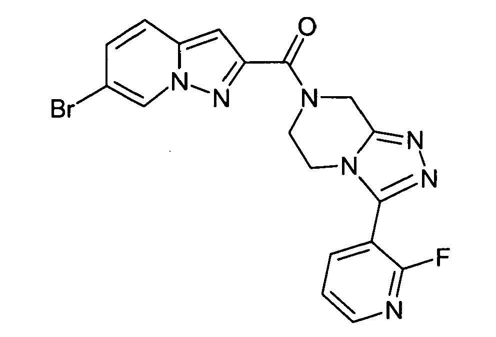

In close analogy to the procedure described in Example 1, 6-bromo-pyrazolo[1,5-a]pyridine-2-carboxylic acid is reacted with 3-(2-fluoro-pyridin-3-yl)-5,6,7,8-tetrahydro-[1,2,4]triazolo[4,3-a]pyrazine to provide the title compound in good to moderate yield.
LC/MS: m/z = 443 (MH⁺)

### Example 598

### (6-Chloro-pyrazolo[1,5-a]pyridin-2-yl)-(1-methyl-3,4-dihydro-1H-cinnolin-2-yl)-methanone

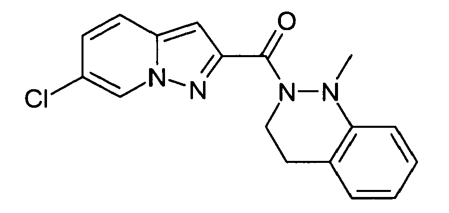

In close analogy to the procedure described in Example 1, 6-chloro-pyrazolo[1,5-a]pyridine-2-carboxylic acid is reacted with 1-methyl-1,2,3,4-tetrahydro-cinnoline to provide the title compound in good to moderate yield.
LC/MS: m/z = 327 (MH⁺)

### Example 599

### (6-Bromo-pyrazolo[1,5-a]pyridin-2-yl)-(1-methyl-3,4-dihydro-1H-cinnolin-2-yl)-methanone

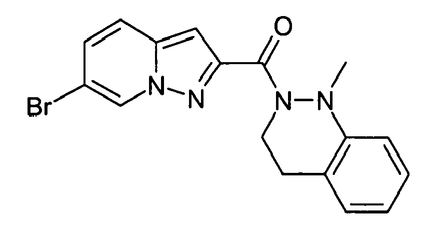

In close analogy to the procedure described in Example 1, 6-bromo-pyrazolo[1,5-a]pyridine-2-carboxylic acid is reacted with 1-methyl-1,2,3,4-tetrahydro-cinnoline to provide the title compound in good to moderate yield.
LC/MS: m/z = 372 (MH⁺)

### Example 600

### (6-Chloro-pyrazolo[1,5-a]pyridin-2-yl)-(1-methyl-3,4-dihydro-1H-thieno[3,2-c]pyridazin-2-yl)-methanone

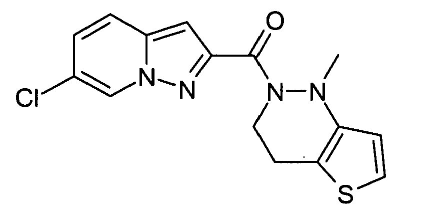

In close analogy to the procedure described in Example 1, 6-chloro-pyrazolo[1,5-a]pyridine-2-carboxylic acid is reacted with 1-methyl-1,2,3,4-tetrahydro-thieno[3,2-c]pyridazine to provide the title compound in good to moderate yield.
LC/MS: m/z = 333 (MH⁺)

### Example 601

### (6-Bromo-pyrazolo[1,5-a]pyridin-2-yl)-(1-methyl-3,4-dihydro-1H-thieno[3,2-c]pyridazin-2-yl)-methanone

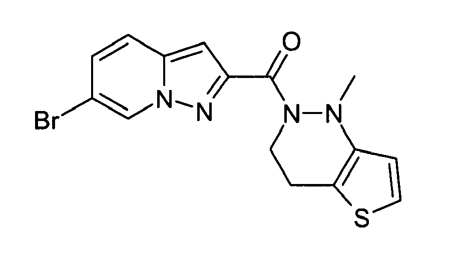

In close analogy to the procedure described in Example 1, 6-bromo-pyrazolo[1,5-a]pyridine-2-carboxylic acid is reacted with 1-methyl-1,2,3,4-tetrahydro-thieno[3,2-c]pyridazine to provide the title compound in good to moderate yield.
LC/MS: m/z = 378 (MH⁺)

### Example 602

### (6-Chloro-pyrazolo[1,5-a]pyridin-2-yl)-(1,6-dimethyl-3,4-dihydro-1H-thieno[3,2-c]pyridazin-2-yl)-methanone

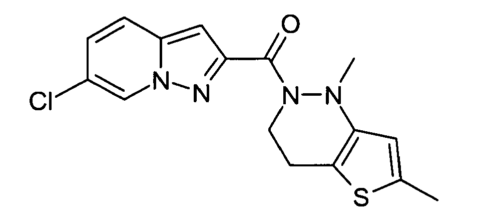

In close analogy to the procedure described in Example 1, 6-chloro-pyrazolo[1,5-a]pyridine-2-carboxylic acid is reacted with 1,6-dimethyl-1,2,3,4-tetrahydro-thieno[3,2-c]pyridazine to provide the title compound in good to moderate yield.
LC/MS: m/z = 347 (MH⁺)

### Example 603

### (6-Bromo-pyrazolo[1,5-a]pyridin-2-yl)-(1,6-dimethyl-3,4-dihydro-1H-thieno[3,2-c]pyridazin-2-yl)-methanone

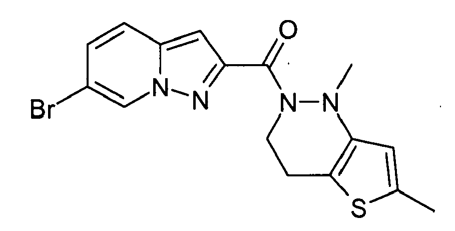

In close analogy to the procedure described in Example 1, 6-bromo-pyrazolo[1,5-a]pyridine-2-carboxylic acid is reacted with 1,6-dimethyl-1,2,3,4-tetrahydro-thieno[3,2-c]pyridazine to provide the title compound in good to moderate yield.
LC/MS: m/z = 392 (MH⁺)

### Example 604

### (6-Chloro-pyrazolo[1,5-a]pyridin-2-yl)-(1,7-dimethyl-3,4-dihydro-1H-thieno[3,2-c]pyridazin-2-yl)-methanone

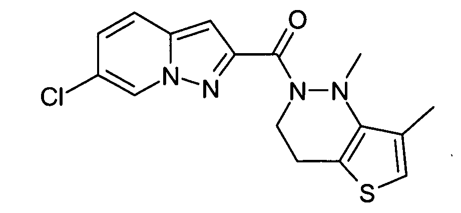

In close analogy to the procedure described in Example 1, 6-chloro-pyrazolo[1,5-a]pyridine-2-carboxylic acid is reacted with 1,7-dimethyl-1,2,3,4-tetrahydro-thieno[3,2-c]pyridazine to provide the title compound in good to moderate yield.
LC/MS: m/z = 347 (MH⁺)

### Example 605

### (6-Bromo-pyrazolo[1,5-a]pyridin-2-yl)-(1,7-dimethyl-3,4-dihydro-1H-thieno[3,2-c]pyridazin-2-yl)-methanone

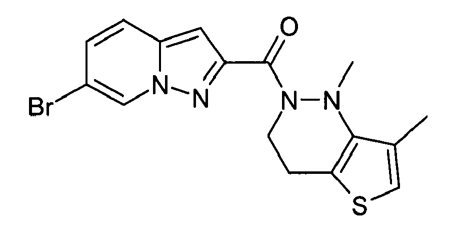

In close analogy to the procedure described in Example 1, 6-bromo-pyrazolo[1,5-a]pyridine-2-carboxylic acid is reacted with 1,7-dimethyl-1,2,3,4-tetrahydro-thieno[3,2-c]pyridazine to provide the title compound in good to moderate yield.
LC/MS: m/z = 392 (MH⁺)

### Example 606

### (6-Chloro-pyrazolo[1,5-a]pyridin-2-yl)-(1,6,7-trimethyl-3,4-dihydro-1H-thieno[3,2-c]pyridazin-2-yl)-methanone

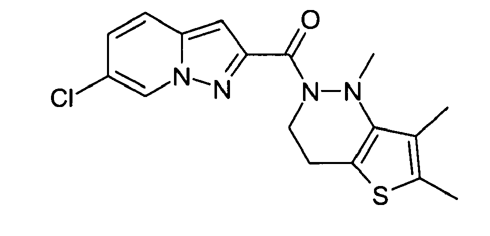

In close analogy to the procedure described in Example 1, 6-chloro-pyrazolo[1,5-a]pyridine-2-carboxylic acid is reacted with 1,6,7-trimethyl-1,2,3,4-tetrahydro-thieno[3,2-c]pyridazine to provide the title compound in good to moderate yield.
LC/MS: m/z = 361 (MH⁺)

### Example 607

### (6-Bromo-pyrazolo[1,5-a]pyridin-2-yl)-(1,6,7-trimethyl-3,4-dihydro-1H-thieno[3,2-c]pyridazin-2-yl)-methanone

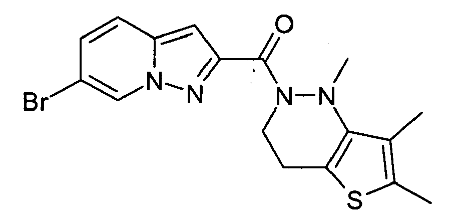

In close analogy to the procedure described in Example 1, 6-bromo-pyrazolo[1,5-a]pyridine-2-carboxylic acid is reacted with 1,6,7-trimethyl-1,2,3,4-tetrahydro-thieno[3,2-c]pyridazine to provide the title compound in good to moderate yield.
LC/MS: m/z = 406 (MH⁺)

### Example 608

### (6-Bromo-1-methyl-3,4-dihydro-1H-thieno[3,2-c]pyridazin-2-yl)-(6-chloro-pyrazolo[1,5-a]pyridin-2-yl)-methanone

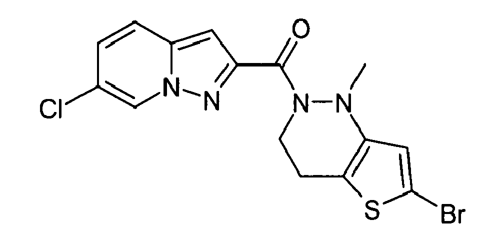

In close analogy to the procedure described in Example 1, 6-chloro-pyrazolo[1,5-a]pyridine-2-carboxylic acid is reacted with 6-bromo-1-methyl-1,2,3,4-tetrahydro-thieno[3,2-c]pyridazine to provide the title compound in good to moderate yield.
LC/MS: m/z = 412 (MH⁺)

### Example 609

### (6-Bromo-1-methyl-3,4-dihydro-H-thieno[3,2-c]pyridazin-2-yl)-(6-bromo-pyrazolo[1,5-a]pyridin-2-yl)-methanone

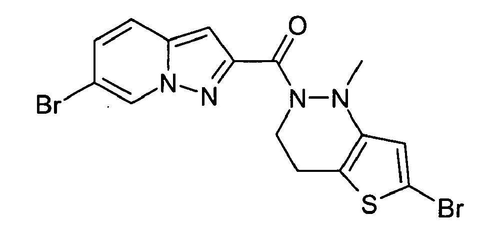

In close analogy to the procedure described in Example 1, 6-bromo-pyrazolo[1,5-a]pyridine-2-carboxylic acid is reacted with 6-bromo-1-methyl-1,2,3,4-tetrahydro-thieno[3,2-c]pyridazine to provide the title compound in good to moderate yield.
LC/MS: m/z = 457 (MH⁺)

### Example 610

### (6-Chloro-1-methyl-3,4-dihydro-1H-thieno[3,2-c]pyridazin-2-yl)-(6-chloro-pyrazolo[1,5-a]pyridin-2-yl)-methanone

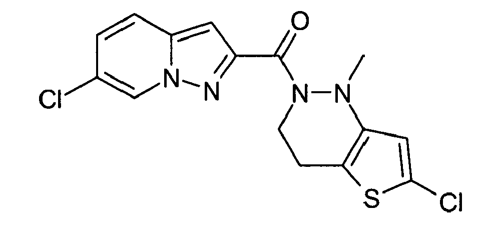

In close analogy to the procedure described in Example 1, 6-chloro-pyrazolo[1,5-a]pyridine-2-carboxylic acid is reacted with 6-chloro-1-methyl-1,2,3,4-tetrahydro-thieno[3,2-c]pyridazine to provide the title compound in good to moderate yield.
LC/MS: m/z = 368 (MH⁺)

### Example 611

### (6-Bromo-1-methyl-3,4-dihydro-1H-thieno[3,2-c]pyridazin-2-yl)-(6-chloro-pyrazolo[1,5-a]pyridin-2-yl)-methanone

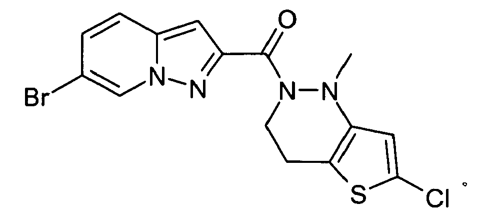

In close analogy to the procedure described in Example 1, 6-bromo-pyrazolo[1,5-a]pyridine-2-carboxylic acid is reacted with 6-chloro-1-methyl-1,2,3,4-tetrahydro-thieno[3,2-c]pyridazine to provide the title compound in good to moderate yield.
LC/MS: m/z = 312 (MH⁺)

### Example 612

### (6-Chloro-pyrazolo[1,5-a]pyridin-2-yl)-(1-methyl-3,4-dihydro-1H-furo[3,2-c]pyridazin-2-yl)-methanone

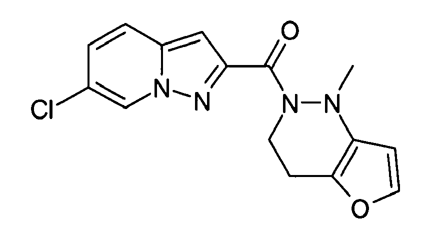

In close analogy to the procedure described in Example 1, 6-chloro-pyrazolo[1,5-a]pyridine-2-carboxylic acid is reacted with 1-methyl-1,2,3,4-tetrahydro-furo[3,2-c]pyridazine to provide the title compound in good to moderate yield.
LC/MS: m/z = 317 (MH⁺)

### Example 613

### (6-Bromo-pyrazolo[1,5-a]pyridin-2-yl)-(-methyl-3,4-dihydro-1H-furo[3,2-c]pyridazin-2-yl)-methanone

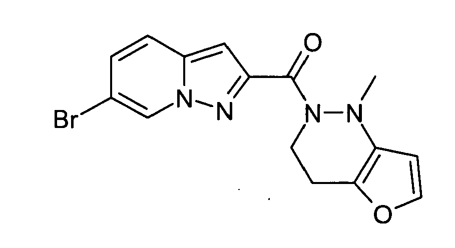

In close analogy to the procedure described in Example 1, 6-bromo-pyrazolo[1,5-a]pyridine-2-carboxylic acid is reacted with 1-methyl-1,2,3,4-tetrahydro-furo[3,2-c]pyridazine to provide the title compound in good to moderate yield.
LC/MS: m/z = 362 (MH⁺)

### Example 614

### (6-Chloro-pyrazolo[1,5-a]pyridin-2-yl)-(1,6-dimethyl-3,4-dihydro-1H-furo[3,2-c]pyridazin-2-yl)-methanone

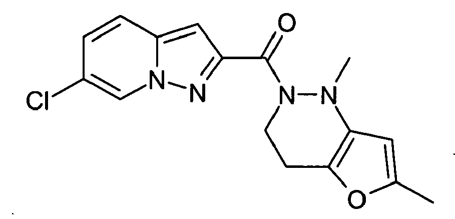

In close analogy to the procedure described in Example 1, 6-chloro-pyrazolo[1,5-a]pyridine-2-carboxylic acid is reacted with 1,6-dimethyl-1,2,3,4-tetrahydro-furo[3,2-c]pyridazine to provide the title compound in good to moderate yield.
LC/MS: m/z = 331 (MH⁺)

### Example 615

### (6-Bromo-pyrazolo[1,5-a]pyridin-2-yl)-(1,6-dimethyl-3,4-dihydro-1H-furo[3,2-c]pyridazin-2-yl)-methanone

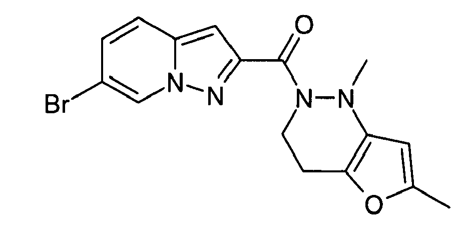

In close analogy to the procedure described in Example 1, 6-bromo-pyrazolo[1,5-a]pyridine-2-carboxylic acid is reacted with 1,6-dimethyl-1,2,3,4-tetrahydro-furo[3,2-c]pyridazine to provide the title compound in good to moderate yield.
LC/MS: m/z = 376 (MH⁺)

### Example 616

### (6-Chloro-pyrazolo[1,5-a]pyridin-2-yl)-(1,7-dimethyl-3,4-dihydro-1H-furo[3,2-c]pyridazin-2-yl)-methanone

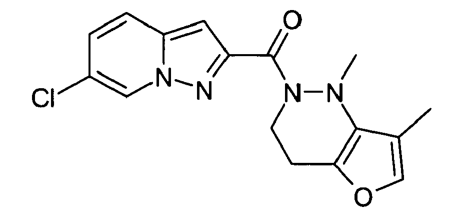

In close analogy to the procedure described in Example 1, 6-chloro-pyrazolo[1,5-a]pyridine-2-carboxylic acid is reacted with 1,7-dimethyl-1,2,3,4-tetrahydro-furo[3,2-c]pyridazine to provide the title compound in good to moderate yield.
LC/MS: m/z = 331 (MH⁺)

### Example 617

### (6-Bromo-pyrazolo[1,5-a]pyridin-2-yl)-(1,7-dimethyl-3,4-dihydro-1H-furo[3,2-c]pyridazin-2-yl)-methanone

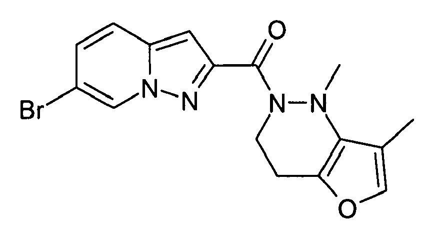

In close analogy to the procedure described in Example 1, 6-bromo-pyrazolo[1,5-a]pyridine-2-carboxylic acid is reacted with 1,7-dimethyl-1,2,3,4-tetrahydro-furo[3,2-c]pyridazine to provide the title compound in good to moderate yield.
LC/MS: m/z = 376 (MH⁺)

### Example 618

### (6-Chloro-pyrazolo[1,5-a]pyridin-2-yl)-(1,6,7-trimethyl-3,4-dihydro-1H-furo[3,2-c]pyridazin-2-yl)-methanone

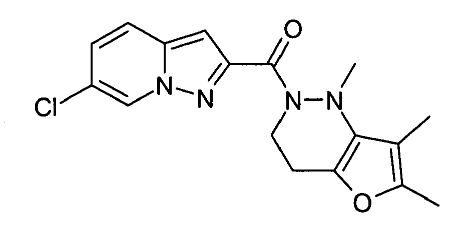

In close analogy to the procedure described in Example 1, 6-chloro-pyrazolo[1,5-a]pyridine-2-carboxylic acid is reacted with 1,6,7-trimethyl-1,2,3,4-tetrahydro-furo[3,2-c]pyridazine to provide the title compound in good to moderate yield.
LC/MS: m/z = 345 (MH⁺)

### Example 619

### (6-Bromo-pyrazolo[1,5-a]pyridin-2-yl)-(1,6,7-trimethyl-3,4-dihydro-1H-furo[3,2-c]pyridazin-2-yl)-methanone

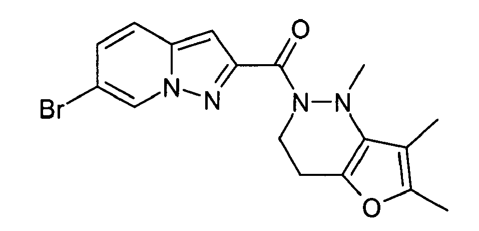

In close analogy to the procedure described in Example 1, 6-bromo-pyrazolo[1,5-a]pyridine-2-carboxylic acid is reacted with 1,6,7-trimethyl-1,2,3,4-tetrahydro-furo[3,2-c]pyridazine to provide the title compound in good to moderate yield.
LC/MS: m/z = 390 (MH⁺)

### Example 620

### (6-Bromo-1-methyl-3,4-dihydro-1H-furo[3,2-c]pyridazin-2-yl)-(6-chloro-pyrazolo[1,5-a]pyridin-2-yl)-methanone

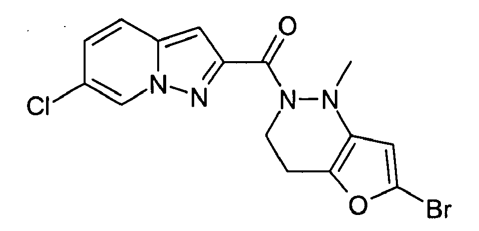

In close analogy to the procedure described in Example 1, 6-chloro-pyrazolo[1,5-a]pyridine-2-carboxylic acid is reacted with 6-bromo-1-methyl-1,2,3,4-tetrahydro-furo[3,2-c]pyridazine to provide the title compound in good to moderate yield.
LC/MS: m/z = 396 (MH⁺)

### Example 621

### (6-Bromo-1-methyl-3,4-dihydro-1H-furo[3,2-c]pyridazin-2-yl)-(6-bromo-pyrazolo[1,5-a]pyridin-2-yl)-methanone

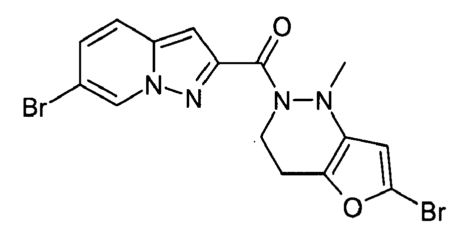

In close analogy to the procedure described in Example 1, 6-bromo-pyrazolo[1,5-a]pyridine-2-carboxylic acid is reacted with 6-bromo-1-methyl-1,2,3,4-tetrahydro-furo[3,2-c]pyridazine to provide the title compound in good to moderate yield.
LC/MS: m/z = 441 (MH⁺)

### Example 622

### (6-Chloro-1-methyl-3,4-dihydro-1H-furo[3,2-c]pyridazin-2-yl)-(6-chloro-pyrazolo[1,5-a]pyridin-2-yl)-methanone

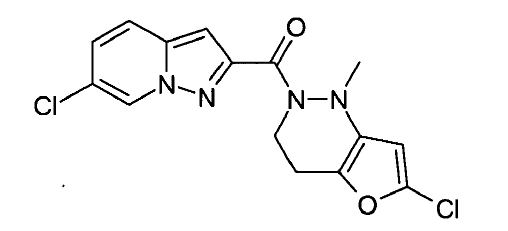

In close analogy to the procedure described in Example 1, 6-chloro-pyrazolo[1,5-a]pyridine-2-carboxylic acid is reacted with 6-chloro-1-methyl-1,2,3,4-tetrahydro-furo[3,2-c]pyridazine to provide the title compound in good to moderate yield.
LC/MS: m/z = 352 (MH⁺)

### Example 623

### (6-Bromo-1-methyl-3,4-dihydro-1H-furo[3,2-c]pyridazin-2-yl)-(6-chloro-pyrazolo[1,5-a]pyridin-2-yl)-methanone

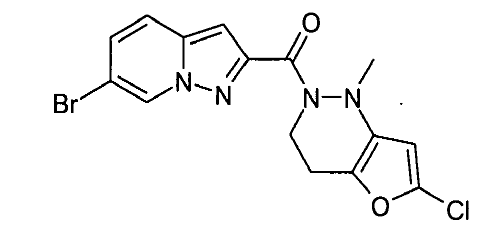

In close analogy to the procedure described in Example 1, 6-bromo-pyrazolo[1,5-a]pyridine-2-carboxylic acid is reacted with 6-chloro-1-methyl-1,2,3,4-tetrahydro-furo[3,2-c]pyridazine to provide the title compound in good to moderate yield
LC/MS: m/z = 396 (MH⁺)

### Example 624

### (6-Chloro-pyrazolo[1,5-a]pyridin-2-yl)-(1-methyl-1,3,4,5-tetrahydro-pyrrolo[3,2-c]pyridazin-2-yl)-methanone

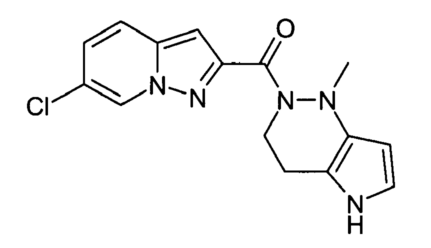

In close analogy to the procedure described in Example 1, 6-chloro-pyrazolo[1,5-a]pyridine-2-carboxylic acid is reacted with 1-methyl-2,3,4,5-tetrahydro-1H-pyrrolo[3,2-c]pyridazine to provide the title compound in good to moderate yield.
LC/MS: m/z = 316 (MH⁺)

### Example 625

### (6-Bromo-pyrazolo[1,5-a]pyridin-2-yl)-(1-methyl-1,3,4,5-tetrahydro-pyrrolo[3,2-c]pyridazin-2-yl)-methanone

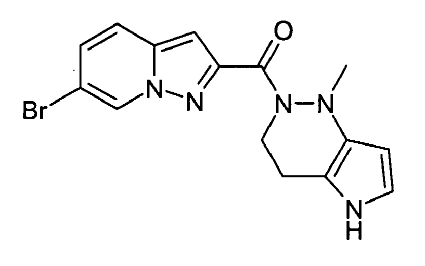

In close analogy to the procedure described in Example 1, 6-bromo-pyrazolo[1,5-a]pyridine-2-carboxylic acid is reacted with 1-methyl-2,3,4,5-tetrahydro-1H-pyrrolo[3,2-c]pyridazine to provide the title compound in good to moderate yield.
LC/MS: m/z = 361 (MH⁺)

### Example 626

### (6-Chloro-pyrazolo[1,5-a]pyridin-2-yl)-(1,6-dimethyl-1,3,4,5-tetrahydro-pyrrolo[3,2-c]pyridazin-2-yl)-methanone

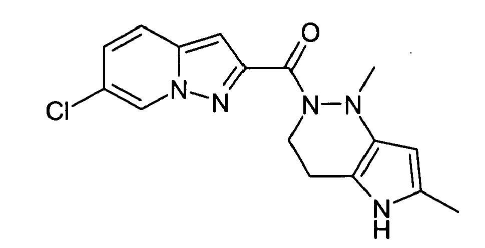

In close analogy to the procedure described in Example 1, 6-chloro-pyrazolo[1,5-a]pyridine-2-carboxylic acid is reacted with 1,6-dimethyl-2,3,4,5-tetrahydro-1H-pyrrolo[3,2-c]pyridazine to provide the title compound in good to moderate yield.
LC/MS: m/z = 330 (MH⁺)

### Example 627

### (6-Bromo-pyrazolo[1,5-a]pyridin-2-yl)-(1,6-dimethyl-1,3,4,5-tetrahydro-pyrrolo[3,2-c]pyridazin-2-yl)-methanone

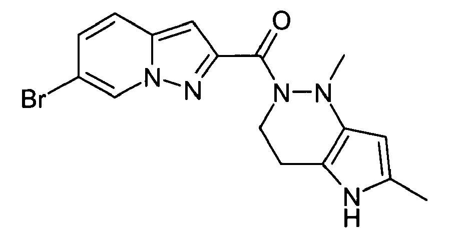

In close analogy to the procedure described in Example 1, 6-bromo-pyrazolo[1,5-a]pyridine-2-carboxylic acid is reacted with 1,6-dimethyl-2,3,4,5-tetrahydro-1H-pyrrolo[3,2-c]pyridazine to provide the title compound in good to moderate yield.
LC/MS: m/z = 375 (MH⁺)

### Example 628

### (6-Chloro-pyrazolo[1,5-a]pyridin-2-yl)-(1,7-dimethyl-1,3,4,5-tetrahydro-pyrrolo[3,2-c]pyridazin-2-yl)-methanone

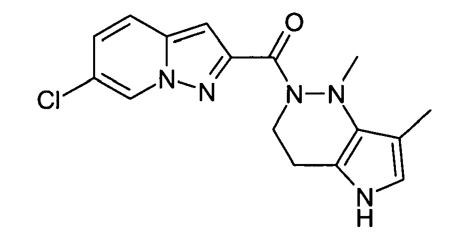

In close analogy to the procedure described in Example 1, 6-chloro-pyrazolo[1,5-a]pyridine-2-carboxylic acid is reacted with 1,7-dimethyl-2,3,4,5-tetrahydro-1H-pyrrolo[3,2-c]pyridazine to provide the title compound in good to moderate yield.
LC/MS: m/z = 330 (MH⁺)

### Example 629

### (6-Bromo-pyrazolo[1,5-a]pyridin-2-yl)-(1,7-dimethyl-1,3,4,5-tetrahydro-pyrrolo[3,2-c]pyridazin-2-yl)-methanone

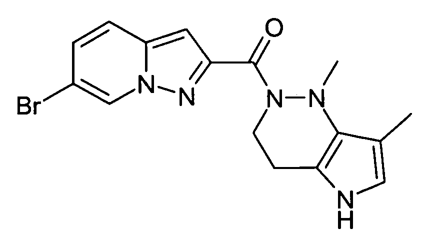

In close analogy to the procedure described in Example 1, 6-bromo-pyrazolo[1,5-a]pyridine-2-carboxylic acid is reacted with 1,7-dimethyl-2,3,4,5-tetrahydro-1H-pyrrolo[3,2-c]pyridazine to provide the title compound in good to moderate yield.
LC/MS: m/z = 375 (MH⁺)

### Example 630

### (6-Chloro-pyrazolo[1,5-a]pyridin-2-yl)-(1,6,7-trimethyl-1,3,4,5-tetrahydro-pyrrolo[3,2-c]pyridazin-2-yl)-methanone

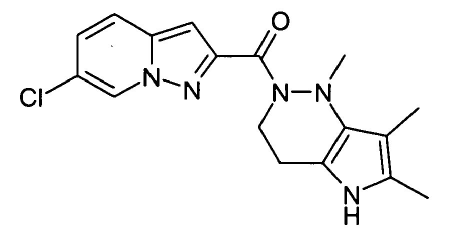

In close analogy to the procedure described in Example 1, 6-chloro-pyrazolo[1,5-a]pyridine-2-carboxylic acid is reacted with 1,6,7-trimethyl-2,3,4,5-tetrahydro-1H-pyrrolo[3,2-c]pyridazine to provide the title compound in good to moderate yield.
LC/MS: m/z = 344 (MH⁺)

### Example 631

### (6-Bromo-pyrazolo[1,5-a]pyridin-2-yl)-(1,6,7-trimethyl-1,3,4,5-tetrahydro-pyrrolo[3,2-c]pyridazin-2-yl)-methanone

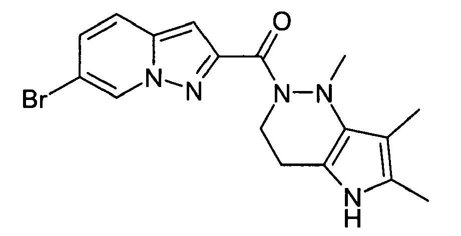

In close analogy to the procedure described in Example 1, 6-bromo-pyrazolo[1,5-a]pyridine-2-carboxylic acid is reacted with 1,6,7-trimethyl-2,3,4,5-tetrahydro-1H-pyrrolo[3,2-c]pyridazine to provide the title compound in good to moderate yield.
LC/MS: m/z = 389 (MH⁺)

### Example 632

### (6-Bromo-1-methyl-1,3,4,5-tetrahydro-pyrrolo[3,2-c]pyridazin-2-yl)-(6-chloro-pyrazolo[1,5-a]pyridin-2-yl)-methanone

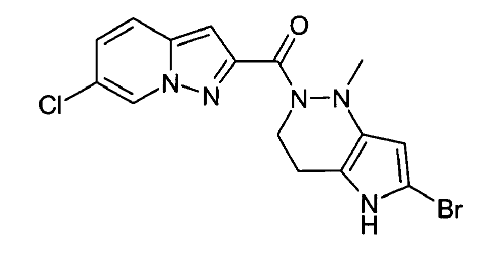

In close analogy to the procedure described in Example 1, 6-chloro-pyrazolo[1,5-a]pyridine-2-carboxylic acid is reacted with 6-bromo-1-methyl-2,3,4,5-tetrahydro-1H-pyrrolo[3,2-c]pyridazine to provide the title compound in good to moderate yield in good to moderate yield.
LC/MS: m/z = 395 (MH⁺)

### Example 633

### (6-Bromo-1-methyl-1,3,4,5-tetrahydro-pyrrolo[3,2-c]pyridazin-2-yl)-(6-bromo-pyrazolo[1,5-a]pyridin-2-yl)-methanone

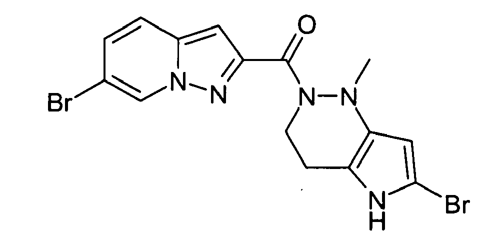

In close analogy to the procedure described in Example 1, 6-bromo-pyrazolo[1,5-a]pyridine-2-carboxylic acid is reacted with 6-bromo-1-methyl-2,3,4,5-tetrahydro-1H-pyrrolo[3,2-c]pyridazine to provide the title compound in good to moderate yield.
LC/MS: m/z = 440 (MH⁺)

### Example 634

### (6-Chloro-1-methyl-1,3,4,5-tetrahydro-pyrrolo[3,2-c]pyridazin-2-yl)-(6-chloro-pyrazolo[1,5-a]pyridin-2-yl)-methanone

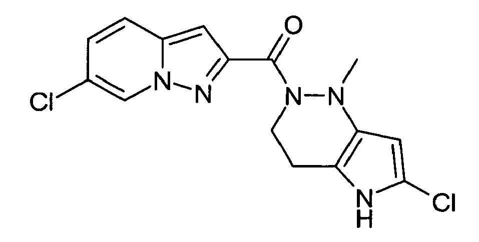

In close analogy to the procedure described in Example 1, 6-chloro-pyrazolo[1,5-a]pyridine-2-carboxylic acid is reacted with 6-chloro-1-methyl-2,3,4,5-tetrahydro-1H-pyrrolo[3,2-c]pyridazine to provide the title compound in good to moderate yield.
LC/MS: m/z = 351 (MH⁺)

### Example 635

### (6-Bromo-1-methyl-1,3,4,5-tetrahydro-pyrrolo[3,2-c]pyridazin-2-yl)-(6-chloro-pyrazolo[1,5-a]pyridin-2-yl)-methanone

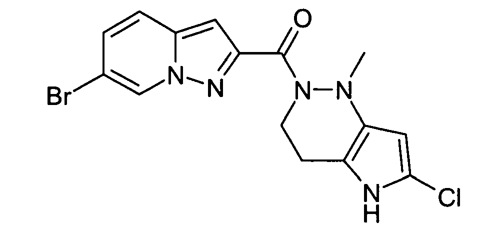

In close analogy to the procedure described in Example 1, 6-bromo-pyrazolo[1,5-a]pyridine-2-carboxylic acid is reacted with 6-chloro-1-methyl-2,3,4,5-tetrahydro-1H-pyrrolo[3,2-c]pyridazine to provide the title compound in good to moderate yield.
LC/MS: m/z = 395 (MH⁺)

### Example 636

### (8-Chloro-1-methyl-3,4-dihydro-1H-cinnolin-2-yl)-(6-chloro-pyrazolo[1,5-a]pyridin-2-yl)-methanone

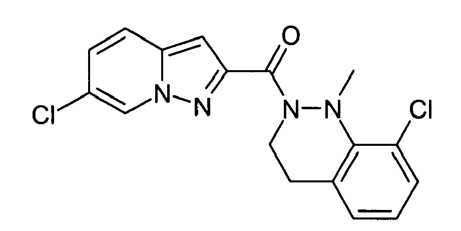

In close analogy to the procedure described in Example 1, 6-chloro-pyrazolo[1,5-a]pyridine-2-carboxylic acid is reacted with 8-chloro-1-methyl-1,2,3,4-tetrahydro-cinnoline to provide the title compound in good to moderate yield.
LC/MS: m/z = 362 (MH⁺)

### Example 637

### (8-Bromo-1-methyl-3,4-dihydro-1H-cinnolin-2-yl)-(6-chloro-pyrazolo[1,5-a]pyridin-2-yl)-methanone

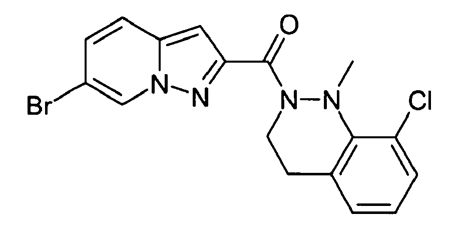

In close analogy to the procedure described in Example 1, 6-bromo-pyrazolo[1,5-a]pyridine-2-carboxylic acid is reacted with 8-chloro-1-methyl-1,2,3,4-tetrahydro-cinnoline to provide the title compound in good to moderate yield.
LC/MS: m/z = 406 (MH⁺)

### Example 638

### (7-Chloro-1-methyl-3,4-dihydro-1H-cinnolin-2-yl)-(6-chloro-pyrazolo[1,5-a]pyridin-2-yl)-methanone

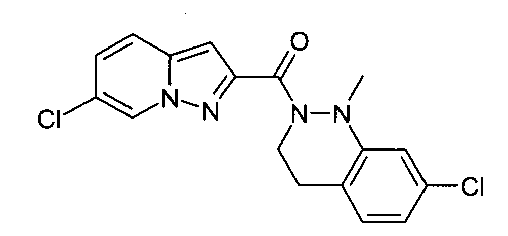

In close analogy to the procedure described in Example 1, 6-chloro-pyrazolo[1,5-a]pyridine-2-carboxylic acid is reacted with 7-chloro-1-methyl-1,2,3,4-tetrahydro-cinnoline to provide the title compound in good to moderate yield.
LC/MS: m/z = 362 (MH⁺)

### Example 639

### (7-Bromo-1-methyl-3,4-dihydro-1H-cinnolin-2-yl)-(6-chloro-pyrazolo[1,5-a]pyridin-2-yl)-methanone

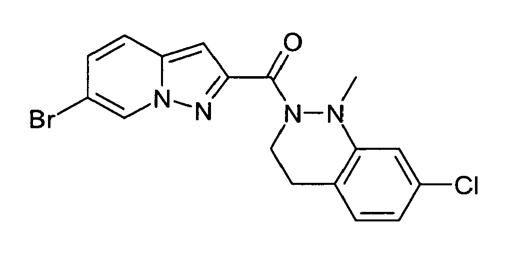

In close analogy to the procedure described in Example 1, 6-bromo-pyrazolo[1,5-a]pyridine-2-carboxylic acid is reacted with 7-chloro-1-methyl-1,2,3,4-tetrahydro-cinnoline to provide the title compound in good to moderate yield.
LC/MS: m/z = 406 (MH⁺)

### Example 640

### (6-Chloro-1-methyl-3,4-dihydro-1H-cinnolin-2-yl)-(6-chloro-pyrazolo[1,5-a]pyridin-2-yl)-methanone

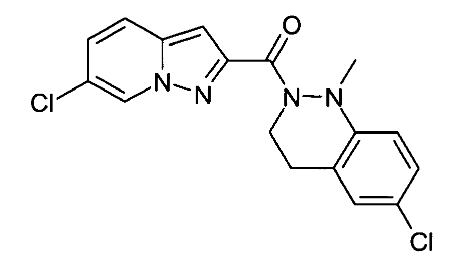

In close analogy to the procedure described in Example 1, 6-chloro-pyrazolo[1,5-a]pyridine-2-carboxylic acid is reacted with 6-chloro-1-methyl-1,2,3,4-tetrahydro-cinnoline to provide the title compound in good to moderate yield.
LC/MS: m/z = 362 (MH⁺)

### Example 641

### (6-Bromo-1-methyl-3,4-dihydro-1H-cinnolin-2-yl)-(6-chloro-pyrazolo[1,5-a]pyridin-2-yl)-methanone

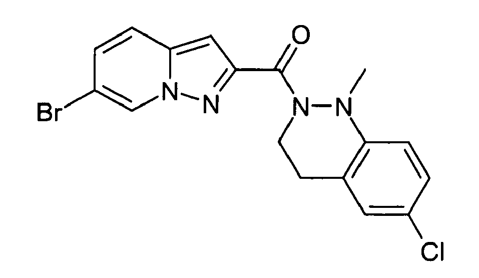

In close analogy to the procedure described in Example 1, 6-bromo-pyrazolo[1,5-a]pyridine-2-carboxylic acid is reacted with 6-chloro-1-methyl-1,2,3,4-tetrahydro-cinnoline to provide the title compound in good to moderate yield.
LC/MS: m/z = 406 (MH⁺)

### Example 642

### (5-Chloro-1-methyl-3,4-dihydro-1H-cinnolin-2-yl)-(6-chloro-pyrazolo[1,5-a]pyridin-2-yl)-methanone

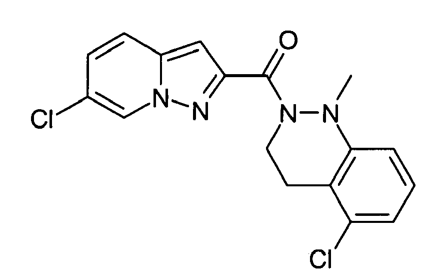

In close analogy to the procedure described in Example 1, 6-chloro-pyrazolo[1,5-a]pyridine-2-carboxylic acid is reacted with c-Chloro-1-methyl-1,2,3,4-tetrahydro-cinnoline to provide the title compound in good to moderate yield.
LC/MS: m/z = 362 (MH⁺)

### Example 643

### (5-Bromo-1-methyl-3,4-dihydro-1H-cinnolin-2-yl)-(6-chloro-pyrazolo[1,5-a]pyridin-2-yl)-methanone

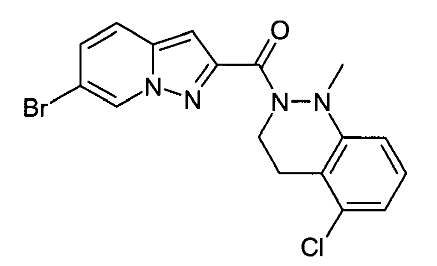

In close analogy to the procedure described in Example 1, 6-bromo-pyrazolo[1,5-a]pyridine-2-carboxylic acid is reacted with c-Chloro-1-methyl-1,2,3,4-tetrahydro-cinnoline to provide the title compound in good to moderate yield.
LC/MS: m/z = 406 (MH⁺)

### Example 644

### (8-Bromo-1-methyl-3,4-dihydro-1H-cinnolin-2-yl)-(6-chloro-pyrazolo[1,5-a]pyridin-2-yl)-methanone

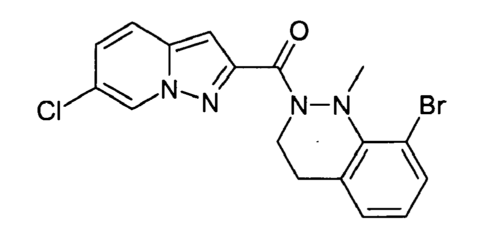

In close analogy to the procedure described in Example 1, 6-chloro-pyrazolo[1,5-a]pyridine-2-carboxylic acid is reacted with 8-bromo-1-methyl-1,2,3,4-tetrahydro-cinnoline to provide the title compound in good to moderate yield.
LC/MS: m/z = 406 (MH⁺)

### Example 645

### (8-Bromo-1-methyl-3,4-dihydro-1H-cinnolin-2-yl)-(6-bromo-pyrazolo[1,5-a]pyridin-2-yl)-methanone

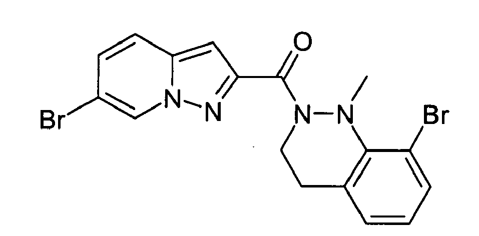

In close analogy to the procedure described in Example 1, 6-bromo-pyrazolo[1,5-a]pyridine-2-carboxylic acid is reacted with 8-bromo-1-methyl-1,2,3,4-tetrahydro-cinnoline to provide the title compound in good to moderate yield.
LC/MS: m/z = 451 (MH⁺)

### Example 646

### (7-Bromo-1-methyl-3,4-dihydro-1H-cinnolin-2-yl)-(6-chloro-pyrazolo[1,5-a]pyridin-2-yl)-methanone

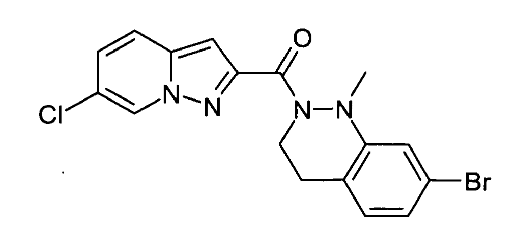

In close analogy to the procedure described in Example 1, 6-chloro-pyrazolo[1,5-a]pyridine-2-carboxylic acid is reacted with 7-bromo-1-methyl-1,2,3,4-tetrahydro-cinnoline to provide the title compound in good to moderate yield.
LC/MS: m/z = 406 (MH⁺)

### Example 647

### (7-Bromo-1-methyl-3,4-dihydro-1H-cinnolin-2-yl)-(6-bromo-pyrazolo[1,5-a]pyridin-2-yl)-methanone

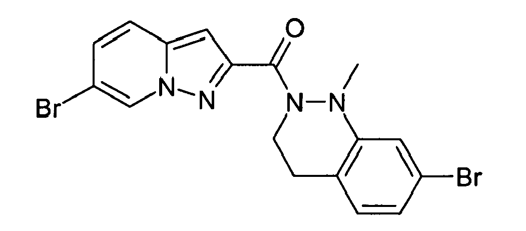

In close analogy to the procedure described in Example 1, 6-bromo-pyrazolo[1,5-a]pyridine-2-carboxylic acid is reacted with 7-bromo-1-methyl-1,2,3,4-tetrahydro-cinnoline to provide the title compound in good to moderate yield.
LC/MS: m/z = 451 (MH⁺)

### Example 648

### (6-Bromo-1-methyl-3,4-dihydro-1H-cinnolin-2-yl)-(6-chloro-pyrazolo[1,5-a]pyridin-2-yl)-methanone

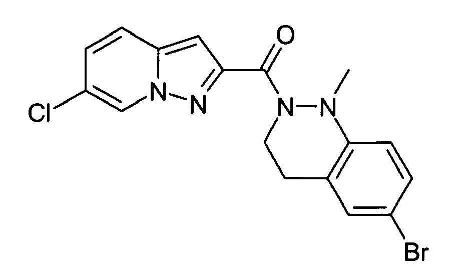

In close analogy to the procedure described in Example 1, 6-chloro-pyrazolo[1,5-a]pyridine-2-carboxylic acid is reacted with 6-bromo-1-methyl-1,2,3,4-tetrahydro-cinnoline to provide the title compound in good to moderate yield.
LC/MS: m/z = 406 (MH⁺)

### Example 649

### (6-Bromo-1-methyl-3,4-dihydro-1H-cinnolin-2-yl)-(6-bromo-pyrazolo[1,5-a]pyridin-2-yl)-methanone

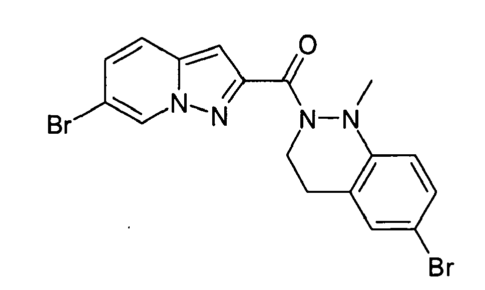

In close analogy to the procedure described in Example 1, 6-bromo-pyrazolo[1,5-a]pyridine-2-carboxylic acid is reacted with 6-bromo-1-methyl-1,2,3,4-tetrahydro-cinnoline to provide the title compound in good to moderate yield.
LC/MS: m/z = 451 (MH⁺)

### Example 650

### (5-Bromo-1-methyl-3,4-dihydro-1H-cinnolin-2-yl)-(6-chloro-pyrazolo[1,5-a]pyridin-2-yl)-methanone

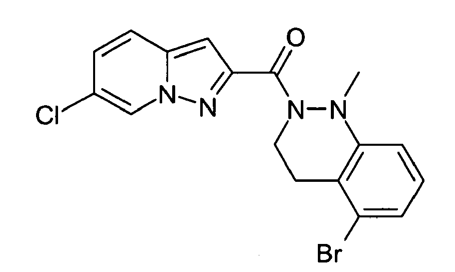

In close analogy to the procedure described in Example 1, 6-chloro-pyrazolo[1,5-a]pyridine-2-carboxylic acid is reacted with 5-bromo-1-methyl-1,2,3,4-tetrahydro-cinnoline to provide the title compound in good to moderate yield.
LC/MS: m/z = 406 (MH⁺)

### Example 651

### (5-Bromo-1-methyl-3,4-dihydro-1H-cinnolin-2-yl)-(6-bromo-pyrazolo[ 1,5-a]pyridin-2-yl)-methanone

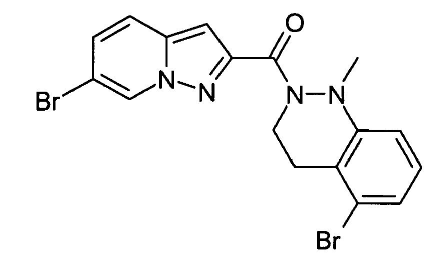

In close analogy to the procedure described in Example 1, 6-bromo-pyrazolo[1,5-a]pyridine-2-carboxylic acid is reacted with 5-bromo-1-methyl-1,2,3,4-tetrahydro-cinnoline to provide the title compound in good to moderate yield.
LC/MS: m/z = 451 (MH⁺)

### Example 652

### (8-Fluoro-1-methyl-3,4-dihydro-1H-cinnolin-2-yl)-(6-chloro-pyrazolo[1,5-a]pyridin-2-yl)-methanone

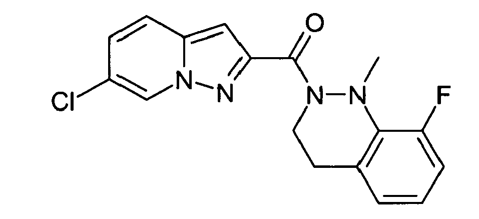

In close analogy to the procedure described in Example 1, 6-chloro-pyrazolo[1,5-a]pyridine-2-carboxylic acid is reacted with 8-fluoro-1-methyl-1,2,3,4-tetrahydro-cinnoline to provide the title compound in good to moderate yield.
LC/MS: m/z = 345 (MH⁺)

### Example 653

### (8-Fluoro-1-methyl-3,4-dihydro-1H-cinnolin-2-yl)-(6-bromo-pyrazolo[1,5-a]pyridin-2-yl)-methanone

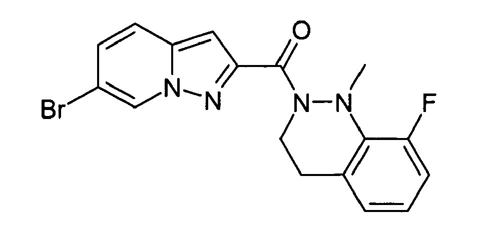

In close analogy to the procedure described in Example 1, 6-bromo-pyrazolo[1,5-a]pyridine-2-carboxylic acid is reacted with 8-fluoro-1-methyl-1,2,3,4-tetrahydro-cinnoline to provide the title compound in good to moderate yield.
LC/MS: m/z = 390 (MH⁺)

### Example 654

### (7-Fluoro-1-methyl-3,4-dihydro-1H-cinnolin-2-yl)-(6-chloro-pyrazolo[ 1,5-a]pyridin-2-yl)-methanone

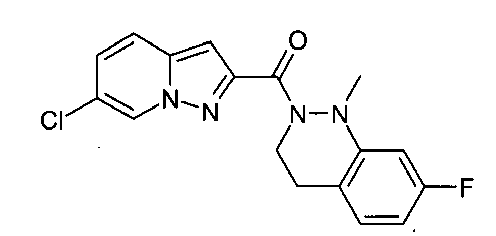

In close analogy to the procedure described in Example 1, 6-chloro-pyrazolo[1,5-a]pyridine-2-carboxylic acid is reacted with 7-fluoro-1-methyl-1,2,3,4-tetrahydro-cinnoline to provide the title compound in good to moderate yield.
LC/MS: m/z = 345 (MH⁺)

### Example 655

### (7-Fluoro-1-methyl-3,4-dihydro-1H-cinnolin-2-yl)-(6-bromo-pyrazolo[1,5-a]pyridin-2-yl)-methanone

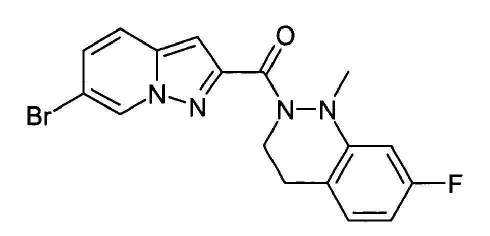

In close analogy to the procedure described in Example 1, 6-bromo-pyrazolo[1,5-a]pyridine-2-carboxylic acid is reacted with 7-fluoro-1-methyl-1,2,3,4-tetrahydro-cinnoline to provide the title compound in good to moderate yield.
LC/MS: m/z = 390 (MH⁺)

### Example 656

### (6-Fluoro-1-methyl-3,4-dihydro-1H-cinnolin-2-yl)-(6-chloro-pyrazolo[1,5-a]pyridin-2-yl)-methanone

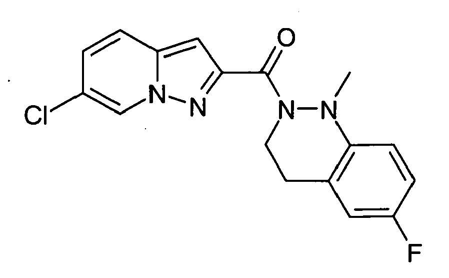

In close analogy to the procedure described in Example 1, 6-chloro-pyrazolo[1,5-a]pyridine-2-carboxylic acid is reacted with 6-fluoro-1-methyl-1,2,3,4-tetrahydro-cinnoline to provide the title compound in good to moderate yield.
LC/MS: m/z = 345 (MH⁺)

### Example 657

### (6-Fluoro-1-methyl-3,4-dihydro-1H-cinnolin-2-yl)-(6-bromo-pyrazolo[1,5-a]pyridin-2-yl)-methanone

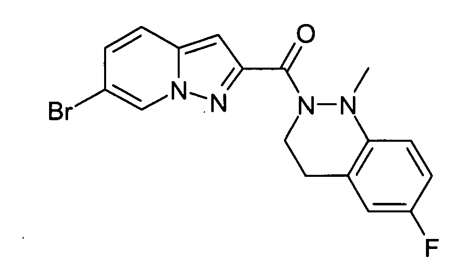

In close analogy to the procedure described in Example 1, 6-bromo-pyrazolo[1,5-a]pyridine-2-carboxylic acid is reacted with 6-fluoro-1-methyl-1,2,3,4-tetrahydro-cinnoline to provide the title compound in good to moderate yield.
LC/MS: m/z = 390 (MH⁺)

### Example 658

### (5-Fluoro-1-methyl-3,4-dihydro-1H-cinnolin-2-yl)-(6-chloro-pyrazolo[1,5-a]pyridin-2-yl)-methanone

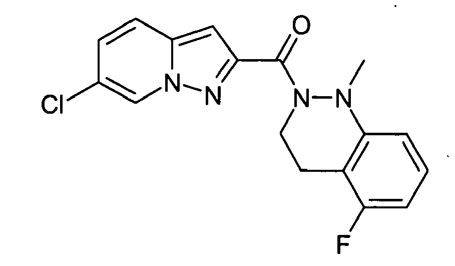

In close analogy to the procedure described in Example 1, 6-chloro-pyrazolo[1,5-a]pyridine-2-carboxylic acid is reacted with 5-fluoro-1-methyl-1,2,3,4-tetrahydro-cinnoline to provide the title compound in good to moderate yield.
LC/MS: m/z = 345 (MH⁺)

### Example 659

### (5-Fluoro-1-methyl-3,4-dihydro-1H-cinnolin-2-yl)-(6-bromo-pyrazolo[1,5-a]pyridin-2-yl)-methanone

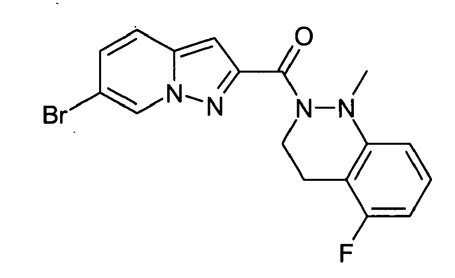

In close analogy to the procedure described in Example 1, 6-bromo-pyrazolo[1,5-a]pyridine-2-carboxylic acid is reacted with 5-fluoro-1-methyl-1,2,3,4-tetrahydro-cinnoline to provide the title compound in good to moderate yield.
LC/MS: m/z = 390 (MH⁺)

### Example 660

### (6-Chloro-pyrazolo[1,5-a]pyridin-2-yl)-(1,8-dimethyl-3,4-dihydro-1H-cinnolin-2-yl)-methanone

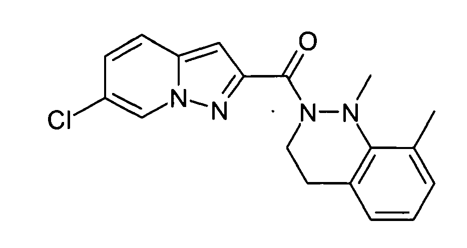

In close analogy to the procedure described in Example 1, 6-chloro-pyrazolo[1,5-a]pyridine-2-carboxylic acid is reacted with 1,8-dimethyl-1,2,3,4-tetrahydro-cinnoline to provide the title compound in good to moderate yield.
LC/MS: m/z = 341 (MH⁺)

### Example 661

### (6-Bromo-pyrazolo[1,5-a]pyridin-2-yl)-(1,8-dimethyl-3,4-dihydro-1H-cinnolin-2-yl)-methanone

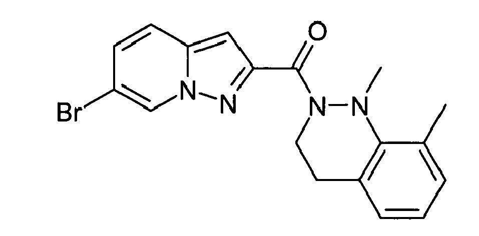

In close analogy to the procedure described in Example 1, 6-bromo-pyrazolo[1,5-a]pyridine-2-carboxylic acid is reacted with 1,8-dimethyl-1,2,3,4-tetrahydro-cinnoline to provide the title compound in good to moderate yield.
LC/MS: m/z = 386 (MH⁺)

### Example 662

### (6-Chloro-pyrazolo[1,5-a]pyridin-2-yl)-(1,7-dimethyl-3,4-dihydro-1H-cinnolin-2-yl)-methanone

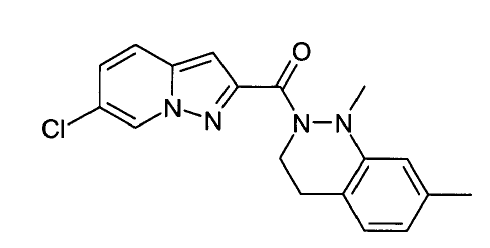

In close analogy to the procedure described in Example 1, 6-chloro-pyrazolo[1,5-a]pyridine-2-carboxylic acid is reacted with 1,7-dimethyl-1,2,3,4-tetrahydro-cinnoline to provide the title compound in good to moderate yield.
LC/MS: m/z = 341 (MH⁺)

### Example 663

### (6-Bromo-pyrazolo[1,5-a]pyridin-2-yl)-(1,7-dimethyl-3,4-dihydro-1H-cinnolin-2-yl)-methanone

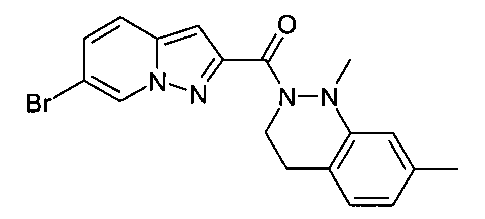

In close analogy to the procedure described in Example 1, 6-bromo-pyrazolo[1,5-a]pyridine-2-carboxylic acid is reacted with 1,7-dimethyl-1,2,3,4-tetrahydro-cinnoline to provide the title compound in good to moderate yield.
LC/MS: m/z = 386 (MH⁺)

### Example 664

### (6-Chloro-pyrazolo[1,5-a]pyridin-2-yl)-(1,6-dimethyl-3,4-dihydro-1H-cinnolin-2-yl)-methanone

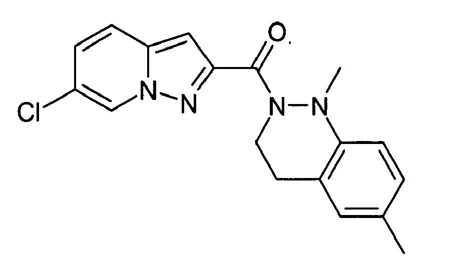

In close analogy to the procedure described in Example 1, 6-chloro-pyrazolo[1,5-a]pyridine-2-carboxylic acid is reacted with 1,6-dimethyl-1,2,3,4-tetrahydro-cinnoline to provide the title compound in good to moderate yield.
LC/MS: m/z = 341 (MH⁺)

### Example 665

### (6-Bromo-pyrazolo[1,5-a]pyridin-2-yl)-(1,6-dimethyl-3,4-dihydro-1H-cinnolin-2-yl)-methanone

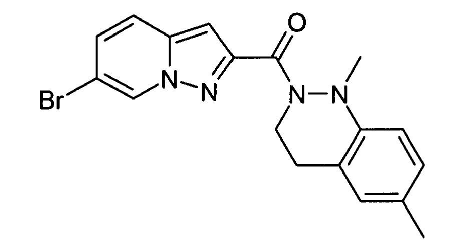

In close analogy to the procedure described in Example 1, 6-bromo-pyrazolo[1,5-a]pyridine-2-carboxylic acid is reacted with 1,6-dimethyl-1,2,3,4-tetrahydro-cinnoline to provide the title compound in good to moderate yield.
LC/MS: m/z = 386 (MH⁺)

### Example 666

### (6-Chloro-pyrazolo[1,5-a]pyridin-2-yl)-(1,5-dimethyl-3,4-dihydro-1H-cinnolin-2-yl)-methanone

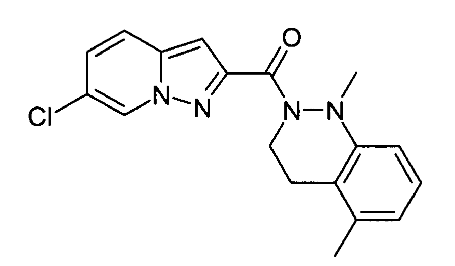

In close analogy to the procedure described in Example 1, 6-chloro-pyrazolo[1,5-a]pyridine-2-carboxylic acid is reacted with 1,5-dimethyl-1,2,3,4-tetrahydro-cinnoline to provide the title compound in good to moderate yield.
LC/MS: m/z = 341 (MH⁺)

### Example 667

### (6-Bromo-pyrazolo[1,5-a]pyridin-2-yl)-(1,5-dimethyl-3,4-dihydro-1H-cinnolin-2-yl)-methanone

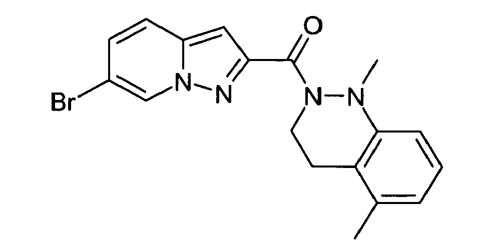

In close analogy to the procedure described in Example 1, 6-bromo-pyrazolo[1,5-a]pyridine-2-carboxylic acid is reacted with 1,5-dimethyl-1,2,3,4-tetrahydro-cinnoline to provide the title compound in good to moderate yield.
LC/MS: m/z = 386 (MH⁺)

### Example 668

### (6-Chloro-pyrazolo[1,5-a]pyridin-2-yl)-(8-methoxy-1-methyl-3,4-dihydro-1H-cinnolin-2-yl)-methanone

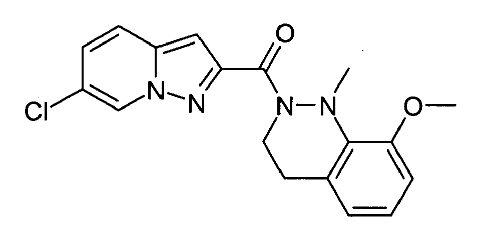

In close analogy to the procedure described in Example 1, 6-chloro-pyrazolo[1,5-a]pyridine-2-carboxylic acid is reacted with 8-methoxy-1-methyl-1,2,3,4-tetrahydro-cinnoline to provide the title compound in good to moderate yield.
LC/MS: m/z = 357 (MH⁺)

### Example 669

### (6-Bromo-pyrazolo[1,5-a]pyridin-2-yl)-(8-methoxy-1-methyl-3,4-dihydro-1H-cinnolin-2-yl)-methanone

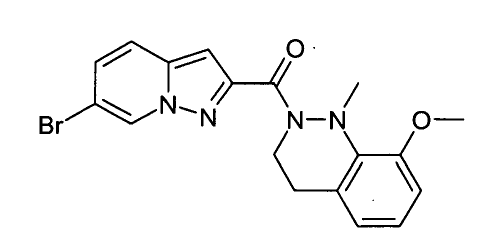

In close analogy to the procedure described in Example 1, 6-bromo-pyrazolo[1,5-a]pyridine-2-carboxylic acid is reacted with 8-methoxy-1-methyl-1,2,3,4-tetrahydro-cinnoline to provide the title compound in good to moderate yield.
LC/MS: m/z = 402 (MH⁺)

### Example 670

### (6-Chloro-pyrazolo[1,5-a]pyridin-2-yl)-(7-methoxy-1-methyl-3,4-dihydro-1H-cinnolin-2-yl)-methanone

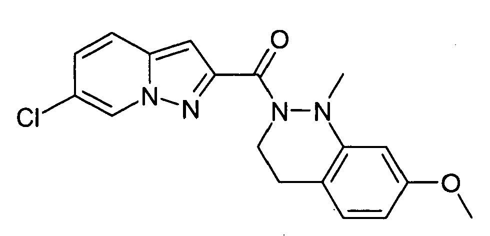

In close analogy to the procedure described in Example 1, 6-chloro-pyrazolo[1,5-a]pyridine-2-carboxylic acid is reacted with 7-methoxy-1-methyl-1,2,3,4-tetrahydro-cinnoline to provide the title compound in good to moderate yield.
LC/MS: m/z = 357 (MH⁺)

### Example 671

### (6-Bromo-pyrazolo[1,5-a]pyridin-2-yl)-(7-methoxy-1-methyl-3,4-dihydro- H-cinnolin-2-yl)-methanone

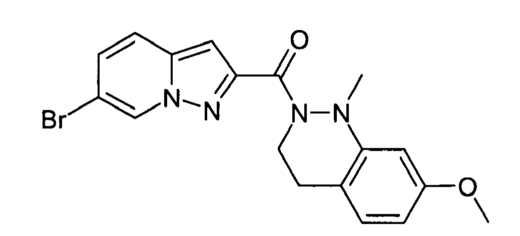

In close analogy to the procedure described in Example 1, 6-bromo-pyrazolo[1,5-a]pyridine-2-carboxylic acid is reacted with 7-methoxy-1-methyl-1,2,3,4-tetrahydro-cinnoline to provide the title compound in good to moderate yield.
LC/MS: m/z = 402 (MH⁺)

### Example 672

### (6-Chloro-pyrazolo[1,5-a]pyridin-2-yl)-(6-methoxy-1-methyl-3,4-dihydro-1H-cinnolin-2-yl)-methanone

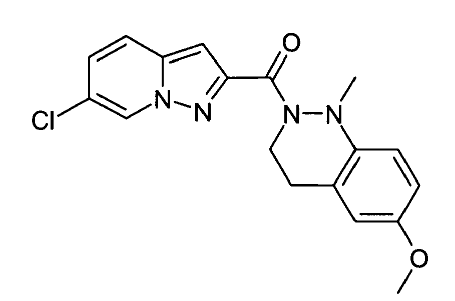

In close analogy to the procedure described in Example 1, 6-chloro-pyrazolo[1,5-a]pyridine-2-carboxylic acid is reacted with 6-methoxy-1-methyl-1,2,3,4-tetrahydro-cinnoline to provide the title compound in good to moderate yield.
LC/MS: m/z = 357 (MH⁺)

### Example 673

### (6-Bromo-pyrazolo[1,5-a]pyridin-2-yl)-(6-methoxy-1-methyl-3,4-dihydro-1H-cinnolin-2-yl)-methanone

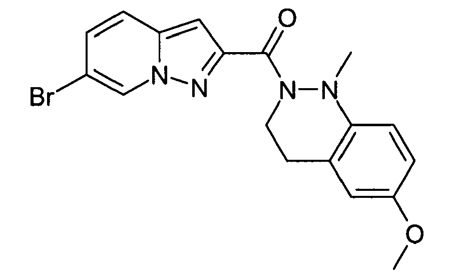

In close analogy to the procedure described in Example 1, 6-bromo-pyrazolo[1,5-a]pyridine-2-carboxylic acid is reacted with 6-methoxy-1-methyl-1,2,3,4-tetrahydro-cinnoline to provide the title compound in good to moderate yield.
LC/MS: m/z = 402 (MH⁺)

### Example 674

### (6-Chloro-pyrazolo[1,5-a]pyridin-2-yl)-(5-methoxy-1-methyl-3,4-dihydro-1H-cinnolin-2-yl)-methanone

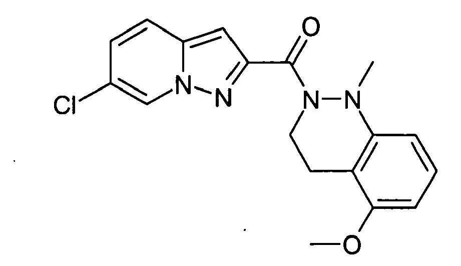

In close analogy to the procedure described in Example 1, 6-chloro-pyrazolo[1,5-a]pyridine-2-carboxylic acid is reacted with 5-methoxy-1-methyl-1,2,3,4-tetrahydro-cinnoline to provide the title compound in good to moderate yield.
LC/MS: m/z = 357 (MH⁺)

### Example 675

### (6-Bromo-pyrazolo[1,5-a]pyridin-2-yl)-(5-methoxy-1-methyl-3,4-dihydro-1H-cinnolin-2-yl)-methanone

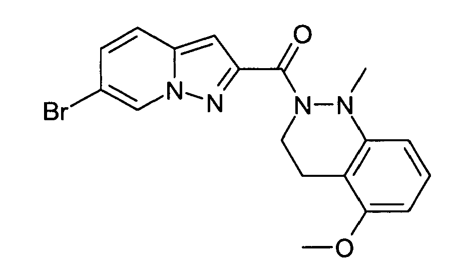

In close analogy to the procedure described in Example 1, 6-bromo-pyrazolo[1,5-a]pyridine-2-carboxylic acid is reacted with 5-methoxy-1-methyl-1,2,3,4-tetrahydro-cinnoline to provide the title compound in good to moderate yield.
LC/MS: m/z = 402 (MH⁺)

### Example 676

### (6-Chloro-pyrazolo[1,5-a]pyridin-2-yl)-(1-methyl-8-trifluoromethyl-3,4-dihydro-1H-cinnolin-2-yl)-methanone

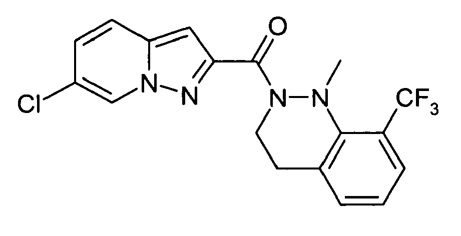

In close analogy to the procedure described in Example 1, 6-chloro-pyrazolo[1,5-a]pyridine-2-carboxylic acid is reacted with 1-methyl-8-trifluoromethyl-1,2,3,4-tetrahydro-cinnoline to provide the title compound in good to moderate yield.
LC/MS: m/z = 341 (MH⁺)

### Example 677

### (6-Bromo-pyrazolo[1,5-a]pyridin-2-yl)-(1-methyl-5-trifluoromethyl-3,4-dihydro-1H-cinnolin-2-yl)-methanone

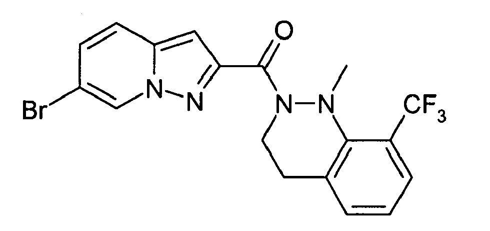

In close analogy to the procedure described in Example 1, 6-bromo-pyrazolo[1,5-a]pyridine-2-carboxylic acid is reacted with 1-methyl-8-trifluoromethyl-1,2,3,4-tetrahydro-cinnoline to provide the title compound in good to moderate yield.
LC/MS: m/z = 386 (MH⁺)

### Example 678

### (6-Chloro-pyrazolo[1,5-a]pyridin-2-yl)-(1-methyl-7-trifluoromethyl-3,4-dihydro-1H-cinnolin-2-yl)-methanone

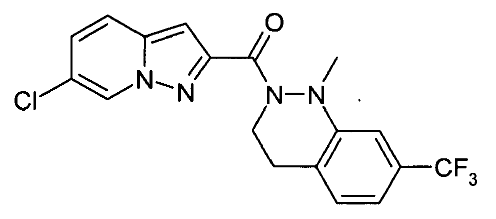

In close analogy to the procedure described in Example 1, 6-chloro-pyrazolo[1,5-a]pyridine-2-carboxylic acid is reacted with 1-methyl-7-trifluoromethyl-1,2,3,4-tetrahydro-cinnoline to provide the title compound in good to moderate yield.
LC/MS: m/z = 341 (MH⁺)

### Example 679

### (6-Bromo-pyrazolo[1,5-a]pyridin-2-yl)-(1-methyl-7-trifluoromethyl-3,4-dihydro-1H-cinnolin-2-yl)-methanone

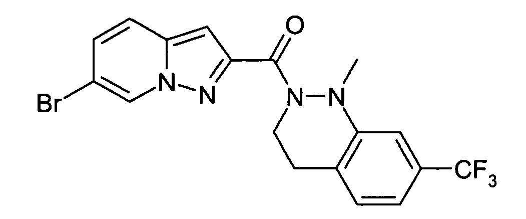

In close analogy to the procedure described in Example 1, 6-bromo-pyrazolo[1,5-a]pyridine-2-carboxylic acid is reacted with 1-methyl-7-trifluoromethyl-1,2,3,4-tetrahydro-cinnoline to provide the title compound in good to moderate yield.
LC/MS: m/z = 386 (MH⁺)

### Example 680

### (6-Chloro-pyrazolo[1,5-a]pyridin-2-yl)-(1-methyl-6-trifluoromethyl-3,4-dihydro-1H-cinnolin-2-yl)-methanone

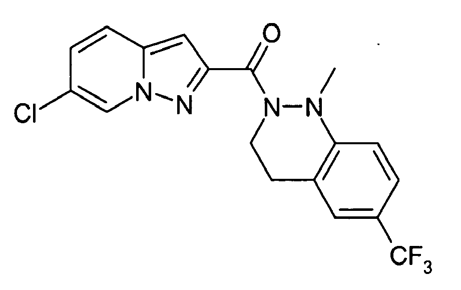

In close analogy to the procedure described in Example 1, 6-chloro-pyrazolo[1,5-a]pyridine-2-carboxylic acid is reacted with 1-methyl-6-trifluoromethyl-1,2,3,4-tetrahydro-cinnoline to provide the title compound in good to moderate yield.
LC/MS: m/z = 341 (MH⁺)

### Example 681

### (6-Bromo-pyrazolo[1,5-a]pyridin-2-yl)-(1-methyl-6-trifluoromethyl-3,4-dihydro-1H-cinnolin-2-yl)-methanone

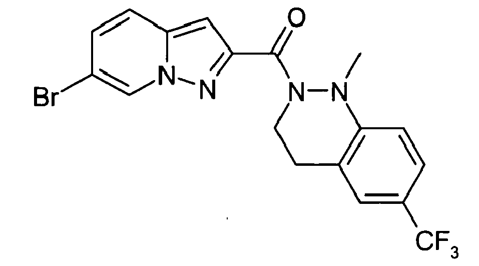

In close analogy to the procedure described in Example 1, 6-bromo-pyrazolo[1,5-a]pyridine-2-carboxylic acid is reacted with 1-methyl-6-trifluoromethyl-1,2,3,4-tetrahydro-cinnoline to provide the title compound in good to moderate yield.
LC/MS: m/z = 386 (MH⁺)

### Example 682

### (6-Chloro-pyrazolo[1,5-a]pyridin-2-yl)-(1-methyl-5-trifluoromethyl-3,4-dihydro-1H-cinnolin-2-yl)-methanone

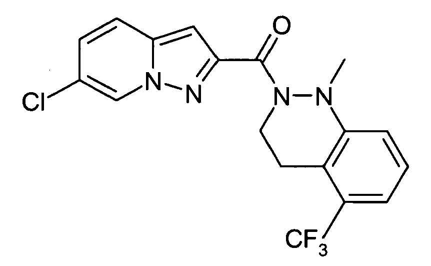

In close analogy to the procedure described in Example 1, 6-chloro-pyrazolo[1,5-a]pyridine-2-carboxylic acid is reacted with 1-methyl-5-trifluoromethyl-1,2,3,4-tetrahydro-cinnoline to provide the title compound in good to moderate yield.
LC/MS: m/z = 341 (MH⁺)

### Example 683

### (6-Bromo-pyrazolo[1,5-a]pyridin-2-yl)-(1-methyl-5-trifluoromethyl-3,4-dihydro-1H-cinnolin-2-yl)-methanone

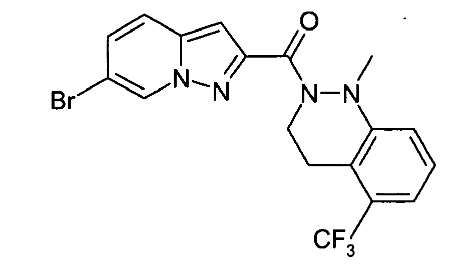

In close analogy to the procedure described in Example 1, 6-bromo-pyrazolo[1,5-a]pyridine-2-carboxylic acid is reacted with 1-methyl-5-trifluoromethyl-1,2,3,4-tetrahydro-cinnoline to provide the title compound in good to moderate yield.
LC/MS: m/z = 386 (MH⁺)

### Example 684

### N-[2-(6-Chloro-pyrazolo[1,5-a]pyridine-2-carbonyl)-1-methyl-1,2,3,4-tetrahydro-cinnolin-8-yl]-acetamide

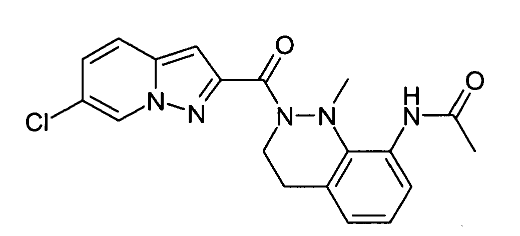

In close analogy to the procedure described in Example 1, 6-chloro-pyrazolo[1,5-a]pyridine-2-carboxylic acid is reacted with N-(1-methyl-1,2,3,4-tetrahydro-cinnolin-8-yl)-acetamide to provide the title compound in good to moderate yield.
LC/MS: m/z = 384 (MH⁺)

### Example 685

### N-[2-(6-Bromo-pyrazolo[1,5-a]pyridine-2-carbonyl)-1-methyl-1,2,3,4-tetrahydro-cinnolin-8-yl]-acetamide

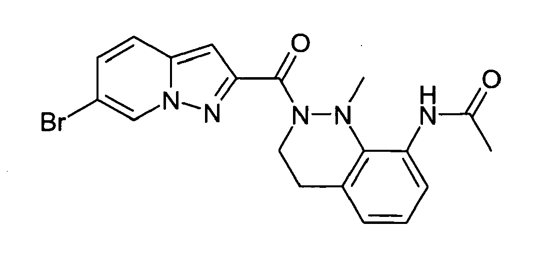

In close analogy to the procedure described in Example 1, 6-bromo-pyrazolo[1,5-a]pyridine-2-carboxylic acid is reacted with N-(1-methyl-1,2,3,4-tetrahydro-cinnolin-8-yl)-acetamide to provide the title compound in good to moderate yield.
LC/MS: m/z = 429 (MH⁺)

### Example 686

### N-[2-(6-Chloro-pyrazolo[1,5-a]pyridine-2-carbonyl)-1-methyl-1,2,3,4-tetrahydro-cinnolin-7-yl]-acetamide

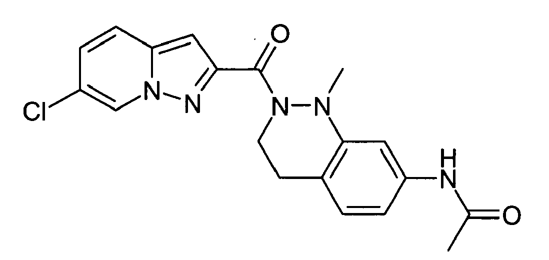

In close analogy to the procedure described in Example 1, 6-chloro-pyrazolo[1,5-a]pyridine-2-carboxylic acid is reacted with N-(1-methyl-1,2,3,4-tetrahydro-cinnolin-7-yl)-acetamide to provide the title compound in good to moderate yield.
LC/MS: m/z = 384 (MH⁺)

### Example 687

### N-[2-(6-Bromo-pyrazolo[1,5-a]pyridine-2-carbonyl)-1-methyl-1,2,3,4-tetrahydro-cinnolin-7-yl]-acetamide

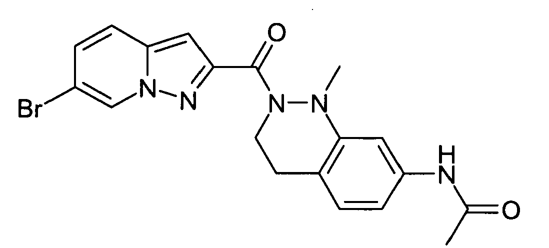

In close analogy to the procedure described in Example 1, 6-bromo-pyrazolo[1,5-a]pyridine-2-carboxylic acid is reacted with N-(1-methyl-1,2,3,4-tetrahydro-cinnolin-7-yl)-acetamide to provide the title compound in good to moderate yield.
LC/MS: m/z = 429 (MH⁺)

### Example 688

### N-[2-(6-Chloro-pyrazolo[1,5-a]pyridine-2-carbonyl)-1-methyl-1,2,3,4-tetrahydro-cinnolin-6-yl]-acetamide

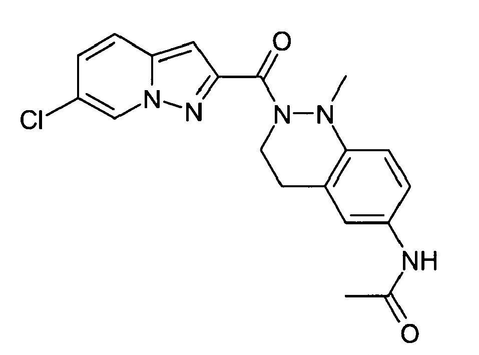

In close analogy to the procedure described in Example 1, 6-chloro-pyrazolo[1,5-a]pyridine-2-carboxylic acid is reacted with N-(1-methyl-1,2,3,4-tetrahydro-cinnolin-6-yl)-acetamide to provide the title compound in good to moderate yield.
LC/MS: m/z = 384 (MH⁺)
Example 689

### N-[2-(6-Bromo-pyrazolo[1,5-a]pyridine-2-carbonyl)-1-methyl-1,2,3,4-tetrahydro-cinnolin-6-yl]-acetamide

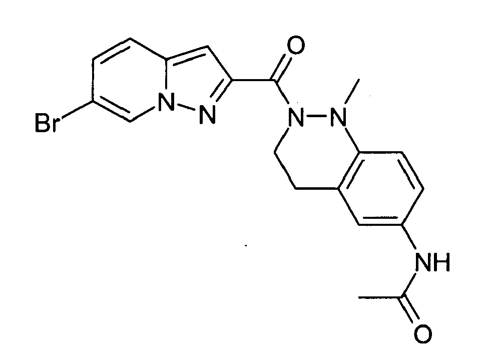

In close analogy to the procedure described in Example 1, 6-bromo-pyrazolo[1,5-a]pyridine-2-carboxylic acid is reacted with N-(1-methyl-1,2,3,4-tetrahydro-cinnolin-6-yl)-acetamide to provide the title compound in good to moderate yield.
LC/MS: m/z = 429 (MH⁺)

### Example 690

### N-[2-(6-Chloro-pyrazolo[1,5-a]pyridine-2-carbonyl)-1-methyl-1,2,3,4-tetrahydro-cinnolin-5-yl]-acetamide

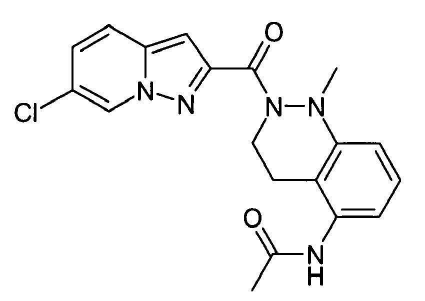

In close analogy to the procedure described in Example 1, 6-chloro-pyrazolo[1,5-a]pyridine-2-carboxylic acid is reacted with N-(1-methyl-1,2,3,4-tetrahydro-cinnolin-5-yl)-acetamide to provide the title compound in good to moderate yield.
LC/MS: m/z = 384 (MH⁺)

### Example 691

### N-[2-(6-Bromo-pyrazolo[1,5-a]pyridine-2-carbonyl)-1-methyl-1,2,3,4-tetrahydro-cinnolin-5-yl]-acetamide

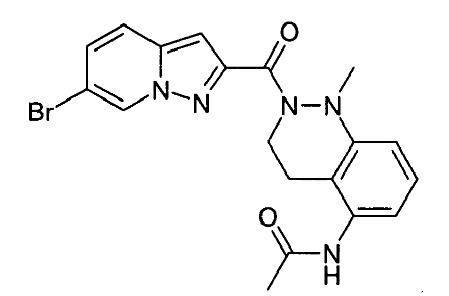

In close analogy to the procedure described in Example 1, 6-bromo-pyrazolo[1,5-a]pyridine-2-carboxylic acid is reacted with N-(I-methyl-1,2,3,4-tetrahydro-cinnolin-5-yl)-acetamide to provide the title compound in good to moderate yield.
LC/MS: m/z = 429 (MH⁺)

### Example 692

### (6-Chloro-pyrazolo[1,5-a]pyridin-2-yl)-(8-methanesulfonyl-1-methyl-3,4-dihydro-1H-cinnolin-2-yl)-methanone

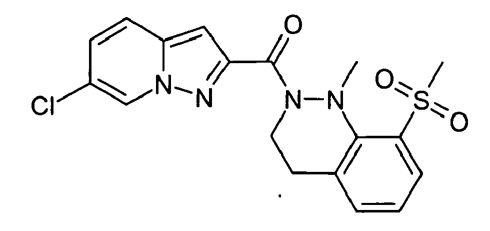

In close analogy to the procedure described in Example 1, 6-chloro-pyrazolo[1,5-a]pyridine-2-carboxylic acid is reacted with 8-methanesulfonyl-1-methyl-1,2,3,4-tetrahydro-cinnoline to provide the title compound in good to moderate yield.
LC/MS: m/z = 408 (MH⁺)

### Example 693

### (6-Bromo-pyrazolo[1,5-a]pyridin-2-yl)-(8-methanesulfonyl-1-methyl-3,4-dihydro-1H-cinnolin-2-yl)-methanone

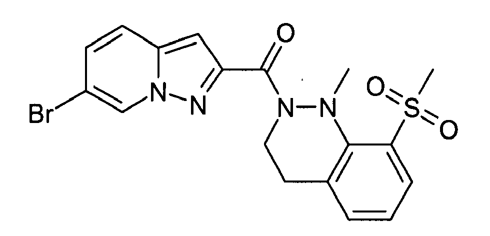

In close analogy to the procedure described in Example 1, 6-bromo-pyrazolo[1,5-a]pyridine-2-carboxylic acid is reacted with 8-methanesulfonyl-1-methyl-1,2,3,4-tetrahydro-cinnoline to provide the title compound in good to moderate yield.
LC/MS: m/z = 450 (MH⁺)

### Example 694

### (6-Chloro-pyrazolo[1,5-a]pyridin-2-yl)-(7-methanesulfonyl-1-methyl-3,4-dihydro-1H-cinnolin-2-yl)-methanone

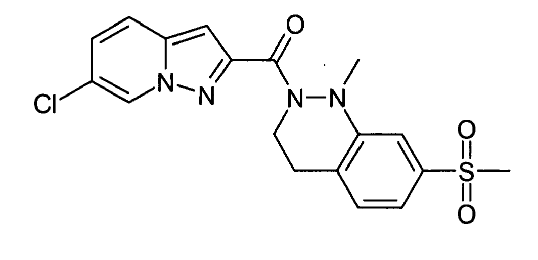

In close analogy to the procedure described in Example 1, 6-chloro-pyrazolo[1,5-a]pyridine-2-carboxylic acid is reacted with 7-methanesulfonyl-l-methyl-1,2,3,4-tetrahydro-cinnoline to provide the title compound in good to moderate yield.
LC/MS: m/z = 406 (MH⁺)

### Example 695

### (6-Bromo-pyrazolo[1,5-a]pyridin-2-yl)-(7-methanesulfonyl-1-methyl-3,4-dihydro-1H-cinnolin-2-yl)-methanone

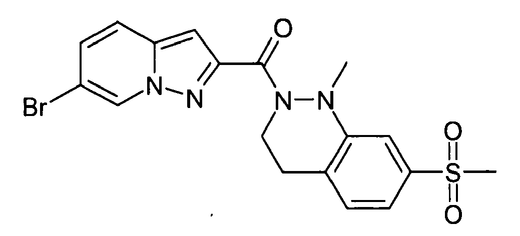

In close analogy to the procedure described in Example 1, 6-bromo-pyrazolo[1,5-a]pyridine-2-carboxylic acid is reacted with 7-methanesulfonyl-1-methyl-1,2,3,4-tetrahydro-cinnoline to provide the title compound in good to moderate yield.
LC/MS: m/z = 450 (MH⁺)

### Example 696

### (6-Chloro-pyrazolo[1,5-a]pyridin-2-yl)-(6-methanesulfonyl-1-methyl-3,4-dihydro-1H-cinnolin-2-yl)-methanone

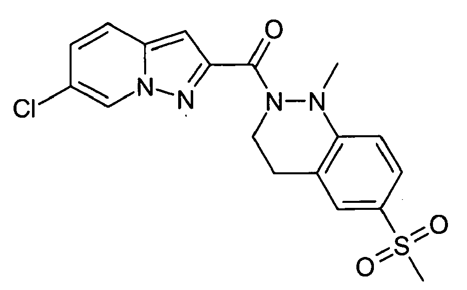

In close analogy to the procedure described in Example 1, 6-chloro-pyrazolo[1,5-a]pyridine-2-carboxylic acid is reacted with 6-methanesulfonyl-l-methyl-1,2,3,4-tetrahydro-cinnoline to provide the title compound in good to moderate yield.
LC/MS: m/z = 406 (MH⁺)

### Example 697

### (6-Bromo-pyrazolo[1,5-a]pyridin-2-yl)-(6-methanesulfonyl-1-methyl-3,4-dihydro-1H-cinnolin-2-yl)-methanone

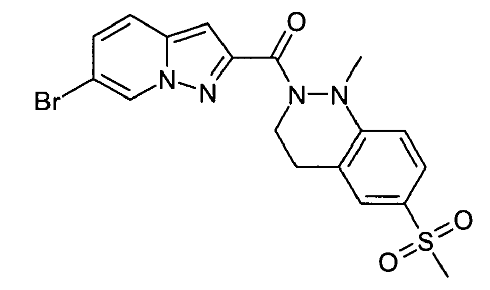

In close analogy to the procedure described in Example 1, 6-bromo-pyrazolo[1,5-a]pyridine-2-carboxylic acid is reacted with 6-methanesulfonyl-1-methyl-1,2,3,4-tetrahydro-cinnoline to provide the title compound in good to moderate yield.
LC/MS: m/z = 450 (MH⁺)

### Example 698

### (6-Chloro-pyrazolo[1,5-a]pyridin-2-yl)-(5-methanesulfonyl-1-methyl-3,4-dihydro-1H-cinnolin-2-yl)-methanone

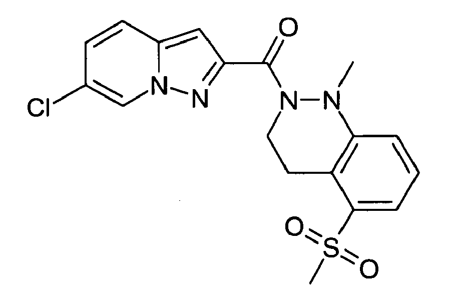

In close analogy to the procedure described in Example 1, 6-chloro-pyrazolo[1,5-a]pyridine-2-carboxylic acid is reacted with 5-methanesulfonyl-1-methyl-1,2,3,4-tetrahydro-cinnoline to provide the title compound in good to moderate yield.
LC/MS: m/z = 406 (MH⁺)

### Example 699

### (6-Bromo-pyrazolo[1,5-a]pyridin-2-yl)-(5-methanesulfonyl-1-methyl-3,4-dihydro-1H-cinnolin-2-yl)-methanone

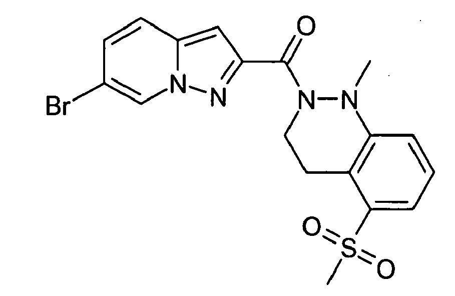

In close analogy to the procedure described in Example 1, 6-bromo-pyrazolo[1,5-a]pyridine-2-carboxylic acid is reacted with 5-methanesulfonyl-l-methyl-1,2,3,4-tetrahydro-cinnoline to provide the title compound in good to moderate yield.
LC/MS: m/z = 450 (MH⁺)

### Example 700

### (6-Chloro-pyrazolo[1,5-a]pyridin-2-yl)-(1-methyl-3,4-dihydro-1H-pyrido[2,3-c]pyridazin-2-yl)-methanone

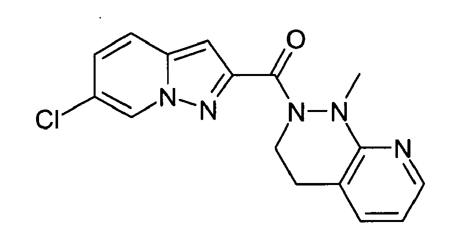

In close analogy to the procedure described in Example 1, 6-chloro-pyrazolo[1,5-a]pyridine-2-carboxylic acid is reacted with 1-methyl-1,2,3,4-tetrahydro-pyrido[2,3-c]pyridazine to provide the title compound in good to moderate yield.
LC/MS: m/z = 329 (MH⁺)

### Example 701

### (6-Bromo-pyrazolo[1,5-a]pyridin-2-yl)-(1-methyl-3,4-dihydro-1H-pyrido[2,3-c]pyridazin-2-yl)-methanone

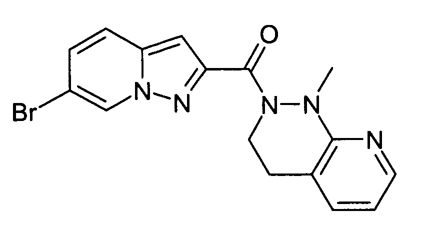

In close analogy to the procedure described in Example 1, 6-bromo-pyrazolo[1,5-a]pyridine-2-carboxylic acid is reacted with 1-methyl-1,2,3,4-tetrahydro-pyrido[2,3-c]pyridazine to provide the title compound in good to moderate yield.
LC/MS: m/z = 373 (MH⁺)

### Example 702

### (6-Chloro-pyrazolo[1,5-a]pyridin-2-yl)-(1-methyl-3,4-dihydro-1H-pyrido[3,4-c]pyridazin-2-yl)-methanone

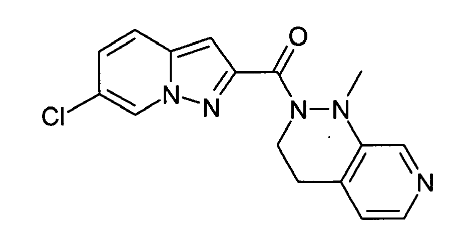

In close analogy to the procedure described in Example 1, 6-chloro-pyrazolo[1,5-a]pyridine-2-carboxylic acid is reacted with 1-methyl-1,2,3,4-tetrahydro-pyrido[3,4-c]pyridazine to provide the title compound in good to moderate yield.
LC/MS: m/z = 329 (MH⁺)

### Example 703

### (6-Bromo-pyrazolo[1,5-a]pyridin-2-yl)-(1-methyl-3,4-dihydro-1H-pyrido[3,4-c]pyridazin-2-yl)-methanone

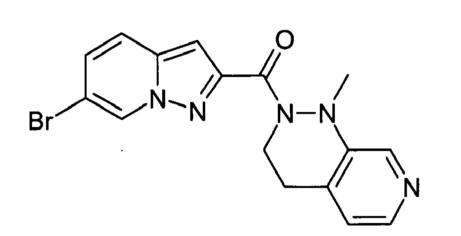

In close analogy to the procedure described in Example 1, 6-bromo-pyrazolo[1,5-a]pyridine-2-carboxylic acid is reacted with 1-methyl-1,2,3,4-tetrahydro-pyrido[3,4-c]pyridazine to provide the title compound in good to moderate yield.
LC/MS: m/z = 373 (MH⁺)

### Example 704

### (6-Chloro-pyrazolo[1,5-a]pyridin-2-yl)-(1-methyl-3,4-dihydro-1H-pyrido[4,3-c]pyridazin-2-yl)-methanone

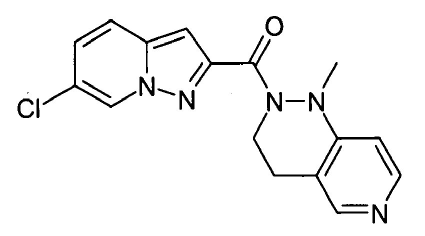

In close analogy to the procedure described in Example 1, 6-chloro-pyrazolo[1,5-a]pyridine-2-carboxylic acid is reacted with 1-methyl-1,2,3,4-tetrahydro-pyrido[4,3-c]pyridazine to provide the title compound in good to moderate yield.
LC/MS: m/z = 329 (MH⁺)

### Example 705

### (6-Bromo-pyrazolo[1,5-a]pyridin-2-yl)-(1-methyl-3,4-dihydro-1H-pyrido[4,3-c]pyridazin-2-yl)-methanone

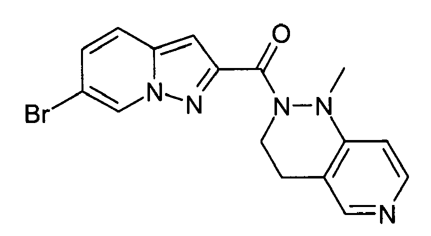

In close analogy to the procedure described in Example 1, 6-bromo-pyrazolo[1,5-a]pyridine-2-carboxylic acid is reacted with 1-methyl-1,2,3,4-tetrahydro-pyrido[4,3-c]pyridazine to provide the title compound in good to moderate yield.
LC/MS: m/z = 373 (MH⁺)

### Example 706

### (6-Chloro-pyrazolo[1,5-a]pyridin-2-yl)-(1-methyl-3,4-dihydro-1H-pyrido[3,2-c]pyridazin-2-yl)-methanone

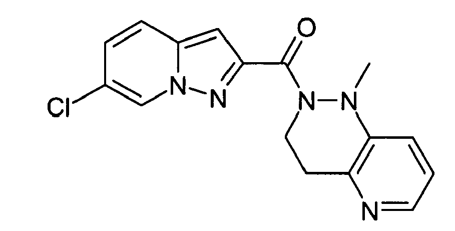

In close analogy to the procedure described in Example 1, 6-chloro-pyrazolo[1,5-a]pyridine-2-carboxylic acid is reacted with 1-methyl-1,2,3,4-tetrahydro-pyrido[3,2-c]pyridazine to provide the title compound in good to moderate yield.
LC/MS: m/z = 329 (MH⁺)

### Example 707

### (6-Bromo-pyrazolo[1,5-a]pyridin-2-yl)-(1-methyl-3,4-dihydro-1H-pyrido[3,2-c]pyridazin-2-yl)-methanone

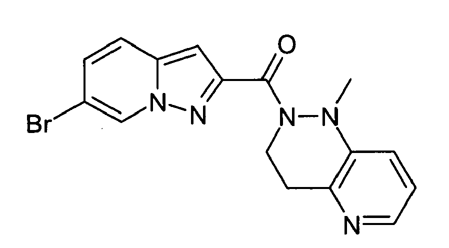

In close analogy to the procedure described in Example 1, 6-bromo-pyrazolo[1,5-a]pyridine-2-carboxylic acid is reacted with 1-methyl-1,2,3,4-tetrahydro-pyrido[3,2-c]pyridazine to provide the title compound in good to moderate yield.
LC/MS: m/z = 373 (MH⁺)

### Example 708

### (7-Bromo-1-methyl-3,4-dihydro-1H-pyrido[3,2-c]pyridazin-2-yl)-(6-chloro-pyrazolo[1,5-a]pyridin-2-yl)-methanone

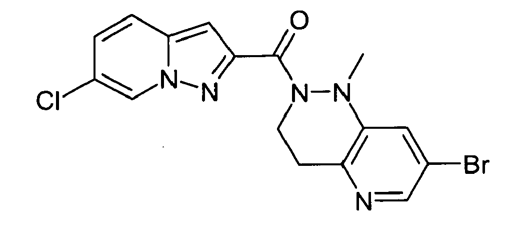

In close analogy to the procedure described in Example 1, 6-chloro-pyrazolo[1,5-a]pyridine-2-carboxylic acid is reacted with 7-bromo-1-methyl-1,2,3,4-tetrahydro-pyrido[3,2-c]pyridazine to provide the title compound in good to moderate yield.
LC/MS: m/z = 407 (MH⁺)

### Example 709

### (7-Bromo-1-methyl-3,4-dihydro-1H-pyrido[3,2-c]pyridazin-2-yl)-(6-bromo-pyrazolo[1,5-a]pyridin-2-yl)-methanone

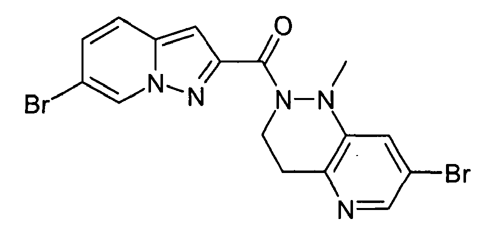

In close analogy to the procedure described in Example 1, 6-bromo-pyrazolo[1,5-a]pyridine-2-carboxylic acid is reacted with 7-bromo-1-methyl-1,2,3,4-tetrahydro-pyrido[3,2-c]pyridazine to provide the title compound in good to moderate yield.
LC/MS: m/z = 452 (MH⁺)

### Example 710

### (6-Chloro-pyrazolo[1,5-a]pyridin-2-yl)-(1-methyl-8-pyridin-4-yl-3,4-dihydro-1H-cinnolin-2-yl)-methanone

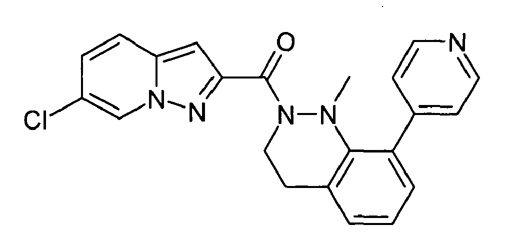

In close analogy to the procedure described in Example 1, 6-chloro-pyrazolo[1,5-a]pyridine-2-carboxylic acid is reacted with 1-methyl-8-pyridin-4-yl-1,2,3,4-tetrahydro-cinnoline to provide the title compound in good to moderate yield.
LC/MS: m/z = 405 (MH⁺)

### Example 711

### (6-Bromo-pyrazolo[1,5-a]pyridin-2-yl)-(1-methyl-8-pyridin-4-yl-3,4-dihydro-1H-cinnolin-2-yl)-methanone

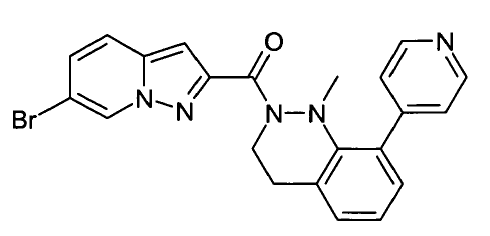

In close analogy to the procedure described in Example 1, 6-bromo-pyrazolo[1,5-a]pyridine-2-carboxylic acid is reacted with 1-methyl-8-pyridin-4-yl-1,2,3,4-tetrahydro-cinnoline to provide the title compound in good to moderate yield.
LC/MS: m/z = 449 (MH⁺)

### Example 712

### (6-Chloro-pyrazolo[1,5-a]pyridin-2-yl)-(1-methyl-7-pyridin-4-yl-3,4-dihydro-1H-cinnolin-2-yl)-methanone

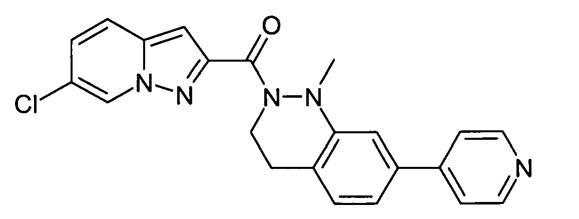

In close analogy to the procedure described in Example 1, 6-chloro-pyrazolo[1,5-a]pyridine-2-carboxylic acid is reacted with 1-methyl-7-pyridin-4-yl-1,2,3,4-tetrahydro-cinnoline to provide the title compound in good to moderate yield.
LC/MS: m/z = 405 (MH⁺)

### Example 713

### (6-Bromo-pyrazolo[1,5-a]pyridin-2-yl)-(1-methyl-7-pyridin-4-yl-3,4-dihydro-1H-cinnolin-2-yl)-methanone

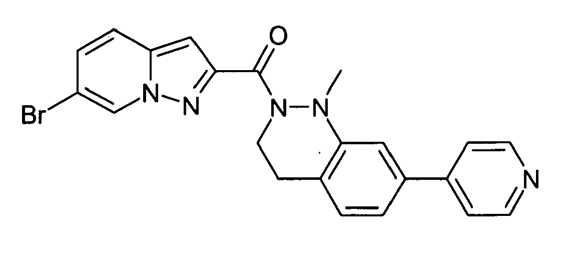

In close analogy to the procedure described in Example 1, 6-bromo-pyrazolo[1,5-a]pyridine-2-carboxylic acid is reacted with 1-methyl-7-pyridin-4-yl-1,2,3,4-tetrahydro-cinnoline to provide the title compound in good to moderate yield.
LC/MS: m/z = 449 (MH⁺)

### Example 714

### (6-Chloro-pyrazolo[1,5-a]pyridin-2-yl)-(1-methyl-6-pyridin-4-yl-3,4-dihydro-1H-cinnolin-2-yl)-methanone

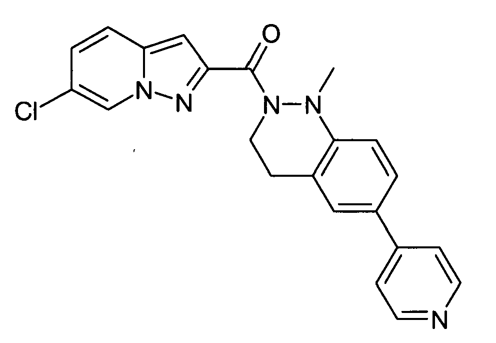

In close analogy to the procedure described in Example 1, 6-chloro-pyrazolo[1,5-a]pyridine-2-carboxylic acid is reacted with 1-methyl-6-pyridin-4-yl-1,2,3,4-tetrahydro-cinnoline to provide the title compound in good to moderate yield.
LC/MS: m/z = 405 (MH⁺)

### Example 715

### (6-Bromo-pyrazolo[1,5-a]pyridin-2-yl)-(1-methyl-6-pyridin-4-yl-3,4-dihydro-1H-cinnolin-2-yl)-methanone

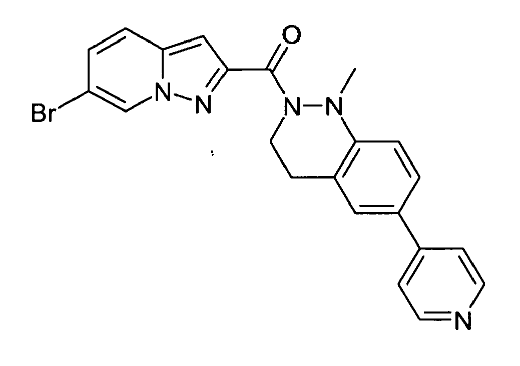

In close analogy to the procedure described in Example 1, 6-bromo-pyrazolo[1,5-a]pyridine-2-carboxylic acid is reacted with 1-methyl-6-pyridin-4-yl-1,2,3,4-tetrahydro-cinnoline to provide the title compound in good to moderate yield.
LC/MS: m/z = 449 (MH⁺)

### Example 716

### (6-Chloro-pyrazolo[1,5-a]pyridin-2-yl)-(1-methyl-5-pyridin-4-yl-3,4-dihydro-1H-cinnolin-2-yl)-methanone

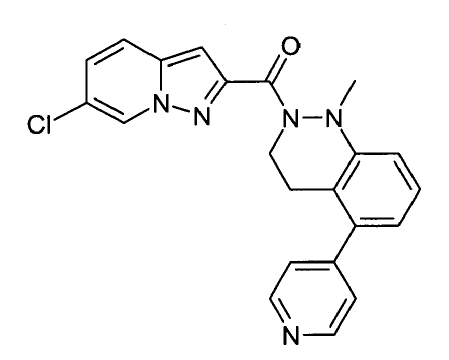

In close analogy to the procedure described in Example 1, 6-chloro-pyrazolo[1,5-a]pyridine-2-carboxylic acid is reacted with 1-methyl-5-pyridin-4-yl-1,2,3,4-tetrahydro-cinnoline to provide the title compound in good to moderate yield.
LC/MS: m/z = 405 (MH⁺)

### Example 717

### (6-Bromo-pyrazolo[1,5-a]pyridin-2-yl)-(1-methyl-5-pyridin-4-yl-3,4-dihydro-1H-cinnolin-2-yl)-methanone

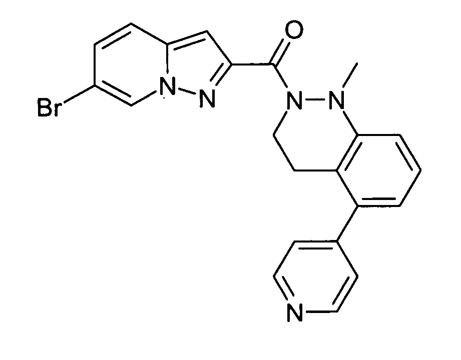

In close analogy to the procedure described in Example 1, 6-bromo-pyrazolo[1,5-a]pyridine-2-carboxylic acid is reacted with 1-methyl-5-pyridin-4-yl-1,2,3,4-tetrahydro-cinnoline to provide the title compound in good to moderate yield.
LC/MS: m/z = 449 (MH⁺)

### Example 718

### (6-Chloro-pyrazolo[1,5-a]pyridin-2-yl)-[8-(2-fluoro-pyridin-4-yl)-1-methyl-3,4-dihydro-1H-cinnolin-2-yl]-methanone

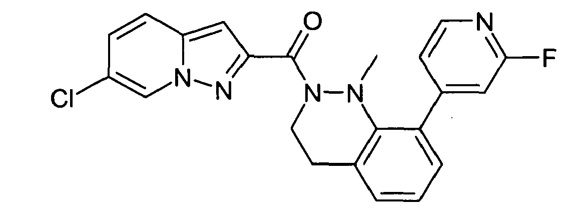

In close analogy to the procedure described in Example 1, 6-chloro-pyrazolo[1,5-a]pyridine-2-carboxylic acid is reacted with 8-(2-fluoro-pyridin-4-yl)-1-methyl-1,2,3,4-tetrahydro-cinnoline to provide the title compound in good to moderate yield.
LC/MS: m/z = 423 (MH⁺)

### Example 719

### (6-Bromo-pyrazolo[1,5-a]pyridin-2-yl)-[8-(2-fluoro-pyridin-4-yl)-1-methyl-3,4-dihydro-1H-cinnolin-2-yl]-methanone

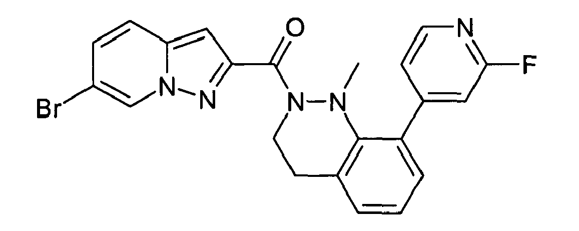

In close analogy to the procedure described in Example 1, 6-bromo-pyrazolo[1,5-a]pyridine-2-carboxylic acid is reacted with 8-(2-fluoro-pyridin-4-yl)-1-methyl-1,2,3,4-tetrahydro-cinnoline to provide the title compound in good to moderate yield.
LC/MS: m/z = 467 (MH⁺)

### Example 720

### (6-Chloro-pyrazolo[1,5-a]pyridin-2-yl)-[7-(2-fluoro-pyridin-4-yl)-1-methyl-3,4-dihydro-1H-cinnolin-2-yl]-methanone

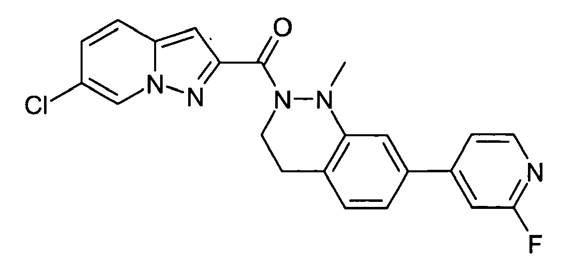

In close analogy to the procedure described in Example 1, 6-chloro-pyrazolo[1,5-a]pyridine-2-carboxylic acid is reacted with 7-(2-fluoro-pyridin-4-yl)-1-methyl-1,2,3,4-tetrahydro-cinnoline to provide the title compound in good to moderate yield.
LC/MS: m/z = 423 (MH⁺)

### Example 721

### (6-Bromo-pyrazolo[1,5-a]pyridin-2-yl)-[7-(2-fluoro-pyridin-4-yl)-1-methyl-3,4-dihydro-1H-cinnolin-2-yl]-methanone

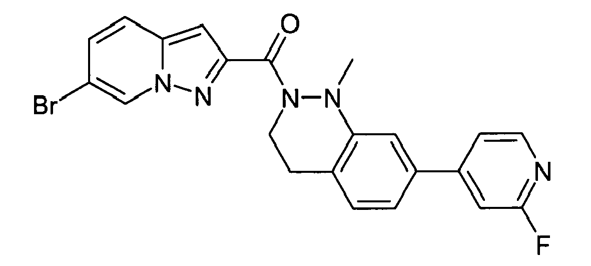

In close analogy to the procedure described in Example 1, 6-bromo-pyrazolo[1,5-a]pyridine-2-carboxylic acid is reacted with 7-(2-fluoro-pyridin-4-yl)-1-methyl-1,2,3,4-tetrahydro-cinnoline to provide the title compound in good to moderate yield.
LC/MS: m/z = 467 (MH⁺)

### Example 722

### (6-Chloro-pyrazolo[1,5-a]pyridin-2-yl)-[6-(2-fluoro-pyridin-4-yl)-1-methyl-3,4-dihydro-1 H-cinnolin-2-yl]-methanone

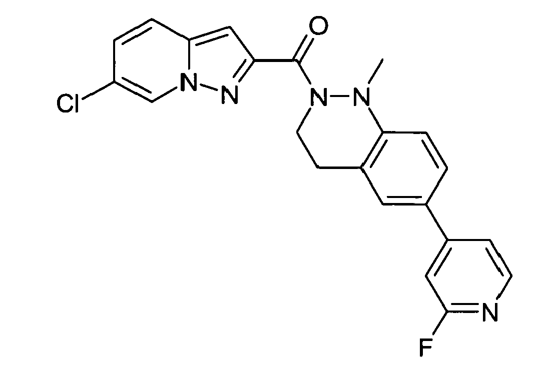

In close analogy to the procedure described in Example 1, 6-chloro-pyrazolo[1,5-a]pyridine-2-carboxylic acid is reacted with 6-(2-fluoro-pyridin-4-yl)-1-methyl-1,2,3,4-tetrahydro-cinnoline to provide the title compound in good to moderate yield.
LC/MS: m/z = 423 (MH⁺)

### Example 723

### (6-Bromo-pyrazolo[1,5-a]pyridin-2-yl)-[6-(2-fluoro-pyridin-4-yl)-1-methyl-3,4-dihydro-1 H-cinnolin-2-yl]-methanone

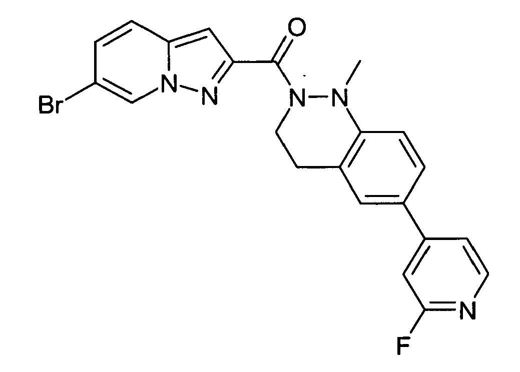

In close analogy to the procedure described in Example 1, 6-bromo-pyrazolo[1,5-a]pyridine-2-carboxylic acid is reacted with 6-(2-fluoro-pyridin-4-yl)-1-methyl-1,2,3,4-tetrahydro-cinnoline to provide the title compound in good to moderate yield.
LC/MS: m/z = 467 (MH⁺)

### Example 724

### (6-Chloro-pyrazolo[1,5-a]pyridin-2-yl)-[5-(2-fluoro-pyridin-4-yl)-1-methyl-3,4-dihydro-1H-cinnolin-2-yl]-methanone

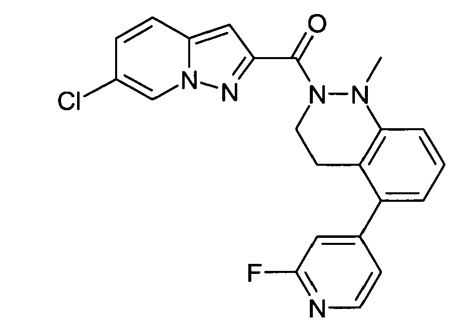

In close analogy to the procedure described in Example 1, 6-chloro-pyrazolo[1,5-a]pyridine-2-carboxylic acid is reacted with 5-(2-fluoro-pyridin-4-yl)-1-methyl-1,2,3,4-tetrahydro-cinnoline to provide the title compound in good to moderate yield.
LC/MS: m/z = 423 (MH⁺)

### Example 725

### (6-Bromo-pyrazolo[1,5-a]pyridin-2-yl)-[5-(2-fluoro-pyfidin-4-yl)-1-methyl-3,4-dihydro-1H-cinnolin-2-yl]-methanone

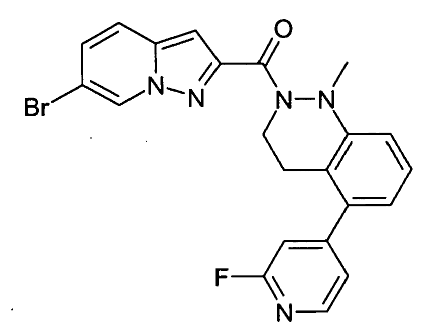

In close analogy to the procedure described in Example 1, 6-bromo-pyrazolo[1,5-a]pyridine-2-carboxylic acid is reacted with 5-(2-fluoro-pyridin-4-yl)-1-methyl-1,2,3,4-tetrahydro-cinnoline to provide the title compound in good to moderate yield.
LC/MS: m/z = 467 (MH⁺)

### Example 726

### 6-Bromo-pyrazolo[1,5-a]pyridine-2-carboxylic acid benzylamide

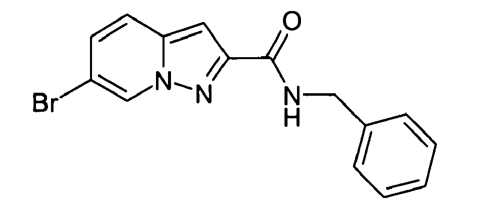

In close analogy to the procedure described in Example 1, 6-bromo-pyrazolo[1,5-a]pyridine-2-carboxylic acid is reacted with benzylamine to provide the title compound in good to moderate yield.
LC/MS: m/z = 331 (MH⁺)

### Example 727

### 6-Chloro-pyrazolo[1,5-a]pyridine-2-carboxylic acid benzylamide

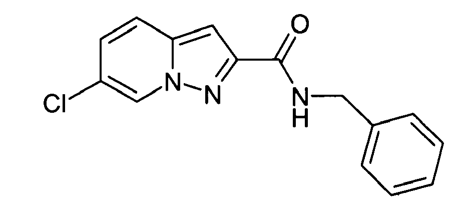

In close analogy to the procedure described in Example 1, 6-chloro-pyrazolo[1,5-a]pyridine-2-carboxylic acid is reacted with benzylamine to provide the title compound in good to moderate yield.
LC/MS: m/z = 286 (MH⁺)

### Example 728

### 6-Chloro-pyrazolo[1,5-a]pyridine-2-carboxylic acid (1-methyl-1-phenyl-ethyl)-amide

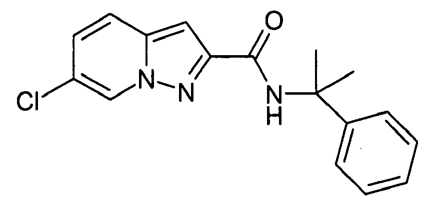

In close analogy to the procedure described in Example 1, 6-chloro-pyrazolo[1,5-a]pyridine-2-carboxylic acid is reacted with 1-methyl-1-phenyl-ethylamine to provide the title compound in good to moderate yield.
LC/MS: m/z = 314 (MH⁺)

### Example 729

### 6-Bromo-pyrazolo[1,5-a]pyridine-2-carboxylic acid (1-methyl-1-phenyl-ethyl)-amide

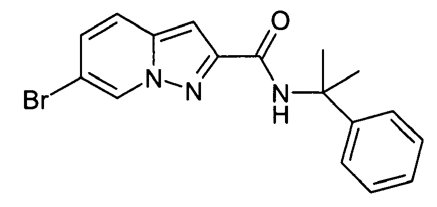

In close analogy to the procedure described in Example 1, 6-bromo-pyrazolo[1,5-a]pyridine-2-carboxylic acid is reacted with 1-methyl-1-phenyl-ethylamine to provide the title compound in good to moderate yield.
LC/MS: m/z = 359 (MH⁺)

### Example 730

### 6-Bromo-pyrazolo[1,5-a]pyridine-2-carboxylic acid (2-methyl-2-phenyl-propyl)-amide

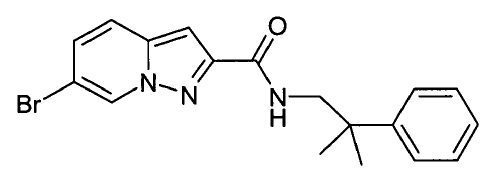

In close analogy to the procedure described in Example 1, 6-bromo-pyrazolo[1,5-a]pyridine-2-carboxylic acid is reacted with 1-methyl-1-phenyl-propylamine to provide the title compound in good to moderate yield.
LC/MS: m/z = 373 (MH⁺)

### Example 731

### 6-Chloro-pyrazolo[1,5-a]pyridine-2-carboxylic acid (2-methyl-2-phenyl-propyl)-amide

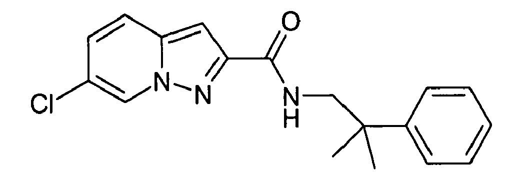

In close analogy to the procedure described in Example 1, 6-chloro-pyrazolo[1,5-a]pyridine-2-carboxylic acid is reacted with 1-methyl-1-phenyl-propylamine to provide the title compound in good to moderate yield.
LC/MS: m/z = 328 (MH⁺)

### Example 732

### 6-Chloro-pyrazolo[1,5-a]pyridine-2-carboxylic acid benzyl-methyl-amide

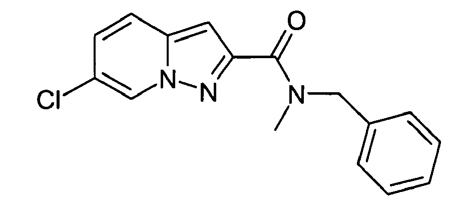

In close analogy to the procedure described in Example 1, 6-chloro-pyrazolo[1,5-a]pyridine-2-carboxylic acid is reacted with benzyl methyl amine to provide the title compound in good to moderate yield.
LC/MS: m/z = 300 (MH⁺)

### Example 733

### 6-Bromo-pyrazolo[1,5-a]pyridine-2-carboxylic acid benzyl-methyl-amide

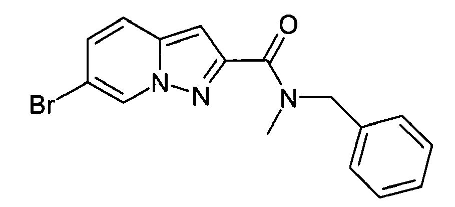

In close analogy to the procedure described in Example 1, 6-bromo-pyrazolo[1,5-a]pyridine-2-carboxylic acid is reacted with benzyl methyl amine to provide the title compound in good to moderate yield.
LC/MS: m/z = 345 (MH⁺)

### Example 734

### 6-Bromo-pyrazolo[1,5-a]pyridine-2-carboxylic acid methyl-(1-phenyl-ethyl)-amide

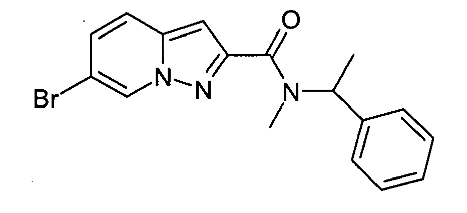

In close analogy to the procedure described in Example 1, 6-bromo-pyrazolo[1,5-a]pyridine-2-carboxylic acid is reacted with methyl-(1-phenyl-ethyl)-amine to provide the title compound in good to moderate yield.
LC/MS: m/z = 359 (MH⁺)
The stereo-isomers of this compound are separated. The S-configurated compound has a different activity than the R-configurated compound.

### Example 735

### 6-Chloro-pyrazolo[1,5-a]pyridine-2-carboxylic acid methyl-(1-phenyl-ethyl)-amide

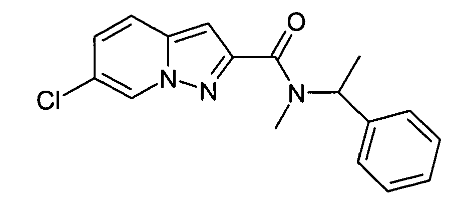

In close analogy to the procedure described in Example 1, 6-chloro-pyrazolo[1,5-a]pyridine-2-carboxylic acid is reacted with methyl-(1-phenyl-ethyl)-amine to provide the title compound in good to moderate yield.
LC/MS: m/z = 314 (MH⁺)
The stereo-isomers of this compound are separated. The S-configurated compound has a different activity than the R-configurated compound.

### Example 736

### (4,6-Dichloro-pyrazolo[1,5-a]pyridin-2-yl)-(3,4-dihydro-1H-isoquinolin-2-yl)-methanone

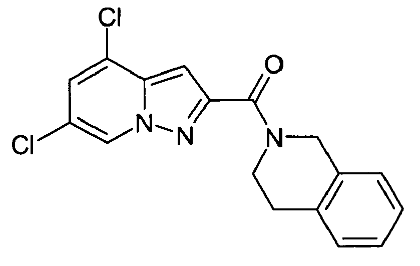

In close analogy to the procedure described in Example 1, 4,6-dichloro-pyrazolo[1,5-a]pyridine-2-carboxylic acid is reacted with 1,2,3,4-tetrahydroisoquinoline to provide the title compound in good to moderate yield.
LC/MS: m/z = 346 (MH⁺)

### Example 737

### (4,6-Dichloro-pyrazolo[1,5-a]pyridin-2-yl)-(1-methyl-3,4-dihydro-1H-isoquinolin-2-yl)-methanone

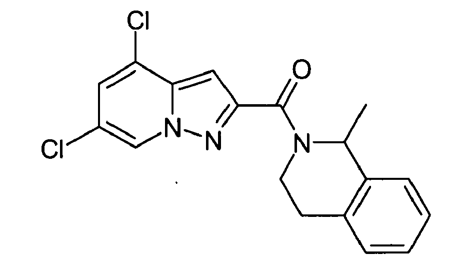

In close analogy to the procedure described in Example 1, 4,6-dichloro-pyrazolo[1,5-a]pyridine-2-carboxylic acid is reacted with 1-methyl-1,2,3,4-tetrahydroisoquinoline to provide the title compound in good to moderate yield.
LC/MS: m/z = 360 (MH⁺)
The stereo-isomers of this compound are separated. The S-configurated compound has a different activity than the R-configurated compound.

### Example 738

### (4,6-Dichloro-pyrazolo[1,5-a]pyridin-2-yl)-(7-fluoro-1-methyl-3,4-dihydro-1H-isoquinolin-2-yl)-methanone

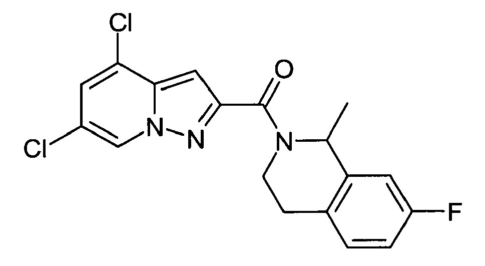

In close analogy to the procedure described in Example 1, 4,6-dichloro-pyrazolo[1,5-a]pyridine-2-carboxylic acid is reacted with 7-fluoro-1-methyl-1,2,3,4-tetrahydroisoquinoline to provide the title compound in good to moderate yield.
LC/MS: m/z = 378 (MH⁺)
The stereo-isomers of this compound are separated. The S-configurated compound has a different activity than the R-configurated compound.

### Example 739

### (3-Bromo-7,8-dihydro-5H-1,6-naphthyridin-6-yl)-(4,6-dichloro-pyrazolo[1,5-a]pyridin-2-yl)-methanone

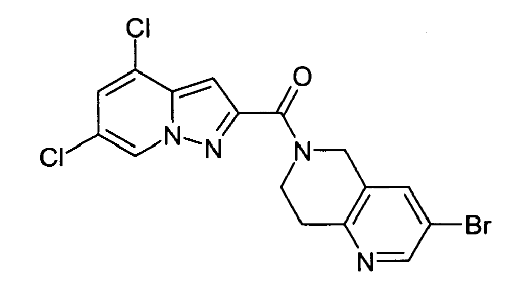

In close analogy to the procedure described in Example 1, 4,6-dichloro-pyrazolo[1,5-a]pyridine-2-carboxylic acid is reacted with 3-bromo-5,6,7,8-tetrahydro-1,6-naphthyridineto provide the title compound in good to moderate yield.
LC/MS: m/z = 426 (MH⁺)

### Example 740

### (4,6-Dichloro-pyrazolo[1,5-a]pyridin-2-yl)-(7-pyridin-2-yl-3,4-dihydro-1H-isoquinolin-2-yl)-methanone

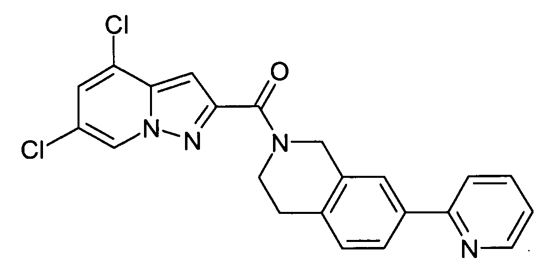

In close analogy to the procedure described in Example 1, 4,6-dichloro-pyrazolo[1,5-a]pyridine-2-carboxylic acid is reacted with 7-pyridin-2-yl-1,2,3,4-tetrahydro-isoquinolineto provide the title compound in good to moderate yield.
LC/MS: m/z = 423 (MH⁺)

### Example 741

### (4,6-Dichloro-pyrazolo[1,5-a]pyridin-2-yl)-(4-methyl-6,7-dihydro-4H-thieno[3,2-c]pyridin-5-yl)-methanone

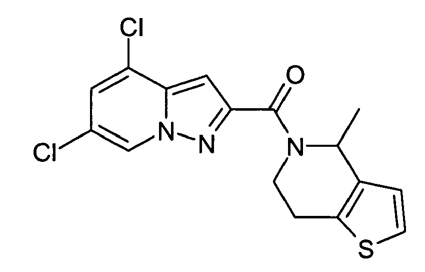

In close analogy to the procedure described in Example 1, 4,6-dichloro-pyrazolo[1,5-a]pyridine-2-carboxylic acid is reacted with 4-methyl-4,5,6,7-tetrahydro-thieno[3,2-c]pyridine to provide the title compound in good to moderate yield.
LC/MS: m/z = 366 (MH⁺)
The stereo-isomers of this compound are separated. The S-configurated compound has a different activity than the R-configurated compound.

### Example 742

### (4,6-Dichloro-pyrazolo[1,5-a]pyridin-2-yl)-(4-methyl-6,7-dihydro-4H-furo[3,2-c]pyridin-5-yl)-methanone

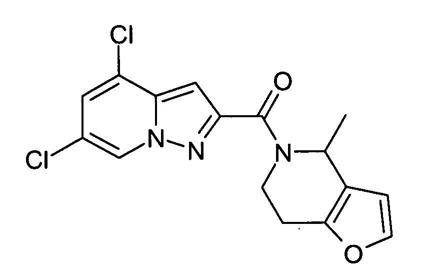

In close analogy to the procedure described in Example 1, 4,6-dichloro-pyrazolo[1,5-a]pyridine-2-carboxylic acid is reacted with 4-methyl-4,5,6,7-tetrahydro-furo[3,2-c]pyridine to provide the title compound in good to moderate yield.
LC/MS: m/z = 350 (MH⁺)
The stereo-isomers of this compound are separated. The S-configurated compound has a different activity than the R-configurated compound.

### Example 743

### (4,6-Dichloro-pyrazolo[1,5-a]pyridin-2-yl)-(1-methyl-3,4-dihydro-1H-cinnolin-2-yl)-methanone

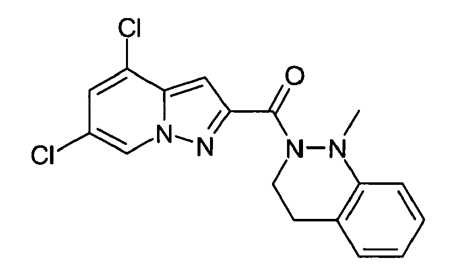

In close analogy to the procedure described in Example 1, 4,6-dichloro-pyrazolo[1,5-a]pyridine-2-carboxylic acid is reacted with 1-metbyl-1,2,3,4-tetrahydro-cinnoline to provide the title compound in good to moderate yield.
LC/MS: m/z = 361 (MH⁺)

### Example 744

### (4,6-Dichloro-pyrazolo[1,5-a]pyridin-2-yl)-(5,6-dihydro-8H-imidazo[1,2-a]pyrazin-7-yl)-methanone

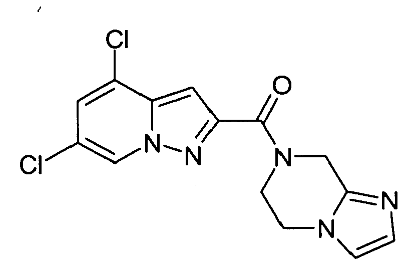

In close analogy to the procedure described in Example 1, 4,6-dichloro-pyrazolo[1,5-a]pyridine-2-carboxylic acid is reacted with 5,6,7,8-tetrahydro-imidazo[1,2-a]pyrazine to provide the title compound in good to moderate yield.
LC/MS: m/z = 336 (MH⁺)

### Example 745

### (4,6-Dichloro-pyrazolo[1,5-a]pyridin-2-yl)-(5,6-dihydro-8H-[1,2,4]triazolo[4,3-a]pyrazin-7-yl)-methanone

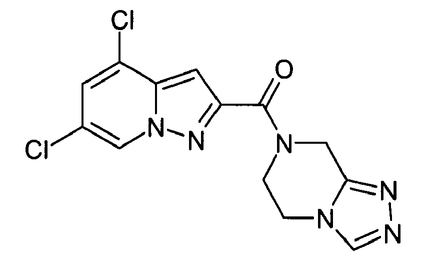

In close analogy to the procedure described in Example 1, 4,6-dichloro-pyrazolo[1,5-a]pyridine-2-carboxylic acid is reacted with 5,6,7,8-tetrahydro-[1,2,4]triazolo[4,3-a]pyrazine to provide the title compound in good to moderate yield.
LC/MS: m/z = 337 (MH⁺)

### Example 746

### (4,6-Dichloro-pyrazolo[1,5-a]pyridin-2-yl)-(1-methyl-3,4-dihydro-1H-pyrrolo[1,2-a]pyrazin-2-yl)-methanone

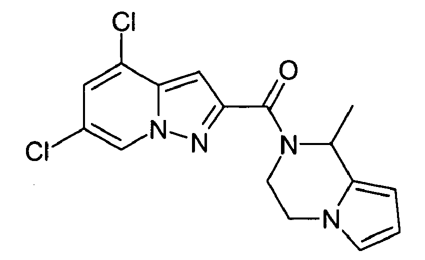

In close analogy to the procedure described in Example 1, 4,6-dichloro-pyrazolo[1,5-a]pyridine-2-carboxylic acid is reacted with 1-methyl-1,2,3,4-tetrahydro-pyrrolo[1,2-a]pyrazine to provide the title compound in good to moderate yield in good to moderate yield.
LC/MS: m/z = 349 (MH⁺)
The stereo-isomers of this compound are separated. The S-configurated compound has a different activity than the R-configurated compound.

### Example 747

### 4,6-Dichloro-pyrazolo[1,5-a]pyridine-2-carboxylic acid methyl-(1-phenyl-ethyl)-amide

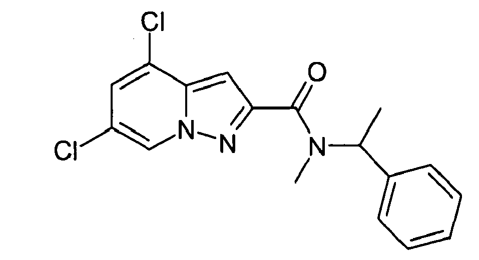

In close analogy to the procedure described in Example 1, 4,6-dichloro-pyrazolo[1,5-a]pyridine-2-carboxylic acid is reacted with methyl-(1-phenyl-ethyl)-amine to provide the title compound in good to moderate yield.
LC/MS: m/z = 348 (MH⁺)
The stereo-isomers of this compound are separated. The S-configurated compound has a different activity than the R-configurated compound.

### Example 748

### 4,6-Dichloro-pyrazolo[1,5-a]pyridine-2-carboxylic acid benzyl-methyl-amide

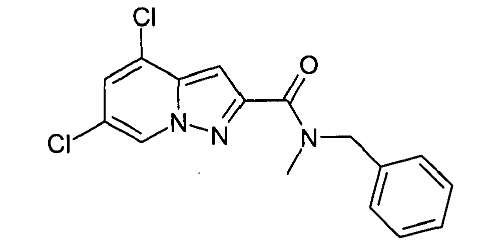

In close analogy to the procedure described in Example 1, 4,6-dichloro-pyrazolo[1,5-a]pyridine-2-carboxylic acid is reacted with benzyl methyl amine to provide the title compound in good to moderate yield.
LC/MS: m/z = 334 (MH⁺)

### Example 749

### 4,6-Dichloro-pyrazolo[1,5-a]pyridine-2-carboxylic acid (2-methyl-2-phenyl-propyl)-amide

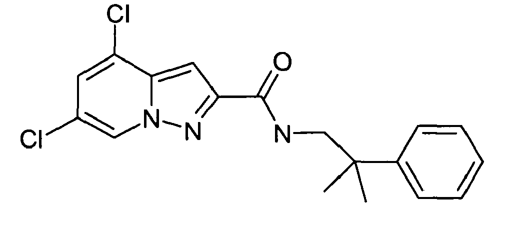

In close analogy to the procedure described in Example 1, 4,6-dichloro-pyrazolo[1,5-a]pyridine-2-carboxylic acid is reacted with 1-methyl-1-phenyl-propylamine to provide the title compound in good to moderate yield.
LC/MS: m/z = 362 (MH⁺)

### Example 750

### 4,6-Dichloro-pyrazolo[1,5-a]pyridine-2-carboxylic acid adamantyl-1 amide

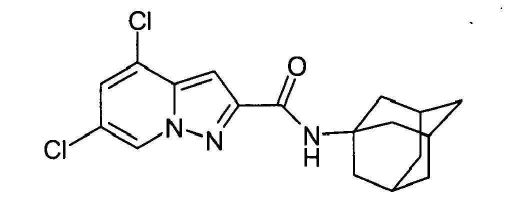

In close analogy to the procedure described in Example 1, 4,6-dichloro-pyrazolo[1,5-a]pyridine-2-carboxylic acid is reacted with adamantyl-1 amine to provide the title compound in moderate yield.
LC/MS: m/z = 364 (MH⁺)

### Example 751

### (4,6-Dichloro-pyrazolo[1,5-a]pyridin-2-yl)-((R)-1-methyl-3,4-dihydro-1H-isoquinolin-2-yl)-methanone

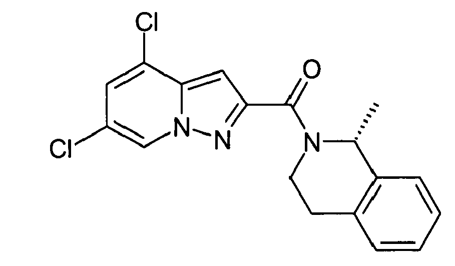

In close analogy to the procedure described in Example 1, 4,6-dichloro-pyrazolo[1,5-a]pyridine-2-carboxylic acid is reacted with (R)-1-methyl-1,2,3,4-tetrahydroisoquinoline to provide the title compound in good to moderate yield.
LC/MS: m/z = 360 (MH⁺)

### Example 752

### (4,6-Dibromo-pyrazolo[1,5-a]pyridin-2-yl)-(3,4-dihydro-1H-isoquinolin-2-yl)-methanone

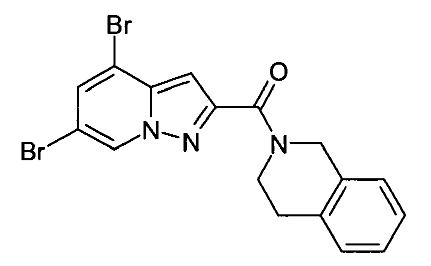

In close analogy to the procedure described in Example 1, 4,6-dibromo-pyrazolo[1,5-a]pyridine-2-carboxylic acid is reacted with 1,2,3,4-tetrahydroisoquinoline to provide the title compound in good to moderate yield.
LC/MS: m/z = 436 (MH⁺)

### Example 753

### (4,6-Dibromo-pyrazolo[1,5-a]pyridin-2-yl)-(1-methyl-3,4-dihydro-1H-isoquinolin-2-yl)-methanone

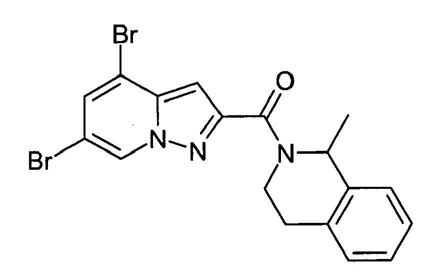

In close analogy to the procedure described in Example 1, 4,6-dibromo-pyrazolo[1,5-a]pyridine-2-carboxylic acid is reacted with 1-methyl-1,2,3,4-tetrahydroisoquinoline to provide the title compound in good to moderate yield.
LC/MS: m/z = 450 (MH⁺)
The stereo-isomers of this compound are separated. The S-configurated compound has a different activity than the R-configurated compound.

### Example 754

### (4,6-Dibromo-pyrazolo[1,5-a]pyridin-2-yl)-(7-fluoro-1-methyl-3,4-dihydro-1H-isoquinolin-2-yl)-methanone

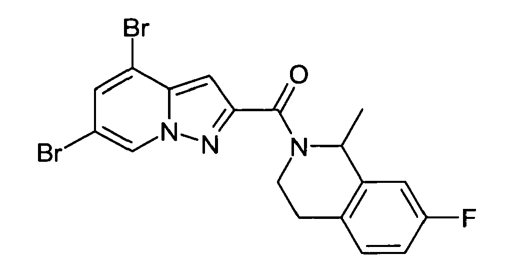

In close analogy to the procedure described in Example 1, 4,6-dibromo-pyrazolo[1,5-a]pyridine-2-carboxylic acid is reacted with 7-fluoro-1-methyl-1,2,3,4-tetrahydroisoquinoline to provide the title compound in good to moderate yield.
LC/MS: m/z = 468 (MH⁺)
The stereo-isomers of this compound are separated. The S-configurated compound has a different activity than the R-configurated compound.

### Example 755

### (3-Bromo-7,8-dihydro-5H-1,6-naphthyridin-6-yl)-(4,6-dibromo-pyrazolo[1,5-a]pyridin-2-yl)-methanone

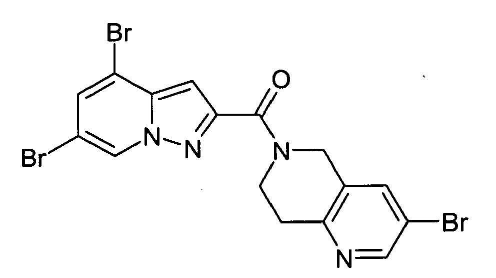

In close analogy to the procedure described in Example 1, 4,6-dibromo-pyrazolo[1,5-a]pyridine-2-carboxylic acid is reacted with 3-bromo-5,6,7,8-tetrahydro-1,6-naphthyridineto provide the title compound in good to moderate yield.
LC/MS: m/z = 516 (MH⁺)

### Example 756

### (4,6-Dibromo-pyrazolo[1,5-a]pyridin-2-yl)-(7-pyridin-2-yl-3,4-dihydro-1H-isoquinolin-2-yl)-methanone

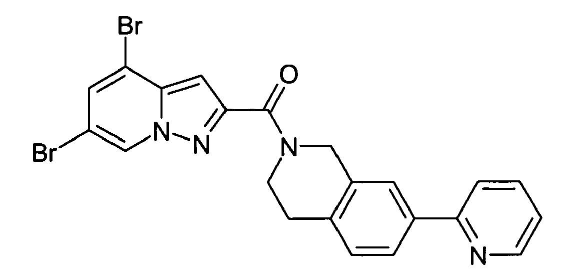

In close analogy to the procedure described in Example 1, 4,6-dibromo-pyrazolo[1,5-a]pyridine-2-carboxylic acid is reacted with 7-pyridin-2-yl-1,2,3,4-tetrahydro-isoquinolineto provide the title compound in good to moderate yield.
LC/MS: m/z = 513 (MH⁺)

### Example 757

### (4,6-Dibromo-pyrazolo[1,5-a]pyridin-2-yl)-(4-methyl-6,7-dihydro-4H-thieno[3,2-c]pyridin-5-yl)-methanone

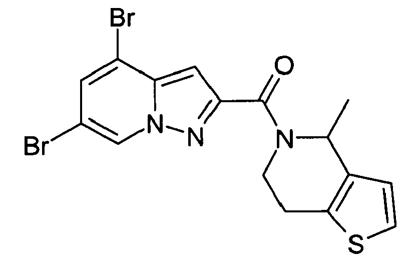

In close analogy to the procedure described in Example 1, 4,6-dibromo-pyrazolo[1,5-a]pyridine-2-carboxylic acid is reacted with 4-methyl-4,5,6,7-tetrahydro-thieno[3,2-c]pyridine to provide the title compound in good to moderate yield.
LC/MS: m/z = 456 (MH⁺)
The stereo-isomers of this compound are separated. The S-configurated compound has a different activity than the R-configurated compound.

### Example 758

### (4,6-Dibromo-pyrazolo[1,5-a]pyridin-2-yl)-(4-methyl-6,7-dihydro-4H-furo[3,2-c]pyridin-5-yl)-methanone

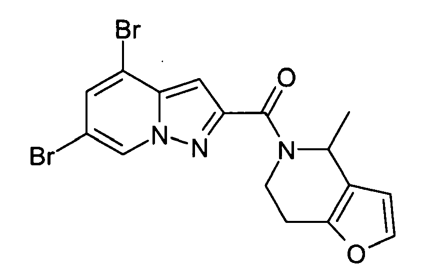

In close analogy to the procedure described in Example 1, 4,6-dibromo-pyrazolo[1,5-a]pyridine-2-carboxylic acid is reacted with 4-methyl-4,5,6,7-tetrahydro-furo[3,2-c]pyridine to provide the title compound in good to moderate yield.
LC/MS: m/z = 440 (MH⁺)
The stereo-isomers of this compound are separated. The S-configurated compound has a different activity than the R-configurated compound.

### Example 759

### (4,6-Dibromo-pyrazolo[1,5-a]pyridin-2-yl)-(1-methyl-3,4-dihydro-1H-cinnolin-2-yl)-methanone

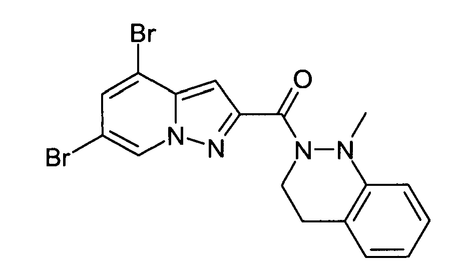

In close analogy to the procedure described in Example 1, 4,6-dibromo-pyrazolo[1,5-a]pyridine-2-carboxylic acid is reacted with 1-methyl-1,2,3,4-tetrahydro-cinnoline to provide the title compound in good to moderate yield.
LC/MS: m/z = 451 (MH⁺)

### Example 760

### (4,6-Dibromo-pyrazolo[1,5-a]pyridin-2-yl)-(5,6-dihydro-8H-imidazo[1,2-a]pyrazin-7-yl)-methanone

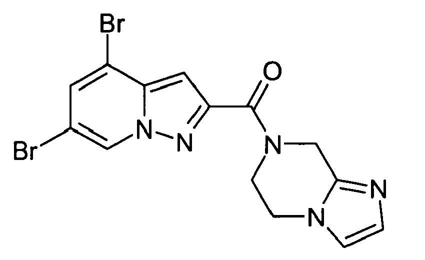

In close analogy to the procedure described in Example 1, 4,6-dibromo-pyrazolo[1,5-a]pyridine-2-carboxylic acid is reacted with 5,6,7,8-tetrahydro-imidazo[1,2-a]pyrazine to provide the title compound in good to moderate yield.
LC/MS: m/z = 426 (MH⁺)

### Example 761

### (4,6-Dibromo-pyrazolo[1,5-a]pyridin-2-yl)-(5,6-dihydro-8H-[1,2,4]triazolo[4,3-a]pyrazin-7-yl)-methanone

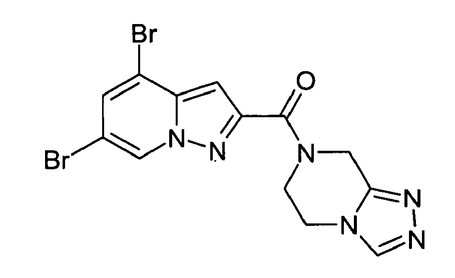

In close analogy to the procedure described in Example 1, 4,6-dibromo-pyrazolo[1,5-a]pyridine-2-carboxylic acid is reacted with 5,6,7,8-tetrahydro-[1,2,4]triazolo[4,3-a]pyrazine to provide the title compound in good to moderate yield.
LC/MS: m/z = 427 (MH⁺)

### Example 762

### (4,6-Dibromo-pyrazolo[1,5-a]pyridin-2-yl)-(1-methyl-3,4-dihydro-1H-pyrrolo[1,2-a]pyrazin-2-yl)-methanone

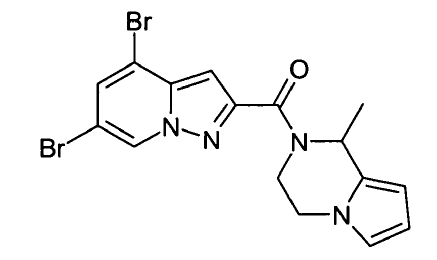

In close analogy to the procedure described in Example 1, 4,6-dibromo-pyrazolo[1,5-a]pyridine-2-carboxylic acid is reacted with 1-methyl-1,2,3,4-tetrahydro-pyrrolo[1,2-a]pyrazine to provide the title compound in good to moderate yield in good to moderate yield.
LC/MS: m/z = 439 (MH⁺)
The stereo-isomers of this compound are separated. The S-configurated compound has a different activity than the R-configurated compound.

### Example 763

### 4,6-Dibromo-pyrazolo[1,5-a]pyridine-2-carboxylic acid methyl-(1-phenyl-ethyl)-amide

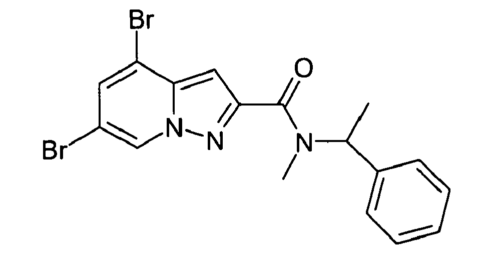

In close analogy to the procedure described in Example 1, 4,6-dibromo-pyrazolo[1,5-a]pyridine-2-carboxylic acid is reacted with methyl-(1-phenyl-ethyl)-amine to provide the title compound in good to moderate yield.
LC/MS: m/z = 438 (MH⁺)
The stereo-isomers of this compound are separated. The S-configurated compound has a different activity than the R-configurated compound.

### Example 764

### 4,6-Dibromo-pyrazolo[1,5-a]pyridine-2-carboxylic acid benzyl-methyl-amide

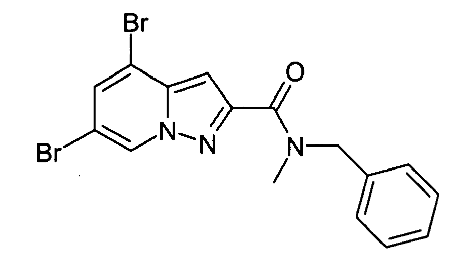

In close analogy to the procedure described in Example 1, 4,6-dibromo-pyrazolo[1,5-a]pyridine-2-carboxylic acid is reacted with benzyl methyl amine to provide the title compound in good to moderate yield.
LC/MS: m/z = 424 (MH⁺)

### Example 765

### 4,6-Dibromo-pyrazolo[1,5-a]pyridine-2-carboxylic acid (2-methyl-2-phenyl-propyl)-amide

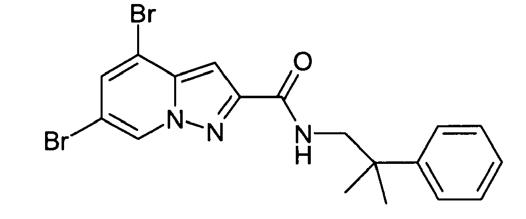

In close analogy to the procedure described in Example 1, 4,6-dibromo-pyrazolo[1,5-a]pyridine-2-carboxylic acid is reacted with 1-methyl-1-phenyl-propylamine to provide the title compound in good to moderate yield.
LC/MS: m/z = 452 (MH⁺)

### Example 766

### 4,6-Dibromo-pyrazolo[1,5-a]pyridine-2-carboxylic acid adamantyl-1 amide

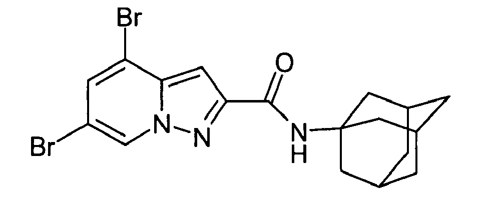

In close analogy to the procedure described in Example 1, 4,6-dibromo-pyrazolo[1,5-a]pyridine-2-carboxylic acid is reacted with adamantyl-1 amine to provide the title compound in moderate yield.
LC/MS: m/z = 454 (MH⁺)

### Example 767

### (4,6-Dibromo-pyrazolo[1,5-a]pyridin-2-yl)-((R)-1-methyl-3,4-dihydro-1H-isoquinolin-2-yl)-methanone

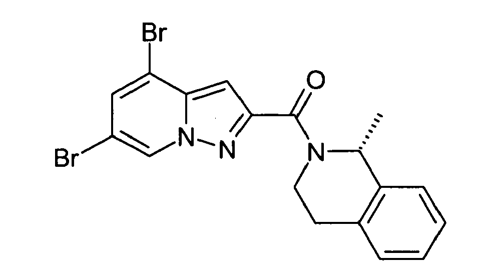

In close analogy to the procedure described in Example 1, 4,6-dibromo-pyrazolo[1,5-a]pyridine-2-carboxylic acid is reacted with (R)-1-methyl-1,2,3,4-tetrahydroisoquinoline to provide the title compound in good to moderate yield.
LC/MS: m/z = 450 (MH⁺)

### Example 768

### (6-Bromo-4-methoxy-pyrazolo[1,5-a]pyridin-2-yl)-(3,4-dihydro-1H-isoquinolin-2-yl)-methanone

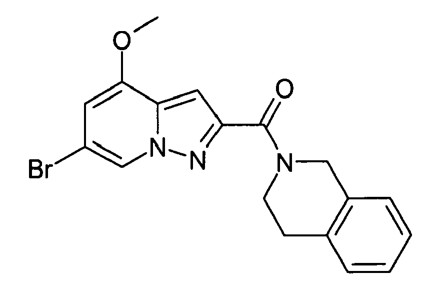

In close analogy to the procedure described in Example 1, 6-bromo-4-methoxy-pyrazolo[1,5-a]pyridine-2-carboxylic acid is reacted with 1,2,3,4-tetrahydroisoquinoline to provide the title compound in good to moderate yield.
LC/MS: m/z = 387 (MH⁺)

### Example 769

### (6-Bromo-4-methoxy-pyrazolo[1,5-a]pyhdin-2-yl)-(1-methyl-3,4-dihydro-1H-isoquinolin-2-yl)-methanone

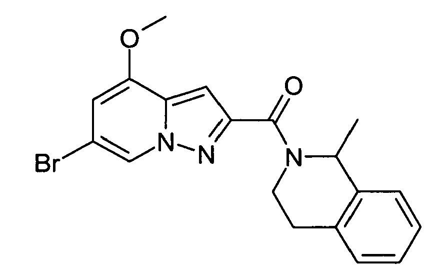

In close analogy to the procedure described in Example 1, 6-bromo-4-methoxy-pyrazolo[1,5-a]pyridine-2-carboxylic acid is reacted with 1-methyl-1,2,3,4-tetrahydroisoquinoline to provide the title compound in good to moderate yield.
LC/MS: m/z = 401 (MH⁺)
The stereo-isomers of this compound are separated. The S-configurated compound has a different activity than the R-configurated compound.

### Example 770

### (6-Bromo-4-methoxy-pyrazolo[1,5-a]pyridin-2-yl)-(7-fluoro-1-methyl-3,4-dihydro-1H-isoquinolin-2-yl)-methanone

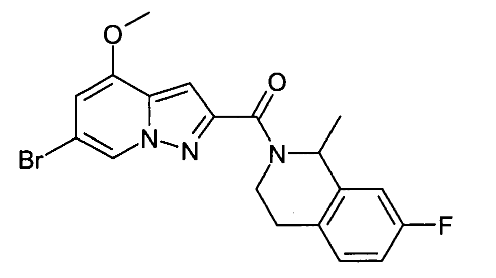

In close analogy to the procedure described in Example 1, 6-bromo-4-methoxy-pyrazolo[1,5-a]pyridine-2-carboxylic acid is reacted with 7-fluoro-1-methyl-1,2,3,4-tetrahydroisoquinoline to provide the title compound in good to moderate yield.
LC/MS: m/z = 419 (MH⁺)
The stereo-isomers of this compound are separated. The S-configurated compound has a different activity than the R-configurated compound.

### Example 771

### (3-Bromo-7,8-dihydro-5H-1,6-naphthyridin-6-yl)-(6-bromo-4-methoxy-pyrazolo[1,5-a]pyridin-2-yl)-methanone

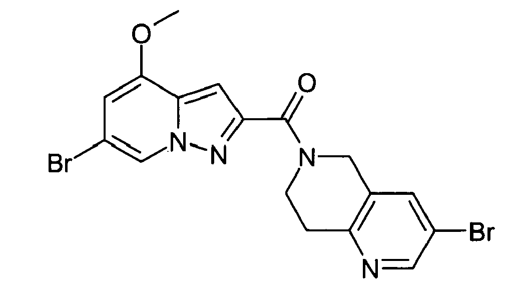

In close analogy to the procedure described in Example 1, 6-bromo-4-methoxy-pyrazolo[1,5-a]pyridine-2-carboxylic acid is reacted with 3-bromo-5,6,7,8-tetrahydro-1,6-naphthyridineto provide the title compound in good to moderate yield.
LC/MS: m/z = 467 (MH⁺)

### Example 772

### (6-Bromo-4-methoxy-pyrazolo[1,5-a]pyridin-2-yl)-(7-pyridin-2-yl-3,4-dihydro-1H-isoquinolin-2-yl)-methanone

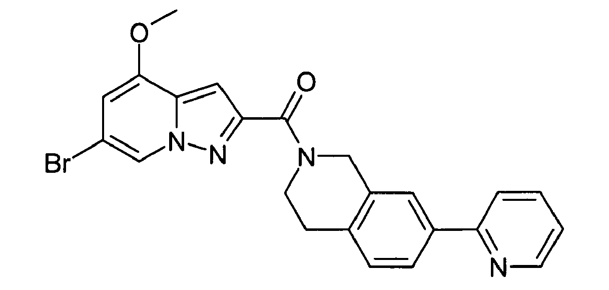

In close analogy to the procedure described in Example 1, 6-bromo-4-methoxy-pyrazolo[1,5-a]pyridine-2-carboxylic acid is reacted with 7-pyridin-2-yl-1,2,3,4-tetrahydro-isoquinolineto provide the title compound in good to moderate yield.
LC/MS: m/z = 464 (MH⁺)

### Example 773

### (6-Bromo-4-methoxy-pyrazolo[1,5-a]pyridin-2-yl)-(4-methyl-6,7-dihydro-4H-thieno[3,2-c]pyridin-5-yl)-methanone

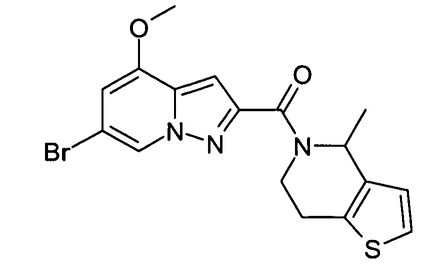

In close analogy to the procedure described in Example 1, 6-bromo-4-methoxy-pyrazolo[1,5-a]pyridine-2-carboxylic acid is reacted with 4-methyl-4,5,6,7-tetrahydro-thieno[3,2-c]pyridine to provide the title compound in good to moderate yield.
LC/MS: m/z = 407 (MH⁺)
The stereo-isomers of this compound are separated. The S-configurated compound has a different activity than the R-configurated compound.

### Example 774

### (6-Bromo-4-methoxy-pyrazolo[1,5-a]pyridin-2-yl)-(4-methyl-6,7-dihydro-4H-furo[3,2-c]pyridin-5-yl)-methanone

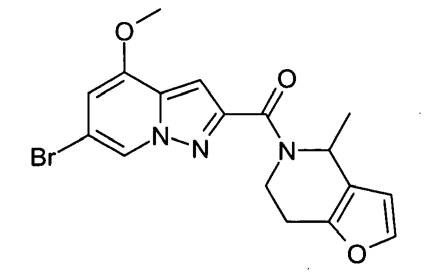

In close analogy to the procedure described in Example 1, 6-bromo-4-methoxy-pyrazolo[1,5-a]pyridine-2-carboxylic acid is reacted with 4-methyl-4,5,6,7-tetrahydrofuro[3,2-c]pyridine to provide the title compound in good to moderate yield.
LC/MS: m/z = 391 (MH⁺)
The stereo-isomers of this compound are separated. The S-configurated compound has a different activity than the R-configurated compound.

### Example 775

### (6-Bromo-4-methoxy-pyrazolo[1,5-a]pyridin-2-yl)-(1-methyl-3,4-dihydro-1H-cinnolin-2-yl)-methanone

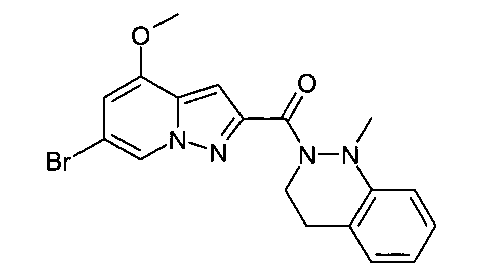

In close analogy to the procedure described in Example 1, 6-bromo-4-methoxy-pyrazolo[1,5-a]pyridine-2-carboxylic acid is reacted with 1-methyl-1,2,3,4-tetrahydro-cinnoline to provide the title compound in good to moderate yield.
LC/MS: m/z = 402 (MH⁺)

### Example 776

### (6-Bromo-4-methoxy-pyrazolo[1,5-a]pyridin-2-yl)-(5,6-dihydro-8H-imidazo[1,2-a]pyrazin-7-yl)-methanone

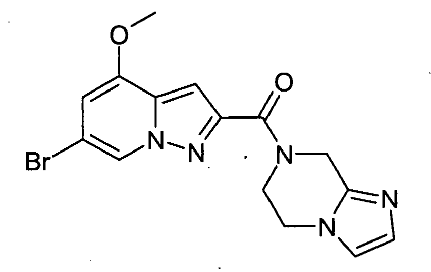

In close analogy to the procedure described in Example 1, 6-bromo-4-methoxy-pyrazolo[1,5-a]pyridine-2-carboxylic acid is reacted with 5,6,7,8-tetrahydro-imidazo[1,2-a]pyrazine to provide the title compound in good to moderate yield.
LC/MS: m/z = 377 (MH⁺)

### Example 777

### (6-Bromo-4-methoxy-pyrazolo[1,5-a]pyridin-2-yl)-(5,6-dihydro-8H-[1,2,4]triazolo[4,3-a]pyrazin-7-yl)-methanone

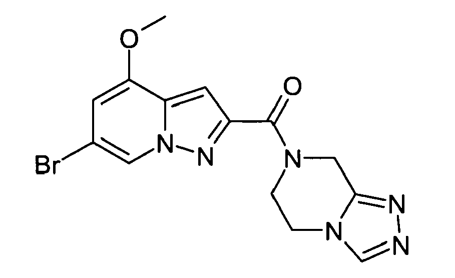

In close analogy to the procedure described in Example 1, 6-bromo-4-methoxy-pyrazolo[1,5-a]pyridine-2-carboxylic acid is reacted with 5,6,7,8-tetrahydro-[1,2,4]triazolo[4,3-a]pyrazine to provide the title compound in good to moderate yield.
LC/MS: m/z = 378 (MH⁺)

### Example 778

### (6-Bromo-4-methoxy-pyrazolo[1,5-a]pyridin-2-yl)-(1-methyl-3,4-dihydro-1H-pyrrolo[1,2-a]pyrazin-2-yl)-methanone

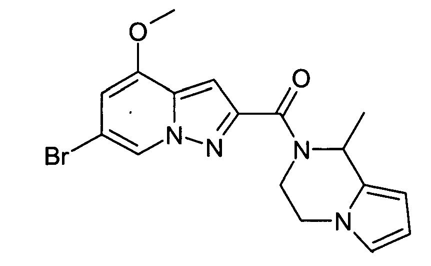

In close analogy to the procedure described in Example 1, 6-bromo-4-methoxy-pyrazolo[1,5-a]pyridine-2-carboxylic acid is reacted with 1-methyl-1,2,3,4-tetrahydro-pyrrolo[1,2-a]pyrazine to provide the title compound in good to moderate yield in good to moderate yield.
LC/MS: m/z = 390 (MH⁺)
The stereo-isomers of this compound are separated. The S-configurated compound has a different activity than the R-configurated compound.

### Example 779

### 6-Bromo-4-methoxy-pyrazolo[1,5-a]pyridine-2-carboxylic acid methyl-(I-phenyl-ethyl)-amide

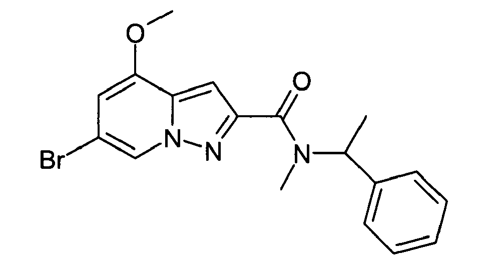

In close analogy to the procedure described in Example 1, 6-bromo-4-methoxy-pyrazolo[1,5-a]pyridine-2-carboxylic acid is reacted with methyl-(1-phenyl-ethyl)-amine to provide the title compound in good to moderate yield.
LC/MS: m/z = 389 (MH⁺)
The stereo-isomers of this compound are separated. The S-configurated compound has a different activity than the R-configurated compound.

### Example 780

### 6-Bromo-4-methoxy-pyrazolo[1,5-a]pyridine-2-carboxylic acid benzyl-methyl-amide

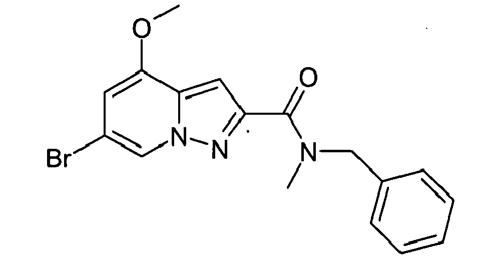

In close analogy to the procedure described in Example 1, 6-bromo-4-methoxy-pyrazolo[1,5-a]pyridine-2-carboxylic acid is reacted with benzyl methyl amine to provide the title compound in good to moderate yield.
LC/MS: m/z = 375 (MH⁺)

### Example 781

### 6-Bromo-4-methoxy-pyrazolo[1,5-a]pyridine-2-carboxylic acid (2-methyl-2-phenylpropyl)-amide

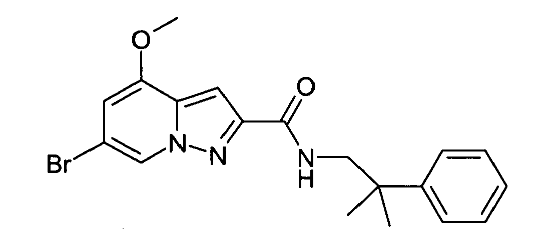

In close analogy to the procedure described in Example 1, 6-bromo-4-methoxy-pyrazolo[1,5-a]pyridine-2-carboxylic acid is reacted with 1-methyl-1-phenyl-propylamine to provide the title compound in good to moderate yield.
LC/MS: m/z = 403 (MH⁺)

### Example 782

### 6-Bromo-4-methoxy-pyrazolo[1,5-a]pyridine-2-carboxylic acid adamantyl-1 amide

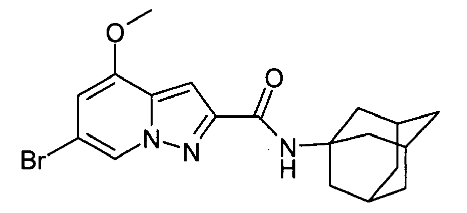

In close analogy to the procedure described in Example 1, 6-bromo-4-methoxy-pyrazolo[1,5-a]pyridine-2-carboxylic acid is reacted with adamantyl-1 amine to provide the title compound in moderate yield.
LC/MS: m/z = 405 (MH⁺)

### Example 783

### (6-Bromo-4-methoxy-pyrazolo[1,5-a]pyridin-2-yl)-((R)-1-methyl-3,4-dihydro-1H-isoquinolin-2-yl)-methanone

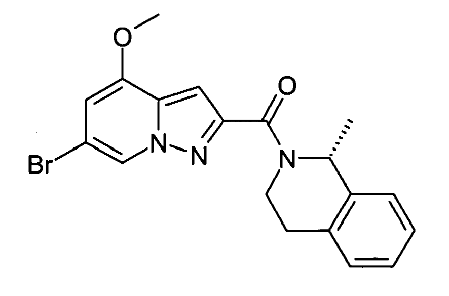

In close analogy to the procedure described in Example 1, 6-bromo-4-methoxy-pyrazolo[1,5-a]pyridine-2-carboxylic acid is reacted with (R)-1-methyl-1,2,3,4-tetrahydroisoquinoline to provide the title compound in good to moderate yield.
LC/MS: m/z = 401 (MH⁺)

### Example 784

### (6-Chloro-4-methoxy-pyrazolo[1,5-a]pyridin-2-yl)-(3,4-dihydro-1H-isoquinolin-2-yl)-methanone

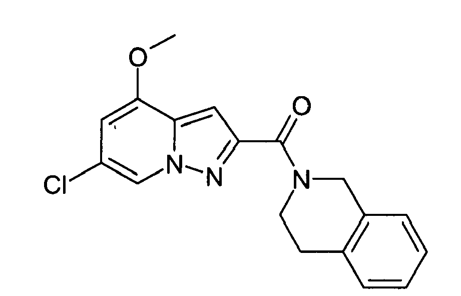

In close analogy to the procedure described in Example 1, 6-chloro-4-methoxy-pyrazolo[1,5-a]pyridine-2-carboxylic acid is reacted with 1,2,3,4-tetrahydroisoquinoline to provide the title compound in good to moderate yield.
LC/MS: m/z = 342 (MH⁺)

### Example 785

### (6-Chloro-4-methoxy-pyrazolo[1,5-a]pyridin-2-yl)-(1-methyl-3,4-dihydro-1H-isoquinolin-2-yl)-methanone

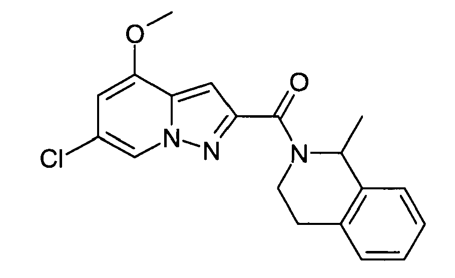

In close analogy to the procedure described in Example 1, 6-chloro-4-methoxy-pyrazolo[1,5-a]pyridine-2-carboxylic acid is reacted with 1-methyl-1,2,3,4-tetrahydroisoquinoline to provide the title compound in good to moderate yield.
LC/MS: m/z = 356 (MH⁺)

The stereo-isomers of this compound are separated. The S-configurated compound has a different activity than the R-configurated compound.

### Example 786

### (6-Chloro-4-methoxy-pyrazolo[1,5-a]pyridin-2-yl)-(7-fluoro-1-methyl-3,4-dihydro-1H-isoquinolin-2-yl)-methanone

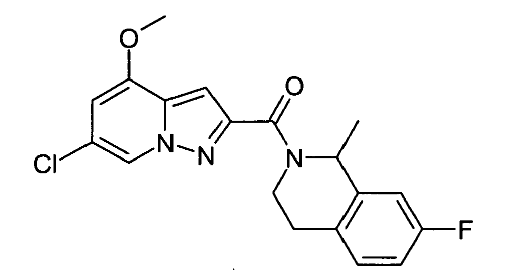

In close analogy to the procedure described in Example 1, 6-chloro-4-methoxy-pyrazolo[1,5-a]pyridine-2-carboxylic acid is reacted with 7-fluoro-1-methyl-1,2,3,4-tetrahydroisoquinoline to provide the title compound in good to moderate yield.
LC/MS: m/z = 374 (MH⁺)
The stereo-isomers of this compound are separated. The S-configurated compound has a different activity than the R-configurated compound.

### Example 787

### (3-Bromo-7,8-dihydro-5H-1,6-naphthyridin-6-yl)-(6-chloro-4-methoxy-pyrazolo[1,5-a]pyridin-2-yl)-methanone

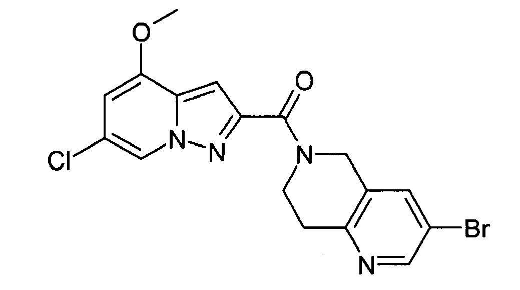

In close analogy to the procedure described in Example 1, 6-chloro-4-methoxy-pyrazolo[1,5-a]pyridine-2-carboxylic acid is reacted with 3-bromo-5,6,7,8-tetrahydro-1,6-naphthyridineto provide the title compound in good to moderate yield.
LC/MS: m/z = 422 (MH⁺)

### Example 788

### (6-Chloro-4-methoxy-pyrazolo[1,5-a]pyridin-2-yl)-(7-pyridin-2-yl-3,4-dihydro-1H-isoquinolin-2-yl)-methanone

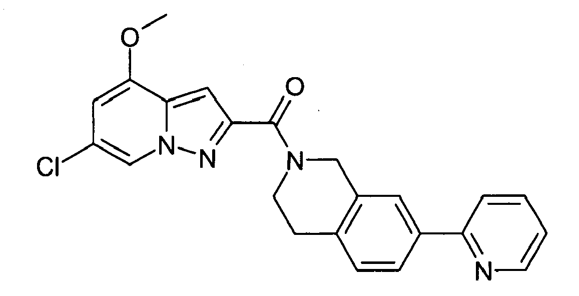

In close analogy to the procedure described in Example 1, 6-chloro-4-methoxy-pyrazolo[1,5-a]pyridine-2-carboxylic acid is reacted with 7-pyridin-2-yl-1,2,3,4-tetrahydro-isoquinolineto provide the title compound in good to moderate yield.
LC/MS: m/z = 419 (MH⁺)

### Example 789

### (6-Chloro-4-methoxy-pyrazolo[1,5-a]pyridin-2-yl)-(4-methyl-6,7-dihydro-4H-thieno[3,2-c]pyridin-5-yl)-methanone

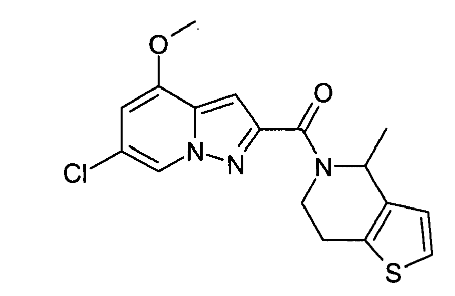

In close analogy to the procedure described in Example 1, 6-chloro-4-methoxy-pyrazolo[1,5-a]pyridine-2-carboxylic acid is reacted with 4-methyl-4,5,6,7-tetrahydro-thieno[3,2-c]pyridine to provide the title compound in good to moderate yield.
LC/MS: m/z = 362 (MH⁺)
The stereo-isomers of this compound are separated. The S-configurated compound has a different activity than the R-configurated compound.

### Example 790

### (6-Chloro-4-methoxy-pyrazolo[1,5-a]pyridin-2-yl)-(4-methyl-6,7-dihydro-4H-furo[3,2-c]pyridin-5-yl)-methanone

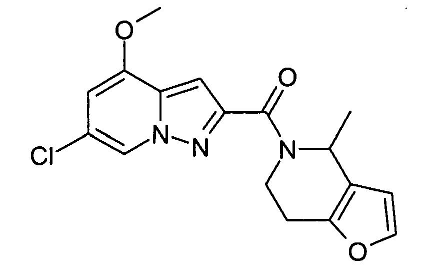

In close analogy to the procedure described in Example 1, 6-chloro-4-methoxy-pyrazolo[1,5-a]pyridine-2-carboxylic acid is reacted with 4-methyl-4,5,6,7-tetrahydro-furo[3,2-c]pyridine to provide the title compound in good to moderate yield.
LC/MS: m/z = 346 (MH⁺)
The stereo-isomers of this compound are separated. The S-configurated compound has a different activity than the R-configurated compound.

### Example 791

### (6-Chloro-4-methoxy-pyrazolo[1,5-a]pyridin-2-yl)-(1-methyl-3,4-dihydro-1H-cinnolin-2-yl)-methanone

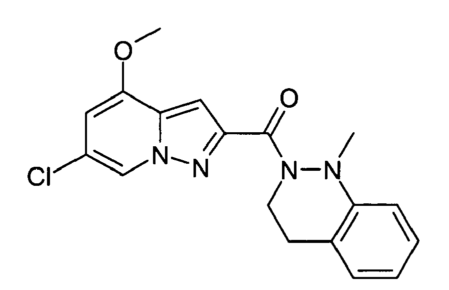

In close analogy to the procedure described in Example 1, 6-chloro-4-methoxy-pyrazolo[1,5-a]pyridine-2-carboxylic acid is reacted with 1-methyl-1,2,3,4-tetrahydro-cinnoline to provide the title compound in good to moderate yield.
LC/MS: m/z = 357 (MH⁺)

### Example 792

### (6-Chloro-4-methoxy-pyrazolo[1,5-a]pyridin-2-yl)-(5,6-dihydro-8H-imidazo[1,2-a]pyrazin-7-yl)-methanone

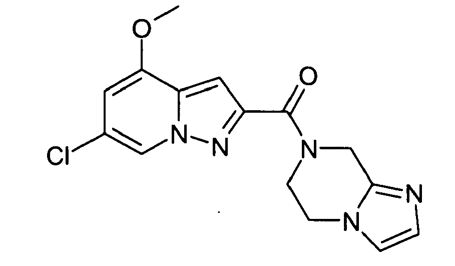

In close analogy to the procedure described in Example 1, 6-chloro-4-methoxy-pyrazolo[1,5-a]pyridine-2-carboxylic acid is reacted with 5,6,7,8-tetrahydro-imidazo[1,2-a]pyrazine to provide the title compound in good to moderate yield.
LC/MS: m/z = 332 (MH⁺)

### Example 793

### (6-Chloro-4-methoxy-pyrazolo[1,5-a]pyridin-2-yl)-(5,6-dihydro-8H-[1,2,4]triazolo[4,3-a]pyrazin-7-yl)-methanone

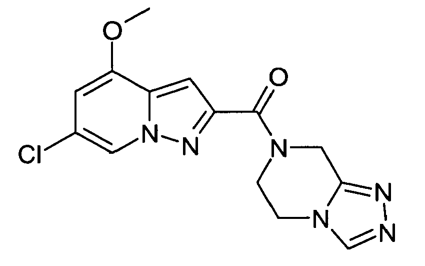

In close analogy to the procedure described in Example 1, 6-chloro-4-methoxy-pyrazolo[1,5-a]pyridine-2-carboxylic acid is reacted with 5,6,7,8-tetrahydro-[1,2,4]triazolo[4,3-a]pyrazine to provide the title compound in good to moderate yield.
LC/MS: m/z = 333 (MH⁺)

### Example 794

### (6-Chloro-4-methoxy-pyrazolo[1,5-a]pyridin-2-yl)-(1-methyl-3,4-dihydro-1H-pyrrolo[1,2-a]pyrazin-2-yl)-methanone

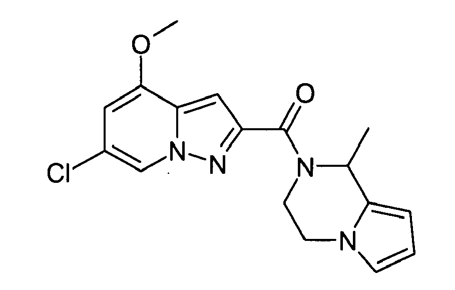

In close analogy to the procedure described in Example 1, 6-chloro-4-methoxy-pyrazolo[1,5-a]pyridine-2-carboxylic acid is reacted with 1-methyl-1,2,3,4-tetrahydro-pyrrolo[1,2-a]pyrazine to provide the title compound in good to moderate yield in good to moderate yield.
LC/MS: m/z = 345 (MH⁺)
The stereo-isomers of this compound are separated. The S-configurated compound has a different activity than the R-configurated compound.

### Example 795

### 6-Chloro-4-methoxy-pyrazolo[1,5-a]pyridine-2-carboxylic acid methyl-(1-phenyl-ethyl)-amide

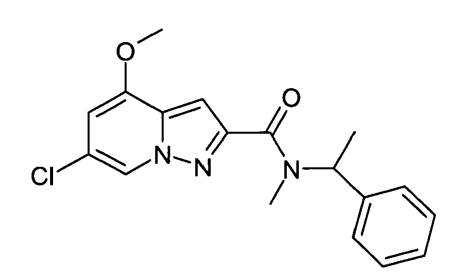

In close analogy to the procedure described in Example 1, 6-chloro-4-methoxy-pyrazolo[1,5-a]pyridine-2-carboxylic acid is reacted with methyl-(1-phenyl-ethyl)-amine to provide the title compound in good to moderate yield.
LC/MS: m/z = 344 (MH⁺)
The stereo-isomers of this compound are separated. The S-configurated compound has a different activity than the R-configurated compound.

### Example 796

### 6-Chloro-4-methoxy-pyrazolo[1,5-a]pyridine-2-carboxylic acid benzyl-methyl-amide

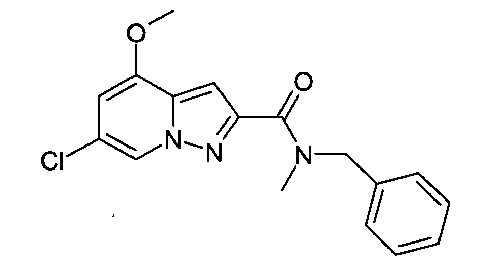

In close analogy to the procedure described in Example 1, 6-chloro-4-methoxy-pyrazolo[1,5-a]pyridine-2-carboxylic acid is reacted with benzyl methyl amine to provide the title compound in good to moderate yield.
LC/MS: m/z = 330 (MH⁺)

### Example 797

### 6-Chloro-4-methoxy-pyrazolo[1,5-a]pyridine-2-carboxylic acid (2-methyl-2-phenylpropyl)-amide

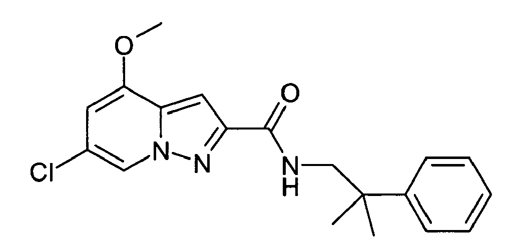

In close analogy to the procedure described in Example 1, 6-chloro-4-methoxy-pyrazolo[1,5-a]pyridine-2-carboxylic acid is reacted with 1-methyl-1-phenyl-propylamine to provide the title compound in good to moderate yield.
LC/MS: m/z = 358 (MH⁺)

### Example 798

### 6-Chloro-4-methoxy-pyrazolo[1,5-a]pyridine-2-carboxylic acid adamantyl-1 amide

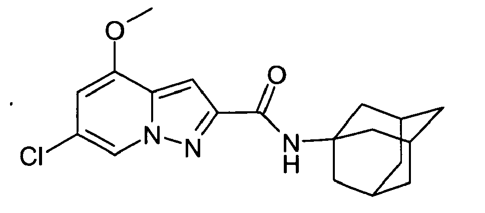

In close analogy to the procedure described in Example 1, 6-chloro-4-methoxy-pyrazolo[1,5-a]pyridine-2-carboxylic acid is reacted with adamantyl-1 amine to provide the title compound in moderate yield.
LC/MS: m/z = 360 (MH⁺)

### Example 799

### (6-Chloro-4-methoxy-pyrazolo[1,5-a]pyridin-2-yl)-((R)-1-methyl-3,4-dihydro-1H-isoquinolin-2-yl)-methanone

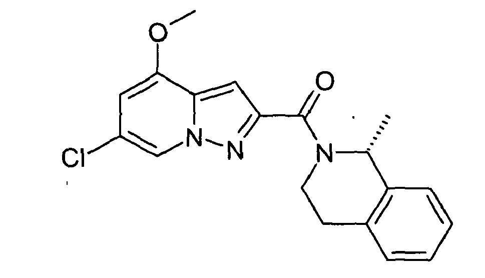

In close analogy to the procedure described in Example 1, 6-chloro-4-methoxy-pyrazolo[1,5-a]pyridine-2-carboxylic acid is reacted with (R)-1-methyl-1,2,3,4-tetrahydroisoquinoline to provide the title compound in good to moderate yield.
LC/MS: m/z = 356 (MH⁺)

### Example 800

### (6-Chloro-4-nitro-pyrazolo[1,5-a]pyridin-2-yl)-(3,4-dihydro-1H-isoquinolin-2-yl)-methanone

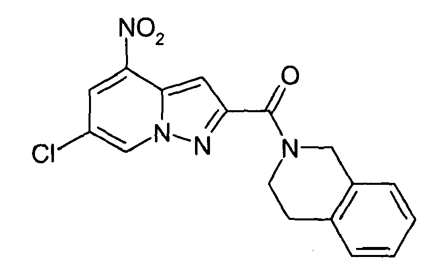

In close analogy to the procedure described in Example 1, 6-chloro-4-nitro-pyrazolo[1,5-a]pyridine-2-carboxylic acid is reacted with 1,2,3,4-tetrahydroisoquinoline to provide the title compound in good to moderate yield.
LC/MS: m/z = 356 (MH⁺)

### Example 801

### (6-Chloro-4-nitro-pyrazolo[1,5-a]pyridin-2-yl)-(1-methyl-3,4-dihydro-1H-isoquinolin-2-yl)-methanone

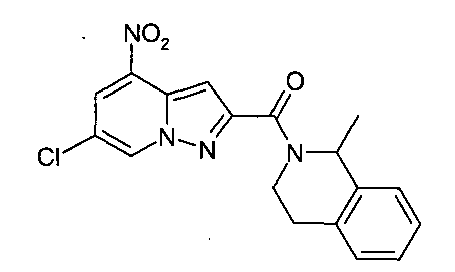

In close analogy to the procedure described in Example 1, 6-chloro-4-nitro-pyrazolo[1,5-a]pyridine-2-carboxylic acid is reacted with 1-methyl-1,2,3,4-tetrahydroisoquinoline to provide the title compound in good to moderate yield.
LC/MS: m/z = 371 (MH⁺)
The stereo-isomers of this compound are separated. The S-configurated compound has a different activity than the R-configurated compound.

### Example 802

### (6-Chloro-4-nitro-pyrazolo[1,5-a]pyridin-2-yl)-(7-fluoro-1-methyl-3,4-dihydro-1H-isoquinolin-2-yl)-methanone

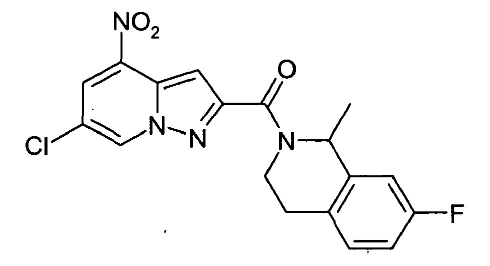

In close analogy to the procedure described in Example 1, 6-chloro-4-nitro-pyrazolo[1,5-a]pyridine-2-carboxylic acid is reacted with 7-fluoro-1-methyl-1,2,3,4-tetrahydroisoquinoline to provide the title compound in good to moderate yield.
LC/MS: m/z = 389 (MH⁺)
The stereo-isomers of this compound are separated. The S-configurated compound has a different activity than the R-configurated compound.

### Example 803

### (3-Bromo-7,8-dihydro-5H-1,6-naphthyridin-6-yl)-(6-chloro-4-nitro-pyrazolo[1,5-a]pyridin-2-yl)-methanone

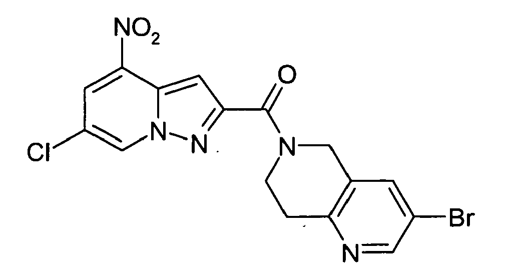

In close analogy to the procedure described in Example 1, 6-chloro-4-nitro-pyrazolo[1,5-a]pyridine-2-carboxylic acid is reacted with 3-bromo-5,6,7,8-tetrahydro-1,6-naphthyridineto provide the title compound in good to moderate yield.
LC/MS: m/z = 437 (MH⁺)

### Example 804

### (6-Chloro-4-nitro-pyrazolo[1,5-a]pyridin-2-yl)-(7-pyridin-2-yl-3,4-dihydro-1H-isoquinolin-2-yl)-methanone

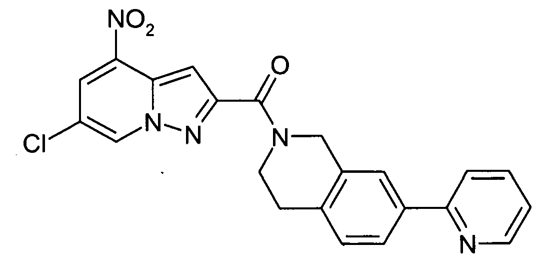

In close analogy to the procedure described in Example 1, 6-chloro-4-nitro-pyrazolo[1,5-a]pyridine-2-carboxylic acid is reacted with 7-pyridin-2-yl-1,2,3,4-tetrahydro-isoquinolineto provide the title compound in good to moderate yield.
LC/MS: m/z = 434 (MH⁺)

### Example 805

### (6-Chloro-4-nitro-pyrazolo[1,5-a]pyridin-2-yl)-(4-methyl-6,7-dihydro-4H-thieno[3,2-c]pyridin-5-yl)-methanone

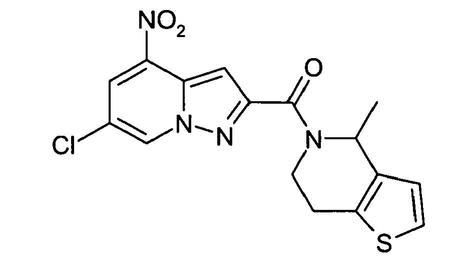

In close analogy to the procedure described in Example 1, 6-chloro-4-nitro-pyrazolo[1,5-a]pyridine-2-carboxylic acid is reacted with 4-methyl-4,5,6,7-tetrahydro-thieno[3,2-c]pyridine to provide the title compound in good to moderate yield.
LC/MS: m/z = 377 (MH⁺)
The stereo-isomers of this compound are separated. The S-configurated compound has a different activity than the R-configurated compound.

### Example 806

### (6-Chloro-4-nitro-pyrazolo[1,5-a]pyridin-2-yl)-(4-methyl-6,7-dihydro-4H-furo[3,2-c]pyridin-5-yl)-methanone

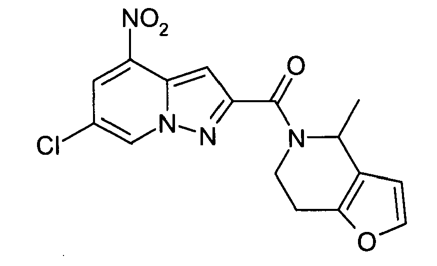

In close analogy to the procedure described in Example 1, 6-chloro-4-nitro-pyrazolo[1,5-a]pyridine-2-carboxylic acid is reacted with 4-methyl-4,5,6,7-tetrahydro-furo[3,2-c]pyridine to provide the title compound in good to moderate yield.
LC/MS: m/z = 361 (MH⁺)
The stereo-isomers of this compound are separated. The S-configurated compound has a different activity than the R-configurated compound.

### Example 807

### (6-Chloro-4-nitro-pyrazolo[1,5-a]pyridin-2-yl)-(1-methyl-3,4-dihydro-1H-cinnolin-2-yl)-methanone

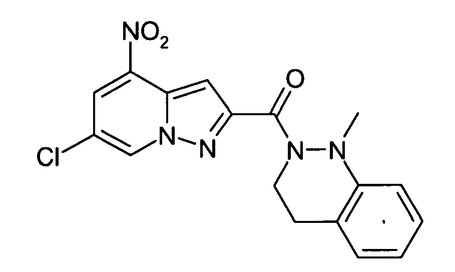

In close analogy to the procedure described in Example 1, 6-chloro-4-nitro-pyrazolo[1,5-a]pyridine-2-carboxylic acid is reacted with 1-methyl-1,2,3,4-tetrahydro-cinnoline to provide the title compound in good to moderate yield.
LC/MS: m/z = 372 (MH⁺)

### Example 808

### (6-Chloro-4-nitro-pyrazolo[1,5-a]pyridin-2-yl)-(5,6-dihydro-8H-imidazo[1,2-a]pyrazin-7-yl)-methanone

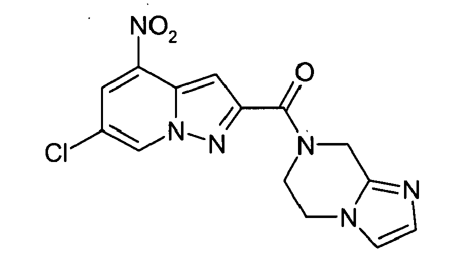

In close analogy to the procedure described in Example 1, 6-chloro-4-nitro-pyrazolo[1,5-a]pyridine-2-carboxylic acid is reacted with 5,6,7,8-tetrahydro-imidazo[1,2-a]pyrazine to provide the title compound in good to moderate yield.
LC/MS: m/z = 347 (MH⁺)

### Example 809

### (6-Chloro-4-nitro-pyrazolo[1,5-a]pyridin-2-yl)-(5,6-dihydro-8H-[1,2,4]triazolo[4,3-a]pyrazin-7-yl)-methanone

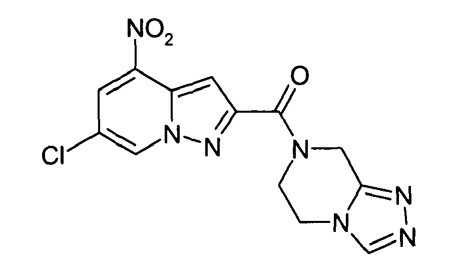

In close analogy to the procedure described in Example 1, 6-chloro-4-nitro-pyrazolo[1,5-a]pyridine-2-carboxylic acid is reacted with 5,6,7,8-tetrahydro-[1,2,4]triazolo[4,3-a]pyrazine to provide the title compound in good to moderate yield.
LC/MS: m/z = 348 (MH⁺)

### Example 810

### (6-Chloro-4-nitro-pyrazolo[1,5-a]pyridin-2-yl)-(1-methyl-3,4-dihydro-1H-pyrrolo[1,2-a]pyrazin-2-yl)-methanone

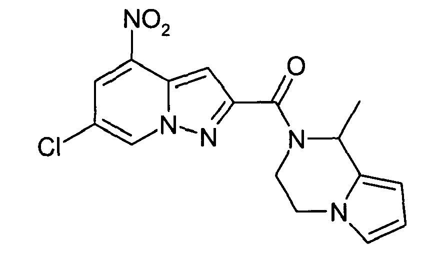

In close analogy to the procedure described in Example 1, 6-chloro-4-nitro-pyrazolo[1,5-a]pyridine-2-carboxylic acid is reacted with 1-methyl-1,2,3,4-tetrahydro-pyrrolo[1,2-a]pyrazine to provide the title compound in good to moderate yield in good to moderate yield.
LC/MS: m/z = 360 (MH⁺)
The stereo-isomers of this compound are separated. The S-configurated compound has a different activity than the R-configurated compound.

### Example 811

### 6-Chloro-4-nitro-pyrazolo[1,5-a]pyridine-2-carboxylic acid methyl-(1-phenyl-ethyl)-amide

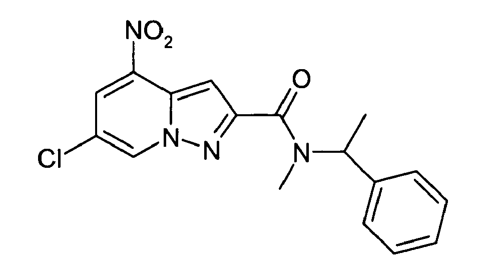

In close analogy to the procedure described in Example 1, 6-chloro-4-nitro-pyrazolo[1,5-a]pyridine-2-carboxylic acid is reacted with methyl-(1-phenyl-ethyl)-amine to provide the title compound in good to moderate yield.
LC/MS: m/z = 359 (MH⁺)
The stereo-isomers of this compound are separated. The S-configurated compound has a different activity than the R-configurated compound.

### Example 812

### 6-Chloro-4-nitro-pyrazolo[1,5-a]pyridine-2-carboxylic acid benzyl-methyl-amide

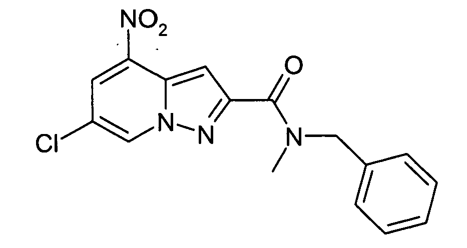

In close analogy to the procedure described in Example 1, 6-chloro-4-nitro-pyrazolo[1,5-a]pyridine-2-carboxylic acid is reacted with benzyl methyl amine to provide the title compound in good to moderate yield.
LC/MS: m/z = 345 (MH⁺)

### Example 813

### 6-Chloro-4-nitro-pyrazolo[1,5-a]pyridine-2-carboxylic acid (2-methyl-2-phenyl-propyl)-amide

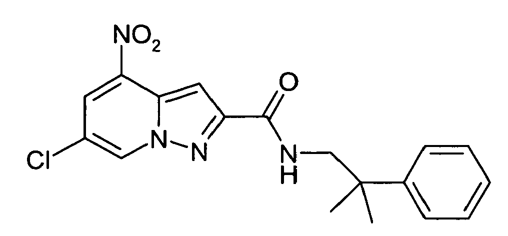

In close analogy to the procedure described in Example 1, 6-chloro-4-nitro-pyrazolo[1,5-a]pyridine-2-carboxylic acid is reacted with 1-methyl-1-phenyl-propylamine to provide the title compound in good to moderate yield.
LC/MS: m/z = 373 (MH⁺)

### Example 814

### 6-Chloro-4-nitro-pyrazolo[1,5-a]pyridine-2-carboxylic acid adamantyl-1 amide

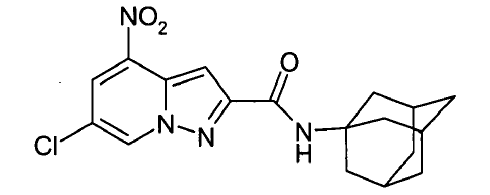

In close analogy to the procedure described in Example 1, 6-chloro-4-nitro-pyrazolo[1,5-a]pyridine-2-carboxylic acid is reacted with adamantyl-1 amine to provide the title compound in moderate yield.
LC/MS: m/z = 375 (MH⁺)

### Example 815

### (6-Chloro-4-nitro-pyrazolo[1,5-a]pyridin-2-yl)-((R)-1-methyl-3,4-dihydro-1H-isoquinolin-2-yl)-methanone

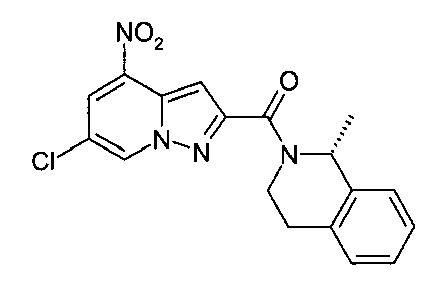

In close analogy to the procedure described in Example 1, 6-chloro-4-nitro-pyrazolo[1,5-a]pyridine-2-carboxylic acid is reacted with (R)-1-methyl-1,2,3,4-tetrahydroisoquinoline to provide the title compound in good to moderate yield.
LC/MS: m/z = 371 (MH⁺)

### Example 816

### (6-Bromo-4-nitro-pyrazolo[1,5-a]pyridin-2-yl)-(3,4-dihydro-1H-isoquinolin-2-yl)-methanone

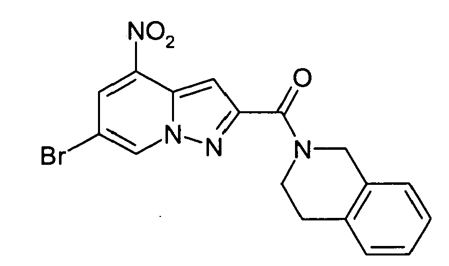

In close analogy to the procedure described in Example 1, 6-bromo-4-nitro-pyrazolo[1,5-a]pyridine-2-carboxylic acid is reacted with 1,2,3,4-tetrahydroisoquinoline to provide the title compound in good to moderate yield.
LC/MS: m/z = 401 (MH⁺)

### Example 817

### (6-Bromo-4-nitro-pyrazolo[1,5-a]pyridin-2-yl)-(1-methyl-3,4-dihydro-1H-isoquinolin-2-yl)-methanone

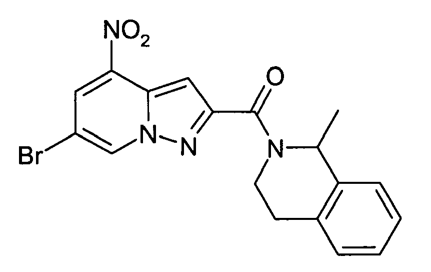

In close analogy to the procedure described in Example 1, 6-bromo-4-nitro-pyrazolo[1,5-a]pyridine-2-carboxylic acid is reacted with 1-methyl-1,2,3,4-tetrahydroisoquinoline to provide the title compound in good to moderate yield.
LC/MS: m/z = 426 (MH⁺)

The stereo-isomers of this compound are separated. The S-configurated compound has a different activity than the R-configurated compound.

### Example 818

### (6-bromo-4-nitro-pyrazolo[1,5-a]pyridin-2-yl)-(7-fluoro-1-methyl-3,4-dihydro-1H-isoquinolin-2-yl)-methanone

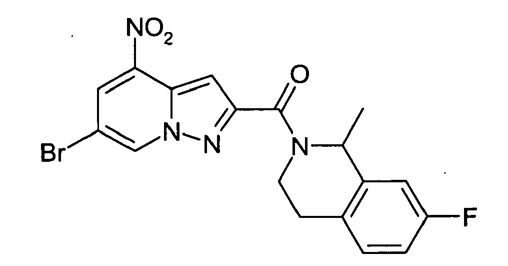

In close analogy to the procedure described in Example 1, 6-bromo-4-nitro-pyrazolo[1,5-a]pyridine-2-carboxylic acid is reacted with 7-fluoro-1-methyl-1,2,3,4-tetrahydroisoquinoline to provide the title compound in good to moderate yield.
LC/MS: m/z = 434 (MH⁺)
The stereo-isomers of this compound are separated. The S-configurated compound has a different activity than the R-configurated compound.

### Example 819

### (3-Bromo-7,8-dihydro-5H-1,6-naphthyndin-6-yl)-(6-bromo-4-nitro-pyrazolo[1,5-a]pyridin-2-yl)-methanone

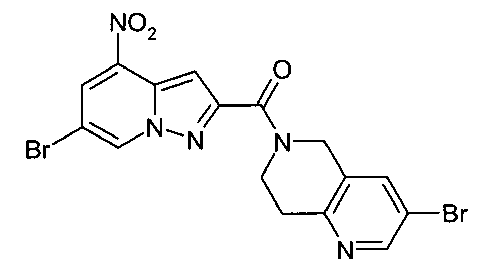

In close analogy to the procedure described in Example 1, 6-bromo-4-nitro-pyrazolo[1,5-a]pyridine-2-carboxylic acid is reacted with 3-bromo-5,6,7,8-tetrahydro-1,6-naphthyridineto provide the title compound in good to moderate yield.
LC/MS: m/z = 482 (MH⁺)

### Example 820

### (6-Bromo-4-nitro-pyrazolo[1,5-a]pyridin-2-yl)-(7-pyridin-2-yl-3,4-dihydro-1H-isoquinolin-2-yl)-methanone

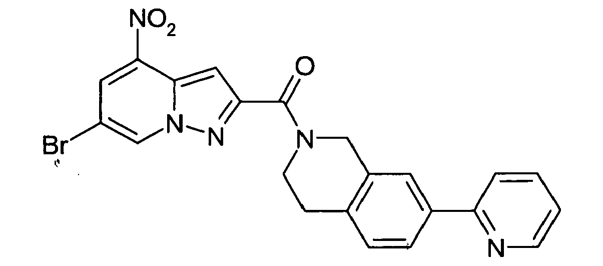

In close analogy to the procedure described in Example 1, 6-bromo-4-nitro-pyrazolo[1,5-a]pyridine-2-carboxylic acid is reacted with 7-pyridin-2-yl-1,2,3,4-tetrahydro-isoquinolineto provide the title compound in good to moderate yield.
LC/MS: m/z = 479 (MH⁺)

### Example 821

### (6-bromo-4-nitro-pyrazolo[1,5-a]pyridin-2-yl)-(4-methyl-6,7-dihydro-4H-thieno[3,2-c]pyridin-5-yl)-methanone

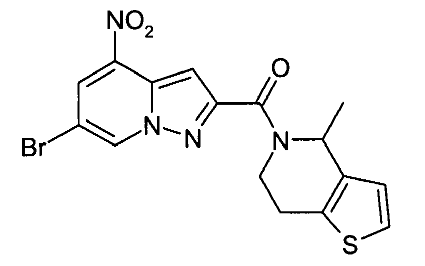

In close analogy to the procedure described in Example 1, 6-bromo-4-nitro-pyrazolo[1,5-a]pyridine-2-carboxylic acid is reacted with 4-methyl-4,5,6,7-tetrahydro-thieno[3,2-c]pyridine to provide the title compound in good to moderate yield.
LC/MS: m/z = 422 (MH⁺)
The stereo-isomers of this compound are separated. The S-configurated compound has a different activity than the R-configurated compound.

### Example 822

### (6-Bromo-4-nitro-pyrazolo[1,5-a]pyridin-2-yl)-(4-methyl-6,7-dihydro-4H-furo[3,2-c]pyridin-5-yl)-methanone

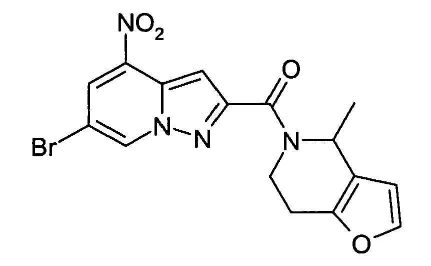

In close analogy to the procedure described in Example 1, 6-bromo-4-nitro-pyrazolo[1,5-a]pyridine-2-carboxylic acid is reacted with 4-methyl-4,5,6,7-tetrahydro-furo[3,2-c]pyridine to provide the title compound in good to moderate yield.
LC/MS: m/z = 406 (MH⁺)
The stereo-isomers of this compound are separated. The S-configurated compound has a different activity than the R-configurated compound.

### Example 823

### (6-Bromo-4-nitro-pyrazolo[1,5-a]pyridin-2-yl)-(1-methyl-3,4-dihydro-1H-cinnolin-2-yl)-methanone

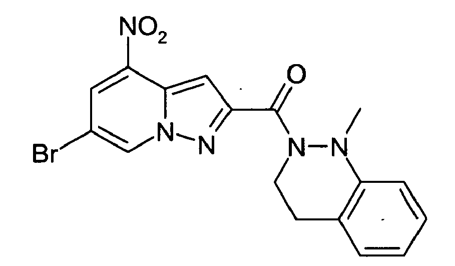

In close analogy to the procedure described in Example 1, 6-bromo-4-nitro-pyrazolo[1,5-a]pyridine-2-carboxylic acid is reacted with 1-methyl-1,2,3,4-tetrahydro-cinnoline to provide the title compound in good to moderate yield.
LC/MS: m/z = 417 (MH⁺)

### Example 824

### (6-Bromo-4-nitro-pyrazolo[1,5-a]pyridin-2-yl)-(5,6-dihydro-8H-imidazo[1,2-a]pyrazin-7-yl)-methanone

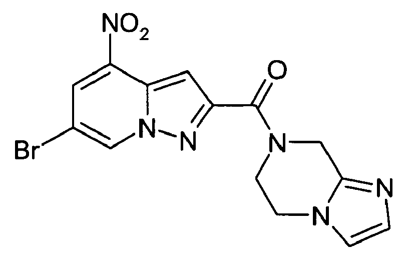

In close analogy to the procedure described in Example 1, 6-bromo-4-nitro-pyrazolo[1,5-a]pyridine-2-carboxylic acid is reacted with 5,6,7,8-tetrahydro-imidazo[1,2-a]pyrazine to provide the title compound in good to moderate yield.
LC/MS: m/z = 392 (MH⁺)

### Example 825

### (6-Bromo-4-nitro-pyrazolo[1,5-a]pyridin-2-yl)-(5,6-dihydro-8H-[1,2,4]triazolo[4,3-a]pyrazin-7-yl)-methanone

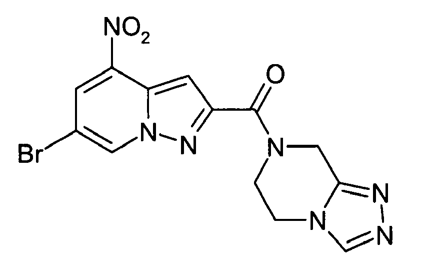

In close analogy to the procedure described in Example 1, 6-bromo-4-nitro-pyrazolo[1,5-a]pyridine-2-carboxylic acid is reacted with 5,6,7,8-tetrahydro-[1,2,4]triazolo[4,3-a]pyrazine to provide the title compound in good to moderate yield.
LC/MS: m/z = 393 (MH⁺)

### Example 826

### (6-Bromo-4-nitro-pyrazolo[1,5-a]pyridin-2-yl)-(1-methyl-3,4-dihydro-1H-pyrrolo[1,2-a]pyrazin-2-yl)-methanone

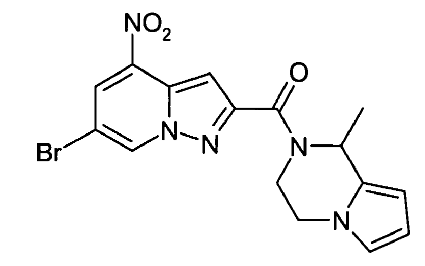

In close analogy to the procedure described in Example 1, 6-bromo-4-nitro-pyrazolo[1,5-a]pyridine-2-carboxylic acid is reacted with 1-methyl-1,2,3,4-tetrahydro-pyrrolo[1,2-a]pyrazine to provide the title compound in good to moderate yield in good to moderate yield.
LC/MS: m/z = 405 (MH⁺)
The stereo-isomers of this compound are separated. The S-configurated compound has a different activity than the R-configurated compound.

### Example 827

### 6-Bromo-4-nitro-pyrazolo[1,5-a]pyridine-2-carboxylic acid methyl-(1-phenyl-ethyl)-amide

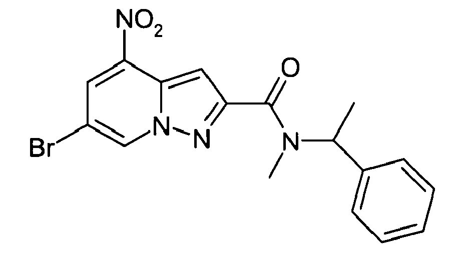

In close analogy to the procedure described in Example 1, 6-bromo-4-nitro-pyrazolo[1,5-a]pyridine-2-carboxylic acid is reacted with methyl-(1-phenyl-ethyl)-amine to provide the title compound in good to moderate yield.
LC/MS: m/z = 404 (MH⁺)
The stereo-isomers of this compound are separated. The S-configurated compound has a different activity than the R-configurated compound.

### Example 828

### 6-Bromo-4-nitro-pyrazolo[1,5-a]pyridine-2-carboxylic acid benzyl-methyl-amide

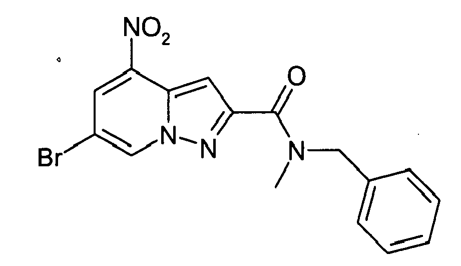

In close analogy to the procedure described in Example 1, 6-bromo-4-nitro-pyrazolo[1,5-a]pyridine-2-carboxylic acid is reacted with benzyl methyl amine to provide the title compound in good to moderate yield.
LC/MS: m/z = 390 (MH⁺)

### Example 829

### 6-Bromo-4-nitro-pyrazolo[1,5-a]pyridine-2-carboxylic acid (2-methyl-2-phenyl-propyl)-amide

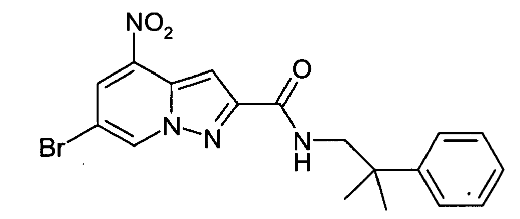

In close analogy to the procedure described in Example 1, 6-bromo-4-nitro-pyrazolo[1,5-a]pyridine-2-carboxylic acid is reacted with 1-methyl-1-phenyl-propylamine to provide the title compound in good to moderate yield.
LC/MS: m/z = 418 (MH⁺)

### Example 830

### 6-Bromo-4-nitro-pyrazolo[1,5-a]pyridine-2-carboxylic acid adamantyl-1 amide

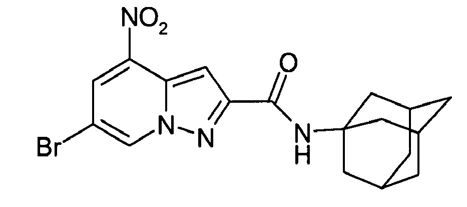

In close analogy to the procedure described in Example 1, 6-bromo-4-nitro-pyrazolo[1,5-a]pyridine-2-carboxylic acid is reacted with adamantyl-1 amine to provide the title compound in moderate yield.
LC/MS: m/z = 420 (MH⁺)

### Example 831

### (6-Bromo-4-nitro-pyrazolo[1,5-a]pyridin-2-yl)-((R)-1-methyl-3,4-dihydro-1H-isoquinolin-2-yl)-methanone

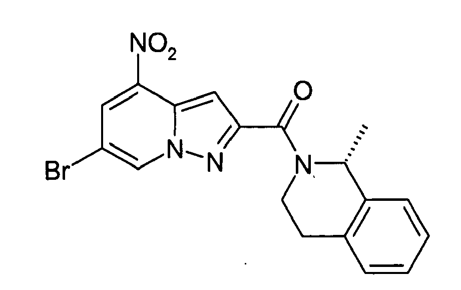

In close analogy to the procedure described in Example 1, 6-bromo-4-nitro-pyrazolo[1,5-a]pyridine-2-carboxylic acid is reacted with (R)-1-methyl-1,2,3,4-tetrahydroisoquinoline to provide the title compound in good to moderate yield.
LC/MS: m/z = 416 (MH⁺)

The pure stereoisomeric forms (including optical isomers) of the compounds and the intermediates of this invention may be obtained by the application of art-known procedures. Diastereomers may be separated by physical separation methods such as selective crystallization and chromatographic techniques, e.g. liquid chromatography using chiral stationary phases. Enantiomers (optically active isomers) may be separated from each other by selective crystallization of their diastereomeric salts with optically active acids. Alternatively, enantiomers may be separated by chromatographic techniques using chiral stationary phases.

Said pure stereoisomeric forms may also be derived from the corresponding pure stereoisomeric form of appropriate starting materials, provided that the reaction occur stereoselectively. Stereoisomeric forms of Formula I are included within the scope of this invention.

For therapeutic use, salts of the compounds of Formula **I** are those wherein the counterion is pharmaceutically acceptable. However, salts of acids and bases, which are non-pharmaceutically acceptable, may also find use, for example, in the preparation and purification of pharmaceutically acceptable compounds. All salts whether pharmaceutically acceptable or not are included within the ambit of the present invention.

The pharmaceutically acceptable salts as mentioned above are meant to comprise the therapeutically active non-toxic salt forms, which the compounds of Formula **I** are able to form. The latter can conveniently be obtained by treating the base form with such appropriate acids as inorganic acids, e.g. hydrohalic acids such as hydrochloric, hydrobromic and the like; sulfuric acid; nitric acid; phosphoric acid and the like; or organic acids such as acetic, propanoic, hydroxyacetic, 2-hydroxypropanoic, oxopropanoic, oxalic, malonic, succinic, maleic, fumaric, malic, tartaric, 2-hydroxy-1,2,3-propanetricarboxylic, methanesulfonic, ethanesulfonic, benzenesulfonic, 4-methylbenzenesulfonic, cyclohexane-sulfonic, 2-hydroxybenzoic, 4-amino-2-hydroxybenzoic and the like acids. Conversely, the salt form can be converted by treatment with alkali into the free base form.

### Pharmaceutical Compositions

The active ingredients of Formula **I** of the invention, together with one or more excipient/s like adjuvants, carriers, or diluents, may be placed into the form of pharmaceutical compositions, unit dosages or dosage forms. The pharmaceutical compositions may be employed as solid dosage forms, such as powders, granules, pellets, coated or uncoated tablets or filled capsules, or liquid dosage forms, such as solutions, suspensions, emulsions, or capsules filled with the same, or semi solid dosage forms, such as gels, creams and ointments. The active ingredient(s) dissolution and release profiles of the pharmaceutical dosage forms can be varied from seconds to months.

The pharmaceutical compositions are designed for the use in animals and humans and can be applied via all application routes. Preferred application routes will be the oral route, the dermal route, the pulmonary route, the nasal route, the rectal route, the parenterale route.

Such pharmaceutical compositions and unit dosage forms thereof may comprise conventional or new ingredients in conventional or special proportions, with or without additional active compounds or principles, and such unit dosage forms may contain any suitable effective amount of the active ingredient commensurate with the intended daily dosage range to be employed. Tablets containing one (1) to one hundred (100) milligrams of active ingredient or, more broadly, zero point five (0.5) to five hundred (500) milligrams per tablet, are accordingly suitable representative unit dosage forms.

The term "carrier" applied to pharmaceutical compositions of the invention refers to a diluent, excipient, or vehicle with which an active compound is administered. Such pharmaceutical carriers can be sterile liquids, such as water, saline solutions, aqueous dextrose solutions, aqueous glycerol solutions, and oils, including those of petroleum, animal, vegetable or synthetic origin, such as peanut oil, soybean oil, mineral oil, sesame oil and the like. A.R. Gennaro, 20th Edition, describes suitable pharmaceutical carriers in "Remington: The Science and Practice of Pharmacy".

### Method of treating and pharmaceutical formulations

Due to their high degree of activity and their low toxicity, together presenting a most favorable therapeutic index, the active principles of Formula **I** of the invention may be administered to a subject, e.g., a living animal (including a human) body, in need thereof, for the treatment, alleviation, or amelioration, palliation, or elimination of an indication or condition which is susceptible thereto, or representatively of an indication or condition set forth elsewhere in this application, preferably concurrently, simultaneously, or together with one or more pharmaceutically-acceptable excipients, carriers, or diluents, especially and preferably in the form of a pharmaceutical composition thereof, whether by oral, rectal, or parental (including intravenous and subcutaneous) or in some cases even topical route, in an effective amount. Suitable dosage ranges are 1-1000 milligrams daily, preferably 10-500 milligrams daily, and especially 50-500 milligrams daily, depending as usual upon the exact mode of administration, form in which administered, the indication toward which the administration is directed, the subject involved and the body weight of the subject involved, and the preference and experience of the physician or veterinarian in charge.

The term "treat" is used herein to mean to relieve or alleviate at least one symptom of a disease in a subject. Within the meaning of the present invention, the term "treat" also denotes to arrest, delay the onset (*i.e.*, the period prior to clinical manifestation of a disease) and/or reduce the risk of developing or worsening a disease.

The term "therapeutically effective" applied to dose or amount refers to that quantity of a compound or pharmaceutical composition that is sufficient to result in a desired activity upon administration to a living animal body in need thereof.

The active agents of Formula **I** of the present invention may be administered orally, topically, parenterally, or mucosally (e.g., buccally, by inhalation, or rectally) in dosage unit formulations containing conventional non-toxic pharmaceutically acceptable carriers. It is usually desirable to use the oral route. The active agents may be administered orally in the form of a capsule, a tablet, or the like (see Remington: The Science and Practice of Pharmacy, 20th Edition). The orally administered pharmaceutical compositions may be administered in the form of a time-controlled release vehicle, including diffusion-controlled systems, osmotic devices, dissolution-controlled matrices, and erodible/degradable matrices.

For oral administration in the form of a tablet or capsule, the active drug component of Formula **I** may be combined with a non-toxic, pharmaceutically acceptable excipients such as binding agents (e.g., pregelatinized maize starch, polyvinylpyrrolidone or hydroxypropyl methylcellulose); fillers (e.g., lactose, sucrose, glucose, mannitol, sorbitol and other reducing and non-reducing sugars, microcrystalline cellulose, calcium sulfate, or calcium hydrogen phosphate); lubricants (e.g., magnesium stearate, talc, or silica, steric acid, sodium stearyl fumarate, glyceryl behenate, calcium stearate, and the like); disintegrants (e.g., potato starch or sodium starch glycolate); or wetting agents (e.g., sodium lauryl sulphate), coloring and flavoring agents, gelatin, sweeteners, natural and synthetic gums (such as acacia, tragacanth or alginates), buffer salts, carboxymethylcellulose, polyethyleneglycol, waxes, and the like. For oral administration in liquid form, the drug components may be combined with non-toxic, pharmaceutically acceptable inert carriers (e.g., ethanol, glycerol, water), suspending agents (e.g., sorbitol syrup, cellulose derivatives or hydrogenated edible fats), emulsifying agents (e.g., lecithin or acacia), non-aqueous vehicles (e.g., almond oil, oily esters, ethyl alcohol or fractionated vegetable oils), preservatives (e.g., methyl or propyl-p-hydroxybenzoates or sorbic acid), and the like. Stabilizing agents such as antioxidants (BHA, BHT, propyl gallate, sodium ascorbate, citric acid) may also be added to stabilize the dosage forms.

The tablets containing as active compound a compound of Formula **I** may be coated by methods well known in the art. The compositions of the invention containing as active compound a compound of Formula **I** may be also introduced in beads, microspheres or microcapsules, e.g., fabricated from polyglycolic acid/lactic acid (PGLA). Liquid preparations for oral administration may take the form of, for example, solutions, syrups, emulsions or suspensions, or they may be presented as a dry product for reconstitution with water or other suitable vehicle before use. Preparations for oral administration may be suitably formulated to give controlled or postponed release of the active compound.

The active drugs of Formula **I** may also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines, as is well known.

Drugs of the invention containing as active compound a compound of Formula **I** may also be delivered by the use of monoclonal antibodies as individual carriers to which the compound molecules are coupled. Active drugs may also be coupled with soluble polymers as targetable drug carriers. Such polymers include polyvinyl-pyrrolidone, pyran copolymer, polyhydroxy-propyl methacrylamide-phenol, polyhydroxy-ethyl-aspartamide-phenol, or polyethyleneoxide-polylysine substituted with palmitoyl residues. Furthermore, active drug may be coupled to a class of biodegradable polymers useful in achieving controlled release of a drug, for example, polylactic acid, polyglycolic acid, copolymers of polylactic and polyglycolic acid, polyepsilon caprolactone, polyhydroxybutyric acid, polyorthoesters, polyacetals, polyhydropyrans, polycyanoacrylates, and cross-linked or amphipathic block copolymers of hydrogels.

For administration by inhalation, the therapeutics according to the present invention containing as active compound a compound of Formula **I** may be conveniently delivered in the form of an aerosol spray presentation from pressurized packs or a nebulizer, with the use of a suitable propellant, e.g., dichlorodifluoromethane, trichlorofluoromethane, dichlorotetra-fluoroethane, carbon dioxide, or other suitable gas. In the case of a pressurized aerosol, the dosage unit may be determined by providing a valve to deliver a metered amount. Capsules and cartridges of, e.g., gelatin for use in an inhaler or insufflator can be formulated containing a powder mix of the compound and a suitable powder base such as lactose or starch.

The formulations of the invention containing a compound of Formula **I** may be delivered parenterally, i.e., by intravenous (i.v.), intracerebroventricular (i.c.v.), subcutaneous (s.c.), intraperitoneal (i.p.), intramuscular (i.m.), subdermal (s.d.), or intradermal (i.d.) administration, by direct injection, via, for example, bolus injection or continuous infusion. Formulations for injection can be presented in unit dosage form, e.g., in ampoules or in multi-dose containers, with an added preservative. The compositions can take such forms as excipients, suspensions, solutions, or emulsions in oily or aqueous vehicles, and can contain formulatory agents such as suspending, stabilizing and/or dispersing agents. Alternatively, the active ingredient can be in powder form for reconstitution with a suitable vehicle, e.g., sterile pyrogen-free water, before use.

Compositions of the present invention containing a compound of Formula **I** may also be formulated for rectal administration, e.g., as suppositories or retention enemas (e.g., containing conventional suppository bases such as cocoa butter or other glycerides).

The compositions containing a compound of Formula **I** may, if desired, be presented in a pack or dispenser device, which may contain one or more unit dosage forms containing the active ingredient and/or may contain different dosage levels to facilitate dosage titration. The pack may, for example, comprise metal or plastic foil, such as a blister pack. The pack or dispenser device may be accompanied by instructions for administration. Compositions of the invention formulated in a compatible pharmaceutical carrier may also be prepared, placed in an appropriate container, and labeled for treatment of an indicated condition.

As disclosed herein, the dose of the components in the compositions of the present invention is determined to ensure that the dose administered continuously or intermittently will not exceed an amount determined after consideration of the results in test animals and the individual conditions of a patient. A specific dose naturally varies depending on the dosage procedure, the conditions of a patient or a subject animal such as age, body weight, sex, sensitivity, feed, dosage period, drugs used in combination, seriousness of the disease. The appropriate dose and dosage times under certain conditions can be determined by the test based on the above-described indices but may be refined and ultimately decided according to the judgment of the practitioner and each patient's circumstances (age, general condition, severity of symptoms, sex, etc.) according to standard clinical techniques.

Toxicity and therapeutic efficacy of the compositions of the invention can be determined by standard pharmaceutical procedures in experimental animals, e.g., by determining the LD₅₀ (the dose lethal to 50% of the population) and the ED₅₀ (the dose therapeutically effective in 50% of the population). The dose ratio between therapeutic and toxic effects is the therapeutic index and it may be expressed as the ratio LD₅₀/ED₅₀. Compositions that exhibit large therapeutic indices are preferred.

### Examples of Representative Pharmaceutical Compositions

With the aid of commonly used solvents, auxiliary agents and carriers, the reaction products can be processed into tablets, coated tablets, capsules, drip solutions, suppositories, injection and infusion preparations, and the like and can be therapeutically applied by the oral, rectal, parenteral, and additional routes. Representative pharmaceutical compositions according to the present invention follow:
(a) Tablets suitable for oral administration which contain the active ingredient, may be prepared by conventional tabletting techniques.
(b) For suppositories, any usual suppository base may be employed for incorporation thereinto by usual procedure of the active ingredient, such as a polyethyleneglycol which is a solid at normal room temperature but which melts at or about body temperature.
(c) For parental (including intravenous and subcutaneous) sterile solutions, the active ingredient together with conventional ingredients in usual amounts are employed, such as for example sodium chloride and double-distilled water q.s., according to conventional procedure, such as filtration, aseptic filling into ampoules or IV-drip bottles, and autoclaving for sterility.

Other suitable pharmaceutical compositions will be immediately apparent to one skilled in the art.

### Formulation Examples for the compounds of Formula I:

The following examples are given by way of illustration. As active ingredient, the compound according to example 76 can be used.

### Example 1 Tablet Formulation

A suitable formulation for a tablet containing 10 milligrams of active ingredient is as follows:

| | mg |
|---|---|
| Active Ingredient | 10 |
| Lactose | 61 |
| Microcrystalline Cellulose | 25 |
| Talcum | 2 |
| Magnesium stearate | 1 |
| Colloidal silicon dioxide | 1 |

### Example 2 Coated Tablet Formulation

Another suitable formulation for a tablet containing 100 mg is as follows:

| | mg |
|---|---|
| Active Ingredient | 100 |
| Polyvinylpyrrolidone, crosslinked | 10 |
| Potato starch | 20 |
| Polyvinylpyrrolidone | 19 |
| Magnesium stearate | 1 |
| Microcrystalline Cellulose | 50 |
| Film coated and colored. | |
| The film coating material consists of: | |
| Hypromellose | 10 |
| Microcryst. Cellulose | 5 |
| Talcum | 5 |
| Polyethylene glycol | 2 |
| Color pigments | 5 |

### Example 3 Capsule Formulation

A suitable formulation for a capsule containing 50 milligrams of active ingredient is as follows:

| | mg |
|---|---|
| Active Ingredient | 50 |
| Corn starch | 26 |
| Dibasic calcium phosphate | 50 |
| Talcum | 2 |
| Colloidal silicon dioxide | 2 |

This formulation is filled in a gelatin capsule.

### Example 4 Solution for injection

A suitable formulation for an injectable solution is as follows:

| | | |
|---|---|---|
| Active Ingredient | mg | 10 |
| Sodium chloride | mg | q.s. |
| Water for Injection | ml | add 1.0 |

### Example 5 Liquid oral formulation

A suitable formulation for 1 liter of an oral solution containing 2 milligrams of active ingredient in one milliliter of the mixture is as follows:

| | mg |
|---|---|
| Active Ingredient | 2 |
| Saccharose | 250 |
| Glucose | 300 |
| Sorbitol | 150 |
| Orange flavor | 10 |
| Colorant | q.s. |
| Purified water | add 1000 ml |

### Example 6 Liquid oral formulation

Another suitable formulation for 1 liter of a liquid mixture containing 20 milligrams of active ingredient in one milliliter of the mixture is as follows:

| | g |
|---|---|
| Active Ingredient | 20.00 |
| Tragacanth | 7.00 |
| Glycerol | 50.00 |
| Saccharose | 400.00 |
| Methylparaben | 0.50 |
| Propylparaben | 0.05 |
| Black currant-flavor | 10.00 |
| Soluble Red color | 0.02 |
| Purified water | add 1000 ml |

### Example 7 Liquid oral formulation

Another suitable formulation for 1 liter of a liquid mixture containing 2 milligrams of active ingredient in one milliliter of the mixture is as follows:

| | g |
|---|---|
| Active Ingredient | 2 |
| Saccharose | 400 |
| Bitter orange peel tincture | 20 |
| Sweet orange peel tincture | 15 |
| Purified water | add 1000 ml |

### Example 8 Aerosol formulation

180 g of the aerosol solution contain:

| | g |
|---|---|
| Active Ingredient | 10 |
| Oleic acid | 5 |
| Ethanol | 81 |
| Purified Water | 9 |
| Tetrafluoroethane | 75 |

15 ml of the solution are filled into aluminum aerosol cans, capped with a dosing valve, purged with 3.0 bar.

### Example 9 Trans-Dermal-System formulation

100 g of the solution contain:

| | g |
|---|---|
| Active Ingredient | 10.0 |
| Ethanol | 57.5 |
| Propyleneglycol | 7.5 |
| Dimethylsulfoxide | 5.0 |
| Hydroxyethylcellulose | 0.4 |
| Purified water | 19.6 |

1.8 ml of the solution is placed on a fleece covered by an adhesive backing foil. The system is closed by a protective liner which will be removed before use.

### Example 10 Nanoparticle formulation

10 g of polybutylcyanoacrylate nanoparticles contain:

| | g |
|---|---|
| Active Ingredient | 1.00 |
| Poloxamer | 0.10 |
| Butylcyanoacrylate | 8.75 |
| Mannitol | 0.10 |
| Sodium chloride | 0.05 |

Polybutylcyanoacrylate nanoparticles are prepared by emulsion polymerization in a water/0.1 N HCl/ethanol mixture as polymerizsation medium. The nanoparticles in the suspension are finally lyophilized under vacuum.

### Example 11 Suspension formulation

1.0 g of the suspension contains the following:

| | g |
|---|---|
| Active Ingredient | 0.10 |
| Hypromellose | 0.01 |
| Purified water | Ad 1.0 g |

Hypromellose is dispersed in water homogeneously with a high speed mixer/blender. After about one hour of hydration time of the hypromellose, the active ingredient is blended homogeneously into the hypromellose solution. The viscosity of the suspension can be adjusted by the amount of hypromellose, resulting in a very stable suspension with a very slow tendency of particle sedimentation and particle agglomeration.

### Example 12 Solution for Injection

1.0 ml of solution contain:

| | g |
|---|---|
| Active Ingredient | 0.05 |
| Mannitol | q.s. |
| DMSO | 0.10 |
| Water for injection | Ad 1.0 ml |

The active ingredient is dissolved in DMSO by stirring and heating (solution 1). The mannitol is dissolved in WFI (solution 2). After cooling down to room temperature solution 1 is mixed with solution 2 by continuous stirring. The solution is sterilized by filtration of by autoclaving.

### Pharmacology

The active principles of the present invention, and pharmaceutical compositions containing them and method of treating therewith, are characterized by unique and advantageous properties. The compounds and pharmaceutical compositions thereof exhibit, in standard accepted reliable test procedures, the following valuable properties and characteristics

### METHODS

Binding Assays for the Characterization of mGluR5 Antagonists
[³H]-MPEP (2-methyl-6-(phenylethynyl)pyridine) binding to transmembrane allosteric modulatory sites of mGluR5 receptors in cortical membranes.

### Preparation of rat cortical membranes:

Male Sprague-Dawley rats (200-250 g) are decapitated and their brains are removed rapidly. The cortex is dissected and homogenized in 20 volumes of ice-cold 0.32 M sucrose using a glass-Teflon homogenizer. The homogenate is centrifuged at 1000 x g for 10 minutes. The pellet is discarded and the supernatant centrifuged at 20,000 x g for 20 minutes. The resulting pellet is re-suspended in 20 volumes of distilled water and centrifuged for 20 minutes at 8000 x g. Then the supernatant and the buffy coat are centrifuged at 48,000 x g for 20 minutes in the presence of 50 mM Tris-HCl, pH 8.0. The pellet is then re-suspended and centrifuged two to three more times at 48,000 x g for 20 minutes in the presence of 50 mM Tris-HCl, pH 8.0. All centrifugation steps are carried out at 4°C. After resuspension in 5 volumes of 50 mM Tris-HCl, pH 8.0 the membrane suspension is frozen rapidly at -80°C.

On the day of assay the membranes are thawed and washed four times by resuspension in 50 mM Tris-HCl, pH 8.0 and centrifugation at 48,000 x g for 20 minutes and finally re-suspended in 50 mM Tris-HCl, pH 7.4. The amount of protein in the final membrane preparation (500-700 µg/ml) is determined according to the method of Lowry (Lowry O. H. et al. 1951. J. Biol. Chem. 193, 256-275).

### [³H]-MPEP Assay

Incubations are started by adding [³H]-MPEP (50.2 Ci/mmol, 5 nM, Tocris, GB) to vials with 125-250 µg protein (total volume 0.25 ml) and various concentrations of the agents. Alternatively, assays are performed with [³H]-MMPEP (2-(3-methoxyphenylethynyl)-6-methylpyridine hydrochloride) as radioligand. The incubations are continued at room temperature for 60 minutes (equilibrium is achieved under the conditions used). Nonspecific binding is defined by the addition of unlabeled MPEP (10 µM). Incubations are terminated using a Millipore filter system. The samples are rinsed twice with 4 ml of ice-cold assay buffer over glass fibre filters (Schleicher & Schuell, Germany) under a constant vacuum. Following separation and rinse, the filters are placed into scintillation liquid (5 ml Ultima Gold, Perkin Elmer, Germany) and radioactivity retained on the filters is determined with a conventional liquid scintillation counter (Canberra Packard, Germany).

### Characterization:

Specific binding is extremely high i.e. normally > 85% and essentially independent of buffer (Tris or HEPES both 50 mM) and pH (6.8-8.9). There is a clear saturable protein dependence and the chosen protein concentration used for subsequent assays (500-700 µg/ml) is within the linear portion of this dependence. Cold MPEP displaces hot ligand with an IC₅₀ of 11.2 ± 0.64 nM. The K_{d} of [³H]-MPEP of 13.6 nM is determined by Scatchard analysis and used according to the Cheng Prussoff relationship to calculate the affinity of displacers as K_{d} values (IC₅₀ of cold MPEP equates to a Kᵢ of 8.2 nM). Bₘₐₓ is 0.56 pm / mg protein.

### Functional Assays of mGluR5 Receptors

### Cytosolic Calcium studies with stably transfected cells:

Chinese hamster ovary cells (CHO-K1 cells), stably transfected for inducible expression of a human metabotropic glutamate receptor mGluR5, are seeded into black clear bottom 96 well plates at a density of 35.000 cells per well. The standard growth medium used (Dulbecco's modified Eagle Medium, DMEM plus L-proline) contains the appropriate inducer isopropyl-β-D-thiogalactopyranosid (IPTG) to achieve optimal receptor expression. One day after seeding the growth medium is exchanged for reconstituted Ca-Kit (Molecular Devices, USA) and incubated for one hour. Ca-Kit is reconstituted in an assay buffer containing 20 mM HEPES pH 7.4, glutamic-pyruvate transaminase, pyridoxal phosphate and sodium pyruvate in Hank's balanced salt solution (HBBS). Agonistic compounds to the receptor elicit increases in cytosolic calcium which can be measured as increases in fluorescence signals by use of a fluorescence imaging plate reader (Molecular Devices). To analyze their potency to modulate the Ca-response test compounds, dissolved in a final DMSO concentration of 0.5%, are added on-line 5 minutes before the agonist to the receptor (L-quisqualic acid at a concentration giving ∼80% of the maximal signal).

### Astrocyte culture:

Primary astrocyte cultures are prepared from cortices of newborn rats as described by Booher and Sensenbrenner (1972, Neurobiology 2(3):97-105). Briefly, Sprague-Dawley rat pups (2 - 4 d old) are decapitated and neocortices are dissected, disintegrated with a nylon filter (pore size 80 µm) and carefully triturated. The cell suspension is plated on poly-D-lysine pre-coated flasks (Costar, Netherlands) and cultivated in Dulbecco's Modified Eagle's Medium (DMEM, Invitrogen, Germany) supplemented with 10% foetal calf serum (FCS, Sigma, Germany), 4 mM glutamine and 50 µg/ml gentamycin (both Biochrom, Germany) at 37°C in a humidified atmosphere of 5% CO₂ / 95% air for 7 d with exchanging the medium at day 2 and 6.

After 7 days in vitro (DIV), cells are shaken overnight at 250 rpm to remove oligodendrocytes and microglia. The next day, astrocytes are rinsed twice with CMF-PBS (calcium- and magnesium-free phosphate buffered saline, Biochrom, Germany), trypsinized and subplated on poly-D-lysine pre-coated 96-well plates (Greiner, Germany) at a density of 40,000 cells/well. 24 h after establishing the secondary culture the astrocytes are rinsed with PBS⁺⁺ (phosphate buffered saline, Biochrom, Germany) and fed with astrocyte-defined medium (ADM) consisting of DMEM containing 1x G5-supplement (Invitrogen, Germany), 0.5 µg/ml heparan sulfate, and 1.5 µg/ml fibronectin (both Sigma, Germany) (Miller et al., (1993) Brain Res. 618(1):175-8). 3 d later the medium is exchanged and the cells incubated for another 2-3 d, so that at the time of experiments astrocytes are 14-15 DIV.

### Immunocytochemistry

Immunostaining is performed to confirm the presence of astrocytic markers such as the glial fibrillary acidic protein (GFAP) as well as to monitor the expression of mGluR5 receptors.

### Cytosolic Calcium studies with astrocytes:

The increase of cytosolic calcium after stimulation with the mGluR5 agonist L-quisqualate is measured using a fluorometric imaging plate reader (FLIPR) and the Ca-Kit (both Molecular Devices). Prior to addition of agonist or antagonist the medium is aspirated and cells are loaded for 2 h at RT with 150 µl of loading buffer consisting of Ca-sensitive dye reconstituted in sodium chloride (123 mM), potassium chloride (5.4 mM), magnesium chloride (0.8 mM), calcium chloride (1.8 mM), D-glucose (15 mM), and HEPES (20 mM), pH 7.3. Subsequently, plates are transferred to FLIPR to detect calcium increase with the addition of L-quisqualate (100 nM) measured as relative fluorescence units (RFU). If antagonists are tested, these compounds are pre-incubated for 10 minutes at RT before addition of the respective agonist.

For positive modulators, concentration-response curves for quisqualate are performed in the presence and absence of 10 µM modulator to determine the extent of potentiation / agonist potency increase. Thereafter, concentration-response curves for the positive modulator are performed in the presence of a fixed concentration of quisqualate showing the biggest window for potentiation (normally 10-30 nM).

### Data analysis

The fluorescence signal increase after addition of agonist reflects the increase of cytosolic calcium. Inconsistencies in the amount of cells per well are normalised by using the spatial uniformity correction of the FLIPR software. The mean of replicated temporal data (n=3-5) is calculated and used for graphical representation. For the evaluation of the pharmacology, the calcium changes in response to different concentrations of agonist or antagonist are determined using a maximum minus minimum (MaxMin) calculation.

All responses (RFU-values) are determined as percentage of control (= maximum response). EC₅₀ and IC₅₀ are calculated according the logistic equation using GraFit 5.0 (Erithacus Software, GB) or Prism 4.0 (GraphPad Software, USA). The compounds of the present invention have a potency (IC₅₀) within a range of about 0.5 nM to about 100 µM.

Results for representative compounds of the invention are shown in Tables A1-A3.

In conclusion, from the foregoing, it is apparent that the present invention provides novel and valuable applications and uses of the compounds of the present invention, which compounds comprise the active principle according to the present invention, as well as novel pharmaceutical compositions thereof and methods of preparation thereof and of treating therewith.

The high order of activity of the active agent of the present invention and compositions thereof, as evidenced by the tests reported, is indicative of utility based on its valuable activity in human beings as well as in lower animals. Clinical evaluation in human beings has not been completed. It will be clearly understood that the distribution and marketing of any compound or composition falling within the scope of the present invention for use in human beings will of course have to be predicated upon prior approval by governmental agencies which are responsible for and authorized to pass judgment on such questions.

The instant compounds of Formula **I** represent a novel class of mGluR5 modulators. In view of their potency, they will be useful therapeutics in a wide range of disorders, in particular CNS disorders, which involve excessive glutamate induced excitation.

These compounds accordingly find application in the treatment of the disorders of a living animal body, especially a human, as listed earlier in the description.

These compounds also find application in the treatment of indications in a living animal body, especially a human, wherein a particular condition does not necessarily exist but wherein a particular physiological parameter may be improved through administration of the instant compounds, including cognitive enhancement.

Neuroprotection as well as cognitive enhancement can also be achieved by combining application of these compounds with NMDA receptor antagonists like Memantine and Neramexane.

The method-of-treating a living animal body with a compound of the invention, for the inhibition of progression or alleviation of the selected ailment therein, is as previously stated by any normally-accepted pharmaceutical route, employing the selected dosage which is effective in the alleviation of the particular ailment desired to be alleviated. Use of the compounds of the present invention in the manufacture of a medicament for the treatment of a living animal for inhibition of progression or alleviation of selected ailments or conditions, particularly ailments or conditions susceptible to treatment with a Group I mGluR modulator is carried out in the usual manner comprising the step of admixing an effective amount of a compound of the invention with a pharmaceutically-acceptable diluent, excipient, or carrier, and the method-of-treating, pharmaceutical compositions, and use of a compound of the present invention in the manufacture of a medicament.

Representative pharmaceutical compositions prepared by admixing the active ingredient with a suitable pharmaceutically-acceptable excipient, diluent, or carrier, include tablets, capsules, solutions for injection, liquid oral formulations, aerosol formulations, TDS formulations, and nanoparticle formulations, thus to produce medicaments for oral, injectable, or dermal use, also in accord with the foregoing.

**Table A1 (Cytosolic Calcium studies with stably transfected cells)**

| **compound** | **Chemical name** | **mGluR5_FLIPR_h_ CHO_NAM IC50** **[µM]** |
|---|---|---|
| 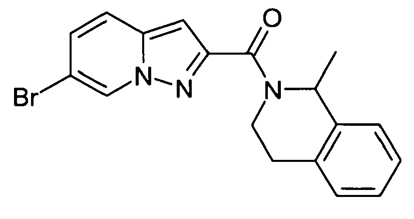 | (6-Bromopyrazolo[1,5-a]pyridin-2-yl)-(1-methyl-3,4-dihydro-1H-isoquinolin-2-yl)-methanone | 0,2176 |
| 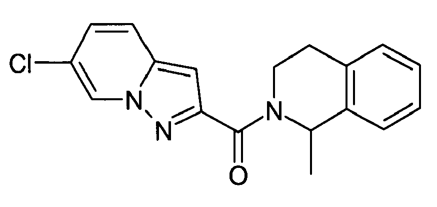 | (6-Chloro-pyrazolo[1,5-a]pyridin-2-yl)-(1-methyl-3,4-dihydro-1H-isoquinolin-2-yl)-methanone | 0,1021 |
| 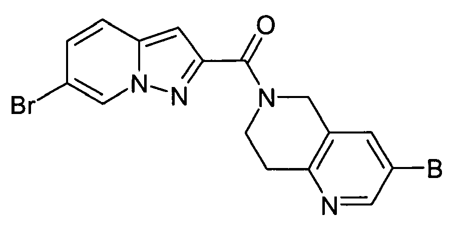 | (3-Bromo-7,8-dihydro-5H-[1,6]naphthyridin-6-yl)-(6-bromo-pyrazolo[1,5-a]pyridin-2-yl)-methanone | 0,3061 |
| 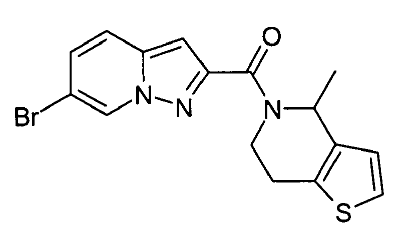 | (6-Bromo-pyrazolo[1,5-a]pyridin-2-yl)-(4-methyl-6,7-dihydro-4H-thieno[3,2-c]pyridin-5-yl)-methanone | 0,1673 |
| 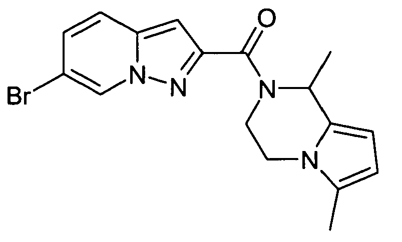 | 6-bromo-2-[(1,6-dimethyl-3,4-dihydropyrrolo[1,2-a]pyrazin-2(1H)-yl)carbonyl]pyrazol o[1,5-a]pyridine | 0,1784 |
| 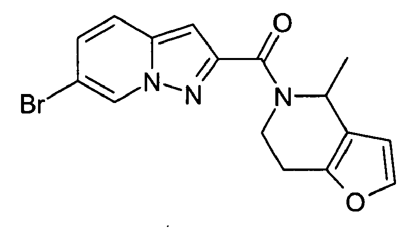 | (6-Bromo-pyrazolo[1,5-a]pyridin-2-yl)-(4-methyl-6,7-dihydro-4H-furo[3,2-c]pyridin-5-yl)-methanone | 0,1660 |
| 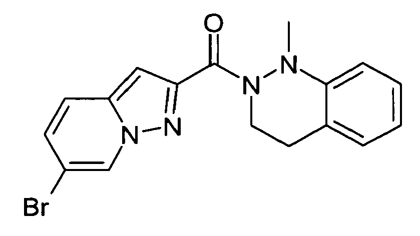 | (6-Bromo-pyrazolo[1,5-a]pyridin-2-yl)-(1-methyl-3,4-dihydro-1H-cinnolin-2-yl)-methanone | 0,4100 |
| 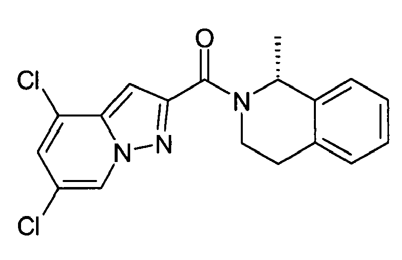 | (4,6-Dichloro-pyrazolo[1,5-a]pyridin-2-yl)-((R)-1-methyl-3,4-dihydro-1H-isoquinolin-2-yl)-methanone | 0,1139 |

**Table A2 (Astrocyte culture test)**

| **compound** | **Chemical name** | **mGluR5_FLIPRr_rpA IC50 [**µ**M]** |
|---|---|---|
| 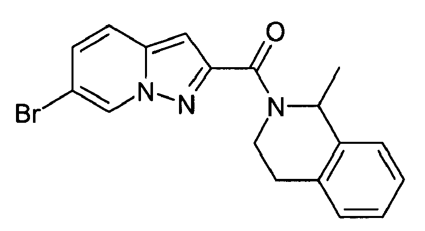 | (6-Bromo-pyrazolo[1,5-a]pyridin-2-yl)-(1-methyl-3,4-dihydro-1H-isoquinolin-2-yl)-methanone | 0,1510 |
| 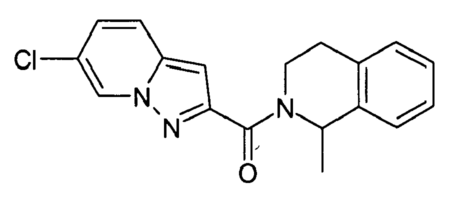 | (6-Chloro-pyrazolo[1,5-a]pyridin-2-yl)-(1-methyl-3,4-dihydro-1H-isoquinolin-2-yl)-methanone | 0,0450 |
| 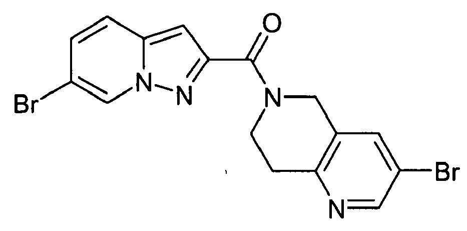 | (3-Bromo-7,8-dihydro-5H-[1,6]naphthyridin-6-yl)-(6-bromo-pyrazolo[1,5-a]pyridin-2-yl)-methanone | 0,1077 |
| 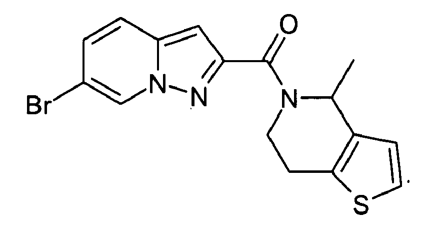 | (6-Bromo-pyrazolo[1,5-a]pyridin-2-yl)-(4-methyl-6,7-dihydro-4H-thieno[3,2-c]pyridin-5-yl)-methanone | 0,0510 |
| 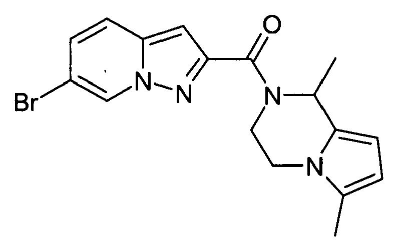 | 6-bromo-2-[(1,6-dimethyl-3,4-dihydropyrrolo[1,2-a]pyrazin-2(1 H)-yl)carbonyl]pyrazolo[1,5-a]pyridine | 0,1480 |
| 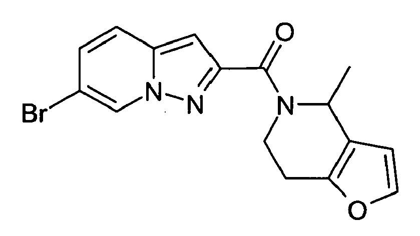 | (6-Bromo-pyrazolo[1,5-a]pyridin-2-yl)-(4-methyl-6,7-dihydro-4H-furo[3,2-c]pyridin-5-yl)-methanone | 0,0425 |
| 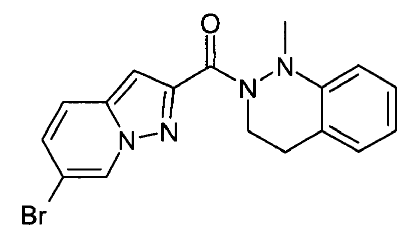 | (6-Bromo-pyrazolo[1,5-a]pyridin-2-yl)-(1-methyl-3,4-dihydro-1H-cinnolin-2-yl)-methanone | 0,156 |

**Table A3 (Results from ³H-MMPEP-Assay)**

| **compound** | **Chemical name** | **mGluR5_M_ MPEP_r_CTX_ IC50 [µM]** |
|---|---|---|
| 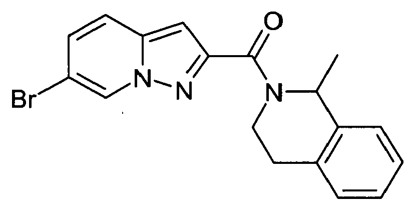 | (6-Bromo-pyrazolo[1,5-a]pyridin-2-yl)-(1-methyl-3,4-dihydro-1H-isoquinolin-2-yl)-methanone | 0,4000 |
| 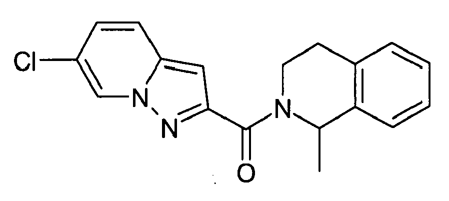 | (6-Chloro-pyrazolo[1,5-a]pyridin-2-yl)-(1-methyl-3,4-dihydro-1H-isoquinolin-2-yl)-methanone | 0,2600 |
| 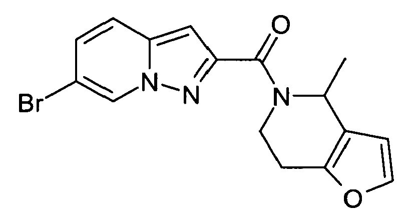 | (6-Bromo-pyrazolo[1,5-a]pyridin-2-yl)-(4-methyl-6,7-dihydro-4H-furo[3,2-c]pyridin-5-yl)-methanone | 0,6550 |
| 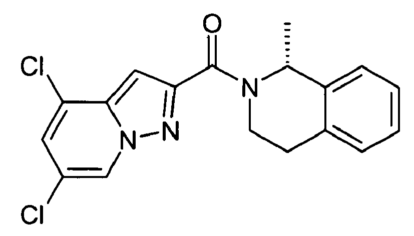 | (4,6-Dichloro-pyrazolo[1,5-a]pyridin-2-yl)-((R)-1-methyl-3,4-dihydro-1H-isoquinolin-2-yl)-methanone | 0,0430 |

## Claims

**1.** A compound selected from those of Formula **I** 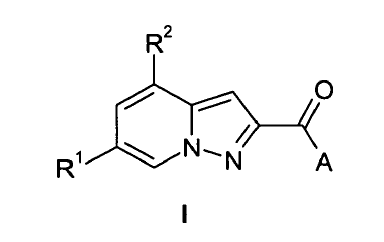 wherein
R¹ represents chloro or bromo;
R² represents hydrogen, C₁₋₆alkyl, amino, hydroxy, halogen, trifluoromethyl, C₁₋₆alkoxy, nitro, cyano, trifluoromethoxy, aminocarbonyl, C₁₋₆alkylaminocarbonyl, di-C₁₋₆alkylaminocarbonyl, hydroxyC₁₋₆alkyl, C₂₋₆alkenyl, C₁₋₆alkoxycarbonyl, C₁₋₆alkylcarbonyloxy, C₁₋₆alkylamino, aryl, heteroaryl, heterocyclyl, or cycloC₃₋₁₂alkyl; and
A represents NR³R⁴, wherein
R³ represents hydrogen, C₁₋₆alkyl, cydoC₃₋₁₂alkyl, aryl, heteroaryl, arylC₁₋₆alkyl, and heteroarylC₁₋₆alkyl and
R⁴ represents C₁₋₆alkyl, cycloC₃₋₁₂alkyl, aryl, heteroaryl, arylC₁₋₆alkyl, and heteroarylC₁₋₆alkyl; or
R³ and R⁴, together with the nitrogen to which they are attached, combine to form a partially unsaturated bicyclic or tricyclic ring system having 9 to 14 members comprising 1 to 8 heteroatoms selected from oxygen, sulfur, and nitrogen, wherein the ring system is optionally substituted with a group selected from halogen, amino, hydroxy, nitro, cyano, trifluoromethyl, trifluoromethoxy, aryl, C₁₋₆alkyl, cycloC₃₋₁₂alkyl, C₂₋₆alkenyl, C₂₋₆alkynyl, C₁₋₆alkoxy, cydoC₃₋₁₂alkyloxy, C₂₋₆alkenyloxy, C₂₋₆alkynyloxy, heteroaryl, heterocyclyl, aryloxy, heteroaryloxy, heterocyclyloxy, C₁₋₆alkylamino, di-C₁₋₆alkylamino, cycloC₃₋₁₂alkylamino, di-cycloC₃₋₁₂alkylamino, N-C₁₋₆alkyl-N- cycloC₃₋₁₂alkylamino, C₂₋₆alkenylamino, C₂₋₆alkynylamino, di-C₂₋₆alkenylamino, di-C₂₋₆alkynylamino, N-C₁₋₆alkyl-N-C₂₋₆alkenylamino, N-C₁₋₆alkyl-N-C₂₋₆alkynylamino, N-C₂₋₆alkenyl-N-cycloC₃₋₁₂alkylamino, N-C₂₋₆alkynyl-N-cycloC₃₋₁₂alkylamino, N-C₂₋₆alkenyl-N-C₂₋₆alkynylamino arylamino, diarylamino, aryl-C₁₋₆alkylamino, aryl-C₂-₆alkenylamino, aryl-C₂₋₆alkynylamino, N-aryl-N-cycloC₃₋₁₂alkylamino, heteroarylamino, diheteroarylamino, heteroaryl-C₁₋₆alkylamino, heteroaryl-C₂₋₆alkenylamino, heteroaryl-C₂₋₆alkynylamino, N-heteroaryl-N-cycloC₃₋₁₂alkylamino, N-heteroaryl-N-arylamino, heterocyclylamino, diheterocyclylamino, heterocyclyl-C₁₋₆alkylamino, heterocyclyl-C₂₋₆alkenylamino, heterocyclyl-C₂₋₆alkynylamino, N-heterocyclyl-N-cycloC₃₋₁₂alkylamino, N-heterocyclyl-N-arylamino, N-heterocyclyl-N-heteroarylamino, acyl, acyloxy, acylamino, C₁₋₆alkoxycarbonyl, cycloC₃₋₁₂alkoxycarbonyl, C₂₋₆alkenyloxycarbonyl, C₂₋₆alkynyloxycarbonyl, aryloxycarbonyl, heteroaryloxycarbonyl, heterocyclyloxycarbonyl, aminocarbonyl, C₁₋₆alkylaminocarbonyl, di-C₁₋₆alkylaminocarbonyl, cycloC₃₋₁₂alkylaminocarbonyl, di-cycloC₃₋₁₂alkylaminocarbonyl, N-C₁₋₆alkyl-N-cycloC₃₋₁₂alkylaminocarbonyl, C₂₋₆alkenylaminocarbonyl, C₂₋₆alkynylaminocarbonyl, di-C₂₋₆alkenylaminocarbonyl, di-C₂₋₆alkynylaminocarbonyl, N-C₁₋₆alkyl-N-C₂₋₆alkenylaminocarbonyl, N-C₁₋₆alkyl-N-C₂₋₆alkynylaminocarbonyl, N-C₂₋₆alkenyl-N-cycloC₃₋₁₂alkylaminocarbonyl, N-C₂₋₆alkynyl-N-cyclo₃₋₁₂alkylaminocarbonyl, N-C₂₋₆alkenyl-N-C₂₋₆alkynylaminocarbonyl, arylaminocarbonyl, diarylaminocarbonyl, aryl-C₁₋₆alkylaminocarbonyl, aryl-C₂₋₆alkenylaminocarbonyl, aryl-C₂₋₆alkynylamino-carbonyl, N-aryl-N-cyclo C₃₋₁₂alkylaminocarbonyl, heteroarylaminocarbonyl, diheteroarylaminocarbonyl, heteroarylC₁₋₆alkylaminocarbonyl, heteroaryl-C₂₋₆alkenylaminocarbonyl, heteroaryl-C₂₋₆alkynylaminocarbonyl, N-heteroaryl-N-cycloC₃₋₁₂alkylaminocarbonyl, N-heteroaryl-N-arylaminocarbonyl, heterocyclylaminocarbonyl, diheterocyclylaminocarbonyl, heterocyclyl-C₁₋₆alkylaminocarbonyl, heterocyclyl-C₂₋₆alkenylaminocarbonyl, heterocyclyl-C₂₋₆alkynylaminocarbonyl, N-heterocyclyl-N-cycloC₃₋₁₂alkylaminocarbonyl, N-heterocyclyl-N-arylaminocarbonyl, N-heterocyclyl-Nheteroarylaminocarbonyl, C₁₋₆alkylsulfinyl, cycloC₃₋₁₂alkylsulfinyl, C₂₋₆alkenylsulfinyl, C₂₋₆alkynylsulfinyl, arylsulfinyl, heteroarylsulfinyl, heterocyclylsulfinyl, C₁₋₆alkylsulfonyl, cycloC₃₋₁₂alkylsulfonyl, C₂₋₆alkenylsulfonyl, C₂₋₆alkynylsulfonyl, arylsulfonyl, heteroarylsulfonyl, heterocyclylsulfonyl, C₁₋₆alkylsulfonylamino, arylsulfonylamino, and oxo;
and optical isomers, pharmaceutically acceptable salts, hydrates, solvates and polymorphs thereof.

**2.** A compound as claimed in claim 1, wherein A represents 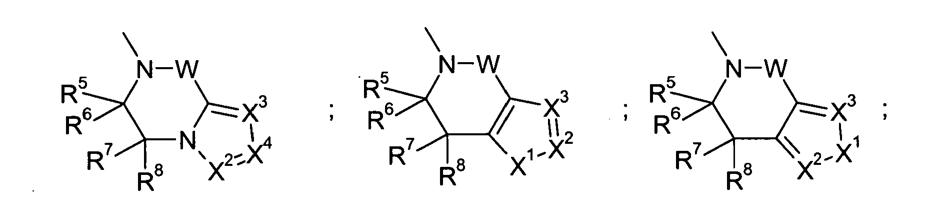 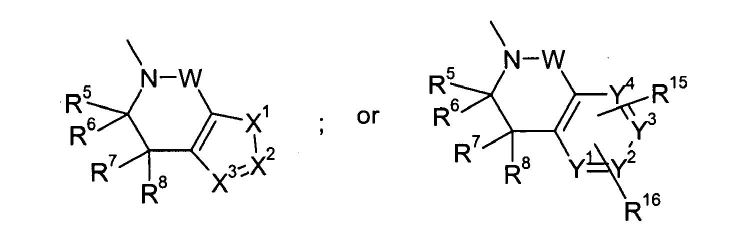 wherein
R⁵, R⁶, R⁷, and R⁸, which may be the same or different, each independently represent hydrogen, C₁₋₆alkyl, cycloC₃₋₁₂alkyl, amino, hydroxy, halogen, or trifluoromethyl;
W represents NR⁹ or CR¹⁰R¹¹, wherein
R⁹ represents hydrogen, C₁₋₆alkyl, trifluoromethyl, or cycloC₃₋₁₂alkyl and
R¹⁰ and R¹¹, which may be the same or different each independently
represent hydrogen, C₁₋₆alkyl, cycloC₃₋₁₂alkyl, or trifluoromethyl;
X¹ represents CR¹²R¹³, NR¹⁴, S, or O; and X², X³, and X⁴, which may be the same or different each independently represent CR¹² or N, wherein
R¹² and R¹³, which may be the same or different, each independently represent hydrogen, halogen, amino, hydroxy, nitro, cyano, trifluoromethyl, trifluoromethoxy, aryl, C₁₋₆alkyl, cycloC₃₋₁₂alkyl, C₂₋₆alkenyl, C₂₋₆alkynyl, C₁₋₆alkoxy, cycloC₃₋₁₂alkyloxy, C₂₋₆alkenyloxy, C₂₋₆alkynyloxy, heteroaryl, heterocyclyl, aryloxy, heteroaryloxy, heterocyclyloxy, C₁₋₆alkylamino, di-C₁₋₆alkylamino, cycloC₃₋₁₂alkylamino, di-cycloC₃₋₁₂alkylamino, N-C₁₋₆alkyl-N- cycloC₃₋₁₂alkylamino, C₂₋₆alkenylamino, C₂₋₆alkynylamino, di-C₂₋₆alkenylamino, di-C₂₋₆alkynylamino, N-C₁₋₆alkyl-N-C₂₋₆alkenylamino, N-C₁₋₆alkyl-N-C₂₋₆alkynylamino, N-C₂₋₆alkenyl-N-cycloC₃₋₁₂alkylamino, N-C₂₋₆alkynyl-N-cycloC₃₋₁₂alkylamino, N-C₂₋₆alkenyl-N-C₂₋₆alkynylamino arylamino, diarylamino, aryl-C₁₋₆alkylamino, aryl-C₂₋₆alkenylamino, aryl-C₂₋₆alkynylamino, N-aryl-N-cycloC₃₋₁₂alkylamino, heteroarylamino, diheteroarylamino, heteroaryl-C₁₋₆alkylamino, heteroaryl-C₂₋₆alkenylamino, heteroaryl-C₂₋₆alkynylamino, N-heteroaryl-N-cycloC₃₋₁₂alkylamino, N-heteroaryl-N-arylamino, heterocyclylamino, diheterocyclylamino, heterocyclyl-C₁₋₆alkylamino, heterocyclyl-C₂₋₆alkenylamino, heterocyclyl-C₂₋₆alkynylamino, N-heterocyclyl-N-cycloC₃₋₁₂alkylamino, N-heterocyclyl-N-arylamino, N-heterocyclyl-N-heteroarylamino, acyl, acyloxy, acylamino, C₁₋₆alkoxycarbonyl, cycloC₃₋₁₂alkoxycarbonyl, C₂₋₆alkenyloxycarbonyl, C₂₋₆alkynyloxycarbonyl, aryloxycarbonyl, heteroaryloxycarbonyl, heterocyclyloxycarbonyl, aminocarbonyl, C₁₋₆alkylaminocarbonyl, di-C₁₋₆alkylaminocarbonyl, cycloC₃₋₁₂alkylaminocarbonyl, di-cycloC₃₋₁₂alkylaminocarbonyl, N-C₁₋₆alkyl-N-cycloC₃₋₁₂alkylaminocarbonyl, C₂₋₆alkenylaminocarbonyl, C₂₋₆alkynylaminocarbonyl, di-C₂₋₆alkenylaminocarbonyl, di-C₂₋₆alkynylaminocarbonyl, N-C₁₋₆alkyl-N-C₂₋₆alkenylaminocarbonyl, N-C₁₋₆alkyl-N-C₂₋₆alkynylaminocarbonyl, N-C₂₋₆alkenyl-N-cycloC₃₋₁₂alkylaminocarbonyl, N-C₂₋₆alkynyl-N-cycloC₃₋₁₂alkylaminocarbonyl, N-C₂₋₆alkenyl-N-C₂₋₆alkynylaminocarbonyl, arylaminocarbonyl, diarylaminocarbonyl, aryl-C₁₋₆alkylaminocarbonyl, aryl-C₂₋₆alkenylaminocarbonyl, aryl-C₂₋₆alkynylaminocarbonyl, N-aryl-N-cyclo C₃₋₇alkylaminocarbonyl, heteroarylaminocarbonyl, diheteroarylaminocarbonyl, heteroaryl-C₁₋₆alkylaminocarbonyl, heteroaryl-C₂₋₆alkenylaminocarbonyl, heteroaryl-C₂₋₆alkynylaminocarbonyl, N-heteroaryl-N-cycloC₃₋₁₂alkylaminocarbonyl, N-heteroaryl-N-arylaminocarbonyl, heterocyclylaminocarbonyl, diheterocyclylaminocarbonyl, heterocyclyl-C₁₋₆alkylaminocarbonyl, heterocyclyl-C₂₋₆alkenylaminocarbonyl, heterocyclyl-C₂₋₆alkynylaminocarbonyl, N-heterocyclyl-N-cycloC₃₋₁₂alkylaminocarbonyl, N-heterocyclyl-N-arylaminocarbonyl, N-heterocyclyl-Nheteroarylaminocarbonyl, C₁₋₆alkylsulfinyl, cycloC₃₋₁₂alkylsulfinyl, C₂₋₆alkenylsulfinyl, C₂₋₆alkynylsulfinyl, arylsulfinyl, heteroarylsulfinyl, heterocyclylsulfinyl, C₁₋₆alkylsulfonyl, cycloC₃₋₁₂alkylsulfonyl, C₂₋₆alkenylsulfonyl, C₂₋₆alkynylsulfonyl, arylsulfonyl, heteroarylsulfonyl, heterocyclylsulfonyl, C₁₋₆alkylsulfonylamino, or arylsulfonylamino and
R¹⁴ represents hydrogen, C₁₋₆alkyl, cycloC₃₋₁₂alkyl, C₂₋₆alkenyl, acyl, aryl, heteroaryl, heterocyclyl, C₁₋₆alkylaminocarbonyl, di-C₁₋₆alkylaminocarbonyl, C₁₋₆alkylsulfonyl, arylsulfonyl or heteroarylsulfonyl;
Y¹, Y², Y³, and Y⁴ represent C or N, wherein at least two of Y¹, Y², Y³, and Y⁴ represent C;
R¹⁵ and R¹⁶, which may be the same or different, each independently represent hydrogen, halogen, amino, hydroxy, nitro, cyano, trifluoromethyl, trifluoromethoxy, aryl, C₁₋₆alkyl, cycloC₃₋₁₂alkyl, C₂₋₆alkenyl, C₂₋₆alkynyl, C₁₋₆alkoxy, cycloC₃₋₁₂alkyloxy, C₂₋₆alkenyloxy, C₂₋₆alkynyloxy, heteroaryl, heterocyclyl, aryloxy, heteroaryloxy, heterocyclyloxy, C₁₋₆alkylamino, di-C₁₋₆alkylamino, cycloC₃₋₁₂alkylamino, di-cycloC₃₋₁₂alkylamino, N-C₁₋₆alkyl-N-cycloC₃₋₁₂alkylamino, C₂₋₆alkenylamino, C₂₋₆alkynylamino, di-C₂₋₆alkenylamino, di-C₂₋₆alkynylamino, N-C₁₋₆alkyl-N-C₂₋₆alkenylamino, N-C₁₋₆alkyl-N-C₂₋₆alkynylamino, N-C₂₋₆alkenyl-N-cycloC₃₋₁₂alkylamino, N-C₂₋₆alkynyl-N-cycloC₃₋₁₂alkylamino, N-C₂₋₆alkenyl-N-C₂₋₆alkynylamino arylamino, diarylamino, aryl-C₁₋₆alkylamino, aryl-C₂₋₆alkenylamino, aryl-C₂₋₆alkynylamino, N-aryl-N-cycloC₃₋₁₂alkylamino, heteroarylamino, diheteroarylamino, heteroaryl-C₁₋₆alkylamino, heteroaryl-C₂₋₆alkenylamino, heteroaryl-C₂₋₆alkynylamino, N-heteroaryl-N-cycloC₃₋₁₂alkylamino, N-heteroaryl-N-arylamino, heterocyclylamino, diheterocyclylamino, heterocyclyl-C₁₋₆alkylamino, heterocyclyl-C₂₋₆alkenylamino, heterocyclyl-C₂₋₆alkynylamino, N-heterocyclyl-N-cycloC₃₋₁₂alkylamino, N-heterocyclyl-N-arylamino, N-heterocyclyl-N-heteroarylamino, acyl, acyloxy, acylamino, C₁₋₆alkoxycarbonyl, cycloC₃₋₁₂alkoxycarbonyl, C₂₋₆alkenyloxycarbonyl, C₂₋₆alkynyloxycarbonyl, aryloxycarbonyl, heteroaryloxycarbonyl, heterocyclyloxycarbonyl, aminocarbonyl, C₁₋₆alkylaminocarbonyl, di-C₁₋₆alkylaminocarbonyl, cycloC₃₋₁₂alkylaminocarbonyl, di-cycloC₃₋₁₂alkylaminocarbonyl, N-C₁₋₆alkyl-N-cycloC₃₋₁₂alkylaminocarbonyl, C₂₋₆alkenylaminocarbonyl, C₂₋₆alkynylaminocarbonyl, di-C₂₋₆alkenylaminocarbonyl, di-C₂₋₆alkynylaminocarbonyl, N-C₁₋₆alkyl-N-C₂₋₆alkenylaminocarbonyl, N-C₁₋₆alkyl-N-C₂₋₆alkynylaminocarbonyl, N-C₂₋₆alkenyl-N-cycloC₃₋₁₂alkylaminocarbonyl, N-C₂₋₆alkynyl-N-cycloC₃₋₁₂alkylaminocarbonyl, N-C₂₋₆alkenyl-N-C₂₋₆alkynylaminocarbonyl, arylaminocarbonyl, diarylaminocarbonyl, aryl-C₁₋₆alkylaminocarbonyl, aryl-C₂₋₆alkenylaminocarbonyl, aryl-C₂₋₆alkynylamino-carbonyl, N-aryl-N-cyclo C₃₋₇alkylaminocarbonyl, heteroarylaminocarbonyl, diheteroarylaminocarbonyl, heteroaryl-C₁₋₆alkylaminocarbonyl, heteroaryl-C₂₋₆alkenylaminocarbonyl, heteroaryl-C₂₋₆alkynylaminocarbonyl, N-heteroaryl-N-cycloC₃₋₁₂alkylaminocarbonyl, N-heteroaryl-N-arylaminocarbonyl, heterocyclylaminocarbonyl, diheterocyclylaminocarbonyl, heterocyclyl-C₁₋₆alkylaminocarbonyl, heterocyclyl-C₂₋₆alkenylaminocarbonyl, heterocyclyl-C₂₋₆alkynylaminocarbonyl, N-heterocyclyl-N-cycloC₃₋₁₂alkylaminocarbonyl, N-heterocyclyl-N-arylaminocarbonyl, N-heterocyclyl-Nheteroarylaminocarbonyl, C₁₋₆alkylsulfinyl, cycloC₃₋₁₂alkylsulfinyl, C₂₋₆alkenylsulfinyl, C₂₋₆alkynylsulfinyl, arylsulfinyl, heteroarylsulfinyl, heterocyclylsulfinyl, C₁₋₆alkylsulfonyl, cycloC₃₋₁₂alkylsulfonyl, C₂₋₆alkenylsulfonyl, C₂₋₆alkynylsulfonyl, arylsulfonyl, heteroarylsulfonyl, heterocyclylsulfonyl, C₁₋₆alkylsulfonylamino, or arylsulfonylamino;
or R¹⁵ and R¹⁶ together with the two carbon atoms carying them represent an aryl group which may be optionally substituted by a group selected from halogen, hydroxy, nitro, cyano, trifluoromethyl, trifluoromethoxy, C₁₋₆alkyl, and C₁₋₆alkoxy;
a heteroaryl group having 5 or 6 ring members which may be optionally substituted by a group selected from halogen, hydroxy, nitro, cyano, trifluoromethyl, trifluoromethoxy, C₁₋₆alkyl, and C₁₋₆alkoxy; or a heterocyclyl group having 5 or 6 ring members, which may be optionally substituted by a group selected from oxo, halogen, hydroxy, nitro, cyano, trifluoromethyl, trifluoromethoxy, C₁₋₆alkyl, and C₁₋₆alkoxy;
and optical isomers, pharmaceutically acceptable salts, hydrates, solvates, and polymorphs thereof.

**3.** A compound as claimed in claim 2, wherein A represents 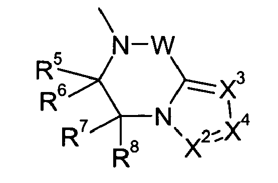 wherein R⁵, R⁶, R⁷, and R⁸, which may be the same or different, each independently represent hydrogen, C₁₋₆alkyl, and X², X³, and X⁴, which may be the same or different, each independently represent CR¹² or N.

**4.** A compound as claimed in claim 3, wherein W represents CR¹⁰R¹¹, and R¹⁰ and R¹¹, which may be the same or different, each independently represent hydrogen or C₁₋₆a]kyl, and R¹² represents hydrogen, halogen, cyano, C₁₋₆alkyl, C₁₋₆alkoxycarbonyl, C₁₋₆alkylaminocarbonyl, cycloC₃₋₁₂alkylaminocarbonyl, aryl or heteroaryl.

**5.** A compound as claimed in claim 4, wherein R¹⁰ and R¹¹, which may be the same or different, each independently represent hydrogen or methyl, and R¹² represents hydrogen, halogen, cyano, C₁₋₆alkyl, C₁₋₆alkylcarbonyl, C₁₋₆alkoxycarbonyl, C₁₋₆alkylaminocarbonyl, cycloC₃₋₁₂alkylaminocarbonyl or a heteroaryl group selected from tetrazolyl, furyl, thienyl, pyridinyl, pyrimidinyl, wherein the heteroaryl group may be optionally substituted by one or more groups selected from halogen and C₁₋₆alkyl.

**6.** A compound as claimed in claim 2, wherein A represents 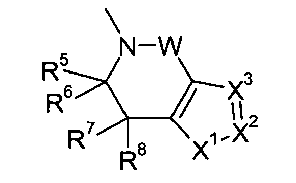 wherein R⁵, R⁶, R⁷, and R⁸ which may be the same or different, each independently represent hydrogen or C₁₋₆alkyl.

**7.** A compound as claimed in claim 6, wherein W represents CR¹⁰R¹¹; X¹ represents CR¹²R¹³, NR¹⁴, S, or O; and X² and X³, which may be the same or different, each independently represent CR¹² or N.

**8.** A compound as claimed in claim 7, wherein R¹⁰ and R¹¹, which may be the same or different, each independently represent hydrogen, C₁₋₆alkyl, or trifluoromethyl; R¹² and R¹³, which may be the same or different, each independently represent hydrogen, halogen, C₁₋₆alkyl, trifluoromethyl, C₁₋₆alkoxy, or aryl; and R¹⁴ represents hydrogen, C₁₋₆alkylcarbonyl, or heteroaryl.

**9.** A compound as claimed in claim 8, wherein R¹⁴ represents tetrazolyl.

**10.** A compound as claimed in claim 6, wherein W represents NR⁹; X¹ represents NR¹⁴, S, or O, and X² and X³, which may be the same or different, each independently represent CR¹² or N.

**11.** A compound as claimed in claim 10, wherein R⁹ represents hydrogen or C₁₋₆alkyl and R¹² represents hydrogen, halogen, or C₁₋₆alkyl.

**12.** A compound as claimed in claim 2, wherein A represents 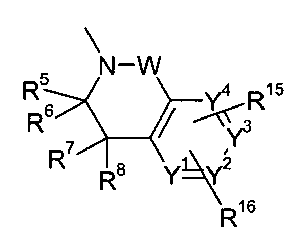 wherein R⁵, R⁶, R⁷, and R⁸ which may be the same or different, each independently represent hydrogen or C₁₋₆alkyl.

**13.** A compound as claimed in claim 12, wherein R⁵, R⁶, R⁷, and R⁸, which may be the same or different, each independently represent hydrogen, methyl, or ethyl.

**14.** A compound as claimed in claim 12, wherein W represents CR¹⁰R¹¹ and R¹⁰ and R¹¹, which may be the same or different, each independently represent hydrogen, C₁₋₆alkyl, trifluromethyl, or cycloC₃₋₁₂alkyl.

**15.** A compound as claimed in claim 14, wherein one of R¹⁰ and R¹¹ represents hydrogen or methyl and the other represents methyl, ethyl, propyl, isopropyl, trifluoromethyl, or cyclohexyl.

**16.** A compound as claimed in any of claims 12 to 15, wherein R¹⁵ and R¹⁶, which may be the same or different, each independently represent hydrogen, hydroxy, halogen, trifluoromethyl, cyano, nitro, C₁₋₆alkyl, C₁₋₆alkoxy, C₁₋₆alkylcarbonylamino, di-C₁₋₆alkylaminocarbonyl, C₁₋₆alkylsulfonyl, C₁₋₆alkoxycarbonyl, C₁₋₆alkylcarbonyl, di-C₁₋₆alkylamino, heterocyclylcarbonyl, heterocycyl, or heteroaryl.

**17.** A compound as claimed in claim 16, wherein R¹⁵ and R¹⁶, which may be the same or different, each independently represent hydrogen, hydroxy, halogen, trifluoromethyl, cyano, nitro, C₁₋₆alkyl, C₁₋₆alkoxy, C₁₋₆alkylcarbonylamino, di-C₁₋₆alkylaminocarbonyl, C₁₋₆alkylsulfonyl, C₁₋₆alkoxycarbonyl, C₁₋₆alkylcarbonyl, di-C₁₋₆alkylamino, heterocyclylcarbonyl, morpholinyl, piperidinyl, pyrrolidinyl, or a heteroaryl group selected from pyridinyl, pyrimidinyl, furyl, isoxazolyl, pyrazolyl, and tetrazolyl, wherein the heteroaryl group may be optionally substituted by one or more groups selected from halogen, C₁₋₆alkyl, and C₁₋₆alkoxy.

**18.** A compound as claimed in any of claims 12 to 15, wherein R¹⁵ and R¹⁶, together with the carbon atoms carrying them, represent a heterocyclyl group having 5 or 6 ring members, which may be optionally substituted by a group selected from oxo, halogen, or C₁₋₆alkyl.

**19.** A compound as claimed in claim 12, wherein W represents NR⁹ and R⁹ represents hydrogen or C₁₋₆alkyl.

**20.** A compound as claimed in claim 19, wherein R⁹ represents hydrogen or methyl.

**21.** A compound as claimed in claim 19 or claim 20, wherein R¹⁵ and R¹⁶, which may be the same or different, each independently represent hydrogen, halogen, trifluoromethyl, C₁₋₆alkyl, C₁₋₆alkoxy, C₁₋₆alkylcarbonylamino, C₁₋₆alkylsulfonyl, or heteroaryl.

**22.** A compound as claimed in claim 1 selected from:
(6-Bromo-pyrazolo[1,5-a]pyridin-2-yl)-(4-methyl-6,7-dihydro-4H-thieno[3,2-c]pyridin-5-yl)-methanone,
(6-Chloro-pyrazolo[1,5-a]pyridin-2-yl)-(4-methyl-6,7-dihydro-4H-thieno[3,2-c]pyridin-5-yl)-methanone,
(6-Chloro-pyrazolo[1,5-a]pyridin-2-yl)-(6,7-dihydro-4H-thieno[3,2-c]pyridin-5-yl)-methanone,
(6-Bromo-pyrazolo[1,5-a]pyridin-2-yl)-(6,7-dihydro-4H-thieno[3,2-c]pyridin-5-yl)-methanone,
(6-Chloro-pyrazolo[1,5-a]pyridin-2-yl)-(4,4-dimethyl-6,7-dihydro-4H-thieno[3,2-c]pyridin-5-yl)-methanone,
(6-Bromo-pyrazolo[1,5-a]pyridin-2-yl)-(4,4-dimethyl-6,7-dihydro-4H-thieno[3,2-c]pyridin-5-yl)-methanone,
(6-Chloro-pyrazolo[1,5-a]pyridin-2-yl)-(6-methyl-6,7-dihydro-4H-thieno[3,2-c]pyridin-5-yl)-methanone,
(6-Bromo-pyrazolo[1,5-a]pyridin-2-yl)-(6-methyl-6,7-dihydro-4H-thieno[3,2-c]pyridin-5-yl)-methanone,
(6-Bromo-pyrazolo[1,5-a]pyridin-2-yl)-(4-ethyl-6,7-dihydro-4H-thieno[3,2-c]pyridin-5-yl)-methanone,
(6-Chloro-pyrazolo[1,5-a]pyridin-2-yl)-(4-ethyl-6,7-dihydro-4H-thieno[3,2-c]pyridin-5-yl)-methanone,
(6-Bromo-pyrazolo[1,5-a]pyridin-2-yl)-(4-trifluoromethyl-6,7-dihydro-4H-thieno[3,2-c]pyridin-5-yl)-methanone,
(6-Chloro-pyrazolo[1,5-a]pyridin-2-yl)-(4-trifluoromethyl-6,7-dihydro-4H-thieno[3,2-c]pyridin-5-yl)-methanone,
6-Bromo-pyrazolo[1,5-a]pyridin-2-yl)-(6,6-dimethyl-6,7-dihydro-4H-thieno[3,2-c]pyridin-5-yl)-methanone,
6-Chloro-pyrazolo[1,5-a]pyridin-2-yl)-(6,6-dimethyl-6,7-dihydro-4H-thieno[3,2-c]pyridin-5-yl)-methanone,
(6-Bromo-pyrazolo[1,5-a]pyridin-2-yl)-(2,4-dimethyl-6,7-dihydro-4H-thieno[3,2-c]pyridin-5-yl)-methanone,
(6-Chloro-pyrazolo[1,5-a]pyridin-2-yl)-(2,4-dimethyl-6,7-dihydro-4H-thieno[3,2-c]pyridin-5-yl)-methanone,
(6-Bromo-pyrazolo[1,5-a]pyridin-2-yl)-(3,4-dimethyl-6,7-dihydro-4H-thieno[3,2-c]pyridin-5-yl)-methanone,
6-Bromo-2-[(2,3,4-trimethyl-6,7-dihydrothieno[3,2-c]pyridin-5(4H)-yl)carbonyl]-pyrazolo[1,5-a]pyridine,
6-Chloro-2-[(2,3,4-trimethyl-6,7-dihydrothieno[3,2-c]pyridin-5(4H)-yl)carbonyl]-pyrazolo[1,5-a]pyridine,
(6-Bromo-pyrazolo[1,5-a]pyridin-2-yl)-(2-fluoro-4-methyl-6,7-dihydro-4H-thieno[3,2-c]pyridin-5-yl)-methanone,
(6-Chloro-pyrazolo[1,5-a]pyridin-2-yl)-(2-fluoro-4-methyl-6,7-dihydro-4H-thieno[3,2-c]pyridin-5-yl)-methanone,
(6-Bromo-pyrazolo[1,5-a]pyridin-2-yl)-(3-fluoro-4-methyl-6,7-dihydro-4H-thieno[3,2-c]pyridin-5-yl)-methanone,
(6-Chloro-pyrazolo[1,5-a]pyridin-2-yl)-(3-fluoro-4-methyl-6,7-dihydro-4H-thieno[3,2-c]pyridin-5-yl)-methanone,
(6-Bromo-pyrazolo[1,5-a]pyridin-2-yl)-(2,3-difluoro-4-methyl-6,7-dihydro-4H-thieno[3,2-c]pyridin-5-yl)-methanone,
(6-Chloro-pyrazolo[1,5-a]pyridin-2-yl)-(2,3-difluoro-4-methyl-6,7-dihydro-4H-thieno [3,2-c]pyridin-5-yl)-methanone,
(6-Bromo-pyrazolo[1,5-a]pyridin-2-yl)-(4-methyl-2-trifluoromethyl-6,7-dihydro-4H-thieno[3,2-c]pyridin-5-yl)-methanone,
(6-Chloro-pyrazolo[1,5-a]pyridin-2-yl)-(4-methyl-2-trifluoromethyl-6,7-dihydro-4H-thieno[3,2-c]pyridin-5-yl)-methanone,
6-Bromo-2-[(4-methyl-6,7-dihydrofuro[3,2-c]pyridin-5(4H)-yl)carbonyl]pyrazolo[1,5-a]pyridine,
6-Chloro-2-[(4-methyl-6,7-dihydrofuro[3,2-c]pyridin-5(4H)-yl)carbonyl]pyrazolo[1,5-a]pyridine,
(6-Chloro-pyrazolo[1,5-a]pyridin-2-yl)-(6,7-dihydro-4H-furo[3,2-c]pyridin-5-yl)-methanone,
(6-Bromo-pyrazolo[1,5-a]pyridin-2-yl)-(6,7-dihydro-4H-furo[3,2-c]pyridin-5-yl)-methanone,
(6-Bromo-pyrazolo[1,5-a]pyridin-2-yl)-(6-methyl-6,7-dihydro-4H-furo[3,2-c]pyridin-5-yl)-methanone,
(6-Chloro-pyrazolo[1,5-a]pyridin-2-yl)-(6-methyl-6,7-dihydro-4H-furo[3,2-c]pyridin-5-yl)-methanone,
(6-Bromo-pyrazolo[1,5-a]pyridin-2-yl)-(1,4,6,7-tetrahydro-pyrrolo[3,2-c]pyridin-5-yl)-Methanone,
(6-Chloro-pyrazolo[1,5-a]pyridin-2-yl)-(1,4,6,7-tetrahydro-pyrrolo[3,2-c]pyridin-5-yl)-methanone,
(6-Chloro-pyrazolo[1,5-a]pyridin-2-yl)-(4-methyl-1,4,6,7-tetrahydro-pyrrolo[3,2-c]pyridin-5-yl)-methanone,
(6-Bromo-pyrazolo[1,5-a]pyridin-2-yl)-(4-methyl-1,4,6,7-tetrahydro-pyrrolo[3,2-c]pyridin-5-yl)-methanone,
(6-Bromo-pyrazolo[1,5-a]pyridin-2-yl)-(1-methyl-1,3,4,5-tetrahydro-2-pyrindin-2-yl)-methanone,
(6-Chloro-pyrazolo[1,5-a]pyridin-2-yl)-(1-methyl-1,3,4,5-tetrahydro-2-pyrindin-2-yl)-methanone,
5-[(6-Bromopyrazolo[1,5-a]pyridin-2-yl)carbonyl]-3-phenyl-4,5,6,7-tetrahydro-isoxazolo[4,5-c]pyridine,
(6-Bromo-pyrazolo[1,5-a]pyridin-2-yl)-(4-methyl-6,7-dihydro-4H-thieno[3,2-c]pyridin-5-yl)-methanone,
(6-Chloro-pyrazolo[1,5-a]pyridin-2-yl)-(2,4-dimethyl-6,7-dihydro-4H-furo[3,2-c]pyridin-5-yl)-methanone,
(6-Bromo-pyrazolo[1,5-a]pyridin-2-yl)-(2,4-dimethyl-6,7-dihydro-4H-furo[3,2-c]pyridin-5-yl)-methanone,
(6-Chloro-pyrazolo[1,5-a]pyridin-2-yl)-(3,4-dimethyl-6,7-dihydro-4H-thieno[3,2-c]pyridin-5-yl)-methanone,
(6-Bromo-pyrazolo[1,5-a]pyridin-2-yl)-(1,7,7-trimethyl-1,3,4,7-tetrahydro-2-pyrindin-2-yl)-methanone,
(6-Chloro-pyrazolo[1,5-a]pyridin-2-yl)-(1,7,7-trimethyl-1,3,4,7-tetrahydro-2-pyrindin-2-yl)-methanone,
(6-Bromo-pyrazolo[1,5-a]pyridin-2-yl)-(1,6,6-trimethyl-1,3,4,6-tetrahydro-2-pyrindin-2-yl)-methanone,
(6-Chloro-pyrazolo[1,5-a]pyridin-2-yl)-(1,6,6-trimethyl-1,3,4,6-tetrahydro-2-pyrindin-2-yl)-methanone,
(6-Bromo-pyrazolo[1,5-a]pyridin-2-yl)-(1,5,5-trimethyl-1,3,4,5-tetrahydro-2-pyrindin-2-yl)-methanone,
(6-Chloro-pyrazolo[1,5-a]pyridin-2-yl)-(1,5,5-trimethyl-1,3,4,5-tetrahydro-2-pyrindin-2-yl)-methanone,
(6-Bromo-pyrazolo[1,5-a]pyridin-2-yl)-[4-methyl-1-(1H-tetrazol-5-yl)-1,4,6,7-tetrahydro-pyrrolo[3,2-c]pyridin-5-yl]-methanone,
(6-Chloro-pyrazolo[1,5-a]pyridin-2-yl)- [4-methyl-1-(1H-tetrazol-5-yl)-1,4,6,7-tetrahydro-pyrrolo[3,2-c]pyridin-5-yl]-methanone,
(6-Bromo-pyrazolo[1,5-a]pyridin-2-yl)-[7-methyl-1-(1H-tetrazol-5-yl)-1,4,5,7-tetrahydro-pyrrolo[2,3-c]pyridin-6-yl]-methanone,
(6-Chloro-pyrazolo[1,5-a]pyridin-2-yl)-[7-methyl-1-(1H-tetrazol-5-yl)-1,4,5,7-tetrahydro-pyrrolo[2,3-c]pyridin-6-yl]-methanone,
(6-Bromo-pyrazolo[1,5-a]pyridin-2-yl)-[4-methyl-1-(1H-tetrazol-5-yl)-2,4,6,7-tetrahydro-pyrrolo[3,4-c]pyridin-5-yl]-methanone,
(6-Chloro-pyrazolo[1,5-a]pyridin-2-yl)-[4-methyl-1-(1H-tetrazol-5-yl)-2,4,6,7-tetrahydro-pyrrolo[3,4-c]pyridin-5-yl]-methanone,
(2-Bromo-7-methyl-4,7-dihydro-5H-furo[2,3-c]pyridin-6-yl)-(6-bromo-pyrazolo[1,5-a]pyridin-2-yl)-methanone,
(2-Chloro-7-methyl-4,7-dihydro-5H-furo[2,3-c]pyridin-6-yl)-(6-bromo-pyrazolo[1,5-a]pyridin-2-yl)-methanone,
2-Bromo-7-methyl-4,7-dihydro-5H-furo[2,3-c]pyridin-6-yl)-(6-chloro-pyrazolo[1,5-a]pyridin-2-yl)-methanone,
2-Chloro-7-methyl-4,7-dihydro-5H-furo[2,3-c]pyridin-6-yl)-(6-chloro-pyrazolo[1,5-a]pyridin-2-yl)-methanone,
(2-Bromo-7-methyl-4,7-dihydro-5H-thieno[2,3-c]pyridin-6-yl)-(6-bromo-pyrazolo[1,5-a]pyridin-2-yl)-methanone,
(2-Chloro-7-methyl-4,7-dihydro-5H-thieno[2,3-c]pyridin-6-yl)-(6-bromo-pyrazolo[1,5-a]pyridin-2-yl)-methanone,
(2-Bromo-7-methyl-4,7-dihydro-5H-thieno[2,3-c]pyridin-6-yl)-(6-chloro-pyrazolo[1,5-a]pyridin-2-yl)-methanone,
(2-Chloro-7-methyl-4,7-dihydro-5H-thieno[2,3-c]pyridin-6-yl)-(6-chloro-pyrazolo[1,5-a]pyridin-2-yl)-methanone,
(3-Bromo-7-methyl-4,7-dihydro-5H-furo[2,3-c]pyridin-6-yl)-(6-bromo-pyrazolo[1,5-a]pyridin-2-yl)-methanone,
(3-Chloro-7-methyl-4,7-dihydro-5H-furo[2,3-c]pyridin-6-yl)-(6-bromo-pyrazolo[1,5-a]pyridin-2-yl)-methanone,
(3-Bromo-7-methyl-4,7-dihydro-5H-furo[2,3-c]pyridin-6-yl)-(6-chloro-pyrazolo[1,5-a]pyridin-2-yl)-methanone,
(3-Chloro-7-methyl-4,7-dihydro-5H-furo[2,3-c]pyridin-6-yl)-(6-chloro-pyrazolo[1,5-a]pyridin-2-yl)-methanone,
(3-Bromo-7-methyl-4,7-dihydro-5H-thieno[2,3-c]pyridin-6-yl)-(6-bromo-pyrazolo[1,5-a]pyridin-2-yl)-methanone,
(3-Chloro-7-methyl-4,7-dihydro-5H-thieno[2,3-c]pyridin-6-yl)-(6-bromo-pyrazolo[1,5-a]pyridin-2-yl)-methanone,
(3-Bromo-7-methyl-4,7-dihydro-5H-thieno[2,3-c]pyridin-6-yl)-(6-chloro-pyrazolo[1,5-a]pyridin-2-yl)-methanone,
(3-Chloro-7-methyl-4,7-dihydro-5H-thieno[2,3-c]pyridin-6-yl)-(6-chloro-pyrazolo[1,5-a]pyridin-2-yl)-methanone,
1-[5-(6-Bromo-pyrazolo[1,5-a]pyridine-2-carbonyl)-4-methyl-4,5,6,7-tetrahydro-pyrrolo[3,2-c]pyridin-1-yl]-ethanone,
1-[5-(6-Chloro-pyrazolo[1,5-a]pyridine-2-carbonyl)-4-methyl-4,5,6,7-tetrahydro-pyrrolo[3,2-c]pyridin-1-yl]-ethanone,
1-[6-(6-Bromo-pyrazolo[1,5-a]pyridine-2-carbonyl)-7-methyl-4,5,6,7-tetrahydro-pyrrolo[2,3-c]pyridin-1-yl]-ethanone,
1-[6-(6-Chloro-pyrazolo[1,5-a]pyridine-2-carbonyl)-7-methyl-4,5,6,7-tetrahydro-pyrrolo[2,3-c]pyridin-1-yl]-ethanone,
(6-Bromo-pyrazolo[1,5-a]pyridin-2-yl)-(4-methyl-6,7-dihydro-4H-oxazolo[4,5-c]pyridin-5-yl)-methanone,
6-Bromo-pyrazolo[1,5-a]pyridine-2-carboxylic acid (1-phenyl-ethyl)-amide,
6-Chloro-pyrazolo[1,5-a]pyridine-2-carboxylic acid (1-phenyl-ethyl)-amide,
(6-Bromo-pyrazolo[1,5-a]pyridin-2-yl)-(2,4-dimethyl-6,7-dihydro-4H-oxazolo[4,5-c]pyridin-5-yl)-methanone,
(6-Bromo-pyrazolo[1,5-a]pyridin-2-yl)-(4-methyl-2-trifluoromethyl-6,7-dihydro-4H-oxazolo[4,5-c]pyridin-5-yl)-methanone,
(6-Bromo-pyrazolo[1,5-a]pyridin-2-yl)-(2-methoxy-4-methyl-6,7-dihydro-4H-oxazolo[4,5-c]pyridin-5-yl)-methanone,
(6-Bromo-pyrazolo[1,5-a]pyridin-2-yl)-(6-hydroxy-7-methoxy-1-methyl-3,4-dihydro-1H-isoquinolin-2-yl)-methanone,
(6-Chloro-pyrazolo[1,5-a]pyridin-2-yl)-(1-methyl-3,4-dihydro-1H-isoquinolin-2-yl)-methanone,
(6-Chloro-pyrazolo[1,5-a]pyridin-2-yl)-(3-methyl-3,4-dihydro-1H-isoquinolin-2-yl)-methanone,
(6-Bromo-pyrazolo[1,5-a]pyridin-2-yl)-(3-methyl-3,4-dihydro-1H-isoquinolin-2-yl)-methanone,
(6-Bromo-pyrazolo[1,5-a]pyridin-2-yl)-(3,3-dimethyl-3,4-dihydro-1H-isoquinolin-2-yl)-methanone,
(6-Chloro-pyrazolo[1,5-a]pyridin-2-yl)-(3,3-dimethyl-3,4-dihydro-1H-isoquinolin-2-yl)-methanone,
(6-Chloro-pyrazolo[1,5-a]pyridin-2-yl)-(3,4-dihydro-1H-isoquinolin-2-yl)-methanone,
(6-Chloro-pyrazolo[1,5-a]pyridin-2-yl)-(6,7-dimethoxy-1-methyl-3,4-dihydro-1H-isoquinolin-2-yl)-methanone,
(6-Chloro-pyrazolo[1,5-a]pyridin-2-yl)-(6,7-dimethoxy-3,4-dihydro-1H-isoquinolin-2-yl)-methanone,
(6-Bromo-pyrazolo[1,5-a]pyridin-2-yl)-(6,7-dimethoxy-3,4-dihydro-1H-isoquinolin-2-yl)-methanone,
(6-Bromo-pyrazolo[1,5-a]pyridin-2-yl)-(6,7-dimethoxy-1-methyl-3,4-dihydro-1H-isoquinolin-2-yl)-methanone,
(6-Bromo-pyrazolo[1,5-a]pyridin-2-yl)-(6-hydroxy-7-methoxy-3,4-dihydro-1H-isoquinolin-2-yl)-methanone,
(6-Chloro-pyrazolo[1,5-a]pyridin-2-yl)-(6-hydroxy-7-methoxy-3,4-dihydro-1H-isoquinolin-2-yl)-methanone,
(6-Chloro-pyrazolo[1,5-a]pyridin-2-yl)-(6-hydroxy-7-methoxy-1-methyl-3,4-dihydro-1H-isoquinolin-2-yl)-methanone,
(6-Bromo-pyrazolo[1,5-a]pyridin-2-yl)-(6,7-dimethoxy-3-methyl-3,4-dihydro-1H-isoquinolin-2-yl)-methanone,
(6-Chloro-pyrazolo[1,5-a]pyridin-2-yl)-(6,7-dimethoxy-3-methyl-3,4-dihydro-1H-isoquinolin-2-yl)-methanone,
(6-Bromo-pyrazolo[1,5-a]pyridin-2-yl)-(6,7-dimethoxy-3,3-dimethyl-3,4-dihydro-1H-isoquinolin-2-yl)-methanone,
(6-Chloro-pyrazolo[1,5-a]pyridin-2-yl)-(6,7-dimethoxy-3,3-dimethyl-3,4-dihydro-1H-isoquinolin-2-yl)-methanone,
(6-Bromo-pyrazolo[1,5-a]pyridin-2-yl)-(8-methyl-5,8-dihydro-6H-[1,7]naphthyridin-7-yl)-methanone,
(6-Chloro-pyrazolo[1,5-a]pyridin-2-yl)-(8-methyl-5,8-dihydro-6H-[1,7]naphthyridin-7-yl)-methanone,
(6-Bromo-pyrazolo[1,5-a]pyridin-2-yl)-(1-methyl-3,4-dihydro-1H-[2,7]naphthyridin-2-yl)-methanone,
(6-Chloro-pyrazolo[1,5-a]pyridin-2-yl)-(1-methyl-3,4-dihydro-1H-[2,7]naphthyridin-2-yl)-methanone,
(6-Bromo-pyrazolo[1,5-a]pyridin-2-yl)-(1-methyl-3,4-dihydro-1H-[2,6]naphthyridin-2-yl)-methanone,
(6-Chloro-pyrazolo[1,5-a]pyridin-2-yl)-(1-methyl-3,4-dihydro-1H-[2,6]naphthyridin-2-yl)-methanone,
(6-Bromo-pyrazolo[1,5-a]pyridin-2-yl)-(5-methyl-7,8-dihydro-5H-[1,6]naphthyridin-6-yl)-methanone,
(6-Chloro-pyrazolo[1,5-a]pyridin-2-yl)-(5-methyl-7,8-dihydro-5H-[1,6]naphthyridin-6-yl)-methanone,
(6-Bromo-pyrazolo[1,5-a]pyridin-2-yl)-(1-methyl-3,4-dihydro-1H-isoquinolin-2-yl)-methanone,
(6-Bromo-pyrazolo[1,5-a]pyridin-2-yl)-(3,4-dihydro-1H-isoquinolin-2-yl)-methanone,
(6-Bromo-pyrazolo[1,5-a]pyridin-2-yl)-(7-fluoro-1-methyl-3,4-dihydro-1H-isoquinolin-2-yl)-methanone,
(6-Bromo-pyrazolo[1,5-a]pyridin-2-yl)-(8-fluoro-1-methyl-3,4-dihydro-1H-isoquinolin-2-yl)-methanone,
(6-Bromo-pyrazolo[1,5-a]pyridin-2-yl)-(6-fluoro-l-methyl-3,4-dihydro-1H-isoquinolin-2-yl)-methanone,
(6-Bromo-pyrazolo[1,5-a]pyridin-2-yl)-(5-fluoro-1-methyl-3,4-dihydro-1H-isoquinolin-2-yl)-methanone,
(6-Chloro-pyrazolo[1,5-a]pyridin-2-yl)-(7-fluoro-1-methyl-3,4-dihydro-1H-isoquinolin-2-yl)-methanone,
(6-Chloro-pyrazolo[1,5-a]pyridin-2-yl)-(8-fluoro-1-methyl-3,4-dihydro-1H-isoquinolin-2-yl)-methanone,
(6-Chloro-pyrazolo[1,5-a]pyridin-2-yl)-(6-fluoro-1-methyl-3,4-dihydro-1H-isoquinolin-2-yl)-methanone,
(6-Chloro-pyrazolo[1,5-a]pyridin-2-yl)-(5-fluoro-1-methyl-3,4-dihydro-1H-isoquinolin-2-yl)-methanone,
(6-Bromo-pyrazolo[1,5-a]pyridin-2-yl)-(8-fluoro-1-ethyl-3,4-dihydro-1H-isoquinolin-2-yl)-methanone,
(6-Bromo-pyrazolo[1,5-a]pyridin-2-yl)-(7-fluoro-1-ethyl-3,4-dihydro-1H-isoquinolin-2-yl)-methanone,
(6-Bromo-pyrazolo[1,5-a]pyridin-2-yl)-(6-fluoro-1-ethyl-3,4-dihydro-1H-isoquinolin-2-yl)-methanone,
(6-Bromo-pyrazolo[1,5-a]pyridin-2-yl)-(5-fluoro-1-ethyl-3,4-dihydro-1H-isoquinolin-2-yl)-methanone,
(6-Chloro-pyrazolo[1,5-a]pyridin-2-yl)-(8-fluoro-1-ethyl-3,4-dihydro-1H-isoquinolin-2-yl)-methanone,
(6-Chloro-pyrazolo[1,5-a]pyridin-2-yl)-(7-fluoro-1-ethyl-3,4-dihydro-1H-isoquinolin-2-yl)-methanone,
(6-Chloro-pyrazolo[1,5-a]pyridin-2-yl)-(6-fluoro-1-ethyl-3,4-dihydro-1H-isoquinolin-2-yl)-methanone,
(6-Chloro-pyrazolo[1,5-a]pyridin-2-yl)-(5-fluoro-1-ethyl-3,4-dihydro-1H-isoquinolin-2-yl)-methanone,
(6-Bromo-pyrazolo[1,5-a]pyridin-2-yl)-(8-fluoro-1-trifluoromethyl-3,4-dihydro-1H-isoquinolin-2-yl)-methanone,
(6-Bromo-pyrazolo[1,5-a]pyridin-2-yl)-(7-fluoro-1-trifluoromethyl-3,4-dihydro-1H-isoquinolin-2-yl)-methanone,
(6-Bromo-pyrazolo[1,5-a]pyridin-2-yl)-(6-fluoro-1-trifluoromethyl-3,4-dihydro-1H-isoquinolin-2-yl)-methanone,
(6-Bromo-pyrazolo[1,5-a]pyridin-2-yl)-(5-fluoro-1-trifluoromethyl-3,4-dihydro-1H-isoquinolin-2-yl)-methanone,
(6-Chloro-pyrazolo[1,5-a]pyridin-2-yl)-(8-fluoro-1-trifluoromethyl-3,4-dihydro-1H-isoquinolin-2-yl)-methanone,
(6-Chloro-pyrazolo[1,5-a]pyridin-2-yl)-(7-fluoro-1-trifluoromethyl-3,4-dihydro-1H-isoquinolin-2-yl)-methanone,
(6-Chloro-pyrazolo[1,5-a]pyridin-2-yl)-(6-fluoro-1-trifluoromethyl-3,4-dihydro-1H-isoquinolin-2-yl)-methanone,
(6-Chloro-pyrazolo[1,5-a]pyridin-2-yl)-(5-fluoro-1-trifluoromethyl-3,4-dihydro-1H-isoquinolin-2-yl)-methanone,
(6-Bromo-pyrazolo[1,5-a]pyridin-2yl)-(3-ethyl-1-methyl-3,4-dihydro-1H-isoquinolin-2-yl)methanone,
(6-Bromo-pyrazolo[1,5-a]pyridin-2yl)-((R)-3-methyl-3,4-dihydro-1H-isoquinolin-2-yl)-methanone,
(6-Bromo-pyrazolo[1,5-a]pyridin-2yl)-((S)-3-methyl-3,4-dihydro-1H-isoquinolin-2-yl)-methanone,
(6-Chloro-pyrazolo[1,5-a]pyridin-2-yl)-(1-(S)-methyl-3,4-dihydro-1H-isoquinolin-2-yl)-methanone,
(7-Bromo-3,4-dihydro-1H-isoquinolin-2-yl)-(6-chloro-pyrazolo[1,5-a]pyridin-2-yl)-methanone,
(7-Bromo-3,4-dihydro-1H-isoquinolin-2-yl)-(6-bromo-pyrazolo[1,5-a]pyridin-2-yl)-methanone,
(6-Chloro-pyrazolo[1,5-a]pyridin-2-yl)-(7-fluoro-1-methyl-3,4-dihydro-1H-isoquinolin-2-yl)-methanone,
(6-Chloro-pyrazolo[1,5-a]pyridin-2-yl)-(1,4-dimethyl-3,4-dihydro-1H-isoquinolin-2-yl)-methanone,
(6-Bromo-pyrazolo[1,5-a]pyridin-2-yl)-(1,4-dimethyl-3,4-dihydro-1H-isoquinolin-2-yl)-methanone,
(6-Bromo-pyrazolo[1,5-a]pyridin-2-yl)-((1R,4S)-1,4-dimethyl-3,4-dihydro-1H-isoquinolin-2-yl)-methanone,
(6-Bromo-pyrazolo[1,5-a]pyridin-2-yl)-((1S,4S)-1,4-dimethyl-3,4-dihydro-1H-isoquinolin-2-yl)-methanone,
(6-Bromo-pyrazolo[1,5-a]pyridin-2-yl)-((1S,4R)-1,4-dimethyl-3,4-dihydro-1H-isoquinolin-2-yl)-methanone,
(6-Bromo-pyrazolo[11,5-a]pyridin-2-yl)-((1R,4R)-1,4-dimethyl-3,4-dihydro-1H-isoquinolin-2-yl)-methanone,
(6-Bromo-pyrazolo[1,5-a]pyridin-2-yl)-(6-methoxy-1-methyl-3,4-dihydro-1H-isoquinolin-2-yl)-methanone,
(6-Chloro-pyrazolo[1,5-a]pyridin-2-yl)-(6-methoxy-1-methyl-3,4-dihydro-1H-isoquinolin-2-yl)-methanone,
(6-Bromopyrazolo[1,5-a]pyridin-2yl)-(1-ethyl-3,4-dihydro-1H-isoquinolin-2-yl)methanone,
(6-Bromopyrazolo[1,5-a]pyridin-2yl)-(1-isopropyl-3,4-dihydro-1H-isoquinolin-2-yl)methanone,
(6-Chloro-pyrazolo[1,5-a]pyridin-2-yl)-(1-isopropyl-3,4-dihydro-1H-isoquinolin-2-yl)-methanone,
(6-Chloro-pyrazolo[1,5-a]pyridin-2-yl)-(1-ethyl-3,4-dihydro-1H-isoquinolin-2-yl)-methanone,
(6-Bromo-pyrazolo[1,5-a]pyridin-2-yl)-(1-trifluoromethyl-3,4-dihydro-1H-isoquinolin-2-yl)-methanone,
(6-Choro-pyrazolo[1,5-a]pyridin-2-yl)-(1-trifluoromethyl-3,4-dihydro-1H-isoquinolin-2-yl)-methanone,
(7-Bromo-1-methyl-3,4-dihydro-1H-isoquinolin-2-yl)-(6-bromo-pyrazolo[1,5-a]pyridin-2-yl)-methanone,
(7-Bromo-1-methyl-3,4-dihydro-1H-isoquinolin-2-yl)-(6-chloro-pyrazolo[1,5-a]pyridin-2-yl)-methanone,
(6-Bromo-pyrazolo[1,5-a]pyridin-2-yl)-(1-cyclohexyl-3,4-dihydro-1H-isoquinolin-2-yl)-methanone,
(6-Chloro-pyrazolo[1,5-a]pyridin-2-yl)-(1-cyclohexyl-3,4-dihydro-1H-isoquinolin-2-yl)-methanone,
(6-Bromo-pyrazolo[1,5-a]pyridin-2-yl)-(1-propyl-3,4-dihydro-1H-isoquinnolin-2yl)-methanone,
(6-Bromo-pyrazolo[1,5-a]pyridin-2-yl)-((S)1-methyl-3,4-dihydro-1H-isoquinolin-2yl)-methanone,
(6-Bromo-pyrazolo[1,5-a]pyridin-2-yl)-((R)1-methyl-3,4-dihydro-1H-isoquinolin-2yl)-methanone,
(5-Bromo-1-methyl-3,4-dihydro-1H-isoquinolin-2-yl)-(6-chloro-pyrazolo[1,5-a]pyridin-2yl)-methanone,
(6-Bromo-pyrazolo[1,5-a]pyridin-2-yl)-5-methoxy-1-methyl-3,4-dihydro-1H-isoquinolin-2-yl)-methanone,
(5-Bromo-1-methyl-3,4-dihydro-1H-isoquinolin-2-yl)-(6-bromo-pyrazolo[1,5-a]pyridin-2-yl)-methanone,
(6-Bromo-pyrazolo[1,5-a]pyridin-2-yl)-(7-pyridin-4-yl-3,4-dihydro-1H-isoquinolin-2-yl)-methanone,
(6-Bromo-pyrazolo[1,5-a]pyridin-2-yl)-(7-methoxy-1-methyl-3,4-dihydro-1H-isoquinolin-2-yl)-methanone,
(6-Chloro-pyrazolo[1,5-a]pyridin-2-yl)-(7-methoxy-1-methyl-3,4-dihydro-1H-isoquinolin-2-yl)-methanone,
(6-Bromo-1-methyl-3,4-dihydro-1H-isoquinolin-2yl)-(6-bromo-pyrazolo[1,5-a]pyridin-2-yl)-methanone,
(6-Bromo-pyrazolo[1,5-a]pyridin-2-yl)-(5,6-dimethoxy-1-methyl-3,4-dihydro-1H-isoquinolin-2-yl)-methanone,
(6-Bromo-pyrazolo[1,5-a]pyridin-2-yl)-(5,8-difluoro-1-methyl-3,4-dihydro-1H-isoquinolin-2-yl)-methanone,
(6-Chloro-pyrazolo[1,5-a]pyridin-2-yl)-(5,8-difluoro-1-methyl-3,4-dihydro-1H-isoquinolin-2-yl)-methanone,
(6-Bromopyrazolo[1,5-a]pyridin-2-yl)-(1-methyl-5-trifluoromethyl-3,4-dihydro-1H-isoquinolin-2-yl)-methanone,
(6-Chloro-pyrazolo[1,5-a]pyridin-2-yl)-(1-methyl-5-trifluoromethyl-3,4-dihydro-1H-isoquinolin-2-yl)-methanone,
(6-Chloro-pyrazolo[1,5-a]pyridin-2-yl)-(5-methyl-7,8-dihydro-5H-[1,6]naphthyridin-6-yl)-methanone,
(3-Bromo-7,8-dihydro-5H-[1,6]naphthyridin-6-yl)-(6-chloro-pyrazolo[1,5-a]pyridin-2-yl)-methanone,
(6-Bromo-pyrazolo[1,5-a]pyridin-2-yl)-(l-methyl-6-pyridin-3-yl-3,4-dihydro-1H-isoquinolin-2-yl)-methanone,
(3-Bromo-7,8-dihydro-5H-[1,6]naphthyridin-6-yl)-(6-bromo-pyrazolo[1,5-a]pyridin-2-yl)-methanone,
(6-Bromo-pyrazolo[1,5-a]pyridin-2-yl)-(1-methyl-7-pyridin-3-yl-3,4-dihydro-1H-isoquinolin-2-yl)-methanone,
2-(6-Bromo-pyrazolo[1,5-a]pyridine-2-carbonyl-)1,2,3,4-tetrahydro-isoquinoline-7-carbonitrile,
(6-Bromo-pyrazolo[1,5-a]pyridin-2-yl)-(1-methyl-7-pyridin-4-yl-3,4-dihydro-1H-isoquinolin-2-yl)-methanone,
(6-Bromo-pyrazolo[1,5-a]pyridin-2-yl)-(5-nitro-3,4-dihydro-1H-isoquinolin-2-yl)-methanone,
(6-Chloro-pyrazolo[1,5-a]pyridin-2-yl)-(5-nitro-3,4-dihydro-1H-isoquinolin-2-yl)-methanone,
(6-Bromo-pyrazolo[1,5-a]pyridin-2-yl)-(1-methyl-7-pyrimidin-5-yl-3,4-dihydro-1H-isoquinolin-2-yl)-methanone,
(6-Bromo-pyrazolo[1,5-a]pyridin-2-yl)-(7-morpholin-4-yl-3,4-dihydro-1H-isoquinolin-2-yl)-methanone,
(6-Chloro-pyrazolo[1,5-a]pyridin-2-yl)-(7-morpholin-4-yl-3,4-dihydro-1H-isoquinolin-2-yl)-methanone,
(6-Bromo-pyrazolo[1,5-a]pyridin-2-yl)-(8-morpholin-4-yl-3,4-dihydro-1H-isoquinolin-2-yl)-methanone,
(6-Chloro-pyrazolo[1,5-a]pyridin-2-yl)-(8-morpholin-4-yl-3,4-dihydro-1H-isoquinolin-2-yl)-methanone,
(6-Bromo-pyrazolo[1,5-a]pyridin-2-yl)-(6-morpholin-4-yl-3,4-dihydro-1H-isoquinolin-2-yl)-methanone,
(6-Chloro-pyrazolo[1,5-a]pyridin-2-yl)-(6-morpholin-4-yl-3,4-dihydro-1H-isoquinolin-2-yl)-methanone,
(6-Bromo-pyrazolo[1,5-a]pyridin-2-yl)-(5-morpholin-4-yl-3,4-dihydro-1H-isoquinolin-2-yl)-methanone,
(6-Chloro-pyrazolo[1,5-a]pyridm-2-yl)-(5-morpholin-4-yl-3,4-dihydro-1H-isoquinolin-2-yl)-methanone,
(6-Bromo-pyrazolo[1,5-a]pyridin-2-yl)-(1-methyl-6-pyrimidin-5-yl-3,4-dihydro-1H-isoquinolin-2-yl)-methanone,
(6-Bromo-pyrazolo[1,5-a]pyridin-2-yl)-(1-methyl-5-pyrimidin-5-yl-3,4-dihydro-1H-isoquinolin-2-yl)-methanone,
(6-Bromo-pyrazolo[1,5-a]pyridin-2-yl)-(1-methyl-5-pyridin-3-yl-3,4-dihydro-1H-isoquinolin-2-yl)-methanone,
(3-Bromo-7,8-dihydro-5H-[1,6]naphthyridin-6-yl)-(6-bromo-pyrazolo[1,5-a]pyridin-2-yl)-methanone,
(6-Chloro-pyrazolo[1,5-a]pyridin-2-yl)-(1-methyl-7-trifluoromethyl-3,4-dihydro-1H-isoquinolin-2-yl)-methanone,
(6-Bromo-pyrazolo[1,5-a]pyridin-2-yl)-(1-methyl-7-trifluoromethyl-3,4-dihydro-1H-isoquinolin-2-yl)-methanone,
(6-Chloro-pyrazolo[1,5-a]pyridin-2-yl)-(1,5-dimethyl-3,4-dihydro-1H-isoquinolin-2-yl)-methanone,
(6-Chloro-pyrazolo[1,5-a]pyridin-2-yl)-(1,7-dimethyl-3,4-dihydro-1H-isoquinolin-2-yl)-methanone,
(6-Bromo-pyrazolo[1,5-a]pyridin-2-yl)-(1,7-dimethyl-3,4-dihydro-1H-isoquinolin-2-yl)-methanone,
(6-Bromo-pyrazolo[1,5-a]pyridin-2-yl)-(1,5-dimethyl-3,4-dihydro-1H-isoquinolin-2-yl)-methanone,
(7-Chloro-1-methyl-3,4-dihydro-1H-isoquinolin-2-yl)-(6-chloro-pyrazolo[1,5-a]pyridin-2-yl)-methanone,
(6-Bromo-pyrazolo[1,5-a]pyridin-2-yl)-(7-chloro-1-methyl-3,4-dihydro-1H-isoquinolin-2-yl)-methanone,
(5-Chloro-1-methyl-3,4-dihydro-1H-isoquinolin-2-yl)-(6-chloro-pyrazolo[1,5-a]pyridin-2-yl)-methanone,
N-[2-(6-Chloro-pyrazolo[1,5-a]pyridine-2-carbonyl)-1,2,3,4-tetrahydro-isoquinolin-5-yl]-acetamide,
N-[2-(6-Bromo-pyrazolo[1,5-a]pyridine-2-carbonyl)-1,2,3,4-tetrahydro-isoquinolin-5-yl]-acetamide,
N-[2-(6-Chloro-pyrazolo[1,5-a]pyridine-2-carbonyl)-1-methyl-1,2,3,4-tetrahydro-isoquinolin-5-yl]-acetamide,
N-[2-(6-Bromo-pyrazolo[1,5-a]pyridine-2-carbonyl)-1-methyl-1,2,3,4-tetrahydro-isoquinolin-5-yl]-acetamide,
N-[2-(6-Coro-pyrazolo[1,5-a]pyridine-2-carbonyl)-1-methyl-1,2,3,4-tetrahydro-isoquinolin-5-yl]-acetamide,
2-(6-Bromo-pyrazolo[1,5-a]pyridine-2-carbonyl)-1-methyl-1,2,3,4-tetrahydro-isoquinoline-5-carboxylic acid dimethylamide,
2-(6-Chloro-pyrazolo[1,5-a]pyridine-2-carbonyl)-1-methyl-1,2,3,4-tetrahydro-isoquinoline-5-carboxylic acid dimethylamide,
(6-Bromo-pyrazolo[1,5-a]pyridin-2-yl)-(8-methyl-2,3,5,8-tetrahydro-6H-1,4-dioxa-7-azaphenanthren-7-yl)-methanone,
2-(6-Bromo-pyrazolo[1,5-a]pyridine-2-carbonyl)-1,2,3,4-tetrahydro-isoquinoline-5-carbonitrile,
2-(6-Bromo-pyrazolo[1,5-a]pyridine-2-carbonyl)-1,2,3,4-tetrahydro-isoquinoline-7-carbonitrile,
(6-Bromo-pyrazolo[1,5-a]pyridin-2-yl)-(6-pyridin-4-yl-3,4-dihydro-1H-isoquinolin-2-yl)-methanone,
(6-Chloro-pyrazolo[1,5-a]pyridin-2-yl)-(6-pyridin-4-yl-3,4-dihydro-1H-isoquinolin-2-yl)-methanone,
(6-Chloro-pyrazolo[1,5-a]pyridin-2-yl)-(1-methyl-6-pyridin-4-yl-3,4-dihydro-1H-isoquinolin-2-yl)-methanone,
(6-Bromo-pyrazolo[1,5-a]pyridin-2-yl)-(1-methyl-6-pyridin-4-yl-3,4-dihydro-1H-isoquinolin-2-yl)-methanone,
(6-Bromo-pyrazolo[1,5-a]pyridin-2-yl)-(6-methyl-8,9-dihydro-6H-[1,3]dioxolo[4,5-f]isoquinolin-7-yl)-methanone,
(6-Bromo-pyrazolo[1,5-a]pyridin-2-yl)-(1-methyl-5-pyridin-4-yl-3,4-dihydro-1H-isoquinolin-2-yl)-methanone,
(6-Chloro-pyrazolo[1,5-a]pyridin-2-yl)-(1-methyl-5-pyridin-4-yl-3,4-dihydro-1H-isoquinolin-2-yl)-methanone,
(6-Bromo-3,4-dihydro-1H-isoquinolin-2-yl)-(6-bromo-pyrazolo[1,5-a]pyridin-2-yl)-methanone,
(6-Bromo-pyrazolo[1,5-a]pyridin-2-yl)-(6-pyridin-2-yl-3,4-dihydro-1H-isoquinolin-2-yl)-methanone,
(6-Bromo-pyrazolo[1,5-a]pyridin-2-yl)-(1-methyl-6-pyridin-2-yl-3,4-dihydro-1H-isoquinolin-2-yl)-methanone,
(6-Chloro-pyrazolo[1,5-a]pyridin-2-yl)-(1-methyl-6-pyridin-2-yl-3,4-dihydro-1H-isoquinolin-2-yl)-methanone,
(6-Chloro-pyrazolo[1,5-a]pyridin-2-yl)-(1-methyl-7-pyridin-2-yl-3,4-dihydro-1H-isoquinolin-2-yl)-methanone,
(5-Bromo-3,4-dihydro-1H-isoquinolin-2-yl)-(6-bromo-pyrazolo[1,5-a]pyridin-2-yl)-methanone,
(5-Bromo-3,4-dihydro-1H-isoquinolin-2-yl)-(6-chloro-pyrazolo[1,5-a]pyridin-2-yl)-methanone,
(6-Bromo-pyrazolo[1,5-a]pyridin-2-yl)-(5-pyridin-2-yl-3,4-dihydro-1H-isoquinolin-2-yl)-methanone,
(6-Bromo-pyrazolo[1,5-a]pyridin-2-yl)-[5-(2,4-dimethoxy-pyrimidin-5-yl)-1-methyl-3,4-dihydro-1H-isoquinolin-2-yl]-methanone,
(6-Chloro-pyrazolo[1,5-a]pyridin-2-yl)-[5-(2,4-dimethoxy-pyrimidin-5-yl)-1-methyl-3,4-dihydro-1H-isoquinolin-2-yl]-methanone,
(6-Bromo-pyrazolo[1,5-a]pyridin-2-yl)-[5-(2,6-dimethoxy-pyridin-3-yl)-1-methyl-3,4-dihydro-1H-isoquinolin-2-yl]-methanone,
(6-Chloro-pyrazolo[1,5-a]pyridin-2-yl)-[5-(2,6-dimethoxy-pyridin-3-yl)-1-methyl-3,4-dihydro-1H-isoquinolin-2-yl]-methanone,
(6-Bromo-pyrazolo[1,5-a]pyridin-2-yl)-[5-(6-fluoro-pyridin-3-yl)-1-methyl-3,4-dihydro-1H-isoquinolin-2-yl]-methanone,
(6-Chloro-pyrazolo[1,5-a]pyridin-2-yl)-[5-(6-fluoro-pyridin-3-yl)-1-methyl-3,4-dihydro-1H-isoquinolin-2-yl]-methanone,
(6-Bromo-pyrazolo[1,5-a]pyridin-2-yl)-[5-(2-methoxy-pyridin-3-yl)-1-methyl-3,4-dihydro-1H-isoquinolin-2-yl]-methanone,
(6-Chloro-pyrazolo[1,5-a]pyridin-2-yl)-[5-(2-methoxy-pyridin-3-yl)-1-methyl-3,4-dihydro-1H-isoquinolin-2-yl]-methanone,
(6-Chloro-pyrazolo[1,5-a]pyridin-2-yl)-[5-(6-methoxy-pyridin-3-yl)-1-methyl-3,4-dihydro-1H-isoquinolin-2-yl]-methanone,
(6-Bromo-pyrazolo[1,5-a]pyridin-2-yl)-[5-(6-methoxy-pyridin-3-yl)-1-methyl-3,4-dihydro-1H-isoquinolin-2-yl]-methanone,
(6-Bromo-pyrazolo[1,5-a]pyridin-2-yl)-[6-(2-methoxy-pyridin-3-yl)-1-methyl-3,4-dihydro-1H-isoquinolin-2-yl]-methanone,
(6-Chloro-pyrazolo[1,5-a]pyridin-2-yl)-[6-(2-methoxy-pyridin-3-yl)-1-methyl-3,4-dihydro-1H-isoquinolin-2-yl]-methanone,
(6-Bromo-pyrazolo[1,5-a]pyridin-2-yl)-[6-(2-fluoro-pyridin-3-yl)-1-methyl-3,4-dihydro-1H-isoquinolin-2-yl]-methanone,
(6-Chloro-pyrazolo[1,5-a]pyridin-2-yl)-[6-(2-fluoro-pyridin-3-yl)-1-methyl-3,4-dihydro-1H-isoquinolin-2-yl]-methanone,
(6-Bromo-pyrazolo[1,5-a]pyridin-2-yl)-[6-(6-fluoro-pyridin-3-yl)-1-methyl-3,4-dihydro-1H-isoquinolin-2-yl]-methanone,
(6-Chloro-pyrazolo[1,5-a]pyridin-2-yl)-[6-(6-fluoro-pyridin-3-yl)-1-methyl-3,4-dihydro-1H-isoquinolin-2-yl]-methanone,
(6-Bromo-pyrazolo[1,5-a]pyridin-2-yl)-[6-(2,6-dimethoxy-pyridin-3-yl)-1-methyl-3,4-dihydro-1H-isoquinolin-2-yl]-methanone,
(6-Chloro-pyrazolo[1,5-a]pyridin-2-yl)-[6-(2,6-dimethoxy-pyridin-3-yl)-1-methyl-3,4-dihydro-1H-isoquinolin-2-yl]-methanone,
(6-Bromo-pyrazolo[1,5-a]pyridin-2-yl)-[6-(2,4-dimethoxy-pyrimidin-5-yl)-1-methyl-3,4-dihydro-1H-isoquinolin-2-yl]-methanone,
(6-Chloro-pyrazolo[1,5-a]pyridin-2-yl)-[6-(2,4-dimethoxy-pyrimidin-5-yl)-1-methyl-3,4-dihydro-1H-isoquinolin-2-yl]-methanone,
(6-Chloro-pyrazolo[1,5-a]pyridin-2-yl)-[7-(6-methoxy-pyridin-3-yl)-1-methyl-3,4-dihydro-1H-isoquinolin-2-yl]-methanone,
(6-Chloro-pyrazolo[1,5-a]pyridin-2-yl)-[7-(2-methoxy-pyridin-3-yl)-1-methyl-3,4-dihydro-1H-isoquinolin-2-yl]-methanone,
(6-Bromo-pyrazolo[1,5-a]pyridin-2-yl)-[7-(2,4-dimethoxy-pyrimidin-5-yl)-1-methyl-3,4-dihydro-1H-isoquinolin-2-yl]-methanone,
(6-Chloro-pyrazolo[1,5-a]pyridin-2-yl)-[7-(2,4-dimethoxy-pyrimidin-5-yl)-1-methyl-3,4-dihydro-1H-isoquinolin-2-yl]-methanone,
(6-Bromo-pyrazolo[1,5-a]pyridin-2-yl)-[7-(2-fluoro-pyridin-3-yl)-1-methyl-3,4-dihydro-1H-isoquinolin-2-yl]-methanone,
(6-Chloro-pyrazolo[1,5-a]pyridin-2-yl)-[7-(2-fluoro-pyridin-3-yl)-1-methyl-3,4-dihydro-1H-isoquinolin-2-yl]-methanone,
(6-Bromo-pyrazolo[1,5-a]pyridin-2-yl)-[5-(2-fluoro-pyridin-3-yl)-1-methyl-3,4-dihydro-1H-isoquinolin-2-yl]-methanone,
(6-Chloro-pyrazolo[1,5-a]pyridin-2-yl)-[5-(2-fluoro-pyridin-3-yl)-1-methyl-3,4-dihydro-1H-isoquinolin-2-yl]-methanone,
(6-Bromo-pyrazolo[1,5-a]pyridin-2-yl)-(8-furan-2-yl-1-methyl-3,4-dihydro-1H-isoquinolin-2-yl)-methanone,
(6-Chloro-pyrazolo[1,5-a]pyridin-2-yl)-(8-furan-2-yl-1-methyl-3,4-dihydro-1H-isoquinolin-2-yl)-methanone,
(6-Bromo-pyrazolo[1,5-a]pyridin-2-yl)-(7-furan-2-yl-1-methyl-3,4-dihydro-1H-isoquinolin-2-yl)-methanone,
(6-Chloro-pyrazolo[1,5-a]pyridin-2-yl)-(7-furan-2-yl-1-methyl-3,4-dihydro-1H-isoquinolin-2-yl)-methanone,
(6-Bromo-pyrazolo[1,5-a]pyridin-2-yl)-(6-furan-2-yl-1-methyl-3,4-dihydro-1H-isoquinolin-2-yl)-methanone,
(6-Chloro-pyrazolo[1,5-a]pyridin-2-yl)-(6-furan-2-yl-1-methyl-3,4-dihydro-1H-isoquinolin-2-yl)-methanone,
(6-Bromo-pyrazolo[1,5-a]pyridin-2-yl)-(5-furan-2-yl-1-methyl-3,4-dihydro-1H-isoquinolin-2-yl)-methanone,
(6-Chloro-pyrazolo[1,5-a]pyridin-2-yl)-(5-furan-2-yl-1-methyl-3,4-dihydro-1H-isoquinolin-2-yl)-methanone,
(6-Bromo-pyrazolo[1,5-a]pyridin-2-yl)-[1-methyl-8-(5-methyl-furan-2-yl)-3,4-dihydro-1H-isoquinolin-2-yl]-methanone,
(6-Chloro-pyrazolo[1,5-a]pyridin-2-yl)-[1-methyl-8-(5-methyl-furan-2-yl)-3,4-dihydro-1H-isoquinolin-2-yl]-methanone,
(6-Bromo-pyrazolo[1,5-a]pyridin-2-yl)-[1-methyl-7-(5-methyl-furan-2-yl)-3,4-dihydro-1H-isoquinolin-2-yl]-methanone,
(6-Chloro-pyrazolo[1,5-a]pyridin-2-yl)-[1-methyl-7-(5-methyl-furan-2-yl)-3,4-dihydro-1H-isoquinolin-2-yl]-methanone,
(6-Bromo-pyrazolo[1,5-a]pyridin-2-yl)-[1-methyl-6-(5-methyl-furan-2-yl)-3,4-dihydro-1H-isoquinolin-2-yl]-methanone,
(6-Chloro-pyrazolo[1,5-a]pyridin-2-yl)-[1-methyl-6-(5-methyl-furan-2-yl)-3,4-dihydro-1H-isoquinolin-2-yl]-methanone,
(6-Bromo-pyrazolo[1,5-a]pyridin-2-yl)-[1-methyl-5-(5-methyl-furan-2-yl)-3,4-dihydro-1H-isoquinolin-2-yl]-methanone,
(6-Chloro-pyrazolo[1,5-a]pyridin-2-yl)-[1-methyl-5-(5-methyl-furan-2-yl)-3,4-dihydro-1H-isoquinolin-2-yl]-methanone,
(6-Bromo-pyrazolo[1,5-a]pyridin-2-yl)-(8-furan-3-yl-1-methyl-3,4-dihydro-1H-isoquinolin-2-yl)-methanone,
(6-Chloro-pyrazolo[1,5-a]pyridin-2-yl)-(8-furan-3-yl-1-methyl-3,4-dihydro-1H-isoquinolin-2-yl)-methanone,
(6-Bromo-pyrazolo[1,5-a]pyridin-2-yl)-(7-furan-3-yl-1-methyl-3,4-dihydro-1H-isoquinolin-2-yl)-methanone,
(6-Chloro-pyrazolo[1,5-a]pyridin-2-yl)-(7-furan-3-yl-1-methyl-3,4-dihydro-1H-isoquinolin-2-yl)-methanone,
(6-Bromo-pyrazolo[1,5-a]pyridin-2-yl)-(6-furan-3-yl-1-methyl-3,4-dihydro-1H-isoquinolin-2-yl)-methanone,
(6-Chloro-pyrazolo[1,5-a]pyridin-2-yl)-(6-furan-3-yl-1-methyl-3,4-dihydro-1H-isoquinolin-2-yl)-methanone,
(6-Bromo-pyrazolo[1,5-a]pyridin-2-yl)-(5-furan-3-yl-1-methyl-3,4-dihydro-1H-isoquinolin-2-yl)-methanone,
(6-Chloro-pyrazolo[1,5-a]pyridin-2-yl)-(5-furan-3-yl-1-methyl-3,4-dihydro-1H-isoquinolin-2-yl)-methanone,
(6-Bromo-pyrazolo[1,5-a]pyridin-2-yl)-[1-methyl-5-(1H-pyrazol-4-yl)-3,4-dihydro-1H-isoquinolin-2-yl]-methanone,
(6-Chloro-pyrazolo[1,5-a]pyridin-2-yl)-[1-methyl-5-(1H-pyrazol-4-yl)-3,4-dihydro-1H-isoquinolin-2-yl]-methanone,
(6-Bromo-pyrazolo[1,5-a]pyridin-2-yl)-[7-(3,5-dimethyl-isoxazol-4-yl)-1-methyl-3,4-dihydro-1H-isoquinolin-2-yl]-methanone,
(6-Chloro-pyrazolo[1,5-a]pyridin-2-yl)-[7-(3,5-dimethyl-isoxazol-4-yl)-1-methyl-3,4-dihydro-1H-isoquinolin-2-yl]-methanone,
(6-Bromo-pyrazolo[1,5-a]pyridin-2-yl)-[6-(3,5-dimethyl-isoxazol-4-yl)-1-methyl-3,4-dihydro-1H-isoquinolin-2-yl]-methanone,
(6-Chloro-pyrazolo[1,5-a]pyridin-2-yl)-[6-(3,5-dimethyl-isoxazol-4-yl)-1-methyl-3,4-dihydro-1H-isoquinolin-2-yl]-methanone,
(6-Bromo-pyrazolo[1,5-a]pyridin-2-yl)-[5-(3,5-dimethyl-isoxazol-4-yl)-1-methyl-3,4-dihydro-1H-isoquinolin-2-yl]-methanone,
(6-Chloro-pyrazolo[1,5-a]pyridin-2-yl)-[5-(3,5-dimethyl-isoxazol-4-yl)-1-methyl-3,4-dihydro-1H-isoquinolin-2-yl]-methanone,
(6-Bromo-pyrazolo[1,5-a]pyridin-2-yl)-[1-methyl-8-(1H-pyrazol-4-yl)-3,4-dihydro-1H-isoquinolin-2-yl]-methanone,
(6-Chloro-pyrazolo[1,5-a]pyridin-2-yl)-[1-methyl-8-(1H-pyrazol-4-yl)-3,4-dihydro-1H-isoquinolin-2-yl]-methanone,
(6-Bromo-pyrazolo[1,5-a]pyridin-2-yl)-[1-methyl-7-(1H-pyrazol-4-yl)-3,4-dihydro-1H-isoquinolin-2-yl]-methanone,
(6-Chloro-pyrazolo[1,5-a]pyridin-2-yl)-[1-methyl-7-(1H-pyrazol-4-yl)-3,4-dihydro-1H-isoquinolin-2-yl]-methanone,
(6-Bromo-pyrazolo[1,5-a]pyridin-2-yl)-[1-methyl-6-(1H-pyrazol-4-yl)-3,4-dihydro-1H-isoquinolin-2-yl]-methanone,
(6-Chloro-pyrazolo[1,5-a]pyridin-2-yl)-[1-methyl-6-(1H-pyrazol-4-yl)-3,4-dihydro-1H-isoquinolin-2-yl]-methanone,
6-Bromo-pyrazolo[1,5-a]pyridine-2-carboxylic acid phenethyl-amide,
6-Chloro-pyrazolo[1,5-a]pyridine-2-carboxylic acid phenethyl-amide,
(6-Bromo-pyrazolo[1,5-a]pyridin-2-yl)-[1-methyl-8-(1-methyl-1H-pyrazol-4-yl)-3,4-dihydro-1H-isoquinolin-2-yl]-methanone,
(6-Chloro-pyrazolo[1,5-a]pyridin-2-yl)-[1-methyl-8-(1-methyl-1H-pyrazol-4-yl)-3,4-dihydro-1H-isoquinolin-2-yl]-methanone,
(6-Bromo-pyrazolo[1,5-a]pyridin-2-yl)-[1-methyl-7-(1-methyl-1H-pyrazol-4-yl)-3,4-dihydro-1H-isoquinolin-2-yl]-methanone,
(6-Chloro-pyrazolo[1,5-a]pyridin-2-yl)-[1-methyl-7-(1-methyl-1H-pyrazol-4-yl)-3,4-dihydro-1H-isoquinolin-2-yl]-methanone,
(6-Bromo-pyrazolo[1,5-a]pyridin-2-yl)-[1-methyl-6-(1-methyl-1H-pyrazol-4-yl)-3,4-dihydro-1H-isoquinolin-2-yl]-methanone,
(6-Chloro-pyrazolo[1,5-a]pyridin-2-yl)-[1-methyl-6-(1-methyl-1H-pyrazol-4-yl)-3,4-dihydro-1H-isoquinolin-2-yl]-methanone,
(6-Bromo-pyrazolo[1,5-a]pyridin-2-yl)-[1-methyl-5-(1-methyl-1H-pyrazol-4-yl)-3,4-dihydro-1H-isoquinolin-2-yl]-methanone,
(6-Chloro-pyrazolo[1,5-a]pyridin-2-yl)-[1-methyl-5-(1-methyl-1H-pyrazol-4-yl)-3,4-dihydro-1H-isoquinolin-2-yl]-methanone,
(6-Bromo-pyrazolo[1,5-a]pyridin-2-yl)-[1-methyl-8-(3-methyl-1H-pyrazol-4-yl)-3,4-dihydro-1H-isoquinolin-2-yl]-methanone,
(6-Chloro-pyrazolo[1,5-a]pyridin-2-yl)-[1-methyl-8-(3-methyl-1H-pyrazol-4-yl)-3,4-dihydro-1H-isoquinolin-2-yl]-methanone,
(6-Bromo-pyrazolo[1,5-a]pyridin-2-yl)-[1-methyl-7-(3-methyl-1H-pyrazol-4-yl)-3,4-dihydro-1H-isoquinolin-2-yl]-methanone,
(6-Chloro-pyrazolo[1,5-a]pyridin-2-yl)-[1-methyl-7-(3-methyl-1H-pyrazol-4-yl)-3,4-dihydro-1H-isoquinolin-2-yl]-methanone,
(6-Bromo-pyrazolo[1,5-a]pyridin-2-yl)-[1-methyl-6-(3-methyl-1H-pyrazol-4-yl)-3,4-dihydro-1H-isoquinolin-2-yl]-methanone,
(6-Chloro-pyrazolo[1,5-a]pyridin-2-yl)-[1-methyl-6-(3-methyl-1H-pyrazol-4-yl)-3,4-dihydro-1H-isoquinolin-2-yl]-methanone,
(6-Bromo-pyrazolo[1,5-a]pyridin-2-yl)-[1-methyl-5-(3-methyl-1H-pyrazol-4-yl)-3,4-dihydro-1H-isoquinolin-2-yl]-methanone,
(6-Chloro-pyrazolo[1,5-a]pyridin-2-yl)-[1-methyl-5-(3-methyl-1H-pyrazol-4-yl)-3,4-dihydro-1H-isoquinolin-2-yl]-methanone,
(6-Bromo-pyrazolo[1,5-a]pyridin-2-yl)-[1-methyl-8-(2H-tetrazol-5-yl)-3,4-dihydro-1H-isoquinolin-2-yl]-methanone,
(6-Chloro-pyrazolo[1,5-a]pyridin-2-yl)-[1-methyl-8-(2H-tetrazol-5-yl)-3,4-dihydro-1H-isoquinolin-2-yl]-methanone,
(6-Bromo-pyrazolo[1,5-a]pyridin-2-yl)-[1-methyl-7-(2H-tetrazol-5-yl)-3,4-dihydro-1H-isoquinolin-2-yl]-methanone,
(6-Chloro-pyrazolo[1,5-a]pyridin-2-yl)-[1-methyl-7-(2H-tetrazol-5-yl)-3,4-dihydro-1H-isoquinolin-2-yl]-methanone,
(6-Bromo-pyrazolo[1,5-a]pyridin-2-yl)-[1-methyl-6-(2H-tetrazol-5-yl)-3,4-dihydro-1H-isoquinolin-2-yl]-methanone,
(6-Chloro-pyrazolo[1,5-a]pyridin-2-yl)-[1-methyl-6-(2H-tetrazol-5-yl)-3,4-dihydro-1H-isoquinolin-2-yl]-methanone,
(6-Bromo-pyrazolo[1,5-a]pyridin-2-yl)-(1-methyl-5-nitro-3,4-dihydro-1H-2,7-naphthyridin-2-yl)-methanone,
(6-Chloro-pyrazolo[1,5-a]pyridin-2-yl)-(1-methyl-5-nitro-3,4-dihydro-1H-2,7-naphthyridin-2-yl)-methanone,
6-Bromo-pyrazolo[1,5-a]pyridin-2-yl)-(1-methyl-3,4-dihydro-1H-[2,7]naphthyridin-2-yl)-methanone,
6-Chloro-pyrazolo[1,5-a]pyridin-2-yl)-(1-methyl-3,4-dihydro-1H-[2,7]naphthyridin-2-yl)-methanone,
(6-Bromo-pyrazolo[1,5-a]pyridin-2-yl)-(1-methyl-3,4-dihydro-1H-[2,6]naphthyridin-2-yl)-methanone,
(6-Chloro-pyrazolo[1,5-a]pyridin-2-yl)-(1-methyl-3,4-dihydro-1H-[2,6]naphthyridin-2-yl)-methanone,
(6-Bromo-pyrazolo[1,5-a]pyridin-2-yl)-(5-methyl-7,8-dihydro-5H-pyrido[4,3-d]pyrimidin-6-yl)-methanone,
(6-Chloro-pyrazolo[1,5-a]pyridin-2-yl)-(5-methyl-7,8-dihydro-5H-pyrido[4,3-d]pyrimidin-6-yl)-methanone,
(6-Bromo-pyrazolo[1,5-a]pyridin-2-yl)-(8-methyl-5,8-dihydro-6H-pyrido[3,4-d]pyrimidin-7-yl)-methanone,
(6-Chloro-pyrazolo[1,5-a]pyridin-2-yl)-(8-methyl-5,8-dihydro-6H-pyrido[3,4-d]pyrimidin-7-yl)-methanone,
(6-Bromo-pyrazolo[1,5-a]pyridin-2-yl)-(1-methyl-5-nitro-3,4-dihydro-1H-isoquinolin-2-yl)-methanone,
(6-Chloro-pyrazolo[1,5-a]pyridin-2-yl)-(1-methyl-5-nitro-3,4-dihydro-1H-isoquinolin-2-yl)-methanone,
(6-Bromo-pyrazolo[1,5-a]pyridin-2-yl)-(1-methyl-6-nitro-3,4-dihydro-1H-isoquinolin-2-yl)-methanone,
(6-Chloro-pyrazolo[1,5-a]pyridin-2-yl)-(1-methyl-6-nitro-3,4-dihydro-1H-isoquinolin-2-yl)-methanone,
(6-Bromo-pyrazolo[1,5-a]pyridin-2-yl)-(1-methyl-7-nitro-3,4-dihydro-1H-isoquinolin-2-yl)-methanone,
(6-Chloro-pyrazolo[1,5-a]pyridin-2-yl)-(1-methyl-7-nitro-3,4-dihydro-1H-isoquinolin-2-yl)-methanone,
6-Bromo-pyrazolo[1,5-a]pyridine-2-carboxylic acid cycloheptylamide,
6-Chloro-pyrazolo[1,5-a]pyridine-2-carboxylic acid cycloheptylamide,
6-Bromo-pyrazolo[1,5-a]pyridine-2-carboxylic acid cyclohexylamide,
6-Chloro-pyrazolo[1,5-a]pyridine-2-carboxylic acid cyclohexylamide,
2-(6-Bromo-pyrazolo[1,5-a]pyridine-2-carbonyl)-1-methyl-1,2,3,4-tetrahydro-isoquinoline-5-carbonitrile,
2-(6-Chloro-pyrazolo[1,5-a]pyridine-2-carbonyl)-1-methyl-1,2,3,4-tetrahydro-isoquinoline-5-carbonitrile,
2-(6-Bromo-pyrazolo[1,5-a]pyridine-2-carbonyl)-1-methyl-1,2,3,4-tetrahydro-isoquinoline-5-carboxylic acid methyl ester,
2-(6-Chloro-pyrazolo[1,5-a]pyridine-2-carbonyl)-1-methyl-1,2,3,4-tetrahydro-isoquinoline-5-carboxylic acid methyl ester,
(6-Bromo-pyrazolo[1,5-a]pyridin-2-yl)-(2,6-dimethyl-8,9-dihydro-6H-3-oxa-1,7-diaza-cyclopenta[a]naphthalen-7-yl)-methanone,
(6-Chloro-pyrazolo[1,5-a]pyridin-2-yl)-(2,6-dimethyl-8,9-dihydro-6H-3-oxa-1,7-diazacyclopenta[a]naphthalen-7-yl)-methanone,
(6-Bromo-pyrazolo[1,5-a]pyridin-2-yl)-(4-methyl-1,4-dihydro-2H-[3,7]phenanthrolin-3-yl)-methanone,
(6-Chloro-pyrazolo[1,5-a]pyridin-2-yl)-(4-methyl-1,4-dihydro-2H-[3,7]phenanthrolin-3-yl)-methanone,
(6-Bromo-pyrazolo[1,5-a]pyridin-2-yl)-(2,6-dimethyl-8,9-dihydro-6H-thiazolo[4,5-f]isoquinolin-7-yl)-methanone,
(6-Chloro-pyrazolo[1,5-a]pyridin-2-yl)-(2,6-dimethyl-8,9-dihydro-6H-thiazolo[4,5-f]isoquinolin-7-yl)-methanone,
(6-Bromo-pyrazolo[1,5-a]pyridin-2-yl)-(5-methanesulfonyl-1-methyl-3,4-dihydro-1H-isoquinolin-2-yl)-methanone,
(6-Chloro-pyrazolo[1,5-a]pyridin-2-yl)-(5-methanesulfonyl-1-methyl-3,4-dihydro-1H-isoquinolin-2-yl)-methanone,
(6-Bromo-pyrazolo[1,5-a]pyridin-2-yl)-(6-methanesulfonyl-1-methyl-3,4-dihydro-1H-isoquinolin-2-yl)-methanone,
(6-Chloro-pyrazolo[1,5-a]pyridin-2-yl)-(6-methanesulfonyl-1-methyl-3,4-dihydro-1H-isoquinolin-2-yl)-methanone,
(6-Bromo-pyrazolo[1,5-a]pyridin-2-yl)-(7-methanesulfonyl-1-methyl-3,4-dihydro-1H-isoquinolin-2-yl)-methanone,
(6-Chloro-pyrazolo[1,5-a]pyridin-2-yl)-(7-methanesulfonyl-1-methyl-3,4-dihydro-1H-isoquinolin-2-yl)-methanone,
2-(6-Bromo-pyrazolo[1,5-a]pyridine-2-carbonyl)-1-methyl-1,2,3,4-tetrahydro-isoquinoline-6-carboxylic acid methyl ester,
2-(6-Chloro-pyrazolo[1,5-a]pyridine-2-carbonyl)-1-methyl-1,2,3,4-tetrahydro-isoquinoline-6-carboxylic acid methyl ester,
(6-Bromo-pyrazolo[1,5-a]pyridin-2-yl)-(4,7-dimethyl-1,4,7,8,9,10-hexahydro-2H-[3,7]phenanthrolin-3-yl)-methanone,
(6-Chloro-pyrazolo[1,5-a]pyridin-2-yl)-(4,7-dimethyl-1,4,7,8,9,10-hexahydro-2H-[3,7]phenanthrolin-3-yl)-methanone,
1-[2-(6-Bromo-pyrazolo[1,5-a]pyridine-2-carbonyl)-1-methyl-1,2,3,4-tetrahydro-isoquinolin-5-yl]-ethanone,
1-[2-(6-Chloro-pyrazolo[1,5-a]pyridine-2-carbonyl)-1-methyl-1,2,3,4-tetrahydro-isoquinolin-5-yl]-ethanone,
6-Bromo-pyrazolo[1,5-a]pyridine-2-carboxylic acid adamantyl-1 amide,
6-Chloro-pyrazolo[1,5-a]pyridine-2-carboxylic acid adamantyl-1 amide,
2-(6-Bromo-pyrazolo[1,5-a]pyridine-2-carbonyl)-1-methyl-1,2,3,4-tetrahydro-isoquinoline-7-carboxylic acid methyl ester,
2-(6-Chloro-pyrazolo[1,5-a]pyridine-2-carbonyl)-1-methyl-1,2,3,4-tetrahydro-isoquinoline-7-carboxylic acid methyl ester,
(6-Bromo-pyrazolo[1,5-a]pyridin-2-yl)-[1-methyl-5-(morpholine-4-carbonyl)-3,4-dihydro-1H-isoquinolin-2-yl]-methanone,
((6-Chloro-pyrazolo[1,5-a]pyridin-2-yl)-[1-methyl-5-(morpholine-4-carbonyl)-3,4-dihydro-1H-isoquinolin-2-yl]-methanone,
(6-Bromo-pyrazolo[1,5-a]pyridin-2-yl)-[1-methyl-7-(morpholine-4-carbonyl)-3,4-dihydro-1H-isoquinolin-2-yl]-methanone,
(6-Chloro-pyrazolo[1,5-a]pyridin-2-yl)-[1-methyl-7-(morpholine-4-carbonyl)-3,4-dihydro-1H-isoquinolin-2-yl]-methanone,
(6-Bromo-pyrazolo[1,5-a]pyridin-2-yl)-[1-methyl-6-(morpholine-4-carbonyl)-3,4-dihydro-1H-isoquinolin-2-yl]-methanone,
(6-Chloro-pyrazolo[1,5-a]pyridin-2-yl)-[1-methyl-6-(morpholine-4-carbonyl)-3,4-dihydro-1H-isoquinolin-2-yl]-methanone,
(6-Bromo-pyrazolo[1,5-a]pyridin-2-yl)-(6-methyl-8,9-dihydro-6H-furo[3,2-f]isoquinolin-7-yl)-methanone,
(6-Chloro-pyrazolo[1,5-a]pyridin-2-yl)-(6-methyl-8,9-dihydro-6H-furo[3,2-f]isoquinolin-7-yl)-methanone,
(6-Bromo-pyrazolo[1,5-a]pyridin-2-yl)-(3,6-dimethyl-3,6,8,9-tetrahydro-pyrrolo[3,2-f]isoquinolin-7-yl)-methanone,
(6-Chloro-pyrazolo[1,5-a]pyridin-2-yl)-(3,6-dimethyl-3,6,8,9-tetrahydro-pyrrolo[3,2-f]isoquinolin-7-yl)-methanone,
(6-Bromo-pyrazolo[1,5-a]pyridin-2-yl)-(8-methyl-5,8-dihydro-6H-furo[3,2-g]isoquinolin-7-yl)-methanone,
(6-Chloro-pyrazolo[1,5-a]pyridin-2-yl)-(8-methyl-5,8-dihydro-6H-furo[3,2-g]isoquinolin-7-yl)-methanone,
7-(6-Bromo-pyrazolo[1,5-a]pyridine-2-carbonyl)-2,6-dimethyl-1,2,6,7,8,9-hexahydro-pyrrolo[3,4-f]isoquinolin-3-one,
7-(6-Chloro-pyrazolo[1,5-a]pyridine-2-carbonyl)-2,6-dimethyl-1,2,6,7,8,9-hexahydro-pyrrolo[3,4-f]isoquinolin-3-one,
7-(6-Bromo-pyrazolo[1,5-a]pyridine-2-carbonyl)-2,6-dimethyl-2,3,6,7,8,9-hexahydro-pyrrolo[3,4-f]isoquinolin-1-one,
7-(6-Chloro-pyrazolo[1,5-a]pyridine-2-carbonyl)-2,6-dimethyl-2,3,6,7,8,9-hexahydro-pyrrolo[3,4-f]isoquinolin-1-one,
(6-Bromo-pyrazolo[1,5-a]pyridin-2-yl)-(6-diethylamino-1-methyl-3,4-dihydro-1H-isoquinolin-2-yl)-methanone,
(6-Chloro-pyrazolo[1,5-a]pyridin-2-yl)-(6-diethylamino-1-methyl-3,4-dihydro-1H-isoquinolin-2-yl)-methanone,
(6-Bromo-pyrazolo[1,5-a]pyridin-2-yl)-(5-diethylamino-1-methyl-3,4-dihydro-1H-isoquinolin-2-yl)-methanone,
(6-Chloro-pyrazolo[1,5-a]pyridin-2-yl)-(5-diethylamino-1-methyl-3,4-dihydro-1H-isoquinolin-2-yl)-methanone,
(6-Bromo-pyrazolo[1,5-a]pyridin-2-yl)-(8-diethylamino-1-methyl-3,4-dihydro-1H-isoquinolin-2-yl)-methanone,
(6-Chloro-pyrazolo[1,5-a]pyridin-2-yl)-(8-diethylamino-1-methyl-3,4-dihydro-1H-isoquinolin-2-yl)-methanone,
(6-Bromo-pyrazolo[1,5-a]pyridin-2-yl)-(7-diethylamino-1-methyl-3,4-dihydro-1H-isoquinolin-2-yl)-methanone,
(6-Chloro-pyrazolo[1,5-a]pyridin-2-yl)-(7-diethylamino-1-methyl-3,4-dihydro-1H-isoquinolin-2-yl)-methanone,
(6-Bromo-pyrazolo[1,5-a]pyridin-2-yl)-(6,7-difluoro-1-methyl-3,4-dihydro-1H-isoquinolin-2-yl)-methanone,
(6-Chloro-pyrazolo[1,5-a]pyridin-2-yl)-(6,7-difluoro-1-methyl-3,4-dihydro-1H-isoquinolin-2-yl)-methanone,
(6-Bromo-pyrazolo[1,5-a]pyridin-2-yl)-(6,7-dichloro-1-methyl-3,4-dihydro-1H-isoquinolin-2-yl)-methanone,
(6-Chloro-pyrazolo[1,5-a]pyridin-2-yl)-(6,7-dichloro-1-methyl-3,4-dihydro-1H-isoquinolin-2-yl)-methanone,
(6-Bromo-pyrazolo[1,5-a]pyridin-2-yl)-(6,7-dibromo-1-methyl-3,4-dihydro-1H-isoquinolin-2-yl)-methanone,
(6-Chloro-pyrazolo[1,5-a]pyridin-2-yl)-(6,7-dibromo-1-methyl-3,4-dihydro-1H-isoquinolin-2-yl)-methanone,
(6-Bromo-pyrazolo[1,5-a]pyridin-2-yl)-(1-methyl-7-morpholin-4-yl-3,4-dihydro-1H-isoquinolin-2-yl)-methanone,
(6-Chloro-pyrazolo[1,5-a]pyridin-2-yl)-(1-methyl-7-morpholin-4-yl-3,4-dihydro-1H-isoquinolin-2-yl)-methanone,
(6-Bromo-pyrazolo[1,5-a]pyridin-2-yl)-(1-methyl-8-morpholin-4-yl-3,4-dihydro-1H-isoquinolin-2-yl)-methanone,
(6-Chloro-pyrazolo[1,5-a]pyridin-2-yl)-(1-methyl-8-morpholin-4-yl-3,4-dihydro-1H-isoquinolin-2-yl)-methanone,
(6-Bromo-pyrazolo[1,5-a]pyridin-2-yl)-(1-methyl-6-morpholin-4-yl-3,4-dihydro-1H-isoquinolin-2-yl)-methanone,
(6-Chloro-pyrazolo[1,5-a]pyridin-2-yl)-(1-methyl-6-morpholin-4-yl-3,4-dihydro-1H-isoquinolin-2-yl)-methanone,
(6-Bromo-pyrazolo[1,5-a]pyridin-2-yl)-(1-methyl-5-morpholin-4-yl-3,4-dihydro-1H-isoquinolin-2-yl)-methanone,
(6-Chloro-pyrazolo[1,5-a]pyridin-2-yl)-(1-methyl-5-morpholin-4-yl-3,4-dihydro-1H-isoquinolin-2-yl)-methanone,
(6-Bromo-pyrazolo[1,5-a]pyridin-2-yl)-(1-methyl-7-piperidin-1-yl-3,4-dihydro-1H-isoquinolin-2-yl)-methanone,
(6-Chloro-pyrazolo[1,5-a]pyridin-2-yl)-(1-methyl-7-piperidin-1-yl-3,4-dihydro-1H-isoquinolin-2-yl)-methanone,
(6-Bromo-pyrazolo[1,5-a]pyridin-2-yl)-(1-methyl-7-pyrrolidin-1-yl-3,4-dihydro-1H-isoquinolin-2-yl)-methanone,
(6-Chloro-pyrazolo[1,5-a]pyridin-2-yl)-(1-methyl-7-pyrrolidin-1-yl-3,4-dihydro-1H-isoquinolin-2-yl)-methanone,
(6-Chloro-pyrazolo[1,5-a]pyridin-2-yl)-(1-methyl-3,4-dihydro-1H-pyrrolo[1,2-a]pyrazin-2-yl)-methanone,
(6-Chloro-pyrazolo[1,5-a]pyridin-2-yl)-(3,4-dihydro-1H-pyrrolo[1,2-a]pyrazin-2-yl)-methanone,
(6-Bromo-pyrazolo[1,5-a]pyridin-2-yl)-(1-methyl-3,4-dihydro-1H-pyrrolo[1,2-a]pyrazin-2-yl)-methanone,
(6-Bromo-pyrazolo[1,5-a]pyridin-2-yl)-(3,4-dihydro-1H-pyrrolo[1,2-a]pyrazin-2-yl)-methanone,
(6-Chloro-pyrazolo[1,5-a]pyridin-2-yl)-(1,6-dimethyl-3,4-dihydro-1H-pyrrolo[1,2-a]pyrazin-2-yl)-methanone,
(6-Chloro-pyrazolo[1,5-a]pyridin-2-yl)-(6-methyl-3,4-dihydro-1H-pyrrolo[1,2-a]pyrazin-2-yl)-methanone,
(6-Bromo-pyrazolo[1,5-a]pyridin-2-yl)-(1,6-dimethyl-3,4-dihydro-1H-pyrrolo[1,2-a]pyrazin-2-yl)-methanone,
(6-Bromo-pyrazolo[1,5-a]pyridin-2-yl)-(6-methyl-3,4-dihydro-1H-pyrrolo[1,2-a]pyrazin-2-yl)-methanone,
(6-Chloro-pyrazolo[1,5-a]pyridin-2-yl)-(4-methyl-6,7-dihydro-4H-[1,2,3]triazolo[1,5-a]pyrazin-5-yl)-methanone,
(6-Chloro-pyrazolo[1,5-a]pyridin-2-yl)-(6,7-dihydro-4H-[1,2,3]triazolo[1,5-a]pyrazin-5-yl)-methanone,
(6-Bromo-pyrazolo[1,5-a]pyridin-2-yl)-(4-methyl-6,7-dihydro-4H-[1,2,3]triazolo[1,5-a]pyrazin-5-yl)-methanone,
(6-Bromo-pyrazolo[1,5-a]pyridin-2-yl)-(6,7-dihydro-4H-[1,2,3]triazolo[1,5-a]pyrazin-5-yl)-methanone,
7-(6-Chloro-pyrazolo[1,5-a]pyridine-2-carbonyl)-8-methyl-5,6,7,8-tetrahydro-imidazo[1,2-a]pyrazine-2-carboxylic acid ethyl ester,
7-(6-Chloro-pyrazolo[1,5-a]pyridine-2-carbonyl)-5,6,7,8-tetrahydro-imidazo[1,2-a]pyrazine-2-carboxylic acid ethyl ester,
7-(6-Bromo-pyrazolo[1,5-a]pyridine-2-carbonyl)-8-methyl-5,6,7,8-tetrahydro-imidazo[1,2-a]pyrazine-2-carboxylic acid ethyl ester,
7-(6-Bromo-pyrazolo[1,5-a]pyridine-2-carbonyl)-5,6,7,8-tetrahydro-imidazo[1,2-a]pyrazine-2-carboxylic acid ethyl ester,
7-(6-Chloro-pyrazolo[1,5-a]pyridine-2-carbonyl)-8-methyl-5,6,7,8-tetrahydro-[1,2,4]triazolo[4,3-a]pyrazine-3-carboxylic acid ethyl ester,
7-(6-Chloro-pyrazolo[1,5-a]pyridine-2-carbonyl)-5,6,7,8-tetrahydro-[1,2,4]triazolo[4,3-a]pyrazine-3-carboxylic acid ethyl ester,
7-(6-Bromo-pyrazolo[1,5-a]pyridine-2-carbonyl)-8-methyl-5,6,7,8-tetrahydro-[1,2,4]triazolo[4,3-a]pyrazine-3-carboxylic acid ethyl ester,
7-(6-Bromo-pyrazolo[1,5-a]pyridine-2-carbonyl)-5,6,7,8-tetrahydro-[1,2,4]triazolo[4,3-a]pyrazine-3-carboxylic acid ethyl ester,
(3-Bromo-8-methyl-5,6-dihydro-8H-[1,2,4]triazolo[4,3-a]pyrazin-7-yl)-(6-chloro-pyrazolo[1,5-a]pyridin-2-yl)-methanone,
(3-Bromo-5,6-dihydro-8H-[1,2,4]triazolo[4,3-a]pyrazin-7-yl)-(6-chloro-pyrazolo[1,5-a]pyridin-2-yl)-methanone,
(3-Bromo-8-methyl-5,6-dihydro-8H-[1,2,4]triazolo[4,3-a]pyrazin-7-yl)-(6-bromo-pyrazolo[1,5-a]pyridin-2-yl)-methanone,
(3-Bromo-5,6-dihydro-8H-[1,2,4]triazolo[4,3-a]pyrazin-7-yl)-(6-bromo-pyrazolo[1,5-a]pyridin-2-yl)-methanone,
(6-Chloro-pyrazolo[1,5-a]pyridin-2-yl)-(8-methyl-5,6-dihydro-8H-[1,2,4]triazolo[4,3-a]pyrazin-7-yl)-methanone,
(6-Chloro-pyrazolo[1,5-a]pyridin-2-yl)-(5,6-dihydro-8H-[1,2,4]triazolo[4,3-a]pyrazin-7-yl)-methanone,
(6-Bromo-pyrazolo[1,5-a]pyridin-2-yl)-(8-methyl-5,6-dihydro-8H-[1,2,4]triazolo[4,3-a]pyrazin-7-yl)-methanone,
(6-Bromo-pyrazolo[1,5-a]pyridin-2-yl)-(5,6-dihydro-8H-[1,2,4]triazolo[4,3-a]pyrazin-7-yl)-methanone,
(6-Chloro-1-methyl-3,4-dihydro-1H-pyrrolo[1,2-a]pyrazin-2-yl)-(6-chloro-pyrazolo[1,5-a]pyridin-2-yl)-methanone,
(6-Chloro-3,4-dihydro-1H-pyrrolo[1,2-a]pyrazin-2-yl)-(6-chloro-pyrazolo[1,5-a]pyridin-2-yl)-methanone,
(6-Chloro-1-methyl-3,4-dihydro-1H-pyrrolo[1,2-a]pyrazin-2-yl)-(6-bromo-pyrazolo[1,5-a]pyridin-2-yl)-methanone,
(6-Chloro-3,4-dihydro-1H-pyrrolo[1,2-a]pyrazin-2-yl)-(6-bromo-pyrazolo[1,5-a]pyridin-2-yl)-methanone,
(6-Chloro-pyrazolo[1,5-a]pyridin-2-yl)-(6,7-dibromo-1-methyl-3,4-dihydro-1H-pyrrolo[1,2-a]pyrazin-2-yl)-methanone,
(6-Chloro-pyrazolo[1,5-a]pyridin-2-yl)-(6,7-dibromo-3,4-dihydro-1H-pyrrolo[1,2-a]pyrazin-2-yl)-methanone,
(6-Bromo-pyrazolo[1,5-a]pyridin-2-yl)-(6,7-dibromo-1-methyl-3,4-dihydro-1H-pyrrolo[1,2-a]pyrazin-2-yl)-methanone,
(6-Bromo-pyrazolo[1,5-a]pyridin-2-yl)-(6,7-dibromo-3,4-dihydro-1H-pyrrolo[1,2-a]pyrazin-2-yl)-methanone,
(6-Bromo-1-methyl-3,4-dihydro-1H-pyrrolo[1,2-a]pyrazin-2-yl)-(6-chloro-pyrazolo[1,5-a]pyridin-2-yl)-methanone,
(6-Bromo-3,4-dihydro-1H-pyrrolo[1,2-a]pyrazin-2-yl)-(6-chloro-pyrazolo[1,5-a]pyridin-2-yl)-methanone,
(6-Bromo-1-methyl-3,4-dihydro-1H-pyrrolo[1,2-a]pyrazin-2-yl)-(6-bromo-pyrazolo[1,5-a]pyridin-2-yl)-methanone,
(6-Bromo-3,4-dihydro-1H-pyrrolo[1,2-a]pyrazin-2-yl)-(6-bromo-pyrazolo[1,5-a]pyridin-2-yl)-methanone,
(6-Chloro-pyrazolo[1,5-a]pyridin-2-yl)-(4-methyl-6,7-dihydro-4H-pyrazolo[1,5-a]pyrazin-5-yl)-methanone,
(6-Chloro-pyrazolo[1,5-a]pyridin-2-yl)-(6,7-dihydro-4H-pyrazolo[1,5-a]pyrazin-5-yl)-methanone,
(6-Bromo-pyrazolo[1,5-a]pyridin-2-yl)-(4-methyl-6,7-dihydro-4H-pyrazolo[1,5-a]pyrazin-5-yl)-methanone,
(6-Bromo-pyrazolo[1,5-a]pyridin-2-yl)-(6,7-dihydro-4H-pyrazolo[1,5-a]pyrazin-5-yl)-methanone,
(6-Chloro-pyrazolo[1,5-a]pyridin-2-yl)-(8-methyl-5,6-dihydro-8H-imidazo[1,2-a]pyrazin-7-yl)-methanone,
(6-Chloro-pyrazolo[1,5-a]pyridin-2-yl)-(5,6-dihydro-8H-imidazo[1,2-a]pyrazin-7-yl)-methanone,
(6-Bromo-pyrazolo[1,5-a]pyridin-2-yl)-(8-methyl-5,6-dihydro-8H-imidazo[1,2-a]pyrazin-7-yl)-methanone,
(6-Bromo-pyrazolo[1,5-a]pyridin-2-yl)-(5,6-dihydro-8H-imidazo[1,2-a]pyrazin-7-yl)-methanone,
(6-Chloro-pyrazolo[1,5-a]pyridin-2-yl)-(2,8-dimethyl-5,6-dihydro-8H-imidazo[1,2-a]pyrazin-7-yl)-methanone,
(6-Chloro-pyrazolo[1,5-a]pyridin-2-yl)-(2-methyl-5,6-dihydro-8H-imidazo[1,2-a]pyrazin-7-yl)-methanone,
(6-Bromo-pyrazolo[1,5-a]pyridin-2-yl)-(2,8-dimethyl-5,6-dihydro-8H-imidazo[1,2-a]pyrazin-7-yl)-methanone,
(6-Bromo-pyrazolo[1,5-a]pyridin-2-yl)-(2-methyl-5,6-dihydro-8H-imidazo[1,2-a]pyrazin-7-yl)-methanone,
(6-Chloro-pyrazolo[1,5-a]pyridin-2-yl)-(3,8-dimethyl-5,6-dihydro-8H-imidazo[1,2-a]pyrazin-7-yl)-methanone,
(6-Chloro-pyrazolo[1,5-a]pyridin-2-yl)-(3-methyl-5,6-dihydro-8H-imidazo[1,2-a]pyrazin-7-yl)-methanone,
(6-Bromo-pyrazolo[1,5-a]pyridin-2-yl)-(3,8-dimethyl-5,6-dihydro-8H-imidazo[1,2-a]pyrazin-7-yl)-methanone,
(6-Bromo-pyrazolo[1,5-a]pyridin-2-yl)-(3-methyl-5,6-dihydro-8H-imidazo[1,2-a]pyrazin-7-yl)-methanone,
(6-Chloro-pyrazolo[1,5-a]pyridin-2-yl)-(3,8-dimethyl-5,6-dihydro-8H-[1,2,4]triazolo[4,3-a]pyrazin-7-yl)-methanone,
(6-Chloro-pyrazolo[1,5-a]pyridin-2-yl)-(3-methyl-5,6-dihydro-8H-[1,2,4]triazolo[4,3-a]pyrazin-7-yl)-methanone,
(6-Bromo-pyrazolo[1,5-a]pyridin-2-yl)-(3,8-dimethyl-5,6-dihydro-8H-[1,2,4]triazolo[4,3-a]pyrazin-7-yl)-methanone,
(6-Bromo-pyrazolo[1,5-a]pyridin-2-yl)-(3-methyl-5,6-dihydro-8H-[1,2,4]triazolo[4,3-a]pyrazin-7-yl)-methanone,
2-(6-Chloro-pyrazolo[1,5-a]pyridine-2-carbonyl)-1-methyl-1,2,3,4-tetrahydro-pyrrolo[1,2-a]pyrazine-6-carbonitrile,
2-(6-Chloro-pyrazolo[1,5-a]pyridine-2-carbonyl)-1,2,3,4-tetrahydro-pyrrolo[1,2-a]pyrazine-6-carbonitrile,
2-(6-Bromo-pyrazolo[1,5-a]pyridine-2-carbonyl)-1-methyl-1,2,3,4-tetrahydro-pyrrolo[1,2-a]pyrazine-6-carbonitrile,
2-(6-Bromo-pyrazolo[1,5-a]pyridine-2-carbonyl)-1,2,3,4-tetrahydro-pyrrolo[1,2-a]pyrazine-6-carbonitrile,
2-(6-Chloro-pyrazolo[1,5-a]pyridine-2-carbonyl)-1-methyl-1,2,3,4-tetrahydro-pyrrolo[1,2-a]pyrazine-6-carboxylic acid methylamide,
2-(6-Chloro-pyrazolo[1,5-a]pyridine-2-carbonyl)-1,2,3,4-tetrahydro-pyrrolo[1,2-a]pyrazine-6-carboxylic acid methylamide,
2-(6-Bromo-pyrazolo[1,5-a]pyridine-2-carbonyl)-1-methyl-1,2,3,4-tetrahydro-pyrrolo[1,2-a]pyrazine-6-carboxylic acid methylamide,
2-(6-Bromo-pyrazolo[1,5-a]pyridine-2-carbonyl)-1,2,3,4-tetrahydro-pyrrolo[1,2-a]pyrazine-6-carboxylic acid methylamide,
2-(6-Chloro-pyrazolo[1,5-a]pyridine-2-carbonyl)-1-methyl-1,2,3,4-tetrahydro-pyrrolo[1,2-a]pyrazine-6-carboxylic acid cyclopropylamide,
2-(6-Chloro-pyrazolo[1,5-a]pyridine-2-carbonyl)-1,2,3,4-tetrahydro-pyrrolo[1,2-a]pyrazine-6-carboxylic acid cyclopropylamide,
2-(6-Bromo-pyrazolo[1,5-a]pyridine-2-carbonyl)-1-methyl-1,2,3,4-tetrahydro-pyrrolo[1,2-a]pyrazine-6-carboxylic acid cyclopropylamide,
2-(6-Bromo-pyrazolo[1,5-a]pyridine-2-carbonyl)-1,2,3,4-tetrahydro-pyrrolo[1,2-a]pyrazine-6-carboxylic acid cyclopropylamide,
2-(6-Chloro-pyrazolo[1,5-a]pyridine-2-carbonyl)-1-methyl-1,2,3,4-tetrahydro-pyrrolo[1,2-a]pyrazine-6-carboxylic acid ethylamide,
2-(6-Chloro-pyrazolo[1,5-a]pyridine-2-carbonyl)-1,2,3,4-tetrahydro-pyrrolo[1,2-a]pyrazine-6-carboxylic acid ethylamide,
2-(6-Bromo-pyrazolo[1,5-a]pyridine-2-carbonyl)-1-methyl-1,2,3,4-tetrahydro-pyrrolo[1,2-a]pyrazine-6-carboxylic acid ethylamide,
2-(6-Bromo-pyrazolo[1,5-a]pyridine-2-carbonyl)-1,2,3,4-tetrahydro-pyrrolo[1,2-a]pyrazine-6-carboxylic acid ethylamide,
2-(6-Chloro-pyrazolo[1,5-a]pyridine-2-carbonyl)-1-methyl-1,2,3,4-tetrahydro-pyrrolo[1,2-a]pyrazine-6-carboxylic acid isopropylamide,
2-(6-Chloro-pyrazolo[1,5-a]pyridine-2-carbonyl)-1,2,3,4-tetrahydro-pyrrolo[1,2-a]pyrazine-6-carboxylic acid isopropylamide,
2-(6-Bromo-pyrazolo[1,5-a]pyridine-2-carbonyl)-1-methyl-1,2,3,4-tetrahydro-pyrrolo[1,2-a]pyrazine-6-carboxylic acid isopropylamide,
2-(6-Bromo-pyrazolo[1,5-a]pyridine-2-carbonyl)-1,2,3,4-tetrahydro-pyrrolo[1,2-a]pyrazine-6-carboxylic acid isopropylamide,
(6-Chloro-pyrazolo[1,5-a]pyridin-2-yl)-[1-methyl-6-(1H-tetrazol-5-yl)-3,4-dihydro-1H-pyrrolo[ 1,2-a]pyrazin-2-yl]-methanone,
(6-Chloro-pyrazolo[1,5-a]pyridin-2-yl)-[6-(1H-tetrazol-5-yl)-3,4-dihydro-1H-pyrrolo[1,2-a]pyrazin-2-yl]-methanone,
(6-Bromo-pyrazolo[1,5-a]pyridin-2-yl)-[1-methyl-6-(1H-tetrazol-5-yl)-3,4-dihydro-1H-pyrrolo[1,2-a]pyrazin-2-yl]-methanone,
(6-Bromo-pyrazolo[1,5-a]pyridin-2-yl)-[6-(1H-tetrazol-5-yl)-3,4-dihydro-1H-pyrrolo[1,2-a]pyrazin-2-yl]-methanone,
(6-Chloro-pyrazolo[1,5-a]pyridin-2-yl)-(1-methyl-6-pyridin-2-yl-3,4-dihydro-1H-pyrrolo[1,2-a]pyrazin-2-yl)-methanone,
(6-Chloro-pyrazolo[1,5-a]pyridin-2-yl)-(6-pyridin-2-yl-3,4-dihydro-1H-pyrrolo[1,2-a]pyrazin-2-yl)-methanone,
(6-Bromo-pyrazolo[1,5-a]pyridin-2-yl)-(1-methyl-6-pyridin-2-yl-3,4-dihydro-1H-pyrrolo[1,2-a]pyrazin-2-yl)-methanone,
(6-Bromo-pyrazolo[1,5-a]pyridin-2-yl)-(6-pyridin-2-yl-3,4-dihydro-1H-pyrrolo[1,2-a]pyrazin-2-yl)-methanone,
(6-Chloro-pyrazolo[1,5-a]pyridin-2-yl)-(1-methyl-6-pyridin-3-yl-3,4-dihydro-1H-pyrrolo[1,2-a]pyrazin-2-yl)-methanone,
(6-Chloro-pyrazolo[1,5-a]pyridin-2-yl)-(6-pyridin-3-yl-3,4-dihydro-1H-pyrrolo[1,2-a]pyrazin-2-yl)-methanone,
(6-Bromo-pyrazolo[1,5-a]pyridin-2-yl)-(1-methyl-6-pyridin-3-yl-3,4-dihydro-1H-pyrrolo[1,2-a]pyrazin-2-yl)-methanone,
(6-Bromo-pyrazolo[1,5-a]pyridin-2-yl)-(6-pyridin-3-yl-3,4-dihydro-1H-pyrrolo[1,2-a]pyrazin-2-yl)-methanone,
(6-Chloro-pyrazolo[1,5-a]pyridin-2-yl)-(1-methyl-6-pyridin-4-yl-3,4-dihydro-1H-pyrrolo[1,2-a]pyrazin-2-yl)-methanone,
(6-Chloro-pyrazolo[1,5-a]pyridin-2-yl)-(6-pyridin-4-yl-3,4-dihydro-1H-pyrrolo[1,2-a]pyrazin-2-yl)-methanone,
(6-Bromo-pyrazolo[1,5-a]pyridin-2-yl)-(1-methyl-6-pyridin-4-yl-3,4-dihydro-1H-pyrrolo[1,2-a]pyrazin-2-yl)-methanone,
(6-Bromo-pyrazolo[1,5-a]pyridin-2-yl)-(6-pyridin-4-yl-3,4-dihydro-1H-pyrrolo[1,2-a]pyrazin-2-yl)-methanone,
(6-Chloro-pyrazolo[1,5-a]pyridin-2-yl)-(1-methyl-6-thiophen-2-yl-3,4-dihydro-1H-pyrrolo[1,2-a]pyrazin-2-yl)-methanone,
(6-Chloro-pyrazolo[1,5-a]pyridin-2-yl)-(6-thiophen-2-yl-3,4-dihydro-1H-pyrrolo[1,2-a]pyrazin-2-yl)-methanone,
(6-Bromo-pyrazolo[1,5-a]pyridin-2-yl)-(1-methyl-6-thiophen-2-yl-3,4-dihydro-1H-pyrrolo[1,2-a]pyrazin-2-yl)-methanone,
(6-Bromo-pyrazolo[1,5-a]pyridin-2-yl)-(6-thiophen-2-yl-3,4-dihydro-1H-pyrrolo[1,2-a]pyrazin-2-yl)-methanone,
(6-Chloro-pyrazolo[1,5-a]pyridin-2-yl)-(6-furan-2-yl-1-methyl-3,4-dihydro-1H-pyrrolo[1,2-a]pyrazin-2-yl)-methanone,
(6-Chloro-pyrazolo[1,5-a]pyridin-2-yl)-(6-furan-2-yl-3,4-dihydro-1H-pyrrolo[1,2-a]pyrazin-2-yl)-methanone,
(6-Bromo-pyrazolo[1,5-a]pyridin-2-yl)-(6-furan-2-yl-1-methyl-3,4-dihydro-1H-pyrrolo[1,2-a]pyrazin-2-yl)-methanone,
(6-Bromo-pyrazolo[1,5-a]pyridin-2-yl)-(6-furan-2-yl-3,4-dihydro-1H-pyrrolo[1,2-a]pyrazin-2-yl)-methanone,
(6-Chloro-pyrazolo[1,5-a]pyridin-2-yl)-(1-methyl-6-thiophen-3-yl-3,4-dihydro-1H-pyrrolo[1,2-a]pyrazin-2-yl)-methanone,
(6-Chloro-pyrazolo[1,5-a]pyridin-2-yl)-(6-thiophen-3-yl-3,4-dihydro-1H-pyrrolo[1,2-a]pyrazin-2-yl)-methanone,
(6-Bromo-pyrazolo[1,5-a]pyridin-2-yl)-(1-methyl-6-thiophen-3-yl-3,4-dihydro-1H-pyrrolo[1,2-a]pyrazin-2-yl)-methanone,
(6-Bromo-pyrazolo[1,5-a]pyridin-2-yl)-(6-thiophen-3-yl-3,4-dihydro-1H-pyrrolo[1,2-a]pyrazin-2-yl)-methanone,
(6-Chloro-pyrazolo[1,5-a]pyridin-2-yl)-(6-furan-3-yl-1-methyl-3,4-dihydro-1H-pyrrolo[1,2-a]pyrazin-2-yl)-methanone,
(6-Chloro-pyrazolo[1,5-a]pyridin-2-yl)-(6-furan-3-yl-3,4-dihydro-1H-pyrrolo[1,2-a]pyrazin-2-yl)-methanone,
(6-Bromo-pyrazolo[1,5-a]pyridin-2-yl)-(6-furan-3-yl-1-methyl-3,4-dihydro-1H-pyrrolo[1,2-a]pyrazin-2-yl)-methanone,
(6-Bromo-pyrazolo[1,5-a]pyridin-2-yl)-(6-furan-3-yl-3,4-dihydro-1H-pyrrolo[1,2-a]pyrazin-2-yl)-methanone,
(6-Chloro-pyrazolo[1,5-a]pyridin-2-yl)-(1-methyl-6-pyrimidin-4-yl-3,4-dihydro-1H-pyrrolo[1,2-a]pyrazin-2-yl)-methanone,
(6-Chloro-pyrazolo[1,5-a]pyridin-2-yl)-(6-pyrimidin-4-yl-3,4-dihydro-1H-pyrrolo[1,2-a]pyrazin-2-yl)-methanone,
(6-Bromo-pyrazolo[1,5-a]pyridin-2-yl)-(1-methyl-6-pyrimidin-4-yl-3,4-dihydro-1H-pyrrolo[1,2-a]pyrazin-2-yl)-methanone,
(6-Bromo-pyrazolo[1,5-a]pyridin-2-yl)-(6-pyrimidin-4-yl-3,4-dihydro-1H-pyrrolo[1,2-a]pyrazin-2-yl)-methanone,
(6-Chloro-pyrazolo[1,5-a]pyridin-2-yl)-(1-methyl-6-pyrimidin-5-yl-3,4-dihydro-1H-pyrrolo[1,2-a]pyrazin-2-yl)-methanone,
(6-Chloro-pyrazolo[1,5-a]pyridin-2-yl)-(6-pyrimidin-5-yl-3,4-dihydro-1H-pyrrolo[1,2-a]pyrazin-2-yl)-methanone,
(6-Bromo-pyrazolo[1,5-a]pyridin-2-yl)-(1-methyl-6-pyrimidin-5-yl-3,4-dihydro-1H-pyrrolo[1,2-a]pyrazin-2-yl)-methanone,
(6-Bromo-pyrazolo[1,5-a]pyridin-2-yl)-(6-pyrimidin-5-yl-3,4-dihydro-1H-pyrrolo[1,2-a]pyrazin-2-yl)-methanone,
(6-Chloro-pyrazolo[1,5-a]pyridin-2-yl)-(8-methyl-3-pyridin-2-yl-5,6-dihydro-8H-[1,2,4]triazolo[4,3-a]pyrazin-7-yl)-methanone,
(6-Chloro-pyrazolo[1,5-a]pyridin-2-yl)-(3-pyridin-2-yl-5,6-dihydro-8H-[1,2,4]triazolo[4,3-a]pyrazin-7-yl)-methanone,
(6-Bromo-pyrazolo[1,5-a]pyridin-2-yl)-(8-methyl-3-pyridin-2-yl-5,6-dihydro-8H-[1,2,4]triazolo[4,3-a]pyrazin-7-yl)-methanone,
(6-Bromo-pyrazolo[1,5-a]pyridin-2-yl)-(3-pyridin-2-yl-5,6-dihydro-8H-[1,2,4]triazolo[4,3-a]pyrazin-7-yl)-methanone,
(6-Chloro-pyrazolo[1,5-a]pyridin-2-yl)-(8-methyl-3-pyridin-3-yl-5,6-dihydro-8H-[1,2,4]triazolo[4,3-a]pyrazin-7-yl)-methanone,
(6-Chloro-pyrazolo[1,5-a]pyridin-2-yl)-(3-pyridin-3-yl-5,6-dihydro-8H-[1,2,4]triazolo[4,3-a]pyrazin-7-yl)-methanone,
(6-Bromo-pyrazolo[1,5-a]pyridin-2-yl)-(8-methyl-3-pyridin-3-yl-5,6-dihydro-8H-[1,2,4]triazolo[4,3-a]pyrazin-7-yl)-methanone,
(6-Bromo-pyrazolo[1,5-a]pyridin-2-yl)-(3-pyridin-3-yl-5,6-dihydro-8H-[1,2,4]triazolo[4,3-a]pyrazin-7-yl)-methanone,
(6-Chloro-pyrazolo[1,5-a]pyridin-2-yl)-(8-methyl-3-pyridin-4-yl-5,6-dihydro-8H-[1,2,4]triazolo[4,3-a]pyrazin-7-yl)-methanone,
(6-Chloro-pyrazolo[1,5-a]pyridin-2-yl)-(3-pyridin-4-yl-5,6-dihydro-8H-[1,2,4]triazolo[4,3-a]pyrazin-7-yl)-methanone,
(6-Bromo-pyrazolo[1,5-a]pyridin-2-yl)-(8-methyl-3-pyridin-4-yl-5,6-dihydro-8H-[1,2,4]triazolo[4,3-a]pyrazin-7-yl)-methanone,
(6-Bromo-pyrazolo[1,5-a]pyridin-2-yl)-(3-pyridin-4-yl-5,6-dihydro-8H-[1,2,4]triazolo[4,3-a]pyrazin-7-yl)-methanone,
(6-Chloro-pyrazolo[1,5-a]pyridin-2-yl)-(8-methyl-3-thiophen-2-yl-5,6-dihydro-8H-[1,2,4]triazolo[4,3-a]pyrazin-7-yl)-methanone,
(6-Chloro-pyrazolo[1,5-a]pyridin-2-yl)-(3-thiophen-2-yl-5,6-dihydro-8H-[1,2,4]triazolo[4,3-a]pyrazin-7-yl)-methanone,
(6-Bromo-pyrazolo[1,5-a]pyridin-2-yl)-(8-methyl-3-thiophen-2-yl-5,6-dihydro-8H-[1,2,4]triazolo[4,3-a]pyrazin-7-yl)-methanone,
(6-Bromo-pyrazolo[1,5-a]pyridin-2-yl)-(3-thiophen-2-yl-5,6-dihydro-8H-[1,2,4]triazolo[4,3-a]pyrazin-7-yl)-methanone,
(6-Chloro-pyrazolo[1,5-a]pyridin-2-yl)-(3-furan-2-yl-8-methyl-5,6-dihydro-8H-[1,2,4]triazolo[4,3-a]pyrazin-7-yl)-methanone,
(6-Chloro-pyrazolo[1,5-a]pyridin-2-yl)-(3-furan-2-yl-5,6-dihydro-8H-[1,2,4]triazolo[4,3-a]pyrazin-7-yl)-methanone,
(6-Bromo-pyrazolo[1,5-a]pyridin-2-yl)-(3-furan-2-yl-8-methyl-5,6-dihydro-8H-[1,2,4]triazolo[4,3-a]pyrazin-7-yl)-methanone,
(6-Bromo-pyrazolo[1,5-a]pyridin-2-yl)-(3-furan-2-yl-5,6-dihydro-8H-[1,2,4]triazolo[4,3-a]pyrazin-7-yl)-methanone,
(6-Chloro-pyrazolo[1,5-a]pyridin-2-yl)-(8-methyl-3-thiophen-3-yl-5,6-dihydro-8H-[1,2,4]triazolo[4,3-a]pyrazin-7-yl)-methanone,
(6-Chloro-pyrazolo[1,5-a]pyridin-2-yl)-(3-thiophen-3-yl-5,6-dihydro-8H-[1,2,4]triazolo[4,3-a]pyrazin-7-yl)-methanone,
(6-Bromo-pyrazolo[1,5-a]pyridin-2-yl)-(8-methyl-3-thiophen-3-yl-5,6-dihydro-8H-[1,2,4]triazolo[4,3-a]pyrazin-7-yl)-methanone,
(6-Bromo-pyrazolo[1,5-a]pyridin-2-yl)-(3-thiophen-3-yl-5,6-dihydro-8H-[1,2,4]triazolo[4,3-a]pyrazin-7-yl)-methanone,
(6-Chloro-pyrazolo[1,5-a]pyridin-2-yl)-(3-furan-3-yl-8-methyl-5,6-dihydro-8H-[1,2,4]triazolo[4,3-a]pyrazin-7-yl)-methanone,
(6-Chloro-pyrazolo[1,5-a]pyridin-2-yl)-(3-furan-3-yl-5,6-dihydro-8H-[1,2,4]triazolo[4,3-a]pyrazin-7-yl)-methanone,
(6-Bromo-pyrazolo[1,5-a]pyridin-2-yl)-(3-furan-3-yl-8-methyl-5,6-dihydro-8H-[1,2,4]triazolo[4,3-a]pyrazin-7-yl)-methanone,
(6-Bromo-pyrazolo[1,5-a]pyridin-2-yl)-(3-furan-3-yl-5,6-dihydro-8H-[1,2,4]triazolo[4,3-a]pyrazin-7-yl)-methanone,
(6-Chloro-pyrazolo[1,5-a]pyridin-2-yl)-(8-methyl-3-pyrimidin-4-yl-5,6-dihydro-8H-[1,2,4]triazolo[4,3-a]pyrazin-7-yl)-methanone,
(6-Chloro-pyrazolo[1,5-a]pyridin-2-yl)-(3-pyrimidin-4-yl-5,6-dihydro-8H-[1,2,4]triazolo[4,3-a]pyrazin-7-yl)-methanone,
(6-Bromo-pyrazolo[1,5-a]pyridin-2-yl)-(8-methyl-3-pyrimidin-4-yl-5,6-dihydro-8H-[1,2,4]triazolo[4,3-a]pyrazin-7-yl)-methanone,
(6-Bromo-pyrazolo[1,5-a]pyridin-2-yl)-(3-pyrimidin-4-yl-5,6-dihydro-8H-[1,2,4]triazolo[4,3-a]pyrazin-7-yl)-methanone,
(6-Chloro-pyrazolo[1,5-a]pyridin-2-yl)-(8-methyl-3-pyrimidin-5-yl-5,6-dihydro-8H-[1,2,4]triazolo[4,3-a]pyrazin-7-yl)-methanone,
(6-Chloro-pyrazolo[1,5-a]pyridin-2-yl)-(3-pyrimidin-5-yl-5,6-dihydro-8H-[1,2,4]triazolo[4,3-a]pyrazin-7-yl)-methanone,
(6-Bromo-pyrazolo[1,5-a]pyridin-2-yl)-(8-methyl-3-pyrimidin-5-yl-5,6-dihydro-8H-[1,2,4]triazolo[4,3-a]pyrazin-7-yl)-methanone,
(6-Bromo-pyrazolo[1,5-a]pyridin-2-yl)-(3-pyrimidin-5-yl-5,6-dihydro-8H-[1,2,4]triazolo[4,3-a]pyrazin-7-yl)-methanone,
(6-Chloro-pyrazolo[1,5-a]pyridin-2-yl)-[3-(6-fluoro-pyridin-3-yl)-8-methyl-5,6-dihydro-8H-imidazo[1,2-a]pyrazin-7-yl]-methanone,
(6-Chloro-pyrazolo[1,5-a]pyridin-2-yl)-[3-(6-fluoro-pyridin-3-yl)-5,6-dihydro-8H-imidazo[1,2-a]pyrazin-7-yl]-methanone,
(6-Bromo-pyrazolo[1,5-a]pyridin-2-yl)-[3-(6-fluoro-pyridin-3-yl)-8-methyl-5,6-dihydro-8H-imidazo[1,2-a]pyrazin-7-yl]-methanone,
(6-Bromo-pyrazolo[1,5-a]pyridin-2-yl)-[3-(6-fluoro-pyridin-3-yl)-5,6-dihydro-8H-imidazo[1,2-a]pyrazin-7-yl]-methanone,
(6-Chloro-pyrazolo[1,5-a]pyridin-2-yl)-[3-(2-fluoro-pyridin-3-yl)-8-methyl-5,6-dihydro-8H-imidazo[1,2-a]pyrazin-7-yl]-methanone,
(6-Chloro-pyrazolo[1,5-a]pyridin-2-yl)-[3-(2-fluoro-pyridin-3-yl)-5,6-dihydro-8H-imidazo[1,2-a]pyrazin-7-yl]-methanone,
(6-Bromo-pyrazolo[1,5-a]pyridin-2-yl)-[3-(2-fluoro-pyridin-3-yl)-8-methyl-5,6-dihydro-8H-imidazo[1,2-a]pyrazin-7-yl]-methanone,
(6-Bromo-pyrazolo[1,5-a]pyridin-2-yl)-[3-(2-fluoro-pyridin-3-yl)-5,6-dihydro-8H-imidazo[1,2-a]pyrazin-7-yl]-methanone,
(6-Chloro-pyrazolo[1,5-a]pyridin-2-yl)-[2-(6-fluoro-pyridin-3-yl)-8-methyl-5,6-dihydro-8H-imidazo[1,2-a]pyrazin-7-yl]-methanone,
(6-Chloro-pyrazolo[1,5-a]pyridin-2-yl)-[2-(6-fluoro-pyridin-3-yl)-5,6-dihydro-8H-imidazo[1,2-a]pyrazin-7-yl]-methanone,
(6-Bromo-pyrazolo[1,5-a]pyridin-2-yl)-[2-(6-fluoro-pyridin-3-yl)-8-methyl-5,6-dihydro-8H-imidazo[1,2-a]pyrazin-7-yl]-methanone,
(6-Bromo-pyrazolo[1,5-a]pyridin-2-yl)-[2-(6-fluoro-pyridin-3-yl)-5,6-dihydro-8H-imidazo[1,2-a]pyrazin-7-yl]-methanone,
(6-Chloro-pyrazolo[1,5-a]pyridin-2-yl)-[2-(2-fluoro-pyridin-3-yl)-8-methyl-5,6-dihydro-8H-imidazo[1,2-a]pyrazin-7-yl]-methanone,
(6-Chloro-pyrazolo[1,5-a]pyridin-2-yl)-[2-(2-fluoro-pyridin-3-yl)-5,6-dihydro-8H-imidazo[1,2-a]pyrazin-7-yl]-methanone,
(6-Bromo-pyrazolo[1,5-a]pyridin-2-yl)-[2-(2-fluoro-pyridin-3-yl)-8-methyl-5,6-dihydro-8H-imidazo[1,2-a]pyrazin-7-yl]-methanone,
(6-Bromo-pyrazolo[1,5-a]pyridin-2-yl)-[2-(2-fluoro-pyridin-3-yl)-5,6-dihydro-8H-imidazo[1,2-a]pyrazin-7-yl]-methanone,
(6-Chloro-pyrazolo[1,5-a]pyridin-2-yl)-[3-(6-fluoro-pyridin-3-yl)-8-methyl-5,6-dihydro-8H-[1,2,4]triazolo[4,3-a]pyrazin-7-yl]-methanone,
(6-Chloro-pyrazolo[1,5-a]pyridin-2-yl)-[3-(6-fluoro-pyridin-3-yl)-5,6-dihydro-8H-[1,2,4]triazolo[4,3-a]pyrazin-7-yl]-methanone,
(6-Bromo-pyrazolo[1,5-a]pyridin-2-yl)-[3-(6-fluoro-pyridin-3-yl)-8-methyl-5,6-dihydro-8H-[1,2,4]triazolo[4,3-a]pyrazin-7-yl]-methanone,
(6-Bromo-pyrazolo[1,5-a]pyridin-2-yl)-[3-(6-fluoro-pyridin-3-yl)-5,6-dihydro-8H-[1,2,4]triazolo[4,3-a]pyrazin-7-yl]-methanone,
(6-Chloro-pyrazolo[1,5-a]pyridin-2-yl)-[3-(2-fluoro-pyridin-3-yl)-8-methyl-5,6-dihydro-8H-[1,2,4]triazolo[4,3-a]pyrazin-7-yl]-methanone,
(6-Chloro-pyrazolo[1,5-a]pyridin-2-yl)-[3-(2-fluoro-pyridin-3-yl)-5,6-dihydro-8H-[1,2,4]triazolo[4,3-a]pyrazin-7-yl]-methanone,
(6-Bromo-pyrazolo[1,5-a]pyridin-2-yl)-[3-(2-fluoro-pyridin-3-yl)-8-methyl-5,6-dihydro-8H-[1,2,4]triazolo[4,3-a]pyrazin-7-yl]-methanone,
(6-Bromo-pyrazolo[1,5-a]pyridin-2-yl)-[3-(2-fluoro-pyridin-3-yl)-5,6-dihydro-8H-[1,2,4]triazolo[4,3-a]pyrazin-7-yl]-methanone,
(6-Chloro-pyrazolo[1,5-a]pyridin-2-yl)-(1-methyl-3,4-dihydro-1H-cinnolin-2-yl)-methanone,
(6-Bromo-pyrazolo[1,5-a]pyridin-2-yl)-(1-methyl-3,4-dihydro-1H-cinnolin-2-yl)-methanone,
(6-Chloro-pyrazolo[1,5-a]pyridin-2-yl)-(1-methyl-3,4-dihydro-1H-thieno[3,2-c]pyridazin-2-yl)-methanone,
(6-Bromo-pyrazolo[1,5-a]pyridin-2-yl)-(1-methyl-3,4-dihydro-1H-thieno[3,2-c]pyridazin-2-yl)-methanone,
(6-Chloro-pyrazolo[1,5-a]pyridin-2-yl)-(1,6-dimethyl-3,4-dihydro-1H-thieno[3,2-c]pyridazin-2-yl)-methanone,
(6-Bromo-pyrazolo[1,5-a]pyridin-2-yl)-(1,6-dimethyl-3,4-dihydro-1H-thieno[3,2-c]pyridazin-2-yl)-methanone,
(6-Chloro-pyrazolo[1,5-a]pyridin-2-yl)-(1,7-dimethyl-3,4-dihydro-1H-thieno[3,2-c]pyridazin-2-yl)-methanone,
(6-Bromo-pyrazolo[1,5-a]pyridin-2-yl)-(1,7-dimethyl-3,4-dihydro-1H-thieno[3,2-c]pyridazin-2-yl)-methanone,
(6-Chloro-pyrazolo[1,5-a]pyridin-2-yl)-(1,6,7-trimethyl-3,4-dihydro-1H-thieno[3,2-c]pyridazin-2-yl)-methanone,
(6-Bromo-pyrazolo[1,5-a]pyridin-2-yl)-(1,6,7-trimethyl-3,4-dihydro-1H-thieno[3,2-c]pyridazin-2-yl)-methanone,
(6-Bromo-1-methyl-3,4-dihydro-1H-thieno[3,2-c]pyridazin-2-yl)-(6-chloro-pyrazolo[1,5-a]pyridin-2-yl)-methanone,
(6-Bromo-1-methyl-3,4-dihydro-1H-thieno[3,2-c]pyridazin-2-yl)-(6-bromo-pyrazolo[1,5-a]pyridin-2-yl)-methanone,
(6-Chloro-1-methyl-3,4-dihydro-1H-thieno[3,2-c]pyridazin-2-yl)-(6-chloro-pyrazolo[1,5-a]pyridin-2-yl)-methanone,
(6-Bromo-1-methyl-3,4-dihydro-1H-thieno[3,2-c]pyridazin-2-yl)-(6-chloro-pyrazolo[1,5-a]pyridin-2-yl)-methanone,
(6-Chloro-pyrazolo[1,5-a]pyridin-2-yl)-(1-methyl-3,4-dihydro-1H-furo[3,2-c]pyridazin-2-yl)-methanone,
(6-Bromo-pyrazolo[1,5-a]pyridin-2-yl)-(1-methyl-3,4-dihydro-1H-furo[3,2-c]pyridazin-2-yl)-methanone,
(6-Chloro-pyrazolo[1,5-a]pyridin-2-yl)-(1,6-dimethyl-3,4-dihydro-1H-furo[3,2-c]pyridazin-2-yl)-methanone,
(6-Bromo-pyrazolo[1,5-a]pyridin-2-yl)-(1,6-dimethyl-3,4-dihydro-1H-furo[3,2-c]pyridazin-2-yl)-methanone,
(6-Chloro-pyrazolo[1,5-a]pyridin-2-yl)-(1,7-dimethyl-3,4-dihydro-1H-furo[3,2-c]pyridazin-2-yl)-methanone,
(6-Bromo-pyrazolo[1,5-a]pyridin-2-yl)-(1,7-dimethyl-3,4-dihydro-1H-furo[3,2-c]pyridazin-2-yl)-methanone,
(6-Chloro-pyrazolo[1,5-a]pyridin-2-yl)-(1,6,7-trimethyl-3,4-dihydro-1H-furo[3,2-c]pyridazin-2-yl)-methanone,
(6-Bromo-pyrazolo[1,5-a]pyridin-2-yl)-(1,6,7-trimethyl-3,4-dihydro-1H-furo[3,2-c]pyridazin-2-yl)-methanone,
(6-Bromo-1-methyl-3,4-dihydro-1H-furo[3,2-c]pyridazin-2-yl)-(6-chloro-pyrazolo[1,5-a]pyridin-2-yl)-methanone,
(6-Bromo-1-methyl-3,4-dihydro-1H-furo[3,2-c]pyridazin-2-yl)-(6-bromo-pyrazolo[1,5-a]pyridin-2-yl)-methanone,
(6-Chloro-1-methyl-3,4-dihydro-1H-furo[3,2-c]pyridazin-2-yl)-(6-chloro-pyrazolo[1,5-a]pyridin-2-yl)-methanone,
(6-Bromo-1-methyl-3,4-dihydro-1H-furo[3,2-c]pyridazin-2-yl)-(6-chloro-pyrazolo[1,5-a]pyridin-2-yl)-methanone,
(6-Chloro-pyrazolo[1,5-a]pyridin-2-yl)-(1-methyl-1,3,4,5-tetrahydro-pyrrolo[3,2-c]pyridazin-2-yl)-methanone,
(6-Bromo-pyrazolo[1,5-a]pyridin-2-yl)-(1-methyl-1,3,4,5-tetrahydro-pyrrolo[3,2-c]pyridazin-2-yl)-methanone,
(6-Chloro-pyrazolo[1,5-a]pyridin-2-yl)-(1,6-dimethyl-1,3,4,5-tetrahydro-pyrrolo[3,2-c]pyridazin-2-yl)-methanone,
(6-Bromo-pyrazolo[,5-a]pyridm-2-yl)-(1,6-dimethyl-1,3,4,5-tetrahydro-pyrrolo[3,2-c]pyridazin-2-yl)-methanone,
(6-Chloro-pyrazolo[ 1,5-a]pyridin-2-yl)-(1,7-dimethyl-1,3,4,5-tetrahydro-pyrrolo[3,2-c]pyridazin-2-yl)-methanone,
(6-Bromo-pyrazolo[1,5-a]pyridin-2-yl)-(1,7-dimethyl-1,3,4,5-tetrahydro-pyrrolo[3,2-c]pyridazin-2-yl)-methanone,
(6-Chloro-pyrazolo[1,5-a]pyridin-2-yl)-(1,6,7-trimethyl-1,3,4,5-tetrahydro-pyrrolo[3,2-c]pyridazin-2-yl)-methanone,
(6-Bromo-pyrazolo[1,5-a]pyridin-2-yl)-(1,6,7-trimethyl-1,3,4,5-tetrahydro-pyrrolo[3,2-c]pyridazin-2-yl)-methanone,
(6-Bromo-1-methyl-1,3,4,5-tetrahydro-pyrrolo[3,2-c]pyridazin-2-yl)-(6-chloro-pyrazolo[1,5-a]pyridin-2-yl)-methanone,
(6-Bromo-1-methyl-1,3,4,5-tetrahydro-pyrrolo[3,2-c]pyridazin-2-yl)-(6-bromo-pyrazolo[1,5-a]pyridin-2-yl)-methanone,
(6-Chloro-1-methyl-1,3,4,5-tetrahydro-pyrrolo[3,2-c]pyridazin-2-yl)-(6-chloro-pyrazolo[1,5-a]pyridin-2-yl)-methanone,
(6-Bromo-1-methyl-1,3,4,5-tetrahydro-pyrrolo[3,2-c]pyridazin-2-yl)-(6-chloro-pyrazolo[1,5-a]pyridin-2-yl)-methanone,
(8-Chloro-1-methyl-3,4-dihydro-1H-cinnolin-2-yl)-(6-chloro-pyrazolo[1,5-a]pyridin-2-yl)-methanone,
(8-Bromo-1-methyl-3,4-dihydro-1H-cinnolin-2-yl)-(6-chloro-pyrazolo[1,5-a]pyridin-2-yl)-methanone,
(7-Chloro-1-methyl-3,4-dihydro-1H-cinnolin-2-yl)-(6-chloro-pyrazolo[1,5-a]pyridin-2-yl)-methanone,
(7-Bromo-1-methyl-3,4-dihydro-1H-cinnolin-2-yl)-(6-chloro-pyrazolo[1,5-a]pyridin-2-yl)-methanone,
(6-Chloro-1-methyl-3,4-dihydro-1H-cinnolin-2-yl)-(6-chloro-pyrazolo[1,5-a]pyridin-2-yl)-methanone,
(6-Bromo-1-methyl-3,4-dihydro-1H-cinnolin-2-yl)-(6-chloro-pyrazolo[1,5-a]pyridin-2-yl)-methanone,
(5-Chloro-1-methyl-3,4-dihydro-1H-cinnolin-2-yl)-(6-chloro-pyrazolo[1,5-a]pyridin-2-yl)-methanone,
(5-Bromo-1-methyl-3,4-dihydro-1H-cinnolin-2-yl)-(6-chloro-pyrazolo[1,5-a]pyridin-2-yl)-methanone,
(8-Bromo-1-methyl-3,4-dihydro-1H-cinnolin-2-yl)-(6-chloro-pyrazolo[1,5-a]pyridin-2-yl)-methanone,
(8-Bromo-1-methyl-3,4-dihydro-1H-cinnolin-2-yl)-(6-bromo-pyrazolo[1,5-a]pyridin-2-yl)-methanone,
(7-Bromo-1-methyl-3,4-dihydro-1H-cinnolin-2-yl)-(6-chloro-pyrazolo[1,5-a]pyridin-2-yl)-methanone,
(7-Bromo-1-methyl-3,4-dihydro-1H-cinnolin-2-yl)-(6-bromo-pyrazolo[1,5-a]pyridin-2-yl)-methanone,
(6-Bromo-1-methyl-3,4-dihydro-1H-cinnolin-2-yl)-(6-chloro-pyrazolo[1,5-a]pyridin-2-yl)-methanone,
(6-Bromo-1-methyl-3,4-dihydro-1H-cinnolin-2-yl)-(6-chloro-pyrazolo[1,5-a]pyridin-2-yl)-methanone,
(5-Bromo-1-methyl-3,4-dihydro-1H-cinnolin-2-yl)-(6-chloro-pyrazolo[1,5-a]pyridin-2-yl)-methanone,
(5-Bromo-1-methyl-3,4-dihydro-1H-cinnolin-2-yl)-(6-chloro-pyrazolo[1,5-a]pyridin-2-yl)-methanone,
(8-Fluoro-1-methyl-3,4-dihydro-1H-cinnolin-2-yl)-(6-chloro-pyrazolo[1,5-a]pyridin-2-yl)-methanone,
(8-Fluoro-1-methyl-3,4-dihydro-1H-cinnolin-2-yl)-(6-bromo-pyrazolo[1,5-a]pyridin-2-yl)-methanone,
(7-Fluoro-1-methyl-3,4-dihydro-1H-cinnolin-2-yl)-(6-chloro-pyrazolo[1,5-a]pyridin-2-yl)-methanone,
(7-Fluoro-1-methyl-3,4-dihydro-1H-cinnolin-2-yl)-(6-bromo-pyrazolo[1,5-a]pyridin-2-yl)-methanone,
(6-Fluoro-1-methyl-3,4-dihydro-1H-cinnolin-2-yl)-(6-chloro-pyrazolo[1,5-a]pyridin-2-yl)-methanone,
(6-Fluoro-1-methyl-3,4-dihydro-1H-cinnolin-2-yl)-(6-bromo-pyrazolo[1,5-a]pyridin-2-yl)-methanone,
(5-Fluoro-1-methyl-3,4-dihydro-1H-cinnolin-2-yl)-(6-chloro-pyrazolo[1,5-a]pyridin-2-yl)-methanone,
(5-Fluoro-1-methyl-3,4-dihydro-1H-cinnolin-2-yl)-(6-bromo-pyrazolo[1,5-a]pyridin-2-yl)-methanone,
(6-Chloro-pyrazolo[1,5-a]pyridin-2-yl)-(1,8-dimethyl-3,4-dihydro-1H-cinnolin-2-yl)-methanone,
(6-Bromo-pyrazolo[1,5-a]pyridin-2-yl)-(1,8-dimethyl-3,4-dihydro-1H-cinnolin-2-yl)-methanone,
(6-Chloro-pyrazolo[1,5-a]pyridin-2-yl)-(1,7-dimethyl-3,4-dihydro-1H-cinnolin-2-yl)-methanone,
(6-Bromo-pyrazolo[1,5-a]pyridin-2-yl)-(1,7-dimethyl-3,4-dihydro-1H-cinnolin-2-yl)-methanone,
(6-Chloro-pyrazolo[1,5-a]pyridin-2-yl)-(1,6-dimethyl-3,4-dihydro-1H-cinnolin-2-yl)-methanone,
(6-Bromo-pyrazolo[1,5-a]pyridin-2-yl)-(1,6-dimethyl-3,4-dihydro-1H-cinnolin-2-yl)-methanone,
(6-Chloro-pyrazolo[1,5-a]pyridin-2-yl)-(1,5-dimethyl-3,4-dihydro-1H-cinnolin-2-yl)-methanone,
(6-Bromo-pyrazolo[1,5-a]pyridin-2-yl)-(1,5-dimethyl-3,4-dihydro-1H-cinnolin-2-yl)-methanone,
(6-Chloro-pyrazolo[1,5-a]pyridin-2-yl)-(8-methoxy-1-methyl-3,4-dihydro-1H-cinnolin-2-yl)-methanone,
(6-Bromo-pyrazolo[1,5-a]pyridin-2-yl)-(8-methoxy-1-methyl-3,4-dihydro-1H-cinnolin-2-yl)-methanone,
(6-Chloro-pyrazolo[1,5-a]pyridin-2-yl)-(7-methoxy-1-methyl-3,4-dihydro-1H-cinnolin-2-yl)-methanone,
(6-Bromo-pyrazolo[1,5-a]pyridin-2-yl)-(7-methoxy-1-methyl-3,4-dihydro-1H-cinnolin-2-yl)-methanone,
(6-Chloro-pyrazolo[1,5-a]pyridin-2-yl)-(6-methoxy-1-methyl-3,4-dihydro-1H-cinnolin-2-yl)-methanone,
(6-Bromo-pyrazolo[1,5-a]pyridin-2-yl)-(6-methoxy-1-methyl-3,4-dihydro-1H-cinnolin-2-yl)-methanone,
(6-Chloro-pyrazolo[1,5-a]pyridin-2-yl)-(5-methoxy-1-methyl-3,4-dihydro-1H-cinnolin-2-yl)-methanone,
(6-Bromo-pyrazolo[1,5-a]pyridin-2-yl)-(5-methoxy-1-methyl-3,4-dihydro-1H-cinnolin-2-yl)-methanone,
(6-Chloro-pyrazolo[1,5-a]pyridin-2-yl)-(1-methyl-8-trifluoromethyl-3,4-dihydro-1H-cinnolin-2-yl)-methanone,
(6-Bromo-pyrazolo[1,5-a]pyridin-2-yl)-(1-methyl-8-trifluoromethyl-3,4-dihydro-1H-cinnolin-2-yl)-methanone,
(6-Chloro-pyrazolo[1,5-a]pyridin-2-yl)-(1-methyl-7-trifluoromethyl-3,4-dihydro-1H-cinnolin-2-yl)-methanone,
(6-Bromo-pyrazolo[1,5-a]pyridin-2-yl)-(1-methyl-7-trifluoromethyl-3,4-dihydro-1H-cinnolin-2-yl)-methanone,
(6-Chloro-pyrazolo[1,5-a]pyridin-2-yl)-(1-methyl-6-trifluoromethyl-3,4-dihydro-1H-cinnolin-2-yl)-methanone,
(6-Bromo-pyrazolo[1,5-a]pyridin-2-yl)-(1-methyl-6-trifluoromethyl-3,4-dihydro-1H-cinnolin-2-yl)-methanone,
(6-Chloro-pyrazolo[1,5-a]pyridin-2-yl)-(1-methyl-5-trifluoromethyl-3,4-dihydro-1H-cinnolin-2-yl)-methanone,
(6-Bromo-pyrazolo[1,5-a]pyridin-2-yl)-(1-methyl-5-trifluoromethyl-3,4-dihydro-1H-cinnolin-2-yl)-methanone,
N-[2-(6-Chloro-pyrazolo[1,5-a]pyridine-2-carbonyl)-1-methyl-1,2,3,4-tetrahydro-cinnolin-8-yl]-acetamide,
N-[2-(6-Bromo-pyrazolo[1,5-a]pyridine-2-carbonyl)-1-methyl-1,2,3,4-tetrahydro-cinnolin-8-yl]-acetamide,
N-[2-(6-Chloro-pyrazolo[1,5-a]pyridine-2-carbonyl)-1-methyl-1,2,3,4-tetrahydro-cinnolin-7-yl]-acetamide,
N-[2-(6-Bromo-pyrazolo[1,5-a]pyridine-2-carbonyl)-1-methyl-1,2,3,4-tetrahydro-cinnolin-7-yl]-acetamide,
N-[2-(6-Chloro-pyrazolo[1,5-a]pyridine-2-carbonyl)-1-methyl-1,2,3,4-tetrahydro-cinnolin-6-yl]-acetamide,
N-[2-(6-Bromo-pyrazolo[1,5-a]pyridine-2-carbonyl)-1-methyl-1,2,3,4-tetrahydro-cinnolin-6-yl]-acetamide,
N-[2-(6-Chloro-pyrazolo[1,5-a]pyridine-2-carbonyl)-1-methyl-1,2,3,4-tetrahydro-cinnolin-5-yl]-acetamide,
N-[2-(6-Bromo-pyrazolo[1,5-a]pyridine-2-carbonyl)-1-methyl-1,2,3,4-tetrahydro-cinnolin-5-yl]-acetamide,
(6-Chloro-pyrazolo[1,5-a]pyridin-2-yl)-(8-methanesulfonyl-1-methyl-3,4-dihydro-1H-cinnolin-2-yl)-methanone,
(6-Bromo-pyrazolo[1,5-a]pyridin-2-yl)-(8-methanesulfonyl-1-methyl-3,4-dihydro-1H-cinnolin-2-yl)-methanone,
(6-Chloro-pyrazolo[11,5-a]pyridin-2-yl)-(7-methanesulfonyl-1-methyl-3,4-dihydro-1H-cinnolin-2-yl)-methanone,
(6-Bromo-pyrazolo[1,5-a]pyridin-2-yl)-(7-methanesulfonyl-1-methyl-3,4-dihydro-1H-cinnolin-2-yl)-methanone,
(6-Chloro-pyrazolo[1,5-a]pyridin-2-yl)-(6-methanesulfonyl-1-methyl-3,4-dihydro-1H-cinnolin-2-yl)-methanone, .
(6-Bromo-pyrazolo[1,5-a]pyridin-2-yl)-(6-methanesulfonyl-1-methyl-3,4-dihydro-1H-cinnolin-2-yl)-methanone,
(6-Chloro-pyrazolo[1,5-a]pyridin-2-yl)-(5-methanesulfonyl-1-methyl-3,4-dihydro-1H-cinnolin-2-yl)-methanone,
(6-Bromo-pyrazolo[1,5-a]pyridin-2-yl)-(5-methanesulfonyl-1-methyl-3,4-dihydro-1H-cinnolin-2-yl)-methanone,
(6-Chloro-pyrazolo[1,5-a]pyridin-2-yl)-(1-methyl-3,4-dihydro-1H-pyrido[2,3-c]pyridazin-2-yl)-methanone,
(6-Bromo-pyrazolo[1,5-a]pyridin-2-yl)-(1-methyl-3,4-dihydro-1H-pyrido[2,3-c]pyridazin-2-yl)-methanone,
(6-Chloro-pyrazolo[1,5-a]pyridin-2-yl)-(1-methyl-3,4-dihydro-1H-pyrido[3,4-c]pyridazin-2-yl)-methanone,
(6-Bromo-pyrazolo[1,5-a]pyridin-2-yl)-(1-methyl-3,4-dihydro-1H-pyrido[3,4-c]pyridazin-2-yl)-methanone,
(6-Chloro-pyrazolo[1,5-a]pyridin-2-yl)-(1-methyl-3,4-dihydro-1H-pyrido[4,3-c]pyridazin-2-yl)-methanone,
(6-Bromo-pyrazolo[1,5-a]pyridin-2-yl)-(1-methyl-3,4-dihydro-1H-pyrido[4,3-c]pyridazin-2-yl)-methanone,
(6-Chloro-pyrazolo[1,5-a]pyridin-2-yl)-(1-methyl-3,4-dihydro-1H-pyrido[3,2-c]pyridazin-2-yl)-methanone,
(6-Bromo-pyrazolo[1,5-a]pyridin-2-yl)-(1-methyl-3,4-dihydro-1H-pyrido[3,2-c]pyridazin-2-yl)-methanone,
(7-Bromo-1-methyl-3,4-dihydro-1H-pyrido[3,2-c]pyridazin-2-yl)-(6-chloro-pyrazolo[1,5-a]pyridin-2-yl)-methanone,
(7-Bromo-1-methyl-3,4-dihydro-1H-pyrido[3,2-c]pyridazin-2-yl)-(6-bromo-pyrazolo[1,5-a]pyridin-2-yl)-methanone,
(6-Chloro-pyrazolo[1,5-a]pyridin-2-yl)-(1-methyl-8-pyridin-4-yl-3,4-dihydro-1H-cinnolin-2-yl)-methanone,
(6-Bromo-pyrazolo[1,5-a]pyridin-2-yl)-(1-methyl-8-pyridin-4-yl-3,4-dihydro-1H-cinnolin-2-yl)-methanone,
(6-Chloro-pyrazolo[1,5-a]pyridin-2-yl)-(1-methyl-7-pyridin-4-yl-3,4-dihydro-1H-cinnolin-2-yl)-methanone,
(6-Bromo-pyrazolo[1,5-a]pyridin-2-yl)-(1-methyl-7-pyridin-4-yl-3,4-dihydro-1H-cinnolin-2-yl)-methanone,
(6-Chloro-pyrazolo[1,5-a]pyridin-2-yl)-(1-methyl-6-pyridin-4-yl-3,4-dihydro-1H-cinnolin-2-yl)-methanone,
(6-Bromo-pyrazolo[1,5-a]pyridin-2-yl)-(1-methyl-6-pyridin-4-yl-3,4-dihydro-1H-cinnolin-2-yl)-methanone,
(6-Chloro-pyrazolo[1,5-a]pyridin-2-yl)-(1-methyl-5-pyridin-4-yl-3,4-dihydro-1H-cinnolin-2-yl)-methanone,
(6-Bromo-pyrazolo[1,5-a]pyridin-2-yl)-(1-methyl-5-pyridin-4-yl-3,4-dihydro-1H-cinnolin-2-yl)-methanone,
(6-Chloro-pyrazolo[1,5-a]pyridin-2-yl)-[8-(2-fluoro-pyridin-4-yl)-1-methyl-3,4-dihydro-1H-cinnolin-2-yl]-methanone,
(6-Bromo-pyrazolo[1,5-a]pyridin-2-yl)-[8-(2-fluoro-pyridin-4-yl)-1-methyl-3,4-dihydro-1H-cinnolin-2-yl]-methanone,
(6-Chloro-pyrazolo[1,5-a]pyridin-2-yl)-[7-(2-fluoro-pyridin-4-yl)-1-methyl-3,4-dihydro-1H-cinnolin-2-yl]-methanone,
(6-Bromo-pyrazolo[1,5-a]pyridin-2-yl)-[7-(2-fluoro-pyridin-4-yl)-1-methyl-3,4-dihydro-1H-cinnolin-2-yl]-methanone,
(6-Chloro-pyrazolo[1,5-a]pyridin-2-yl)-[6-(2-fluoro-pyridin-4-yl)-1-methyl-3,4-dihydro-1H-cinnolin-2-yl]-methanone,
(6-Bromo-pyrazolo[1,5-a]pyridin-2-yl)-[6-(2-fluoro-pyridin-4-yl)-1-methyl-3,4-dihydro-1H-cinnolin-2-yl]-methanone,
(6-Chloro-pyrazolo[1,5-a]pyridin-2-yl)-[5-(2-fluoro-pyridin-4-yl)-1-methyl-3,4-dihydro-1H-cinnolin-2-yl]-methanone,
(6-Bromo-pyrazolo[1,5-a]pyridin-2-yl)-[5-(2-fluoro-pyridin-4-yl)-1-methyl-3,4-dihydro-1H-cinnolin-2-yl]-methanone,
6-Bromo-pyrazolo[1,5-a]pyridine-2-carboxylic acid benzylamide,
6-Chloro-pyrazolo[1,5-a]pyridine-2-carboxylic acid benzylamide,
6-Chloro-pyrazolo[1,5-a]pyridine-2-carboxylic acid (1-methyl-1-phenyl-ethyl)-amide,
6-Bromo-pyrazolo[1,5-a]pyridine-2-carboxylic acid (1-methyl-1-phenyl-ethyl)-amide,
6-Bromo-pyrazolo[1,5-a]pyridine-2-carboxylic acid (2-methyl-2-phenyl-propyl)-amide,
6-Chloro-pyrazolo[1,5-a]pyridine-2-carboxylic acid (2-methyl-2-phenyl-propyl)-amide,
6-Chloro-pyrazolo[1,5-a]pyridine-2-carboxylic acid benzyl-methyl-amide,
6-Bromo-pyrazolo[1,5-a]pyridine-2-carboxylic acid benzyl-methyl-amide,
6-Bromo-pyrazolo[1,5-a]pyridine-2-carboxylic acid methyl-(1-phenyl-ethyl)-amide,
6-Chloro-pyrazolo[1,5-a]pyridine-2-carboxylic acid methyl-(1-phenyl-ethyl)-amide,
(4,6-Dichloro-pyrazolo[1,5-a]pyridin-2-yl)-(3,4-dihydro-1H-isoquinolin-2-yl)-methanone,
(4,6-Dichloro-pyrazolo[1,5-a]pyridin-2-yl)-(1-methyl-3,4-dihydro-1H-isoquinolin-2-yl)-methanone,
(4,6-Dichloro-pyrazolo[1,5-a]pyridin-2-yl)-(7-fluoro-1-methyl-3,4-dihydro-1H-isoquinolin-2-yl)-methanone,
(3-Bromo-7,8-dihydro-5H-1,6-naphthyridin-6-yl)-(4,6-dichloro-pyrazolo[1,5-a]pyridin-2-yl)-methanone,
(4,6-Dichloro-pyrazolo[1,5-a]pyridin-2-yl)-(7-pyridin-2-yl-3,4-dihydro-1H-isoquinolin-2-yl)-methanone,
(4,6-Dichloro-pyrazolo[1,5-a]pyridin-2-yl)-(4-methyl-6,7-dihydro-4H-thieno[3,2-c]pyridin-5-yl)-methanone,
(4,6-Dichloro-pyrazolo[1,5-a]pyridin-2-yl)-(4-methyl-6,7-dihydro-4H-furo[3,2-c]pyridin-5-yl)-methanone,
(4,6-Dichloro-pyrazolo[1,5-a]pyridin-2-yl)-(1-methyl-3,4-dihydro-1H-cinnolin-2-yl)-methanone,
(4,6-Dichloro-pyrazolo[1,5-a]pyridin-2-yl)-(5,6-dihydro-8H-imidazo[1,2-a]pyrazin-7-yl)-methanone,
(4,6-Dichloro-pyrazolo[1,5-a]pyridin-2-yl)-(5,6-dihydro-8H-[1,2,4]triazolo[4,3-a]pyrazin-7-yl)-methanone,
(4,6-Dichloro-pyrazolo[1,5-a]pyridin-2-yl)-(1-methyl-3,4-dihydro-1H-pyrrolo[1,2-a]pyrazin-2-yl)-methanone,
4,6-Dichloro-pyrazolo[1,5-a]pyridine-2-carboxylic acid methyl-(1-phenyl-ethyl)-amide,
4,6-Dichloro-pyrazolo[1,5-a]pyridine-2-carboxylic acid benzyl-methyl-amide,
4,6-Dichloro-pyrazolo[1,5-a]pyridine-2-carboxylic acid (2-methyl-2-phenyl-propyl)-amide,
4,6-Dichloro-pyrazolo[1,5-a]pyridine-2-carboxylic acid adamantyl-1 amide,
(4,6-Dichloro-pyrazolo[1,5-a]pyridin-2-yl)-((R)-1-methyl-3,4-dihydro-1H-isoquinolin-2-yl)-methanone,
(4,6-Dibromo-pyrazolo[1,5-a]pyridin-2-yl)-(3,4-dihydro-1H-isoquinolin-2-yl)-methanone,
(4,6-Dibromo-pyrazolo[1,5-a]pyridin-2-yl)-(1-methyl-3,4-dihydro-1H-isoquinolin-2-yl)-methanone,
(4,6-Dibromo-pyrazolo[1,5-a]pyridin-2-yl)-(7-fluoro-1-methyl-3,4-dihydro-1H-isoquinolin-2-yl)-methanone,
(3-Bromo-7,8-dihydro-5H-1,6-naphthyridin-6-yl)-(4,6-dibromo-pyrazolo[1,5-a]pyridin-2-yl)-methanone,
(4,6-Dibromo-pyrazolo[1,5-a]pyridin-2-yl)-(7-pyridin-2-yl-3,4-dihydro-1H-isoquinolin-2-yl)-methanone,
(4,6-Dibromo-pyrazolo[1,5-a]pyridin-2-yl)-(4-methyl-6,7-dihydro-4H-thieno[3,2-c]pyridin-5-yl)-methanone,
(4,6-Dibromo-pyrazolo[1,5-a]pyridin-2-yl)-(4-methyl-6,7-dihydro-4H-furo[3,2-c]pyridin-5-yl)-methanone,
(4,6-Dibromo-pyrazolo[1,5-a]pyridin-2-yl)-(1-methyl-3,4-dihydro-1H-cinnolin-2-yl)-methanone,
(4,6-Dibromo-pyrazolo[1,5-a]pyridin-2-yl)-(5,6-dihydro-8H-imidazo[1,2-a]pyrazin-7-yl)-methanone,
(4,6-Dibromo-pyrazolo[1,5-a]pyridin-2-yl)-(5,6-dihydro-8H-[1,2,4]triazolo[4,3-a]pyrazin-7-yl)-methanone,
(4,6-Dibromo-pyrazolo[1,5-a]pyridin-2-yl)-(1-methyl-3,4-dihydro-1H-pyrrolo[1,2-a]pyrazin-2-yl)-methanone,
4,6-Dibromo-pyrazolo[1,5-a]pyridine-2-carboxylic acid methyl-(1-phenyl-ethyl)-amide,
4,6-Dibromo-pyrazolo[1,5-a]pyridine-2-carboxylic acid benzyl-methyl-amide,
4,6-Dibromo-pyrazolo[1,5-a]pxidine-2-carboxylic acid (2-methyl-2-phenyl-propyl)-amide,
4,6-Dibromo-pyrazolo[1,5-a]pyridine-2-carboxylic acid adamantyl-1 amide,
(4,6-Dibromo-pyrazolo[1,5-a]pyridin-2-yl)-((R)-1-methyl-3,4-dihydro-1H-isoquinolin-2-yl)-methanone,
(6-Bromo-4-methoxy-pyrazolo[1,5-a]pyridin-2-yl)-(3,4-dihydro-1H-isoquinolin-2-yl)-methanone,
(6-Bromo-4-methoxy-pyrazolo[1,5-a]pyridin-2-yl)-(1-methyl-3,4-dihydro-1H-isoquinolin-2-yl)-methanone,
(6-Bromo-4-methoxy-pyrazolo[1,5-a]pyridin-2-yl)-(7-fluoro-1-methyl-3,4-dihydro-1H-isoquinolin-2-yl)-methanone,
(3-Bromo-7,8-dihydro-5H-1,6-naphthyridin-6-yl)-(6-bromo-4-methoxy-pyrazolo[ 1,5-a]pyridin-2-yl)-methanone,
(6-Bromo-4-methoxy-pyrazolo[1,5-a]pyridin-2-yl)-(7-pyridin-2-yl-3,4-dihydro-1H-isoquinolin-2-yl)-methanone,
(6-Bromo-4-methoxy-pyrazolo[1,5-a]pyridin-2-yl)-(4-methyl-6,7-dihydro-4H-thieno[3,2-c]pyridin-5-yl)-methanone,
(6-Bromo-4-methoxy-pyrazolo[1,5-a]pxidin-2-yl)-(4-methyl-6,7-dihydro-4H-furo[3,2-c]pyridin-5-yl)-methanone,
(6-Bromo-4-methoxy-pyrazolo[1,5-a]pyridin-2-yl)-(1-methyl-3,4-dihydro-1H-cinnolin-2-yl)-methanone,
(6-Bromo-4-methoxy-pyrazolo[1,5-a]pyridin-2-yl)-(5,6-dihydro-8H-imidazo[1,2-a]pyrazin-7-yl)-methanone,
(6-Bromo-4-methoxy-pyrazolo[1,5-a]pyridin-2-yl)-(5,6-dihydro-8H-[1,2,4]triazolo[4,3-a]pyrazin-7-yl)-methanone,
(6-Bromo-4-methoxy-pyrazolo[1,5-a]pyridin-2-yl)-(1-methyl-3,4-dihydro-1H-pyrrolo[1,2-a]pyrazin-2-yl)-methanone,
6-Bromo-4-methoxy-pyrazolo[1,5-a]pyridine-2-carboxylic acid methyl-(1-phenyl-ethyl)-amide,
6-Bromo-4-methoxy-pyrazolo[1,5-a]pyridine-2-carboxylic acid benzyl-methyl-amide,
6-Bromo-4-methoxy-pyrazolo[1,5-a]pyridine-2-carboxylic acid (2-methyl-2-phenylpropyl)-amide,
6-Bromo-4-methoxy-pyrazolo[1,5-a]pyridine-2-carboxylic acid adamantyl-1 amide,
(6-Bromo-4-methoxy-pyrazolo[1,5-a]pyridin-2-yl)-((R)-1-methyl-3,4-dihydro-1H-isoquinolin-2-yl)-methanone,
(6-Chloro-4-methoxy-pyrazolo[1,5-a]pyridin-2-yl)-(3,4-dihydro-H-isoquinolin-2-yl)-methanone,
(6-Chloro-4-methoxy-pyrazolo[1,5-a]pyridin-2-yl)-(1-methyl-3,4-dihydro-1H-isoquinolin-2-yl)-methanone,
(6-Chloro-4-methoxy-pyrazolo[1,5-alpyridin-2-yl)-(7-fluoro-1-methyl-3,4-dihydro-1H-isoquinolin-2-yl)-methanone,
(3-Bromo-7,8-dihydro-5H-1,6-naphthyridin-6-yl)-(6-chloro-4-methoxy-pyrazolo[1,5-a]pyridin-2-yl)-methanone,
(6-Chloro-4-methoxy-pyrazolo[1,5-a]pyridin-2-yl)-(7-pyridin-2-yl-3,4-dihydro-1H-isoquinolin-2-yl)-methanone,
(6-Chloro-4-methoxy-pyrazolo[1,5-a]pyridin-2-yl)-(4-methyl-6,7-dihydro-4H-thieno[3,2-c]pyridin-5-yl)-methanone,
(6-Chloro-4-methoxy-pyrazolo[1,5-a]pyridin-2-yl)-(4-methyl-6,7-dihydro-4H-furo[3,2-c]pyridin-5-yl)-methanone,
(6-Chloro-4-methoxy-pyrazolo[1,5-a]pyridin-2-yl)-(1-methyl-3,4-dihydro-1H-cinnolin-2-yl)-methanone,
(6-Chloro-4-methoxy-pyrazolo[1,5-a]pyridin-2-yl)-(5,6-dihydro-8H-imidazo[1,2-a]pyrazin-7-yl)-methanone,
(6-Chloro-4-methoxy-pyrazolo[1,5-a]pyridin-2-yl)-(5,6-dihydro-8H-[1,2,4]triazolo[4,3-a]pyrazin-7-yl)-methanone,
(6-Chloro-4-methoxy-pyrazolo[1,5-a]pyridin-2-yl)-(1-methyl-3,4-dihydro-1H-pyrrolo[ 1,2-a]pyrazin-2-yl)-methanone,
6-Chloro-4-methoxy-pyrazolo[1,5-a]pyridine-2-carboxylic acid methyl-(1-phenyl-ethyl)-amide,
6-Chloro-4-methoxy-pyrazolo[1,5-a]pyridine-2-carboxylic acid benzyl-methyl-amide,
6-Chloro-4-methoxy-pyrazolo[1,5-a]pyridine-2-carboxylic acid (2-methyl-2-phenylpropyl)-amide,
6-Chloro-4-methoxy-pyrazolo[1,5-a]pyridine-2-carboxylic acid adamantyl-1 amide,
(6-Chloro-4-methoxy-pyrazolo[1,5-a]pyridin-2-yl)-((R)-1-methyl-3,4-dihydro-1H-isoquinolin-2-yl)-methanone,
(6-Chloro-4-nitro-pyrazolo[1,5-a]pyridin-2-yl)-(3,4-dihydro-1H-isoquinolin-2-yl)-methanone,
(6-Chloro-4-nitro-pyrazolo[1,5-a]pyridin-2-yl)-(1-methyl-3,4-dihydro-1H-isoquinolin-2-yl)-methanone,
(6-Chloro-4-nitro-pyrazolo[1,5-a]pyridin-2-yl)-(7-fluoro-1-methyl-3,4-dihydro-1H-isoquinolin-2-yl)-methanone,
(3-Bromo-7,8-dihydro-5H-1,6-naphthyridin-6-yl)-(6-chloro-4-nitro-pyrazolo[1,5-a]pyridin-2-yl)-methanone,
(6-Chloro-4-nitro-pyrazolo[1,5-a]pyridin-2-yl)-(7-pyridin-2-yl-3,4-dihydro-1H-isoquinolin-2-yl)-methanone,
(6-Chloro-4-nitro-pyrazolo[1,5-a]pyridin-2-yl)-(4-methyl-6,7-dihydro-4H-thieno[3,2-c]pyridin-5-yl)-methanone,
(6-Chloro-4-nitro-pyrazolo[1,5-a]pyridin-2-yl)-(4-methyl-6,7-dihydro-4H-furo[3,2-c]pyridin-5-yl)-methanone,
(6-Chloro-4-nitro-pyrazolo[1,5-a]pyridin-2-yl)-(1-methyl-3,4-dihydro-1H-cinnolin-2-yl)-methanone,
(6-Chloro-4-nitro-pyrazolo[1,5-a]pyridin-2-yl)-(5,6-dihydro-8H-imidazo[1,2-a]pyrazin-7-yl)-methanone,
(6-Chloro-4-nitro-pyrazolo[1,5-a]pyridin-2-yl)-(5,6-dihydro-8H-[1,2,4]triazolo[4,3-a]pyrazin-7-yl)-methanone,
(6-Chloro-4-nitro-pyrazolo[1,5-a]pyridin-2-yl)-(1-methyl-3,4-dihydro-1H-pyrrolo[1,2-a]pyrazin-2-yl)-methanone,
6-Chloro-4-nitro-pyrazolo[1,5-a]pyridine-2-carboxylic acid methyl-(1-phenyl-ethyl)-amide,
6-Chloro-4-nitro-pyrazolo[1,5-a]pyridine-2-carboxylic acid benzyl-methyl-amide,
6-Chloro-4-nitro-pyrazolo[1,5-a]pyridine-2-carboxylic acid (2-methyl-2-phenyl-propyl)-amide,
6-Chloro-4-nitro-pyrazolo[1,5-a]pyridine-2-carboxylic acid adamantyl-1 amide,
(6-Chloro-4-nitro-pyrazolo[1,5-a]pyridin-2-yl)-((R)-1-methyl-3,4-dihydro-1H-isoquinolin-2-yl)-methanone,
(6-Bromo-4-nitro-pyrazolo[1,5-a]pyridin-2-yl)-(3,4-dihydro-1H-isoquinolin-2-yl)-methanone,
(6-Bromo-4-nitro-pyrazolo[1,5-a]pyridin-2-yl)-(1-methyl-3,4-dihydro-1H-isoquinolin-2-yl)-methanone,
(6-bromo-4-nitro-pyrazolo[1,5-a]pyridin-2-yl)-(7-fluoro-1-methyl-3,4-dihydro-1H-isoquinolin-2-yl)-methanone,
(3-Bromo-7,8-dihydro-5H-1,6-naphthyridin-6-yl)-(6-bromo-4-nitro-pyrazolo[1,5-a]pyridin-2-yl)-methanone,
(6-Bromo-4-nitro-pyrazolo[1,5-a]pyridin-2-yl)-(7-pyridin-2-yl-3,4-dihydro-1H-isoquinolin-2-yl)-methanone,
(6-bromo-4-nitro-pyrazolo[1,5-a]pyridin-2-yl)-(4-methyl-6,7-dihydro-4H-thieno[3,2-c]pyridin-5-yl)-methanone,
(6-Bromo-4-nitro-pyrazolo[1,5-a]pyridin-2-yl)-(4-methyl-6,7-dihydro-4H-furo[3,2-c]pyridin-5-yl)-methanone,
(6-Bromo-4-nitro-pyrazolo[1,5-a]pyridin-2-yl)-(1-methyl-3,4-dihydro-1H-cinnolin-2-yl)-methanone,
(6-Bromo-4-nitro-pyrazolo[1,5-a]pyridin-2-yl)-(5,6-dihydro-8H-imidazo[1,2-a]pyrazin-7-yl)-methanone,
(6-Bromo-4-nitro-pyrazolo[1,5-a]pyridin-2-yl)-(5,6-dihydro-8H-[1,2,4]triazolo[4,3-a]pyrazin-7-yl)-methanone,
(6-Bromo-4-nitro-pyrazolo[1,5-a]pyridin-2-yl)-(1-methyl-3,4-dihydro-1H-pyrrolo[1,2-a]pyrazin-2-yl)-methanone,
6-Bromo-4-nitro-pyrazolo[1,5-a]pyridine-2-carboxylic acid methyl-(1-phenyl-ethyl)-amide,
6-Bromo-4-nitro-pyrazolo[1,5-a]pyridine-2-carboxylic acid benzyl-methyl-amide,
6-Bromo-4-nitro-pyrazolo[1,5-a]pyridine-2-carboxylic acid (2-methyl-2-phenyl-propyl)-amide,
6-Bromo-4-nitro-pyrazolo[1,5-a]pyridine-2-carboxylic acid adamantyl-1 amide,
(6-Bromo-4-nitro-pyrazolo[1,5-a]pyridin-2-yl)-((R)-1-methyl-3,4-dihydro-1H-isoquinolin-2-yl)-methanone
and optical isomers, polymorphs, pharmaceutically-acceptable acid and base addition salts, hydrates, and solvates thereof.

**23.** Use of a compound as defined in claim 1 for treating or preventing a condition or disease associated with abnormal glutamate neurotransmission, or for modulating mGluR5 receptors to achieve therapeutic benefit.

**24.** Use as claimed in claim 23, wherein the condition associated with abnormal glutamate transmission, or wherein modulation of mGluR5 receptors results in therapeutic benefit is selected from: Alzheimer's disease, Creutzfeld-Jakob's syndrome/disease, bovine spongiform encephalopathy (BSE), prion related infections, diseases involving mitochondrial dysfunction, diseases involving β-amyloid and/or tauopathy, Down's syndrome, hepatic encephalopathy, Huntington's disease, motor neuron diseases, amyotrophic lateral sclerosis (ALS), olivoponto-cerebellar atrophy, post-operative cognitive deficit (POCD), systemic lupus erythematosus, systemic clerosis, Sjogren's syndrome, Neuronal Ceroid Lipofuscinosis, neurodegenerative cerebellar ataxias, Parkinson's disease, Parkinson's dementia, mild cognitive impairment, cognitive deficits in various forms of mild cognitive impairment, cognitive deficits in various forms of dementia, dementia pugilistica, vascular and frontal lobe dementia, cognitive impairment, learning impairment, eye injuries, eye diseases, eye disorders, glaucoma, retinopathy, macular degeneration, head or brain or spinal cord injuries, head or brain or spinal cord trauma, trauma, hypoglycaemia, hypoxia, perinatal hypoxia, ischaemia, ischaemia resulting from cardiac arrest or stroke or bypass operations or transplants, convulsions, epileptic convulsions, epilepsy, temporal lobe epilepsy, myoclonic epilepsy, inner ear insult, inner ear insult in tinnitus, tinnitus, sound- or drug-induced inner ear insult, sound- or drug-induced tinnitus, L-dopa-induced dykinesias, L-dopa-induced dykinesias in Parkinson's disease therapy, dyskinesias, dyskinesia in Huntington's disease, drug induced dyskinesias, neuroleptic-induced dyskinesias, haloperidol-induced dyskinesias, dopaminomimetic-induced dyskinesias, chorea, Huntington's chorea, athetosis, dystonia, stereotypy, ballism, tardive dyskinesias, tic disorder, torticollis spasmodicus, blepharospasm, focal and generalized dystonia, nystagmus, hereditary cerebellar ataxias, corticobasal degeneration, tremor, essential tremor, abuse, addiction, nicotine addiction, nicotine abuse, alcohol addiction, alcohol abuse, opiate addiction, opiate abuse, cocaine addiction, cocaine abuse, amphetamine addiction, amphetamine abuse, anxiety disorders, panic disorders, anxiety and panic disorders, social anxiety disorder (SAD), attention deficit hyperactivity disorder (ADHD), attention deficit syndrome (ADS), restless leg syndrome (RLS), hyperactivity in children, autism, dementia, dementia in Alzheimer's disease, dementia in Korsakoff syndrome, Korsakoff syndrome, vascular dementia, dementia related to HIV infections, HIV-1 encephalopathy, AIDS encephalopathy, AIDS dementia complex, AIDS-related dementia, major depressive disorder, major depression, depression, depression resulting from Borna virus infection, major depression resulting from Borna virus infection, bipolar manic-depressive disorder, drug tolerance, drug tolerance to opioids, movement disorders, fragile-X syndrome, irritable bowel syndrome (IBS), migraine, multiple sclerosis (MS), muscle spasms, pain, chronic pain, acute pain, inflammatory pain, neuropathic pain, diabetic neuropathic pain (DNP), pain related to rheumatic arthritis, allodynia, hyperalgesia, nociceptive pain, cancer pain, posttraumatic stress disorder (PTSD), schizophrenia, positive or cognitive or negative symptoms of schizophrenia, spasticity, Tourette's syndrome, urinary incontinence, vomiting, pruritic conditions, pruritis, sleep disorders, micturition disorders, neuromuscular disorder in the lower urinary tract, gastroesophageal reflux disease (GERD), gastrointestinal dysfunction, lower esophageal sphincter (LES) disease, functional gastrointestinal disorders, dyspepsia, regurgitation, respiratory tract infection, bulimia nervosa, chronic laryngitis, asthma, reflux-related asthma, lung disease, eating disorders, obesity, obesity-related disorders, obesity abuse, food addiction, binge eating disorders, agoraphobia, generalized anxiety disorder, obsessive-compulsive disorder, panic disorder, posttraumatic stress disorder, social phobia, phobic disorders, substance-induced anxiety disorder, delusional disorder, schizoaffective disorder, schizophreniform disorder, substance-induced psychotic disorder, or delirium; inhibition of tumour cell growth, migration, invasion, adhesion and toxicity in the peripheral tissues, peripheral nervous system and CNS; neoplasia, hyperplasia, dysplasia, cancer, carcinoma, sarcoma, oral cancer, squamous cell carcinoma (SCC), oral squamous cell carcinoma (SCC), lung cancer, lung adenocarcinoma, breast cancer, prostate cancer, gastric cancer, liver cancer, colon cancer, colorectal carcinoma, rhabdomyosarcoma, brain tumour, tumour of a nerve tissue, glioma, malignant glioma, astroglioma, neuroglioma, neuroblastoma, glioblastoma, medulloblastoma, cancer of skin cells, melanoma, malignant melanoma, epithelial neoplasm, lymphoma, myeloma, Hodgkin's disease, Burkitt's lymphoma, leukemia, thymoma, tumours, diabetes, hyperammonemia and liver failure and sleep disturbances..

**25.** Use of a compound as defined in claim 1 for the manufacture of a medicament for treating or preventing a condition or disease associated with abnormal glutamate neurotransmission, or for modulating mGluR5 receptors to achieve therapeutic benefit.

**26.** Use as claimed in claim 25, wherein the condition associated with abnormal glutamate transmission, or wherein modulation of mGluR5 receptors results in therapeutic benefit is selected from: Alzheimer's disease, Creutzfeld-Jakob's syndrome/disease, bovine spongiform encephalopathy (BSE), prion related infections, diseases involving mitochondrial dysfunction, diseases involving β-amyloid and/or tauopathy, Down's syndrome, hepatic encephalopathy, Huntington's disease, motor neuron diseases, amyotrophic lateral sclerosis (ALS), olivoponto-cerebellar atrophy, post-operative cognitive deficit (POCD), systemic lupus erythematosus, systemic clerosis, Sjogren's syndrome, Neuronal Ceroid Lipofuscinosis, neurodegenerative cerebellar ataxias, Parkinson's disease, Parkinson's dementia, mild cognitive impairment, cognitive deficits in various forms of mild cognitive impairment, cognitive deficits in various forms of dementia, dementia pugilistica, vascular and frontal lobe dementia, cognitive impairment, learning impairment, eye injuries, eye diseases, eye disorders, glaucoma, retinopathy, macular degeneration, head or brain or spinal cord injuries, head or brain or spinal cord trauma, trauma, hypoglycaemia, hypoxia, perinatal hypoxia, ischaemia, ischaemia resulting from cardiac arrest or stroke or bypass operations or transplants, convulsions, epileptic convulsions, epilepsy, temporal lobe epilepsy, myoclonic epilepsy, inner ear insult, inner ear insult in tinnitus, tinnitus, sound- or drug-induced inner ear insult, sound- or drug-induced tinnitus, L-dopa-induced dykinesias, L-dopa-induced dykinesias in Parkinson's disease therapy, dyskinesias, dyskinesia in Huntington's disease, drug induced dyskinesias, neuroleptic-induced dyskinesias, haloperidol-induced dyskinesias, dopaminomimetic-induced dyskinesias, chorea, Huntington's chorea, athetosis, dystonia, stereotypy, ballism, tardive dyskinesias, tic disorder, torticollis spasmodicus, blepharospasm, focal and generalized dystonia, nystagmus, hereditary cerebellar ataxias, corticobasal degeneration, tremor, essential tremor, abuse, addiction, nicotine addiction, nicotine abuse, alcohol addiction, alcohol abuse, opiate addiction, opiate abuse, cocaine addiction, cocaine abuse, amphetamine addiction, amphetamine abuse, anxiety disorders, panic disorders, anxiety and panic disorders, social anxiety disorder (SAD), attention deficit hyperactivity disorder (ADHD), attention deficit syndrome (ADS), restless leg syndrome (RLS), hyperactivity in children, autism, dementia, dementia in Alzheimer's disease, dementia in Korsakoff syndrome, Korsakoff syndrome, vascular dementia, dementia related to HIV infections, HIV-1 encephalopathy, AIDS encephalopathy, AIDS dementia complex, AIDS-related dementia, major depressive disorder, major depression, depression, depression resulting from Borna virus infection, major depression resulting from Borna virus infection, bipolar manic-depressive disorder, drug tolerance, drug tolerance to opioids, movement disorders, fragile-X syndrome, irritable bowel syndrome (IBS), migraine, multiple sclerosis (MS), muscle spasms, pain, chronic pain, acute pain, inflammatory pain, neuropathic pain, diabetic neuropathic pain (DNP), pain related to rheumatic arthritis, allodynia, hyperalgesia, nociceptive pain, cancer pain, posttraumatic stress disorder (PTSD), schizophrenia, positive or cognitive or negative symptoms of schizophrenia, spasticity, Tourette's syndrome, urinary incontinence, vomiting, pruritic conditions, pruritis, sleep disorders, micturition disorders, neuromuscular disorder in the lower urinary tract, gastroesophageal reflux disease (GERD), gastrointestinal dysfunction, lower esophageal sphincter (LES) disease, functional gastrointestinal disorders, dyspepsia, regurgitation, respiratory tract infection, bulimia nervosa, chronic laryngitis, asthma, reflux-related asthma, lung disease, eating disorders, obesity, obesity-related disorders, obesity abuse, food addiction, binge eating disorders, agoraphobia, generalized anxiety disorder, obsessive-compulsive disorder, panic disorder, posttraumatic stress disorder, social phobia, phobic disorders, substance-induced anxiety disorder, delusional disorder, schizoaffective disorder, schizophreniform disorder, substance-induced psychotic disorder, or delirium; inhibition of tumour cell growth, migration, invasion, adhesion and toxicity in the peripheral tissues, peripheral nervous system and CNS; neoplasia, hyperplasia, dysplasia, cancer, carcinoma, sarcoma, oral cancer, squamous cell carcinoma (SCC), oral squamous cell carcinoma (SCC), lung cancer, lung adenocarcinoma, breast cancer, prostate cancer, gastric cancer, liver cancer, colon cancer, colorectal carcinoma, rhabdomyosarcoma, brain tumour, tumour of a nerve tissue, glioma, malignant glioma, astroglioma, neuroglioma, neuroblastoma, glioblastoma, medulloblastoma, cancer of skin cells, melanoma, malignant melanoma, epithelial neoplasm, lymphoma, myeloma, Hodgkin's disease, Burkitt's lymphoma, leukemia, thymoma, tumours, diabetes, hyperammonemia and liver failure and sleep disturbances.

**27.** Use as claimed in claim 25, wherein the condition associated with abnormal glutamate transmission, or wherein modulation of mGluR5 receptors results in therapeutic benefit is selected from: chronic pain, neuropathic pain, diabetic neuropathic pain (DNP), cancer pain, pain related to rheumathic arthritis, inflammatory pain, L-dopa-induced dyskinesias, dopaminomimetic-induced dyskinesias, L-dopa-induced dyskinesias in Parkinson's disease therapy, dopaminomimetic-induced dyskinesias in Parkinson's disease therapy, tardive dyskinesias, Parkinson's disease, anxiety disorders, panic disorders, anxiety and panic disorders, social anxiety disorder (SAD), generalized anxiety disorder, substance-induced anxiety disorder, eating disorders, obesity, binge eating disorders, Huntington's chorea, epilepsy, Alzheimer's disease, positive and negative symptoms of schizophrenia, cognitive impairment, functional gastrointestinal disorders, gastroesophageal reflux disease (GERD), migraine, irritable bowel syndrome (IBS), cognitive enhancement and neuroprotection.

**28.** Use as claimed in claim 25, wherein the condition associated with abnormal glutamate transmission, or wherein modulation of mGluR5 receptors results in therapeutic benefit is selected from: chronic pain, neuropathic pain, diabetic neuropathic pain (DNP), cancer pain, pain related to rheumathic arthritis, inflammatory pain, L-dopa-induced dyskinesias, dopaminomimetic-induced dyskinesias, L-dopa-induced dyskinesias in Parkinson's disease therapy, dopaminomimetic-induced dyskinesias in Parkinson's disease therapy, tardive dyskinesias, Parkinson's disease, anxiety disorders, panic disorders, anxiety and panic disorders, social anxiety disorder (SAD), generalized anxiety disorder, substance-induced anxiety disorder, eating disorders, obesity, binge eating disorders, migraine, irritable bowel syndrome (IBS), functional gastrointestinal disorders, gastroesophageal reflux disease (GERD), Huntington's chorea and epilepsy.

**29.** Use as claimed in claim 25, wherein the condition associated with abnormal glutamate transmission, or wherein modulation of mGluR5 receptors results in therapeutic benefit is selected from: Alzheimer's disease, positive and/or negative symptoms of schizophrenia, cognitive impairment, cognitive enhancement and neuroprotection.

**30.** Use as claimed in claim 25, wherein the condition associated with abnormal glutamate transmission, or wherein modulation of mGluR5 receptors results in therapeutic benefit, is a binge eating disorder.

**31.** A pharmaceutical composition comprising as active ingredient at least one compound as claimed in any of claims 1 to 22, together with one or more pharmaceutically acceptable excipients.

**32.** A pharmaceutical composition comprising a combination of at least one compound as claimed in any of claims 1 to 22 and at least one NMDA receptor antagonist, together with one or more pharmaceutically acceptable excipients.

**33.** A process for the synthesis of a compound selected from those of Formula **I** 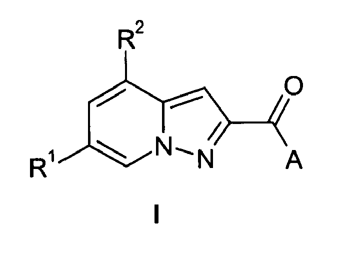 and optical isomers, pharmaceutically acceptable salts, hydrates, solvates, and polymorphs thereof, wherein a compound of Formula **II** 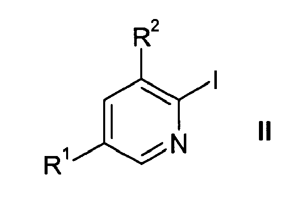 is dissolved in THF and treated with *iso*-propylmagnesium chloride solution (in THF), followed by reaction with dimethyl foramide and hydrochloric acid to yield a compound of Formula **III** 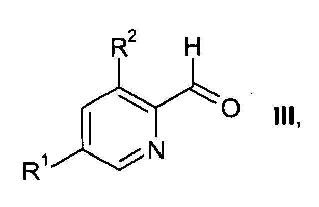 which then is reacted with a compound of Formula **IV** 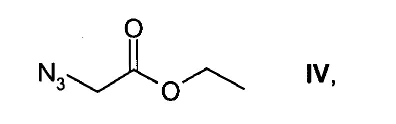 which compound **IV** may be obtained by treatment of a compound of Formula **VI** with sodium azide 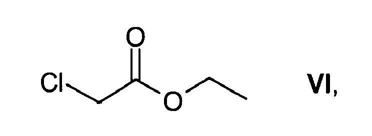 to yield a compound of Formula **V** 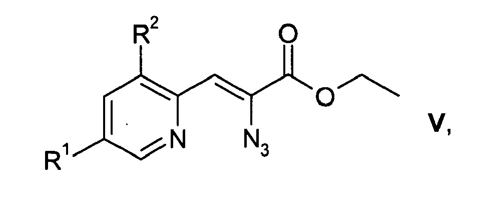 which compound **V** is heated to yield a pyrazolopyridine compound of Formula **VII** 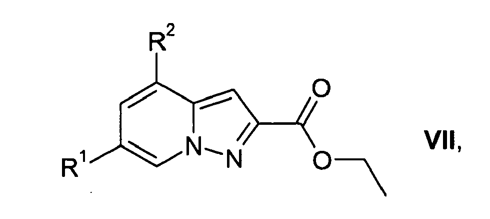 which is hydrolyzed under basic conditions to yield a compound of Formula **VIII** 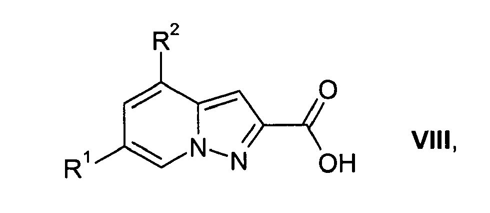 which then is treated with an amine of Formula **IX**
A-H IX
in the presence of a condensing agent, to yield a compound of Formula **I,** which is converted, if desired, to a pharmaceutically acceptable salt, hydrate, solvate, or polymorph.

**34.** A process for the synthesis of a compound selected from those of Formula **I** 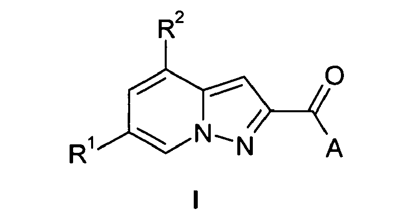 and optical isomers, pharmaceutically acceptable salts, hydrates, solvates, and polymorphs thereof, wherein a compound of Formula **X** 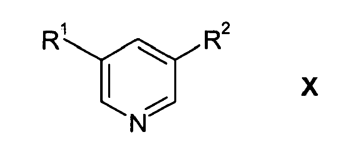 is treated with hydroxylamine-O-sulfonic acid in the presence of potassium carbonate to form an intermediate N-iminopyridinium species **XI** 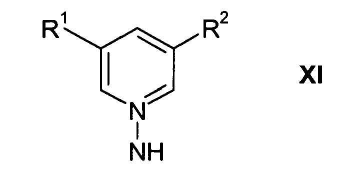 which is treated with hydriodic acid to yield a 1-aminopyridinium iodide **XII** 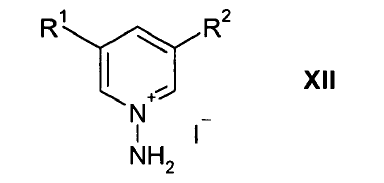 which is treated with dimethyl acetylenedicarboxylate **XIII** 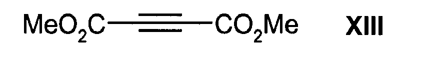 to yield a mixture of compounds **XIV** and **XV** 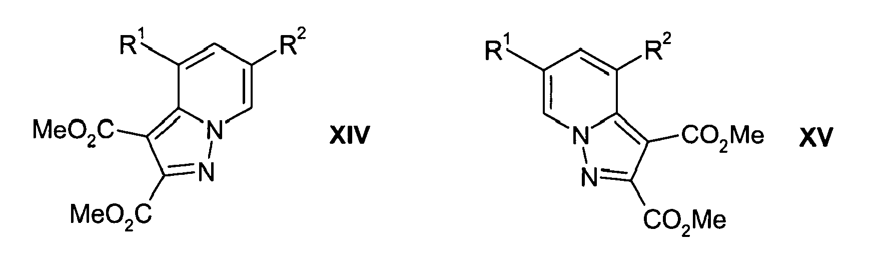 which compounds are separated via column chromatography followed by heating with concentrated sulfuric acid to yield a compound of Formula **VIII** 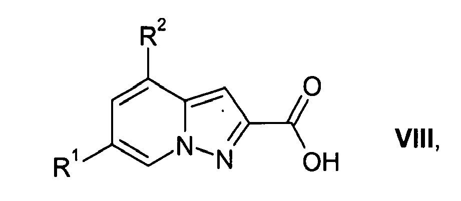 which then is treated with an amine of Formula **IX**
A-H IX
in the presence of a condensing agent to yield a compound of Formula I, which is converted, if desired, to a pharmaceutically acceptable salt, hydrate, solvate, or polymorph.
